(19) Europäisches Patentamt
European Patent Office
Office européen des brevets

(11) **EP 4 553 077 A1**

(12) **EUROPEAN PATENT APPLICATION**
published in accordance with Art. 153(4) EPC


(43) Date of publication:
**14.05.2025 Bulletin 2025/20**

(21) Application number: **23834895.7**

(22) Date of filing: **05.07.2023**

(51) International Patent Classification (IPC):
***C07D 487/04*** *(2006.01)* ***A61K 31/519*** *(2006.01)*
***A61P 35/00*** *(2006.01)*

(52) Cooperative Patent Classification (CPC):
**A61K 31/498; A61K 31/519; A61K 31/541; A61K 31/553; A61P 35/00; C07D 487/04; C07D 498/14; C07D 498/22**

(86) International application number:
**PCT/CN2023/105960**

(87) International publication number:
**WO 2024/008128 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.07.2022 CN 202210798720**



(71) Applicant: **Kinoteck Therapeutics Co., Ltd**
**Guangzhou, Guangdong 510630 (CN)**

(72) Inventors:
- **CHANG, Shaohua**
  **Shanghai 201210 (CN)**
- **CHEN, Xiaofei**
  **Shanghai 201210 (CN)**
- **LI, Jiaguo**
  **Shanghai 201210 (CN)**
- **LI, Huiqiong**
  **Shanghai 201210 (CN)**
- **WU, Pinglian**
  **Shanghai 201210 (CN)**
- **WU, Yinhui**
  **Shanghai 201210 (CN)**
- **REN, Xiaomei**
  **Shanghai 201210 (CN)**
- **MA, Dawei**
  **Shanghai 201210 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**


# (54) SULFOXIMINE COMPOUND HAVING FGFR INHIBITORY EFFECT, PHARMACEUTICAL COMPOSITION COMPRISING SAME, AND USE THEREOF


(57) Provided are a sulfoximine compound having an FGFR inhibitory effect, a pharmaceutical composition comprising said sulfoximine compound, and a use thereof. Specifically provided are a sulfoximine compound as represented by formula (I) which can be used as a fibroblast growth factor receptor (FGFR) inhibitor, a tautomer, a stereoisomer, a hydrate, a solvate, a pharmaceutically acceptable salt thereof, a pharmaceutical composition comprising the compound, and a use thereof in the preparation of a pharmaceutical composition; wherein $R_1$, $R_2$, $R_3$, $R_4$, $R_5$, $Cy^A$, $Cy^B$, $Cy^C$, etc., are as defined in the description.


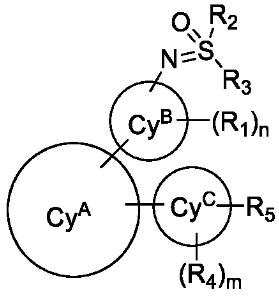

(I)

EP 4 553 077 A1

## Description

### TECHNICAL FIELD

The present invention relates to the field of medicinal chemistry, and specifically provides a structurally novel FGFR inhibitor.

### BACKGROUND

Fibroblast growth factor receptor (FGFR) is a receptor for fibroblast growth factor (FGF) signaling, and its family consists of four members (FGFR1, FGFR2, FGFR3, FGFR4), which are glycoproteins consisting of extracellular immunoglobulin (Ig) like domains, hydrophobic transmembrane regions, and intracellular portion including tyrosine kinase regions. The binding of FGF ligands induces receptor dimerization and conformational changes in the intracellular domain, which cause intermolecular phosphorylation of the kinase domain and intracellular tail. Phosphorylated residues act as docking sites for attachment proteins, which promote downstream signaling cascades that lead to cellular behavior including proliferation, survival, differentiation, migration, and angiogenesis. FGFRs signal abnormalities involve a variety of cancer types, including liver cancer, intrahepatic cholangiocarcinoma, bladder cancer, endometrial cancer, breast cancer and lung cancer, and disease progression occurs through overexpression, point mutation and/or chromosomal translocation.

With the continuous deepening of research, pan FGFR1-3 inhibitors have shown clinical responses to various cancers with altered FGFR, but also exhibit target-limited toxicity, which lead to adverse side effects such as hyperphosphatemia and tissue mineralization, which originate from the fact that the regulation of phosphate reabsorption is mediated by FGFR1 and FGFR3. Some studies have summarized that FGFR2 translocation occurred in 14% of intrahepatic cholangiocarcinoma; FGFR2 mutations occurred in 12-14% of endometrial cancers and 5% of squamous non-small cell lung cancers; FGFR2 was amplified in 12-14% of gastric cancer and 4% of breast cancer. FGFR2 plays a role in promoting acquired resistance to human epidermal growth factor receptor 2 (HER2) targeted therapy by indirectly overactivating FGFR2 in tumor associated fibroblasts.

Therefore, based on the clinical needs to be met, the development of FGFR2 selective inhibitors for treatment has great value and prospects.

### SUMMARY OF THE INVENTION

The purpose of the present invention is to develop a small molecule compound as FGFR inhibitor.

In the first aspect of the present invention, provided is a compound as shown in formula (I), a tautomer, a stereoisomer, a hydrate, a solvate, or a pharmaceutically acceptable salt thereof:

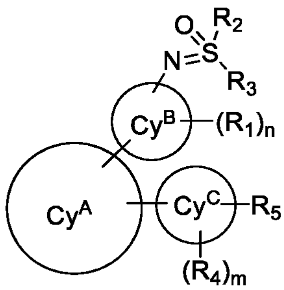

(I)

wherein, n and m are each independently 0, 1, 2, or 3;

$Cy^A$ is selected from the group consisting of:

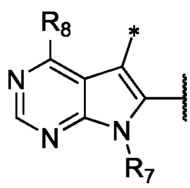 , 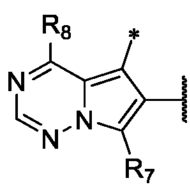 , 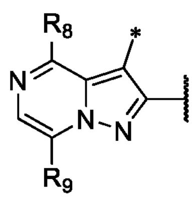 , 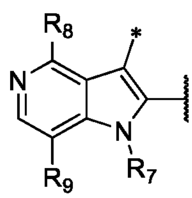 ,

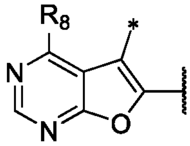, 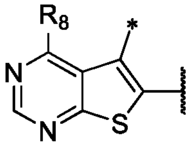, 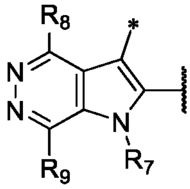, and 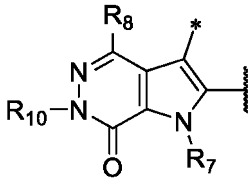;

wherein 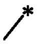 denotes a bond connected to $Cy^B$, and 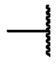 denotes a bond connected to $Cy^C$;

$Cy^B$ is selected from the group consisting of: $C_{6-10}$ aryl and 5-12 membered heteroaryl;

$Cy^C$ is selected from the group consisting of: $C_{6-10}$ aryl, 5-12-membered heteroaryl, saturated or partially unsaturated $C_{3-6}$ carbocyclyl, saturated or partially unsaturated 4-12-membered heterocyclyl;

$R_1$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ saturated or partially unsaturated carbocyclyl-O-, $SF_5$ and 4-12 membered heterocyclyl;

$R_2$ and $R_3$ are each independently selected from the group consisting of: $C_{1-6}$ alkyl, $C_{1-6}$ alkoxy, $C_{1-6}$ haloalkyl, $C_{1-6}$ haloalkoxy, $C_{1-6}$ alkylamino, $C_{3-6}$ saturated or partially unsaturated carbocyclyl, and 4-12-membered heterocyclyl; wherein $R_2$ and $R_3$ can be the same or different;

or $R_2$ and $R_3$ together with the sulfur atom to which they are connected form a 4-12-membered heterocyclyl;

$R_4$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ carbocyclyl-O-, $SF_5$ and 4-12-membered heterocyclyl;

$R_5$ is -L-Rw, wherein L is selected from the group consisting of: covalent bond, -$C_{1-4}$ alkyl-, -$NR_6$-, and -$C_{1-4}$ alkyl $NR_6$-;

$R_6$ is selected from the group consisting of: H, $C_{1-6}$ alkyl, $C_{1-6}$ alkoxy, $C_{1-6}$ haloalkyl and $C_{1-6}$ haloalkoxy;

Rw is selected from the group consisting of: halogen, cyano,

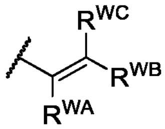, 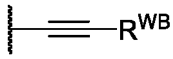,

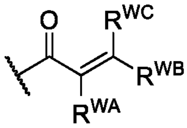, 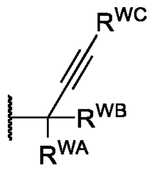, 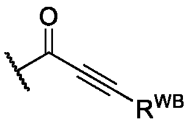, 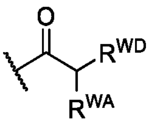, 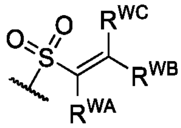, 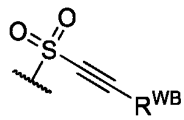,

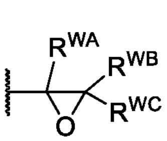, 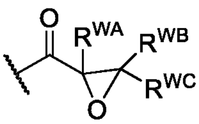, 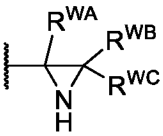, 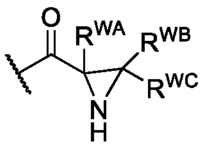, 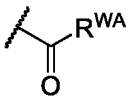, 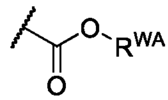,

and 5-12-membered nitrogen-containing heterocyclyl;

$R^{WA}$, $R^{WB}$, and $R^{WC}$ are each independently selected from the group consisting of: H, D, halogen, CN, C(O)Ra, C(O)ORa, NRaRb, C(O)NRaRb, C(O)NRaORb, or selected from the group consisting of: $C_{1-6}$ alkyl, $C_{1-6}$ alkoxy, $C_{1-6}$ haloalkyl, $C_{1-6}$ haloalkoxy, phenyl, 3 to 7-membered saturated or partially unsaturated heterocyclyl with 1 to 2 heteroatoms independently selected from nitrogen, oxygen and sulfur, and 5 to 6-membered heteroaryl with 1 to 4 heteroatoms independently selected from nitrogen, oxygen and sulfur; and $R^{WA}$, $R^{WB}$, and $R^{WC}$ can each be independently substituted with 1, 2, or 3 substituents selected from the group consisting of: $C_{1-6}$ alkylamino and $C_{1-6}$ alkoxy;

or $R_6$ and Rw together form a ring; and the ring is selected from the group consisting of: 5-12 membered heteroaryl and 4-12 membered heterocyclyl;

$R_7$ is selected from the group consisting of: H, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{6-10}$ aryl, 5-12 membered heteroaryl and 4-12-membered heterocyclyl; or $R_4$ and $R_7$, as well as the atoms between them, together form a ring, and the ring is selected from the group consisting of: $C_{6-8}$ saturated or partially unsaturated carbocyclyl, $C_{6-10}$ aryl, 6-12 membered heteroaryl and 6-12 membered saturated or partially unsaturated heterocyclyl;

$R_8$ is selected from the group consisting of: H, OH, $NH_2$, $C_{1-6}$ alkyl, $C_{1-6}$ alkoxy, $C_{1-6}$ haloalkyl, $C_{1-6}$ haloalkoxy and $C_{1-6}$ alkylamino;

$R_9$ is selected from the group consisting of: H, D, halogen, CN, $NH_2$, $C_{1-6}$ alkyl, $C_{1-6}$ alkoxy, $C_{1-6}$ haloalkyl, $C_{1-6}$ haloalkoxy and $C_{1-6}$ alkylamino;

unless otherwise specified, each of the above-mentioned alkyl, alkoxy, haloalkyl, haloalkoxy, alkylamino, alkenyl, haloalkenyl, alkynyl, haloalkynyl, saturated or partially unsaturated carbocyclyl, halogenated saturated or partially unsaturated carbocyclyl, saturated or partially unsaturated heterocyclyl, aryl and heteroaryl can be substituted with one or more Ra;

Ra and Rb are each independently selected from H, D, halogen, CN, oxo (=O), OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkyenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ saturated or partially unsaturated carbocyclyl-O- and 4-12 membered heterocyclyl;

wherein, the heterocyclyl is a saturated or partially unsaturated non-aromatic group; the carbocyclyl or heterocyclyl can be optionally in the form of a monocycle, bridged ring, spirocycle, or fused ring.

In some embodiments, the compound has a structure as shown in formula (I-1):

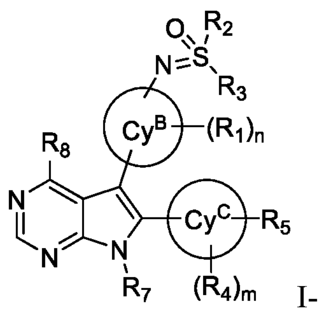

I-1

wherein, $R_1$, $R_2$, $R_3$, $R_4$, $R_5$, $R_7$, $R_8$, $Cy^B$, and $Cy^C$ have the definitions as claimed in claim 1;

or $R_7$, $R_4$, and atoms to which they connected together form a 5-12 membered carbocyclyl or heterocyclyl.

In some embodiments, the compound has a structure as shown in formula (I-2):

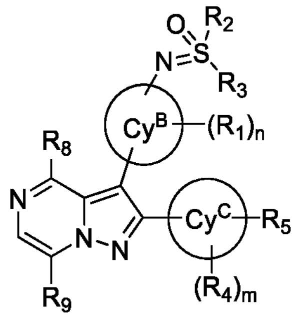

I-2.

**In** another preferred embodiment, $R_8$ is selected from the group consisting of: H, $NH_2$, $C_{1-6}$ alkyl, and $C_{1-6}$ alkylamino.

In another preferred embodiment, $R_9$ is selected from the group consisting of: H, D, halogen, CN, $C_{1-6}$ alkyl and $C_{1-6}$ haloalkyl.

In some embodiments, $Cy^B$ is selected from the group consisting of: phenyl and 5-7 membered heteroaryl.

In another preferred embodiment, $Cy^B$ is selected from the group consisting of: phenyl, pyridyl, pyrimidinyl, pyridazinyl, pyrrolyl, furanyl, thiophenyl, imidazolyl, pyrazolyl, thiazolyl, triazolyl, and oxazolyl.

In some embodiments, $Cy^C$ is selected from the group consisting of: phenyl, 5-7 membered heteroaryl, saturated or partially unsaturated $C_{3-6}$ saturated or partially unsaturated carbocyclyl, and saturated or partially unsaturated 4-7 membered heterocyclyl.

In another preferred embodiment, $Cy^C$ is selected from the group consisting of: phenyl, pyridyl, pyrimidinyl, pyridazinyl, pyrrolyl, furanyl, thiophenyl, imidazolyl, pyrazolyl, thiazolyl, triazolyl, oxazolyl, 5-7 membered saturated or partially unsaturated heterocyclyl.

In some embodiments, $R_5$ is -L-Rw, wherein L is selected from the group consisting of: covalent bonds, $-C_{1-4}$ alkyl, $-NR_6-$;

$R_6$ is selected from the group consisting of: H, $C_{1-6}$ alkyl;
Rw is selected from the group consisting of: cyano,

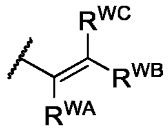 , 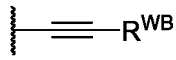 ,

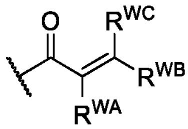 , 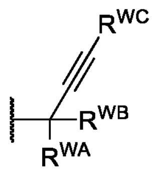 , 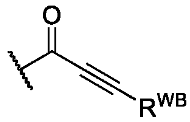 , 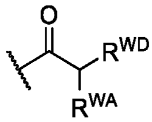 , 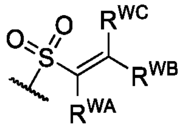 , 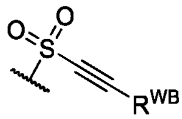 ,

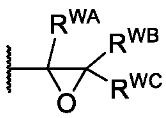 , 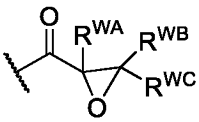 , 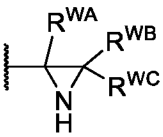 , 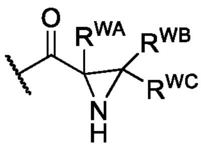 , 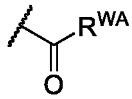 , 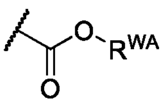 ,

and 5-7 membered nitrogen-containing heterocyclyl;
$R^{WA}$, $R^{WB}$, and $R^{WC}$ are each independently selected from the group consisting of: H, D, halogen, $C_{1-6}$ alkyl and $C_{1-6}$ haloalkyl; and $R^{WA}$, $R^{WB}$, and $R^{WC}$ can each be independently substituted with 1, 2, or 3 substituents selected from the group consisting of: $C_{1-6}$ alkylamino, and $C_{1-6}$ alkoxy;
or $R_6$ and Rw together form a ring; and the ring is selected from the group consisting of: 5-7 membered heteroaryl and 4-7 membered heterocyclyl.

In some embodiment,

$R_1$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl and $SF_5$;
$R_2$ and $R_3$ are each independently selected from the group consisting of: $C_{1-6}$ alkyl, $C_{1-6}$ alkoxy, $C_{1-6}$ haloalkyl, $C_{1-6}$ haloalkoxy and $C_{1-6}$ alkylamino; wherein, $R_2$ and $R_3$ can be the same or different;
or $R_2$ and $R_3$ together with the sulfur atom to which they are connected form a 3-7 membered heterocyclyl (preferably a 4-6 membered heterocyclyl);
$R_4$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy and $C_{1-6}$ alkylamino.

In another preferred embodiment, $R_1$ is selected from the group consisting of: H, D, halogen, $C_{1-6}$ alkyl, $C_{1-6}$ alkoxy, $C_{1-6}$ haloalkyl and $C_{1-6}$ haloalkoxy;

$R_2$ and $R_3$ are each independently selected from the group consisting of: $C_{1-6}$ alkyl, $C_{1-6}$ haloalkyl, $C_{1-6}$ alkylamino and $C_{1-6}$ haloalkylamino; wherein, $R_2$ and $R_3$ can be the same or different; or $R_2$ and $R_3$ together with the sulfur atom to which they are connected form a 4-8 membered heterocyclyl;
$R_4$ is selected from the group consisting of: H, D, halogen, $C_{1-6}$ alkyl, $C_{1-6}$ alkoxy, $C_{1-6}$ haloalkyl and $C_{1-6}$ haloalkoxy.

In some embodiments, the compound is selected from the group consisting of:

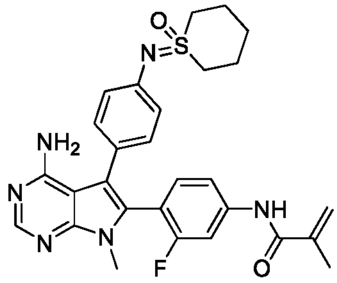

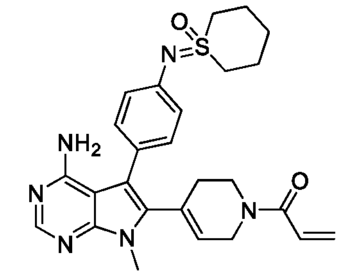

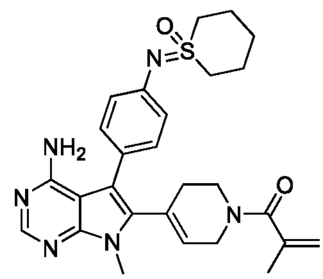

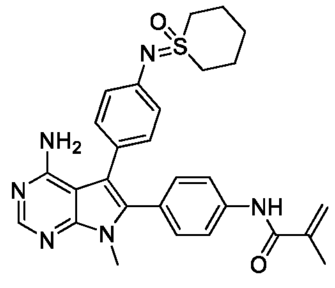

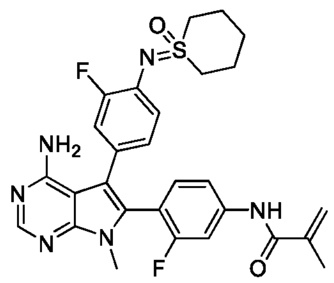

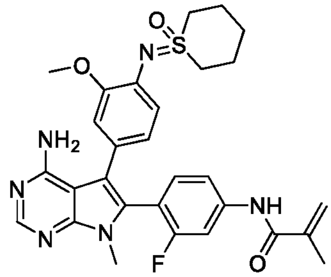

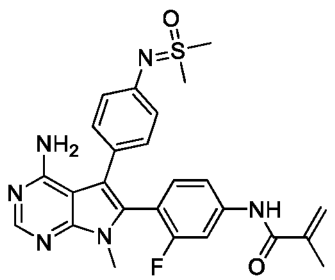

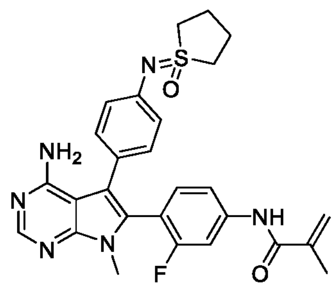

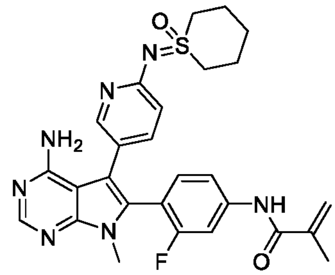

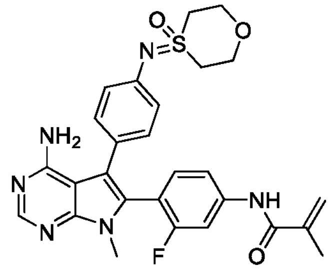

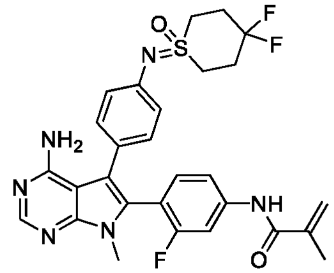

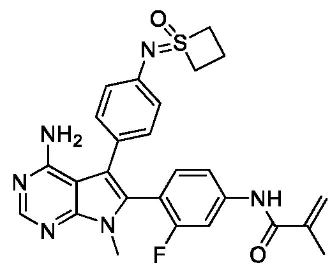

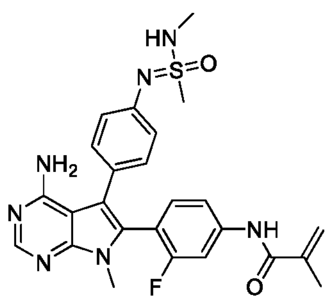

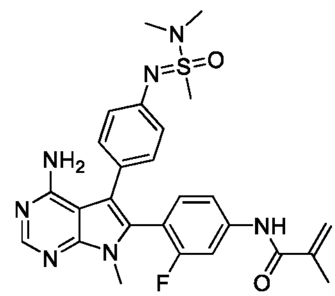

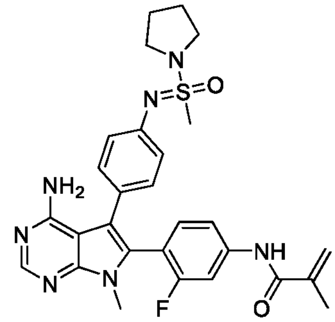

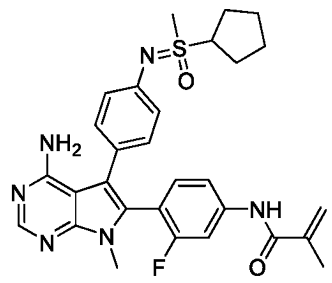
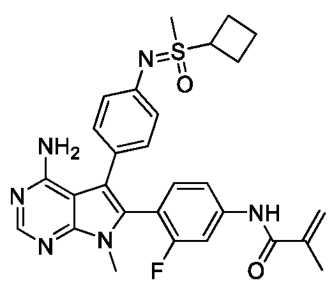
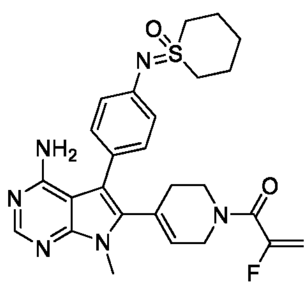

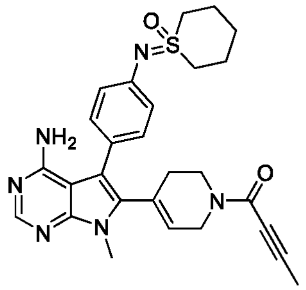
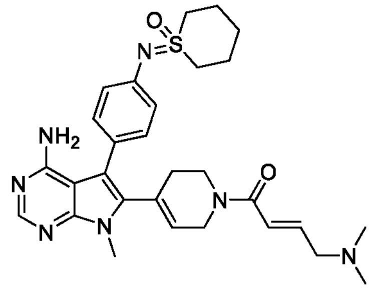
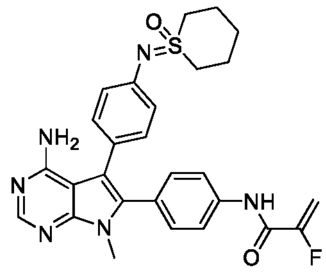

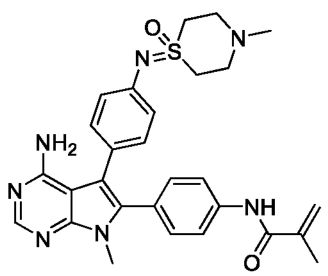
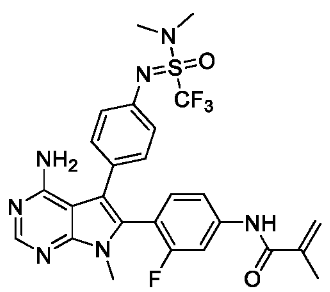
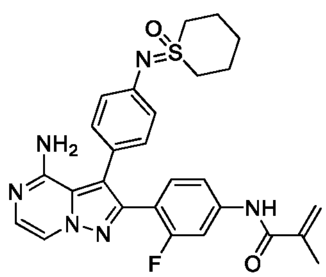

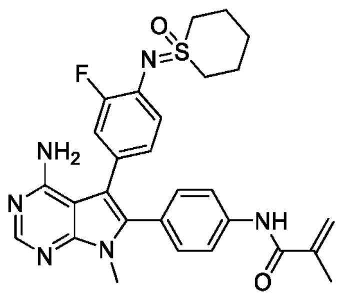
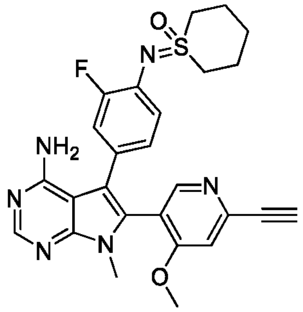
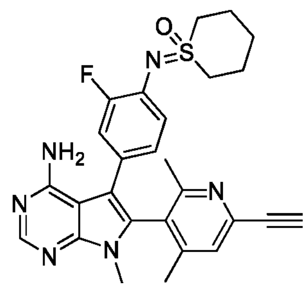

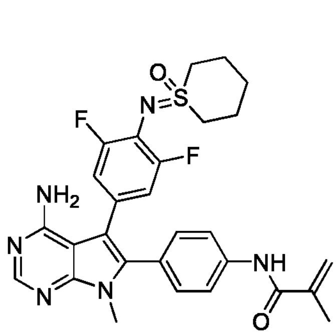
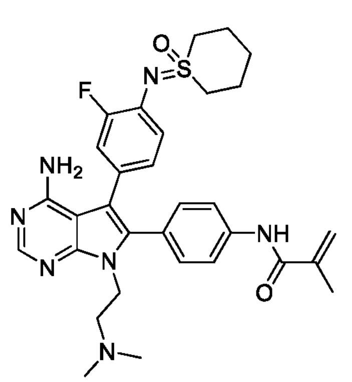
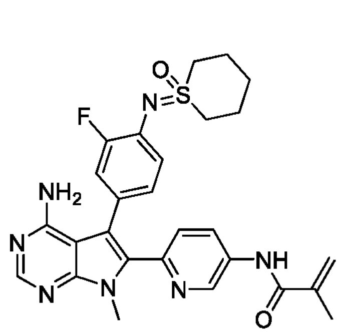

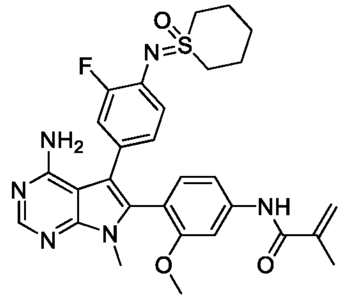
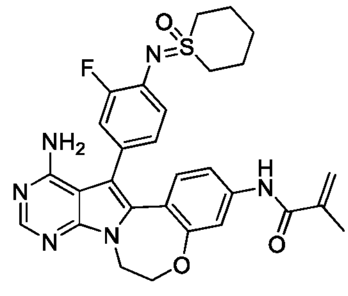
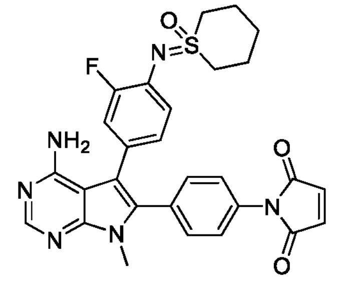

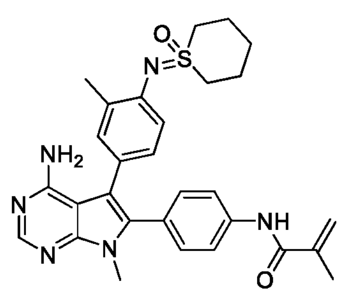
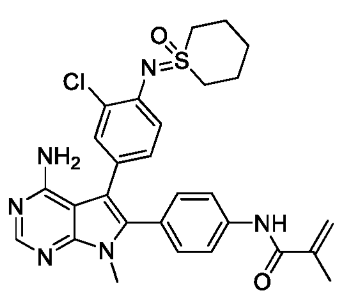
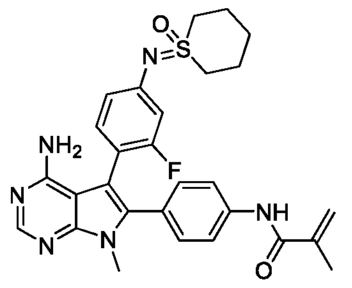

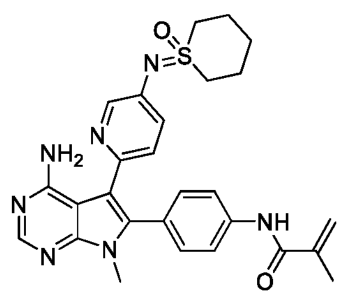
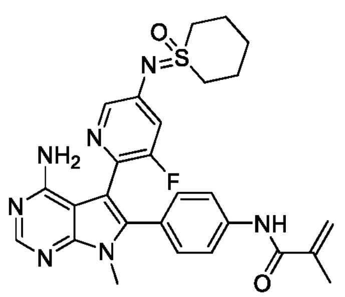
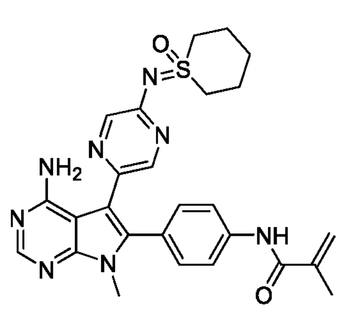

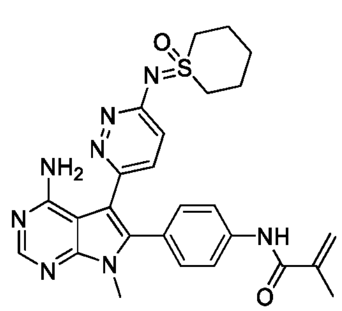
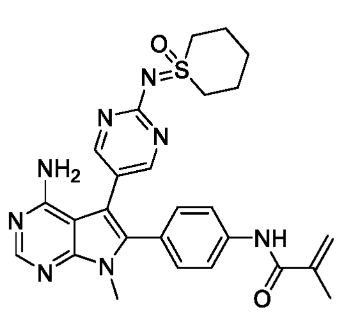
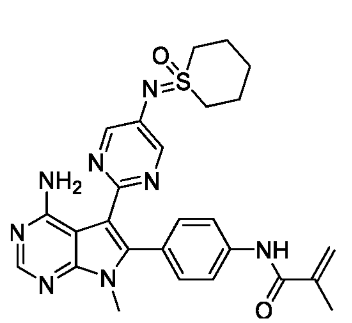

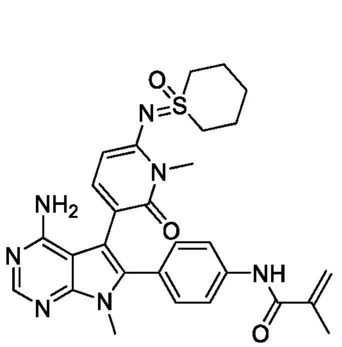
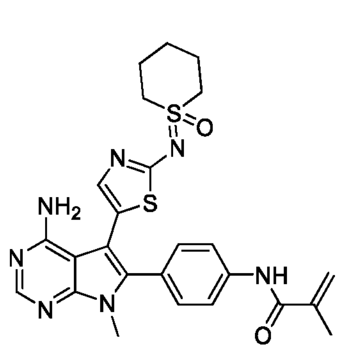
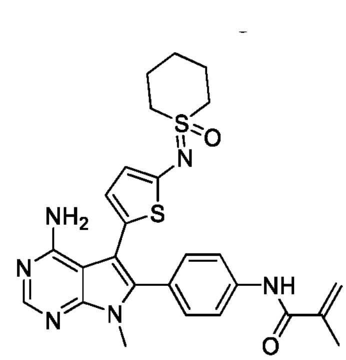

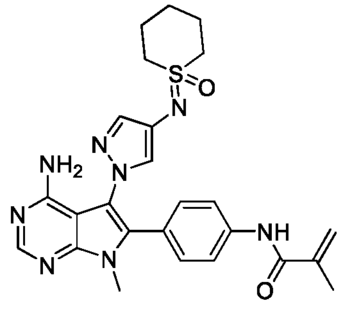

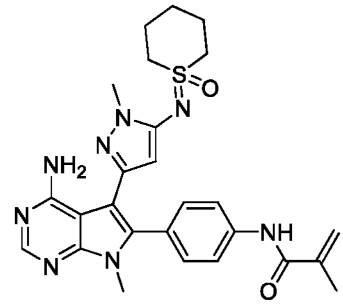

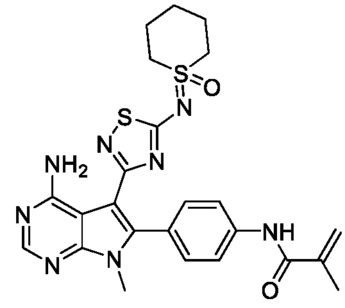

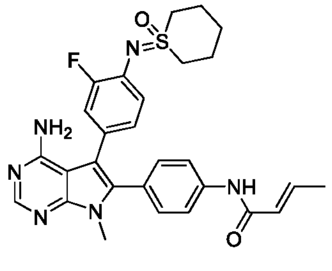

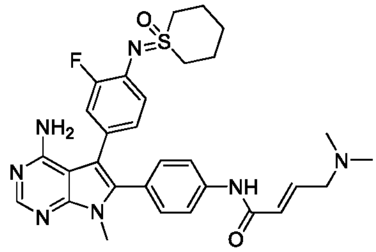

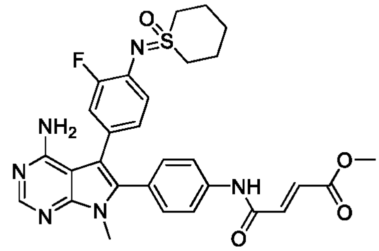

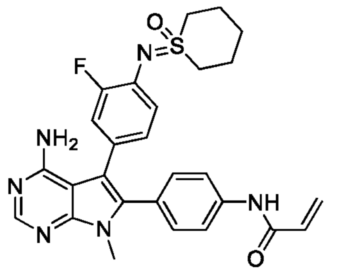

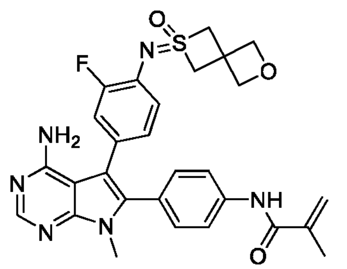

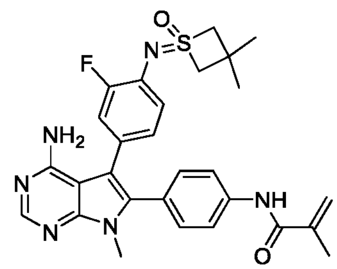

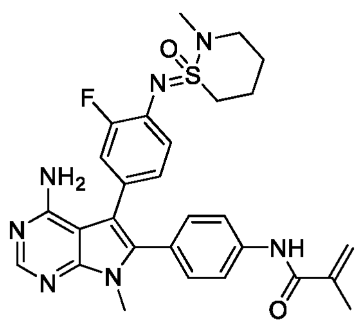

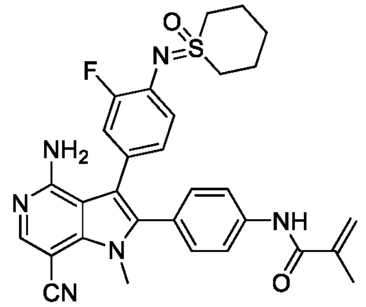

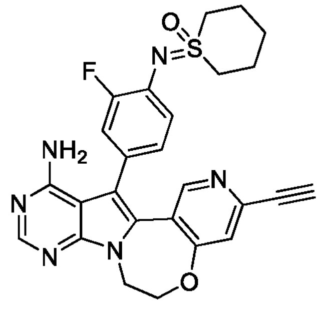

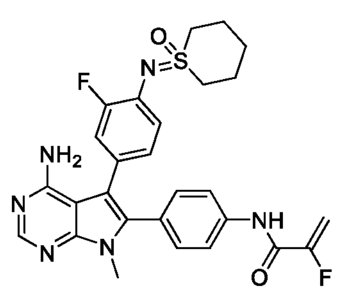

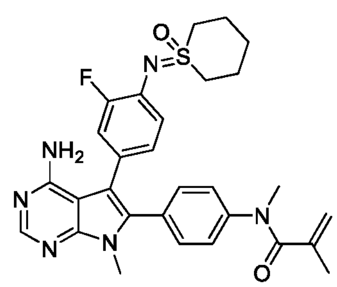

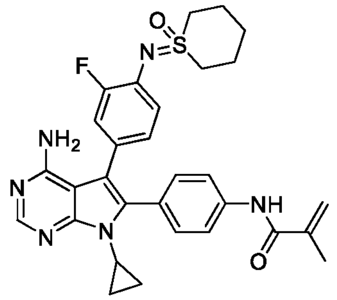

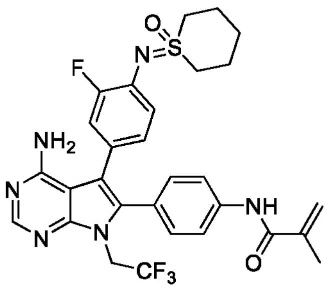
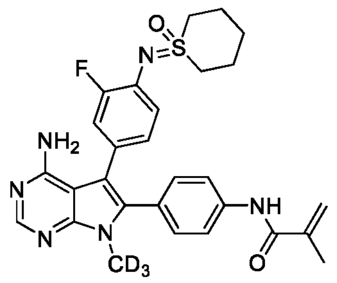
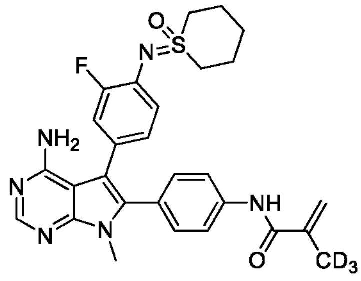

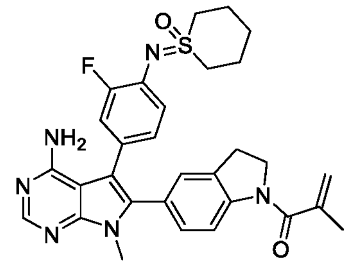
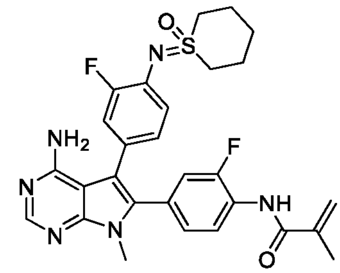
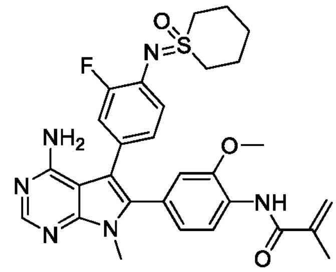

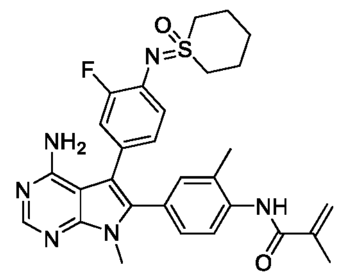
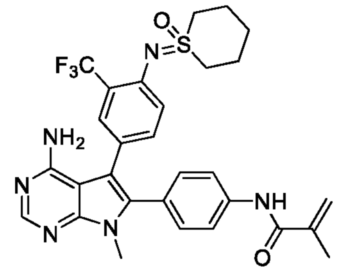
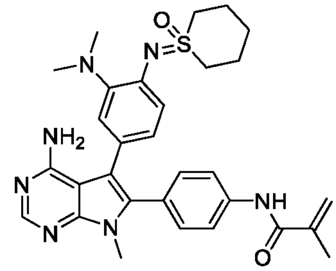

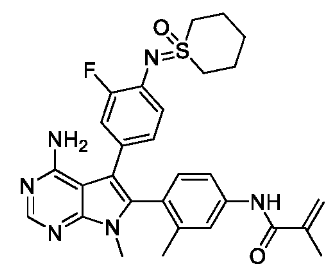
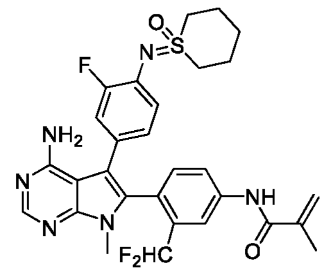
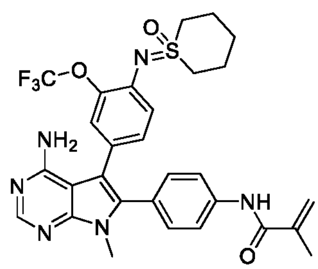

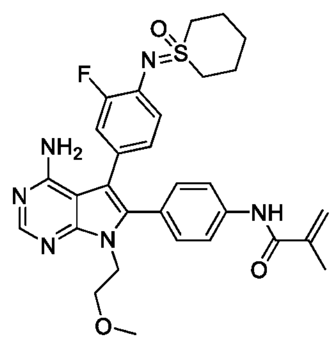
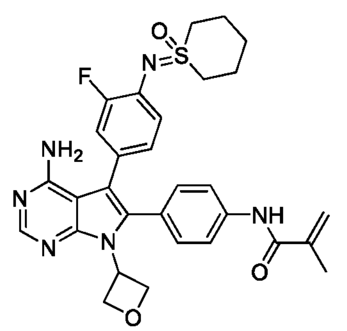
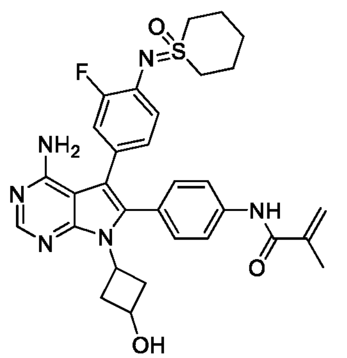

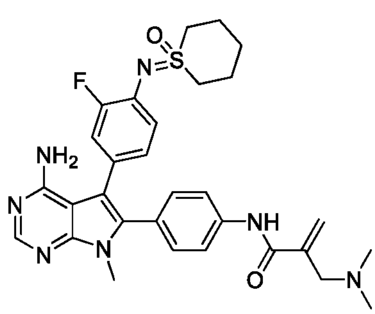
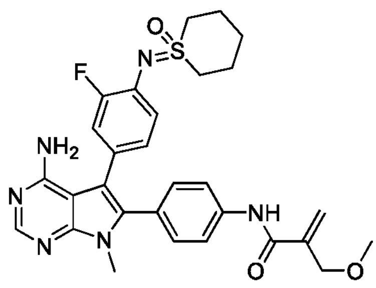
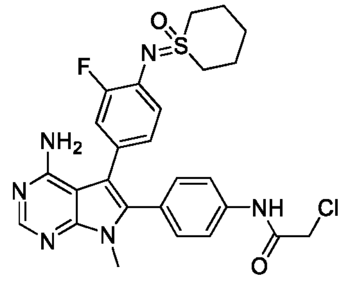

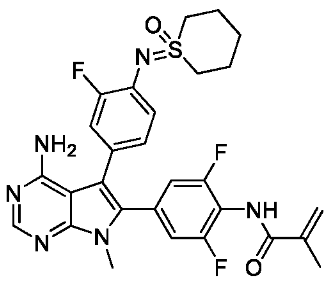
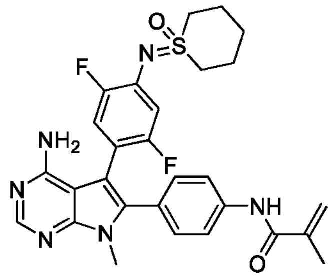
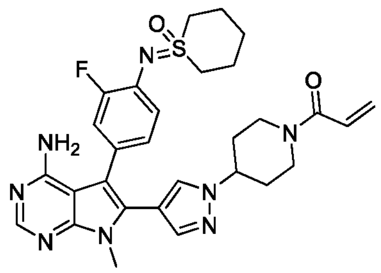

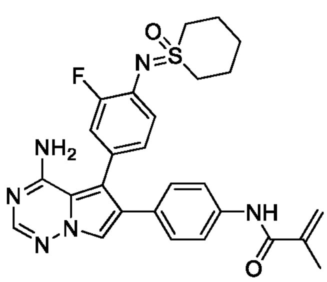
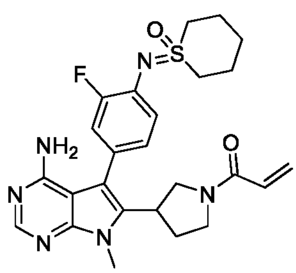
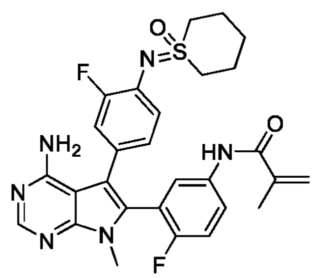

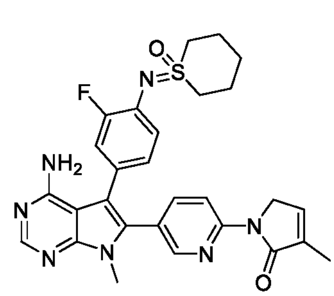
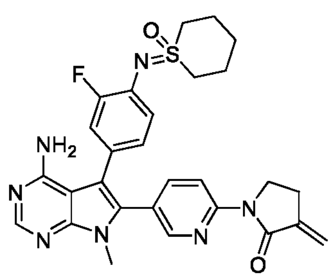
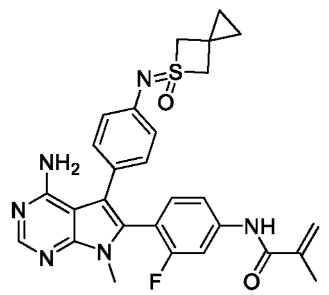

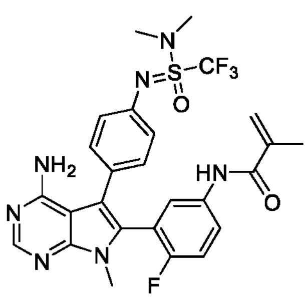
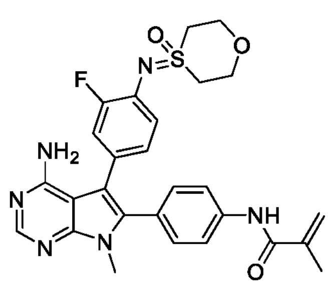
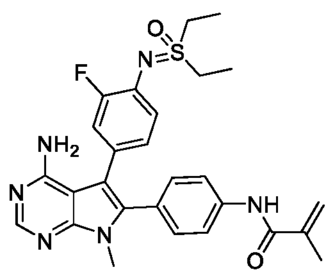

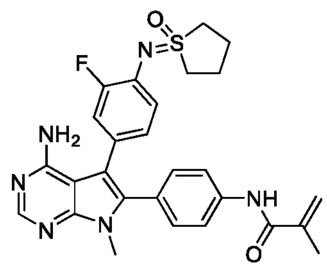

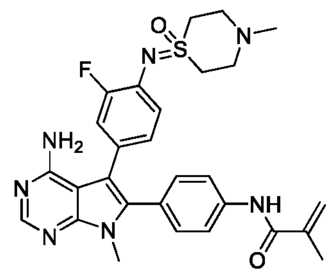

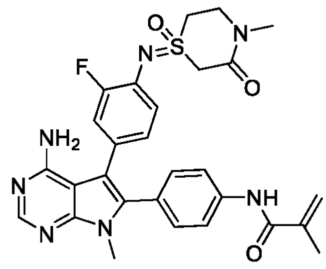

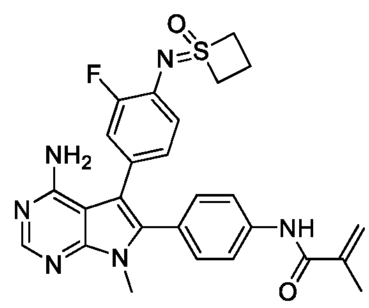

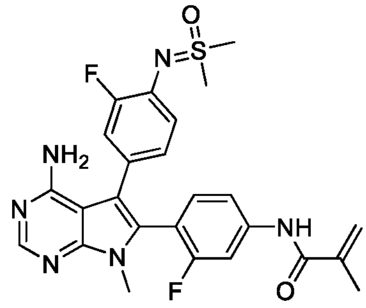

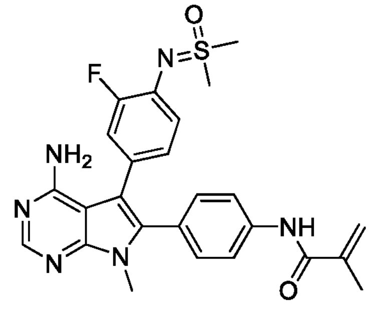

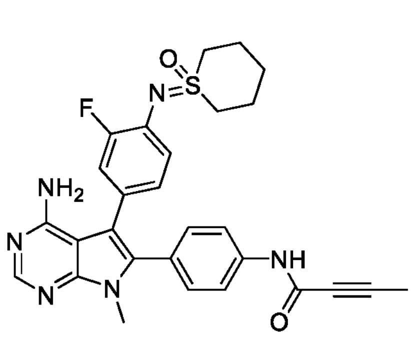

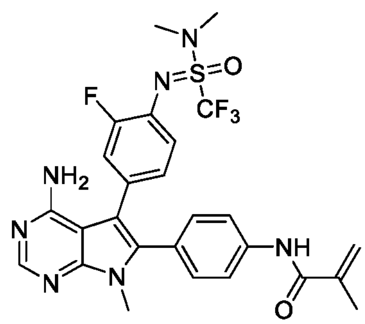

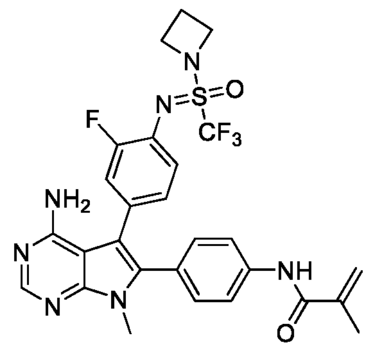

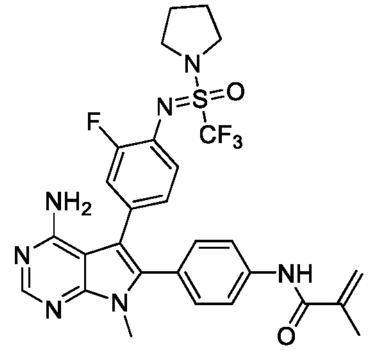

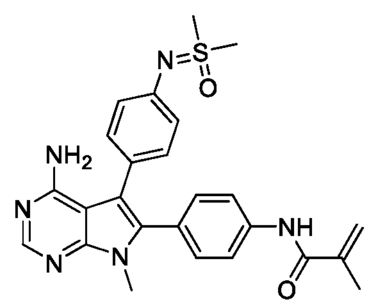

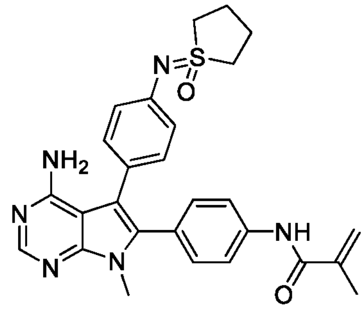

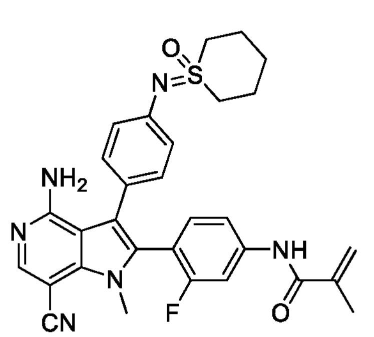

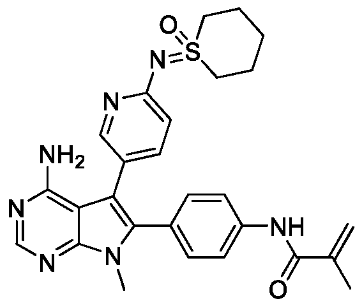

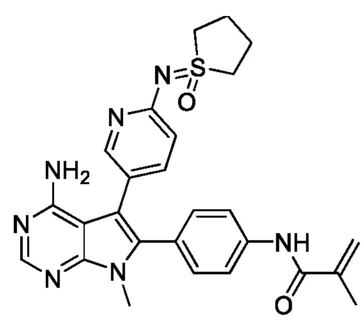

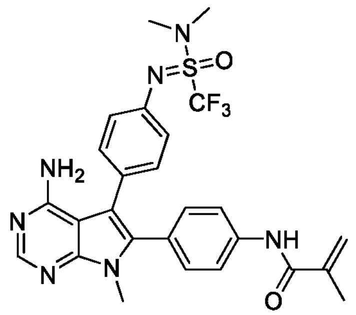
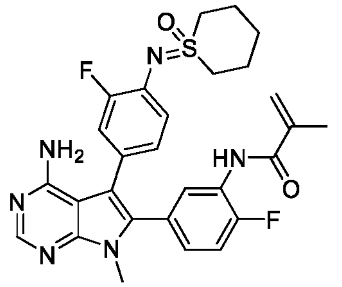
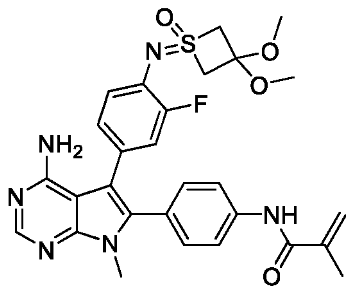

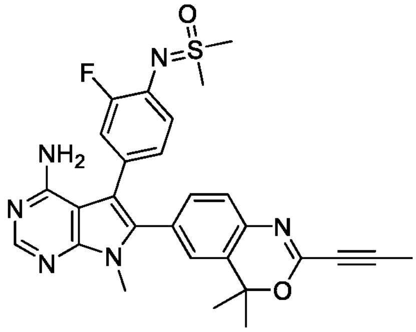
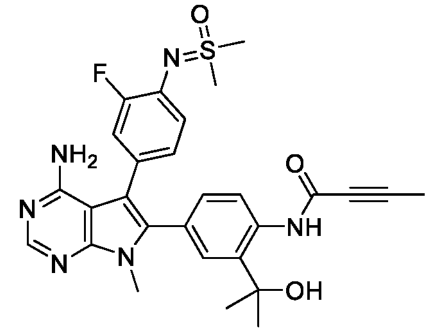
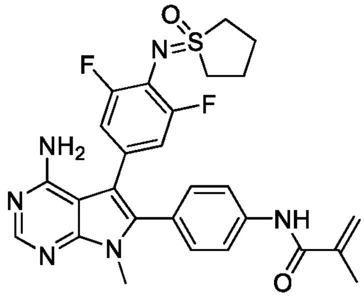

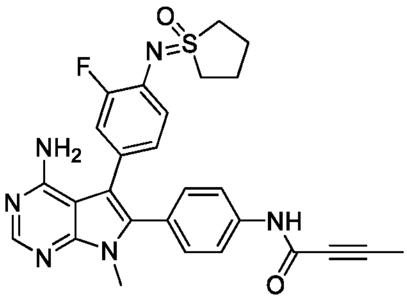
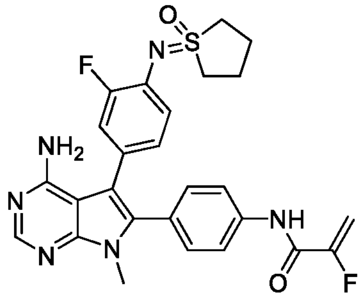
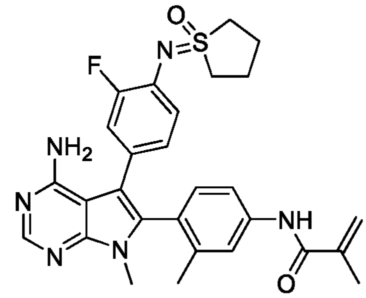

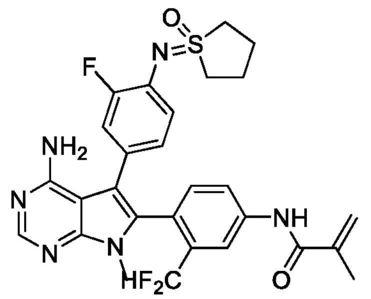
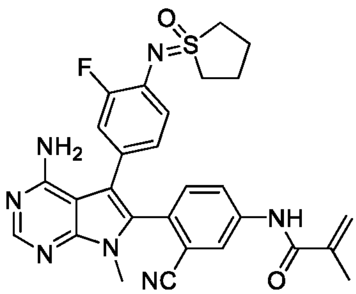
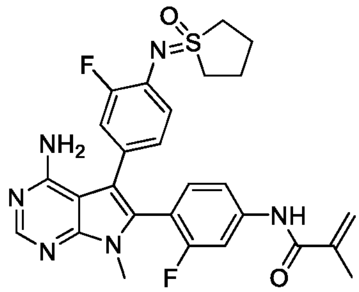

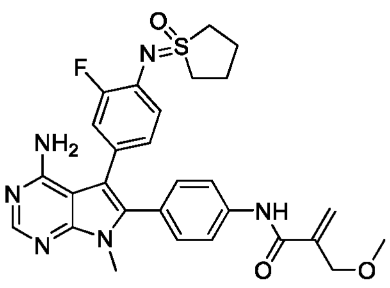
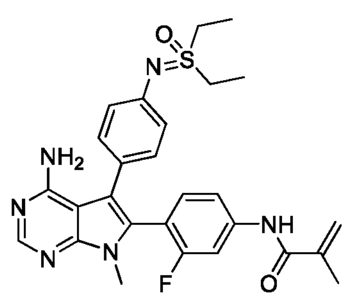
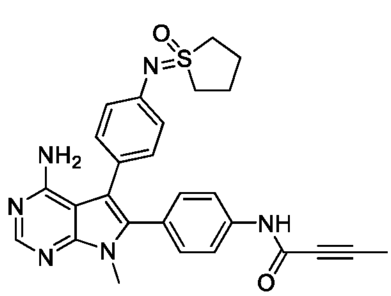

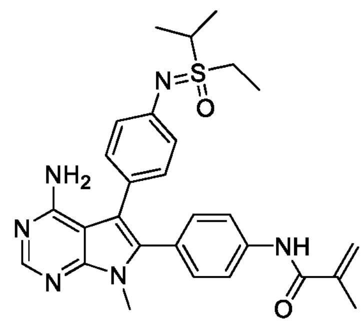

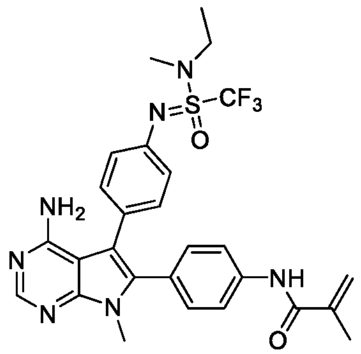

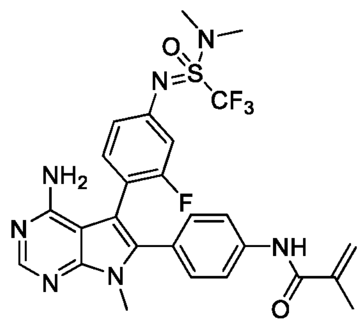

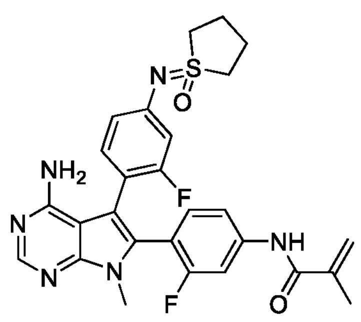

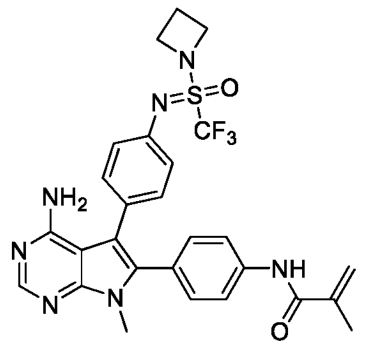

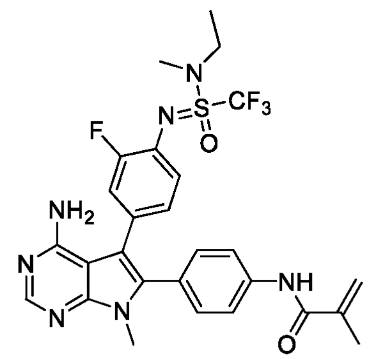

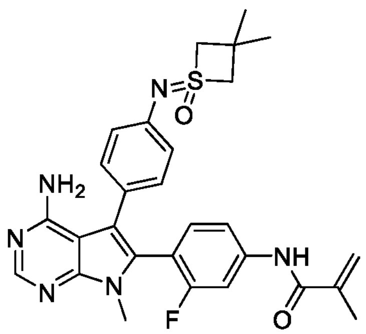

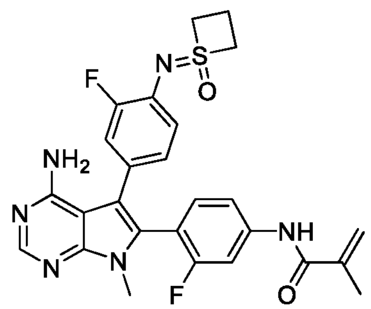

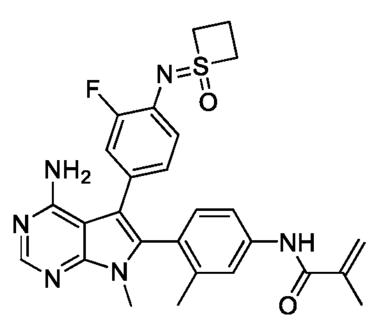

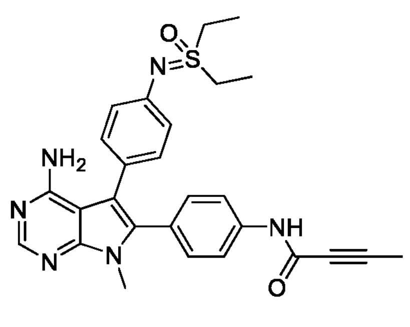

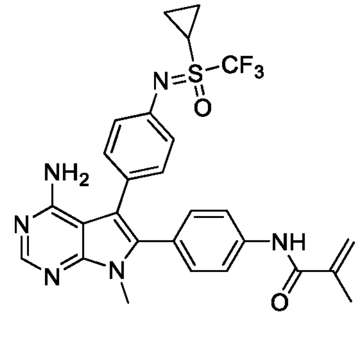

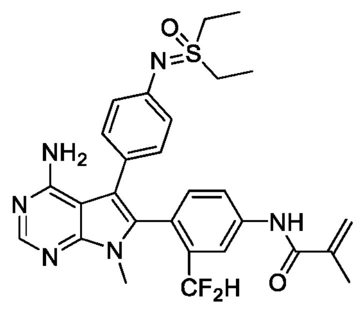

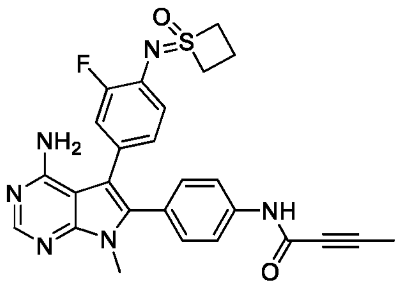

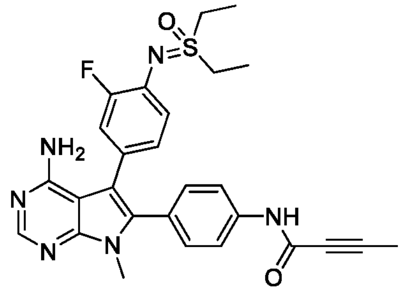

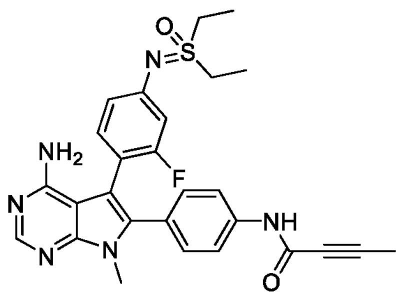

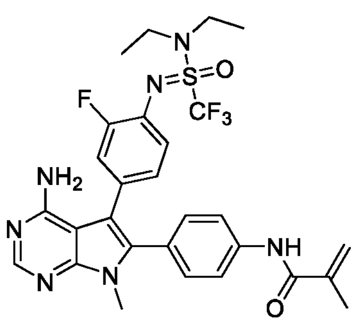

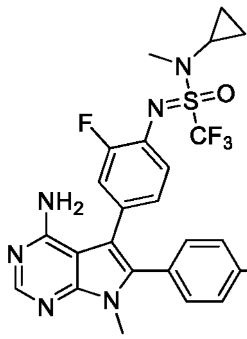

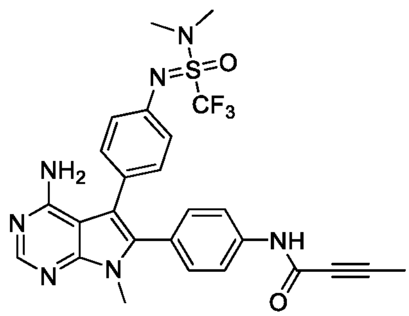

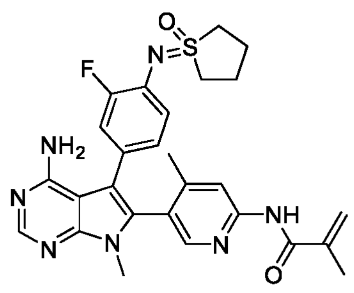

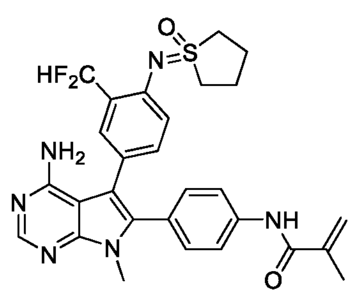

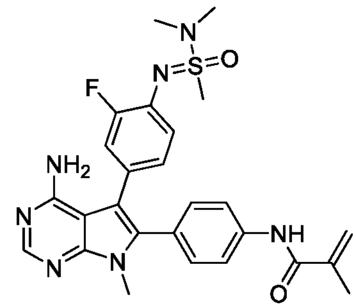

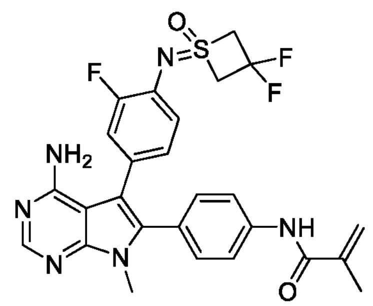

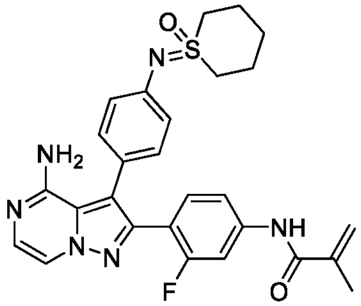

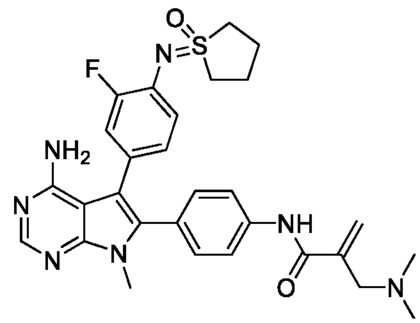

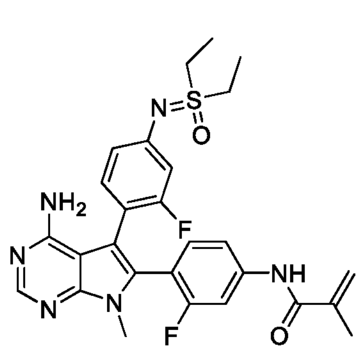

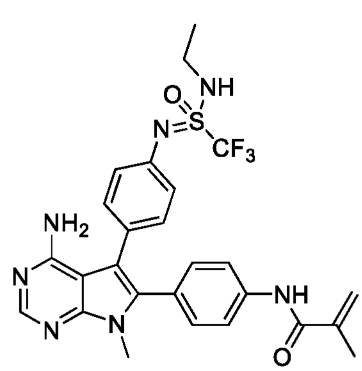

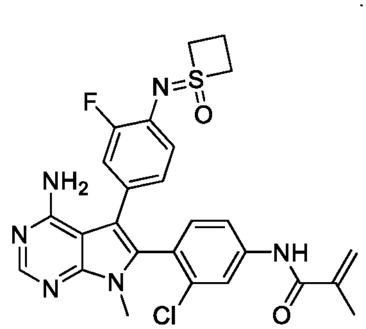

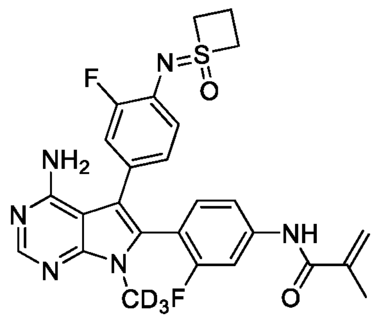
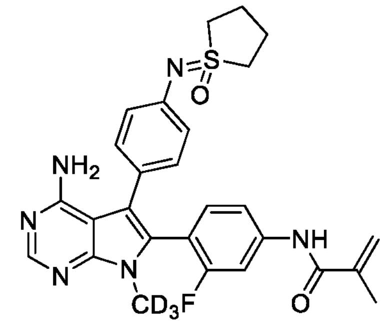
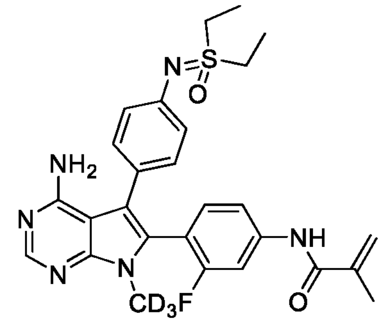

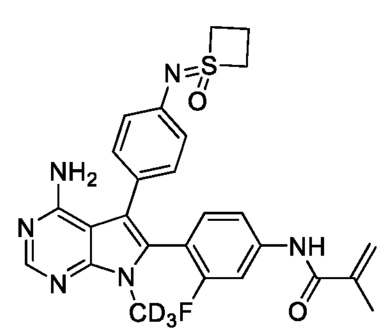
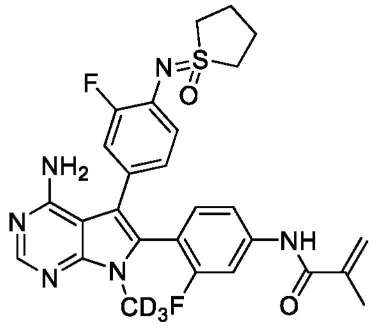
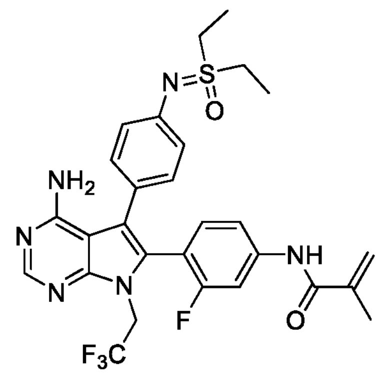

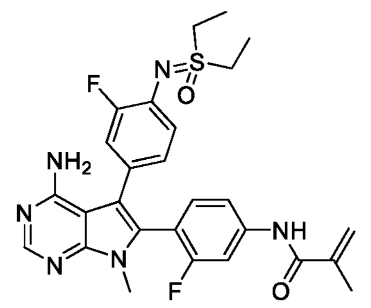
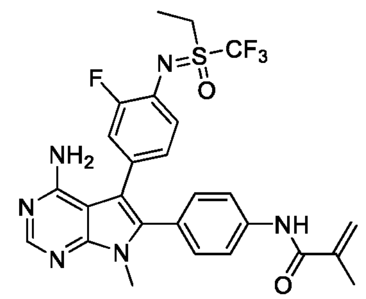
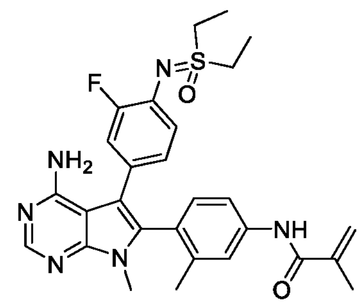

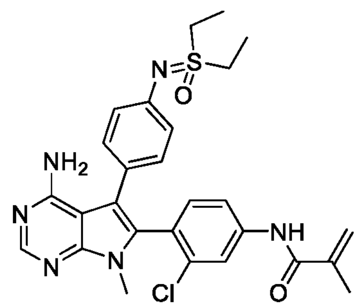
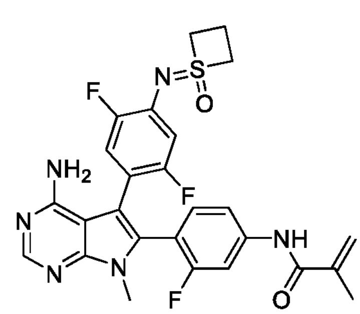
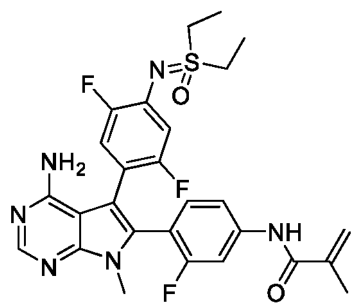

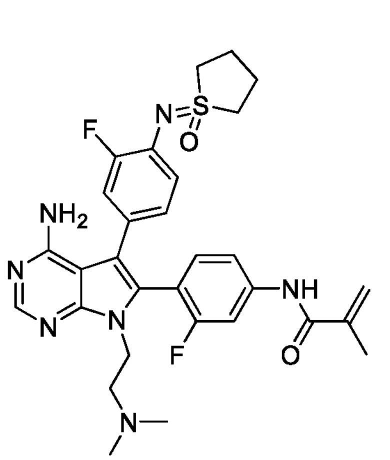

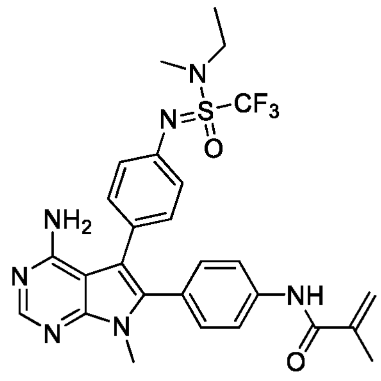

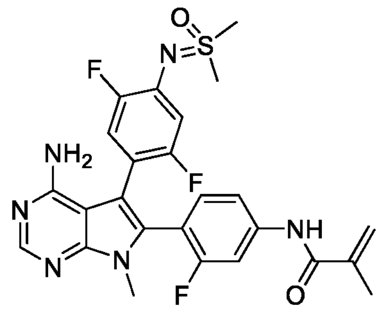

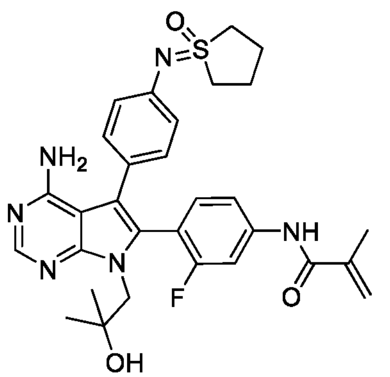

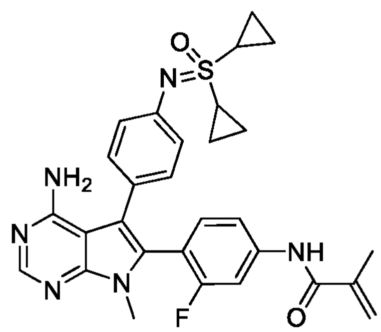

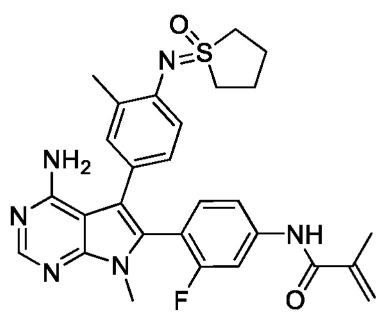

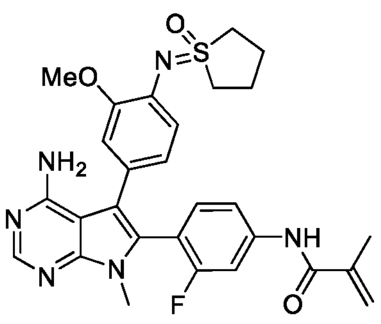

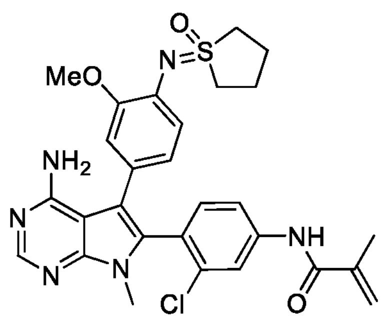

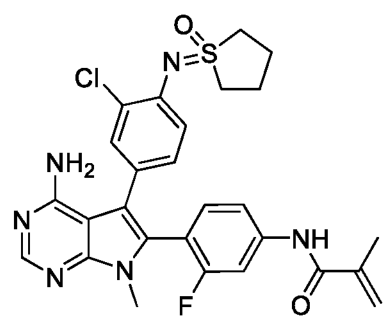

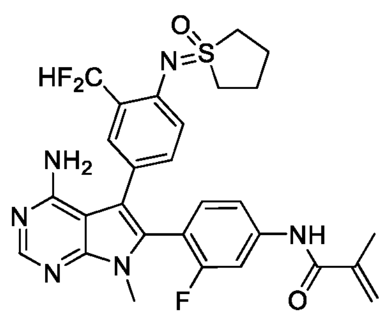

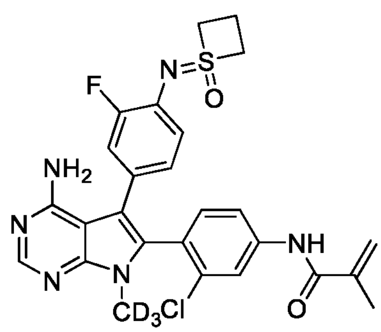

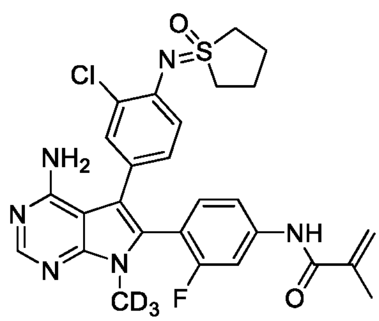

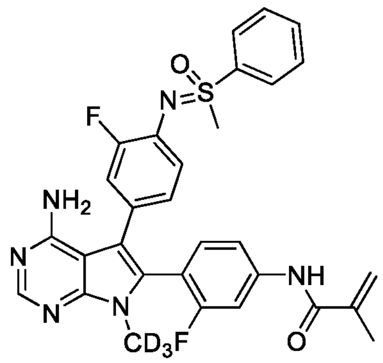
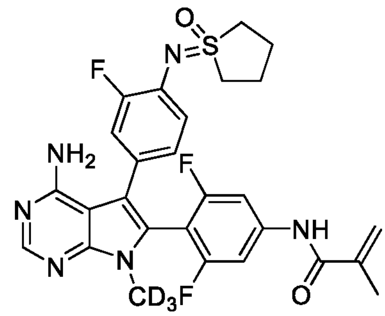
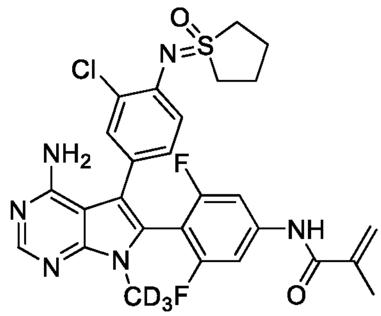
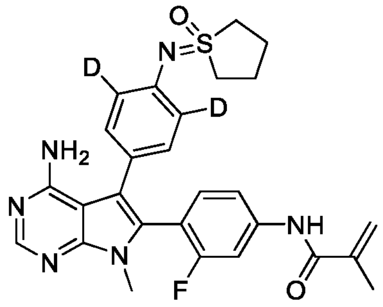
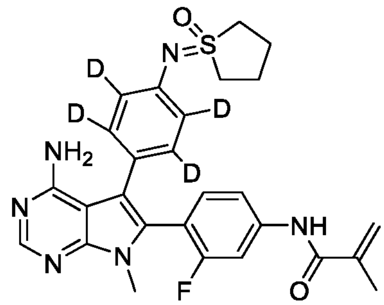
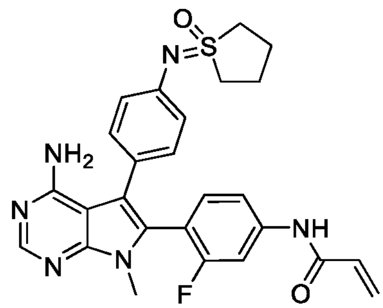
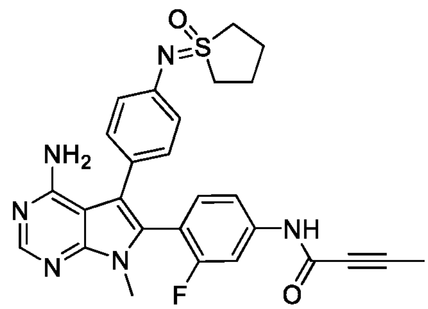
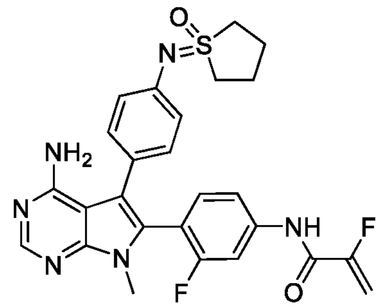
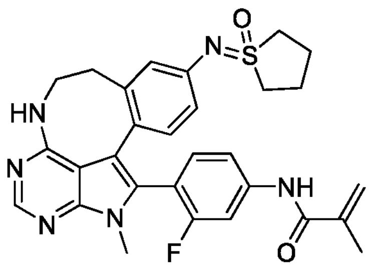

In the second aspect of the present invention, provided is a pharmaceutical composition comprising:

(1) one or more the compound as described in the first aspect of the present invention, a tautomer, a stereoisomer, a hydrate, a solvate, or a pharmaceutically acceptable salt thereof,
(2) one or more pharmaceutically acceptable carriers, excipients, adjuvants, excipients, and/or diluents.

In another preferred embodiment, the pharmaceutical composition is used for treating or preventing diseases or condition associated with abnormal activity or expression levels of FGFR; preferably, the disease or condition is selected from the group consisting of: cholangiocarcinoma, hepatic carcinoma, breast carcinoma, prostate carcinoma, lung carcinoma, thyroid carcinoma, gastric carcinoma, ovarian carcinoma, rectal carcinoma, endometrial carcinoma and uroepithelial carcinoma.

In another preferred embodiment, the cholangiocarcinoma is intrahepatic cholangiocarcinoma.

In another preferred embodiment, the hepatic carcinoma is hepatocellular carcinoma.

In another preferred embodiment, the lung carcinoma is squamous cell carcinoma or non-small cell lung carcinoma.

In the third aspect of the present invention, provided is a use of the compound as described in the first aspect of the present invention, a tautomer, a stereoisomer, a hydrate, a solvate, or a pharmaceutically acceptable salt thereof, or mixture thereof, or a pharmaceutical composition as described in the second aspect of the present invention in the preparation a pharmaceutical composition for treating or preventing diseases or conditions associated with abnormal activity or expression levels of FGFR.

In another preferred embodiment, the disease or condition is specifically associated with a subtype of FGFR; preferably, the FGFR subtype is selected from the group consisting of: FGFR2.

It should be understood that, within the scope of the present invention, each of the above-mentioned technical

features of the present invention and each of the technical features specifically described in the following (such as the examples) can be combined with each other to form new or preferred technical solutions. Due to space limitations, it will not be elaborated herein.

## EMBODIMENTS FOR CARRYING OUT THE INVENTION

After long-term and intensive research, the inventors provided a sulfoximine derivative with FGFR inhibitory activity, which has good FGFR inhibitory activity and can be used for preventing, treating, and/or alleviating indications associated with FGFR dysregulation. Based on the above discoveries, the inventors have completed the present invention.

## DEFINITION

As used herein, the term "alkyl" includes straight or branched alkyl. For example, a $C_1$ -$C_8$ alkyl refers to a straight or branched alkyl with 1 to 8 carbon atoms, such as methyl, ethyl, propyl, isopropyl, butyl, isobutyl, tert-butyl, etc.
As used herein, the term "alkenyl" includes straight or branched alkenyl. For example, $C_2$-$C_6$ alkenyl refers to a straight or branched alkenyl with 2-6 carbon atoms, such as vinyl, allyl, 1-propenyl, isopropenyl, 1-butenyl, 2-butenyl, or similar groups.
As used herein, the term "alkynyl" includes straight or branched alkynyl. For example, $C_2$-$C_6$ alkynyl refers to a straight or branched alkynyl with 2-6 carbon atoms, such as ethynyl, propynyl, butynyl, or similar groups.
As used herein, the term "carbocyclyl" refers to a cyclic saturated or partially unsaturated aliphatic hydrocarbyl with a specific number of carbon atoms. For example, $C_{3-10}$ carbocyclyl refers to a cyclic saturated or partially unsaturated aliphatic hydrocarbyl with 3-10 carbon atoms. It can be a monocyclic group, such as cyclopropyl, cyclobutyl, cyclopentyl, cyclohexyl, or similar groups. It can also be in the form of a bicyclic group, such, bridged ring or a spiro.
As used herein, the term "alkylamino" refers to an amine group substituted with alkyl. For example, "$C_1$-$C_8$ alkylamino" refers to an amino group substituted with a $C_1$-$C_8$ alkyl, which can be monosubstituted or disubstituted; for example, methylamino, ethylamino, propylamino, isopropylamino, butylamino, isobutyramimino, tert-butylamino, dimethylamino, diethylamino, dipropylamino, diisopropylamino, dibutylamino, diisobutylamino, di tert-butylamino, etc.
As used herein, the term "alkoxy" refers to a group with a structure of alkyl-O-. For example, "$C_1$-$C_8$ alkoxy" refers to a straight or branched alkoxy with 1-8 carbon atoms, including methoxy, ethoxy, propoxy, isopropoxy, butoxy, isobutoxy, tert-butoxy, etc.
As used herein, the term "haloalkyl" refers to an alkyl in which one or more hydrogen atoms are substituted with halogens, wherein, the definition of alkyl is described above.
As used herein, the term "haloalkoxy" refers to an alkoxy in which one or more hydrogen atoms are substituted with halogens, wherein, the definition of alkoxy is described above.
As used herein, the term "heterocyclyl" or "heterocyclic alkyl" refers to a saturated or partially saturated cyclic group with a specific number of ring atoms (such as 3-10 ring atoms), wherein 1-3 atoms are heteroatoms selected from N, S, and O. It can be in the form of a monocyclic ring, a bicyclic ring, or a multiple ring, such, bridged ring or a spirocycle. Specific examples can include oxetanyl, azetidinyl, tetrahydro-2H-pyranyl, piperidinyl, tetrahydrofuranyl, morpholinyl, and pyrrolidinyl.
As used herein, the term "aryl" refers to an aromatic ring group with a specific number of carbon atoms, such as $C_6$-$C_{10}$ aryl refers to an aromatic ring group with 6-10 carbon atoms, such as phenyl, naphthyl, and the like.
As used herein, the term "heteroaryl" refers to a cyclic aromatic group with a specific number of atoms, wherein 1-3 atoms are heteroatoms selected from N, S, and O. For example, a 5-12 membered heteroaryl refers to an aromatic ring group with 5-12 carbon atoms. It can be in the form of a monocyclic ring or a fused ring. Specific examples can include pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furanyl, thiophenyl, imidazolyl, thiazolyl, oxazolyl, etc.
Unless otherwise specified as "substituted or unsubstituted", the groups of the present invention may be substituted with substituents selected from the group consisting of: halogen, cyano, nitro, hydroxyl, amino, $C_1$-$C_6$ alkyl-amino, $C_1$-$C_6$ alkyl, $C_2$-$C_6$ alkenyl, $C_2$-$C_6$ alkynyl, $C_1$-$C_6$ alkoxy, halogenated $C_1$-$C_6$ alkyl, halogenated $C_2$-$C_6$ alkenyl, halogenated $C_2$-$C_6$ alkynyl, halogenated $C_1$-$C_6$ alkoxy, allyl, benzyl, $C_6$-$C_{12}$ aryl, $C_1$-$C_6$ alkoxy-$C_1$-$C_6$ alkyl, $C_1$-$C_6$-alkoxy carbonyl, phenoxycarbonyl, $C_2$-$C_6$ alkynyl-carbonyl, $C_2$-$C_6$ alkynyl carbonyl, $C_3$-$C_6$ carbocyclyl-carbonyl, $C_1$-$C_6$ alkyl-sulfonyl, etc.
As used herein, "halogen" or "halogen atom" refers to F Cl, Br, and I. More preferably, the halogen or halogen atom is selected from F, Cl, and Br. "Halogenated" refers to being substituted with atoms selected from F Cl, Br, and I.
Unless otherwise specified, the structural formulas described in the present invention are intended to include all isomeric forms (such as enantiomers, diastereomers, and geometric isomers (or conformational isomers)), such as R and S configurations with asymmetric centers, (Z) and (E) isomers with double bonds, etc. Therefore, the individual

stereoisomers or enantiomers, diastereomers, or geometric isomers (or conformational isomers) of the compounds of the present invention, or the combination thereof are within the scope of the present invention.

As used herein, the term "tautomer" refers to structural isomers with different energies that can be transformed into each other by exceed the low energy barrier. For example, proton tautomers (i.e. proton shift) include interconversion through proton transfer, such as 1H-indazole and 2H-indazole. Valence tautomers include interconversion through recombination of some bonding electrons.

As used herein, the term "solvate" refers to a complex formed by the coordination of a compound of the present invention with a solvent molecule in a specific ratio.

As used herein, the term "hydrate" refers to a complex formed by the coordination of a compound of the present invention with water.

## ACTIVE INGREDIENT

In the present invention, an active ingredient that can effectively inhibit FGFR is provided. The active ingredient is a compound represented by formula (I), which can effectively prevent, treat, and/or alleviate FGFR related diseases.

Experiments have shown that the active ingredients of the present invention can effectively inhibit FGFR kinase proteins, thereby preventing, treating, and/or alleviating FGFR related diseases.

It should be understood that the active ingredients of the present invention include compounds represented by formula (I), or pharmaceutically acceptable salts, or prodrugs thereof. It should be understood that the active ingredients of the present invention also include crystal forms, amorphous compounds, and deuterated compounds of compounds of formula (I).

The term "pharmaceutically acceptable salt" refers to a salt formed by the compound of the present invention with an acid or base that is suitable for pharmaceutical use. Pharmaceutically acceptable salts include inorganic salts and organic salts. A preferred type of salt is the salt formed by the compound of the present invention with an acid. Suitable acids for salt formation include but are not limited to inorganic acids such as hydrochloric acid, hydrobromic acid, hydrofluoric acid, sulfuric acid, nitric acid, phosphoric acid, etc; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalene sulfonic acid, etc; and amino acids such as proline, phenylalanine, aspartic acid, glutamic acid, etc. Another preferred type of salt is the salt formed by the compound of the present invention with a base, such, alkali metal salts (e.g. sodium or potassium salts), alkaline earth metal salts (e.g. magnesium or calcium salts), ammonium salts (e.g. lower alkyl alcohol ammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethyl amine salt, dihydroxyethylamine salt, trihydroxyethylamine salt and amine salts formed by morpholine, piperazine, and lysine, respectively.

## PHARMACEUTICAL COMPOSITION AND MODE OF ADMINISTRATIO*N*

Due to the excellent FGFR kinase inhibitory activity of the compounds of the present invention, the compounds of the present invention and various crystal forms, pharmaceutically acceptable inorganic or organic salts, hydrates or solvates thereof, as well as pharmaceutical compositions containing the compounds of the present invention as the main active ingredient, can be used for preventing, treating and/or alleviating FGFR related diseases, such as treating cancer.

The pharmaceutical composition of the present invention comprises a safe and effective amount of the compound of the present invention and a pharmaceutically acceptable excipient or carrier. The term "safe and effective amount" refers to the amount of a compound that is sufficient to significantly improve the condition without causing serious side effects. Generally, pharmaceutical compositions contain 1-2000mg of the compound of the present invention per dose, and more preferably 10-200mg of the compound of the present invention per dose. Preferably, the "one dose" is a capsule or tablet.

"Pharmaceutically acceptable carrier" refers to one or more compatible solid or liquid fillers or gel substances, which are suitable for human use and must have sufficient purity and low toxicity. "Compatible" here refers to each component in the composition can be blended with the compound of the present invention and with each other without significantly reducing the efficacy of the compound. Examples of pharmaceutically acceptable carriers include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agents (such as sodium dodecyl sulfate), coloring agents, flavoring agents, stabilizers, antioxidants, preservatives, pyrogen-free water , etc.

There is no special limitation of mode of administration of the compound or pharmaceutical combination of the

present invention, and representative mode of administration include (but are not limited to): oral, parenteral (intravenous, intramuscular, or subcutaneous).

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or compatibilizers, such as starch, lactose, sucrose, glucose, mannitol, and silica; (b) binders, such as hydroxymethylcellulose, alginate, gelatin, polyvinylpyrrolidone, sucrose, and arabic gum; (c) humectants, such as glycerin; (d) disintegrants, such,gar, calcium carbonate, potato starch or cassava starch, alginic acid, certain composite silicates, and sodium carbonate; (e) dissolution-retarding agents, such as paraffin; (f) absorption accelerators, such as quaternary ammonium salt; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets, and pills, the dosage form may also contain buffering agents.

Solid dosage forms such as tablets, sugar pills, capsules, pills, and granules can be prepared by coating and shell materials, such as enteric coatings and other materials known in the art. They may contain opaque agents, and the active compounds or compounds in this combination can be released in a delayed mode in a certain part of the digestive tract. Examples of usable embedding components are polymeric substances and wax based substances. When necessary, the active compound and one or more of the above-mentioned excipients can form microcapsule.

Liquid dosage forms for oral administration include pharmaceutically acceptable emulsion, solutions, suspensions, syrups or tinctures. In addition to active compounds, liquid formulations may include conventional inert diluents used in this art, such as water or other solvents, solubilizers, and emulsifiers, such as ethanol, isopropanol, ethyl carbonate, ethyl acetate, propylene glycol, 1,3-butanediol, dimethylformamide, as well as oils, particularly cottonseed oil, peanut oil, corn germ oil, olive oil, castor oil, and sesame oil, or the combination thereof.

In addition to these inert diluents, the composition may also contain adjuvants such as humectants, emulsifiers and suspensions, sweeteners, corrigens, and spices.

In addition to the active compounds, the suspensions may contain suspensions, such as ethoxylated isooctadecanol, polyoxyethylene sorbitol and dehydrated sorbitol esters, microcrystalline cellulose, methanol aluminum and agar, or the combination thereof.

The compositions for parenteral injection may include physiologically acceptable sterile aqueous or anhydrous solutions, dispersions, suspensions or emulsions, and sterile powders for being re-dissolved into sterile injectable solutions or dispersions. Suitable aqueous and non-aqueous carriers, diluents, solvents, or excipients include water, ethanol, polyols, and suitable combination thereof.

The compound of the present invention can be administered alone or in combination with other pharmaceutically acceptable therapeutic agents.

When administered in combination, the pharmaceutical composition further comprises one or more (2, 3, 4, or more) other pharmaceutically acceptable therapeutic agents. One or more of the other pharmaceutically acceptable therapeutic agents (2, 3, 4, or more) can be used simultaneously, separately, or sequentially with the compound of the present invention for preventing, treating, and/or alleviating FGFR mediated diseases.

When using a pharmaceutical composition, a safe and effective dosage of the compound of the present invention is applied to a mammal (such, human) in need of treatment, wherein the dosage of administration is a pharmaceutically effective dosage, for a person weighed 60 kg, the daily dosage is usually 1-2000 mg, preferably 20-500 mg. Of course, the specific dosage should also consider factors such as the route of administration and the patient's health condition, which are within the skill range of skilled physicians.

## BENEFICIAL EFFECTS

According to embodiments of the present invention, the present invention provides novel structurally, metabolically stable, and highly effective FGFR inhibitors, which can be used for effectively treating FGFR related diseases and conditions.

The compound of the present invention has a good inhibitory effect on FGFR and good in vitro efficacy. In addition, the results of mouse experiments showed that the compounds of the present invention exhibited excellent pharmacokinetic properties and good druggability.

The following will explain the solution of the present invention in conjunction with the examples. Technicians in this art will understand that the following examples are only used to illustrate the present invention and should not be considered as limiting the scope of the present invention. The specific technology or conditions with no description in the examples are performed under the technology or conditions described in the literature in this art or according to manufacturer's instructions. The reagents or instruments used without specifying the manufacturer are conventional products that can be obtained through commercial purchase.

Unless otherwise specified, the structures of the compounds of the present invention are determined by nuclear

magnetic resonance (NMR) and/or mass spectrometry (MS). The unit of NMR shift is $10^{-6}$ ppm. The solvents used for NMR determination are deuterated dimethyl sulfoxide, deuterated chloroform, deuterated methanol, etc. The internal standard is tetramethylsilane (TMS).

The abbreviation definition of the present invention is as follows:

M: molar concentration, such as 1M hydrochloric acid refers to 1 mol/L hydrochloric acid solution
HATU: O-(7-azabenzotriazole-1-yl)-N,N,N,N-tetramethylurea hexafluorophosphate
T3P: 1-propylphosphamide
DIPEA: also known as DIEA, diisopropylethylamine, i.e. N, N-diisopropylethylamine
NIS: N-iodosuccinimide
NBS: N-bromosuccinimide
EA: ethyl acetate
TFA: trifluoroacetic acid
DMF: N, N-dimethylformamide
THF: tetrahydrofuran
PE: petroleum ether
LC-MS: liquid chromatography-mass spectrometry
DMSO: dimethyl sulfoxide
Xantphos: 4,5-diphenylphosphine-9,9-dimethyloxaanthracene
TLC: thin layer chromatography
IC50: half inhibitory concentration, which refers to the concentration at which half of the maximum inhibitory effect is achieved.

Unless otherwise indicated, the compounds listed herein are named and numbered using ChemBioDraw Ultra 14.0.

**Intermediate 1g 5-iodo-7-methyl-7*H*-pyrrolo [2,3-*d*] pyrimidin-4-amine**

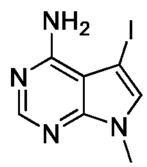

**1g**

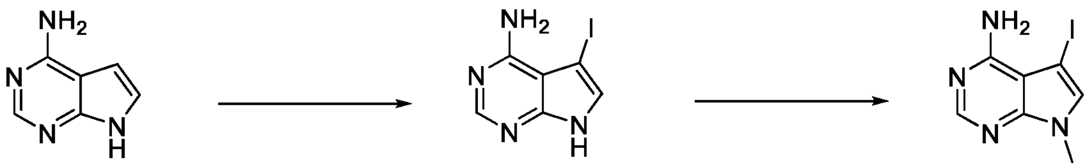

**1g-1** **1g-2** **1g**

Step 1 5-iodo-7*H*-pyrrolo [2,3-*d*] pyrimidin-4-amine **1g-2**

Compound **1g-1** (1.0 g, 7.46 mmol) was dissolved in dichloromethane (15 mL), sodium hydroxide (329 mg, 8.21 mmol) and NIS (1.75 g, 7.84 mmol) were added in sequence at room temperature and reacted for 18 h. The reaction solution was then concentrated added water (20 mL), and stirred for 30 min, filtered, dried to obtain compound **1g-2** (1.85g), gray solid.

MS (ESI) *m/z* 261 $[M + H]^+$.

$^1H$ NMR (400 MHz, DMSO-$d_6$) $\delta$ 11.97 (s, 1H), 8.06 (s, 1H), 7.37 (s, 1H), 6.55 (s, 2H).

Step 2: 5-iodo-7-methyl-7*H*-pyrrolo [2,3-*d*] pyrimidin-4-amine **1g**

The **1g-2** (1.0 g, 3.85 mmol) was dissolved in DMF (20 mL), cesium carbonate (2.5 g, 7.69 mmol) and iodomethane (665 mg, 4.62 mmol) were added. The reaction solution was heated up to 80 °C and reacted for 18 h.

After cooled down to room temperature, the reaction solution was filtered and concentrated to obtain a crude product, the crude product was purified by column chromatography (PE/EtOAc=0/1) to obtain compound **1g** (0.78 g), brown solid.

MS (ESI) *m/z* 275 [M + H]$^+$.

$^1$H NMR (400 MHz, $CDCl_3$) $\delta$ 8.32 (s, 1H), 7.05 (s, 1H), 5.73 (s, 2H), 3.81 (s, 3H).

**Intermediate 1j *N*-(3-fluoro-4-(4,4,5,5-tetramethyl-1,3,2-dioxaborolan-2-yl)phenyl) methacrylamide**

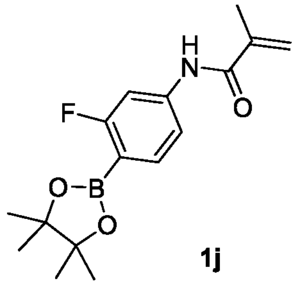

**1j**

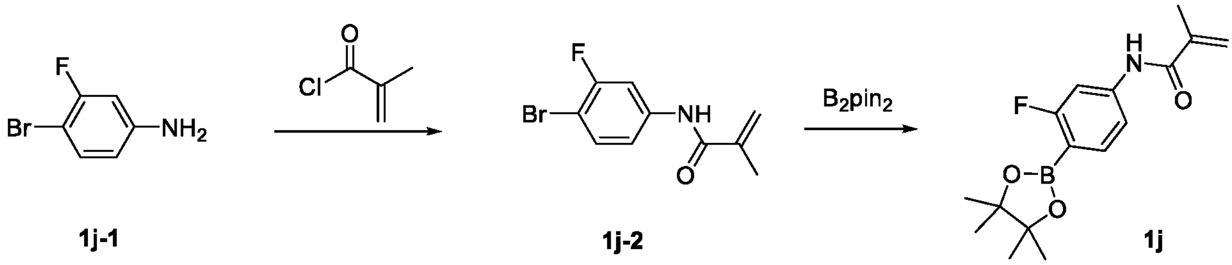


**1j-1** **1j-2** **1j**

Step 1: *N*-(4-bromo-3-fluorophenyl) methacrylamide **1j-2**

Compound **1j-1** (5.0 g, 26.3 mmol) and triethylamine (8.0 g, 11 ml) were dissolved in DCM (50 ml), at 0 °C, 2-methylacryloyl chloride (3.03 g, 2.8 ml) was added dropwise to the reaction solution, and stirred for 1 h. The reaction solution was diluted with water (100 ml), and extracted with DCM (100 ml) three times. The organic phase was washed with saturated saline solution, dried over anhydrous $Na_2SO_4$, and concentrated under reduced pressure, the obtained crude product was purified by silica gel column chromatography (EA/PE=1/4) to obtain compound **1j-2** (4.9 g, 72%), pale yellow white solid.

MS (ESI) *m/z* 258, 260 [M + H]$^+$.

Step 2: *N*-(3-fluoro-4-(4,4,5,5-tetramethyl-1,3,2-dioxoborolan-2-yl)phenyl) methacrylamide **1j**

To a sealed tube was added Compound **1j-2** (2.0 g, 7.75 mmol), bis(pinacolato)diboron (3.94 g, 15.5 mmol), Pd(dppf)$Cl_2$ (567 mg, 0.77 mmol), AcOK (2.28 g, 23.25 mmol), and 1,4-dioxane (20 ml), and replaced with argon gas three times. The mixture reacted at 90 °C for 5 h. The reaction was then quenched with water and extracted with DCM. The organic phase was washed with saturated saline solution, dried over anhydrous $Na_2SO_4$, and concentrated under reduced pressure. The obtained crude product was purified by silica gel column chromatography (EA/PE=1/8 to 1/4) to obtain compound **1j** (1.52 g, 64%), yellow solid.

MS (ESI) *m/z* 306 [M + H]$^+$.

$^1$H NMR (500 MHz, $CDCl_3$) $\delta$ 7.70-7.65 (m, 2H), 7.53 (dd, *J* = 11.5, 1.9 Hz, 1H), 7.20 (dd, *J* = 8.2, 1.9 Hz, 1H), 5.78 (s, 1H), 5.48 (d, *J* = 1.7 Hz, 1H), 2.04 (s, 3H), 1.34 (s, 12H).

**Intermediate 4a *N*-(4-(4,4,5,5-tetramethyl-1,3,2-dioxaborolan-2-yl)phenyl) methacrylamide**

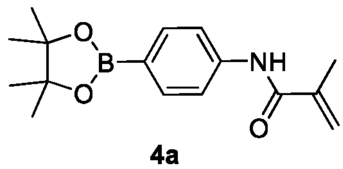

**4a**

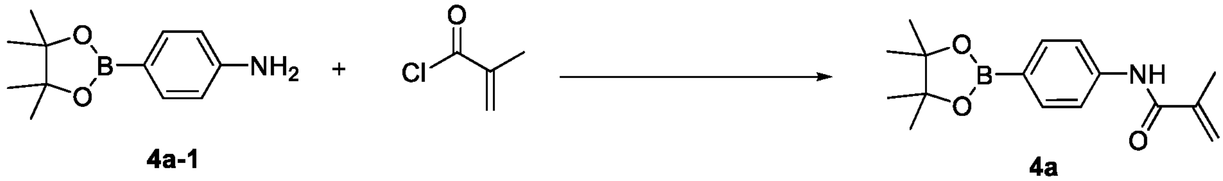

**4a-1** **4a**

Compound **4a-1** (2.0 g, 9.12 mmol) was dissolved in ultra dry DCM (50 mL), triethylamine (2.77g, 27.36 mmol) was added, and stirred at 0 °C for 5 min. The 2-methylacrylamide was diluted with ultra dry DCM (30 mL), slowly dropwise added to the reaction solution and reacted at 0 °C for 2 h. The reaction solution was poured into water, and extracted with DCM three times. The organic layers were combined and washed with saturated salt once, the organic phase was dried over anhydrous sodium sulfate, concentrated under reduced pressure, and purified by column chromatography (PE/EA=2/1) to obtain compound **4a** (2.15 g), white solid.
MS (ESI) *m/z* 288 [M + H]$^+$.
$^1$H NMR (400 MHz, DMSO-$d_6$) $\delta$ 9.87 (s, 1H), 7.71 (d, *J*=8.5 Hz, 2H), 7.61 (d, *J*=8.6 Hz, 2H), 5.81 (t, *J*=1.0 Hz, 1H), 5.55-5.51 (m, 1H), 1.95 (s, 3H), 1.28 (s, 12H).

**Intermediate 8e *N*-(4-(4-amino-5-bromo-7-methyl-7*H*-pyrrolo [2,3-*d*] pyrimidin- 6-yl) -3-fluorophenyl) methacrylamide**

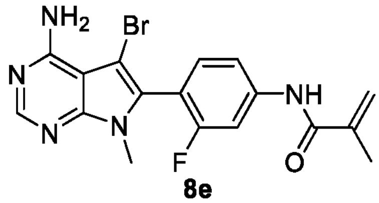

**8e**

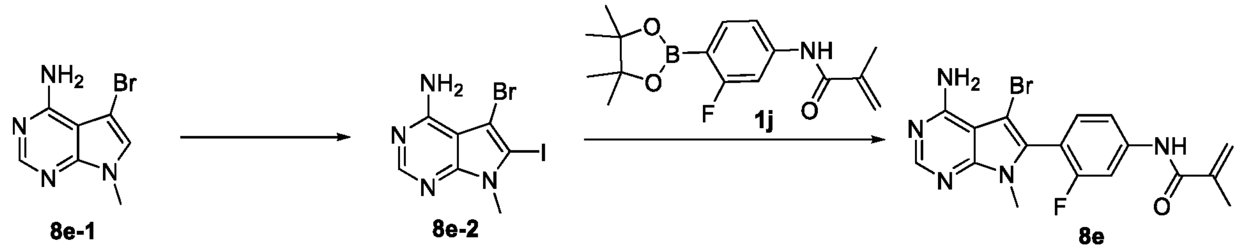

**1j**

**8e-1** **8e-2** **8e**

**Step 1: 5-bromo-6-iodo-7-methyl-7*H*-pyrrolo [2,3-*d*] pyrimidin-4-amino 8e-2**

Compound **8e-1** (10.6 g 0.0466 mol) was dissolved in dichloromethane (100 mL) and trifluoroacetic acid (26.7 g 0.2334 mol), at 10 °C, N-iodosuccinimide (10.5 g 0.0466 mol) was added in batches to react for 4 h. After the reaction was completed, saturated sodium bicarbonate aqueous solution (150 mL) was added slowly at 10 °C, then a solid was precipitated, filtered and dried to obtain compound **8e-2** (13.4 g), purple solid.
MS (ESI) *m/z* 352.6 [M+H]$^+$.
$^1$H NMR (400 MHz, $CDCl_3$) $\delta$ 8.22 (s, 1H), 5.61 (s, 2H), 3.80 (s, 3H).

**Step 2: *N*-(4-(4-amino-5-bromo-7-methyl-7*H*-pyrrolo [2,3-*d*] pyrimidin-6-yl)** -3-**fluorophenyl) methacrylamide 8e**

Compound **1j** (2 g, 6.5 mmol), compound **8e-2** (2.3 g, 6.5 mmol), tetrakis (triphenylphosphine) palladium (0.65 g, 0.56 mmol), and potassium phosphate (3.6 g, 0.017 mol) were added into a reaction flask, and replaced with nitrogen gas three times, then DMF (35 mL) and water (5 mL) were added, and stirred at 50 °C for 16 h. After the reaction was completed,

water (150 mL) and dichloromethane (200 mL) were added to extract the solution, the organic phase was washed with saturated saline and concentrated to obtain a crude product. The crude product was purified by column chromatography to obtain compound **8e** (1.1 g), light yellow solid.

MS (ESI) *m/z* 404.2 $[M+H]^+$.

$^1H$ NMR (400 MHz, $CDCl_3$) $\delta$ 8.35 (s, 1H), 7.82 (dd, *J* = 11.7, 2.1 Hz, 1H), 7.69 (s, 1H), 7.44 - 7.33 (m, 2H), 5.87 (s, 1H), 5.66 (s, 2H), 5.58 (q, *J* = 1.5 Hz, 1H), 3.66 (d, *J* = 1.1 Hz, 3H), 2.12 (dd, *J* = 1.6, 0.9 Hz, 3H).

**Intermediate 9e *N*-(4-(4-amino-5-bromo-7-methyl-7*H*-pyrrolo [2,3-*d*] pyrimidin -6-yl) phenyl) methacrylamide**

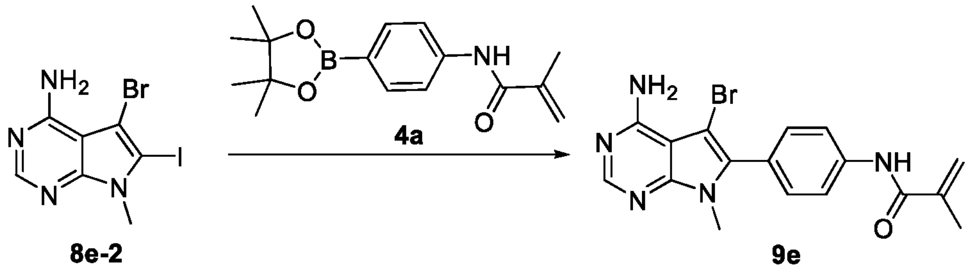


**8e-2** **9e**

Intermediate **9e** was synthesized with reference to intermediate **8e.** MS (ESI) *m/z* 386.32 $[M+H]^+$.

$^1H$ NMR (400 MHz, $CDCl_3$) $\delta$ 8.32 (s, 1H), 7.77 - 7.71 (m, 2H), 7.62 (s, 1H), 7.48 - 7.41 (m, 2H), 5.90 - 5.82 (m, 1H), 5.63 (s, 2H), 5.53 (q, *J* = 1.6 Hz, 1H), 3.67 (s, 3H), 2.10 (dd, *J* = 1.6, 0.9 Hz, 3H).

**Intermediate 12a 1-(4-(4-amino-6-iodo-7-methyl-7*H*-pyrrolo [2,3-*d*] pyrimidin -5-yl) -2-fluorophenyl) imino) hexahydro-1 $\lambda^6$-thiopyran-1-oxide**

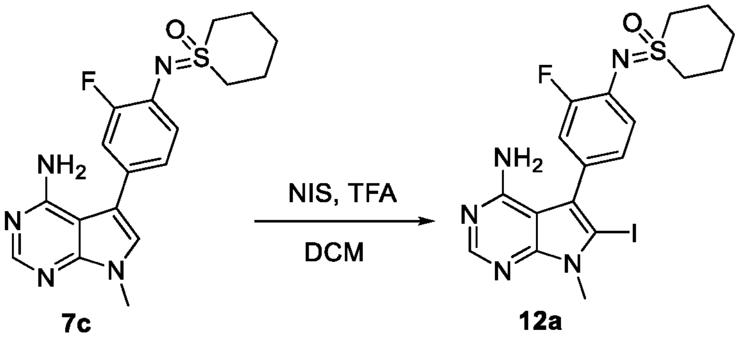


**7c** **12a**

Intermediate **12a** was synthesized with reference to intermediate **84b,** MS (ESI) *m/z* 500 $[M+H]^+$.

**Intermediate 12b *N*-(4-fluoro-3-(4,4,5,5-tetramethyl-1,3,2-dioxaborolan-2-yl) phenyl ) methacrylamide**

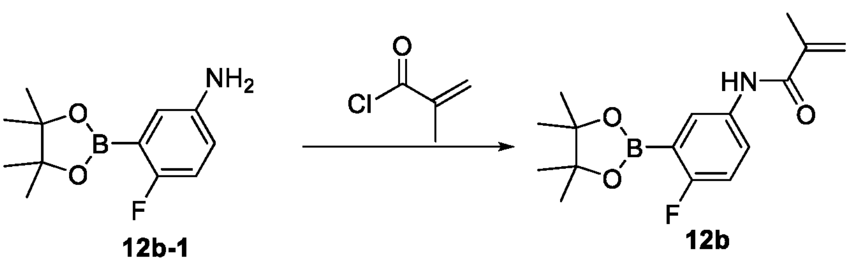

**12b-1** **12b**

Intermediate **12b** was synthesized with reference to compound **4a,** MS (ESI) *m/z* 306 $[M+H]^+$.

**Intermediate 14a *N*-(3-methoxy-4-(4,4,5,5-tetramethyl-1,3,2-dioxaborolan-2-yl) phenyl) methacrylamide**

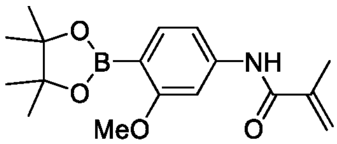

**14a**

Intermediate **14a** was synthesized with reference to WO2020231990A1.

**Intermediate 15a (E)-*N*-(4-(4,4,5,5-tetramethyl-1,3,2-dioxaborolan-2-yl)phenyl)but -2-enamide**

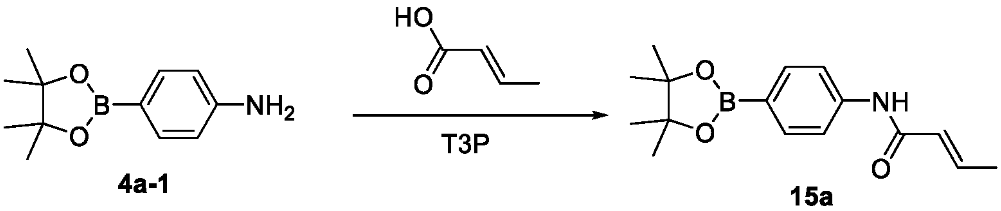


**4a-1** **15a**

Intermediate **15a** was synthesized with reference to compound **21a,** MS (ESI) *m/z* 289 [M+H]+.

$^1$H NMR (400 MHz, $CDCl_3$) $\delta$ 7.80 - 7.73 (m, 2H), 7.57 (d, *J* = 8.1 Hz, *2H),* 7.28 (s, 1H), 6.99 (dq, *J* = 15.2, 6.9 Hz, 1H), 5.94 (dq, *J* = 15.0, 1.7 Hz, 1H), *1.90* (dd, *J* = 6.9, 1.7 Hz, 3H), 1.33 (s, 12H).

**Intermediate 16a (E)-4-(dimethylamino)-N-(4-(4,4,5,5-tetramethyl-1,3,2- dioxaborolane -2-yl) phenyl) but-2-enamide**

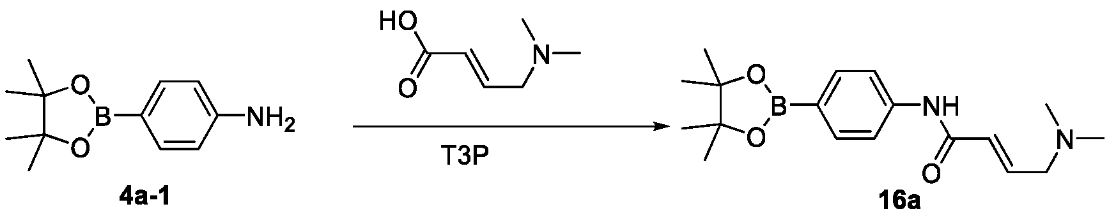


**4a-1** **16a**

Intermediate **16a** was synthesized with reference to compound **21a,** MS (ESI) *m/z* 331 [M+H]+.

**Intermediate 18c 1-((4-(4-amino-6-iodo-7-methyl-7*H*-pyrrolo [2,3-*d*] pyrimidin -5-yl) -2-fluorophenyl) imino) tetrahydro-1H-1 λ⁶-thiophene-1-oxide**

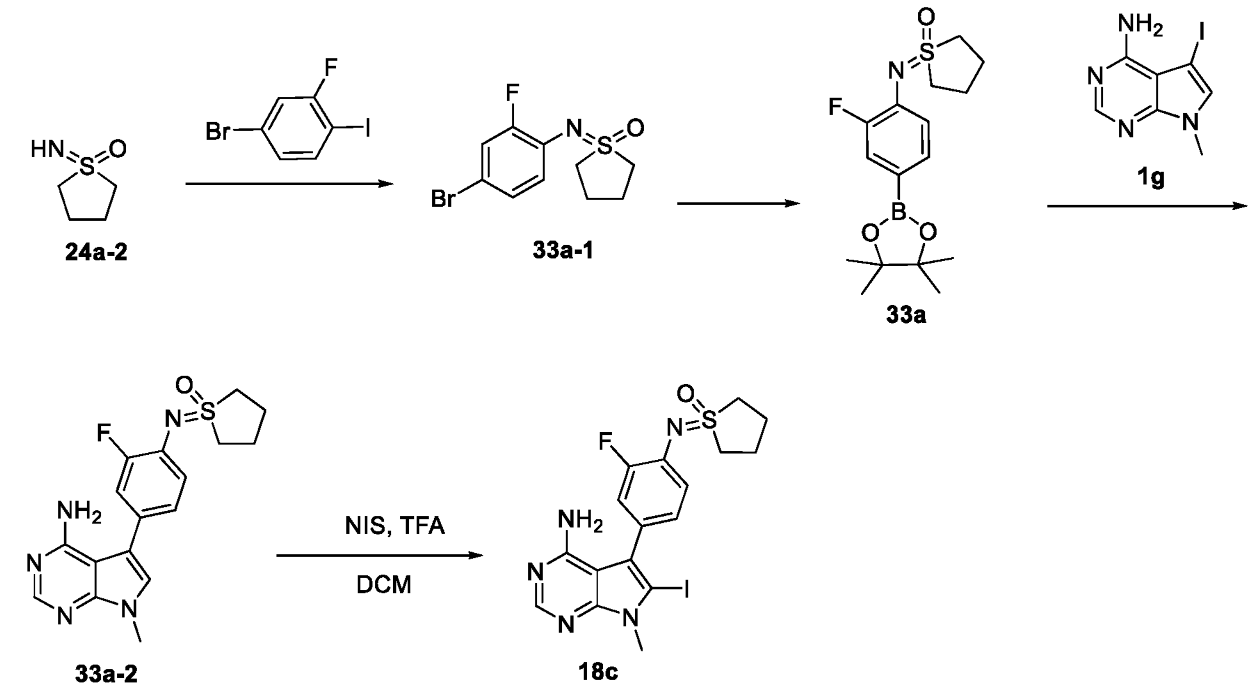

24a-2
33a-1
33a
1g
33a-2
18c

Intermediate **18c** was synthesized with reference to intermediate 77d, MS (ESI) *m*/*z* 486 $[M+H]^+$.

**Intermediate 19f ((4-(4-amino-6-bromo-7-methyl-7*H*-pyrrolo [2,3-*d*] pyrimidin -5-yl)-2-fluorophenyl) imino) dimethyl - λ$^6$-sulfone**

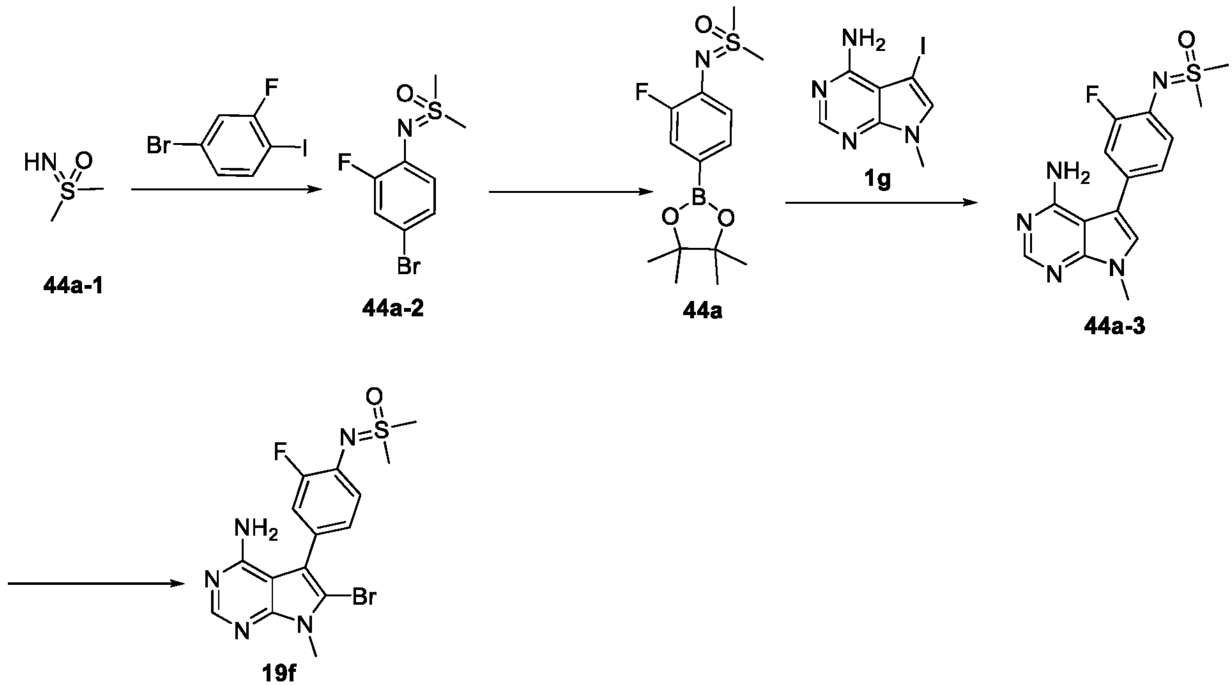
44a-1
44a-2
44a
1g
44a-3
19f

Intermediate **19f** was synthesized with reference to compound 5d, MS (ESI) *m*/*z* 413 $[M+H]^+$.

Intermediate 21a 2-fluoro-*N*-(4-(4,4,5,5-tetramethyl-1,3,2-dioxaborolan-2-yl) phenyl) acrylamide

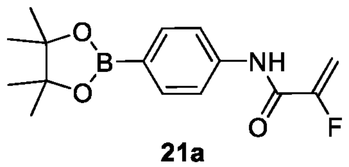

21a

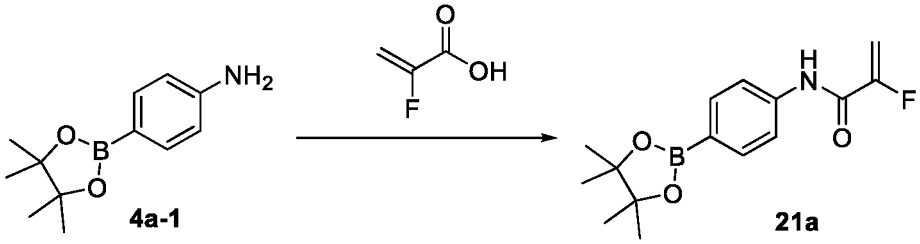

4a-1

21a

Compound **4a-1** (500 mg, 2.28 mmol), 2-fluoroacrylic acid (246.6 mg, 2.74 mmol), and triethylamine (1.38 g, 13.68 mmol) were dissolved in DMF (20 mL), and T3P (1.09 g, 3.42 mmol) was added dropwise at 0 °C to react overnight. After the reaction was completed, $H_2O$ (100 mL) was added and extracted with ethyl acetate (50 mL $\times$ 3) three times. The organic layers were combined, washed with saturated saline solution, the organic layer was dried over anhydrous sodium sulfate, concentrated under reduced pressure, and purified by column chromatography (PE/EA=3/1) to obtain compound **21a** (480 mg), yellow solid.

MS (ESI) *m/z* 292.1 $[M+H]^+$.

$^1H$ NMR (400 MHz, $CDCl_3$) $\delta$ 7.97 (s, 1H), 7.81 (d, *J* = 8.5 Hz, 2H), 7.61 *(d, J* = 8.5 Hz, 2H), 5.83 (dd, *J* = 47.9, 3.4 Hz, 1H), 5.26 *(dd, J* = 15.3, 3.4 Hz, 1H), 1.34 (s, 12H).

**Intermediate 22a N-(3-methyl-4-(4,4,5,5-tetramethyl-1,3,2-dioxaborolan-2-yl) phenyl) methacrylamide**

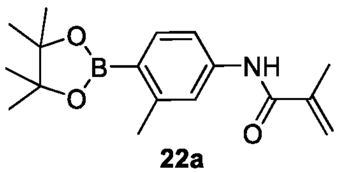

**22a**

Intermediate **22a** was synthesized with reference to WO2022109577 A1.

**Intermediate 24a 1-((4-bromophenyl)imino)tetrahydro-1*H*-1 λ⁶-thiophene-1-oxide**

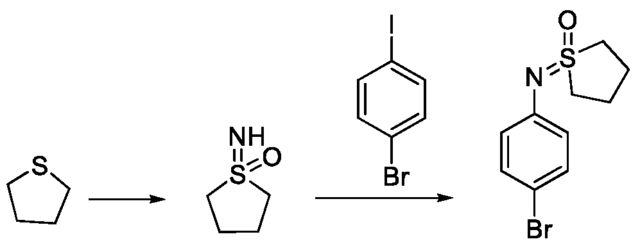

24a-1 24a-2 24a

**Step** 1: **1-iminotetrahydro-1H-1 λ⁶-thiophene-1-oxide 24a-2**

At room temperature, tetrahydrothiophene (1 g, 11.34 mmol) and ammonium carbamate (1.33 g, 17.01 mmol) were added to a round bottom flask and dissolved in methanol (10 mL). It was cooled to 0 °C in an ice water mixture, and iodobenzene diethyl ester (7.67 g, 23.82 mmol) was added in batches. After the addition was completed, the temperature was raised up slowly to room temperature, and the reaction was stirred for 16 h. After the reaction was completed, the

mixture was concentrated under reduced pressure to obtain a crude product. The crude product was filtered under reduced pressure, the filtrate was diluted with water (3 mL), and then sodium bicarbonate solid was added slowly to the diluted filtrate to adjust the pH to 7. The mixture was filtered under reduced pressure, and the filtrate was concentrated under reduced pressure to obtain a crude product. The crude product was purified by column chromatography (DCM/MeOH=20/1) to obtain compound **24a-2** (0.7 g), orange yellow oil.

MS (ESI) *m/z* 120 $[M + H]^+$.

$^1H$ NMR (400 MHz, DMSO-$d_6$) $\delta$ 3.00-2.92 (m, 4H), 2.14-2.01 (m, 4H).

**Step 2 1- ((4-bromophenyl) imino) tetrahydro-1*H*-1 $\lambda^6$-thiophene-1-oxide 24a**

At room temperature, compound **24a-2** (3 g, 25.2 mmol), p-bromoiodobenzene (7.83 g, 27.72 mmol), tris (dibenzylideneacetone) dipalladium (0.46 g, 0.5 mmol), 4,5- bisdiphenyl phosphine-9,9-dimethylxanthene (0.875 g, 1.51 mmol), cesium carbonate (9.86 g, 30.24 mmol), and 1,4-dioxane (30 mL) were sequentially added to a round bottom flask, followed by vacuum pumping and nitrogen gas purging three times, then heated up to 100 °C and stirred for 16 h. After the reaction was completed, the mixture was cooled down to room temperature and filtered under reduced pressure. The filter cake was washed with dichloromethane three times, and all filtrates were combined and concentrated under reduced pressure to obtain a crude product. The crude product was purified by column chromatography (PE/EtOAc=4/1) to obtain compound **24a** (5.3 g), yellow solid.

MS (ESI) *m/z* 273 $[M + H]^+$.

$^1H$ NMR (400 MHz, DMSO-$d_6$) $\delta$ 7.35 (d, J=8.0 Hz, 2H), 6.87 (d, J=8.0 Hz, 2H), 3.33-3.17 (m, 4H), 2.14-2.04 (m, 4H).

**Intermediate 25a N-(3-cyano-4-(4,4,5,5-tetramethyl-1,3,2-dioxaborolan-2-yl) phenyl) methacrylamide**

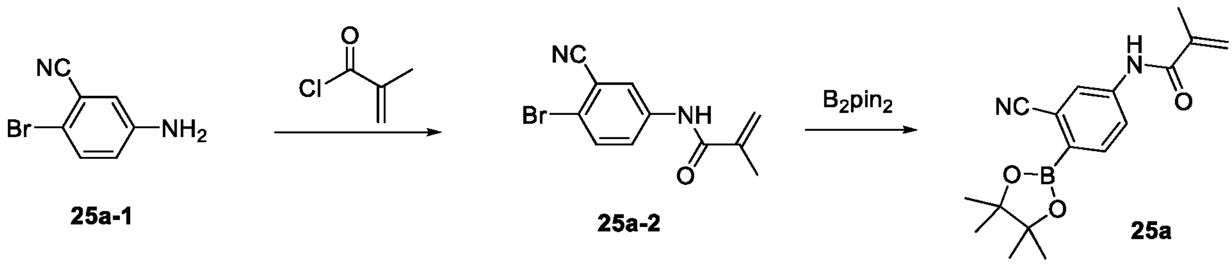


25a-1 25a-2 25a

Intermediate **25a** was synthesized with reference to compound **1j,** MS (ESI) *m/z* 313 $[M+H]^+$.

**Intermediate 16a N-(4-(4,4,5,5-tetramethyl-1,3,2-dioxaborolan-2-yl)phenyl)but-2-ynamide**

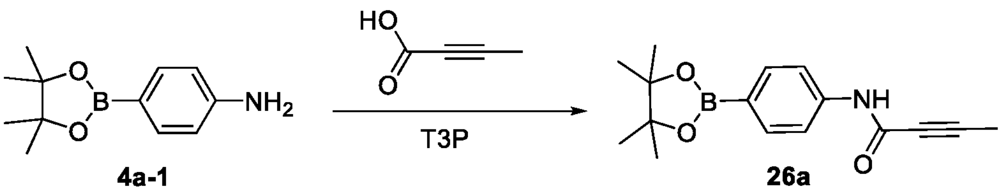


4a-1 26a

Intermediate 26a was synthesized with reference to compound **1a,** MS (ESI) *m/z* 286 $[M+H]^+$.

**Intermediate 27a 1-((2,6-difluoro-4-(4,4,5,5-tetramethyl-1,3,2-dioxaborolan-2-yl) phenyl) imino) tetrahydro-1H-1 $\lambda^6$-thiophene-1-oxide**

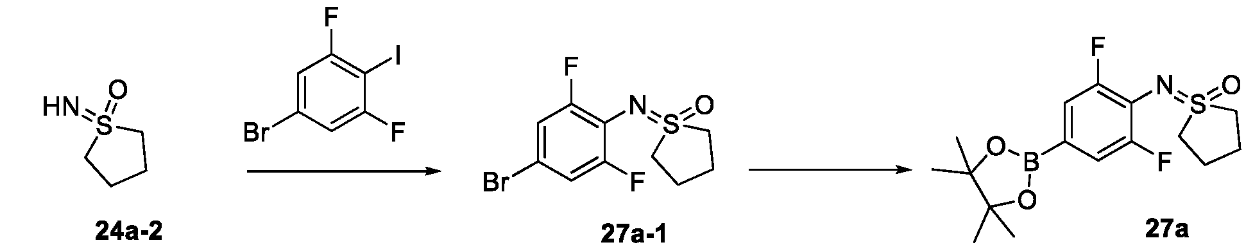

24a-2 27a-1 27a

Intermediate 27a was synthesized with reference to compound **1f,** MS (ESI) *m/z* 358 $[M + H]^+$.

**Intermediate 29a 1- ((5-bromopyridin-2-yl) imino) tetrahydro-1H-1 $\lambda^6$-thiophene -1-oxide**

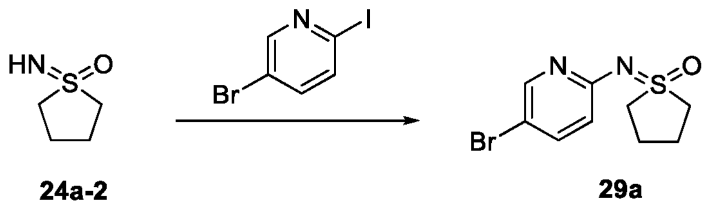

24a-2 29a

Intermediate 29a was synthesized with reference to compound 1d, MS (ESI) *m/z* 276 $[M + H]^+$.

**Intermediate 33a 1-((2-fluoro-4-(4,4,5,5-tetramethyl-1,3,2-dioxaborolan-2-yl) phenyl) imino) tetrahydro-1H-1 $\lambda^6$-thiophene-1-oxide**

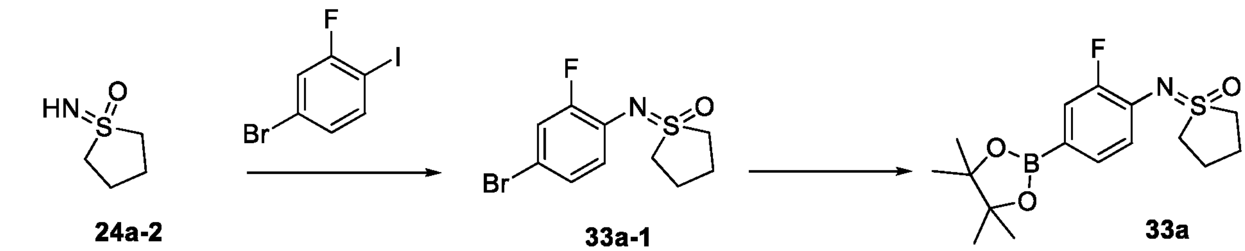

24a-2 33a-1 33a

Intermediate 33a was synthesized with reference to compound **1f,** MS (ESI) *m/z* 340 $[M + H]^+$

**Intermediate 34a 1-((2-methyl-4-(4,4,5,5-tetramethyl-1,3,2-dioxaborolan-2-yl) phenyl) imino) hexahydro-1 $\lambda^6$-thiopyran-1-oxide**

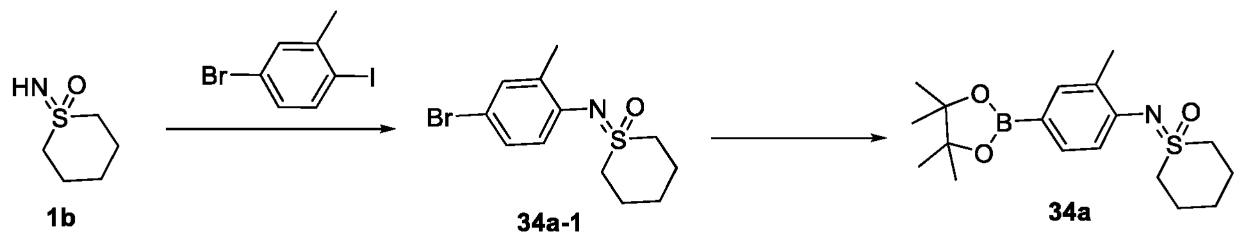

1b 34a-1 34a

Intermediate 34a was synthesized with reference to compound **1f,** MS (ESI) *m/z* 350 $[M + H]^+$.

**Intermediate 60d 1-(4-(4-amino-6-iodo-7-methyl-7*H*-pyrrolo [2,3-*d*] pyrimidin-5- yl) phenyl) imino) tetrahydro-1*H*-λ$^6$-thiophene-1-oxide**

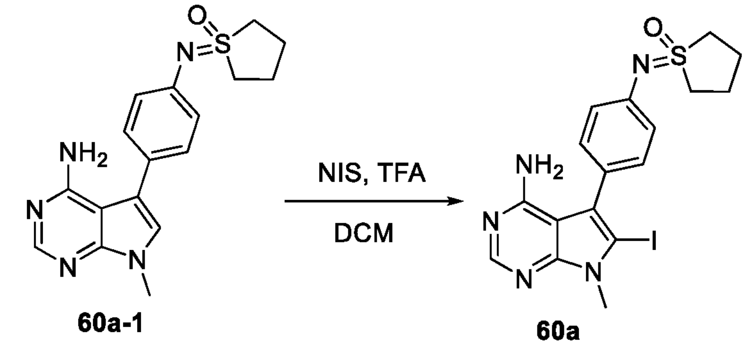


**60a-1** **60a**

Intermediate **60a** was synthesized with reference to intermediate **84b,** MS (ESI) *m*/*z* 468 [M+H]$^+$.

**Intermediate 61a 1-(4-(4-amino-6-iodo-7-methyl-7H-pyrrolo [2,3-d] pyrimidin-5- yl)-3-fluorophenyl) imino) tetrahydro-1H-1λ- thiophene-1-oxide**

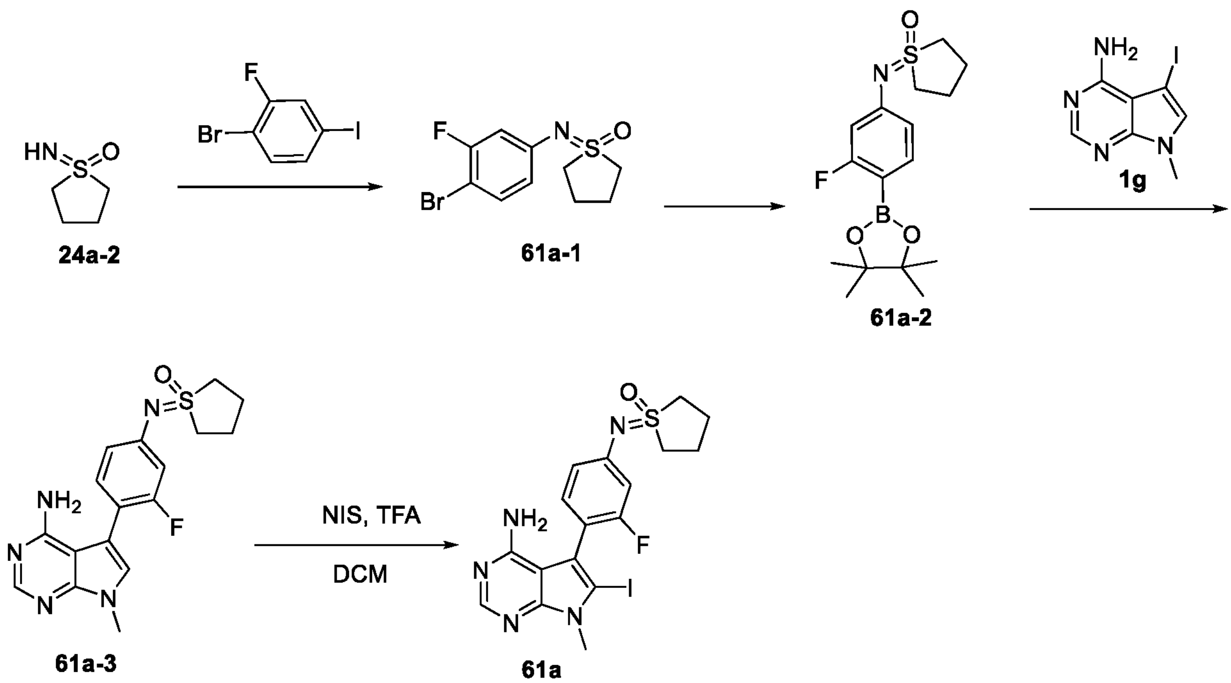


**24a-2** **61a-1** **61a-2** **1g** **61a-3** **61a**

Intermediate 61a was synthesized with reference to intermediate 77d, MS (ESI) *m*/*z* 486 [M+H]$^+$.

**Intermediate 74a N-(3-chloro-4-(4,4,5,5-tetramethyl-1,3,2-dioxaborolan-2-yl) phenyl) methacrylamide**

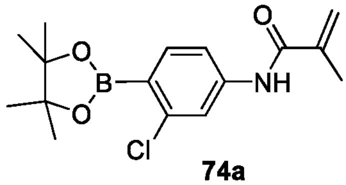

74a

Intermediate 74a was synthesized with reference to WO2023046117 A1.

**Intermediate 84a 1-((4-(4-amino-7-(methyl-$d_3$)-7*H*-pyrrolo [2,3-*d*] pyrimidin-5-yl) -2-fluorophenyl) imino)-1 λ$^6$-thiophene-1-oxide**

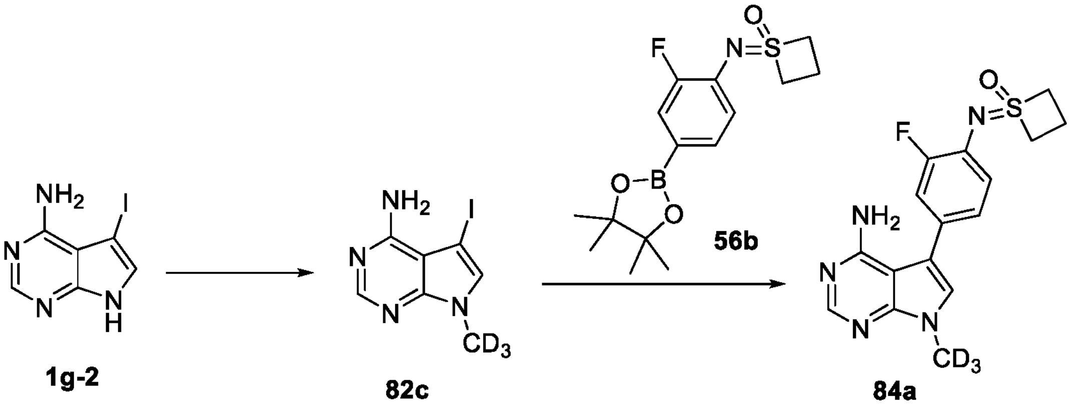

1g-2 82c 56b 84a

**Step 1 5-iodo-7-(methyl-*$d_3$*) -7*H*-pyrrole [2,3-*d*] pyrimidin-4-amine 82c**

Compound **1g-2** (10.0 g, 38.5 mmol) was dissolved in N,N-dimethylformamide (50 mL), cesium carbonate (18.8 g, 57.8 mmol) was added at room temperature, then cooled down to 0 °C, deuterated iodomethane (6.70 g, 46.1 mmol) was added to react at room temperature for 1 h. After the reaction was completed, it was extracted with dichloromethane (100 mL × 3), and the organic phases were combined and concentrated under reduced pressure to obtain a crude product. To the crude product was added water (100 mL × 3), a brown solid was precipitated. The brown solid was collected by filtration to obtain compound **82c** (5.80 g), brown solid.

MS (ESI) *m/z* 277.70 [M + H]$^+$.

$^1$H NMR (400 MHz, DMSO) $\delta$ 8.10(s, 1H), 7.45 (s, 1H), 6.85 - 6.35 (m, 2H).

**Step 2 1- ((4-(4-amino-7-(methyl-*$d_3$*) -7*H*-pyrrolo [2,3-*d*] pyrimidin-5-yl) -2-fluorophenyl) imino) -1 λ$^6$ thiophene-1-oxide 84a**

Compound **82c** (0.50 g, 1.80 mmol) was dissolved in 1,4-dioxane (10 mL) and $H_2O$ (2 mL), compound **56b** (0.65 g, 2.00 mmol), potassium phosphate (1.15 g, 5.40 mmol), and tetrakis (triphenylphosphine) palladium (0.21 g, 0.18 mmol) were added, and reacted at 90 °C for 14 h under $N_2$ protection. After TLC monitored the completion of the reaction , it was extracted with ethyl acetate (50 mL × 3), and the organic phases were combined and concentrated under reduced pressure to obtain a crude product. The crude product was purified by column chromatography (DCM/MeOH=10/1) to obtain compound **84a** (420 mg).

MS (ESI) *m/z* 349.10 [M + H]$^+$.

**Intermediate 85a diethyl ((3-fluoro-4-(4,4,5,5-tetramethyl-1,3,2-dioxaborolan-2- yl) phenyl) imino) - λ$^6$-sulfone**

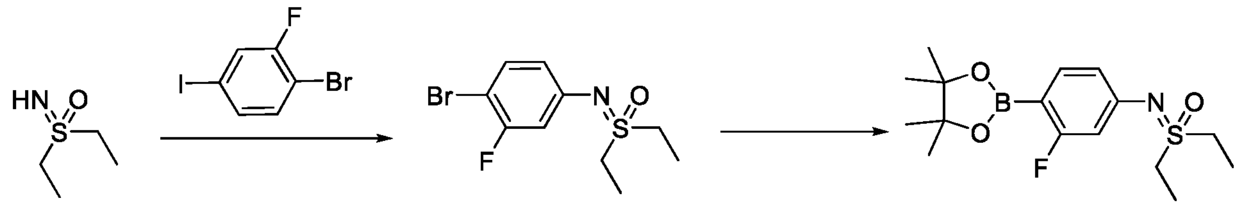

9b    85a-1    85a

Intermediate **85a** was synthesized with reference to compound **1f,** MS (ESI) *m/z* 342 $[M+H]^+$.

**Intermediate 90c *N*-(5-chloro-2-methyl-4-(4,4,5,5-tetramethyl-1,3,2-dioxaborolan- 2-yl) phenyl) methacrylamide**

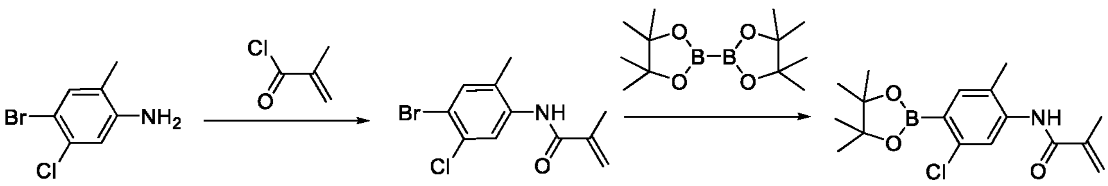

90c-1    90c-2    90c

**Step** 1: **N-(4-bromo-5-chloro-2-methylphenyl) methacrylamide 90c-2**

The **90c-1** (500 mg, 2.27 mmol) and $Et_3$*N*(459 mg, 4.54 mmol) were dissolved in DCM (5 mL), methacryloyl chloride (357 mg, 3.41 mmol) was added to the reaction solution at 0 °C to react for 1 h at 0 °C, then concentrated under reduced pressure, extracted with added $H_2O$ (20 mL) and dichloromethane, the organic phase was washed with saturated saline solution, dried over anhydrous sodium sulfate, concentrated under reduced pressure, and purified by column chromatography to obtain compound **90c-2** (509 mg, 2.27), white solid.
MS (ESI) *m/z* 288 $[M+H]^+$

**Step 2: *N*-(5-chloro-2-methyl-4-(4,4,5,5-tetramethyl-1,3,2-dioxaborolan-2-yl) phenyl) methacrylamide 90c**

Compound **90c-2** (457 mg, 1.29 mmol), anhydrous 1,4-dioxane (6 mL), bis(pinacolato)diboron (606 mg, 2.39 mmol), potassium acetate (468 mg, 4.77 mmol), and Pd(dppf)$Cl_2$ (115 mg, 0.16 mmol) were added to the reaction flask in sequence, after replaced with nitrogen three times, heated up to 90 °C to react for 4 h. TLC showed the formation of new dots. After the reaction was completed, the reaction solution was cooled down to room temperature, diluted with EA and $H_2O$, filtered through diatomaceous earth, extracted with ethyl acetate, and the organic phase was washed with saturated saline solution, dried over anhydrous sodium sulfate, concentrated under reduced pressure, and purified by column chromatography (PE/EA=1/1) to obtain compound **90c** (350 mg), light yellow oil.
MS (ESI) *m/z* 336 $[M+H]^+$

**Intermediate 92a 2-((dimethylamino)methyl)-N-(4-(4,4,5,5-tetramethyl-1,3,2-dioxaborolan-2-yl) phenyl) acrylamide**

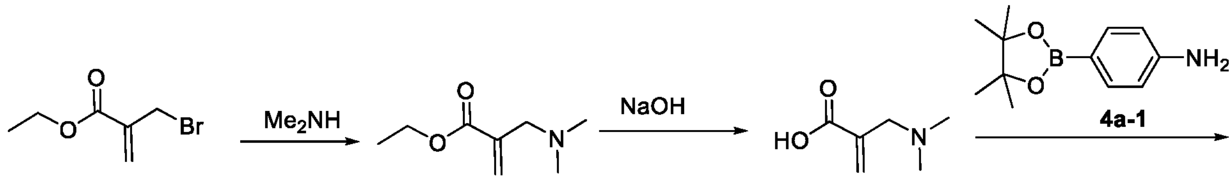


92a-1 92a-2 92a-3

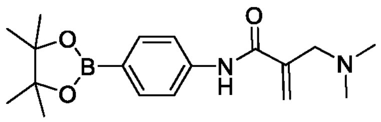

92a

Step 1 **2-((dimethylamino) methyl) ethyl acrylate 92a-2**

Compound **92a** (5 g, 26.04 mmol) was dissolved in tetrahydrofuran (20 mL) at room temperature. After it was cooled down to 0 °C, the dimethylamine (1.40 g, 31.2 mmol) dissolved in tetrahydrofuran (10 mL) was added slowly dropwise to the above solution to react at room temperature for 6 h. After TLC monitored the completion of the reaction, the mixture was filtered through a Buchner funnel, and the filtrate was collected and concentrated under reduced pressure to obtain crude product compound **92a-2** (2 g), yellow oily liquid.

MS (ESI) *m*/*z* 158 $[M + H]^+$.

Step 2 **2-((dimethylamino) methyl) acrylic acid 92a-3**

At room temperature, compound **92a-2** (2 g, 12.7 mmol) was dissolved in tetrahydrofuran (5 mL) and water (5 mL), and lithium hydroxide (365.76 mg, 15.24 mmol) was added in batches. The reaction was stirred at room temperature for additional 4 h. After TLC monitored the completion of the reaction, the mixture was concentrated under reduced pressure to obtain crude product compound **92a-3** (1.2 g), yellow solid.

MS (ESI) *m*/*z* 130 $[M + H]^+$.

**Step 3 2-(dimethylamino)methyl)-*N*-(4-(4,4,5,5-tetramethyl-1,3,2-dioxaborolan-2 -yl** )

**phenyl) acrylamide 92a**

At room temperature, compound **92a-3** (500 mg, 3.88 mmol) and 4-aminophenylboronic acid pinacol ester (679.34 mg, 3.10 mmol) were dissolved in dichloromethane (5 ml), and triethylamine (1.18 g, 11.64 mmol) was added. After it was cooled down to 0 °C, a solution of 1-propylphosphonic anhydride in 50% ethyl acetate (1.85 g, 5.82 mmol) was added slowly dropwise to react at room temperature for 4 h. The reaction solution was diluted with water, extracted with dichloromethane (5 mL × 3), washed with saturated saline solution (10 mL), and the organic phases were combined, dried over anhydrous sodium sulfate, and concentrated under reduced pressure to obtain a crude product. The crude product was purified by column chromatography (DCM/MeOH=15/1) to obtain compound **92a** (130 mg), light yellow solid.

MS (ESI) *m*/*z* 331 $[M + H]^+$.

**Intermediate 93a ((4-bromophenyl) imino) dicyclopropyl-$\lambda^6$- sulfone**

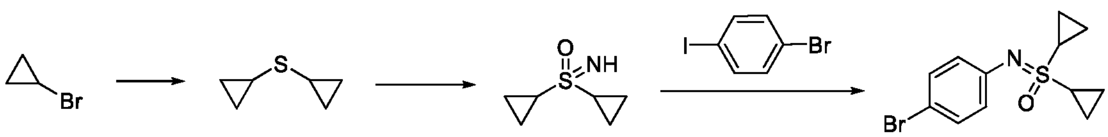

93a-1 93a-2 93a-3 93a

**Step 1 dicyclopropyl sulfide 93a-2**

Compound **93a-1** (2.0 g, 16.7 mmol) was dissolved in DMF (5 mL), $Na_2S$ (640 mg, 8.2 mmol) was added to react in a sealed tube at 100 °C for 16 h. After the reaction was completed, 30mL of ethyl acetate was added, and the organic phase was washed with saline solution (10 mL × 3), concentrated under reduced pressure to obtain a brown crude product **93a-2,** which was directly used in the next step.

**Step 2 dicyclopropyl sulfoximine 93a-3**

The crude product **93a-2** from the previous step was dissolved in methanol (15 mL), $NH_4CO_2NH_2$ (1.3 g, 16.7 mmol) and $PhI(OAc)_2$ (6.7 g, 20.8 mmol) was added in sequence. The reaction solution was left to react at room temperature for 16 h. After TLC monitored the completion of the reaction, it was cooled down to room temperature, the spun-dried crude product was purified by column chromatography (DCM/MeOH=10/1) to obtain compound **93a-3** (264 mg), brown oily substance.

MS (ESI) *m/z* 146.1 $[M + H]^+$.

$^1H$ NMR (400 MHz, $CDCl_3$) $\delta$ 2.54 (m, 2 H), 1.20 (m, 4 H), 1.02 (m, 4 H).

**Step 3 ((4-bromophenyl) imino) dicyclopropyl - λ⁶-sulfone 93a**

Compound **93a-3** (264.0 mg, 1.82 mmol) was dissolved in 1,4-dioxane (5 mL), p-bromoiodobenzene (566 mg, 2.0 mmol), $Pd_2(dba)_3$ (50 mg, 0.05 mmol), xantphos (60 mg, 0.10 mmol), and cesium carbonate (1180 mg, 3.64 mmol) were added at room temperature. The reaction was heated up to 90 °C to react for 4 h, after TLC monitored the completion of the reaction, it was cooled down to room temperature, ethyl acetate (30 mL) and saturated sodium bicarbonate solution (10 mL) was added, and it was extracted with ethyl acetate (20 mL × 3), and the organic phases were combined, washed with saturated saline solution (50 mL), concentrated under reduced pressure to obtain a crude product, and the crude product was purified by column chromatography (PE/EtOAc=3/1) to obtain compound **93a** (60 mg).

MS (ESI) *m/z* 300.0 $[M + H]^+$.

$^1H$ NMR (400 MHz, $CDCl_3$) $\delta$ 7.30-7.25 (m, 2H), 6.97 - 6.92 (m, 2H), 2.54-2.44 (m, 2H), 1.38-1.24 *(m,* 4H), 1.09 - 0.95 (m, 4H).

**Intermediate 103a 5-iodo-7-(2,2,2-trifluoroethyl)-7*H*-pyrrolo [2,3-*d*] pyrimidin -4-amine**

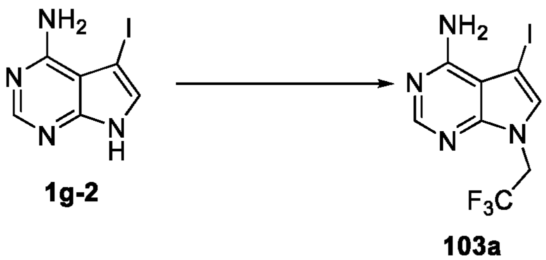

Compound **1g-2** (1.0 g, 3.85 mmol), 2,2,2-trifluoroethyl trifluoromethane sulfonate (1.34 g, 5.78 mmol), and cesium carbonate (2.5 g, 7.7 mmol) was dissolved in DMF (10 mL) to react at room temperature for 3 h, and it was concentrated under reduced pressure to remove the solvent, extracted with $H_2O$ (100 mL) and DCM three times, the organic layers were combined, washed with saturated sodium chloride once, dried over anhydrous sodium sulfate, concentrated under reduced pressure, and purified by column chromatography (PE/EA=1/2) to obtain compound **103a** (850 mg), white solid.

MS (ESI) *m/z* 343.1 $[M + H]^+$.

$^1H$ NMR (400 MHz, DMSO-$d_6$) $\delta$ 8.16 (s, 1H), 7.50 (s, 1H), 6.79 (s, 2H), 5.03 (q, *J* = 9.3 Hz, 2H).

**Intermediate 112a 1-(4-(4-amino-6-(4-amino-2-fluorophenyl)-7-methyl-7*H*- pyrrolo [2,3-*d*] pyrimidin 3-5-yl) phenyl) imino) tetrahydro-1*H*-1 λ⁶-thiophene-1-oxide**

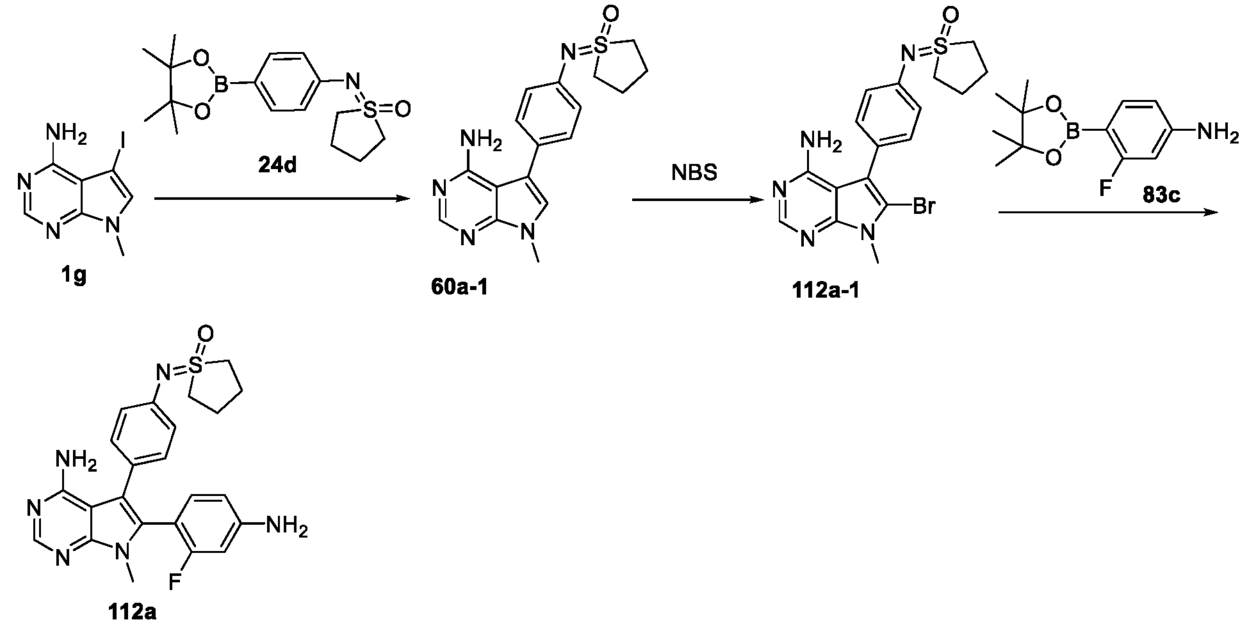


1g 24d 60a-1 112a-1 83c 112a

Compound **112a** was synthesized with reference to the similar synthetic route and steps of compound 83d, wherein the compound **82c** was replaced with compound **1g** in first step, MS (ESI) *m/z* 451.2 [M + H]$^+$.

**Intermediate 113a 1-bromo-4-iodobenzene-3,5-d$_2$**

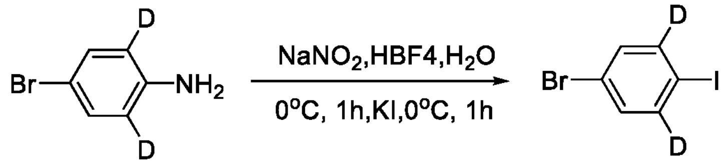


**113a-1** **113a**

Compound **113a-1** was synthesized with reference to ACS Catalysis (2016), 6 (11), 7814-7823. Compound **113a-1** (724 mg, 4.16 mmol), $H_2O$ (33 mL), and 40% $HBF_4$ (2.28 g, 913 mg, 10.40 mmol) were added into the reaction flask in sequence, and it was cooled in an ice salt water bath for 10 min. $NaNO_2$ (344 mg, 4.99 mmol)-$H_2O$ (5.7 mmol) solution was added in the ice salt water bath to react in the ice salt water bath for 1 h. NaI (1.24 g, 8.24 mmol)-$H_2O$ (5.8 mmol) solution was added slowly to the above reaction solution to react in the ice salt water bath for additional 1 h. Then saturated $NaHSO_3$ aqueous solution (20 mL) was added and stirred for additional 10 min, and $H_2O$ and EA were added for extraction, the organic phase was washed with saturated saline solution, dried over anhydrous sodium sulfate, concentrated under reduced pressure, and purified by column chromatography to obtain compound **113a** (225 mg), yellow solid.

MS (GC) *m/z* 284.1 [M]+

**Example 1 *N*-(4-(4-amino-7-methyl-5-(4-(1-oxotetrahydro-2*H*-1λ$^6$-thiopyran-1- imino) phenyl) -7*H*-pyrrolo [2,3-*d*] pyrimidin-6-yl) -3-fluorophenyl) methacrylamide**

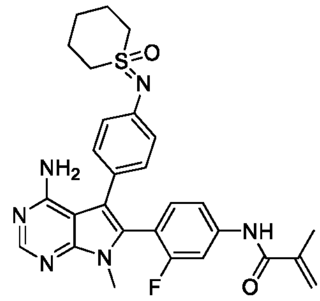

**1**

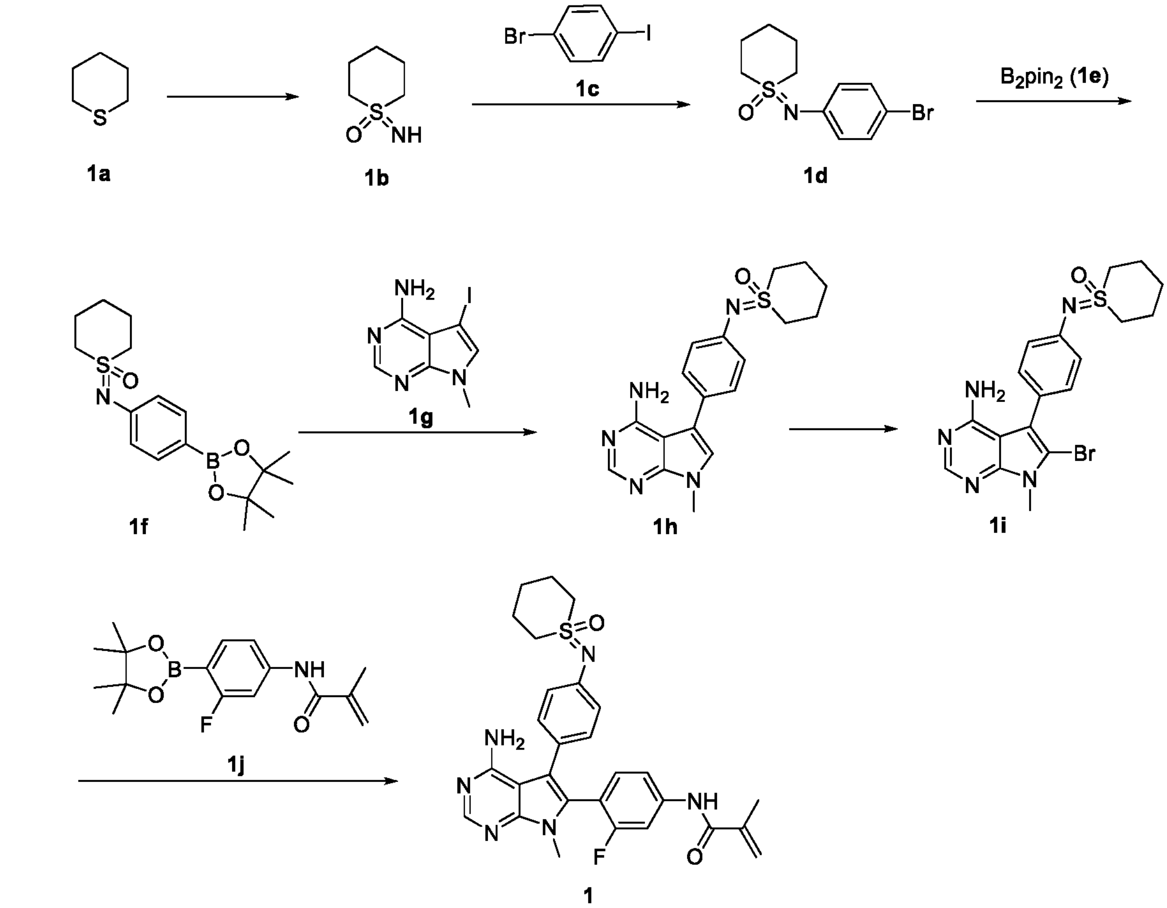


Step 1 1-iminohexahydro-1 λ6-thiopyran-1-oxide **1b**

Tetrahydro-2*H*-pyran **1a** (2.00 g, 19.6 mmol) and ammonium carbamate (2.29 g, *29.4* mmol) were added into a flask in sequence, then MeOH (40 mL) and iodobenzene diethyl ester (13.24 g, 41.1 mmol) were added. The reaction solution was stirred open at room temperature for 30 min, and then it was concentrated under reduced pressure to remove the solvent to obtain a crude product. The crude product was purified by column chromatography (MeOH/DCM=1/80) to obtain compound **1b** (1.62 g), white solid.

MS (ESI) *m/z* 134 [M + H]+.

Step 2 1- ((4-bromophenyl) imino) hexahydro-1 λ6-thiopyran-1-oxide **1d**

Compound **1b** (282 mg, 2.12 mmol), cesium carbonate (806 mg, 2.47 mmol), 1-bromo-4-iodobenzene **1c** (500 mg, 1.77 mmol), $Pd_2(dba)_3$ (40 mg, 0.05 mmol), and Xantphos (77 mg, 0.13 mmol) were added to 1,4-dioxane (10 mL), and the reaction solution was heated up to 105 °C for 13 h. The reaction solution was filtered through diatomaceous earth, the filter cake was washed with ethyl acetate (50 mL), and saturated saline solution (50 mL) was used for washing. The organic phase was dried over anhydrous sodium sulfate and concentrated under reduced pressure to obtain a crude product. The

crude product was purified by column chromatography (PE/EtOAc=4/1 to 1/1) to obtain compound **1d** (410 mg), yellow oily liquid.

MS (ESI) *m/z* 288 $[M + H]^+$.

Step 3 1-(4-(4,4,5,5-methyl-1,3,2-dioxaborolan-2-yl)phenyl)imino)hexahydro-1$\lambda^6$-thiopyran-1-oxide **1f**

Compound **1d** (350 mg, 1.22 mmol), bis(pinacolato)diboron **1e** (372 mg, 1.46 mmol), Pd(dppf)$Cl_2$ (179 mg, 0.24 mmol), potassium acetate (359 mg, 3.66 mmol), and 1,4-dioxane (10 mL) was added into a sealed tube, after replaced with argon gas three times, sealed, and the reaction solution was heated up to 90 °C and stirred for 12 h. Water (10 mL) was added, and it was extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=4/1) to obtain compound **1f** (356 mg), yellow solid.

MS (ESI) *m/z* 336 $[M + H]^+$.

Step 4 1-(4-(4-amino-7-methyl-7*H*-pyrrolo [2,3-*d*] pyrimidin-5-yl)phenyl)imino)hexahydro -1 $\lambda^6$-thiopyran-1-oxide **1h**

Compound **1f** (302 mg, 0.9 mmol), 5-iodo-7-methyl-7*H*-pyrrolo [2,3-*d*] pyrimidin-4-amine **1g** (206 mg, 0.75 mmol), $Pd(PPh_3)_4$ (66 mg, 0.075 mmol), and $K_3PO_4$ (302 mg, 1.88 mmol) were added into a sealed tube, after replaced with argon gas three times, and 1,4-dioxane (5 mL) and $H_2O$ (0.5 mL) were added. The reaction solution was heated up to 90 °C and stirred for 5 h. Then water (30 mL) was added, and extracted with DCM (60 mL × 3). The organic phase was washed with saturated saline solution (50 mL), dried over anhydrous sodium sulfate, and concentrated under reduced pressure to obtain a crude product. The crude product was purified by column chromatography (MeOH/DCM=1/50) to obtain compound **1h** (184 mg), light yellow solid.

MS (ESI) *m/z* 356 $[M + H]^+$.

$^1H$ NMR (500 MHz, $CDCl_3$) $\delta$ 8.31 (s, 1H), 7.33-7.29 (m, 2H), 7.21-7.17 (m, 2H), 6.91 (s, 1H), *5.50* (s, 2H), 3.83 (s, 3H), 3.43-3.35 (m, 2H), 3.14 (ddd, *J*=13.9, 8.9, 5.0 Hz, 2H), 2.15-2.04 (m, 4H), 1.78 (ddd, *J*=12.3, 6.5, 3.8 Hz, 1H), 1.62 (dtt, *J*=13.6, 9.0, 4.6 Hz, 1H).

Step 5 1-(4-(4-amino-6-bromo-7-methyl-7*H*-pyrrolo[2,3-*d*]pyrimidin-5-yl) phenyl) imino) hexahydro-1 $\lambda^6$-thiopyran-1-oxide **1i**

Compound **1h** (150 mg, 0.42 mmol) was dissolved in DMF (3 mL), NBS (83 mg, 0.46 mmol) was added at 0 °C, and the reaction solution was stirred at 0 °C for 1 h. Then DCM (30 mL) was added, and washed with saturated saline solution (10 mL × 3), the organic phase was dried over anhydrous sodium sulfate, and concentrated under reduced pressure to obtain a crude product. The crude product was purified by column chromatography (MeOH/DCM=1/50) to obtain compound **1i** (99 mg), white solid.

MS (ESI) *m/z* 434 $[M + H]^+$.

Step 6 *N*-(4-(4-amino-7-methyl-5-(4-(1-oxotetrahydro-2*H*-1$\lambda^6$-thiopyran-1-imino)phenyl)-7*H*-pyrrolo [2,3-*d*] pyrimidin-6-yl) 3-fluorophenyl) methacrylamide **1**

Compound **1i** (50 mg, 0.12 mmol), *N*- (3-fluoro-4- (4,4,5,5-tetramethyl-1,3,2-dioxaborolan-2-yl) methacrylamide **1j** (42 mg, 0.14 mmol), $Pd(PPh_3)_4$ (28 mg, 0.024 mmol), and $K_3PO_4$ (76 mg, 0.36 mmol) were added into a sealed tube, after replaced with argon gas three times, and 1,4-dioxane (1 mL) and $H_2O$ (0.2 mL) were added, sealed, and the reaction solution was heated up to 100 °C and stirred for 12 h. Then ethyl acetate (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/40) to obtain compound **1** (24 mg), light yellow solid.

MS (ESI) *m/z* 356 $[M + H]^+$.

$^1H$ NMR (500 MHz, $CDCl_3$) $\delta$ 8.30 (s, 1H), 7.95 (s, 1H), 7.71 (dd, *J*=11.6, 2.1 Hz, 1H), 7.12-6.97 (m, 6H), 5.79 (s, 1H), 5.47 (s, 1H), 5.37 (s, 2H), 3.63 (s, 3H), 3.31 (dt, *J*=11.7, 5.0 Hz, 2H), 3.15-3.07 (m, 2H), 2.10-2.03 *(m,* 4H), 2.03 (s, 3H), 1.72 (dp, *J*=15.2, 5.2 Hz, 1H), 1.60 (ddt, *J*=15.2, 11.9, 5.6 Hz, 1H).

**Example 2 1- (4-(4-amino-7-methyl-5-(4-(1-oxotetrahydro-2*H*-1$\lambda^6$-thiopyran-1-imino) phenyl)-7*H*-pyrrolo [2,3-*d*] pyrimidin-6-yl)-3,6-dihydropyridine-1(2*H*)-yl)prop-2-enyl-1-one**

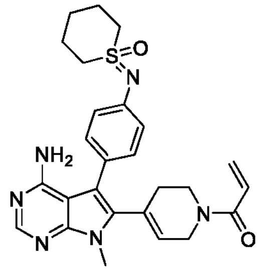

2

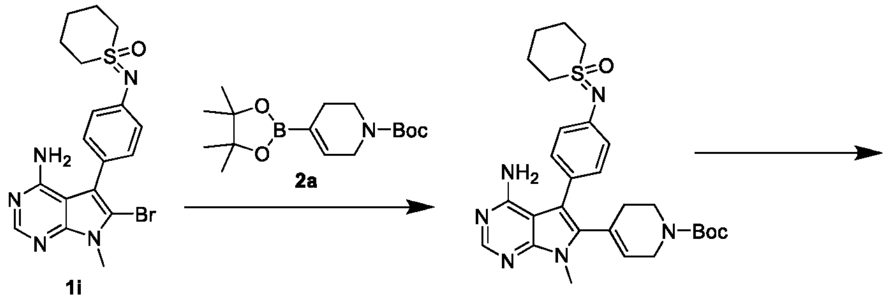


1i

2b

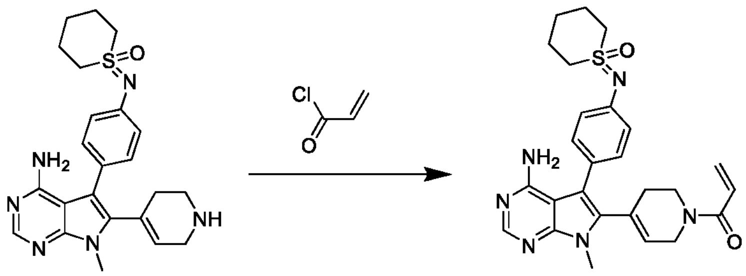

2c

2

Step 1 4- (4-amino-7-methyl-5-(4-((1-oxotetrahydro-2*H*-1λ6-thiopyran-1-imino)phenyl)- 7*H*-pyrrolo [2,3-*d*] pyrimidin-6-yl) -3,6-dihydropyridine-1 (2*H*) - tert-butyl carbonate **2b**

Compound **1i** (345 mg, 0.8 mmol), compound **2a** (295.6 mg, 0.96 mmol), $Pd(PPh_3)_4$ (184 mg, 0.16 mmol), and potassium phosphate (509.5 mg, 2.4 mmol) were dissolved in anhydrous 1,4-dioxane (8 mL) and $H_2O$ (2 mL), after replaced with nitrogen gas three times, the reaction was refluxed at 100 °C for 12 h. After the reaction was completed, the reaction solution was cooled down to room temperature, filtered through diatomaceous earth, concentrated under reduced pressure to remove the solvent, and it was extracted with EA/$H_2O$ three times, the organic layers were combined, washed with saturated saline solution once, dried over anhydrous sodium sulfate, concentrated under reduced pressure, and purified by column chromatography (DCM/MEOH=40/1) to obtain compound **2b** (312 mg), yellow brown solid.

MS (ESI) *m/z* 537 $[M + H]^+$.

Step 2 1- (4-(4-amino-7-methyl-6-(1,2,3,6-tetrahydropyridine-4-yl)-7*H*-pyrrolo[2,3-*d*] pyrimidin-5-yl)phenyl)imino)hexahydro-1λ6-thiopyran-1-oxide **2c**

Compound **2b** (310 mg, 0.58 mmol) was dissolved in dichloromethane (10 mL), trifluoroacetic acid (2 mL) was added dropwise at room temperature to react for 5 h. After the reaction was completed, it was concentrated under reduced pressure to obtain a crude product **2c** (250 mg).

MS (ESI) *m/z* 437 $[M + H]^+$.

Step 3 1-(4-(4-amino-7-methyl-5-(4-(1-oxotetrahydro-2*H*-1λ$^6$-thiopyran-1-imino) phenyl) -7*H*-pyrrolo[2,3-*d*]pyrimidin-6-yl)-3,6-dihydropyridine-1(2H)-yl)prop-2-enyl-1-one **2**

Compound **2c** (125 mg, 0.29 mmol) was dissolved in ultra dry DCM (3 mL), TEA (232 mg, 2.29 mmol) was added at 0 °C and stirred for 5 min. A solution of acryloyl chloride (28.5 mg, 0.32 mmol) in dichloromethane (2 mL) was added slowly dropwise to the above reaction solution to react at 0 °C for 1 h. After the reaction was completed, it was extracted with DCM/$H_2O$ three times, the organic layers were combined, washed with saturated saline solution, dried over anhydrous sodium sulfate, concentrated under reduced pressure, and purified by column chromatography (DCM/MeOH=30/1) to obtain compound **2** (103 mg), white powdery solid.
MS (ESI) *m/z* 491 [M + H]$^+$.
$^1$H NMR (400 MHz, MeOD-d4) δ 8.13 (s, 1H), 7.26 (dd, *J*=8.5, 2.9 Hz, 2H), 7.16 (dd, *J=8.3,* 1.3 Hz, 2H), 6.84-6.65 (m, 1H), 6.29-6.14 (m, 1H), 6.04-5.88 (m, 1H), 5.86-5.67 (m, 1H), 4.32-4.19 (m, 2H), 3.72 *(s,* 3H), 3.70 (t, *J*=5.3 Hz, 2H), 3.47-3.36 (m, 2H), 3.30-3.21 (m, 2H), 2.22 8-2.14 (m, 2H), 2.12-2.01 (m, 4H), 1.81-1.73 (m, 1H), 1.72-1.61 (m, 1H).

**Example 3 1-(4-(4-amino-7-methyl-5-(4-(1-oxotetrahydro-2*H*-1λ$^6$-thiopyran-1-imino) phenyl)-7*H*-pyrrolo[2,3-*d*] pyrimidin-6-yl)-3,6-dihydropyridine-1(2*H*)-yl)2-methylprop-2-enyl-1-one**

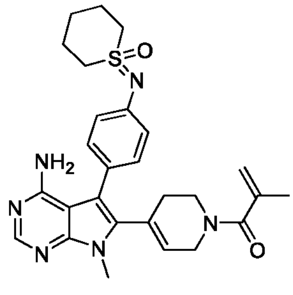

3

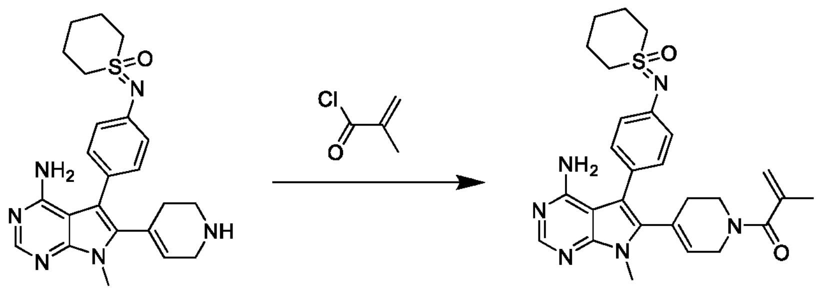

2c

3

Step 1 1-(4-(4-amino-7-methyl-5-(4-(1-oxotetrahydro-2H-1λ$^6$-thiopyran-1-imino) phenyl)-7*H*-pyrrolo[2,3-*d*]pyrimidin-6-yl)-3,6-dihydropyridine-1(2H)-yl)2-methylprop-2-enyl-1- one

Compound **2c** (125 mg, 0.29 mmol) was dissolved in ultra dry DCM (3 mL), TEA (232 mg, 2.29 mmol) was added at 0 °C and stirred for 5 min. A solution of methacryloyl chloride (32.8 mg, 0.32 mmol) in dichloromethane (2 mL) was added dropwise to the above reaction solution to react at 0 °C for 1 h. After the reaction was completed, it was extracted with DCM/$H_2O$ three times, the organic layers were combined, washed with saturated saline solution, dried over anhydrous sodium sulfate, concentrated under reduced pressure, and purified by column chromatography (DCM/MeOH=30/1) to obtain compound **3** (67.8 mg), white powdery solid.
MS (ESI) *m/z* 505 [M + H]$^+$.
$^1$H NMR (400 MHz, MeOD-d4) δ 8.13 (s, 1H), 7.27 (d, *J*=8.0 Hz, 2H), 7.16 (d, *J=8.4 Hz,* 2H), 5.93 (d, *J*=31.5 Hz, 1H), 5.24 (d, *J*=13.4 Hz, 1H), 5.02 (s, 1H), 4.20 (d, *J=3.5* Hz, 2H), 3.77-3.6 (m, 5H), 3.48-3.37 (m, 2H), 3.29-3.22 (m, 2H), 2.25-2.14 (m, 2H), *2.13-1.99* (m, 4H) 1.93 (t, *J*=1.4 Hz, 3H), 1.82-1.72 (m, 1H), 1.71-1.61 (m, 1H).

**Example 4 N-(4-(4-amino-7-methyl-5- (4- (1-oxotetrahydro-2H-1$\lambda^6$-thiopyran-1- imino) phenyl) -7H-pyrrolo [2,3-d] pyrimidin-6-yl) phenyl) methacrylamide**

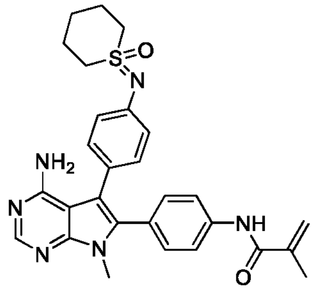

**4**

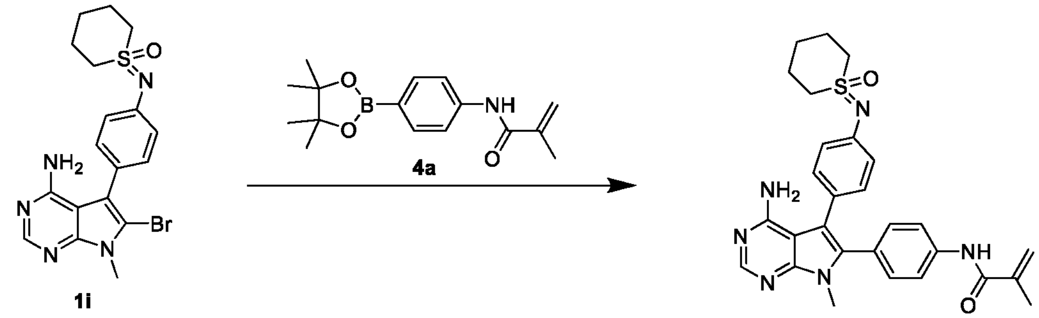

**1i** **4a** **4**

Step 1 *N*-(4-(4-amino-7-methyl-5- (4- (1-oxotetrahydro-2H-1 $\lambda^6$-thiopyran-1-imino) phenyl) -7H-pyrrolo [2,3-d] pyrimidin-6-yl) phenyl) methacrylamide **4**

Compound **1i** (180 mg, 0.42 mmol), compound **4a** (143.2 mg, 0.5 mmol), $Pd(PPh_3)_4$ (97.1 mg, 0.08 mmol), and potassium phosphate (267.5 mg, 1.26 mmol) were dissolved in anhydrous 1,4-dioxane (4 mL) and $H_2O$ (1 mL), after replaced with nitrogen gas three times, heated up to 100 °C, and the reaction was refluxed for 12 h. After the reaction was completed, the reaction solution was cooled down to room temperature, filtered through diatomaceous earth, concentrated under reduced pressure to remove the solvent, and it was extracted with EA/$H_2O$ three times, the organic layers were combined, washed with saturated salt once, dried over anhydrous sodium sulfate, concentrated under reduced pressure, and purified by column chromatography (DCM/MeOH=40/1) to obtain compound **4** (128 mg), light yellow solid.

MS (ESI) *m/z* 515 $[M + H]^+$.

$^1H$ NMR (400 MHz, MeOD-d4) $\delta$ 8.17 (s, 1H), 7.67-7.61 (m, 2H), 7.29-7.24 (m, 2H), 7.17-7.11 (m, 2H), 7.08-7.03 (m, 2H), 5.80 (s, 1H), 5.52 (dd, *J*=1.7, 0.8 Hz, 1H), 3.68 (s, 3H), 3.43-3.33 (m, 2H), 3.28-3.17 (m, 2H), 2.07-1.99 (m, 7H), 1.78-1.69 (m, 1H), 1.68-1.59 (m, 1H)).

**Example 5 *N*-(4-(4-amino-5-(3-methoxy-4- (1-oxotetrahydro-2H-1$\lambda^6$-thiopyran -1-imino) phenyl )-7-methyl -7*H*-pyrrolo [2,3-*d*] pyrimidin-6-yl)- 3- fluorophenyl) methacrylamide**

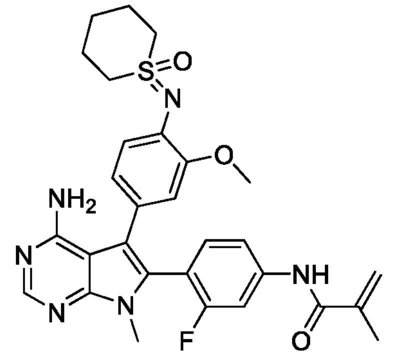

**5**

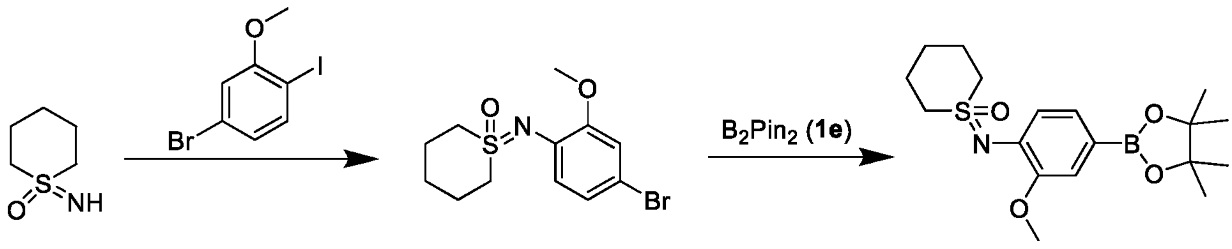


**1b** **5a** **5b**

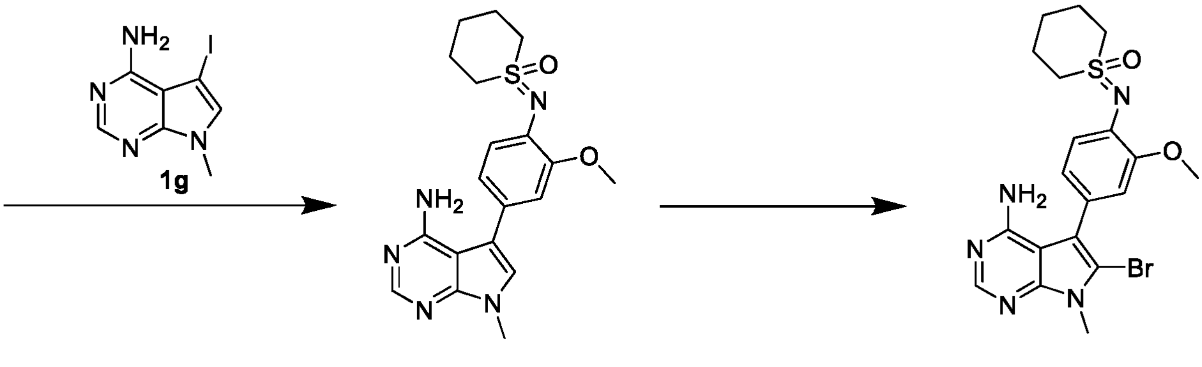

**1g**

**5c** **5d**

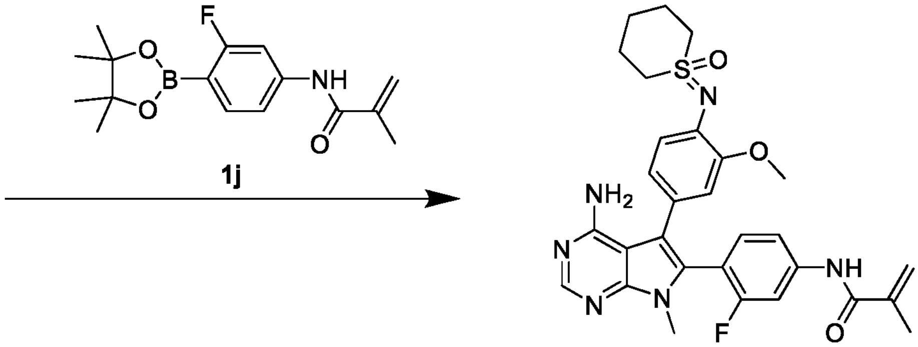

**1j**

**5**

Step 1 1- ((4-bromo-2-methoxyphenyl) imino) hexahydro-1 λ6-thiopyran-1-oxide 5a

Compound **1b** (638 mg, 4.79 mmol), 4-bromo-1-iodo-2-methoxybenzene (1.79 g, 5.75 mmol), $Pd_2(dba)_3$ (109.7 mg, 0.12 mmol), Xantphos (208 mg, 0.36 mmol), cesium carbonate (2.3 g, 7.19 mmol) were sequentially added to the reaction flask, after replaced with nitrogen gas three times, anhydrous 1,4-dioxane (25 mL) was added, the temperature was raised to 100 °C, and the reaction was refluxed for 6 h. After the reaction was completed, the reaction solution was filtered through diatomaceous earth, concentrated under reduced pressure, and purified by column chromatography (PE/EA=5/1) to obtain compound **5a** (650 mg), light yellow oil.

MS (ESI) *m/z* 318 $[M + H]^+$.

Step 2 1- ((2-methoxy-4- (4,4,5,5-tetramethyl-1,3,2-dioxaborolan-2-yl) phenyl) imino) hexahydro-1 λ6-thiopyran-1-oxide **5b**

Compound **5a** (650 mg, 2.05 mmol), bis(pinacolato)diboron **1e** (624.8 mg, 2.46 mmol), potassium acetate (603.6 mg, 6.15 mmol), and $Pd(dppf)Cl_2$ (300 mg, 0.41 mmol) were added into a reaction flask in sequence, after replaced with nitrogen gas three times, anhydrous 1,4-dioxane (15 mL) was added to dissolve, and the reaction solution reacted overnight at 90 °C. After the reaction was completed, the reaction solution was cooled down to room temperature, filtered through diatomaceous earth, concentrated under reduced pressure, extracted with DCM/$H_2O$ three times, the organic layers were combined, washed with saturated salt once, dried over anhydrous sodium sulfate, concentrated and purified by silica gel column chromatography (PE/EA=4/1) to obtain compound **5b** (405 mg), yellow solid.

MS (ESI) *m/z* 366 $[M + H]^+$.

Step 3 1- (4- (4-amino-7-methyl-7*H*-pyrrolo [2,3-*d*] pyrimidin-5-yl) -2-methoxyphenyl) imino) hexahydro-1 λ6-thiopyr-an-1-oxide **5c**

Compound **5b** (401 mg, 1.1 mmol), compound **1g** (274 mg, 1.0 mmol), $Pd(PPh_3)_4$ (116 mg, 0.1 mmol), and potassium phosphate (530.7 mg, 2.5 mmol) were dissolved in 1,4-dioxane (12 mL) and water (3 mL), after replaced with nitrogen gas three times, the reaction was carried out overnight at 90 °C. It was filtered through diatomaceous earth, concentrated under reduced pressure to remove the solvent, extracted with EA/$H_2O$ three times, the organic layers were combined and washed with saturated salt water once, dried over anhydrous sodium sulfate, concentrated under reduced pressure and purified by column chromatography (DCM/MEOH=30/1) to obtain compound **5c** (180 mg), light yellow solid.

MS (ESI) *m/z* 382 $[M + H]^+$.

Step 4 1- (4-(4-amino-6-bromo-7-methyl-7*H*-pyrrolo [2,3-*d*] pyrimidin-5-yl)-2-methoxyphenyl) imino) hexahydro-1 λ6-thiopyran-1-oxide **5d**

Compound **5c** (174 mg, 0.45 mmol) was dissolved in anhydrous DMF (4 mL), NBS (88 mg, 0.50 mmol) was added at 0 °C to react for 1 h. After the reaction was completde, sodium thiosulfate solution was added to quench the reaction, and it was extracted with EA/$H_2O$ 4 times, the organic layers were combined, washed with saturated salt water once, dried over anhydrous sodium sulfate, concentrated under reduced pressure, and purified by column chromatography (DCM/MEOH=30/1) to obtain compound **5d** (107 mg), yellow solid.

MS (ESI) *m/z* 464 $[M + H]^+$.

$^1H$ NMR (400 MHz, $CDCl_3$) δ 8.28 (s, 1H), 7.31 (d, *J*=7.7 Hz, 1H), 6.92 (d, *J*=8.4 *Hz,* 2H), 5.53 (s, 2H), 3.87 (s, 3H), 3.85 (s, 3H), 3.43-3.34 (m, 2H), 3.28-3.16 *(m,* 2H), 2.16-2.03 (m, 4H), 1.79-1.62 (m, 2H).

Step 5 *N*- (4- (4-amino-5- (3-methoxy-4- (1-oxotetrahydro-2*H*-1 λ6-thiopyran-1-imino) phenyl) -7-methyl-7*H*-pyrrolo [2,3-*d*] pyrimidin-6-yl) -3-fluorophenyl) methacrylamide **5**

Compound **5d** (101 mg, 0.22 mmol), compound **1j** (99.8 mg, 0.33 mmol), $Pd(PPh_3)_4$ (50.4 mg, 0.04 mmol), and potassium phosphate (138.9 mg, 0.65 mmol) were dissolved in 1,4-dioxane (8 mL) and water (2 mL), after replaced with nitrogen gas three times, the reaction was refluxed at 100 °C for 12 h. After the reaction was completed, the reaction solution was cooled down to room temperature, filtered through diatomaceous earth, concentrated under reduced pressure to remove the solvent, and it was extracted with DCM/$H_2O$ three times, the organic layers were combined, washed with saturated salt water once, dried over anhydrous sodium sulfate, concentrated under reduced pressure, and purified by column chromatography (DCM/MEOH=40/1) to obtain compound **5** (63 mg), light yellow solid.

MS (ESI) *m/z* 563 $[M + H]^+$.

$^1H$ NMR (400 MHz, Methanol-d4) δ 8.40 (s, 1H), 7.77 (dd, *J*=12.2, 2.1 Hz, 1H), 7.44 (dd, J=8.5, 2.1 Hz, *1H), 7.25* (dd, *J=9.9,* 8.1 Hz, 2H), 6.98-6.82 (m, 2H), 5.82 (s, 1H), 5.57 (q, *J*=1.6 Hz, 1H), 3.75 *(s,* 3H), 3.73 (s, 3H), 3.69-3.59 (m, 2H),

3.57-3.43 (m, 2H), 2.18-2.07 (m, 4H), 2.02 (s, 3H), 1.84-1.65 (m, 2H).

**Example 6 N-(4-(4-amino-5-(4- (4,4-difluoro-1-oxotetrahydro-2*H*-λ$^6$-thiopyran-1-imino) phenyl) -7-methyl-7*H*-pyrrolo [2,3-*d*] pyrimidin-6-yl) -3-fluorophenyl) methacrylamide**

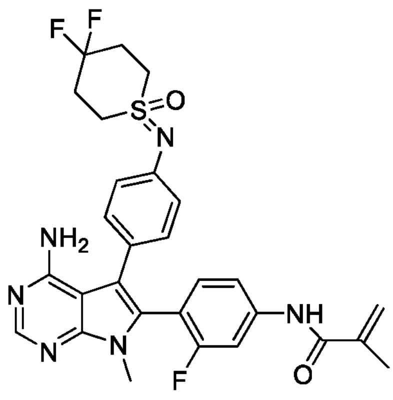

**6**

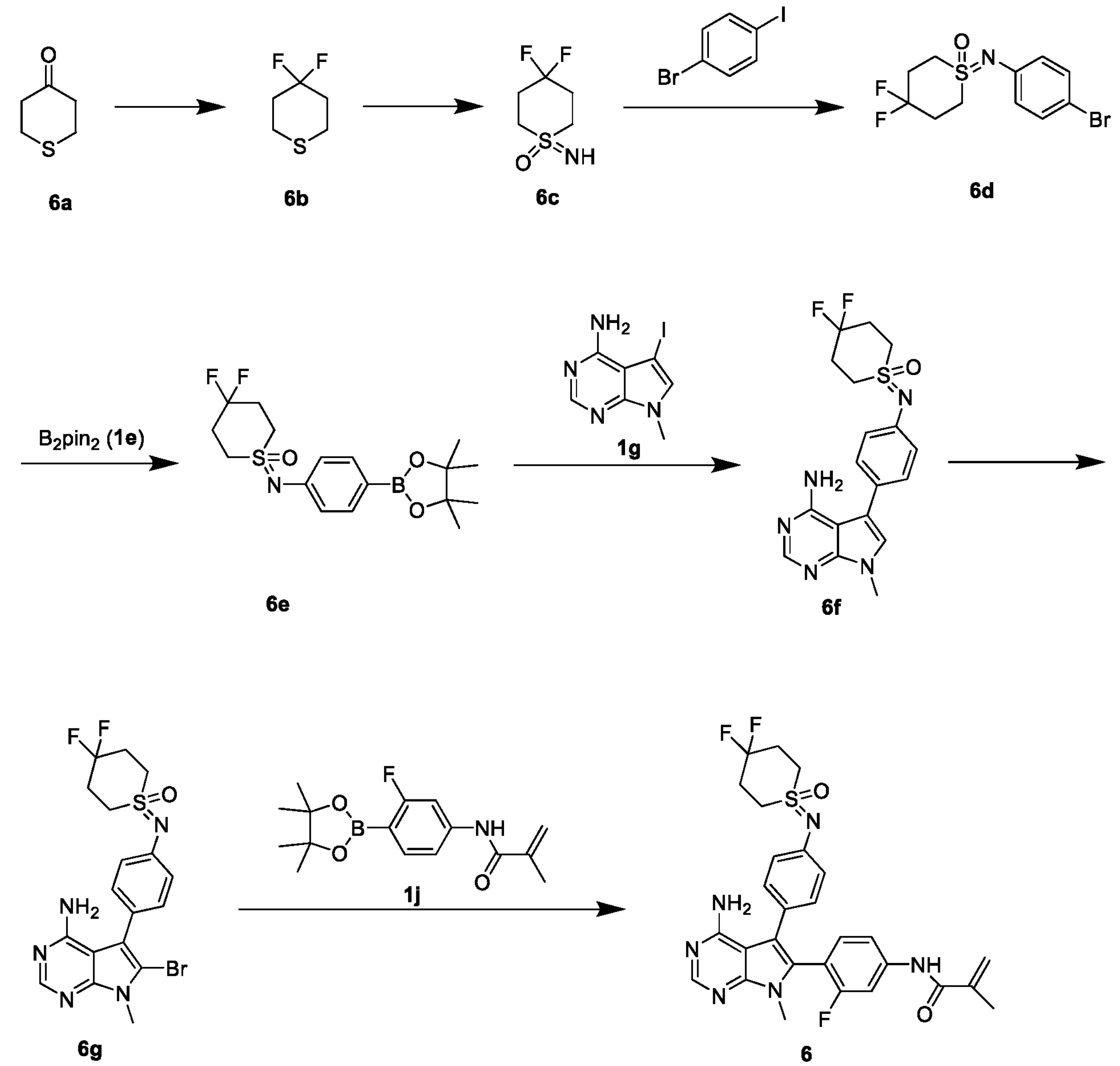


6a 6b 6c 6d 6e 6f 6g 6

Step 1 4,4-difluorotetrahydro-2*H*-pyran **6b**

Compound **6a** (2.0 g, 17.24 mmol) was dissolved in ultra dry DCM (100 mL), stirred at 0 °C for 5 min, BAST (5.6 g, 34.47 mmol) was added to react for additional 4 h. After the reaction was completed, the reaction solution was poured into a cold $NaHCO_3$ aqueous solution, and it was extracted with DCM three times, the organic layers were combined, and washed with saturated salt water once, dried over anhydrous sodium sulfate, concentrated and dried to obtain compound **6b** (2.38 g), oily liquid, which were used directly for the next reaction step without purification.

MS (ESI) *m*/*z* 139 $[M + H]^+$.

Step 2 4,4-difluoro-1-imino-tetrahydro-1$\lambda^6$-thiopyran-1-oxide **6c**

Compound **6b** (2.38 g, 17.24 mmol) was dissolved in anhydrous methanol (100 mL), ammonium carbonate (2.49 g, 25.86 mmol) and iodobenzoic acid (13.9 g, 43.1 mmol) were added to the above reaction solution sequentially, and reacted at room temperature for 4 h. It was concentrated under reduced pressure to remove the solvent, and extracted with DCM/$H_2O$ three times, the organic layers were combined, washed with saturated salt water once, dried over anhydrous sodium sulfate, concentrated and purified by column chromatography (DCM/MEOH=40/1) to obtain compound **6c** (1.07 g), yellow liquid.

MS (ESI) *m*/*z* 170 $[M + H]^+$.

Step 3 1- ((4-bromophenyl)imino)-4,4-difluorotetrahydro-1λ$^6$-thiopyran-1-oxide **6d**

Compound **6c** (1.07 g, 6.33 mmol), p-bromoiodobenzene (2.14 g, 7.60 mmol), $Pd_2(dba)_3$ (144.9 mg, 0.16 mmol), Xantphos (274.7 mg, 0.47 mmol), and cesium carbonate (3.09 g, 9.49 mmol) were added to a reaction flask sequentially, after replaced with nitrogen gas three times, anhydrous 1,4-dioxane (35 mL) was added, the temperature was raised up to 100 °C, and the reaction was refluxed for 6 h. The reaction solution was filtered through diatomaceous earth, concentrated under reduced pressure, and purified by column chromatography (PE/EA=5/1) to obtain compound **6d** (228 mg), yellow solid.

MS (ESI) *m/z* 324 $[M + H]^+$.

Step 4 4,4-difluoro-1-((4-(4,4,5,5-tetramethyl-1,3,2-dioxaborolan-2-yl)phenyl)imino ) hexahydro-1λ$^6$ -thiopyran-1-oxide **6e**

Compound **6d** (224 mg, 0.69 mmol), compound **1e** (211.3 mg, 0.83 mmol), potassium acetate (203 mg, 2.07 mmol), and $Pd(dppf)Cl_2$ (101 mg, 0.14 mmol) were added to the reaction flask in sequence, after replaced with nitrogen three times, anhydrous 1,4-dioxane (8 mL) was added to dissolve, and then heated up to 90 °C and reacted overnight. It was filtered through diatomaceous earth, concentrated under reduced pressure, extracted with DCM/$H_2O$ three times, the organic layers were combined, washed with saturated salt once, dried over anhydrous sodium sulfate, concentrated and purified by column chromatography (PE/EA=3/1) to obtain compound **6e** (164 mg), yellow solid.

MS (ESI) *m/z* 372 $[M + H]^+$.

Step 5 1- (4-(4-amino-7-methyl-7*H*-pyrrolo [2,3-*d*] pyrimidin-5-yl) phenyl) imino)-4,4-difluorohexahydro-1 λ$^6$-thiopyran-1-oxide **6f**

Compound **6e** (158 mg, 0.43 mmol), compound **1g** (106 mg, 0.39 mmol), $Pd(PPh_3)_4$ (45 mg, 0.039 mmol), and potassium phosphate (207 mg, 0.88 mmol) were dissolved in 1,4-dioxane (4 mL) and water (1 mL), after replaced with nitrogen three times, heated up to 90 °C and reacted overnight. It was filtered through diatomaceous earth, concentrated under reduced pressure to remove the solvent, extracted three times with DCM/$H_2O$, the organic layers were combined, and washed with saturated sodium chloride once, dried over anhydrous sodium sulfate, concentrated under reduced pressure and purified by column chromatography (DCM/MEOH=30/1) to obtain compound **6f** (155 mg), light yellow solid.

MS (ESI) *m/z* 392 $[M + H]^+$.

$^1$H NMR (400 MHz, $CDCl_3$) δ 8.31 (s, 1H), 7.34 (d, *J*=8.4 Hz, 2H), 7.19 (d, *J*=8.4 Hz, *2H),* 6.93 (s, 1H), 5.61 (s, 2H), 3.85 (s, 3H), 3.56-3.46 (m, 2H), 3.45-3.35 (m, 2H), 2.65-2.45 (m, 4H).

Step 6 1- (4- (4-amino-6-bromo-7-methyl-7*H*-pyrrolo [2,3-*d*] pyrimidin-5-yl) phenyl) imino ) -4,4-difluorohexahydro-1 λ$^6$-thiopyran-1-oxide **6g**

Compound **6f** (150 mg, 0.38 mmol) was dissolved in anhydrous DMF (4 mL), NBS (75.1 mg, 0.42 mmol) was added at 0 °C to react for 1 h. Sodium thiosulfate solution was added to quench the reaction, and it was extracted with EA/$H_2O$ 4 times, the organic layers were combined, and washed with saturated salt water once, the organic phase was dried over anhydrous sodium sulfate, concentrated under reduced pressure, and purified by column chromatography (DCM/MEOH =30/1) to obtain compound **6g** (52 mg), yellow solid.

MS (ESI) *m/z* 470, 472 $[M + H]^+$.

$^1$H NMR (400 MHz, $CDCl_3$) δ 8.28 (s, 1H), 7.35 (d, *J*=8.4 Hz, 2H), 7.20 (d, *J*=8.4 *Hz,* 2H), 5.12 (s, 2H), 3.83 (s, 3H), 3.56-3.48 (m, 2H), 3.46-3.38 (m, 2H), 2.65-2.52 (m, 4H).

Step 7 *N*-(4-(4-amino-5-(4-(4,4-difluoro-1-oxotetrahydro-2*H*-1λ$^6$-thiopyran-1-imino) phenyl) -7-methyl-7*H*-pyrrolo [2,3-*d*] pyrimidin-6-yl) -3-fluorophenyl) methacrylamide 6

Compound **6g** (40 mg, 0.09 mmol), compound **1j** (39 mg, 0.13 mmol), $Pd(PPh_3)_4$ (19.7 mg, 0.02 mmol), and potassium phosphate (54.3 mg, 0.26 mmol) were dissolved in 1,4-dioxane (4 mL) and water (1 mL), after replaced with nitrogen three times, the reaction was refluxed at 100 °C for 12 h. After the reaction solution was cooled down to room temperature, it was filtered through diatomaceous earth, concentrated under reduced pressure to remove the solvent, and extracted with DCM/$H_2O$ three times, the organic layers were combined, washed with saturated salt water once, dried over anhydrous sodium sulfate, concentrated under reduced pressure, and purified by column chromatography (DCM/MEOH=40/1) to obtain compound **6** (18 mg), white solid.

MS (ESI) *m/z* 569 $[M + H]^+$.

$^1$H NMR (400 MHz, MeOD-d4) δ 8.37 (s, 1H), 7.76 (dd, *J* = 12.3, 2.0 Hz, 1H), *7.41 (dd, J* = 8.5, 2.1 Hz, 1H),

7.26-7.15 (m, 3H), 7.10 (d, *J* = 8.8 Hz, 2H), 5.82 (s, *1H),* 5.57 (q, *J* = 1.5 Hz, 1H), 3.73 (s, 3H), 3.61-3.41 (m, 4H), 2.61-2.40 (m, 4H), 2.02 (dd, *J* = 1.6, 1.0 Hz, 3H).

**Example 7 *N*- (4- (4-amino-5- (3-fluoro-4- ((1-oxotetrahydro-2*H*-1λ$^6$-thiopyran-1 -imino) phenyl) -7-methyl-7*H*-pyrrolo [2,3-*d*] pyrimidin-6-yl) -3-fluorophenyl) methacrylamide**

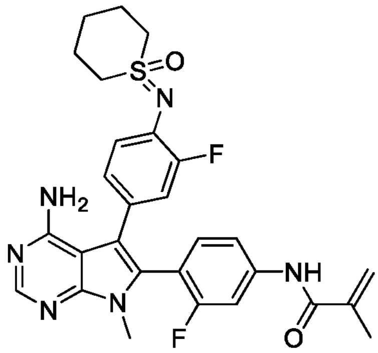

**7**

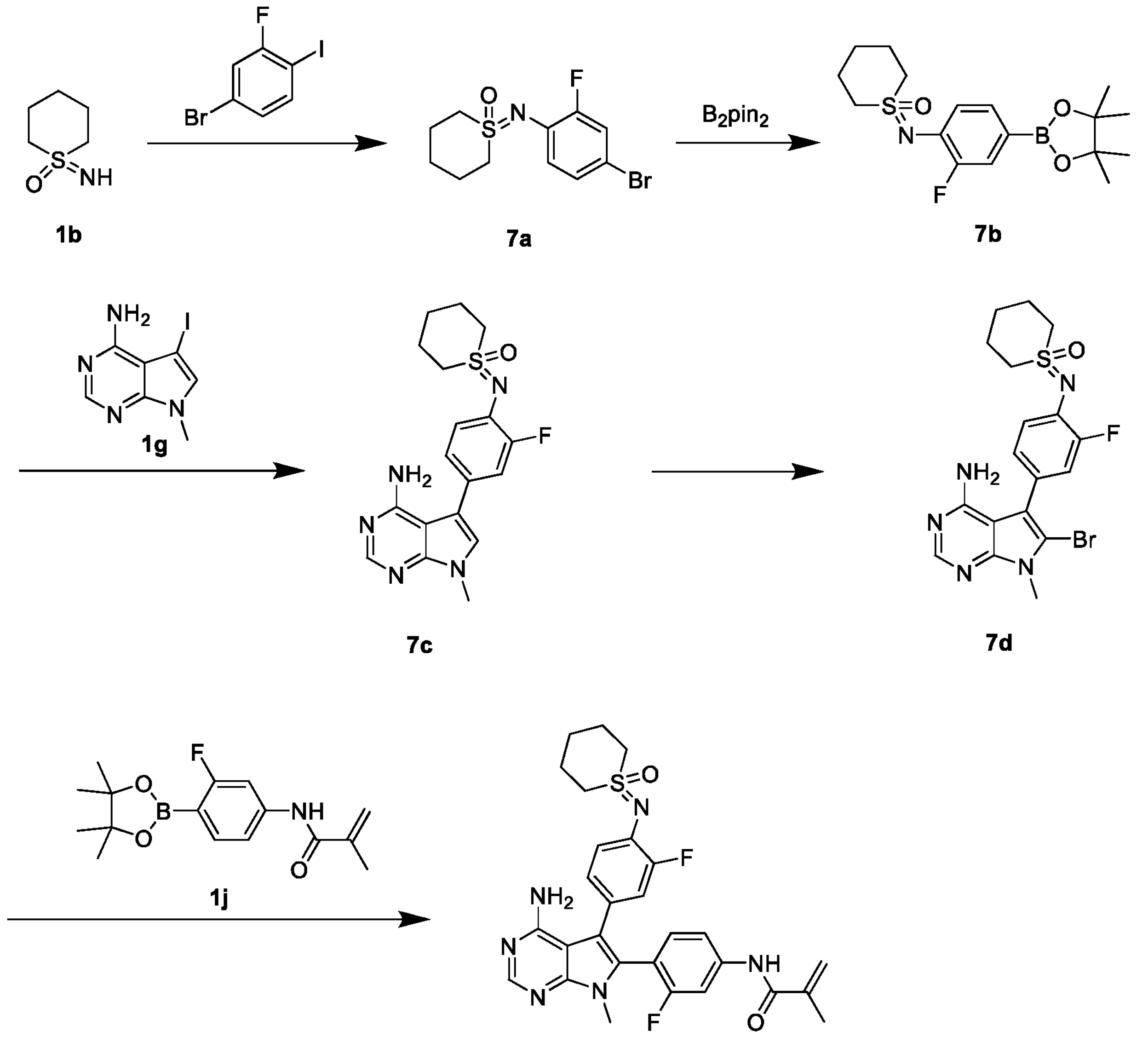


1b 7a 7b 1g 7c 7d 1j 7

**Step 1- ((4-bromo-2-fluorophenyl) imino) hexahydro-1 λ⁶-thiopyran-1-oxide 7a**

Compound 1b (104 mg, 0.78 mmol), 1-bromo-3-fluoro-4-iodobenzene (281.2 mg, 0.94 mmol), $Pd_2(dba)_3$ (17.9 mg, 0.02 mmol), Xantphos (33.8 mg, 0.06 mmol), and cesium carbonate (381.2 mg, 1.2 mmol) were added to a reaction flask sequentially, after replaced with nitrogen three times, anhydrous 1,4-dioxane (8 mL) was added, the temperature was raised up to 100 °C, and the reaction was refluxed for 6 h. It was filtrated through diatomaceous earth, concentrated under reduced pressure, and purified by column chromatography (PE/EA=5/1) to obtain compound **7a** (181 mg), light yellow oil.

MS (ESI) *m/z* 305 $[M + H]^+$.

**Step 2 1- ((2-Fluoro-4- (4,4,5,5-tetramethyl-1,3,2-dioxaborolan-2-yl) phenyl) imino) hexahydro-1 λ⁶-thiopyran-1-oxide 7b**

Compound **7a** (181 mg, 0.59 mmol), bis(pinacolato)diboron (181 mg, 0.71 mmol), potassium acetate (173.7 mg, 1.77 mmol), and $Pd(dppf)Cl_2$ (86.3 mg, 0.12 mmol) were added to the reaction flask in sequence, after replaced with nitrogen three times, anhydrous 1,4-dioxane (8 mL) was added to dissolve, and heated up to 90 °C to react overnight. After the reaction was completed, the reaction solution was cooled down to room temperature, filtered through diatomaceous earth, concentrated under reduced pressure, extracted with DCM/$H_2O$ three times, the organic layers were combined, washed with saturated salt once, the organic phase was dried over anhydrous sodium sulfate, concentrated and purified by column chromatography (PE/EA=4/1) to obtain compound **7b** (231 mg), yellow liquid.

MS (ESI) *m/z* 354 [M + H]$^+$.

$^1$H NMR (400 MHz, $CDCl_3$) $\delta$ 7.20 (dd, *J*=10.1, 2.1 Hz, 1H), 7.14 (t, *J*=8.3 Hz, 1H), 7.10 (dd, *J*=8.7, 2.1 Hz, 1H), 3.39-3.29 (m, 2H), 3.18-3.07 (m, 2H), 2.18-2.01 (m, 4H), 1.79-1.71 (m, 1H), 1.68-1.53 (m, 1H).

**Step 3 1- (4- (4-amino-7-methyl-7*H*-pyrrolo [2,3-*d*] pyrimidin-5-yl) -2-fluorophenyl) imino) hexahydro-1 λ$^6$-thiopyran-1-oxide 7c**

Compound **7b** (231 mg, 0.65 mmol), compound **1g** (149 mg, 0.55 mmol), $Pd(PPh_3)_4$ (62.8 mg, 0.06 mmol), and potassium phosphate (292 mg, 1.38 mmol) were dissolved in 1,4-dioxane (8 mL) and water (2 mL), after replaced with nitrogen three times, the reaction was carried out at 90 °C overnight. After the reaction was completed, it was cooled down to room temperature, filtered through diatomaceous earth, concentrated under reduced pressure to remove the solvent, extracted with DCM/$H_2O$ three times, the organic layers were combined, washed with saturated sodium chloride once, dried over anhydrous sodium sulfate, concentrated under reduced pressure and purified by column chromatography (DCM/MEOH=30/1) to obtain compound **7c** (132 mg), yellow solid.

MS (ESI) *m/z* 374 [M + H]$^+$.

$^1$H NMR (400 MHz, $CDCl_3$) $\delta$ 8.32 (s, 1H), 7.33 (t, J=8.4 Hz, 1H), 7.16 (dd, *J*=11.3, *2.1* Hz, 1H), 7.09 (dd, *J*=8.3, 1.9 Hz, 1H), 6.92 (s, 1H), 5.37 (s, 2H), 3.83 (s, 3H), 3.45-3.33 (m, 2H), 3.23-3.08 (m, 2H), 2.22-2.13 (m, 4H), 1.86-1.73 (m, 1H), 1.70-1.57 *(m,* 1H).

**Step 4 1- (4-(4-amino-6-bromo-7-methyl-7*H*-pyrrolo [2,3-*d*] pyrimidin-5-yl) -2-fluorophenyl) imino) hexahydro-1 λ$^6$-thiopyran-1-oxide 7d**

Compound **7c** (132 mg, 0.35 mmol) was dissolved in anhydrous DMF (4 mL), NBS (69.2 mg, 0.39 mmol) was added at 0 °C, and the reaction was quenched with sodium thiosulfate solution after 2 h of reaction, and it was extracted with EA/$H_2O$ 4 times, the organic layers were combined, washed with saturated salt water once, the organic phase was dried over anhydrous sodium sulfate, concentrated under reduced pressure, and purified by column chromatography (DCM/MEOH=30/1) to obtain compound **7d** (60 mg), yellow solid.

MS (ESI) *m/z* 452 [M + H]$^+$.

$^1$H NMR (400 MHz, $CDCl_3$) $\delta$ 8.28 (s, 1H), 7.36 (t, *J*=8.5 Hz, 1H), 7.17 (dd, *J*=11.3, 2.1 *Hz,* 1H), 7.07 (dd, *J*=8.2, 2.2 Hz, 1H), 5.30 (s, 2H), 3.84 (s, 3H), 3.47-3.34 (m, *2H),* 3.25-3.15 (m, 2H), 2.23-2.08 (m, 4H), 1.82-1.73 (m, 1H), 1.72-1.60 (m, 1H).

**Step 5 *N*-(4-(4-amino-5- (3-fluoro-4- ((1-oxotetrahydro-2*H*-1λ$^6$-thiopyran-1-imino) phenyl) -7-methyl-7*H*-pyrrolo [2,3-*d*] pyrimidin-6-yl)-3-fluorophenyl) methacrylamide 7**

Compound **7d** (60 mg, 0.13 mmol), compound **1j** (61 mg, 0.20 mmol), $Pd(PPh_3)_4$ (30 mg, 0.03 mmol), and potassium phosphate (82.8 mg, 0.39 mmol) were dissolved in 1,4-dioxane (4 mL) and water (1 mL), after replaced with nitrogen three times, the reaction was refluxed at 100 °C for 12 h. The reaction solution was cooled down to room temperature, filtered through diatomaceous earth, concentrated under reduced pressure to remove the solvent, extracted with DCM/$H_2O$ three times, and the organic layers were combined, washed with saturated saline solution, dried over anhydrous sodium sulfate, concentrated under reduced pressure, purified by column chromatography (DCM/MEOH =40/1) to obtain compound 7 (57 mg), light yellow solid.

MS (ESI) *m/z* 551 [M + H]$^+$.

$^1$H NMR (400 MHz, MeOD-$d_4$) $\delta$ 8.19 (s, 1H), 7.73 (dd, J=12.1, 2.1 Hz, 1H), 7.38 (dd, *J*=8.4, 2.1 Hz, 1H), 7.25-7.14 (m, 2H), 7.01-6.88 (m, 2H), 5.82 (s, 1H), 5.55 (q, *J*=1.6 Hz, 1H), 3.62 (s, 3H), 3.42-3.33 (m, 2H), 3.29-3.20 (m, 2H), 2.11-1.98 (m, 7H), 1.79-1.70 (m, 1H), 1.69-1.70 (m, 1H) 1.58 (m, 1H).

**Example 8 N-(4- (4-amino-7-methyl-5-(4-(1-oxo-1λ$^6$-thietan-1-ylidene) amino) phenyl) -7*H*-pyrrolo [2,3-*d*] pyrimidin-6-yl) -3-fluorophenyl) methacrylamide**

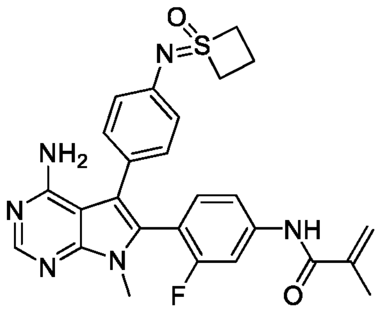

**8**

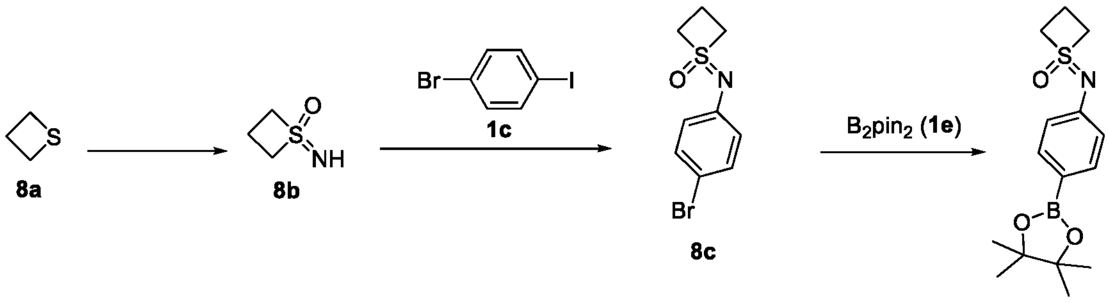


**8a** **8b** **8c** **8d**

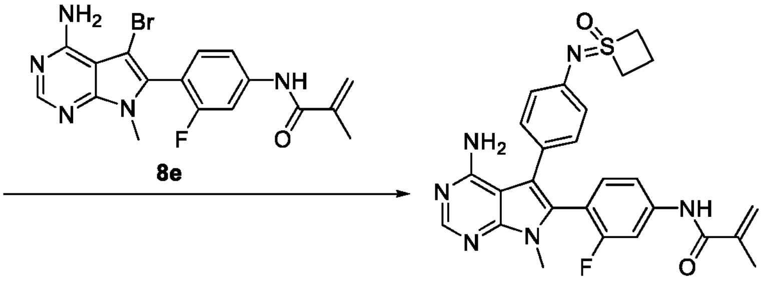

**8e**

**8**

**Step 1 1-Amino-1 λ$^6$-thietane-1-oxide 8b**

Thietane 8a (5 g, 67.5 mmol) and ammonium carbamate (7.85 g, 101 mmol) were added to a flask sequentially, and then MeOH (100 mL) and iodobenzene diethyl ester (43.5 g, 135 mmol) were added. The reaction solution was stirred open at room temperature for 30 min, and then concentrated under reduced pressure to remove the solvent to obtain a crude product. The crude product was purified by column chromatography (MeOH/DCM=1/20) to obtain compound **8b** (3.6 g), colorless oil.

$^1$H NMR (400 MHz, $CDCl_3$) $\delta$ 4.12-3.98 (m, 4H), 3.39 (brs, 1H), 2.24-2.14 (m, 2H).

**Step 2 1- ((4-bromophenyl) imino) -1 λ$^6$-thietane-1-oxide 8c**

Compound **8b** (210 mg, 2.0 mmol), cesium carbonate (0.99 g, 3.0 mmol), 1-bromo-4-iodobenzene **1c** (0.688 g, 2.4 mmol), $Pd_2(dba)_3$ (55 mg, 0.06 mmol), and Xantphos (104 mg, 0.18 mmol) were added to 1,4-dioxane (6 mL), and the reaction solution was heated up to 100 °C to react for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product was purified by column chromatography (PE/EtOAc=4/1 to 3/2) to obtain compound **8c** (470 mg), light yellow solid.

$^1$H NMR (400 MHz, $CDCl_3$) $\delta$ 7.33 (d, $J$ = 8.8 Hz, 2H), 6.90 (d, $J$ = 8.8 *Hz,* 2H), 4.18 (d, $J$= 8.0 *Hz,* 4H), 2.40-2.28 (m, 2H).

**Step 3 1- (4- (4,4,5,5-tetramethyl-1,3,2-dioxaborolan-2-yl) phenyl) imino) -1 λ$^6$-thietane-1-oxide 8d**

Compound **8c** (260 mg, 1.0 mmol), bis(pinacolato)diboron 1e (305 mg, 1.2 mmol), $Pd(dppf)Cl_2$ (146 mg, 0.2 mmol), potassium acetate (294 mg, 3.0 mmol), and 1,4-dioxane (5 mL) were added into a sealed tube, after replaced with

argon gas three times, sealed, and the reaction solution was heated up to 90 °C and stirred for 16 h. Then water (10 mL) was added, and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **8d** (256 mg), white solid.

$^1$H NMR (400 MHz, $CDCl_3$) $\delta$ 7.66 (d, *J* = 8.4 Hz, 2H), 6.95 (d, *J* = 8.4 *Hz,* 2H), 4.10 (t, *J = 8.0* Hz, 4H), 2.27-2.19 (m, 2H), 1.28 (s, 12H).

**Step 4 *N*-(4-(4-amino-7-methyl-5- (4- (1-oxo-1 λ$^6$-thiocyclobutan-1-ylidene) amino) phenyl) -7*H*-pyrrolo [2,3-*d*] pyrimidin-6-yl) -3-fluorophenyl) methacrylamide 8**

Compound **8e** (60 mg, 0.15 mmol), compound **8d** (55 mg, 0.18 mmol), $Pd(PPh_3)_4$ (17 mg, 0.015 mmol), and $K_3PO_4$ (95 mg, 0.45 mmol) were added into a sealed tube, after replaced with argon gas three times, 1,4-dioxane (2 mL) and $H_2O$ (0.2 mL) were added, sealed, the reaction solution was heated up to 100 °C and stirred for 16 h. then dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **8** (12 mg), white solid.

MS (ESI) *m*/*z* 505 $[M + H]^+$.

$^1$H NMR (400 MHz, $CDCl_3$) $\delta$ 8.36 (s, 1H), 7.70 (dd, *J* = 11.6, 1.9 Hz, 1H), 7.58 *(s,* 1H), 7.14-7.03 (m, 4H), 6.94 (d, *J* = 8.4 Hz, 2H), 5.80 (s, 1H), 5.51 *(d, J* = 1.6 Hz, 1H), 5.00 (s, 2H), 4.21 (dd, *J* = 9.4, 7.3 Hz, 4H), 3.65 (s, 3H), 2.40-2.32 (m, 2H), 2.07 (s, 3H).

**Example 9 N-(4-(4-amino-5- (4- (diethyl (oxo) 1 λ$^6$-sulfanylidene) -3- fluorophenyl) -7-methyl 7H-pyrrolo [2,3-d] pyrimidin-6-yl) - phenyl) methacrylamide**

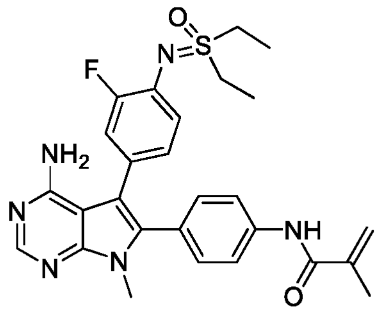

9

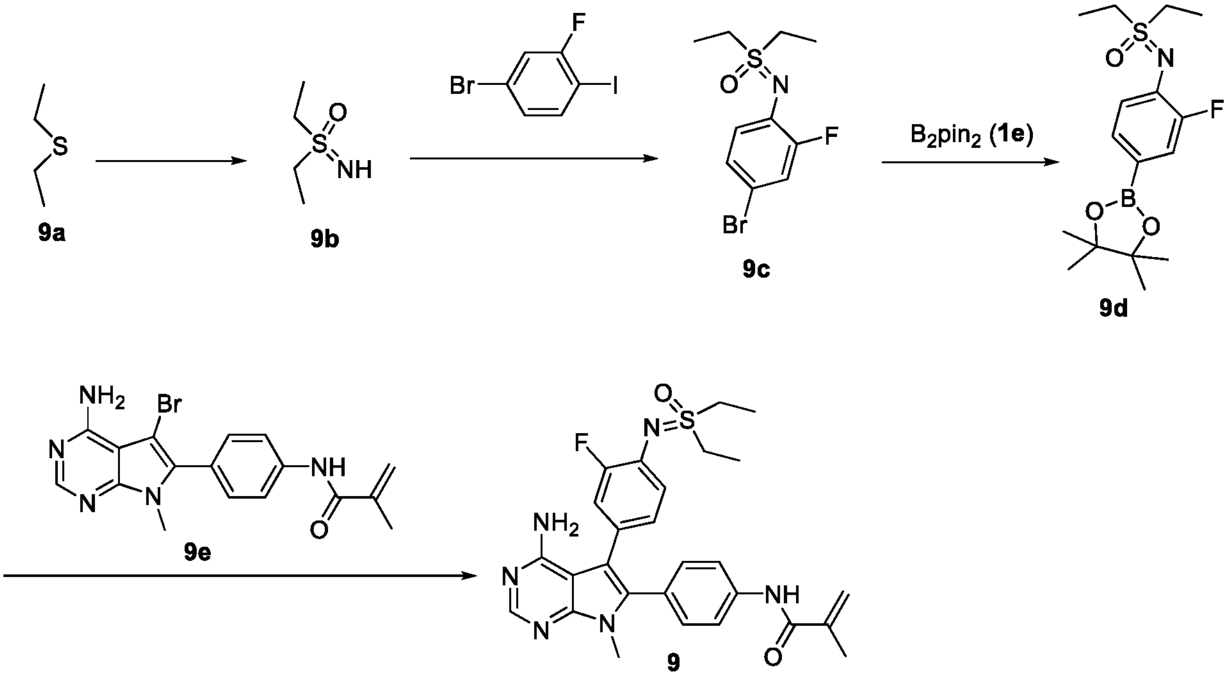


9a 9b 9c 9d 9e 9

**Step 1 imine-1 λ⁶ -diethylsulfoxide 9b**

Diethylthioether 9a (4.5 g, 50 mmol) and ammonium carbamate (5.85 g, 75 mmol) were added into a flask in sequence, then MeOH (100 mL) and iodobenzene diethyl ester (32.2 g, 100 mmol) were added. The reaction solution was stirred open at room temperature for 30 min and then concentrated under reduced pressure to remove the solvent to obtain a crude product. The crude product was purified by column chromatography (MeOH/DCM=1/20) to obtain compound **9b** (3.6 g), colorless oil.

$^1$H NMR (400 MHz, $CDCl_3$) $\delta$ 3.12-2.93 (q, *J* = 7.5 Hz, 4H), 2.66 (s, 1H), 1.38 (t, *J* = 7.5 Hz, 6H).

**Step 2 1- ((4-bromo-2-fluorophenyl) imino) -1 λ⁶**-diethylsulfoxide **9c**

Compound **9b** (400 mg, 3.3 mmol), cesium carbonate (1.6 g, 5 mmol), 1-bromo-3-fluoro-4-iodobenzene (1.2 g, 4 mmol), $Pd_2(dba)_3$ (92 mg, 0.1 mmol), and Xantphos (173 mg, 0.3 mmol) were added to 1,4-dioxane (12 mL), the reaction solution was heated up to 100 °C to react for 16 h. The reaction solution was filtered through diatomaceous earth, the filter cake was washed with ethyl acetate (100 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound **9c** (0.8 g), brownish yellow oil.

$^1$H NMR (400 MHz, $CDCl_3$) $\delta$ 7.18 ((dd, *J* = 10.0, 2.4 Hz, 1H), 7.15 (t, *J* = *8.4* Hz, 1H), 7.09 (dd, *J* = 8.4, 2.4 *Hz,* 1H), 3.27-3.12 (m, 4H), 1.41 (t, *J* = 7.4 Hz, 6H).

**Step 3 1- (4- (4,4,5,5-methyl-1,3,2-dioxaborolan-2-yl) -3-fluorophenyl) imino)-1 λ⁶-diethylsulfoxide 9d**

Compound **9c** (500 mg, 1.7 mmol), bis(pinacolato)diboron **1e** (518 mg, 2.0 mmol), $Pd(dppf)Cl_2$ (249 mg, 0.34

mmol), potassium acetate (500 mg, 5.1 mmol), and 1,4-dioxane (10 mL) were added into a sealed tube, after replaced with argon gas three times, sealed, the reaction solution was heated up to 90 °C and stirred for 16 h. Water (10 mL) was added, and it was extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound 9d (360 mg), white solid.

MS (ESI) *m*/*z* 342 $[M + H]^+$.

$^1$H NMR (400 MHz, $CDCl_3$) $\delta$ 7.45 (dd, *J* = 11.2, 1.6 Hz, 1H), 7.41 (dd, *J* = 8.0, 1.6 Hz, 1H), 7.27 (t, *J = 8.0* Hz, 1H), 3.31-3.16 (m, 4H), 1.41 (t, *J* = 7.4 Hz, 6H), 1.32 (s, 12H).

**Step 4 *N*- (4- (4-amino-5- (4- (diethyl (oxo) 1 $\lambda^6$-sulfanylidene) -3-fluorophenyl) -7-methyl 7*H*-pyrrolo [2,3-*d*] pyrimidin-6-yl) - phenyl) methacrylamide 9**

Compound **9e** (300 mg, 0.88 mmol), compound **9d** (283 mg, 0.73 mmol), $Pd(PPh_3)_4$ (84 mg, 0.072 mmol), and $K_3PO_4$ (464 mg, 2.2 mmol) were added into a sealed tube, after replaced with argon gas three times, 1,4-dioxane (10 mL) and $H_2O$ (1 mL) was added, sealed, the reaction solution was heated up to 100 °C and stirred for 16 h. Dichloromethane (20 mL) and saturated saline solution (10 mL) were added, and it was extracted, the organic phase was dried over anhydrous sodium sulfate, and concentrated under reduced pressure to obtain a crude product. The crude product was purified by column chromatography (MeOH/DCM=1/10) to obtain compound **9** (52 mg), white solid.

MS (ESI) *m*/*z* 521 $[M + H]^+$.

$^1$H NMR (400 MHz, $CDCl_3$) $\delta$ 8.26 (s, 1H), 7.56-7.42 (m, 3H), 7.20-7.12 (m, 3H), 6.82 *(d, J* = 10.5 Hz, 2H), 5.74 (s, 1H), 5.43 (s, 1H), 5.27 (brs, 2H), 3.63 (s, 3H), 3.20-3.09 (m, 4H), 2.01 (s, 3H), 1.37 (t, *J* = 7.4 Hz, 6H).

**Example 10 *N*-(4-(4-amino-5-(4-(3,3-dimethoxy-1-oxo-1$\lambda^6$-thiocyclobutan-1- ylidene) amino)-3-fluorophenyl) -7-methyl-7*H*-pyrrolo [2,3-*d*] pyrimidin-6-yl)- phenyl) methacrylamide**

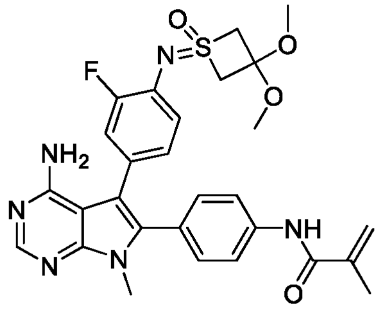

**10**

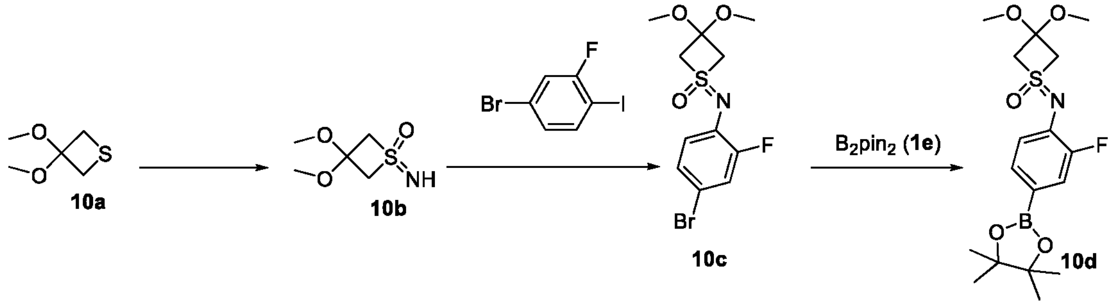


10a 10b 10c 10d

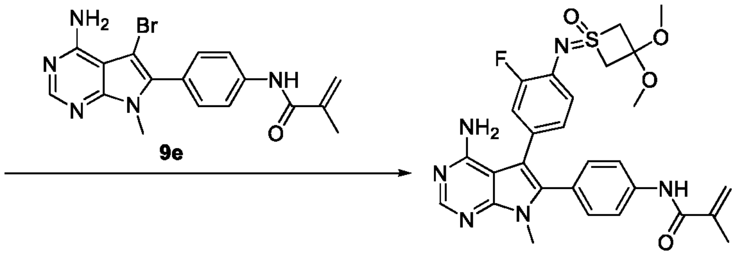

9e

**10**

**Step 1 1-imino-3,3-dimethoxy-1λ⁶-thietane-1-oxide 10b**

3,3-dimethoxythietane **10a** (2.68 g, 20 mmol) and ammonium carbamate (2.34 g, 30 mmol) were added to a flask sequentially, and then MeOH (80 mL) and iodobenzene diethyl ester (12.9 g, 40 mmol) were added. The reaction solution was stirred open at room temperature for 30 min and then concentrated under reduced pressure to remove the solvent to obtain a crude product. The crude product was purified by column chromatography (MeOH/DCM=1/20) to obtain compound 10b (0.9 g), colorless oil.

$^1$H NMR (400 MHz, $CDCl_3$) $\delta$ 4.15 (s, 4H), 3.25 (s, 6H).

**Step 2 1-((4-bromo-2-fluorophenyl) imino)-3,3-dimethoxy-1λ⁶-thietan-1- oxide 10c**

Compound 10b (380 mg, 2.3 mmol), cesium carbonate (1.13 g, 3.45 mmol), 1-bromo-3-fluoro-4-iodobenzene (832 mg, 2.76 mmol), $Pd_2(dba)_3$ (105 mg, 0.12 mmol), and Xantphos (200 mg, 0.36 mmol) were added to 1,4-dioxane (10 mL), and the reaction solution was heated up to 100 °C to react for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product was purified by column chromatography (PE/EtOAc=4/1 to 3/2) to obtain compound 10c (0.4 g), light yellow solid.

$^1$H NMR (400 MHz, $CDCl_3$) $\delta$ 7.20 (dd, $J$ = 10.4, 2.4 Hz, 1H), 7.14 (dd, $J$ = 8.6, 2.4 Hz, 1H), 6.95 (t, $J$ = 8.6 Hz, 1H), 4.35 (d, $J$ = 13.2 Hz, 1H), 4.27 (d, $J$ = 13.2 Hz, 1H), 3.28 (s, 3H), 3.26 (s, 3H).

**Step 3 1-((2-fluoro-4- (4,4,5,5-tetramethyl-1,3,2-dioxaborolan-2-yl) phenyl) imino) -3,3-dimethoxy-1 λ⁶-thietane-1-oxide 10d**

Compound **1d** (600 mg, 1.77 mmol), bis(pinacolato)diboron (541 mg, 2.13 mmol), $Pd(dppf)Cl_2$ (259 mg, 0.35

mmol), potassium acetate (520 mg, 5.31 mmol), and 1,4-dioxane (10 mL) were added into a sealed tube, it was replaced with argon gas three times, sealed, the reaction solution was heated up to 90 °C and stirred for 16 h. Water (10 mL) was added and it was extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **10d** (580 mg), brownish yellow solid.

$^1$H NMR (400 MHz, $CDCl_3$) $\delta$ 7.50-7.45 (m, 1H), 7.45 (s, 1H), 7.05 (t, *J* = 7.9 Hz, *1H),* 4.41-4.25 (m, 4H), 3.28 (s, 3H), 3.25 (s, 3H), 1.32 (s, 12H).

**Step 4 *N-* (4- (4-amino-5- (4- (3,3-dimethoxy-1-oxo-1 λ$^6$-thiocyclobutan-1-ylidene) amino) -3-fluorophenyl) -7-methyl-7*H*-pyrrolo [2,3-*d*] pyrimidin-6-yl) - phenyl) methacrylamide 10**

Compound **9e** (185 mg, 0.48 mmol), compound **10d** (154 mg, 0.4 mmol), $Pd(PPh_3)_4$ (46 mg, 0.04 mmol), and $K_3PO_4$ (254 mg, 1.2 mmol) were added into a sealed tube, after replaced with argon gas three times, 1,4-dioxane (5 mL) and $H_2O$ (0.5 mL) were added, sealed, the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **10** (19 mg), white solid.

MS (ESI) *m*/*z* 565 $[M + H]^+$.

$^1$H NMR (400 MHz, $CDCl_3$) $\delta$ 8.34 (s, 1H), 7.65 (s, 1H), 7.57 (d, *J* = 8.6 Hz, *2H),* 7.20 (d, *J* = 8.6 Hz, 2H), 7.00 (dd, *J* = 9.2, 7.6 Hz, 1H), 6.95-6.88 (m, 2H), 5.79 (s, 1H), 5.47 (d, *J* = 1.6 Hz, 1H), 5.07 (brs, 2H), 4.37 (d, *J* = 13.6 Hz, 2H), 4.29 (d, *J* = 13.6 Hz, 2H), 3.68 (s, 3H), 3.28 (s, 3H), 3.26 (s, 3H), 2.06 (s, 3H).

**Example 11 *N*-(4- (4-amino-5- (3-fluoro-4- (6-oxo-2-oxa-6 λ$^6$-thiaspiro [3.3] heptan-6-ylidene) amino) phenyl) -7-methyl-7*H*-pyrrolo [2,3-*d*] pyrimidin-6-yl) - phenyl) methacrylamide**

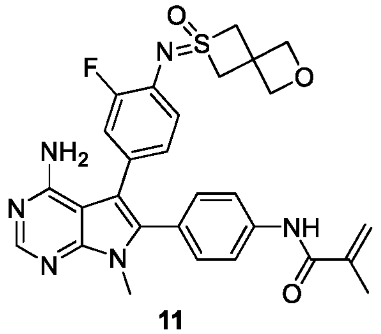

11

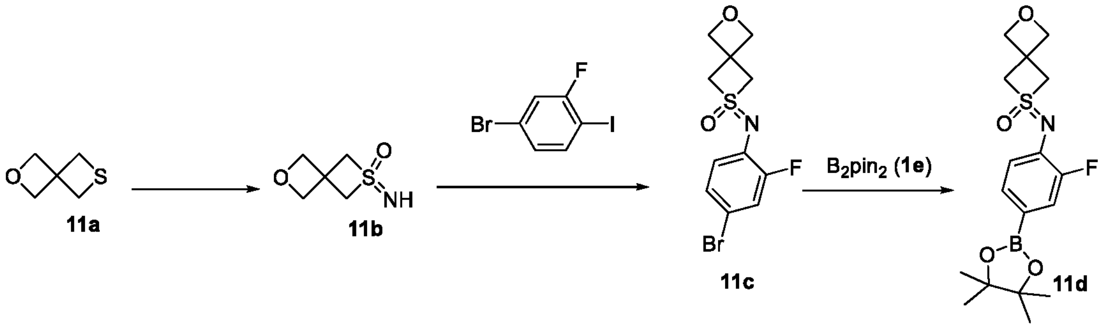


11a 11b 11c 11d

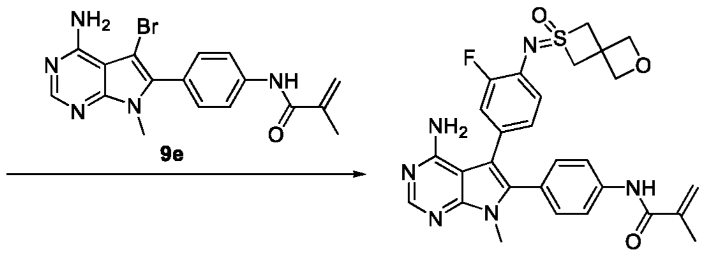

9e

11

**Step 1 6-amino-2-oxo-6 $\lambda^6$-thiaspiro [3.3] heptane-6-oxide 11b**

Compound **11a** (1.74 g, 15 mmol) and ammonium carbamate (1.75 g, 22.5 mmol) were added to a flask sequentially, and then MeOH (100 mL) and iodobenzene diethyl ester (9.66 g, 30 mmol) were added. The reaction solution was stirred open at room temperature for 30 min and then concentrated under reduced pressure to remove the solvent to obtain a crude product. The crude product was purified by column chromatography (MeOH/DCM=1/20) to obtain compound **11b** (0.7 g), colorless oil.

$^1$H NMR (400 MHz, $CDCl_3$) $\delta$ 4.85 (s, 2H), 4.81 (s, 2H), 4.28 (s, 4H), 2.04 (s, 1H).

**Step 2 6- ((4-bromo-2-fluorophenyl) imino) -2-oxo-6 $\lambda^6$-thiaspiro [3.3] heptane-6-oxide 11c**

Compound **11b** (480 mg, 3.3 mmol), cesium carbonate (1.59 g, 4.9 mmol), 1-bromo-3-fluoro-4-iodobenzene (1.2 g, 3.9 mmol), $Pd_2(dba)_3$ (92 mg, 0.1 mmol), and Xantphos (174 mg, 0.3 mmol) were added to 1,4-dioxane (15 mL), and the reaction solution was heated up to 100 °C for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product was purified by column chromatography (PE/EtOAc=4/1 to 3/2) to obtain compound **11c** (0.6 g), brownish yellow solid.

$^1$H NMR (400 MHz, $CDCl_3$) $\delta$ 7.20 (dd, *J* = 10.4, 2.4 Hz, 1H), 7.13 (ddd, *J* = 8.4, 2.4, 1.0 Hz, 1H), 6.93 (t, *J* = 8.8 Hz, 1H), 4.84 (s, 2H), 4.82 (s, 2H), 4.45 (d, *J* = 14.0 Hz, 2H), 4.39 (d, *J* = *14.0* Hz, 2H).

**Step 3 6- ((2-fluoro-4- (4,4,5,5-tetramethyl-1,3,2-dioxaborolan-2-yl) phenyl) imino) -2-oxo-6 $\lambda^6$-thiaspiro [3.3] heptane-6-oxide 11d**

Compound **11c** (260 mg, 0.81 mmol), bis(pinacolato)diboron (248 mg, 0.98 mmol), $Pd(dppf)Cl_2$ (119 mg, 0.16 mmol), potassium acetate (238 mg, 2.43 mmol), and 1,4-dioxane (5 mL) were added into a sealed tube, after replaced with

argon gas three times, sealed, the reaction solution was heated up to 90 °C and stirred for 16 h. Water (10 mL) was added and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **11d** (230 mg), brownish yellow solid.

$^1$H NMR (400 MHz, $CDCl_3$) $\delta$ 7.47 (dd, *J* = 5.2, 1.2 Hz, 1H), 7.45 (s, 1H), 7.04 *(t, J* = 8.0 Hz, 1H), 4.86 *(s,* 2H), 4.85 (s, 2H), 4.50 (d, *J* = 14.0 Hz, 2H), 4.42 (d, *J* = 14.0 Hz, 2H), 1.32 (s, 12H).

**Step 4 *N-* (4- (4-amino-5- (3-fluoro-4- (6-oxo-2-oxa-6 $\lambda^6$-thiaspiro [3.3] heptan-6- ylidene) amino) phenyl) -7-methyl-7*H*-pyrrolo [2,3-*d*] pyrimidin-6-yl) - phenyl) methacrylamide 11**

Compound **8e** (150 mg, 0.4 mmol), compound **11d** (143 mg, 0.37 mmol), $Pd(PPh_3)_4$ (43 mg, 0.037 mmol), and $K_3PO_4$ (235 mg, 1.1 mmol) were added into a sealed tube, after replaced with argon gas three times, 1,4-dioxane (4 mL) and $H_2O$ (0.4 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound 11 (15 mg), white solid.

MS (ESI) *m/z* 547 $[M + H]^+$.

$^1$H NMR (400 MHz, $CDCl_3$) $\delta$ 8.29 (s, 1H), 7.58-7.43 (m, 3H), 7.15 (d, *J* = 8.6 Hz, *2H),* 6.98-6.80 (m, 3H), 5.73 (s, 1H), 5.42 (d, *J* = 1.6 Hz, 1H), 4.92 (s, 2H), 4.82 (s, 2H), 4.79 (s, 2H), 4.45 (d, *J* = 14.1 Hz, 2H), 4.37 (d, *J* = 14.1 Hz, 2H), 3.62 (s, 3H), 2.00 (s, 3H).

**Example 12 *N*-(3- (4-amino-5- (3-fluoro-4- ((1-oxotetrahydro-2*H*-1 $\lambda^6$-thiopyran-1-ylidene) amino) phenyl) -7-methyl-7*H*-pyrrolo [2,3-*d*] pyrimidin-6-yl) -4-fluorophenyl) methacrylamide**

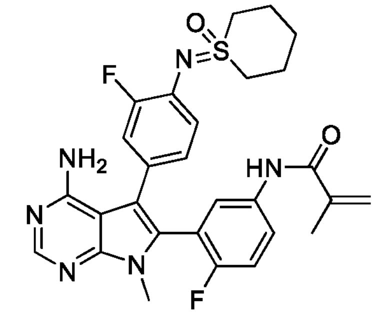

**12**

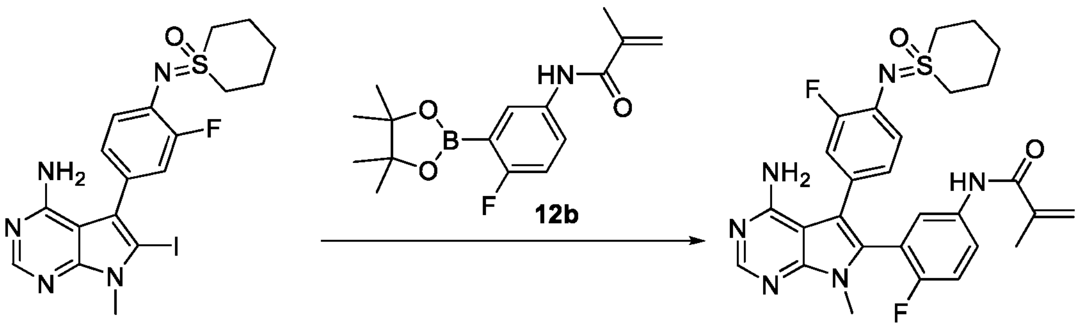

**12a** **12b** **12**

Compound **12a** (150 mg, 0.3 mmol), compound **12b** (105 mg, 0.36 mmol), $Pd(PPh_3)_4$ (35 mg, 0.03 mmol), and $K_3PO_4$ (192 mg, 0.9 mmol) were added into a sealed tube, after replaced with argon gas three times, 1,4-dioxane (3 mL) and $H_2O$ (0.3 mL) was added, sealed, and the reaction solution was heated up to 90 °C and stirred for 16 h. Dichloromethane (40 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **12** (10 mg), white solid.

MS (ESI) *m/z* 551 $[M + H]^+$.

$^1$H NMR (400 MHz, $CDCl_3$) $\delta$ 8.36 (s, 1H), 7.65 (ddd, $J$ = 8.8, 4.4, 2.8 Hz, 1H), 7.45 (s, 1H), 7.30 (dd, $J$ = 6.0, 2.8 Hz, 1H), 7.19 (t, $J$ = 8.8 Hz, 1H), 7.12 (t, $J$ = 8.8 Hz, 1H), 7.00-6.88 (m, 2H), 5.76 (s, 1H), 5.46 (d, $J$ = 1.6 Hz, 1H), 5.11 (brs, 2H), 3.65 (s, 3H), 3.36-3.28 (m, 2H), 3.17-3.10 (m, 2H), *2.18-2.10* (m, 4H), 2.03 (s, 3H), 1.66-1.58 (m, 2H).

**Example 13 *N*- (4- (4-amino-5- (3-fluoro-4- ((1-oxotetrahydro-2*H*-1 $\lambda^6$-thiopyran-1-ylidene) amino) phenyl) -7-methyl-7*H*-pyrrolo [2,3-*d*] pyrimidin-6-yl) phenyl) acrylamide**

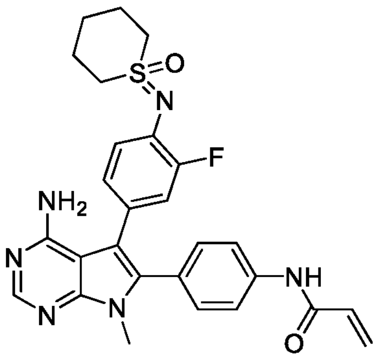

**13**

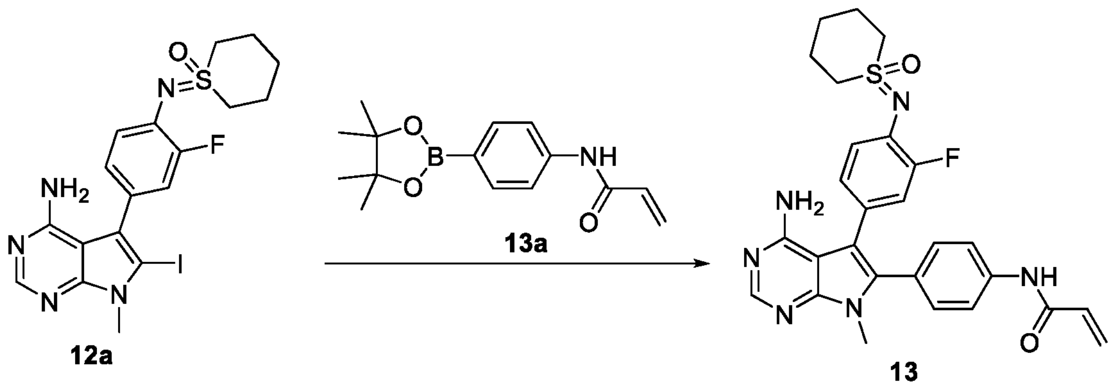

**12a**

**13a**

**13**

Compound **12a** (150 mg, 0.3 mmol), compound **13a** (107 mg, 0.39 mmol), $Pd(PPh_3)_4$ (69 mg, 0.06 mmol), and $K_3PO_4$ (192 mg, 0.9 mmol) were added into a sealed tube, after replaced with argon gas three times, 1,4-dioxane (6 mL) and $H_2O$ (0.6 mL) were added, sealed, and the reaction solution was heated up to 90 °C and stirred for 16 h. Dichloromethane (40 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **13** (13 mg), white solid.

MS (ESI) *m/z* 519 [M + H]$^+$.

$^1$H NMR (400 MHz, $CDCl_3$) $\delta$ 8.28 (s, 1H), 7.76 (s, 1H), 7.59 (d, $J$ = 8.4 *Hz,* 2H), 7.19 (d, $J$ = 8.4 Hz, 2H), 7.17 (m, 1H), 6.90-6.84 (m, 2H), 6.44 (dd, $J$ = 16.8, 1.2 Hz, 1H), 6.28 (dd, $J$ = 16.8, 10.0 Hz, 1H), 5.77 (dd, $J$ = 10.0, 1.2 Hz, 1H), 5.60 *(brs,* 2H), 3.68 (s, 3H), 3.35-3.29 (m, 2H), 3.18-3.11 (m, 2H), 2.13-2.06 (m, 4H), 1.79-1.70 (m, 1H), 1.66-1.58 (m, 1H).

**Example 14 *N*- (4- (4-amino-5- (3-fluoro-4- ((1-oxotetrahydro-2*H*-1 $\lambda^6$-thiopyran-1-ylidene)amino)phenyl)-7-methyl-7*H*-pyrrolo[2,3-*d*]pyrimidin-6-yl)-3-methoxyphenyl) methacrylamide**

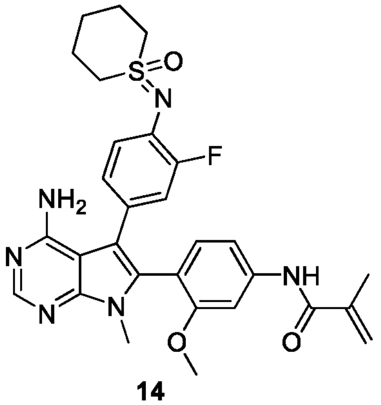

**14**

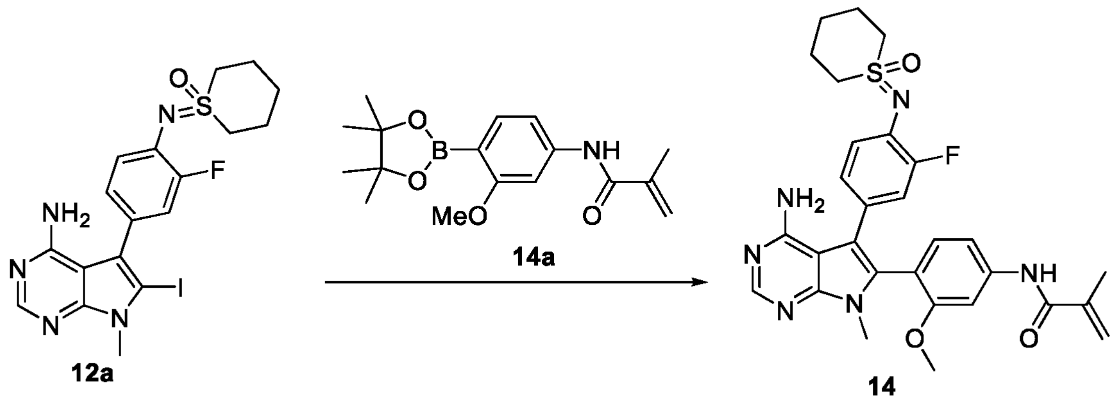

**12a** **14a** **14**

Compound **12a** (50 mg, 0.1 mmol), compound **14a** (38 mg, 0.12 mmol), $Pd(PPh_3)_4$ (23 mg, 0.02 mmol), and $K_3PO_4$ (64 mg, 0.3 mmol) were added into a sealed tube, after replaced with argon gas three times, 1,4-dioxane (2 mL) and $H_2O$ (0.2 mL) were added, sealed, and the reaction solution was heated up to 90 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **14** (10 mg), white solid.

MS (ESI) *m/z* 563 $[M + H]^+$.

$^1H$ NMR (400 MHz, $CDCl_3$) $\delta$ 8.27 (s, 1H), 7.71 (d, *J* = 2.0 Hz, 1H), 7.62 *(s,* 1H), 7.16 (t, *J* = 8.0 Hz, 1H), 6.93 (d, *J* = 8.0 Hz, 2H), *6.91-6.85* (m, 1H), 6.76 (dd, *J* = 8.0, 2.0 Hz, 1H), 5.81 (s, 1H), 5.81 (brs, 2H), 5.50 (s, 1H), 3.79 (s, 3H), 3.58 *(s,* 3H), 3.30 (m, 2H), 3.14 (m, 2H), 2.14-2.06 (m, 4H), 2.07 (s, 3H), 1.78-1.69 (m, *1H),* 1.66-1.56 (m, 1H).

**Example 15 (*E*)-*N*-(4-(4-amino-5-(3-fluoro-4-((1-oxotetrahydro-2*H*-1$\lambda^6$-*thiopyran* -1-ylidene) amino) phenyl)-7-methyl-7*H*-pyrrolo [2,3-*d*] pyrimidin -6-yl) -phenyl)-2-butenamide**

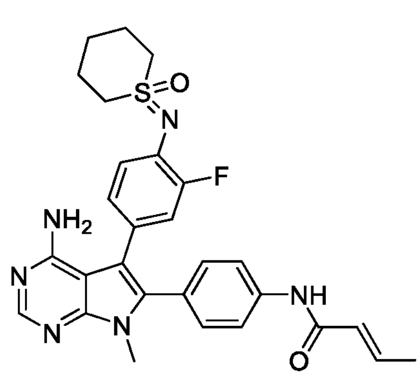

**15**

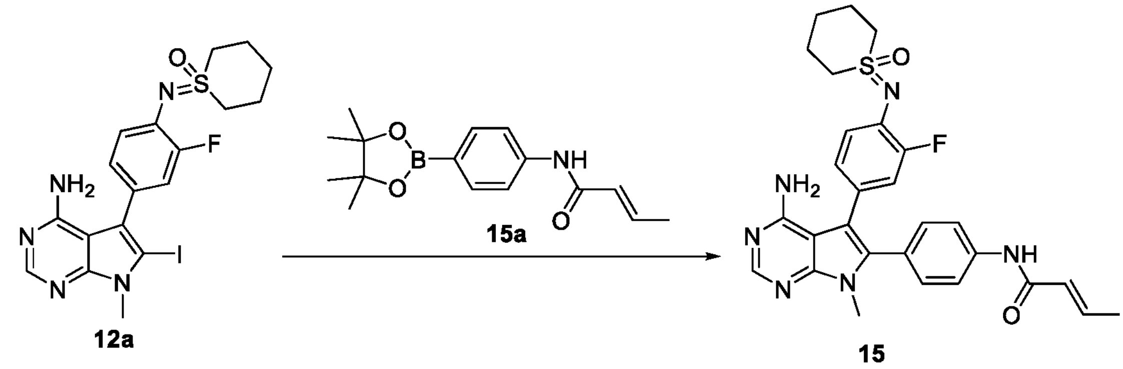


12a 15

Compound **12a** (150 mg, 0.3 mmol), compound **15a** (103 mg, 0.36 mmol), $Pd(PPh_3)_4$ (35 mg, 0.03 mmol), and $K_3PO_4$ (192 mg, 0.9 mmol) were added into a sealed tube, after replaced with argon gas three times, 1,4-dioxane (5 mL) and $H_2O$ (0.5 mL) were added, sealed, and the reaction solution was heated up to 90 °C and stirred for 16 h. Dichloromethane (40 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **15** (7 mg), white solid.

MS (ESI) *m/z* 533 $[M + H]^+$.

$^1H$ NMR (400 MHz, $CDCl_3$) $\delta$ 8.36 (s, 1H), 7.56 (d, *J* = 8.5 Hz, 2H), 7.21-7.16 (m, *4H),* 7.05-6.96 (m, 1H), 6.92-6.87 (m, 2H), 5.96 (dd, *J* = 15.1, 1.8 Hz, 1H), 4.98 (s, 2H), 3.69 (s, 3H), 3.38-3.28 (m, 2H), 3.20-3.11 (m, 2H), 2.15-2.06 (m, 4H), 1.93 (dd, *J* = 6.9, 1.7 Hz, 3H), 1.79-1.71 (m, 1H), 1.69-1.61 (m, 1H).

**Example 16 (*E*)-*N*-(4-(4-amino-5-(3-fluoro-4-((1-oxotetrahydro-2*H*-1 $\lambda^6$-thiopyran -1-ylidene) amino) phenyl) -7-methyl-7H-pyrrolo [2,3-*d*] pyrimidin-6-yl) - phenyl) -4-(dimethylamino) -2-butenamide**

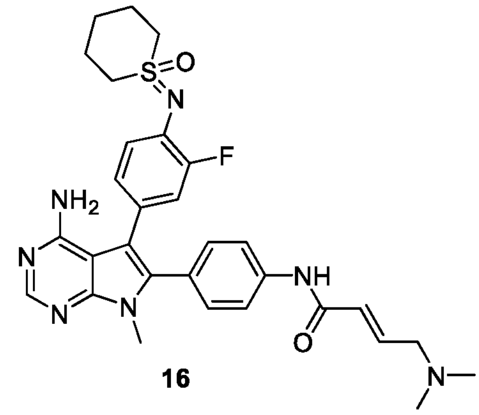

16

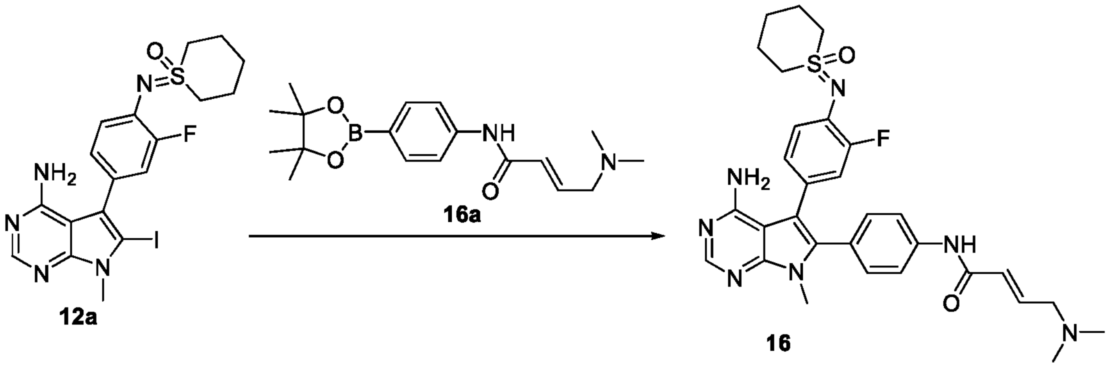


12a 16

Compound **12a** (100 mg, 0.2 mmol), compound **16a** (77 mg, 0.24 mmol), $Pd(PPh_3)_4$ (23 mg, 0.02 mmol), and $K_3PO_4$ (127 mg, 0.6 mmol) were added into a sealed tube, after replaced with argon gas three times, 1,4-dioxane (3 mL)

and $H_2O$ (0.3 mL) were added, sealed, and the reaction solution was heated up to 90 °C and stirred for 16 h. Dichloromethane (40 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **16** (16 mg), white solid.

MS (ESI) *m/z* 574 [M - *H]-.*

$^1H$ NMR (400 MHz, $CDCl_3$) $\delta$ 8.44 (s, 1H), 7.68-7.60 (m, 3H), 7.36-7.28 (m, 3H), 7.11-7.04 (m, *1H),* 6.99 (d, *J* = 9.5 Hz, 2H), 6.23 (d, *J* = 15.6 Hz, 1H), 5.15 (s, 2H), 3.78 (s, 3H), 3.45-3.40 (m, 2H), 3.26-3.20 (m, 4H), 2.37 (s, 6H), 2.28-2.16 (m, 4H), 1.86-1.79 (m, 1H), 1.78-1.66 (m, 1H).

**Example 17 *N*-[4- (4-amino-7-methyl-5- (6- ((1-oxotetrahydro-2*H*-1 $\lambda^6$-thiopyran-1-ylidene) amino) pyridin-3-yl) pyrrolo [2,3-*d*] pyrimidin-6-yl) phenyl) methacrylamide**

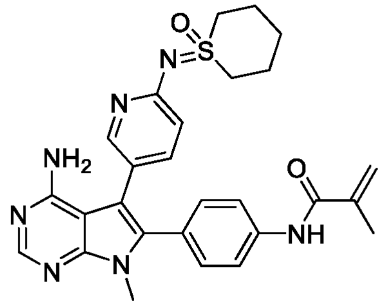

**17**

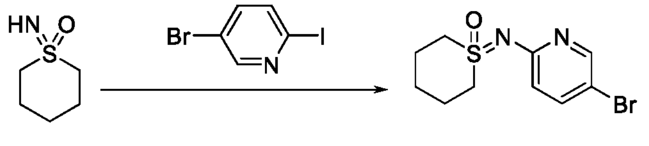

**1b** **17a**

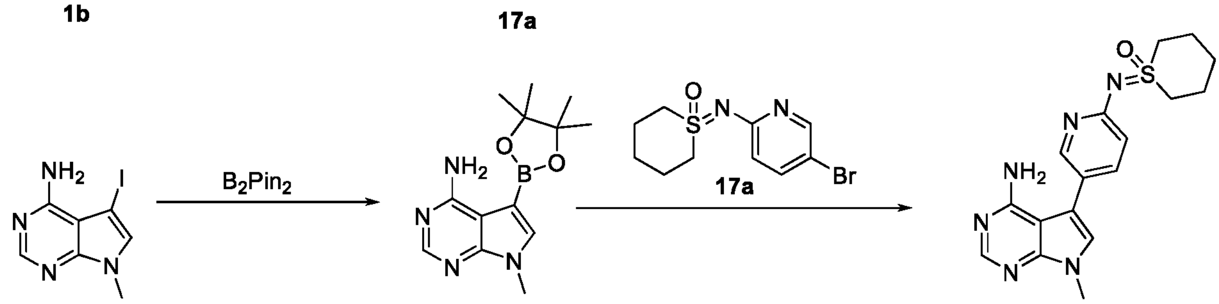


**1g** **17b** **17c**

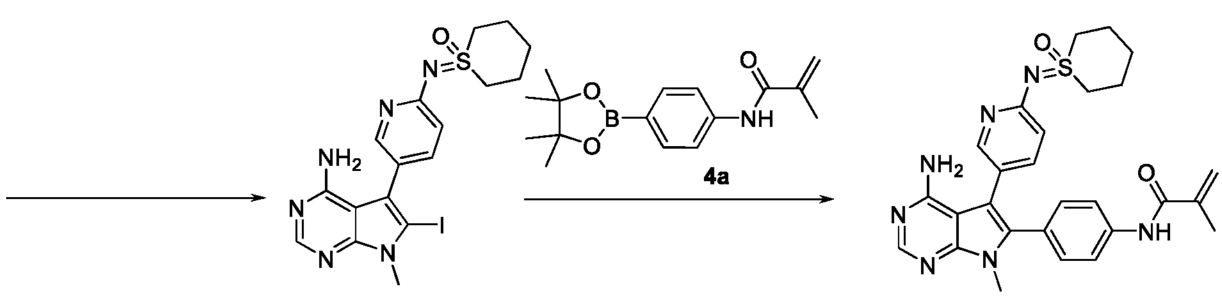


**17d** **17**

**Step 1 1- [(5-bromopyridin-2-yl) imino] -1 $\lambda^6$-thiopyran-1-oxide 17a**

At room temperature, compound **1b** (1 g, 7.52 mmol), 5-bromo-2-iodopyridine (2.35 g, 8.27 mmol), tris

(dibenzylideneacetone) dipalladium (173.99 mg, 0.19 mmol), 4,5-diphenylphosphine-9,9-dimethyloxanthracene (324.03 mg, 0.56 mmol), cesium carbonate (3.43 g, 10.53 mmol), 1,4-dioxane (20 ml), and a stirrer were added to a round bottom flask sequentially, followed by vacuum pumping and nitrogen gas purging three times. The reaction was heated up to 100 °C and stirred for 16 h. After the reaction was completed, the mixture was cooled down to room temperature and concentrated under reduced pressure to obtain a crude product. The crude product was purified by column chromatography (PE/EtOAc=1/1) to obtain compound **17a** (700 mg), light yellow solid.

MS (ESI) *m/z* 289 $[M + H]^+$.

$^1$H NMR (400 MHz, DMSO-$d_6$) $\delta$ 8.16 (d, *J* = 4.0 Hz, 1H), 7.72 (dd, *J = 4.0, 4.0* Hz, *1H),* 6.67 (d, *J* = 8.0 Hz, *1H),* 3.63-3.62 (m, 2H), 3.43-3.42 (m, 2H), 2.02-1.81 (m, 4H), 1.62-1.59 (m, 2H).

**Step 2 7-methyl-5-(4,4,5,5-tetramethyl-1,3,2-dioxaborolan-2-yl) pyrrolo [2,3-*d*] pyrimidin-4-amine**

At room temperature, compound **1g** (5 g, 18.25 mmol), bis(pinacolato)diboron (6.95 g, 27.37 mmol), potassium acetate (5.34 g, 54.75 mmol), of 1,1'- biphenylphosphine ferrocene palladium chloride (1.34 mg, 1.83 mmol), and dimethyl sulfoxide (25 mL) were added to a round bottom flask sequentially, followed by vacuum pumping and nitrogen gas purging three times. The reaction was heated up to 100 °C and stirred for 1 h. After the reaction was completed, the mixture was cooled down to room temperature, diluted with water, extracted with ethyl acetate (20 mL × 3), washed with saturated saline (30 mL), and the organic phases were combined, dried over anhydrous sodium sulfate and concentrated under reduced pressure to obtain a crude product. The crude product was purified by column chromatography (DCM/MeOH=15/1) to obtain compound **7b** (700 mg), reddish brown solid.

MS (ESI) *m/z* 275 $[M + H]^+$.

**Step 3 1- ((5- (4-amino-7-methyl-7*H*-pyrrolo [2,3-*d*] pyrimidin-5-yl) pyridin-2-yl) imino) hexahydro-1 $\lambda^6$-thiopyran-1-oxide 17c**

At room temperature, compound **7b** (500 mg, 1.82 mmol), compound **17a** (471.74 mg, 1.64 mmol), 1,1'- bis (ditert-butylphosphine) ferrocene palladium dichloro (117.68 mg, 0.18 mmol), cesium fluoride (829.37 mg, 5.46 mmol), N, N-dimethylformamide (10 mL), and water (2.5 mL) were added to a round bottom flask in sequence, followed by vacuum pumping and nitrogen gas purging three times. The reaction was heated up to 100 °C and stirred for 1 h. After the reaction was completed, the mixture was cooled down to room temperature, diluted with water, extracted with ethyl acetate (15 ml × 3), washed with saturated saline (20 ml), and the organic phases were combined, dried over anhydrous sodium sulfate and concentrated under reduced pressure to obtain a crude product. The crude product was purified by column chromatography (DCM/MeOH=10/1) to obtain compound **17c** (400 mg), brown solid.

MS (ESI) *m/z* 357 $[M + H]^+$.

$^1$H NMR (400 MHz, DMSO-$d_6$) $\delta$ 8.17-8.16 (m, 2H), 7.61 (dd, *J* = 4.0, 4.0 Hz, *1H), 7.28* (s, *1H),* 6.79 (d, *J* = 8.0 Hz, 1H), 6.07 (s, 2H), 3.74 (s, 3H), 3.69-3.64 (m, 2H), 3.47-3.41 (m, 2H), 1.96-1.94 (m, *4H),* 1.64-1.61 (m, 2H).

**Step 4 1-(5-(4-amino-6-iodo-7-methyl-7*H*-pyrrolo [2,3-*d*] pyrimidin-5-yl) pyridine -2-yl) imino) hexahydro-1 $\lambda^6$-thiopyran-1-oxide 17d**

Compound **17c** (300 mg, 0.84 mmol), dichloromethane (3 ml), and trifluoroacetic acid (287.33 mg, 2.52 mmol) were added to a round bottom flask at room temperature. After it was cooled down to 0 °C, N-iodosuccinimide (283.47 mg, 1.26 mmol) was added in batches, and the reaction was warmed up to room temperature naturally and stirred for additional 2 h. After TLC monitored the completion of the reaction, the mixture was concentrated under reduced pressure to obtain a crude product. The crude product was purified by column chromatography (DCM/MeOH=10/1) to obtain compound **17d** (65 mg), white solid.

MS (ESI) *m/z* 483 $[M + H]^+$.

$^1$H NMR (400 MHz, DMSO-$d_6$) $\delta$ 8.10-8.09 (m, 2H), 7.55 (dd, *J* = 4.0, 4.0 Hz, 1H), *6.81 (d, J = 8.0* Hz, 1H), 5.95 (s, 2H), 3.74 (s, 3H), 3.70-3.66 (m, 1H), 3.51-3.45 (m, 2H), 3.17 (d, *J* = 4.0 *Hz,* 1H), 1.97-1.96 (m, 4H), 1.66-1.62 (m, 2H).

**Step 5 *N*- [4- (4-amino-7-methyl-5- (6- (1-oxotetrahydro-2*H*-1 $\lambda^6$-thiopyran-1- ylidene) amino) pyridin-3-yl) pyrrolo [2,3-*d*] pyrimidin-6-yl) phenyl) methacrylamide 17**

At room temperature, compound **17d** (65 mg, 0.13 mmol), compound **4a** (40.20 mg, 0.14 mmol), chlorine[(n-butyl di (1-adamantanyl) phosphine) -2- (2- aminobiphenyl)] palladium (II) (8.69 mg, 0.013 mmol), potassium phosphate (55.12 mg, 0.26 mmol), N, N-dimethylacetamide (2 mL)/water (0.5 mL) were added to a round bottom flask sequentially, followed by vacuum pumping and nitrogen gas purging three times. The reaction was heated up to 80 °C and stirred for 3 h. After the reaction was completed, the mixture was cooled down to room temperature and concentrated under reduced pressure to

obtain a crude product. The crude product was purified by column chromatography (DCM/MeOH =10/1) to obtain compound 17 (11.2 mg), white solid.

MS (ESI) *m/z* 516 [M + H]+.

$^1$H NMR (400 MHz, DMSO-$d_6$) $\delta$ 9.93 (s, 1H), 8.19 (s, 1H), 7.90 (d, *J* = 4.0 *Hz, 1H),* 7.73 *(d, J* = 8.0 Hz, 2H), 7.40 (dd, *J* = 4.0, 4.0 Hz, 1H), 7.28 (d, *J* = 8.0 Hz, 2H), 6.67 (d, *J = 8.0* Hz, 1H), 5.88 (s, 2H), 5.82 (s, 1H), 5.53 (s, 1H), 3.62 (s, 1H), 3.60 *(s,* 3H), 3.34-3.41 (m, 1H), 3.18-3.17 (m, 2H), 1.96 (s, 3H), 1.95-1.93 (m, 4H), 1.61-1.58 (m, 2H).

**Example 18 *N-* (4- (4-amino-5- (3-fluoro-4- ((1-oxotetrahydro-1 $\lambda^6$-thiophene-1- ylidene) amino) phenyl) -7-methyl-7*H*-pyrrolo [2,3-*d*] pyrimidin-6-yl) phenyl) -2- (methoxymethyl) acrylamide**

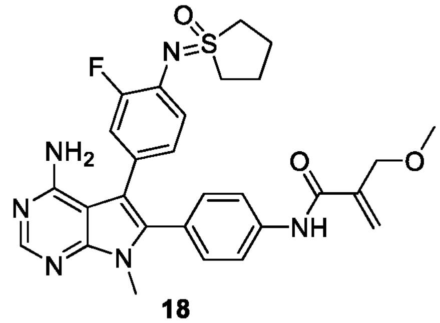

**18**

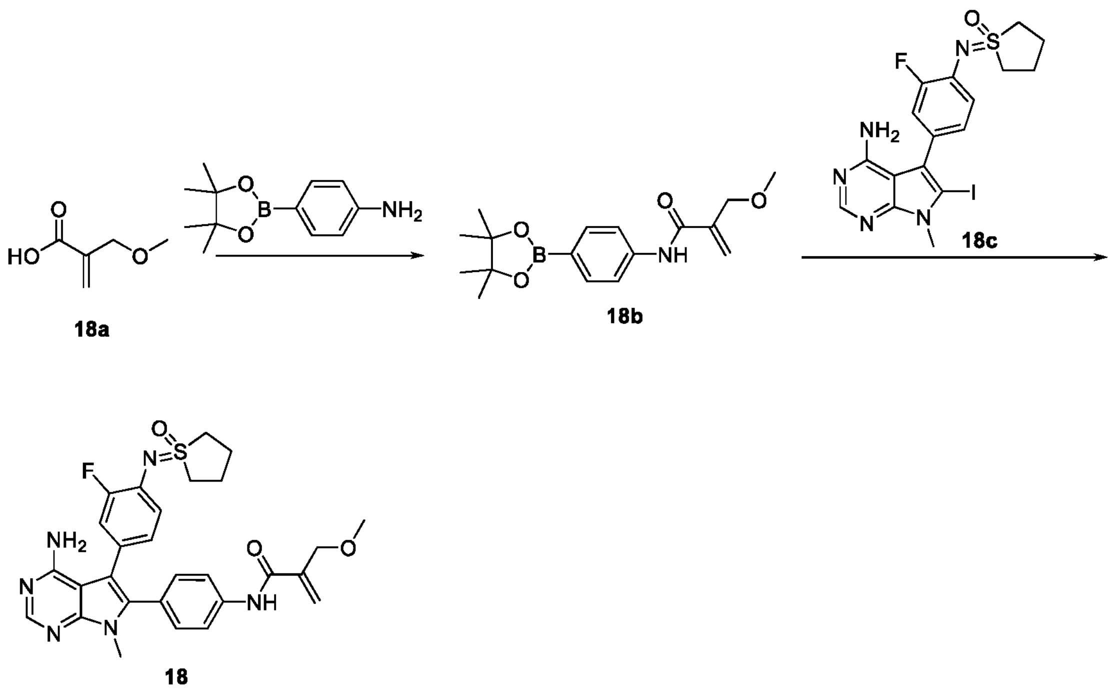

18a

18b

18c

18

**Step** 1 **7-methyl-5- (4,4,5,5-tetramethyl-1,3,2-dioxaborolan-2-yl) pyrrolo [2,3-*d*] pyrimidin-4-amine 18b**

Compound **18a** (150 mg, 1.29 mmol) and 4-(4,4,5,5-tetramethyl-1,3,2-dioxaborolan -2-yl) aniline (197.23 mg, 0.90 mmol) were dissolved in N, N-dimethylformamide (3 mL), and then triethylamine (391.6 mg, 3.87 mmol) was added. It was cooled down to 0 °C, a solution of 1-propylphosphonic anhydride in 50% ethyl acetate (820.90 mg, 1.93 mmol) was added slowly dropwise, and heated up to room temperature and stirred to react for 4 h. After the reaction was completed, the mixture was diluted with water, extracted with ethyl acetate (5 ml × 3), washed with saturated saline solution (10 mL), and the organic phases were combined and dried over anhydrous sodium sulfate. The crude product was concentrated under reduced pressure and purified by column chromatography (DCM/MeOH=15/1) to obtain compound **18b** (60 mg), light yellow solid.

MS (ESI) *m/z* 275 [M + H]+.

$^1$H NMR (400 MHz, DMSO-$d_6$) $\delta$ 10.00 (s, 1H), 7.71 (d, *J* = 8.0 Hz, 2H), *7.62 (d, J* = 8.0 Hz, 2H), 5.97 (s, 1H), 5.70

(s, 1H), 4.16 (s, 2H), 3.30 (s, 3H), *1.29* (s, 12H).

**Step 2 *N*- (4- *(4-amino-5-* (3-fluoro-4- ((1-oxotetrahydro-1 λ6-thiophene-1-ylidene) amino) phenyl) -7-methyl-7*H*-pyrrolo [2,3-*d*] pyrimidin-6-yl) phenyl) -2- (methoxymethyl) acrylamide 18**

At room temperature, compound 18b (60.00 mg, 0.19 mmol), compound 18c (82.50 mg, 0.17 mmol), chlorine [(n-butyl di (1-adamantanyl) phosphine) -2- (2-aminobiphenyl)] palladium (II) (12.70 mg, 0.019 mmol), potassium phosphate (80.56 mg, 0.38 mmol), N, N-dimethylacetamide (2 mL), and water (0.5 mL) were sequentially added to a round bottom flask, followed by vacuum pumping and nitrogen gas purging three times, then heat up to 80 °C and stirred for 3 h. After the reaction was completed, the mixture was cooled to room temperature and concentrated under reduced pressure to obtain a crude product. The crude product was purified by column chromatography (DCM/MeOH=15/1) to obtain compound 18 (12.3 mg), white solid.

MS (ESI) *m/z* 549 $[M + H]^+$.

$^1H$ NMR (400 MHz, DMSO-$d_6$) $\delta$ 10.09 (s, 1H), 8.24 (s, 1H), 7.77 (d, *J* = 8.0 *Hz, 2H),* 7.35 *(d, J= 8.0* Hz, 2H), 7.10 (t, *J* = 20.0 Hz, 1H), 6.99-6.95 (m, 2H), 6.02 (s, 1H), 5.76 (s, 1H), 4.22 (s, 2H), *3.65* (s, 3H), 3.42 (s, 2H), 3.37 (s, 3H), 2.29-2.06 (m, 4H), 1.31 (s, 1H).

**Example 19 (4- (4-amino-6- (4,4-dimethyl-2- (propyl-1-yn-1-yl) -4*H* benzo [d] [1,3] oxazin-6-yl) -7-methyl-7*H*-pyrrolo [2,3-*d*] pyrimidin-5-yl) -2-fluorophenyl) imino) dimethyl - λ6-sulfoxide**

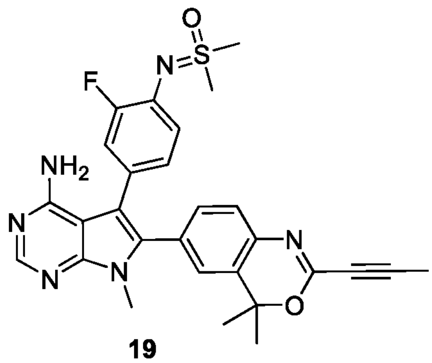

**19**

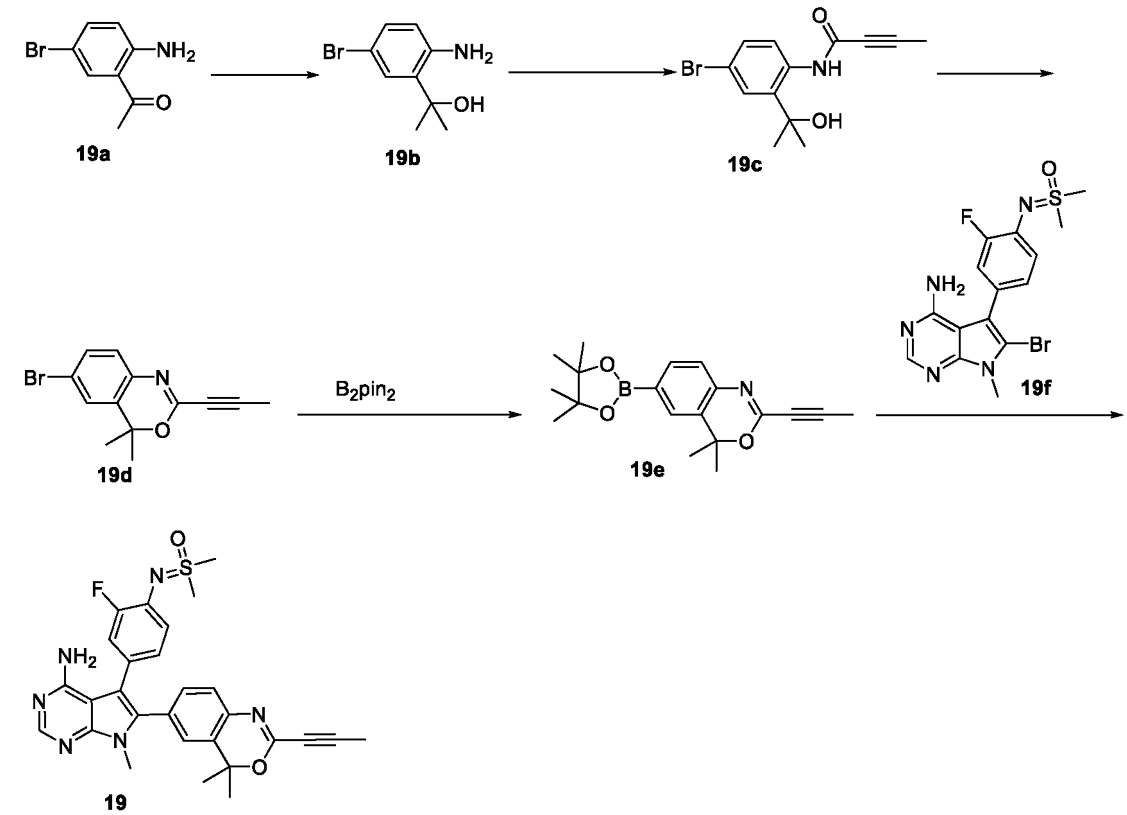


19a 19b 19c 19d 19e 19f 19

**Step 1 2- (2-amino-5-bromophenyl) propan-2-ol 19b**

Compound **19a** (1 g, 4.67 mmol) was dissolved in tetrahydrofuran (5 mL), and it was cooled to around 0 °C, a solution of methyl magnesium bromide in tetrahydrofuran (1 M, 7 mL) was added, and then the temperature was restored to room temperature to react for 2 h. The reaction solution was quenched by added saturated amine chloride solution (20 mL), extracted with ethyl acetate (20 mL × 3), and the organic phases were combined, dried over anhydrous sodium sulfate and concentrated. The crude product was purified by column chromatography (PE/EtOAc=10/1) to obtain compound **19b** (0.7 g), white solid.

MS (ESI) *m/z* 230 $[M + H]^+$.

**Step 2 *N*- (4-bromo-2- (2-hydroxypropyl-2-yl) phenyl) but-2-amide 19c**

Compound **19b** (200 mg, 0.87 mmol) and 2-butyne acid (73 mg, 0.87 mmol) were dissolved in dry dichloromethane (5 mL), after cooled down to around 0 °C, 1-ethyl-(3-dimethylaminopropyl) carbodiimide hydrochloride (183 mg, 0.95 mmol) was added, and then the temperature was restored to room temperature to react for 3 h. The reaction solution was concentrated, and the crude product was purified by column chromatography (PE/EtOAc=10/1) to obtain compound **19c** (200 mg), yellow oil.

MS (ESI) *m/z* 296 $[M + H]^+$.

$^1H$ NMR (400 MHz, DMSO-$d_6$) $\delta$ 10.90 (s, 1H), 8.04 (d, $J$ = 9.2 Hz, 1H), 7.52 - 7.35 (m, 2H), 6.38 (s, 1H), 2.04 (s, 3H), 1.53 (s, 6H).

**Step 3 6-bromo-4,4-dimethyl-2- (propyl-1-acetyl-1-yl) -4H benzo [d] [1,3] oxazine 19d**

Compound **19c** (540 mg, 1.82 mmol) was dissolved in dry dichloromethane (5 mL), methanesulfonic acid (876 mg, 9.13 mmol) was added, and under nitrogen protection, it was heated up to 45 °C to react for 16 h, and then cooled down to room temperature, concentrated to dry solvent. Saturated sodium bicarbonate solution (20 mL) was added and extracted with ethyl acetate (20 mL × 3), and the organic phases were combined, washed with saturated saline water (50 mL), and concentrated under reduced pressure to obtain a crude product. The crude product was purified by column chromatography (PE/EtOAc=50/1) to obtain compound 19d (240 mg), yellow oil.

MS (ESI) *m/z* 278 $[M + H]^+$.

$^1H$ NMR (400 MHz, DMSO-$d_6$) $\delta$ 7.52 (d, $J$ = 2.2 Hz, 1H), 7.49 - 7.45 (m, 1H), 7.01 (d, $J$ = 8.3 Hz, 1H), 2.05 (s, 3H),

1.58 (s, 6H).

**Step 4 4,4-dimethyl-2-(propyl-1-yn-1-yl)-6-(4,4,5,5-tetramethyl-1,3,2- dioxaborolan -2-yl) *-4H* benzo [*d*] [1,3] oxazine 19e**

Compound **19d** (270 mg, 0.97 mmol) was dissolved in dry 1,4-dioxane (10 mL), bis(pinacolato)diboron (370 mg, 1.46 mmol), 1,1'- biphenylphosphine ferrocene palladium chloride (71 mg, 0.097 mmol), and potassium acetate (285 mg, 2.91 mmol) were added sequentially, after replaced with nitrogen three times, heated up to 90 °C under nitrogen protection to react for 18 h. Then cooled down to room temperature, filtered, concentrated, and the crude product was purified by column chromatography (PE/EtOAc =50/1) to obtain compound **19e** (140 mg), yellow oil.
MS (ESI) *m*/*z* 325 $[M + H]^+$.

**Step 5 (4- (4-amino-6-(4,4-dimethyl-2-(propyl-1-yn-1-yl)-4*H* benzo [*d*] [1,3] oxazine -6-yl) -7-methyl-7*H*-pyrrolo [2,3-*d*] pyrimidin-5-yl) -2-fluorophenyl) imino) dimethyl - $\lambda^6$-sulfoxide 19**

Compound **19e** (66 mg, 0.20 mmol), ((4-(4-amino-6-bromo-7-methyl-7H-pyrrolo [2,3-d] pyrimidin-5-yl)-2-fluorophenyl) imino) dimethyl- $\lambda^6$-sulfone **19f** (70 mg, 0.17 mmol) were dissolved in 1,4-dioxane (7 mL) and water (1.5 mL), tetrakis (triphenylphosphine) palladium (39.2 mg, 0.034 mmol), $K_3PO_4$ (108 mg, 0.51 mmol) were added, after replaced with nitrogen gas three times, heated up to 90 °C to react for 2 h. Then cooled down to room temperature, diluted with water (10 mL), extracted with dichloromethane (10 mL × 3), and the organic phases were combined, dried with anhydrous sodium sulfate, concentrated, and the crude product was separated by high-performance liquid chromatography (preparation column: YMCC18, mobile phase A: 0.05% ammonia solution in $H_2O$, mobile phase B: acetonitrile, 30% ~60% acetonitrile, 40mL/min) to obtain compound **19** (13 mg), white solid.
MS (ESI) *m*/*z* 531 $[M + H]^+$.
$^1H$ NMR (400 MHz, DMSO-$d_6$) $\delta$ 8.20 (s, 1H), 7.29 (dd, *J* = 8.0, 1.8 Hz, 1H), *7.12 (dd, J = 15.3,* 5.1 Hz, 2H), 7.04 (d, *J* = 8.0 Hz, 1H), 6.96 - 6.86 (m, 2H), 6.00 *(brs,* 2H), 3.66 (s, 3H), 3.26 (s, 6H), 2.04 (s, 3H), 1.44 (s, 6H).

**Example 20 *N*- (4- (4-amino-5- (4- (dimethyl (oxo) - $\lambda^6$-sulfanylidene) amino) -3-fluorophenyl) -7-methyl-7*H*-pyrrolo [2,3-*d*] pyrimidin-6-yl) -2- (2-hydroxypropan -2-yl) phenyl) but-2-amide**

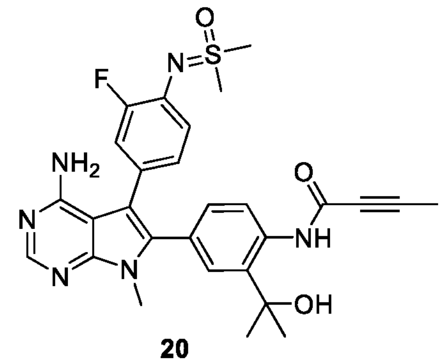

20

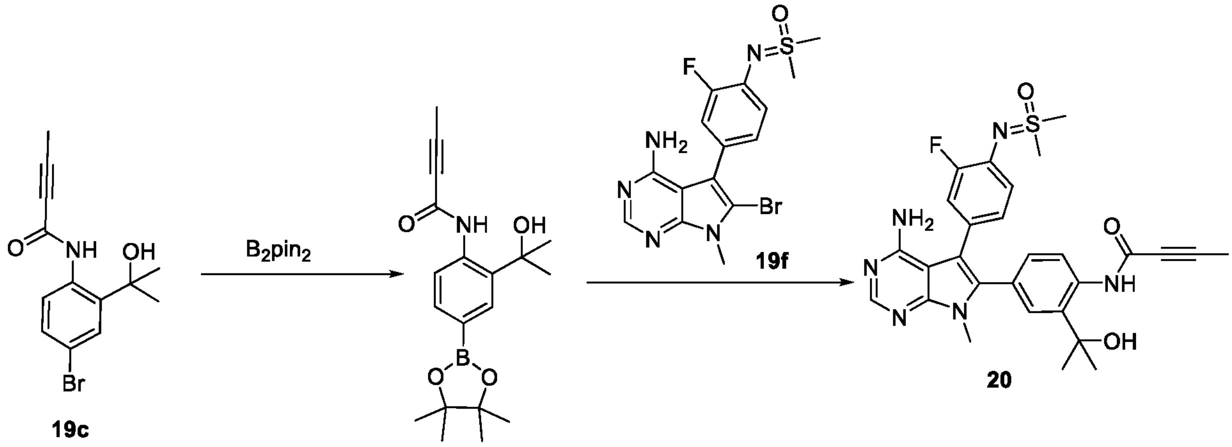


19c 20a 19f 20

**Step 1 *N*-(2- (2-hydroxypropan-2-yl) -4- (4,4,5,5-tetramethyl-1,3,2- dioxaborolan - 2-yl) phenyl) but-2-amide 20a**

Compound **19c** (270 mg, 0.97 mmol) was dissolved in dry 1,4-dioxane (10 mL), bis(pinacolato)diboron (370 mg, 1.46 mmol), Pd(dppf)$Cl_2$ (71 mg, 0.097 mmol), and potassium acetate (285 mg, 2.91 mmol) were added sequentially, after replaced with nitrogen three times, heated up to 90 °C under nitrogen protection, and reacted for 18 h. Then cooled to room temperature, filtered, concentrated, and the crude product was purified by column chromatography (PE/EtOAc=50/1) to obtain compound **20a** (140 mg), yellow oil.

MS (ESI) *m/z* 343 $[M + H]^+$.

**Step 2 *N*- (4- (4-amino-5- (4- (dimethyl (oxo) - λ$^6$-sulfanylidene) amino) -3-fluorophenyl) -7-methyl-7*H*-pyrrolo [2,3-*d*] pyrimidin-6-yl) -2- (2-hydroxypropan-2-yl) phenyl) but-2-amide 20**

Compound **20a** (70 mg, 0.20 mmol), (4- (4-amino-6-bromo-7-methyl-7*H*-pyrrolo [2,3-*d*] pyrimidin-5-yl) -2-fluorophenyl) imino) dimethyl - λ$^6$ -sulfone **19f** (66 mg, 0.16 mmol) were dissolved in 1,4-dioxane (7 mL) and water (1.5 mL), tetrakis (triphenylphosphine) palladium (39.2 mg, 0.034 mmol), $K_3PO_4$ (108 mg, 0.51 mmol) were added, after replaced with nitrogen gas three times, heated up to 90 °C and reacted for 2 h. Then cooled down to room temperature, diluted with water (10 mL), extracted with dichloromethane (10 mL × 3), and the organic phases were combined, dried with anhydrous sodium sulfate, concentrated, and the crude product was separated by high-performance liquid chromatography (preparation column: YMCC18, mobile phase A: 0.05% ammonia solution in $H_2O$, mobile phase B: acetonitrile, 30%~60% acetonitrile, 40mL/min) to obtain compound **20** (7.6 mg), white solid.

MS (ESI) *m/z* 549 $[M + H]^+$.

$^1$H NMR (400 MHz, DMSO-$d_6$) $\delta$ 12.71 (s, 1H), 8.21 (s, 1H), 7.34 (d, *J = 8.2 Hz, 1H),* 7.21 - 7.10 (m, 3H), 6.92 (dd, *J* = 16.5, 11.0 Hz, 2H), 6.07 (brs, 2H), 5.02 (s, 1H), 3.66 (s, 3H), 3.26 (s, 6H), 2.00 (s, 3H), 1.49 (s, 6H).

**Example 21 *N*- (4- (4-amino-5- (3-fluoro-4- ((1-oxotetrahydro-1 λ$^6$-thiophene-1- ylidene) amino) phenyl) -7-methyl-7*H*-pyrrolo [2,3-*d*] pyrimidin-6-yl) phenyl) -2-fluoroacrylamide**

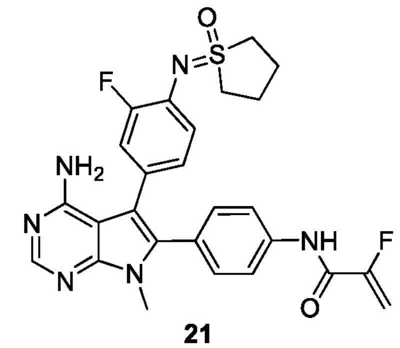

**21**

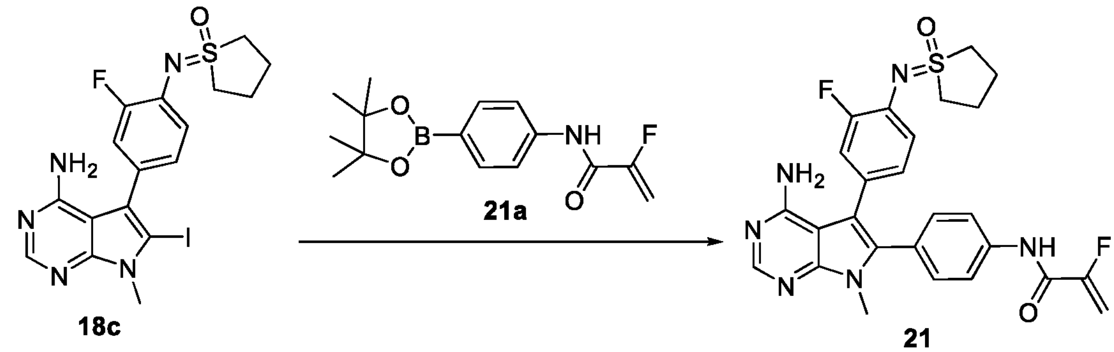

**18c** **21a** **21**

Compound **18c** (100 mg, 0.20 mmol) and compound 21a (71 mg, 0.24 mmol) were dissolved in N,N-dimethylacetamide (4 mL) and water (1 mL), chlorine [(n-butyl di (1-adamantanyl) phosphine) -2- (2-aminobiphenyl)] palladium (II) (13.8 mg, 0.02 mmol) and $K_3PO_4$ (87.4 mg, 0.4 mmol) were added, after replaced with nitrogen gas three times, heated up to 80 °C and reacted for 2 h. Then cooled down to room temperature, diluted with water (20 mL), extracted with dichloromethane (20 mL × 3), and the organic phases were combined, dried with anhydrous sodium sulfate, concentrated, and the crude product were separated by high-performance liquid chromatography (preparation column: YMCC18, mobile phase A: 0.05% ammonia solution in $H_2O$, mobile phase B: acetonitrile, 30%~60% acetonitrile, 40mL/min) to obtain compound **21** (23.6 mg), white solid.

MS (ESI) *m*/*z* 523 $[M + H]^+$.

$^1$H NMR (400 MHz, DMSO-$d_6$) $\delta$ 10.40 (s, 1H), 8.19 (s, 1H), 7.77 (d, *J* = 8.0 *Hz, 2H), 7.33* (d, *J* = 8.1 Hz, 2H), 7.26 - 7.16 (m, 1H), 7.05 (t, *J* = 8.6 Hz, 1H), 6.93 (d, *J* = 8.5 Hz, 1H), 5.93 (brs, 2H), 5.80 (s, 0.5H), 5.68 (s, 0.5H), 5.46 (d, *J* = 14.6 Hz, 1H), 3.60 *(s,* 3H), 3.29-3.12 (m, 4H), 2.27-2.08 (m, 4H).

**Example 22 *N-* (4- (4-amino-5- (3-fluoro-4- ((1-oxotetrahydro-1 $\lambda^6$-thiophene-1-ylidene) amino) phenyl) -7-methyl-7*H*-pyrrolo [2,3-*d*] pyrimidin-6-yl) -3-methylphenyl) methacrylamide**

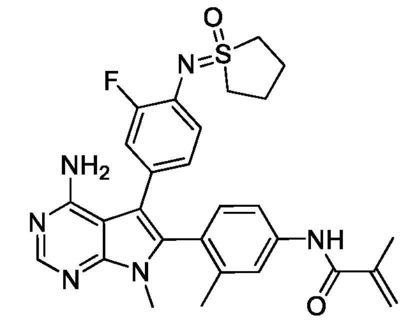

**22**

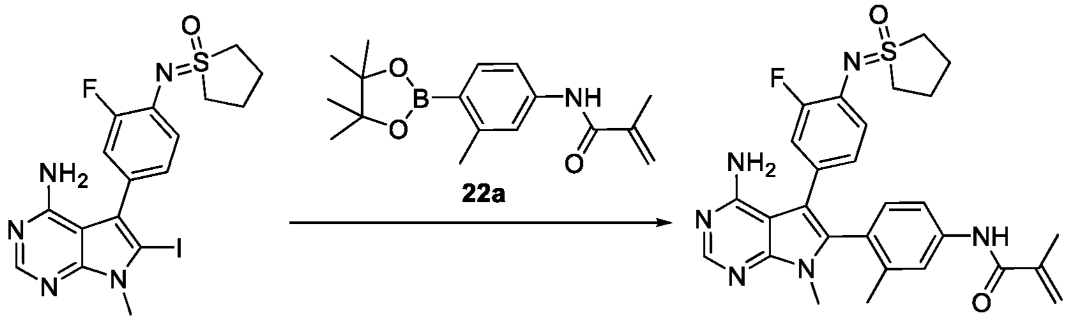

**22a**

**18c** **22**

Compound **18c** (100 mg, 0.20 mmol) and compound **22a** (74 mg, 0.24 mmol) were dissolved in N,N-dimethylacetamide (4 mL) and water (1 mL), chlorine [(n-butyl di (1-adamantanyl) phosphine) -2- (2-aminobiphenyl)] palladium (II) (13.8 mg, 0.02 mmol) and $K_3PO_4$ (87.4 mg, 0.4 mmol) were added, after replaced with nitrogen three times, heated up to 80 °C to react for 2 h. Then cooled down to room temperature, diluted with water (20 mL), extracted with dichloromethane (20 mL × 3), and the organic phases were combined, dried with anhydrous sodium sulfate, concentrated, and the crude product was separated by high-performance liquid chromatography (preparation column: YMCC18, mobile phase A: 0.05% ammonia solution in $H_2O$, mobile phase B: acetonitrile, 30%~60% acetonitrile, 40mL/min) to obtain compound **22** (30.9 mg), white solid.

MS (ESI) *m*/*z* 533 $[M + H]^+$.

$^1H$ NMR (400 MHz, DMSO-$d_6$) $\delta$ 9.83 (s, 1H), 8.19 (s, 1H), 7.67 - 7.58 (m, 2H), 7.27 (m, 2H), 7.01 (m, 1H), 6.87 (m, 1H), 5.99 (brs, 2H), 5.82 (s, 1H), 5.53 (s, 1H), 3.41 (s, 3H), 3.28 (m, 4H), 2.27 - 2.08 (m, 4H), 1.96 (s, 3H), 1.93 (s, 3H).

**Example 23 *N*- (4- (4-amino-5- (3-fluoro-4- ((1-oxotetrahydro-1 λ⁶-thiophene-1- ylidene) amino) phenyl) -7-methyl-7*H*-pyrrolo [2,3-*d*] pyrimidin-6-yl) -3-fluorophenyl) methacrylamide**

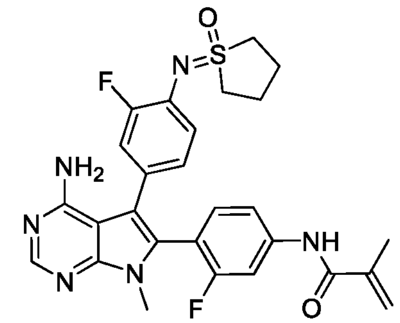

**23**

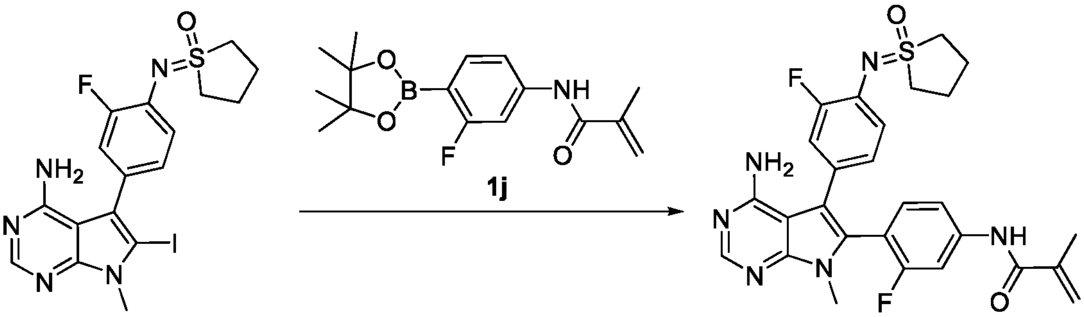


**18c** **23**

Compound **18c** (100 mg, 0.20 mmol) and compound **1j** (70 mg, 0.23 mmol) were dissolved in N, N-dimethylacetamide (4 mL) and water (1 mL), chlorine [(n-butyl di (1-adamantanyl) phosphine) -2- (2-aminobiphenyl)] palladium (II) (13.8 mg, 0.02 mmol), $K_3PO_4$ (87.4 mg, 0.4 mmol) were added, after replaced with nitrogen three times, heated up to 80 °C to react for 2 h. Then cooled to room temperature, diluted with water (20 mL), extracted with dichloromethane (20 mL × 3), and the organic phases were combined, dried with anhydrous sodium sulfate, concentrated, and the crude product was separated by high-performance liquid chromatography (preparation column: YMCC18, mobile phase A: 0.05% ammonia solution in $H_2O$, mobile phase B: acetonitrile, 30%~60% acetonitrile, 40mL/min) to obtain compound **23** (17.5 mg), white solid.

MS (ESI) *m/z* 537 $[M + H]^+$.

$^1H$ NMR (400 MHz, DMSO-$d_6$) δ 10.12 (s, 1H), 8.21 (s, 1H), 7.78 (d, *J* = *12.4 Hz,* 1H), *7.52* (d, *J* = 8.0 Hz, 1H), 7.31 (t, *J* = 8.0 Hz, 1H), 7.05 (t, *J* = 8.4 Hz, 1H), 6.92 (d, *J* = 10.7 *Hz,* 2H), 6.04 (brs, 2H), 5.84 (s, 1H), 5.59 (s, 1H), 3.54 (s, 3H), 3.31 (m, *4H),* 2.18 (dd, *J* = 33.7, 6.6 Hz, 4H), 1.97 (s, 3H).

**Example 24 *N-* (4- (4-amino-7-methyl-5- (4- (1-oxotetrahydro-1 $\lambda^6$-thiophene-1- ylidene) amino) phenyl) -7*H*-pyrrolo [2,3-*d*] pyrimidin-6-yl) -3-fluorophenyl) methacrylamide**

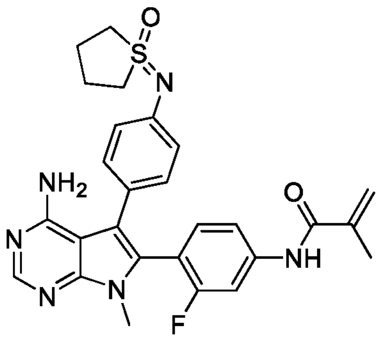

**24**

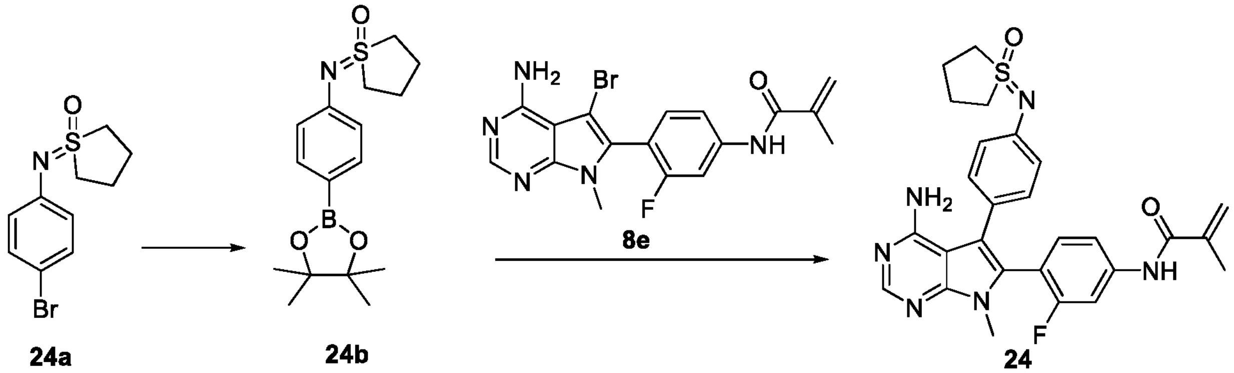

24a 24b 8e 24

**Step 1 1- (4- (4,4,5,5-tetramethyl-1,3,2-dioxaborolan-2-yl) phenyl) imino) tetrahydro-1H-1 λ⁶-thiophene 1-oxide 24b**

To a 100 mL round bottom flask, compound **24h** (1.02 g, 3.10 mmol), bis(pinacolato)diboron (0.973 g, 3.83 mmol), Pd(dppf)$Cl_2$ (0.461 mg, 0.63 mmol), potassium acetate (0.943g, 9.61 mmol), 1,4-dioxane (20 mL) were added sequentially, then heated up to 100 °C to react for 4 h, after TLC monitored the completion of the reaction, it was cooled down to room temperature, filtered through diatomaceous earth, extracted with ethyl acetate (50 mL × 3), and the organic phases were combined, washed with saturated saline (50 mL), and concentrated under reduced pressure to obtain a crude product. The crude product was purified by column chromatography (PE/EtOAc=1/1) to obtain compound **24b** (0.80 g), yellow solid.

MS (ESI) *m/z* 322.2 $[M + H]^+$.

$^1H$ NMR (400 MHz, $CDCl_3$) δ 7.62 (d, *J* = 8.3 Hz, 2H), 6.98 (d, *J* = 8.3 *Hz,* 2H), 3.37 - 3.30 (m, 1H), *3.15* - 3.08 (m, 1H), 2.20 (dd, *J* = 29.3, 6.9 Hz, 2H), 1.26 (s, 6H).

**Step 2 *N-* (4- (4-amino-7-methyl-5- (4- (1-oxotetrahydro-1 λ⁶-thiophene-1-ylidene) amino) phenyl) -7*H*-pyrrolo [2,3-*d*] pyrimidin-6-yl) -3-fluorophenyl) methacrylamide 24**

To a 25 mL round bottom flask, compound **4b** (45 mg, 0.14 mmol), compound **8e** (50 mg, 0.12 mmol), and Pd(dppf)$Cl_2$ (20 mg, 0.03 mmol), potassium phosphate (80 mg, 0.38 mmol), 1,4-dioxane (5 mL), and water (1 mL) were added in sequence, then heated up to 80 °C to react for 0.5 h, after TLC monitored the completion of the reaction, it was cooled down to room temperature, filtered through diatomaceous earth, extracted with ethyl acetate (20 mL × 3), and the organic phases were combined, washed with saturated saline (20 mL), and concentrated under reduced pressure to obtain a crude product. The crude product was purified by column chromatography (DCM/MeOH=6/1) to obtain compound **24** (22 mg), white solid.

MS (ESI) *m/z* 519.41 $[M + H]^+$.

$^1H$ NMR (400 MHz, $CDCl_3$) δ 8.28 (s, 1H), 7.65 - 7.61 (m, 1H), 7.48 (s, 1H), *7.05* (d, *J* = 7.7 Hz, 2H), 7.00 (t, *J* = 4.1 Hz, 2H), 6.91 (d, *J* = 8.5 Hz, 2H), 5.73 (s, 1H), 5.45 (d, *J* = 1.3 Hz, 1H), 4.98 (s, 2H), 3.59 (d, *J* = *0.9* Hz, 3H), 3.36 (dd, *J* = 12.6, 6.0 Hz, 2H), 3.13 (dd, *J* = *12.8,* 6.6 Hz, 2H), 2.29 - 2.19 (m, 3H), 1.99 (d, *J* = 10.1 Hz, *3H).*

**Example 25 *N*- (4- (4-amino-5- (3-fluoro-4- ((1-oxotetrahydro-1 $\lambda^6$-thiophene-1-ylidene) amino) phenyl) -7-methyl-7*H*-pyrrolo [2,3-*d*] pyrimidin-6-yl) -3-cyanophenyl) methacrylamide**

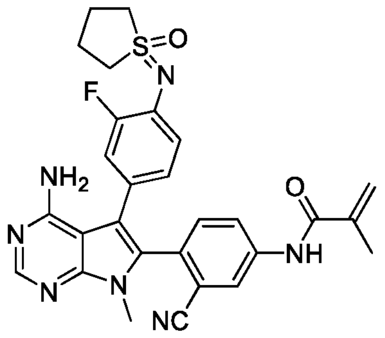

**25**

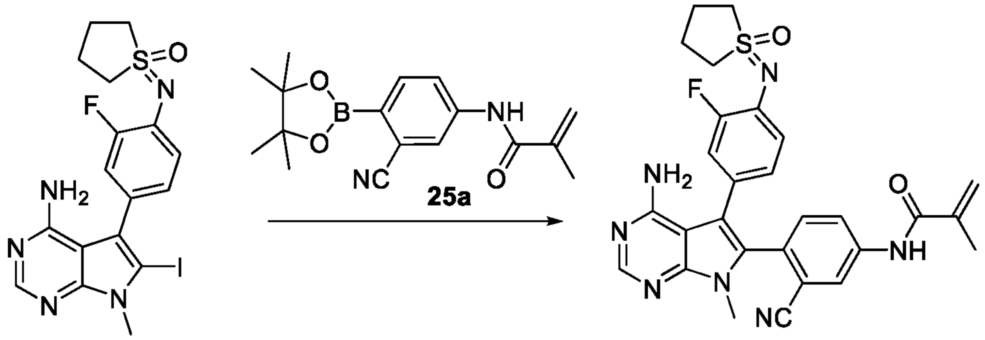


**18c** **25**

To a 25 mL round bottom flask, compound **18c** (80 mg, 0.16 mmol), compound **25a** (57 mg, 0.18 mmol), Pd(dppf) $Cl_2$ (12 mg, 0.016 mmol), potassium phosphate (102 mg, 0.48 mmol), 1,4-dioxane (10 mL), and water (1 mL) were added in sequence, then heated up to 80 °C to react for 1 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (DCM/MeOH=6/1) to obtain compound **25** (4 mg), white solid.

MS (ESI) *m*/*z* 544.73 [M + H]$^+$

$^1H$ NMR (400 MHz, $CDCl_3$) $\delta$ 8.28 (s, 1H), 7.65 - 7.61 (m, 1H), 7.48 (s, 1H), *7.05* (d, *J* = 7.7 Hz, 2H), 7.00 (t, *J* = 4.1 Hz, 2H), 6.91 (d, *J* = 8.5 Hz, 2H), 5.73 (s, 1H), 5.45 (d, *J* = 1.3 Hz, 1H), 4.98 (s, 2H), 3.59 (d, *J* = *0.9* Hz, 3H), 3.36 (dd, *J* = 12.6, 6.0 Hz, 2H), 3.13 (dd, *J* = *12.8,* 6.6 Hz, 2H), 2.29 - 2.19 (m, 3H), 1.99 (d, *J* = 10.1 Hz, *3H).*

**Example 26 *N*- (4- (4-amino-5- (3-fluoro-4- ((1-oxotetrahydro-1 $\lambda^6$-thiophene-1-ylidene) amino) phenyl) -7-methyl-7*H*-pyrrolo [2,3-*d*] pyrimidin-6-yl) -3-cyanophenyl) methacrylamide**

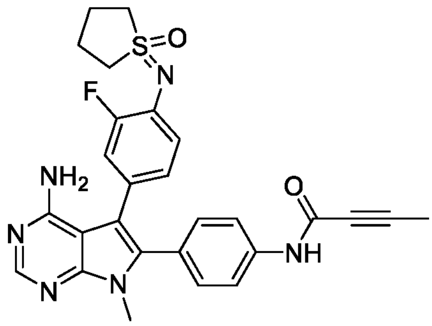

**26**

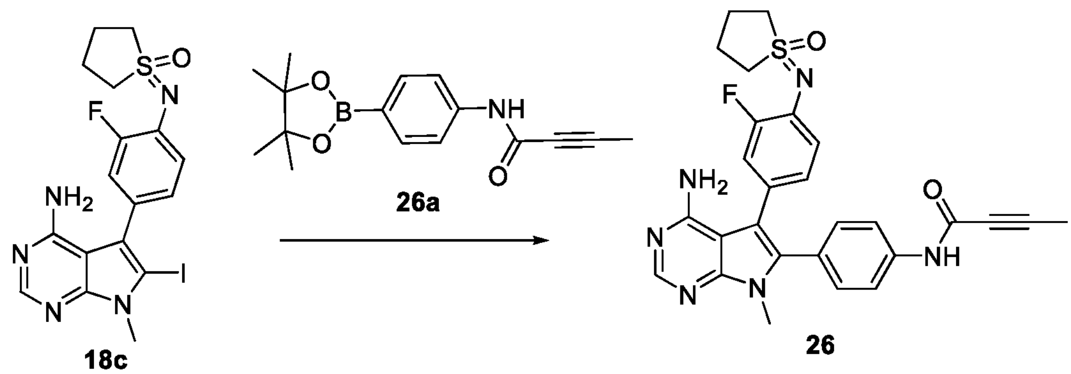

**18c** **26a** **26**

To a 25 mL round bottom flask, compound **18c** (120 mg, 0.25 mmol), compound **26a** (80 mg, 0.28 mmol), tetrakis (triphenylphosphine) palladium (29 mg, 0.025 mmol), anhydrous potassium phosphate (159 mg, 0.75 mmol), 1,4-dioxane (10 mL), and water (1 mL) were added in sequence, then heated up to 90 °C to react for 1 h, after the reaction was completed, it was cooled down to room temperature, filtered through diatomaceous earth, extracted with ethyl acetate (30 mL × 3), the organic phases were combined, washed with saturated saline (30 mL), and concentrated under reduced pressure to obtain a crude product. The crude product was purified by column chromatography (DCM/MeOH=6/1) to obtain compound **26** (13.8 mg), white solid.

MS (ESI) *m/z* 517.97 $[M + H]^+$

$^1H$ NMR (400 MHz, $CDCl_3$) $\delta$ 8.28 (s, 1H), 7.43 (s, 2H), 7.34 (s, 1H), 7.14 (d, *J* = 8.5 Hz, 2H), 7.04 (d, *J* = 7.4 Hz, 1H), 6.84 (dd, *J* = 8.7, 6.8 Hz, 2H), 4.95 (s, 2H), 3.62 (s, 1H), 3.36 (dd, *J* = 12.9, 6.8 Hz, 2H), 3.21 - 3.14 (m, 2H), 2.25 (d, *J* = 7.6 Hz, 4H), 1.96 (d, *J* = 3.9 *Hz,* 3H).

**Example 27 *N*- (4-(4-amino-5- (3,5-difluoro-4- ((1-oxotetrahydro-1 λ$^6$-thiophene-1-ylidene) amino) phenyl) -7-methyl-7*H*-pyrrolo [2,3-*d*] pyrimidin-6-yl) phenyl) methacrylamide**

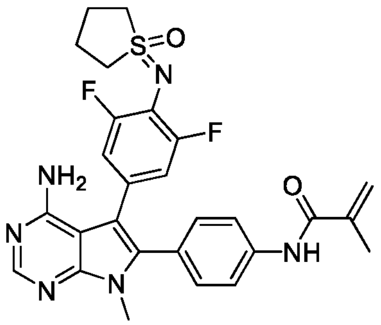

27

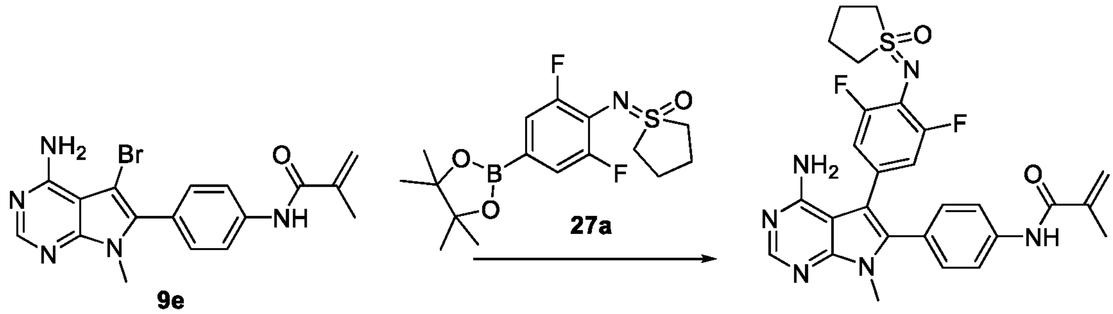

9e 27a 27

To a 25 mL round bottom flask, compound **9e** (78 mg, 0.20 mmol), compound **27a** (80 mg, 0.22 mmol), tetrakis (triphenylphosphine) palladium (25 mg, 0.02 mmol), anhydrous potassium phosphate (127 mg, 0.60 mmol), 1,4-dioxane (10 mL), and water (1 mL) were added in sequence, then heated up to 90 °C to react for 1 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (DCM/MeOH=6/1) to obtain compound **27** (18 mg), white solid.

MS (ESI) *m/z* 537.63 $[M + H]^+$.

$^1$H NMR (400 MHz, $CDCl_3$) $\delta$ 8.38 (s, 1H), 7.62 (d, *J* = 8.5 Hz, 3H), 7.24 *(d, J* = 8.5 Hz, 2H), 6.79 (d, *J* = *8.7* Hz, 2H), 5.84 (s, *1H),* 5.53 (s, 1H), 5.21 (s, 2H), 3.72 (s, 3H), 3.52 - 3.44 (m, 2H), *3.31* (dd, *J* = 12.8, 7.1 Hz, 2H), 2.35 (d, *J* = 2.1 Hz, 4H), *2.11* (s, 3H).

**Example 28 *N*- (4- (4-amino-5- (4- ((dimethylamino) (oxo) (trifluoromethyl) - λ^6^-sulfanylidene) amino) phenyl) -7-methyl-7*H*-pyrrolo [2,3-*d*] pyrimidin-6-yl) phenyl) methacrylamide**

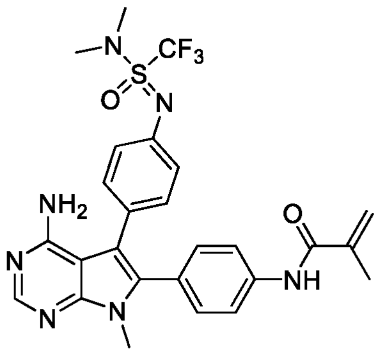

**28**

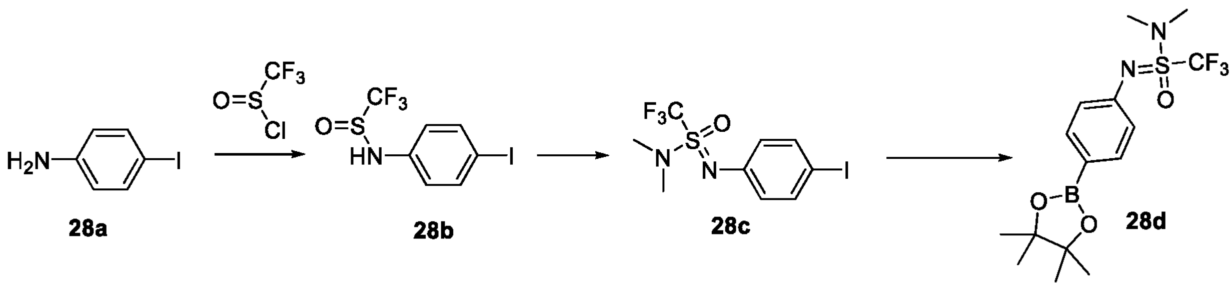

**28a** **28b** **28c** **28d**

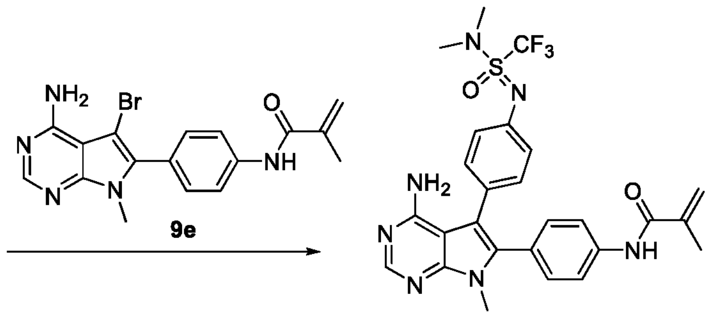

**9e** **28**

**Step 1 1,1,1-trifluoro-*N*- (4-iodophenyl) methylthioamide**

Compound **28a** (1.0 g, 4.6 mmol) was dissolved in DCM (10 mL), triethylamine (0.51 g, 5.0 mmol) was added, and it was cooled down to 0 °C, trifluoromethanesulfonyl chloride (0.73g, 4.8 mmol) was added dropwise, and warmed up to room temperature naturally to react for 1 h. The reaction solution was poured into water, extracted with ethyl acetate three times, dried with anhydrous sodium sulfate, and concentrated to obtain a crude product. The crude product was purified by column chromatography (PE/EtOAc=0-20%) to obtain compound **8b** (1.25 g), yellow oily liquid.

MS (ESI) *m/z* 335 $[M + H]^+$.

$^1H$ NMR (400 MHz, $CDCl_3$) $\delta$ 7.69 (d, *J* = 8.7 Hz, 2H), 6.97 - 6.81 (m, 2H), *6.46* (s, 1H).

**Step 2 1,1,1-trifluoro-*N'*- (4-iodophenyl) - N, N-dimethylmethanesulfonamide 28c**

Compound 28a (1.4 g, 4.2 mmol) and N-chlorosuccinimide (0.62 g, 4.6 mmol) were dissolved in acetonitrile (30 mL), a solution of tetrabutylammonium fluoride in THF (4.6 mL, 4.6 mmol, 1M) was added at 0 °C to react at room temperature for 30 min, and the reaction solution was concentrated, then acetonitrile (20 mL) and a solution of dimethylamine in THF (8.35 mL, 16.7 mmol, 2M) were added, and reacted at room temperature for 2 h. The reaction solution was concentrated and dried, and the obtained crude product was purified by column chromatography (PE/EtOAc=0-20%) to obtain compound **28c** (1.4 g), reddish brown solid.

MS (ESI) *m/z* 379 $[M + H]^+$.

$^1H$ NMR (400 MHz, $CDCl_3$) $\delta$ 7.57 (d, *J* = 8.6 Hz, 2H), 6.87 (d, *J* = 8.6 *Hz,* 2H), 3.08 (s, 6H).

**Step 3 1,1,1-trifluoro-N,N-dimethyl-N'-(4-(4,4,5,5-tetramethyl-1,3,2- dioxaborolan -2-yl) phenyl) methanesulfonamide 28d**

**28c** (1.0 g, 2.6 mmol), bis(pinacolato)diboron (1.0 g, 4.0 mmol), Pd(dppf)$Cl_2$ (193 mg, 0.26 mmol), and potassium acetate (778 mg, 7.9 mmol) were placed in a bottle, 1,4-dioxane (15 mL) was added, after replaced with $N_2$ three times, the reaction was carried out at 100 °C for 18 h. The reaction solution was filtered and concentrated, and the obtained crude product was purified by column chromatography (PE/EtOAc=0-20%) to obtain compound **28d** (0.65 g), white solid.
MS (ESI) *m*/*z*379 $[M + H]^+$.

**Step 4 *N- (4- (4-amino-5-* (4- ((dimethylamino) (oxo) (trifluoromethyl) - λ⁶-sulfanylidene) amino) phenyl) -7-methyl-7*H*-pyrrolo [2,3-*d*] pyrimidin-6-yl) phenyl) methacrylamide 28**

To a 25 mL round bottom flask, compound **9e** (93 mg, 0.24 mmol), compound **28d** (100 mg, 0.26 mmol), tetrakis (triphenylphosphine) palladium (30 mg, 0.024 mmol), anhydrous potassium phosphate (120 mg, 0.54 mmol), 1,4-dioxane (10 mL), and water (1 mL) were added in sequence, and heated up to 90 °C to react for 1 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (DCM/MeOH=6/1) to obtain compound 28 (12.5 mg), white solid.
MS (ESI) *m*/*z* 558.53 $[M + H]^+$.
$^1H$ NMR (400 MHz, $CDCl_3$) $\delta$ 8.37 (s, 1H), 7.63 - 7.54 (m, 3H), 7.24 (d, *J* = *8.5* Hz, 2H), 7.16 (d, *J* = 8.3 Hz, 2H), 7.05 (d, *J* = 8.3 Hz, 2H), 5.83 (s, 1H), 5.52 (s, 1H), 5.19 (s, 2H), 3.74 (s, 3H), 3.11 *(s,* 6H), 2.10 (s, 3H).

**Example 29 *N-* (4- (4-amino-7-methyl-5- (6- (1-oxotetrahydro-1 λ⁶-thiophene-1-ylidene) amino) pyridin-3-yl) -7*H*-pyrrolo [2,3-*d*] pyrimidin-6-yl) phenyl) methacrylamide**

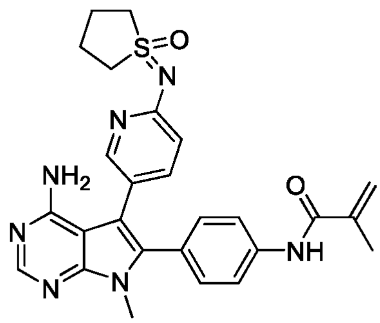

**29**

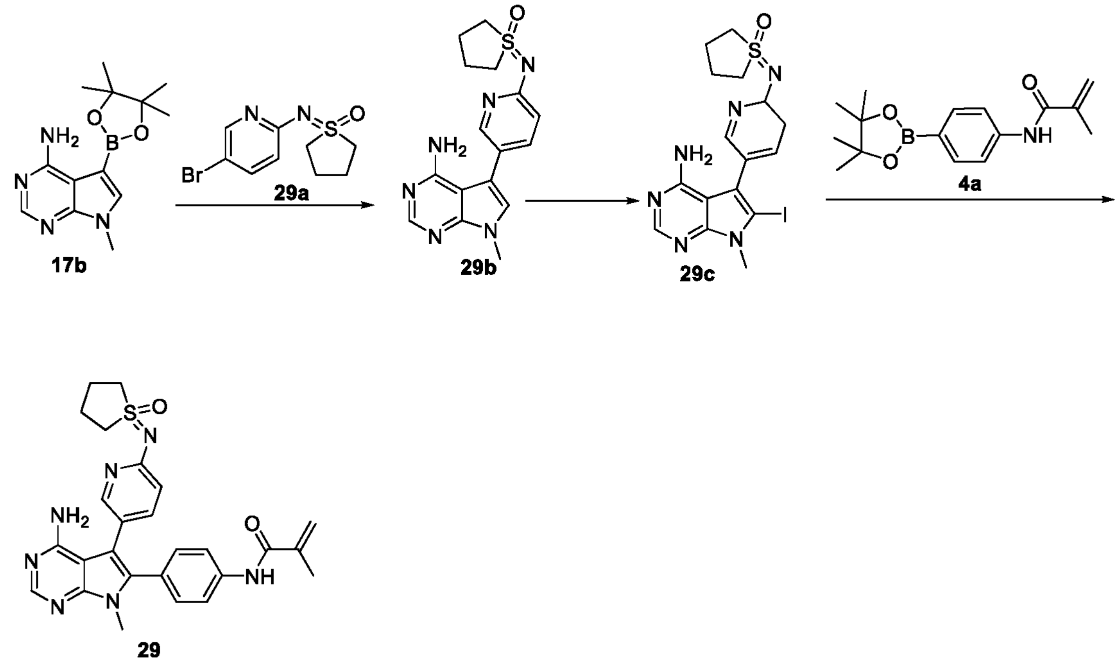


**Step 1 1- (5- (4-amino-7-methyl-7*H*-pyrrolo [2,3-*d*] pyrimidin-5-yl) pyridin-2-yl) imino) tetrahydro-1H-1 λ6-thiophene-1-oxide**

To a 100 mL round bottom flask, compound **7b** (350 mg, 1.28 mmol), compound **9a** (320 mg, 1.17 mmol), tetrakis (triphenylphosphine) palladium (135 mg, 0.012 mmol), anhydrous potassium phosphate (743 mg, 3.50 mmol), 1,4-dioxane (10 mL), and water (1 mL) were added in sequence, and heated up to 90 °C to react for 1 h. After TLC monitored the completion of the reaction, it was cooled down to room temperature, filtered through diatomaceous earth, extracted with ethyl acetate (50 mL × 3), the organic phases were combined, washed with saturated saline (50 mL), and concentrated under reduced pressure to obtain a crude product. The crude product was purified by column chromatography (DCM/MeOH=6/1) to obtain compound **29b** (110 mg), white solid.

MS (ESI) *m/z* 343 $[M + H]^+$.

$^1H$ NMR (400 MHz, $CDCl_3$) δ 8.38 - 8.33 (m, 2H), 7.65 (dd, *J* = 8.3, 2.4 *Hz,* 1H), 6.95 - 6.89 (m, 2H), 5.15 (s, 2H), 3.87 (s, 3H), 3.70 (dd, *J* = 13.3, 6.9 Hz, 2H), 3.44 *(dd, J* = 13.3, 6.8 Hz, 2H), 2.35 (ddd, *J* = 20.5, 12.3, 5.4 Hz, 4H).

**Step 2 1- (5- (4-amino-6-iodo-7-methyl-7*H*-pyrrolo [2,3-*d*] pyrimidin-5-yl) pyridin-2-yl) imino) tetrahydro-1H-1 λ6-thiophene-1-oxide 29c**

To a 100 mL round bottom flask, compound **29b** (110 mg, 0.32 mmol), N-iodosuccinimide (87 mg, 0.38 mmol), trifluoroacetic acid (110 mg, 0.96 mmol) , and dichloromethane (4 mL) were added in sequence, and reacted at room temperature for 2 h, after TLC monitored the completion of the reaction, it was cooled down to room temperature, filtered through diatomaceous earth, extracted with ethyl acetate (30 mL × 3), the organic phases were combined, washed with saturated saline (30 mL), and concentrated under reduced pressure to obtain a crude product. The crude product was purified by column chromatography (DCM/MeOH=10/1) to obtain compound **29c** (20 mg), white solid.

MS (ESI) *m/z* 471 $[M + H]^+$.

**Step 3 *N-* (4- (4-amino-7-methyl-5- (6- (1-oxotetrahydro-1 λ6-thiophene-1- ylidene) amino) pyridine-3-yl) -7*H*-pyrrolo [2,3-*d*] pyrimidin-6-yl) phenyl) methacrylamide 29**

To a 25 mL round bottom flask, compound **29c** (20 mg, 0.05 mmol), compound **4a** (16 mg, 0.05 mmol), tetrakis (triphenylphosphine) palladium (6 mg, 0.005 mmol), anhydrous potassium phosphate (32 mg, 0.15 mmol), 1,4-dioxane (2 mL), and distilled water (0.5 mL) were added in sequence, and heated up to 90 °C to react for 1 h. After TLC monitored the completion of the reaction, it was cooled down to room temperature, filtered through diatomaceous earth, extracted with ethyl acetate (20 mL × 3), the organic phases were combined, washed with saturated saline (20 mL), and concentrated under reduced pressure to obtain a crude product. The crude product was purified by column chromatography (DCM/MeOH=6/1) to obtain compound **29** (7 mg), white solid.

MS (ESI) *m/z* 587.10 $[M + H]^+$.

$^1$H NMR (400 MHz, $CDCl_3$) $\delta$ 8.37 (s, 1H), 8.14 (d, 1.8 Hz, 1H), 7.68 - 7.53 (m, 3H), 7.41 (dd, $J$ = 8.4, 2.4 Hz, 1H), 7.26 (d, $J$ = 8.6 Hz, 2H), 6.78 (d, $J$ = 8.1 Hz, 1H), 5.83 (s, 1H), 5.52 (s, 1H), 5.33 (s, 2H), 3.74 (s, 3H), 3.65 (dd, $J$ = 13.5, 6.8 Hz, *2H),* 3.40 (dd, $J$ = 13.2, 6.7 Hz, 2H), 2.43 - 2.25 (m, 4H), 2.10 (s, 3H).

**Example 30 *N*- (4- (4-amino-5- (3-fluoro-4- ((4-methyl-1-oxo-1 λ$^6$-thiomorpholin-1-ylidene) amino) phenyl) -7-methyl-7*H*-pyrrolo [2,3-*d*] pyrimidin-6-yl) phenyl) methacrylamide**

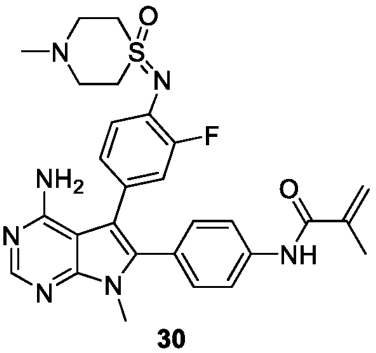

**30**

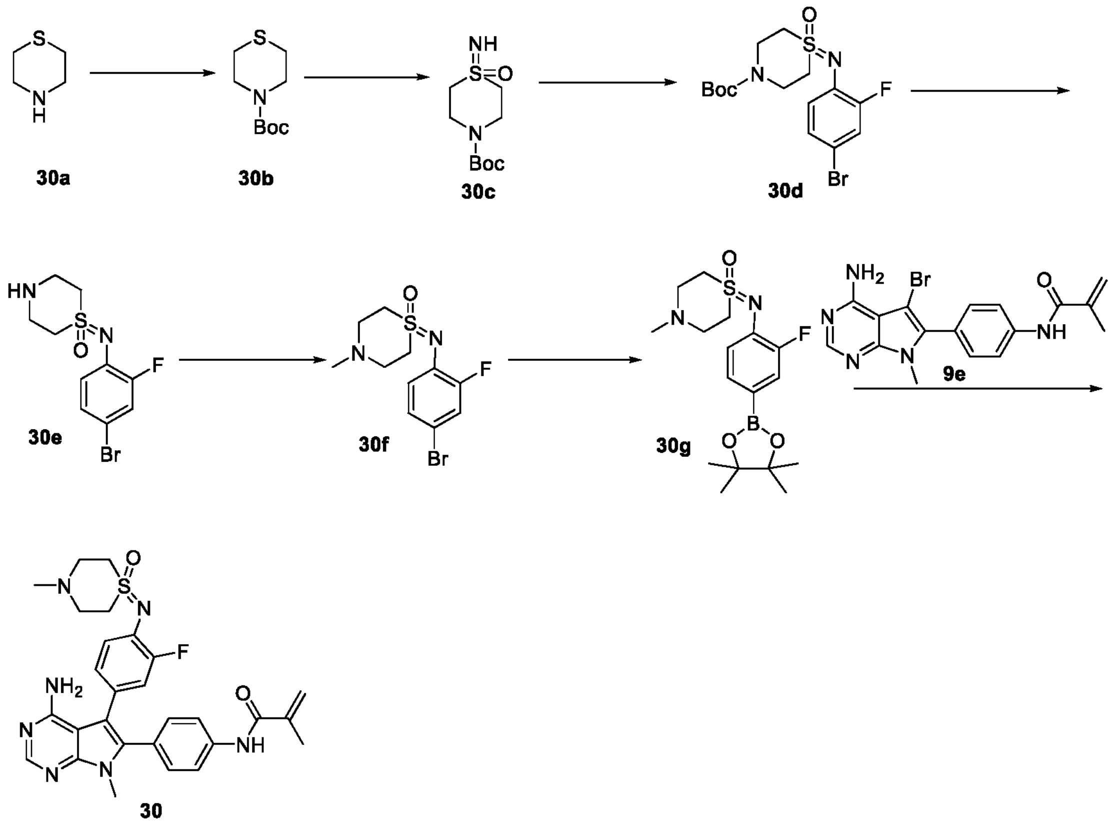

30a 30b 30c 30d 30e 30f 30g 9e 30

**Step 1 thiomorpholine-4-carboxylic acid tert-butyl ester 30b**

To a 250 mL round bottom flask, compound **30a** (3 g, 29.07 mmol), Boc anhydride (6 g, 29.07 mmol), DMAP (350 mg, 2.91 mmol), triethylamine (7 mL, 58.14 mmol), and anhydrous dichloromethane (30 mL) were added in sequence, and reacted at room temperature for 5 h. After TLC monitored the completion of the reaction, it was extracted with dichloromethane (50 mL × 3), and the organic phases were combined, washed with saturated saline solution (50 mL), and concentrated under reduced pressure to obtain a crude product. The crude product was purified by column chromatography (PE/EA=8/1) to obtain compound **30b** (3.5 mg), white solid.

**Step 2 1-imino-1$\lambda^6$-thiomorpholine-4-carboxylic acid tert-butyl ester 1-oxide 30c**

To a 100 mL round bottom flask, compound **30b** (1.00 g, 4.92 mmol), ammonium carbamate (577 mg, 7.38 mmol), iodobenzene acetate (3.33 g, 10.3 mmol), and methanol (40 mL) were added in sequence, and reacted at room temperature for 1 h, after TLC monitored the completion of the reaction, it was concentrated under reduced pressure to obtain a crude product. The crude product was purified by column chromatography (PE/EA=1/2) to obtain compound **30c** (930 mg), white solid.

MS (ESI) *m/z* 235 $[M + H]^+$.

**Step 3 1- (4-bromo-2-fluorophenyl) imino) -1 $\lambda^6$-thiomorpholine-4-carboxylic acid tert-butyl ester 1-oxide 30d**

To a 100 mL round bottom flask, compound **30c** (900 mg, 3.84 mmol), 4-bromo-2-fluoro-1-iodobenzene (930 mg, 3.2 mmol), $Pd_2(dba)_3$ (74 mg, 0.08 mmol), Xantphos (140 mg, 0.24 mmol), and cesium carbonate (1.5 g, 4.60 mmol) were added in sequence, and reacted at 90 °C for 5 h, after TLC monitored the completion of the reaction, it was cooled down to room temperature, filtered through diatomaceous earth, extracted with ethyl acetate (30 mL × 3), and the organic phases were combined, washed with saturated saline solution (30 mL), and concentrated under reduced pressure to obtain a crude product. The crude product was purified by column chromatography (PE/EA=1/1) to obtain compound **30d** (800 mg), light yellow solid.

MS (ESI) *m/z* 407 $[M + H]^+$.

**Step 4 1- ((4-bromo-2-fluorophenyl) imino) -1 $\lambda^6$-thiomorpholine 1-oxide 30e**

To a 100 mL round bottom flask, compound **30d** (400 mg, 1.30 mmol), trifluoroacetic acid (2 mL), and anhydrous dichloromethane (8 mL) were added in sequence, and reacted at room temperature for 1 h, after TLC monitored the completion of the reaction, it was extracted with dichloromethane (30 mL × 3), and the organic phases were combined, washed with saturated saline solution (30 mL), concentrated under reduced pressure to obtain a crude product, dried to obtain compound **30e** (350 mg), light yellow solid, which was directly used in the next step.

MS (ESI) *m/z* 307 $[M + H]^+$.

**Step 5 1- ((4-bromo-2-fluorophenyl) imino) -4-methyl-1 $\lambda^6$-thiomorpholine 1-oxide 30f**

To a 100 mL round bottom flask, compound **30e** (350 mg, 1.15 mmol), polyformaldehyde (71 mg, 2.30 mmol), sodium cyanoborohydride (216 mg, 3.45 mmol) and methanol (10 mL) were added in sequence. After 6 h of reaction at room temperature, the reaction was quenched with sodium bicarbonate and extracted with ethyl acetate (30 mL × 3), and the organic phases were combined and washed with saturated saline solution (30 mL). The crude product was concentrated under reduced pressure and purified by column chromatography (PE/EA=1/1) to obtain compound **30f** (170 mg), white solid.

MS (ESI) *m/z* 321 $[M + H]^+$.

$^1H$ NMR (400 MHz, $CDCl_3$) $\delta$ 7.25 - 7.21 (m, 1H), 7.17 - 7.11 (m, 1H), 3.31 (tdd, *J* = 13.3, 9.2, 4.8 Hz, 1H), 3.02 - 2.92 (m, 1H), 2.44 (s, 3H).

**Step 6 1- ((2-Fluoro-4- (4,4,5,5-tetramethyl-1,3,2-dioxaborolan-2-yl) phenyl) imino) -4-methyl-1 $\lambda^6$-thiomorpholine 1-oxide 30g**

To a 100 mL round bottom flask, compound **30f** (170 mg, 0.53 mmol), bis(pinacolato)diboron (162 mg, 0.64 mmol), $Pd(dppf)Cl_2$ (77 mg, 0.11 mmol), potassium acetate (157 mg, 1.59 mmol) and 1,4-dioxane (10 mL) were added in sequence, and heated up to 90 °C to react for 3 h. After the reaction was completed, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (PE/EA=1/1) to obtain compound **30g** (120 mg), yellow oil.

MS (ESI) *m/z* 369 $[M + H]^+$.

**Step 7 *N-* (4- (4-amino-5- (3-fluoro-4- ((4-methyl-1-oxo-1 $\lambda^6$-thiomorpholine-1-ylidene) amino) phenyl) -7-methyl-7*H*-pyrrolo [2,3-*d*] pyrimidin-6-yl) phenyl) methacrylamide 30**

To a 25 mL round bottom flask, compound **30g** (126 mg, 0.34 mmol), compound **9e** (120 mg, 0.31 mmol), tetrakis (triphenylphosphine) palladium (36 mg, 0.03 mmol), anhydrous potassium phosphate (477 mg, 2.25 mmol), 1,4-dioxane (10 mL), and water (1 mL) were added in sequence, and heated up to 90 °C to react for 1 h. After TLC monitored the

completion of the reaction, it was cooled down to room temperature, filtered through diatomaceous earth, extracted with ethyl acetate (20 mL × 3), and the organic phases were combined, washed with saturated saline (20 mL), and concentrated under reduced pressure to obtain a crude product. The crude product was purified by column chromatography (DCM/MeOH=6/1) to obtain compound 30 (38 mg), white solid.

MS (ESI) *m/z* 548.20 $[M + H]^+$.

$^1H$ NMR (400 MHz, $CDCl_3$) δ 8.37 (s, 1H), 7.62 - 7.55 (m, 3H), 7.23 (dd, *J* = *14.9,* 8.4 Hz, 3H), 6.94 (dd, *J* = 8.3, 4.6 Hz, 2H), 5.83 (s, 1H), 5.52 (s, 1H), 5.20 (s, 2H), 5.20 (s, 4H), 3.73 (s, 3H), 2.45 (s, 4H), 2.10 *(s,* 3H).

**Example 31 *N*- (4- (4-amino-7-methyl-5- (4- (1-oxotetrahydro-1** λ$^6$-thiophene-1-ylidene) **amino) phenyl) -7*H*-pyrrolo [2,3-*d*] pyrimidin-6-yl) phenyl) methacrylamide**

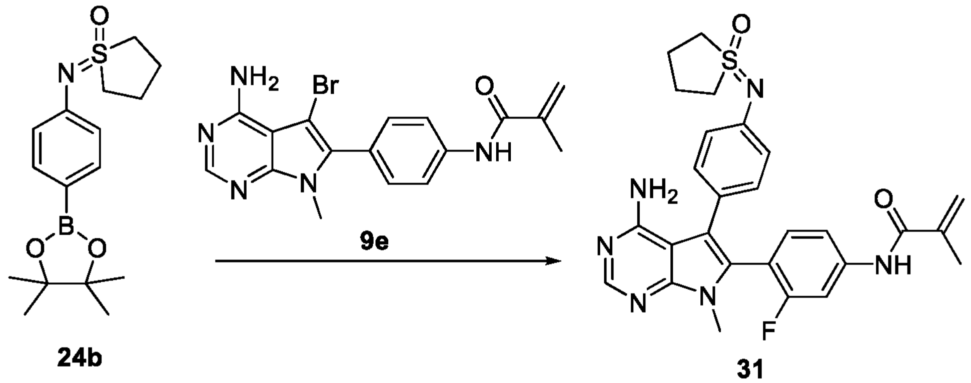


24b

31

To a 25 mL round bottom flask, compound **9e** (150 mg, 0.39 mmol), compound **4b** (137 mg, 0.43 mmol), tetrakis (triphenylphosphine) palladium (50 mg, 0.039 mmol), anhydrous potassium phosphate (200 mg, 0.87 mmol), 1,4-dioxane (10 mL), and water (1 mL) were added in sequence, and heated up to 90 °C to react for 1 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (DCM/MeOH=6/1) to obtain compound **31** (48.3 mg), white solid.

MS (ESI) *m/z* 501.66 $[M + H]^+$.

$^1H$ NMR (400 MHz, $CDCl_3$) δ 8.41 - 8.34 (m, 1H), 7.61 - 7.55 (m, 3H), 7.25 *(d, J* = 8.6 Hz, 2H), 7.14 (d, *J* = 8.3 Hz, 2H), 7.01 (d, *J* = 8.3 Hz, 2H), 5.82 (s, 1H), 5.51 (s, 1H), 5.12 (s, 2H), 3.76 - 3.71 (m, 3H), 3.46 (dd, *J* = 12.5, 6.3 Hz, 2H), 3.25 - 3.19 (m, 2H), 2.39 - 2.27 (m, 4H), 2.08 (d, *J* = 10.1 Hz, 3H).

**Example 32 *N*- (4- (4-amino-5- (4- ((dimethylamino) (oxo) (trifluoromethyl) - λ$^6$-sulfanylidene)amino)phenyl)-7-methyl-7*H*-pyrrolo[2,3-*d*]pyrimidin-6-yl)-3-fluorophenyl) methacrylamide**

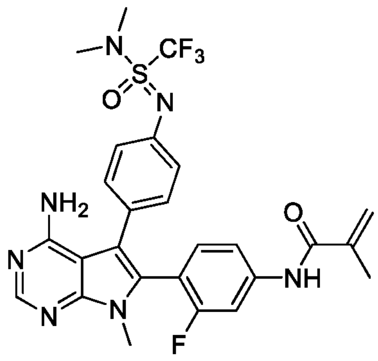

**32**

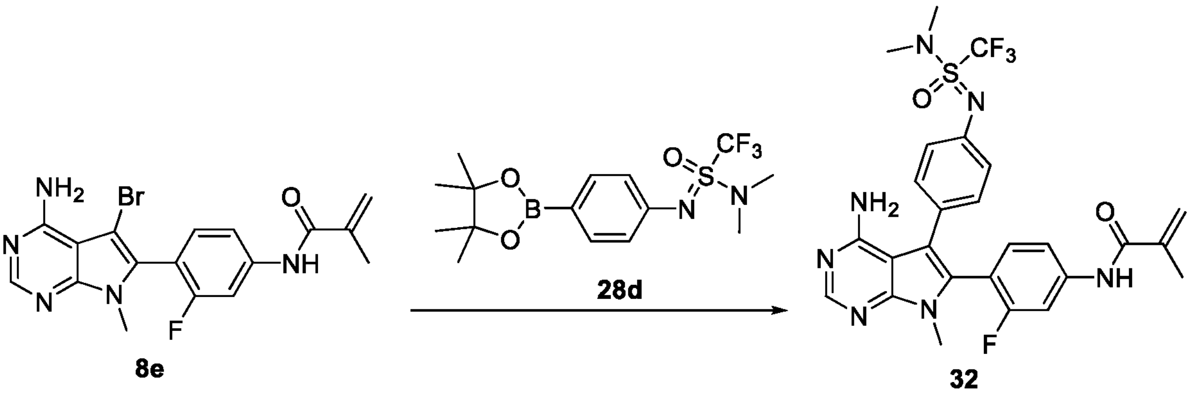


**8e** **32**

To a 25 mL round bottom flask, compound **8e** (100 mg, 0.25 mmol), compound **28d** (104 mg, 0.275 mmol), tetrakis (triphenylphosphine) palladium (29 mg, 0.025 mmol), anhydrous potassium phosphate (118 mg, 0.56 mmol), 1,4-dioxane (10 mL), and water (1 mL) were added in sequence, and heated up to 90 °C to react for 1 h. After TLC monitored the completion of the reaction, it was cooled down to room temperature, filtered through diatomaceous earth, extracted with ethyl acetate (30 mL × 3), the organic phases were combined, washed with saturated saline (30 mL), and concentrated under reduced pressure to obtain a crude product. The crude product was purified by column chromatography (DCM/MeOH=6/1) to obtain compound **32** (6 mg), white solid.

MS (ESI) *m/z* 508.76 $[M + H]^+$.

$^1H$ NMR (400 MHz, $CDCl_3$) $\delta$ 8.40 (s, 1H), 7.79 (dd, *J* = 25.0, 9.0 Hz, 2H), 7.19 *(s,* 2H), 7.07 (s, 4H), 5.84 (s, 1H), 5.56 (s, 1H), 5.15 (s, 2H), 3.70 (s, 3H), 3.12 (s, 7H), 2.11 (s, 4H).

**Example 33 *N*- (4- (4-amino-5- (3-fluoro-4- ((1-oxotetrahydro-1 λ$^6$-thiophene-1-ylidene) amino) phenyl) -7-methyl-7*H*-pyrrolo [2,3-*d*] pyrimidin-6-yl) phenyl) methacrylamide**

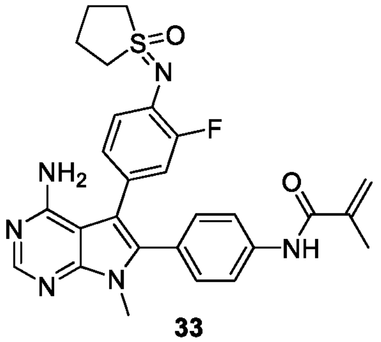

**33**

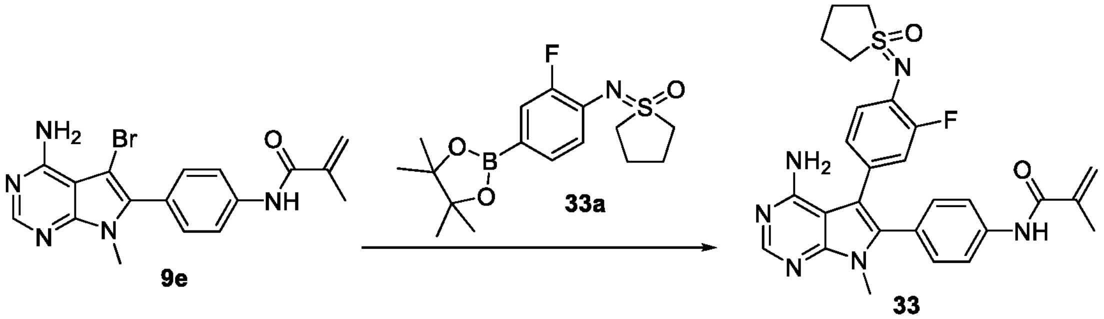

**9e** **33a** **33**

Compound **33** was synthesized with reference to Example **32.**

MS (ESI) *m/z* 519.70 [M + H]+.

$^1$H NMR (400 MHz, $CDCl_3$) δ 8.28 (s, 1H), 7.51 (s, 1H), 7.49 (d, *J* = 3.2 Hz, *2H),* 7.16 (s, 1H), 7.14 (s, 1H), 7.04 (s, 1H), 6.87 - 6.82 (m, 2H), 5.74 (s, 1H), 5.43 (d, *J* = 1.0 Hz, 1H), *5.08* (s, 2H), 3.63 (s, 3H), 3.41 - 3.34 (m, 2H), 3.17 (dd, *J* = 13.0, 6.9 Hz, 2H), 2.25 (d, *J* = 7.5 Hz, 4H), 2.01 (s, 3H).

**Example 34 *N*- (4- (4-amino-7-methyl-5- (3-methyl-4- ((1-oxotetrahydro-2*H*-1λ$^6$-thiopyran-1-ylidene) amino) phenyl) -7*H*-pyrrolo [2,3-*d*] pyrimidin-6-yl) phenyl) methacrylamide**

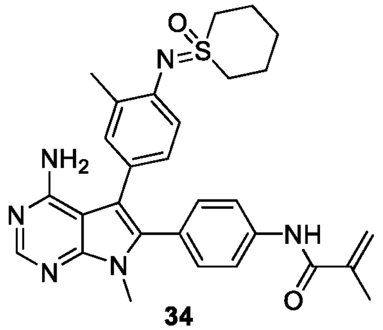

**34**

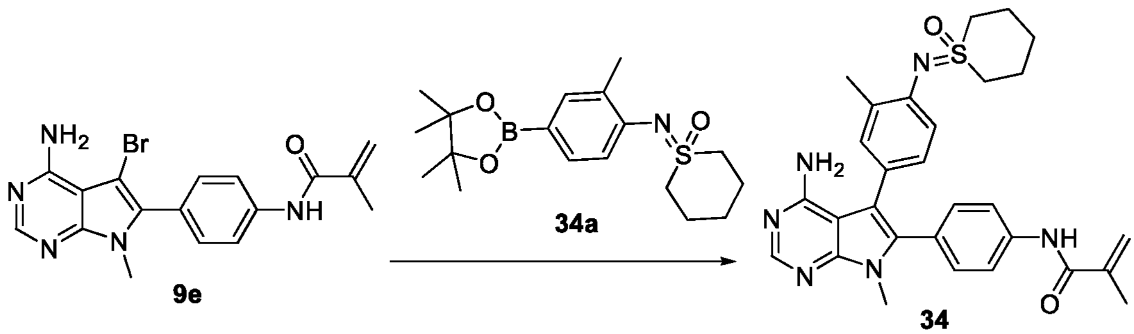

**9e** **34a** **34**

Compound **34** was synthesized with reference to Example 32.

MS (ESI) *m/z* 529.08 [M + H]+.

$^1H$ NMR (400 MHz, $CDCl_3$) $\delta$ 8.35 (s, 1H), 7.57 (d, *J* = 8.6 Hz, 3H), 7.26 (d, *J* = 8.5 Hz, 2H), 7.14 (d, *J* = 8.1 Hz, 1H), 7.07 (s, 1H), *6.91* (d, *J* = 8.0 Hz, 1H), 5.83 (s, 1H), 5.52 (s, 1H), 5.37 (s, 2H), 3.73 (s, 3H), 3.41 (d, *J* = 13.1 Hz, 2H), 3.13 (dd, *J* = 17.1, 10.5 *Hz,* 2H), 2.27 (d, *J* = 7.5 Hz, 3H), 2.11 (d, *J* = 5.5 Hz, 7H), 1.79 (d, *J* = 5.2 Hz, 3H).

**Example 35 *N*- (4- (4-amino-5- (3-fluoro-4- ((1-oxotetrahydro-2H-1 $\lambda^6$-thiopyran -1-ylidene) amino) phenyl) -7-methyl-7*H*-pyrrolo [2,3-*d*] pyrimidin-6-yl) phenyl) but-2-ynamide**

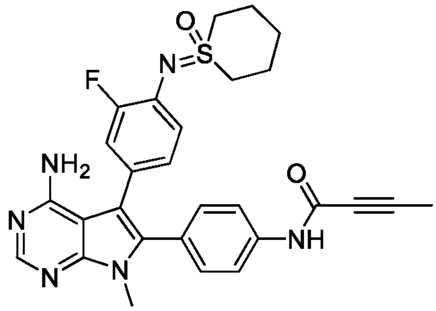

**35**

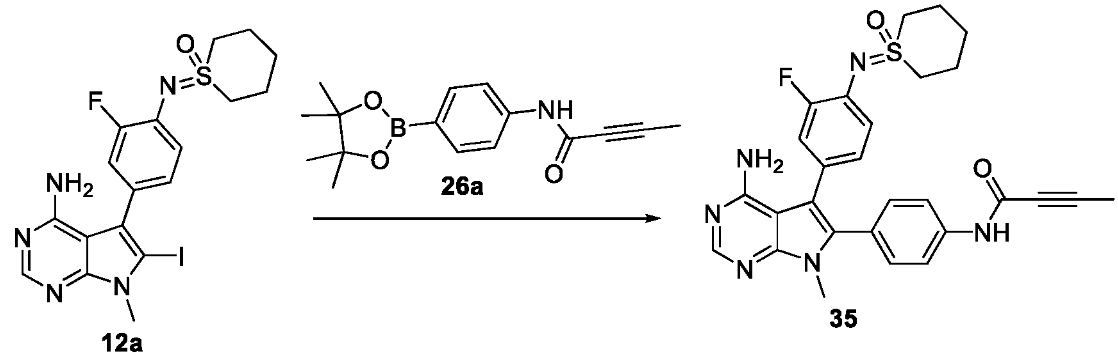


**12a** **35**

Compound **12a** (0.10 g, 0.20 mmol) was dissolved in 1,4-dioxane (6 mL) and $H_2O$ (2 mL), compound **26a** (68.0 mg, 0.24 mmol), potassium phosphate (0.13 g, 0.60 mmol), and tetrakis (triphenylphosphine) palladium (23.0 mg, 0.02 mmol) were added, and reacted at 90 °C for 14 h under $N_2$ protection. After the reaction was completed, it was extracted with ethyl acetate (50 mL × 3), and the organic phases were combined and concentrated under reduced pressure to obtain a crude product. The crude product was purified by preparative TLC plate (EA/THF=3/1) to obtain compound **35** (10.5 mg), white solid.

MS (ESI) *m/z* 531.20 $[M + H]^+$.

$^1H$ NMR (400 MHz, $CDCl_3$) $\delta$ 8.37 (s, 1H), 7.57 - 7.43 (m, 3H), 7.26 - 7.20 (m, *2H),* 6.92 (dd, *J* = 12.6, 5.0 Hz, 2H), 5.23 (s, 2H), 3.72 (s, 3H), 3.36 (d, *J* = 14.3 Hz, 2H), 3.23 - 3.12 (m, 2H), 2.26 - 2.09 (m, 4H), 2.01 (s, 3H), 1.73 (m, 2H).

**Example 36 *N*- (4- (4-amino-5- (3-fluoro-5- ((1-oxotetrahydro-2H-1 $\lambda^6$-thiopyran-1-ylidene) amino) pyridin-2-yl) -7-methyl-7*H*-pyrrole [2,3-*d*] pyrimidin-6-yl) phenyl) methacrylamide**

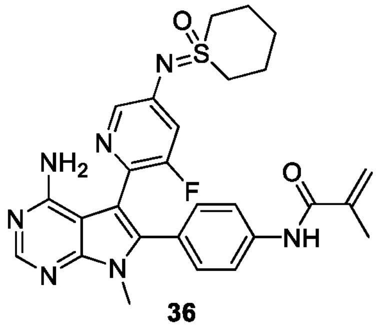

36

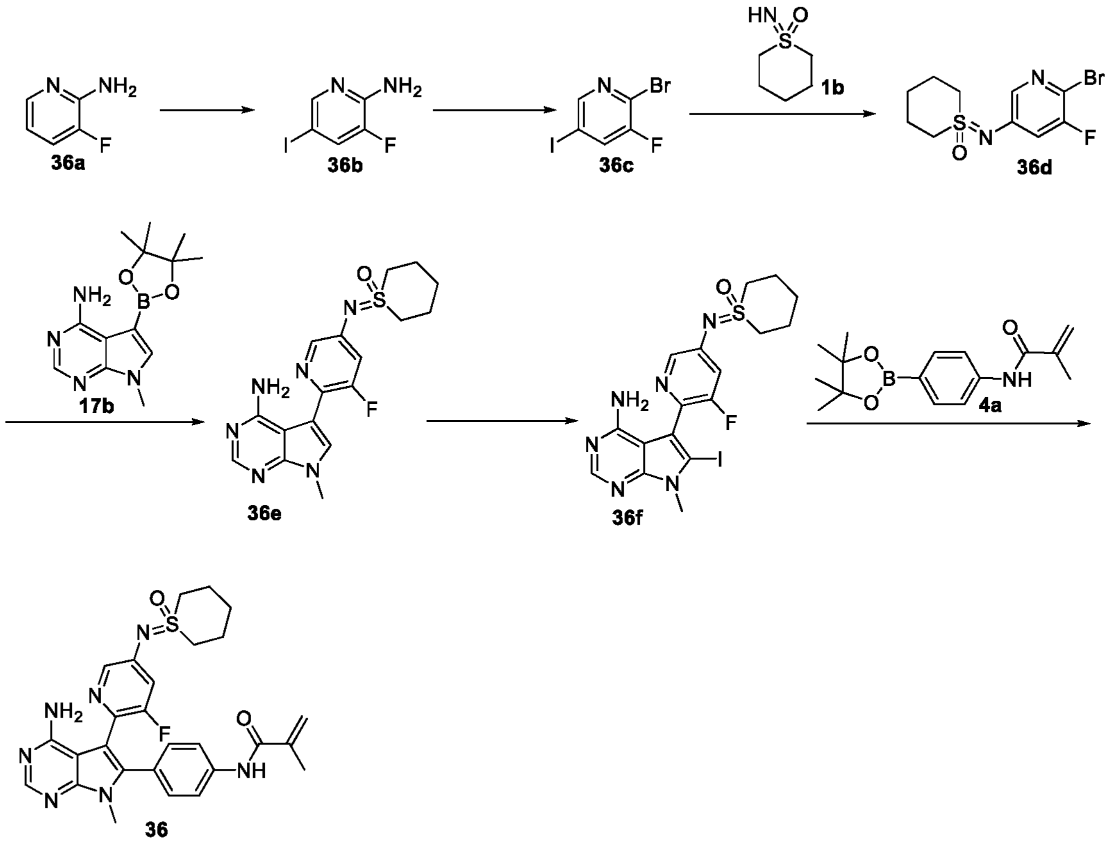

36a 36b 36c 1b 36d 17b 36e 36f 4a 36

**Step 1 3-fluoro-5-iodo-pyridine-2-amine**

Compound **36a** (3.00 g, 26.8 mmol) was dissolved in DMF (20 mL), N-iodosuccinimide (6.63 g, 29.5 mmol) was added at room temperature, and the reaction was carried out at room temperature for 14 h. After the reaction was completed, it was extracted with ethyl acetate (50 mL × 3), and the organic phases were combined and concentrated under reduced pressure to obtain a crude product. The crude product was purified by column chromatography (PE/EtOAc=1/1) to obtain compound **36b** (4.5 g), white solid.

MS (ESI) *m/z* 239.10 $[M + H]^+$.

**Step 2 3-fluoro-5-iodo-pyridine-2-amine 36c**

Compound **6b** (2.00 g, 8.40 mmol) was dissolved in acetonitrile (20 mL), copper bromide (2.25 g, 10.1 mmol) was added at room temperature, and isoamyl nitrite (1.47 g, 12.6 mmol) was added slowly at 50 °C to react at that temperature for 3 h. After the reaction was completed, it was extracted with ethyl acetate (50 mL × 3), and the organic phases were combined and concentrated under reduced pressure to obtain a crude product. The crude product was purified by column chromatography (PE/EtOAc=5/1) to obtain compound **36c** (1.5 g), white solid.

MS (ESI) *m/z* 302.20 $[M + H]^+$.

**Step 3 1- ((6-bromo-5-fluoropyridin-3-yl) imino) hexahydro-1 λ$^6$-thiopyran-1-oxide 36d**

Compound **36c** (0.50 g, 1.66 mmol) was dissolved in 1,4-dioxane (10 mL), compound **1b** (0.22 g, 1.66 mmol), $Cs_2CO_3$ (1.62 g, 4.98 mmol), Xantphos (0.19 g, 0.332 mmol), and $Pd_2(dba)_3$ (0.15 g, 0.166 mmol) were added at room temperature, and it was protected with $N_2$ and reacted at 100 °C for 14 h. After TLC monitored the completion of the reaction, it was extracted with ethyl acetate (50 mL × 3), and the organic phases were combined and concentrated under reduced pressure to obtain a crude product. The crude product was purified by column chromatography (PE/EtOAc=1/1) to obtain compound **36d** (0.25 g), white solid.
MS (ESI) *m*/*z* 307.20 $[M + H]^+$.

**Step 4 1-(6-(4-amino-7-methyl-7*H*-pyrrolo[2,3-*d*]pyrimidin-5-yl)-5-fluoropyridine -3-yl) imino) hexahydro-1 λ$^6$-thiopyran-1-oxide 36e**

Compound **17b** (0.20 g, 0.73 mmol) was dissolved in 1,4-dioxane (6 mL) and $H_2O$ (2 mL), compound **36d** (0.25 g, 0.80 mmol), potassium phosphate (0.46 g, 2.20 mmol), and tetrakis (triphenylphosphine) palladium (84.0 mg, 0.073 mmol) were added, and it was protected with $N_2$ and reacted at 90 °C for 14 h. After the reaction was completed, it was extracted with ethyl acetate (50 mL × 3), and the organic phases were combined and concentrated under reduced pressure to obtain a crude product. The crude product was purified by column chromatography (DCM/MeOH=10/1) to obtain compound **36e** (180 mg), white solid.
MS (ESI) *m*/*z* 375.10 $[M + H]^+$.

**Step 5 1-(6-(4-amino-6-iodo-7-methyl-7*H*-pyrrolo[2,3-*d*]pyrimidin-5-yl)-5-fluoropyridine -3-yl) imino) hexahydro-1 λ$^6$-thiopyran-1-oxide 36f**

Compound **36e** (0.15 g, 0.40 mmol) was dissolved in dichloromethane (10 mL), and it was cooled down to 0 °C, and at this temperature, trifluoroacetic acid (0.14 g, 1.2 mmol) and N-iodobutylimide (110 mg, 0.48 mmol) were added to react at 0 °C for 1 h. After the reaction was completed, it was extracted with ethyl acetate (50 mL × 3), and the organic phases were combined and concentrated under reduced pressure to obtain crude product compound **36f** (180 mg), white solid.
MS (ESI) *m*/*z* 501.11 $[M + H]^+$.

**Step 6 *N*- (4- (4-amino-5- (3-fluoro-5- ((1-oxotetrahydro-2H-1λ$^6$-thiopyran-1- ylidene) amino) pyridine-2-yl) -7-methyl-7*H*-pyrrole [2,3-*d*] pyrimidin-6-yl) phenyl) methacrylamide 36**

Compound **36f** (0.18 g, 0.36 mmol) was dissolved in dioxane (6 mL) and $H_2O$ (2 mL), compound **4a** (124 mg, 0.43 mmol), potassium phosphate (0.23 g, 1.10 mmol), and tetrakis (triphenylphosphine) palladium (41.0 mg, 0.036 mmol) were added, and reacted at 90 °C for 14 h under $N_2$ protection. After the reaction was completed, it was extracted with ethyl acetate (50 mL × 3), and the organic phases were combined and concentrated under reduced pressure to obtain a crude product. The crude product was purified by preparative TLC plate (EtOAc/THF=3/1) to obtain compound **36** (78.3 mg), white solid.
MS (ESI) *m*/*z* 534.21 $[M + H]^+$.
$^1$H NMR (400 MHz, $CDCl_3$) δ 8.38 (s, 1H), 8.33 - 8.29 (m, 1H), 7.70 - 7.55 (m, *4H),* 7.30 (s, 1H), 7.00 (dt, *J* = 11.5, 4.0 Hz, 1H), 6.30 (s, 2H), 5.84 (s, 1H), 5.52 (d, *J* = 1.1 Hz, 1H), 3.74 (d, *J* = 3.4 Hz, 4H), 3.40 - 3.33 (m, 3H), 3.24 - 3.16 (m, 3H), 2.19 - 2.09 (m, 9H), 1.83 - 1.76 (m, 3H).

**Example 37 *N*- (4- (4-amino-5- (3-fluoro-4- ((1-oxotetrahydro-2H-1 λ$^6$-thiopyran-1-ylidene) amino) phenyl) -7-methyl-7*H*-pyrrolo [2,3-*d*] pyrimidin-6-yl) -2-fluorophenyl) methacrylamide**

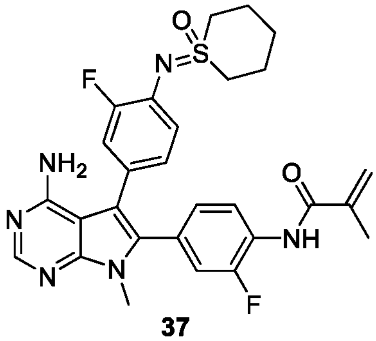

37

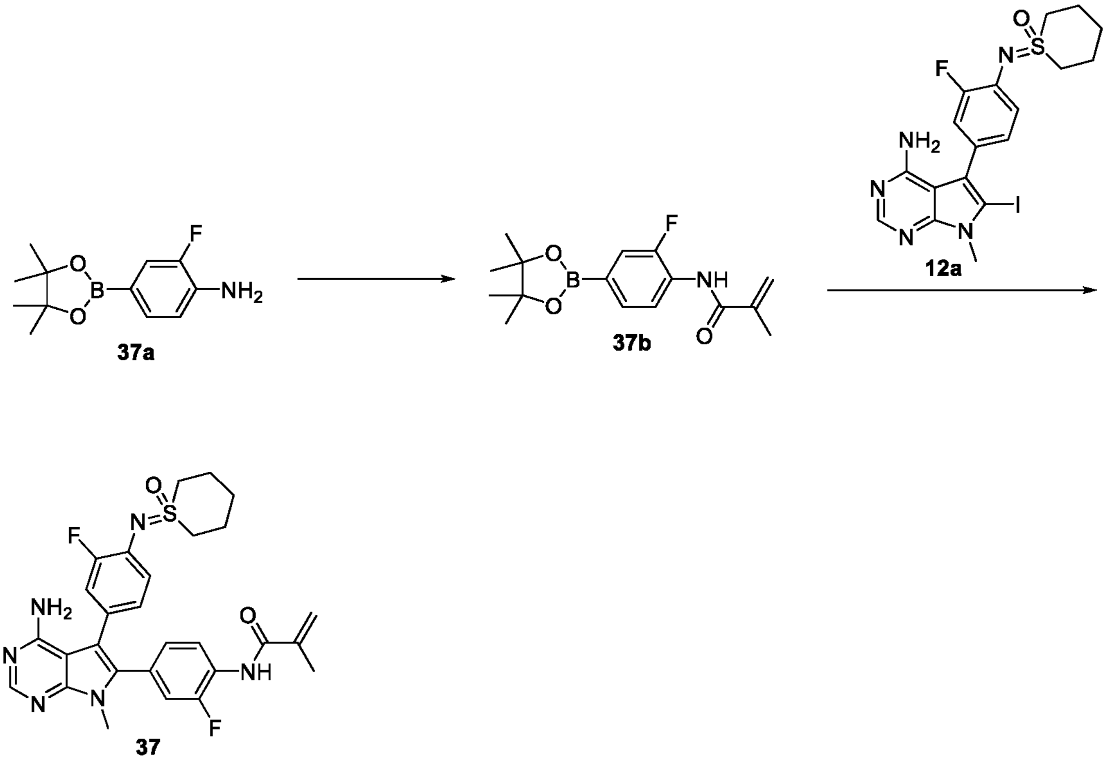

37a

37b

12a

37

**Step 1 *N*- (2-fluoro-4- (4,4,5,5-tetramethyl-1,3,2-dioxaborolan-2-yl) phenyl) methacrylamide 37b**

Compound **37a** (1.00 g, 4.22 mmol) was dissolved in dichloromethane (20 mL), triethylamine (0.85 g, 8.44 mmol) was added at room temperature, cooled down to 0 °C, methacryloyl chloride (0.46 g, 4.33 mmol) was added to react at room temperature for 1 h. After the reaction was completed, it was extracted with ethyl acetate (50 mL × 3), and the organic phases were combined and concentrated under reduced pressure to obtain a crude product. The crude product was purified by column chromatography (PE/EtOAc=5/1) to obtain compound **37b** (0.95 g), colorless oily liquid.

MS (ESI) *m/z* 306.20 [M + H]$^+$.

$^1$H NMR (400 MHz, $CDCl_3$) $\delta$ 8.62 - 8.23 (m, 1H), 8.04 - 7.75 (m, 1H), 7.52 (ddd, *J* = 25.4, 13.8, 11.0 Hz, 2H), 6.02 - 5.58 (m, 1H), 5.59 (s, 1H), 2.07 (s, 3H), 1.33 (s, 12H).

**Step 2 *N*- (4- (4-amino-5- (3-fluoro-4- ((1-oxotetrahydro-2H-1 λ$^6$-thiopyran-1- ylidene) amino) phenyl) -7-methyl-7*H*-pyrrolo [2,3-*d*] pyrimidin-6-yl) -2-fluorophenyl) methacrylamide 37**

Compound **12a** (0.10 g, 0.20 mmol) was dissolved in 1,4-dioxane (6 mL) and $H_2O$ (2 mL), compound **37b** (73.0 mg, 0.24 mmol), potassium phosphate (0.13 g, 0.60 mmol), and tetrakis (triphenylphosphine) palladium (23.0 mg, 0.02 mmol) were added, and reacted at 90 °C for 14 h under $N_2$ protection. After the reaction was completed, it was extracted with ethyl acetate (50 mL × 3), and the organic phases were combined and concentrated under reduced pressure to obtain a crude product. The crude product was purified by preparative TLC plate (EtOAc/THF=3/1) to obtain compound **37** (36.3 mg)

MS (ESI) *m/z* 551.10 [M + H]$^+$.

$^1H$ NMR (400 MHz, $CDCl_3$) $\delta$ 8.46 (t, *J* = 8.4 Hz, 1H), 8.38 (s, 1H), 7.82 (s, 1H), 7.25 (t, *J = 8.5* Hz, 1H), 7.11 (d, *J* = 8.9 Hz, 1H), 6.99 (dd, *J* = 11.6, 1.9 Hz, 1H), 6.94 (dd, *J* = 7.1, 5.0 Hz, 2H), 5.89 (s, 1H), 5.56 (d, *J* = 1.3 *Hz,* 1H), 5.27 (s, 2H), 3.77 (d, *J* = 20.7 Hz, 3H), 3.37 (d, *J* = 14.5 Hz, 2H), 3.19 (ddd, *J* = 13.3, 8.9, 4.2 Hz, 2H), 2.23 - *2.07 (m,* 6H), 1.84 - 1.73 (m, 3H).

**Example 38 *N*-(5- (4-amino-5- (3-fluoro-4- ((1-oxotetrahydro-2H-1 $\lambda^6$-thiopyran-1-ylidene) amino) phenyl) -7-methyl-7*H*-pyrrolo [2,3-*d*] pyrimidin-6-yl) -2-fluorophenyl) methacrylamide KTH021115**

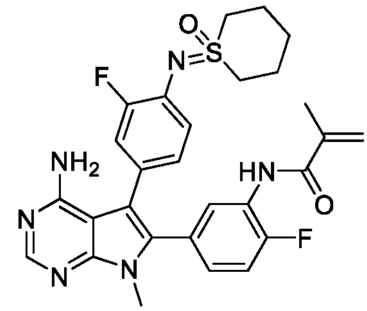

**38**

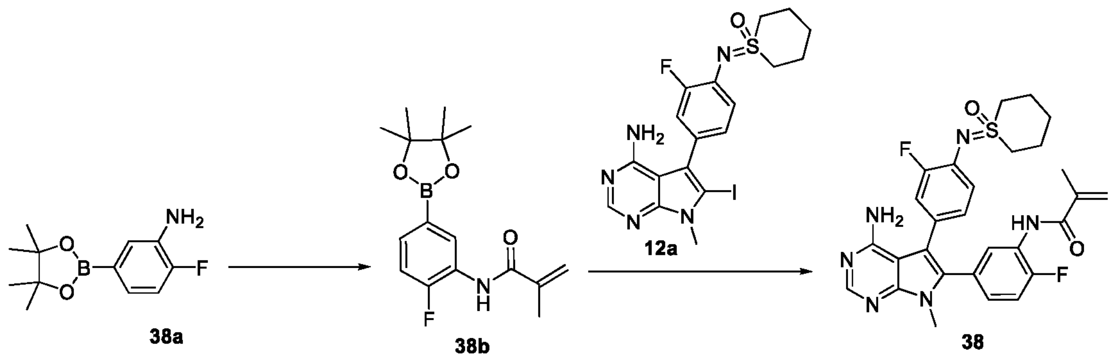

12a

38a 38b 38

**Step 1 *N-* (2-fluoro-5- (4,4,5,5-tetramethyl-1,3,2-dioxaborolan-2-yl) phenyl) methacrylamide**

Compound **38a** (1.00 g, 4.22 mmol) was dissolved in dichloromethane (20 mL), triethylamine (0.85 g, 8.44 mmol) was added at room temperature, cooled down to 0 °C, methacryloyl chloride (0.46 g, 4.33 mmol) was added to react at room temperature for 1 h. After the reaction was completed, it was extracted with ethyl acetate (50 mL × 3), and the organic phases were combined and concentrated under reduced pressure to obtain a crude product. The crude product was purified by column chromatography (PE/EtOAc=5/1) to obtain compound **38b** (1.1 g), colorless oily liquid.

MS (ESI) *m/z* 306.20 $[M + H]^+$.

$^1H$ NMR (400 MHz, $CDCl_3$) $\delta$ 8.74 (dd, *J* = 8.5, 1.3 Hz, 1H), 7.72 (s, 1H), 7.61 - 7.49 (m, 1H), 7.16 - 7.06 *(m,* 1H), 5.84 (d, *J* = 19.4 Hz, 1H), 5.57 - 5.47 (m, 1H), 2.10 (s, 3H), 1.34 (s, 14H).

**Step 2 *N-* (5- (4-amino-5- (3-fluoro-4- ((1-oxotetrahydro-2H-1$\lambda^6$-thiopyran-1- ylidene) amino) phenyl) -7-methyl-7*H*-pyrrolo [2,3-*d*] pyrimidin-6-yl) -2-fluorophenyl) methacrylamide 38**

Compound **12a** (0.10 g, 0.20 mmol) was dissolved in 1,4-dioxane (6 mL) and $H_2O$ (2 mL), compound **38b** (73.0 mg, 0.24 mmol), potassium phosphate (0.13 g, 0.60 mmol), and tetrakis (triphenylphosphine) palladium (23.0 mg, 0.02 mmol) were added, and reacted at 90 °C for 14 h under N2 protection. After the reaction was completed, it was extracted with ethyl acetate (50 mL × 3), and the organic phases were combined and concentrated under reduced pressure to obtain a crude product. The crude product was purified by preparative TLC plate (EA/THF=3/1) to obtain compound **38** (37.3 mg).

MS (ESI) *m/z* 551.10 $[M + H]^+$.

$^1H$ NMR (400 MHz, $CDCl_3$) $\delta$ 8.45 (dd, *J* = 7.5, 2.1 Hz, 1H), 8.37 (d, *J = 5.4* Hz, 1H), 7.80 (s, 1H), 7.23 (t, *J* = 8.6 Hz, 1H), 7.07 (dd, *J* = 10.7, 8.5 Hz, 1H), 6.98 - 6.92 (m, 2H), *6.91* - 6.86 (m, 1H), 5.88 (s, 1H), 5.56 (t, *J* = 4.8 Hz, 1H), 5.14 (s, 2H), 3.76 (s, 3H), 3.41 - 3.31 (m, 2H), 3.17 (ddd, *J* = 13.5, 9.0, 4.3 Hz, 2H), 2.20 - 2.06 (m, 7H), 1.77 (d, *J* = 15.1 Hz, 2H).

**Example 39 *N*- (3- (4-amino-5- (4- ((dimethylamino) (oxo) (trifluoromethyl) - λ⁶-sulfanylidene) amino) phenyl) -7-methyl-7*H*-pyrrole [2,3-*d*] pyrimidin-6-yl) -4- fluorophenyl) methacrylamide**

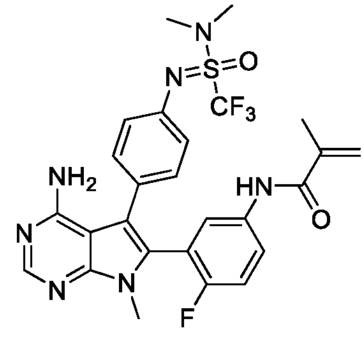

**39**

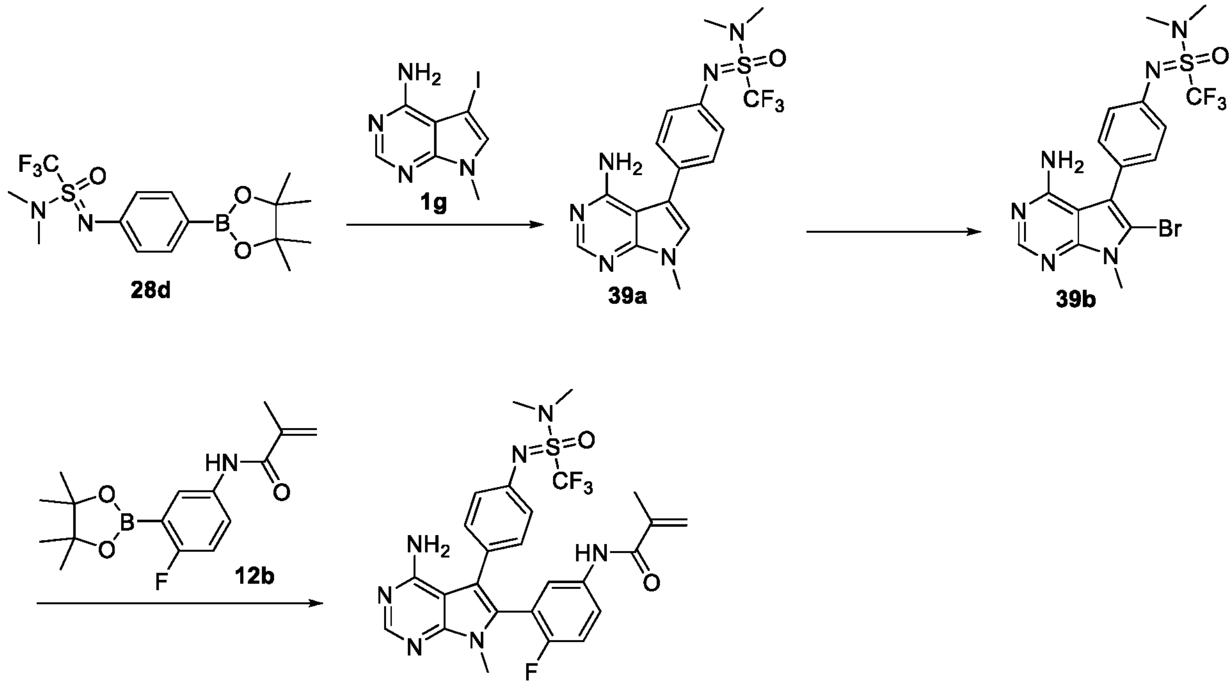

28d 1g 39a 39b 12b

**39**

**Step 1 N'- (4-(4-amino-7-methyl-7*H*-pyrrole [2,3-*d*] pyrimidin-5-yl) phenyl) -1,1,1-trifluoro-N, N-dimethylmethane sulfonamide 39a**

Compound **28** (500 mg, 1.3 mmol), compound **1g** (302 mg, 1.1 mmol), Pd(dppf)$Cl_2$ (81 mg, 0.11 mmol), and potassium phosphate (584 mg, 2.8 mmol) were placed in a bottle, and 1,4-dioxane/$H_2O$ (10 mL/1 mL) was added. After replaced with $N_2$ three times, the reaction was carried out at 85 °C for 16 h. The reaction solution was filtered and concentrated, and the obtained crude product was subjected to column chromatography (DCM/MeOH=0-10%) to obtain compound **39a** (200 mg), brown solid.

MS (ESI) *m*/*z* 399 $[M + H]^+$.

**Step 2 N'-(4-(4-amino-6-bromo-7-methyl- 7*H*-pyrrole[2,3-*d*]pyrimidin-5-yl) phenyl) -1,1,1-trifluoro-N,N-dimethylmethane sulfonamide 39b**

Compound **39a** (100mg, 0.25 mmol) was dissolved in DMF (3 mL), after it was cooled down to 0 °C, N-bromosuccinimide (49 mg, 0.28 mmol) was added to react at 0 °C for 1 h. The reaction solution was diluted with DCM,

washed with water three times, and the organic phase was dried over anhydrous sodium sulfate and concentrated, the obtained crude product was purified by column chromatography (DCM/MeOH=0-10%) to obtain compound **39b** (100mg), red solid.

MS (ESI) *m/z* 476 [M + H]+.

**Step 3 *N*- (3- (4-amino-5- (4- ((dimethylamino) (oxo) (trifluoromethyl) - λ⁶-sulfanylidene) amino) phenyl) -7-methyl-7*H*-pyrrole [2,3-*d*] pyrimidin-6-yl) -4- fluorophenyl) methacrylamide 39**

Compound **39b** (100 mg, 0.21 mmol), compound **12b** (83 mg, 0.27 mmol), $Pd(PPh_3)_4$ (49 mg, 0.042 mmol), and potassium phosphate (133 mg, 0.63 mmol) were placed in a bottle, and 1,4-dioxane/$H_2O$ (4 mL/0.8 mL) was added. After replaced with $N_2$ three times, the reaction was carried out at 100 °C for 16 h. The reaction solution was filtered and concentrated, and the obtained crude product was separated by column chromatography (DCM/MeOH=10/1) to obtain compound **39** (28 mg), light yellow solid.

MS (ESI) *m/z* 576 [M + H]+.

$^1H$ NMR (400 MHz, DMSO-$d_6$) $\delta$ 9.93 (s, 1H), 8.27 (s, 1H), 7.87 - 7.80 (m, 1H), 7.71 (dd, *J* = 6.6, 2.6 Hz, 1H), 7.32 (t, *J* = 9.2 Hz, 1H), 7.21 (d, *J* = 8.3 Hz, 2H), 7.05 (d, *J* = 8.4 Hz, 2H), 5.82 (s, 1H), 5.57 (s, 1H), 3.60 (s, 3H), 3.08 (s, 6H), 1.98 (s, 3H), 1.30 (s, 2H).

**Example 40 *N*-(4-(4-amino-5-(3-chloro-4-((1-oxotetrahydro-2*H*-1λ⁶ - thiopyran -1-ylidene) amino) phenyl) -7-methyl-7*H*-pyrrolo [2,3-*d*] pyrimidin-6-yl) phenyl) methacrylamide**

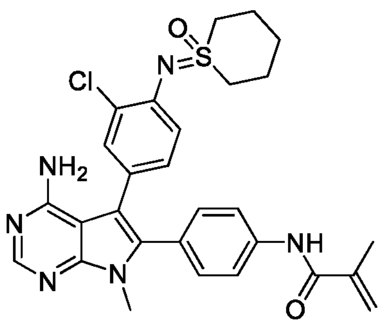

**40**

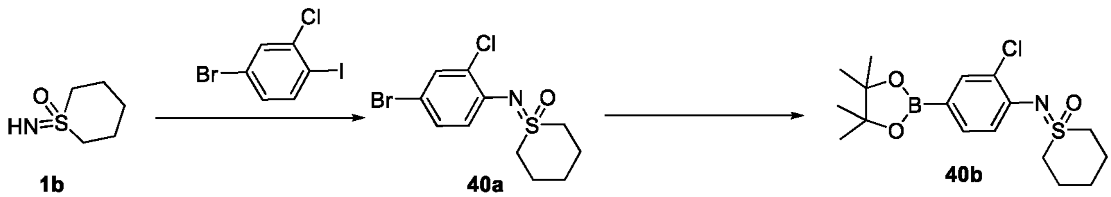

1b

40a

40b

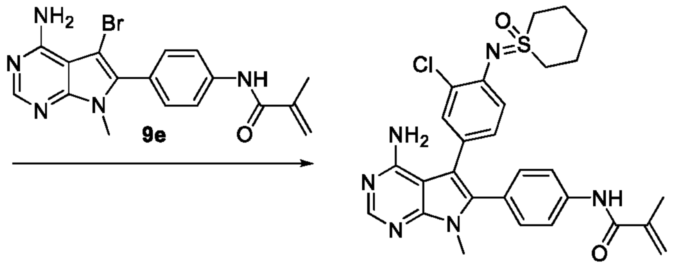

9e

**40**

**Step 1 1- ((4-bromo-2-chlorophenyl) imino) hexahydro-1 λ⁶ - thiopyran-1-oxide**

Compound **1b** (0.25 g, 1.9 mmol), cesium carbonate (0.72 g, 2.2 mmol), 4-bromo-2-chloro-1-iodobenzene (0.5 g, 1.58 mmol), $Pd_2(dba)_3$ (36 mg, 0.0395 mmol), and Xantphos (68 mg, 0.12 mmol) were added to 1,4-dioxane (8 mL), after

replaced with $N_2$ three times, the reaction solution was heated up to 100 °C for 5 h. The reaction solution was filtered through diatomaceous earth, and the filter cake was washed with ethyl acetate (30 mL) and saturated saline solution (50 mL). The organic phase was dried over anhydrous sodium sulfate and concentrated under reduced pressure to obtain a crude product. The crude product was purified by column chromatography (PE/EtOAc=4/1 to 3/2) to obtain compound **40a** (0.43 g), light yellow solid.

MS (ESI) *m/z* 321.90[M + H]$^+$.

**Step 2 1- ((2-chloro-4- (4,4,5,5-tetramethyl-1,3,2-dioxaborolan-2-yl) phenyl) imino) hexahydro-1 λ $^6$ - thiophene 1-oxide 40a**

Compound **40a** (0.43 g, 1.33 mmol), bis(pinacolato)diboron (0.41g, 1.6 mmol), Pd(dppf)$Cl_2$ (200 mg, 0.266 mmol), potassium acetate (390 mg, 3.99 mmol), and 1,4-dioxane (8 mL) were added into a sealed tube, after replaced with $N_2$ three times, sealed, the reaction solution was heated up to 100 °C and stirred for 16 h. Then water (10 mL) was added and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=5/1) to obtain compound 40b (450 mg), yellow oily liquid.

MS (ESI) *m/z* 369[M + H]$^+$.

**Step 3 *N*- (4- (4-amino-5- (3-chloro-4- (1-oxotetrahydro-2*H*-1λ$^6$ - thiopyran-1- ylidene) amino) phenyl) -7-methyl-7*H*-pyrrolo [2,3-*d*] pyrimidin-6-yl) phenyl) methacrylamide 40**

Compound **40b** (115 mg, 0.31 mmol), compound **9e** (100 mg, 0.26 mmol), Pd($PPh_3$)$_4$ (60 mg, 0.052 mmol), and $K_3PO_4$ (165 mg, 0.78 mmol) were added into a sealed tube, after replaced with $N_2$ three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/20) to obtain compound **40** (100 mg), white solid.

MS (ESI) *m/z* 549.09 [M + H]$^+$.

$^1$H NMR (400 MHz, $CDCl_3$) $\delta$ 8.35 (s, 1H), 7.61 - 7.47 (m, 3H), 7.26 - 7.16 (m, 3H), 6.98 - 6.90 (m, 1H), 5.81 (s, 1H), 5.50 (d, *J* = 1.7 Hz, 1H), 5.40 - 5.00 (m, 3H), 3.70 (s, 3H), 3.40 (d, *J* = 13.6 Hz, 2H), 3.18 - 3.03 (m, 2H), 2.26 - 2.12 (m, 3H), 2.05 (s, 3H), 2.04 - 2.00 (m, 1H), 1.89 - 1.75 (m, 2H).

**Example 41 *N*- (4- (4-amino-5- (3-fluoro-4- (4-oxo-1,4λ$^6$ - thiohexane-4-ylidene) amino) phenyl) -7-methyl-7*H*-pyrrolo [2,3-*d*] pyrimidin-6-yl) phenyl) methacrylamide**

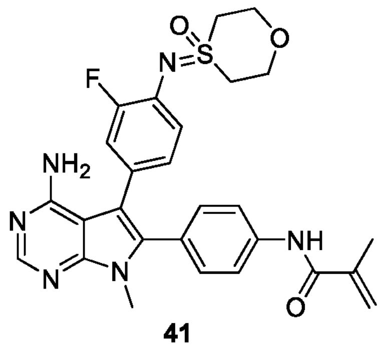

**41**

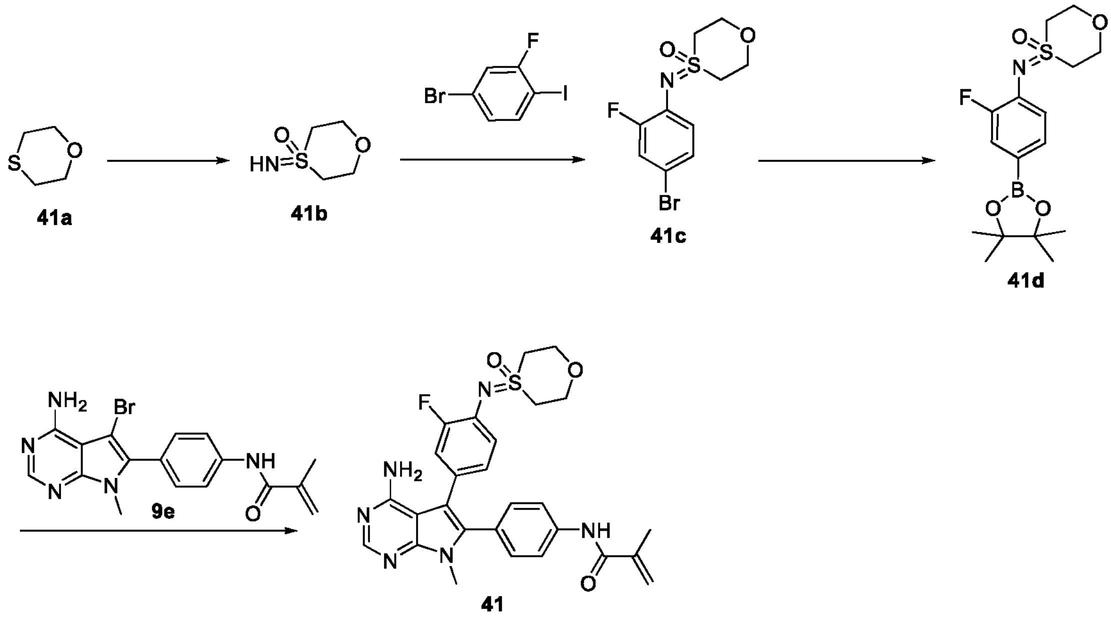

41a 41b 41c 41d

9e 41

**Step 1 4-imino-1,4 λ 6 - oxythiazole 4-oxide 41b**

1,4-thiazole **41a** (2.0 g, 19.2 mmol) and ammonium carbamate (2.24 g, 28.8 mmol) were added into a flask in sequence, then methanol (40 mL) and iodobenzene diethyl ester (13.9 g, 43.2 mmol) were added. The reaction solution was stirred open at room temperature for 30 min, and then concentrated under reduced pressure to remove the solvent to obtain a crude product. The crude product was purified by column chromatography (MeOH/DCM =1/20) to obtain compound **41b** (1.9 g), white solid.

MS (ESI) *m/z* 136.04 $[M + H]^+$.

**Step 2 4- ((4-bromo-2-fluorophenyl) imino) -1,4-λ 6 - thiohexane-4-oxide 41c**

Compound **41b** (700 mg, **5**.17 mmol), cesium carbonate (1.9 g, 6.03 mmol), 4-bromo-2-fluoro-1-iodobenzene (1.28 g, 4.31 mmol), $Pd_2(dba)_3$ (99 mg, 0.108 mmol), and Xantphos (190 mg, 0.32 mmol) were added to 1,4-dioxane (15 mL), after replaced with $N_2$ three times, the reaction solution was heated up to 100 °C for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product was purified by column chromatography (PE/EtOAc=10/1 to 3/1) to obtain compound **41c** (1.1 g), light yellow solid.

MS (ESI) *m/z* 307.97 $[M + H]^+$.

**Step 3 4- ((2-fluoro-4- (4,4,5,5-tetramethyl-1,3,2-dioxaborolan-2-yl) phenyl) imino) -1,4-λ6-thiooxane-4-oxide 41d**

Compound **41c** (300 mg, 0.97 mmol), bis(pinacolato)diboron (300 mg, 1.17 mmol), $Pd(dppf)Cl_2$ (140 mg, 0.194 mmol), potassium acetate (300 mg, 2.91 mmol), and 1,4-dioxane (6 mL) were added into a sealed tube, after replaced with $N_2$ three times, sealed, and the reaction solution was heated up to 100 °C and stirred for 16 h. Then water (10 mL) was added and extract with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=5/1) to obtain compound **41d** (240 mg), yellow oily liquid.

MS (ESI) *m/z* 356 $[M + H]^+$.

**Step 4 *N*- (4- (4-amino-5- (3-fluoro-4- (4-oxo-1,4 λ 6 - thiohexane-4-ylidene) amino) phenyl) -7-methyl-7H-pyrrolo [2,3-*d*] pyrimidin-6-yl) phenyl) methacrylamide 41**

Compound **41d** (165 mg, 0.47 mmol), compound **9e** (130 mg, 0.36 mmol), $Pd(PPh_3)_4$ (83 mg, 0.072 mmol), and $K_3PO_4$ (230 mg, 1.08 mmol) were added into a sealed tube, after replaced with $N_2$ three times, 1,4-dioxane (5 mL) and $H_2O$ (0.5 mL) were added, sealed, and the reaction solution was heated up to 100 °C and stirred for 16 h. Then dichloromethane

(20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **41** (90 mg), white solid.

MS (ESI) *m/z* 535.08[M + H]$^+$.

$^1$H NMR (400 MHz, $CDCl_3$) $\delta$ 8.35 (s, 1H), 7.61 - 7.48 (m, 3H), 7.26 - 7.15 *(m,* 3H), 6.96 - 6.86 (m, 2H), 5.81 (s, 1H), 5.54 - 5.45 (s, 1H), 4.24 - 4.12 (m, 4H), 3.69 (s, 3H), 3.43 - 3.23 (m, 4H), 2.22 (s, 3H), 2.02 - 1.99 (m, 2H).

**Example 42 *N*- (4- (4-amino-5- (4- (dimethyl (oxo) - λ$^6$ - sulfanylidene) amino) -3-fluorophenyl) -7-methyl-7*H*-pyrrolo [2,3-*d*] pyrimidin-6-yl) -3-fluorophenyl) methacrylamide**

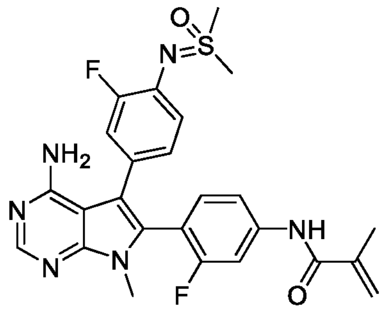

**42**

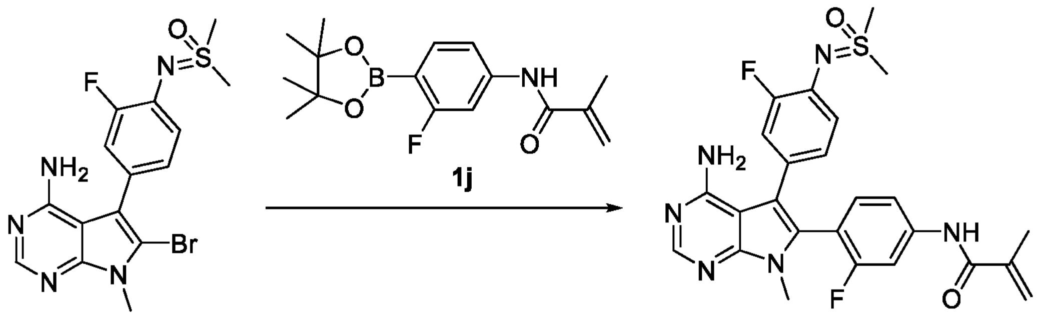

1j

**19f** **42**

Compound **19f** (100 mg, 0.24 mmol), compound **1j** (89 mg, 0.29 mmol), $Pd(PPh_3)_4$ (56 mg, 0.049 mmol), and $K_3PO_4$ (156 mg, 0.73 mmol) were added into a sealed tube, after replaced with $N_2$ three times, 1,4-dioxane (5 mL) and $H_2O$ (0.5 mL) were added, sealed, and the reaction solution was heated up to 100 °C and stirred for 16 h. Then dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **42** (40 mg), white solid.

MS (ESI) *m/z* 511.75 [M + H]$^+$.

$^1$H NMR (400 MHz, MeOD-d4) $\delta$ 8.22 (s, 1H), 7.76 (dd, *J* = 12.1, 2.1 Hz, 1H), *7.41 (dd, J* = 8.4, 2.1 Hz, 1H), 7.21 (td, *J* = 8.5, 4.7 Hz, 2H), 6.98 (d, *J = 11.1* Hz, 2H), 5.84 (s, 1H), 5.57 (d, *J* = 1.6 Hz, 1H), 4.60 (s, 1H), 3.64 (d, *J* = 0.9 *Hz,* 3H), 3.26 (s, 6H), 2.05 (s, 3H).

**Example 43 *N*- (4- (4-amino-5-(2-fluoro-4- ((1-oxotetrahydro-2*H*-1λ$^6$- thiopyran-1-ylidene) amino) phenyl) -7-methyl-7*H*-pyrrolo [2,3-*d*] pyrimidin-6-yl) phenyl) methacrylamide**

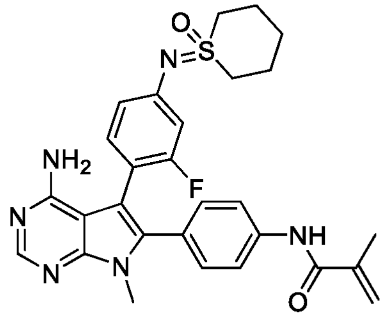

**43**

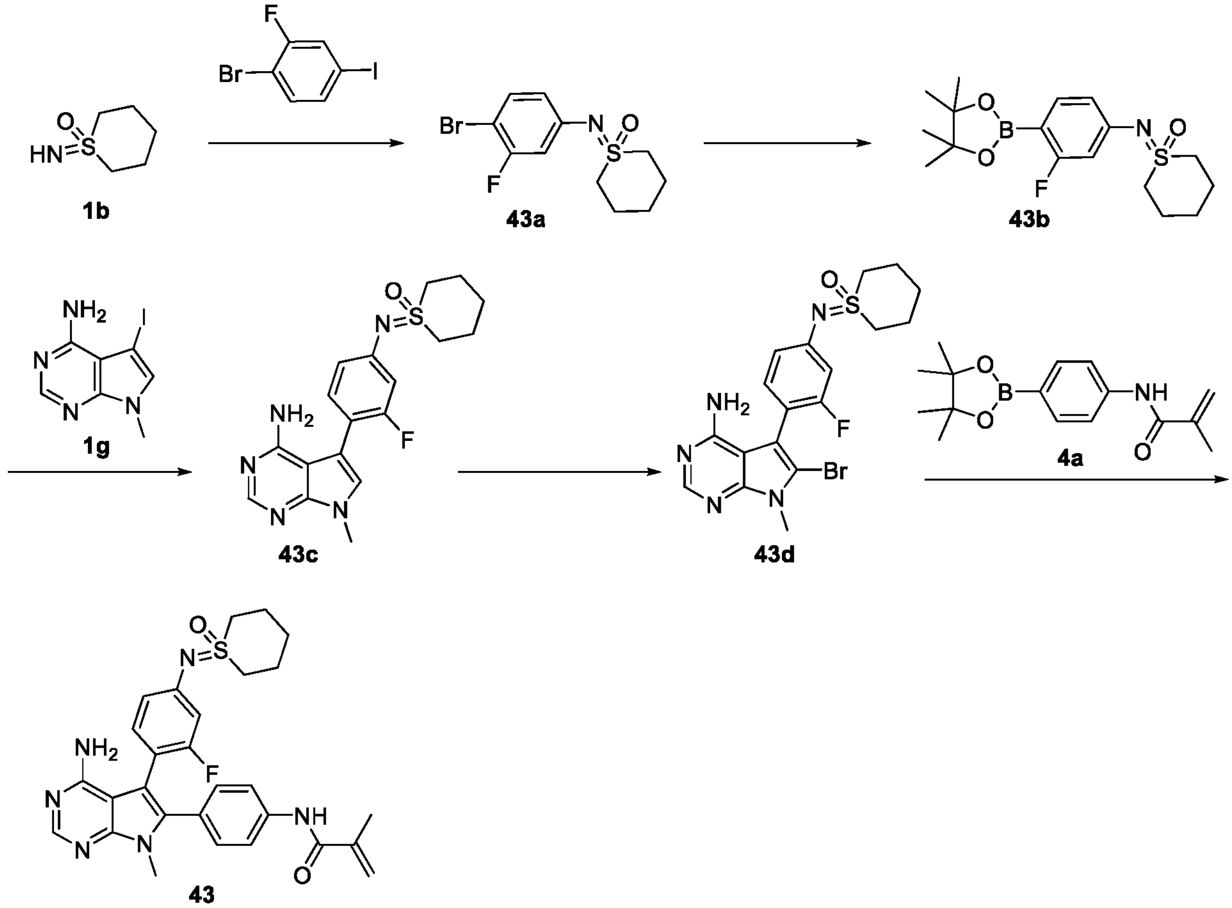

**1b** **43a** **43b** **1g** **43c** **43d** **4a** **43**

**Step 1 1- ((4-bromo-3-fluorophenyl) imino) hexahydro-1 λ 6 - thiopyran-1-oxide 43a**

Compound **1b** (270 mg, 2.02 mmol), carbofuran (820 mg, 2.52 mmol), 1-bromo-2-fluoro-4-iodobenzene (500 mg, 1.68 mmol), $Pd_2(dba)_3$ (39 mg, 0.042 mmol), and Xantphos (73 mg, 0.126 mmol) were added to 1,4-dioxane (10 mL), after replaced with $N_2$ three times, the reaction solution was heated up to 100 °C for 13 h. The reaction solution was filtered through diatomaceous earth, the filter cake was washed with ethyl acetate (50 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 1/1) to obtain compound **43a** (560 mg).

MS (ESI) *m/z* 306.2 $[M + H]^+$.

**Step 2 1- ((3-Fluoro-4- (4,4,5,5-tetramethyl-1,3,2-dioxaborolan-2-yl) phenyl) imino) hexahydro-1 λ 6 - thiophene 1-oxide 43b**

Compound **43a** (0.56g, 1.73 mmol), bis(pinacolato)diboron **(0.53 g, 2.1** mmol), Pd(dppf)$Cl_2$ (0.25 mg, 0.34 mmol), and potassium acetate (0.51 mg, 5.2 mmol) were placed in a bottle, 1,4-dioxane (5mL) was added, after replaced

with $N_2$ three times, the reaction was carried out at 100 °C for 18 h. The reaction solution was filtered and concentrated, and the obtained crude product was purified by column chromatography (PE/EtOAc=0-20%) to obtain compound **43b** (427 mg), yellow oily liquid.

**Step 3 1- (4- (4-amino-7-methyl-7H-pyrrolo [2,3-d] pyrimidin-5-yl) -3- fluorophenyl) imino) hexahydro-1 λ $^6$ - thiopyran-1-oxide 43c**

Compound **43b** (100 mg, 0.28 mmol), compound **1g** (65 mg, 0.24 mmol), $Pd(PPh_3)_4$ (54 mg, 0.056 mmol), and $K_3PO_4$ (150 mg, 0.84 mmol) were added into a sealed tube, after replaced with argon gas three times, 1,4-dioxane (4 mL) and $H_2O$ (0.5 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **43c** (80 mg), yellow solid.
MS (ESI) *m*/*z* 373.4 $[M + H]^+$.

**Step 4 (4- (4-amino-6-bromo-7-methyl-7*H*-pyrrolo [2,3-*d*] pyrimidin-5-yl) -2-fluorophenyl) imino) dimethyl - λ$^6$ - sulfonylone 43d**

Compound **43c** (55 mg, 0.15 mmol) was dissolved in DMF (3 mL), and a solution of N-bromosuccinimide (29 mg, 0.16 mmol) in DMF (0.5 mL) was added dropwise at 0 °C, the reaction solution was stirred at 0 °C for 1 h. Then DCM (30 mL) was added, and washed with saturated saline solution (10 mL × 3), and the organic phase was dried over anhydrous sodium sulfate, and concentrated under reduced pressure to obtain a crude product. The crude product was purified by column chromatography (MeOH/DCM=1/50) to obtain compound **43d** (54 mg), yellow solid.
MS (ESI) *m*/*z* 412.3 $[M + H]^+$.

**Step *5 N-* (4- (4-amino-5- (2-fluoro-4- (1-oxotetrahydro-2*H*-1λ $^6$ - thiopyran-1- ylidene) amino) phenyl) -7-methyl-7*H*-pyrrolo [2,3-*d*] pyrimidin-6-yl) phenyl) methacrylamide 43**

Compound **43d** (57 mg, 0.15 mmol), compound **4a** (50 mg, 0.18 mmol), $Pd(PPh_3)_4$ (30 mg, 0.03 mmol), and $K_3PO_4$ (100 mg, 0.45 mmol) were added into a sealed tube, after replaced with argon gas three times, 1,4-dioxane (3 mL) and $H_2O$ (0.4 mL) were added, sealed, and the reaction solution was heated up to 100 °C and stirred for 16 h. Then dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **43** (12 mg), white solid.
MS (ESI) *m*/*z* 533.54 $[M + H]^+$.
$^1$H NMR (400 MHz, MeOD-d4) δ 8.18 (s, 1H), 7.70 - 7.62 (m, 2H), 7.32 - *7.26 (m, 2H),* 7.21 (t, *J* = 8.6 Hz, 1H), 7.00 - 6.91 (m, 2H), 5.81 (s, 1H), 5.53 (d, *J* = 1.8 Hz, 1H), 4.58 (s, 1H), 3.68 (s, 3H), 3.43 - 3.34 (m, 2H), 3.30 - 3.19 (m, 2H), 2.13 - 1.99 (m, 5H), 1.82 - 1.52 (m, 4H).

**Example 44 *N-* (4- (4-amino-5- (4- (dimethyl (oxo) - λ$^6$ -sulfanylidene) amino) -3-fluorophenyl) -7-methyl-7*H*-pyrrolo [2,3-*d*] pyrimidin-6-yl) phenyl) methacrylamide**

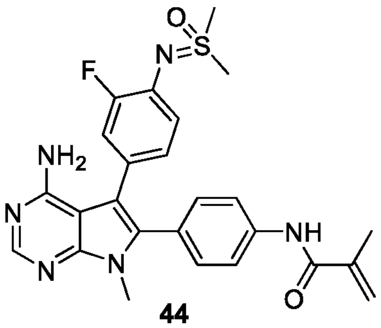

44

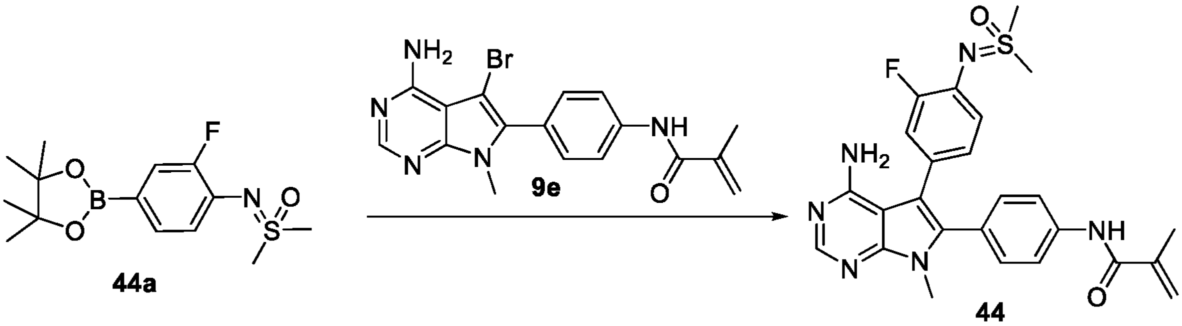

44a 9e 44

Compound **9e** (210 mg, 0.53 mmol), compound **44a** (200 mg, 0.64 mmol), $Pd(PPh_3)_4$ (120 mg, 0.11 mmol), and $K_3PO_4$ (340 mg, 1.59 mmol) were added into a sealed tube, after replaced with argon gas three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **44** (100 mg), white solid.

MS (ESI) *m/z* 493.26 $[M + H]^+$.

$^1H$ NMR (400 MHz, MeOD-d4) $\delta$ 8.17 (s, 1H), 7.71 - 7.60 (m, 2H), 7.28 (d, *J* = 8.6 *Hz,* 2H), 7.19 (t, *J* = 8.4 Hz, 1H), 7.03 - 6.89 (m, 2H), 5.80 (s, 1H), 5.52 (s, 1H), 4.57 (s, 1H), 3.67 (s, 3H), 3.24 (s, 6H), 2.02 *(s,* 3H).

**Example 45 *N*- (4- (4-amino-5- (4- (dimethyl (oxo) - $\lambda^6$ - sulfanylidene) amino) phenyl) -7-methyl-7*H*-pyrrolo [2,3-*d*] pyrimidin-6-yl) phenyl) methacrylamide**

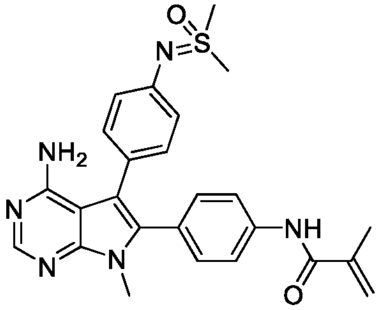

**45**

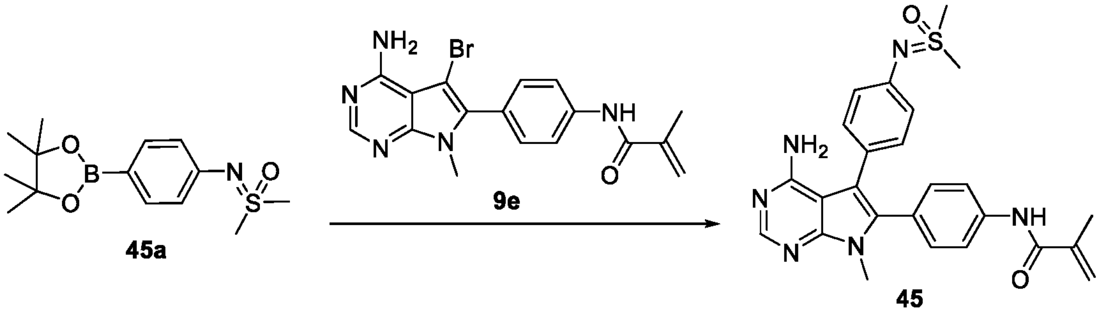

**45a**

**9e**

**45**

Compound **9e** (120 mg, 0.31 mmol), compound **45a** (110 mg, 0.37 mmol), $Pd(PPh_3)_4$ (72 mg, 0.062 mmol), and $K_3PO_4$ (198 mg, 0.93 mmol) were added into a sealed tube, after replaced with argon gas three times, 1,4-dioxane (5 mL) and $H_2O$ (0.5 mL) were added, seal, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM =1/30) to obtain compound **45** (80 mg), white solid.

MS (ESI) *m/z* 475.61 $[M + H]^+$.

$^1$H NMR (400 MHz, MeOD-d4) $\delta$ 8.17 (s, 1H), 7.68 - 7.60 (m, 2H), 7.31 - 7.23 *(m, 2H),* 7.19 - 7.10 (m, 2H), 7.07 - 6.99 (m, 2H), 5.80 (s, 1H), 5.57 - 5.49 (m, 1H), 4.56 (s, 1H), 3.68 (s, 3H), 3.22 (s, 6H), 2.02 (t, *J* = 1.2 Hz, 3H).

**Example 46 *N*- (4- (4-amino-5- (3-fluoro-4- ((1-oxotetrahydro 2*H*-1$\lambda^6$ - thiopyran -1-ylidene) amino) phenyl) -7-methyl-7*H*-pyrrolo [2,3-*d*] pyrimidin-6-yl) phenyl) - N-methylmethacrylamide**

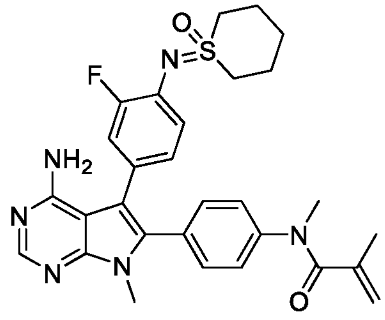

46

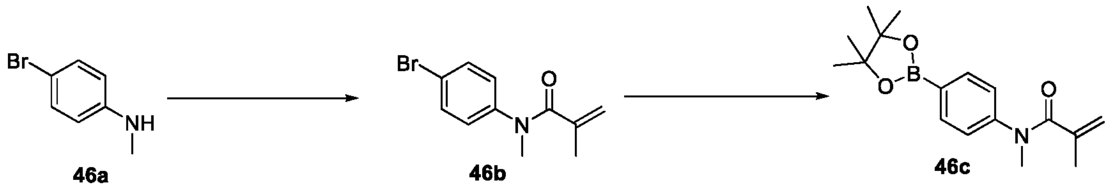

46a 46b 46c

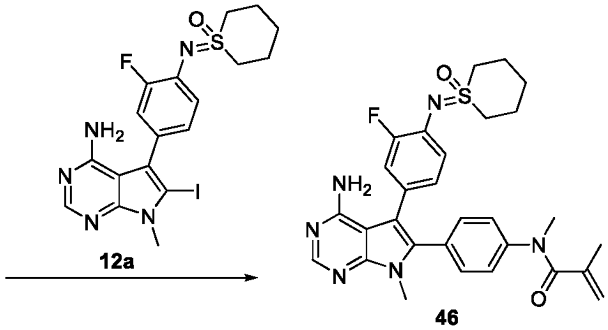

12a 46

**Step 1 N- (4-bromophenyl) - N-methylmethacrylamide 46b**

Compound **46a** (500 mg, 2.69 mmol) and triethylamine (0.75 mL, 5.38 mmol) were dissolved in DCM (50 ml) at 0 °C, 2-methylacryloyl chloride (290 mg, 2.82 mmol) was added dropwise to the reaction solution, and the reaction was stirred for 1 h. The reaction solution was then diluted with water (100 ml) and extracted with DCM (100 ml) three times. The organic phase was washed with saturated saline solution, dried over anhydrous $Na_2SO_4$, and concentrated under reduced pressure, the obtained crude product was purified by silica gel column chromatography (EA/PE=1/4) to obtain compound **46b** (400 mg), yellow solid.

MS (ESI) *m/z* 254.1 $[M + H]^+$.

**Step 2 *N-* methyl *-N-* (4- (4,4,5,5-tetramethyl-1,3,2-dioxaborolan-2-yl) phenyl) methacrylamide 46c**

Compound **46b** (300 mg, 1.18 mmol), bis(pinacolato)diboron (360 mg, 1.42 mmol), Pd(dppf)$Cl_2$ (172 mg, 0.236 mmol), and potassium acetate (347 mg, 3.54 mmol) were placed in a bottle, and dioxane (8 mL) was added, after replaced with $N_2$ three times, the reaction was carried out at 100 °C for 18 h. The reaction solution was filtered and concentrated, and the obtained crude product was purified by column chromatography (PE/EtOAc=0-20%) to obtain compound **46c** (260 mg), yellow oily liquid.

MS (ESI) *m/z* 302 $[M + H]^+$.

**Step 3 *N-* (4- (4-amino-5- (3-fluoro-4- ((1-oxotetrahydro2H-1 λ $^6$ - thiopyran-1- ylidene) amino) phenyl) -7-methyl-7H-pyrrolo [2,3-d] pyrimidin-6-yl) phenyl)** - **N-methylmethacrylamide 46**

Compound **12a** (110 mg, 0.22 mmol), compound **46c** (80 mg, 0.27 mmol), $Pd(PPh_3)_4$ (51 mg, 0.044 mmol), and $K_3PO_4$ (140 mg, 0.66 mmol) were added into a sealed tube, after replaced with argon gas three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product

was purified by column chromatography (MeOH/DCM=1/30) to obtain compound **46** (75 mg), white solid.

MS (ESI) *m/z* 547.56 [M + H]$^+$.

$^1$H NMR (400 MHz, $CDCl_3$) $\delta$ 8.36 (s, 1H), 7.22 (dd, *J* = 8.5, 2.6 Hz, 3H), 7.13 *(d, J* = 8.4 Hz, 2H), 6.92 (dd, *J* = *8.1,* 2.1 Hz, 1H), 6.82 (dd, *J* = 11.5, 2.0 Hz, 1H), 5.08 (d, *J* = 10.0 Hz, 3H), 5.01 (s, 1H), 3.71 (s, 3H), 3.38 *(s,* 5H), 3.15 (ddd, *J* = 13.6, 8.7, 4.2 Hz, 2H), 2.12 (td, *J* = 9.3, 8.5, *5.1* Hz, 4H), 1.76 (s, 3H), 1.25 (s, 2H).

**Example 47 *N*- (4- (4-amino-5- (3-fluoro-4-((oxo (pyrrolidine-1-yl) (trifluoromethyl) - λ$^6$-sulfanylidene) amino) phenyl) -7-methyl-7*H*-pyrrolo [2,3-*d*] pyrimidin-6-yl) phenyl) methacrylamide**

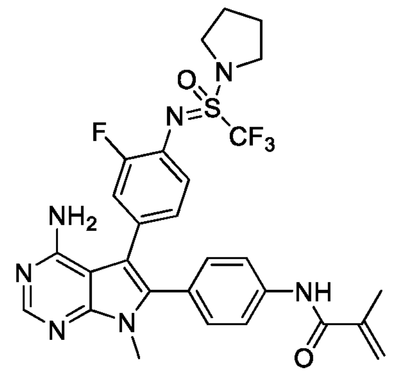

**47**

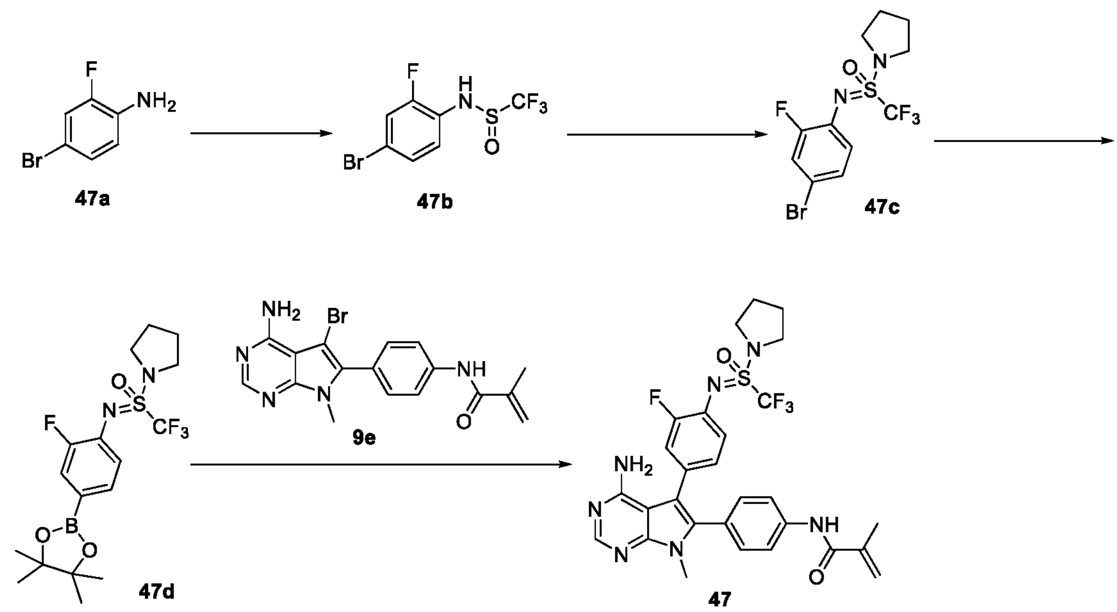

47a 47b 47c 47d 9e 47

**Step 1 N- (4-bromo-2-fluorophenyl) -1,1,1-trifluoromethanesulfonamide 47b**

Trifluoromethanesulfonyl chloride (2.4 g, 15.79 mmol) was dissolved in dichloromethane (30 mL), cooled down to 0 °C, a solution of compound **47a** (3.0 g, 15.79 mmol) in dichloromethane (10 mL) was added dropwise, then DIPEA (2.04 g, 15.79 mmol) was added, and heated up to room temperature to react for 2 h. The reaction solution was poured into water and extracted with DCM three times, dried over anhydrous sodium sulfate, and concentrated to obtain a crude product. The crude product was purified by column chromatography (PE/EtOAc=0-3%) to obtain compound **47b** (2.8 g), white solid.

MS (ESI) *m/z* 306.56 [M + H]$^+$.

**Step 2 1- (*N*- (4-bromo-2-fluorophenyl) - S - (trifluoromethyl) sulfonylimino) pyrrolidine 47c**

Compound **47b** (300 mg, 0.99 mmol) and N-chlorosuccinimide (145 mg, 1.1 mmol) were dissolved in acetonitrile (8 mL), tetrabutylammonium fluoride (1.1 mL, 1.1 mmol, 1M) was added at 0 °C, and the reaction was carried out at room

temperature for 30 min, the reaction solution was concentrated, and then acetonitrile (8 mL) and tetrahydropyridine (280 mg, 4.0 mmol) were added, and reacted at room temperature for 2 h. The reaction solution was concentrated and dried, and the crude product was purified by column chromatography (PE/EtOAc=0-20%) to obtain compound **47c** (124 mg), yellow oily liquid

MS (ESI) *m/z* 374.2 $[M + H]^+$.

**Step 3 1- (*N*- (2-fluoro-4- (4,4,5,5-tetramethyl-1,3,2-dioxaborolan-2-yl) phenyl) - S - (trifluoromethyl) sulfonylimide) pyrrolidine 47d**

Compound **47c** (124 mg, 0.33 mmol), bis(pinacolato)diboron (100 mg, 0.4 mmol), Pd(dppf)$Cl_2$ (48 mg, 0.066 mmol), and potassium acetate (98 mg, 0.99 mmol) were placed in a bottle, 1,4-dioxane (3 mL) was added, after replaced with $N_2$ three times, the reaction was carried out at 100 °C for 18 h. The reaction solution was filtered and concentrated, and the crude product was purified by column chromatography (PE/EtOAc =0-20%) to obtain compound **47d** (70 mg).

MS (ESI) *m/z* 423 $[M + H]^+$.

**Step 4 *N*- (4- (4-amino-5- (3-fluoro-4- ((oxo (pyrrolidine-1-yl) (trifluoromethyl) - $\lambda^6$-sulfanylidene) amino) phenyl) -7-methyl-7*H*-pyrrolo [2,3-*d*] pyrimidin-6-yl) phenyl) methacrylamide 47**

Compound **9e** (25 mg, 0.065 mmol), compound **47d** (30 mg, 0.07 mmol), $Pd(PPh_3)_4$ (15 mg, 0.013 mmol), and $K_3PO_4$ (42 mg, 0.2 mmol) were added into a sealed tube, after replaced with argon gas three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/30) to obtain compound **47** (10 mg), white solid.

MS (ESI) *m/z* 602.3 $[M + H]^+$.

$^1$H NMR (400 MHz, MeOD-d4) $\delta$ 8.19 (s, 1H), 7.71 - 7.59 (m, 2H), 7.31 - *7.21 (m*, 2H), *7.15* (t, *J* = 8.4 Hz, 1H), 7.03 - 6.94 (m, 2H), 5.81 (s, 1H), 5.52 (d, *J* = 1.8 Hz, 1H), 3.68 (s, 3H), 3.55 (d, *J* = 7.5 Hz, 2H), 3.43 (d, *J* = 8.6 Hz, 2H), 2.05 - 1.91 (m, 6H), *1.31* (d, *J* = 17.8 Hz, 2H).

**Example 48 *N*- (4- (4-amino-5- (4- (((dimethylamino) (oxo) (trifluoromethyl) - $\lambda^6$-sulfanylidene) amino) -3-fluorophenyl) -7-methyl-7*H*-pyrrolo [2,3) - d] pyrimidin-6-yl) phenyl) methacrylamide**

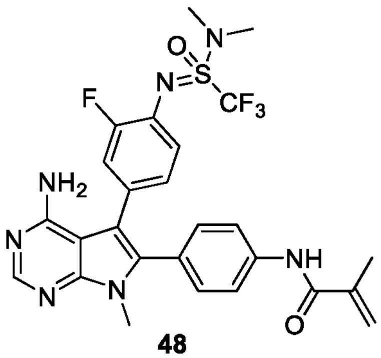

48

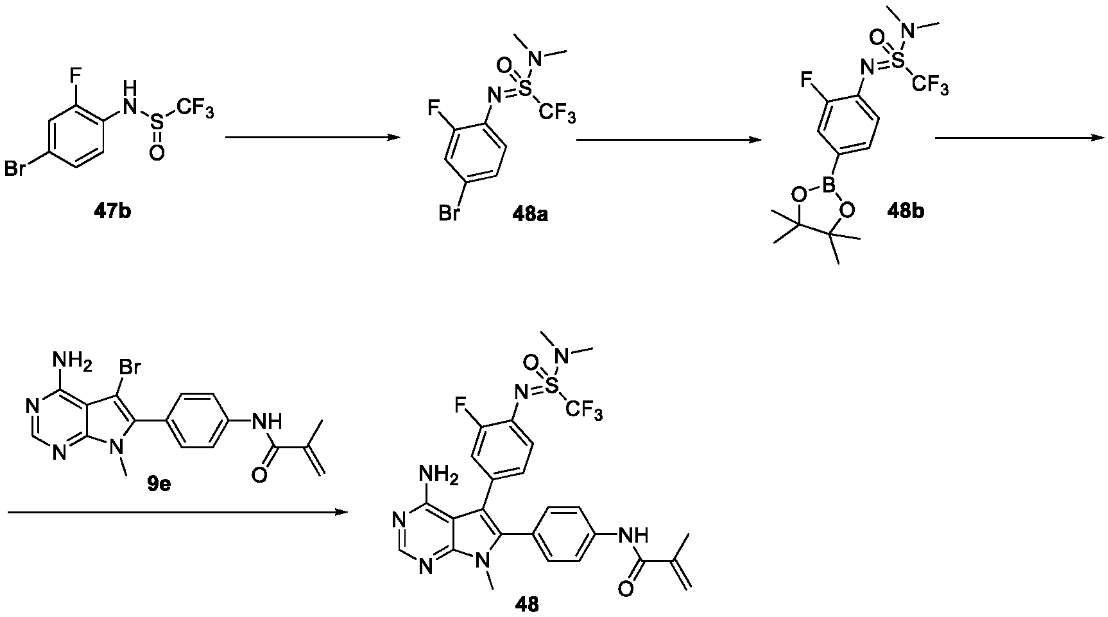

47b

48a

48b

9e

48

**Step 1 N'-(4-bromo-2-fluorophenyl)-1,1,1-trifluoro-N,N- dimethylmethanesulfonimide 48a**

Compound **47b** (500 mg, 1.6 mmol) and N-chlorosuccinimide (235 mg, 1.76 mmol) were dissolved in acetonitrile (8 mL), tetrabutylammonium fluoride (1.76 mL, 1.76 mmol, 1M) was added at 0 °C, and the reaction was carried out at room temperature for 30 min, the reaction solution was concentrated, and then acetonitrile (8 mL) and dimethylamine (3.5 mL, 7.0 mmol, 2M in THF) were added, and reacted at room temperature for 2 h. The reaction solution was concentrated and dried, and the crude product was purified by Flash (PE/EtOAc=0-20%) to obtain compound **48a** (400 mg), yellow oily liquid

MS (ESI) *m/z* 349.2 $[M + H]^+$.

**Step 2 1,1,1-trifluoro-N'-(2-fluoro-4-(4,4,5,5-tetramethyl-1,3,2-dioxaborolan-2-yl) phenyl)- N, N-dimethylsulfonamide 48b**

Compound **48a** (100 mg, 0.29 mmol), bis(pinacolato)diboron (87 mg, 0.34 mmol), Pd(dppf)$Cl_2$ (42 mg, 0.058 mmol), and potassium acetate (85 mg, 0.87 mmol) were placed in a bottle, 1,4-dioxane (5 mL) was added, after replaced with $N_2$ three times, the reaction was carried out at 100 °C for 18 h. The reaction solution was filtered and concentrated, and the crude product was purified by column chromatography (PE/EtOAc=0-20%) to obtain compound **48b** (90 mg), yellow oily liquid.

MS (ESI) *m/z* 397 $[M + H]^+$.

**Step 3 *N-* (4- (4-amino-5- (4- ((dimethylamino) (oxo) (trifluoromethyl) - $\lambda^6$ - sulfanylidene) amino) -3-fluorophenyl) -7-methyl-7*H*-pyrrolo [2,3) - *d*] pyrimidin-6-yl) phenyl) methacrylamide 48**

Compound **9e** (65 mg, 0.17 mmol), compound **48b** (80 mg, 0.20 mmol), $Pd(PPh_3)_4$ (38 mg, 0.034 mmol), and $K_3PO_4$ (110 mg, 0.51 mmol) were added into a sealed tube, after replaced with argon gas three times, 1,4-dioxane (5 mL) and $H_2O$ (0.5 mL) were added, sealed, and the reaction solution was heated up to 100 °C and stirred for 16 h.

Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/30) to obtain compound **48** (30 mg), white solid.

MS (ESI) *m/z* 576.54 $[M + H]^+$.

$^1H$ NMR (400 MHz, MeOD-d4) $\delta$ 8.09 (s, 1H), 7.60 - 7.55 (m, 2H), 7.18 (d, *J* = 8.7 *Hz, 2H),* 7.05 (t, *J* = 8.4 Hz, 1H), 6.95 - 6.83 (m, 2H), 5.71 (s, 1H), 5.42 (d, *J* = 1.8 Hz, 1H), 4.45 (s, 1H), 3.58 (s, 2H), 2.97 (s, 5H), 1.93 (d, *J* = 1.3 Hz, 2H), 1.19 (s, 3H).

**Example 49 *N*- (4- (4-amino-5- (4- (cyclopentyl (methyl) (oxo) - $\lambda^6$-sulfanylidene) amino) phenyl) -7-methyl-7*H*-pyrrole [2,3-*d*] pyrimidin-6-yl) -3-fluorophenyl) methacrylamide**

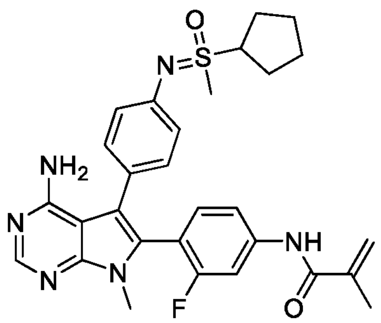

**49**

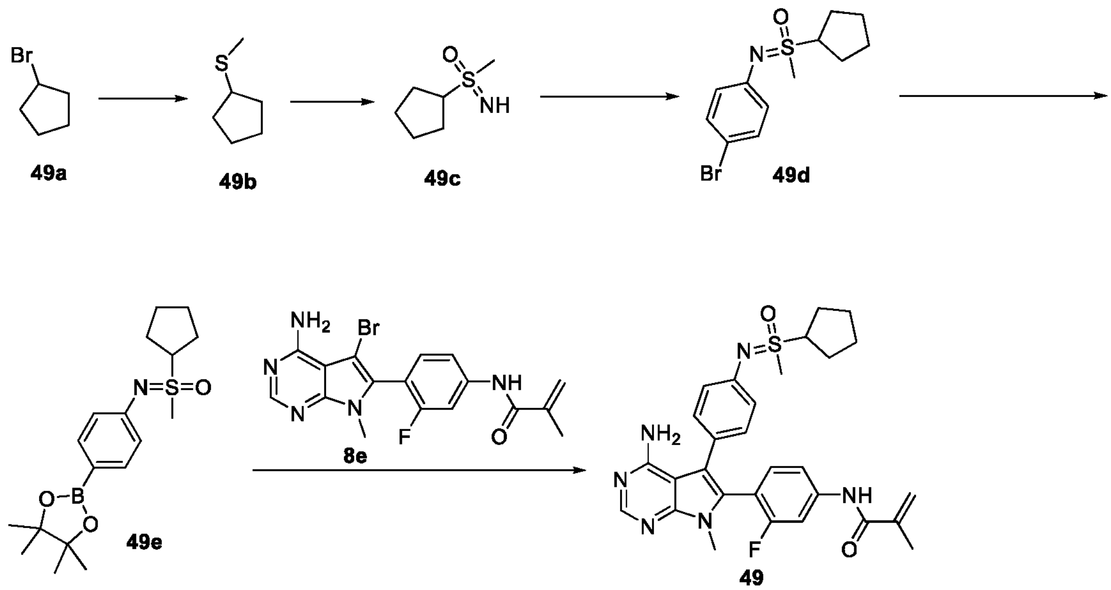

49a 49b 49c 49d

49e 8e 49

**Step 1 cyclopentyl-1-thioethane 49b**

Compound **49a** (1.4 g, 9.4 mmol) was dissolved in ethanol (20 mL), sodium methylthionate aqueous solution (4.9g, 14.0mmol) was added at room temperature sequentially to react at room temperature for 16 h. The reaction solution was cooled down to room temperature and extracted with ethyl acetate (50 mL). The organic phase was concentrated under reduced pressure to obtain crude product **49b,** which was directly used in the next step.

**Step 2 cyclopentylmethylsulfoximine 49c**

Compound **49b** (0.9 g, 9.4 mmol) was dissolved in 20 mL of ethanol, ammonium formate (1.46 g, 15.0 mmol) and iodobenzene diethyl ester (7.5 g, 24 mmol) were added in an ice bath sequentially, the reaction was carried out at room temperature for 3 h, after TLC monitored the completion of the reaction, it was cooled down to room temperature, and the

crude product was purified by column chromatography (DCM/MeOH=10/1) to obtain compound **49c** (100 mg), brown oily substance.

MS (ESI) *m/z* 148 [M + H]$^+$.

$^1$H NMR (400 MHz, $CDCl_3$) $\delta$ 3.52 (t, J=8.13 Hz, 1 H), 2.85 - 2.99 (m, 3 *H),* 1.94 - 2.15 (m, 4 *H),* 1.73 - 1.87 (m, 2 H), 1.59 - 1.73 (m, 2 H).

**Step 3 4-bromophenyl-cyclopentylmethylsulfoximine 49d**

To a 50 mL three necked bottle, 4-bromoiodobenzene (210 mg, 0.75 mmol) was dissolved in degassed 1,4-dioxane (3 mL), and then compound **49c** (100 mg, 0.68 mmol), $Pd_2(dba)_3$ (60 mg, 0.06 mmol), Xantphos (68 mg, 0.12 mmol), and cesium carbonate (442 mg, 1.36 mmol) were added, the reaction was heated up to 90 °C and stirred for 4 h. After the reaction was completed, it was cooled down to room temperature, ethyl acetate (20 mL) and saturated sodium bicarbonate solution (10 mL) were added and extracted with ethyl acetate (20 mL $\times$ 3), and the organic phases were combined, washed with saturated saline solution (50 mL), and concentrated under reduced pressure to obtain a crude product, and the crude product was purified by column chromatography (PE/EtOAc=3/1) to obtain compound **49d** (150 mg).

MS (ESI) *m/z* 302 [M + H]$^+$.

**Step 4 cyclopentyl (methyl) (4- (4,4,4,5,5-tetramethyl-1,3,2-dioxaborolan-2-yl) phenyl) imino) - $\lambda^6$-sulfone 49e**

In a 50 mL three necked bottle, compound **49d** (150 mg, 0.5 mmol) was dissolved in degassed 1,4-dioxane (5 mL), and then bis(pinacolato)diboron (152 mg, 0.6 mmol), KOAc (150 mg, 1.5 mmol), and $Pd(dppf)Cl_2$ (50 mg, 0.05 mmol) were added, the reaction was heated up to 90 °C and stirred for 3 h, after the reaction was completed, it was cooled down to room temperature, ethyl acetate (20 mL) and saturated sodium chloride solution (10 mL) were added and extracted with ethyl acetate (20 mL $\times$ 3), and the organic phases were combined, washed with saturated saline solution (50 mL), and concentrated under reduced pressure to obtain a crude product, and the crude product was purified by column chromatography (PE/EtOAc=2/1) to obtain compound **49e** (50 mg).

MS (ESI) *m/z* 350 [M + H]$^+$.

**Step 5 *N-* (4- (4-amino-5- (4- (cyclopentyl (methyl) (oxo) - $\lambda^6$-sulfanylidene) amino) phenyl) -7-methyl-7*H*-pyrrole [2,3-*d*] pyrimidin-6-yl) -3-fluorophenyl) methacrylamide 49**

In a 50 mL three necked bottle, compound **49e** (50 mg, 0.14 mmol) was dissolved in degassed DMF (2 mL) and water (0.2 mL), and then, compound **8e** (111 mg, 0.27 mmol), $K_3PO_4$ (176 mg, 0.83 mmol), and $Pd(dppf)Cl_2$ (30 mg, 0.03 mmol) were added, the reaction was heated up to 90 °C and stirred for 3 h, after the reaction was completed, it was cooled down to room temperature, ethyl acetate (20 mL) and saturated sodium chloride solution (10 mL) were added and extracted with ethyl acetate (20 mL $\times$ 3), and the organic phases were combined, washed with saturated saline solution (50 mL), and concentrated under reduced pressure to obtain a crude product, and the crude product was purified by column chromatography (DCM/MeOH=10/1) to obtain compound **49** (7.7 mg), white solid.

MS (ESI) *m/z* 533 [M + H]$^+$.

$^1$H NMR (400 MHz, $CDCl_3$) $\delta$ 8.32 (s, 1 H), 7.66 - 7.76 (m, 2 H), 7.59 (s, *1* H), 6.97 - 7.13 (m, 5 H), 5.81 (s, 1H), 5.53 (s, 1 H), 5.31 (s, 2 H), 3.64 - 3.69 (m, 3 H), 2.99 (s, 3 H), 2.18 - 2.28 (m, 3 H), 2.14 (br. s., 3 H), 2.06 - 2.12 (m, 3 H), 2.01 (br. s., 3 H).

**Example 50 *N-(4-* (4-amino-5- (4- ((cyclobutyl (methyl) (oxo) - $\lambda^6$-sulfanylidene) amino) phenyl) -7-methyl-7*H*-pyrrole [2,3-*d*] pyrimidin-6-yl) -3-fluorophenyl) methacrylamide**

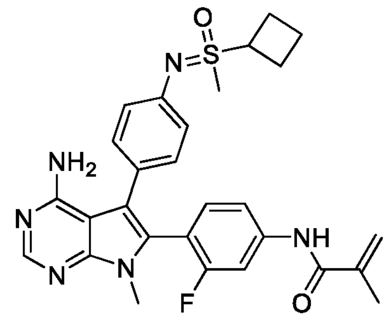

**50**

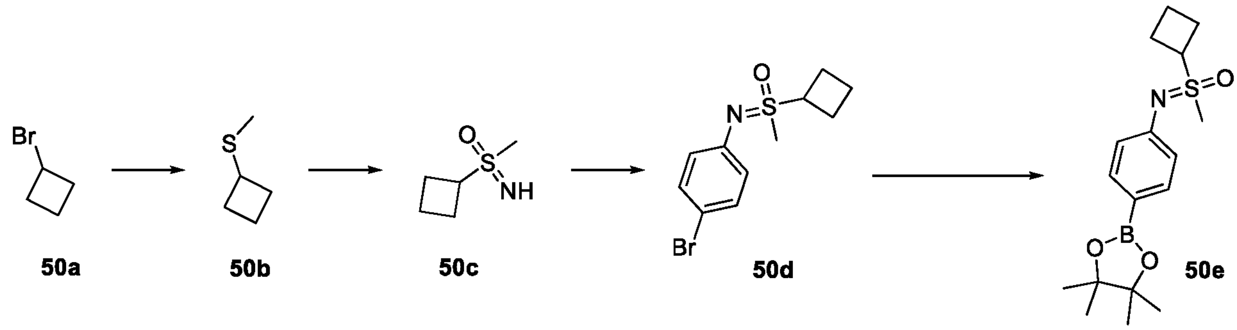

**50a** **50b** **50c** **50d** **50e**

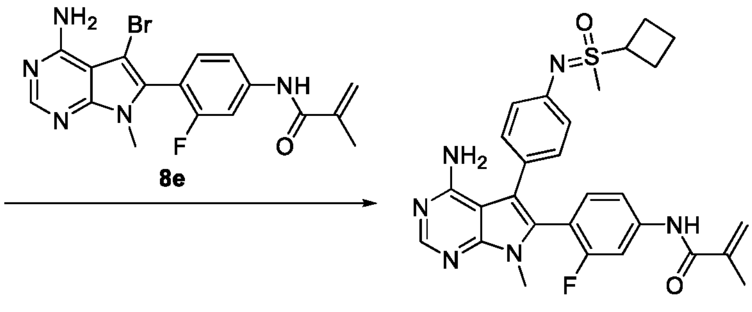

**8e**

**50**

**Step 1 cyclobutyl methyl sulfide 50b**

Compound **50a** (1.26 g, 9.4 mmol) was dissolved in ethanol (20 mL), sodium methylthionate aqueous solution (4.9 g, 14.0 mmol) was added at room temperature sequentially, and then the reaction was carried out at room temperature for 16 h. The reaction solution was cooled down to room temperature and extracted with 50 mL of ethyl acetate. The extracted mixture was concentrated under reduced pressure to obtain crude product **50b,** which was directly used in the next step.

**Step 2 cyclobutylmethylsulfonimide 50c**

Compound **50b** (0.9 g, 9.4 mmol) was dissolved in 20 mL of ethanol, ammonium formate (1.46 g, 15.0 mmol) was added sequentially, in ice bath, iodobenzene diacetate (7.5 g, 24 mmol) was added, and the reaction was carried out at room temperature for 3 h, after TLC monitored the completion of the reaction, it was cooled down to room temperature, concentrated under reduced pressure, and the crude product was purified by column chromatography (DCM/MeOH=10/1) to obtain compound **50c** (75 mg), brown oily substance.

MS (ESI) *m/z* 134 $[M + H]^+$.

$^1H$ NMR (400 MHz, $CDCl_3$) $\delta$ 3.90 (t, *J*=8.33 Hz, 1 H), 2.86 (s, 3 H), 2.45 - 2.63 (m, 2 H), 2.32 (dtd, *J=12.22,* 8.23, 8.23, 4.23 Hz, 2 H), 1.95 - 2.13 (m, 3 H).

**Step 3 4-bromophenyl-cyclobutylmethylsulfonimide 50d**

To a 50 mL three necked bottle, 4-bromoiodobenzene (100 mg, 0.33 mmol) was dissolved in degassed 1,4-dioxane (3 mL), and then compound **50c** (40 mg, 0.3 mmol), $Pd_2(dba)_3$ (30 mg, 0.03 mmol), Xantphos (34 mg, 0.06 mmol), cesium carbonate (295mg, 0.9mmol) were added, heated up to 90 °C and stirred for 4 h. After the reaction was completed, it was cooled down to room temperature, ethyl acetate (20 mL) and saturated sodium bicarbonate solution (10 mL) were added and extracted with ethyl acetate (20 mL × 3), and the organic phases were combined, washed with saturated saline solution (50 mL), and concentrated under reduced pressure to obtain a crude product, and the crude product was purified by column chromatography (PE/EtOAc=3/1) to obtain compound **50d** (75 mg).

MS (ESI) *m*/*z* 288 $[M + H]^+$.

**Step 4 cyclobutyl (methyl) (4- (4,4,4,5,5-tetramethyl-1,3,2-dioxaborolan-2-yl) phenyl) imino) - $\lambda^6$-thianone 50e**

To a 50 mL three necked bottle, compound **50d** (75 mg, 0.26 mmol) was dissolved in degassed 1,4-dioxane (5 mL), and then bis(pinacolato)diboron ester (75 mg, 0.3 mmol), KOAc (75 mg, 0.6 mmol), and $Pd(dppf)Cl_2$ (22 mg, 0.03 mmol) were added, the reaction was heated up to 90 °C and stirred for 3 h, after the reaction was completed, it was cooled down to room temperature, ethyl acetate (20 mL) and saturated sodium chloride solution (10 mL) were added and extracted with ethyl acetate (20 mL × 3), and the organic phases were combined, washed with saturated saline solution (50 mL), and concentrated under reduced pressure to obtain a crude product, and the crude product was purified by column chromatography (PE/EtOAc=2/1) to obtain compound **50e** (93 mg).

MS (ESI) *m*/*z* 335 $[M + H]^+$.

**Step 5 *N*-(4-(4-amino-5-(4-((cyclobutyl(methyl)(oxo)-$\lambda^6$-sulfanylidene)amino) phenyl)-7-methyl-7*H*-pyrrole[2,3-*d*]pyrimidin-6-yl)-3-fluorophenyl)methacrylamide 50**

In a 50 mL three necked bottle, compound **50e** (93 mg, 0.27 mmol) was dissolved in degassed DMF (3 mL) and water (0.3 mL), and then compound **8e** (111 mg, 0.27 mmol), $K_3PO_4$ (176 mg, 0.83 mmol), and $Pd(dppf)Cl_2$ (30 mg, 0.03 mmol) were added, the reaction was heated up to 90 °C and stirred for 3 h, after the reaction was completed, it was cooled down to room temperature, ethyl acetate (20 mL) and saturated sodium chloride solution (10 mL) were added and extracted with ethyl acetate (20 mL × 3), and the organic phases were combined, washed with saturated saline solution (50 mL), and concentrated under reduced pressure to obtain a crude product, and the crude product was purified by column chromatography (DCM/MeOH=10/1) to obtain compound **50** (4.5 mg), white solid.

MS (ESI) *m*/*z* 533 $[M + H]^+$.

$^1H$ NMR (400 MHz, $CDCl_3$) $\delta$ 8.38 (s, 1 H), 7.72 (d, *J*=12.09 Hz, 1 H), 7.59 *(s,* 1 H), 6.98 - 7.20 (m, 5 H), 5.83 (s, 1 H), 5.54 (s, *1* H), 5.06 (br. s., 2 H), 4.00 (s, 1 H), 3.68 (s, 3 H), 2.94 (s, 3 H), 2.68 (br. s., 2 H), 2.35 (d, *J*=8.60 Hz, 2 H), 1.96 - 2.15 (m, 5 H).

**Example 51 *N*-(4-(4-amino-5-(4-((azetidin-1-yl(oxo)(trifluoromethyl)-$\lambda^6$-sulfanylidene) amino) phenyl) -7-methyl-7*H*-pyrrolo [2,3-*d*] pyrimidin-6-yl) phenyl) methacrylamide**

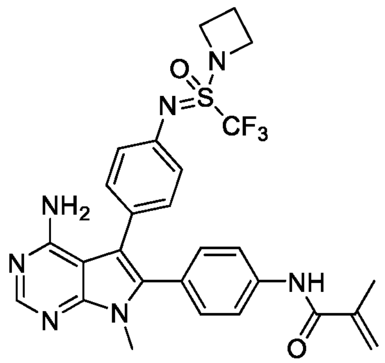

**51**

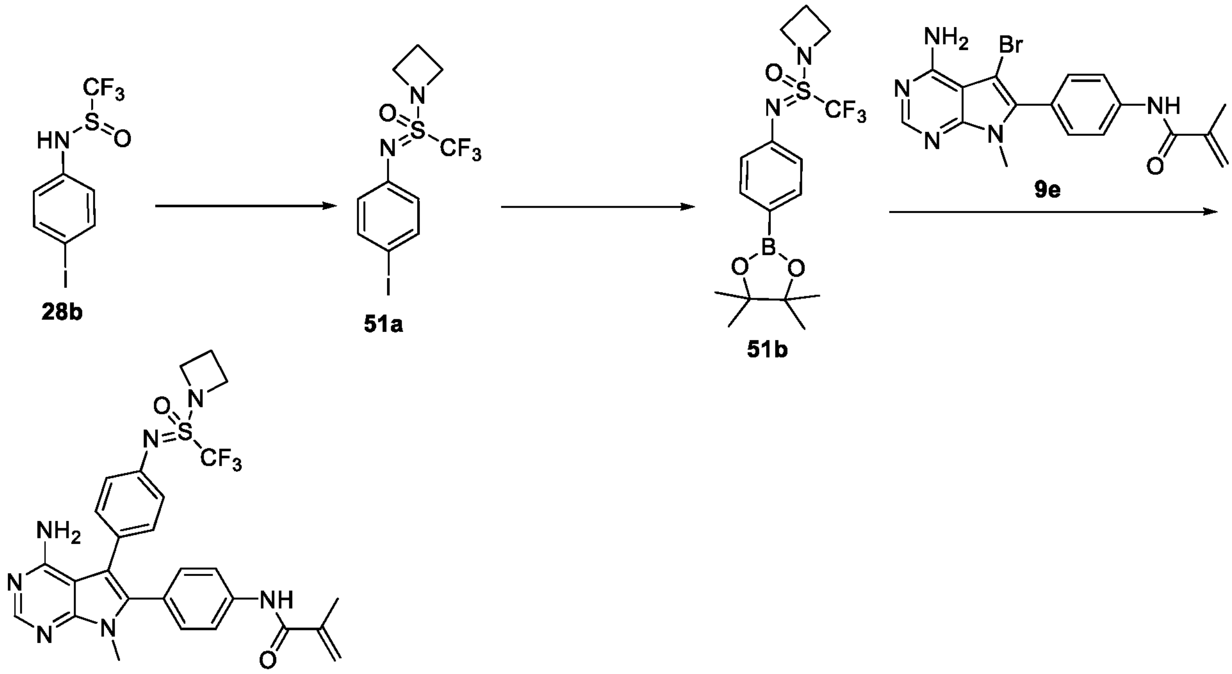

28b 51a 51b 9e 51

**Step 1 1- (*N*-(4-iodobenzene)-S-(trifluoromethyl)sulfonylimide)azetidine 51a**

Compound **28b** (500 mg, 1.49 mmol) and N-chlorosuccinimide (292 mg, 1.64 mmol) were dissolved in dry acetonitrile (8 mL), a solution of tetrabutylammonium fluoride in tetrahydrofuran (1.64 mL, 1.64 mmol, 1M) was added at 0 °C, the reaction was carried out at room temperature for 30 min, the reaction solution was concentrated, and then dry acetonitrile (8 mL) and heterocyclic cyclohexane (340 mg, 6.0 mmol) were added, and reacted at room temperature for 2 h. The reaction solution was concentrated and dried, and the obtained crude product was purified by Flash (PE/EtOAc=0-20%) to obtain compound **51a** (230 mg), yellow oily liquid.

MS (ESI) *m/z* 390 $[M + H]^+$.

**Step 2 1-*(N-* (4- (4,4,5,5-tetramethyl-1,3,2-dioxaborolan-2-yl) phenyl) - S - (trifluoromethyl) sulfonylimide) azetidine 51b**

Compound **51a** (230 mg, 0.59 mmol), bis(pinacolato)diboron (180 mg, 0.71 mmol), $Pd(dppf)Cl_2$ (87 mg, 0.12 mmol), and potassium acetate (154 mg, 1.77 mmol) were placed in a bottle, 1,4-dioxane (6 mL) was added, after replaced with $N_2$ three times, the reaction was carried out at 100 °C for 18 h. The reaction solution was filtered and concentrated, and the crude product was purified by column chromatography (PE/EtOAc=0-20%) to obtain compound 51b (180 mg), yellow solid.

MS (ESI) *m/z* 391 $[M + H]^+$.

**Step 3 *N*- (4- (4-amino-5- (4-((azetidine-1-yl-(oxo) (trifluoromethyl) -λ⁶-sulfanylidene) amino) phenyl) -7-methyl-7*H*-pyrrolo[2,3-*d*]pyrimidin-6-yl) phenyl) methacrylamide 51**

Compound **9e** (42 mg, 0.11 mmol), compound **51b** (50 mg, 0.13 mmol), $Pd(PPh_3)_4$ (25 mg, 0.021 mmol), and $K_3PO_4$ (68 mg, 0.32 mmol) were added into a sealed tube, and it was replace with argon gas three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/30) to obtain compound **51** (11 mg), white solid.

MS (ESI) *m*/*z* 570.05 $[M + H]^+$.

$^1H$ NMR (400 MHz, MeOD-d4) $\delta$ 8.19 (s, 1H), 7.72 - 7.59 (m, 2H), 7.39 (d, *J* = 8.5 *Hz,* 2H), 7.33 (d, *J* = 8.5 Hz, 2H), 7.30 - 7.21 (m, 2H), 5.80 (s, 1H), 5.52 (d, *J* = 1.8 Hz, 1H), 4.58 (s, 1H), 4.28 - 4.19 *(m,* 1H), 3.86 - 3.72 (m, 2H), 3.68 (s, 4H), 2.30 - 2.18 (m, 1H), *2.07* - 1.93 (m, 4H).

**Example 52 *N*-(4- (4-amino-5- (4- (((dimethylamino) (oxo) (trifluoromethyl) -λ⁶-sulfanylidene) amino) -2-fluorophenyl) -7-methyl-7H-pyrrolo [2,3-d] pyrimidin-6-yl) phenyl) methacrylamide**

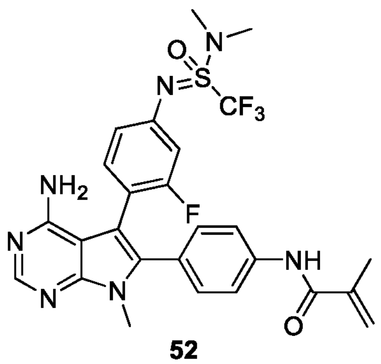

**52**

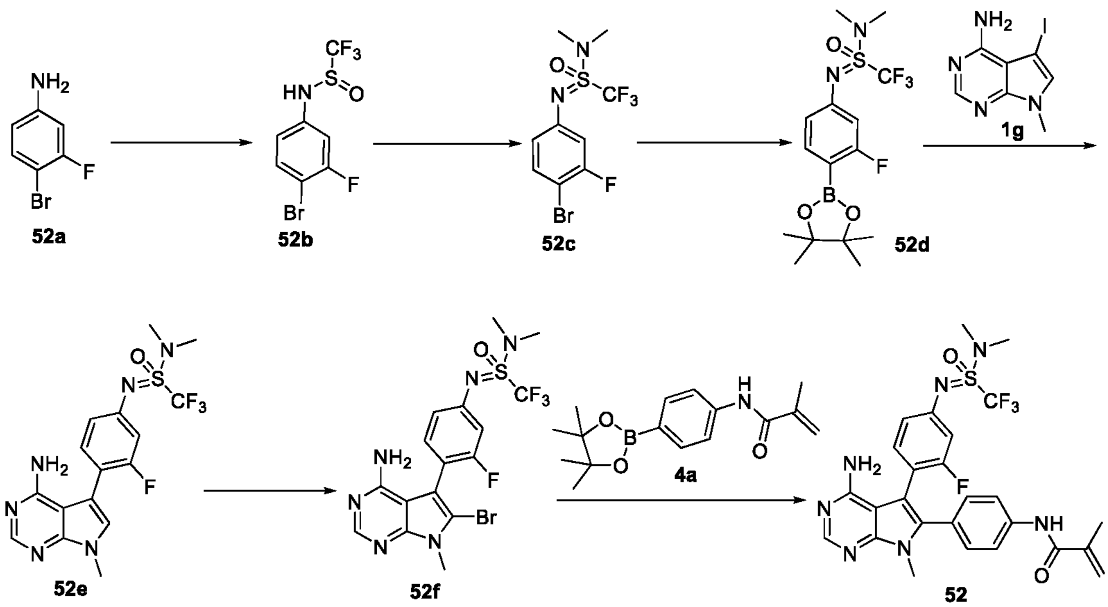

52a 52b 52c 52d 1g 52e 52f 4a 52

**Step 1 N- (4-bromo-3-fluorophenyl) -1,1,1-trifluoromethylsulfonimide 52b**

Trifluoromethanesulfonyl chloride (2.41g, 15.79 mmol) was dissolved in dichloromethane (15 mL), cooled down to 0 °C, a solution of compound **52a** (3.0 g, 15.79 mmol) in dichloromethane (10 mL) was added dropwise, and N,N-diisopropylethylamine (2.04 g, 15.79 mmol) was added, then heated up to room temperature and reacted for 2 h. The reaction solution was poured into water and extracted with dichloromethane three times, dried over anhydrous sodium

sulfate, and concentrated to obtain a crude product, and the crude product was purified by column chromatography (PE/EtOAc=0-3%) to obtain compound **52b** (2.6 g), brown solid.

MS (ESI) *m/z* 306.56 $[M + H]^+$.

### Step 2 N'-(4-bromo-3-fluorophenyl)-1,1,1-trifluoro-*N,N*-dimethylmethanesulfonimide 52c

Compound **52b** (600 mg, 1.98 mmol) and N-chlorosuccinimide (318 mg, 2.18 mmol) were dissolved in dry acetonitrile (10 mL), at 0 °C, a solution of tetrabutylammonium fluoride in tetrahydrofuran (2.2 mL, 2.2 mmol, 1M) was added, and the reaction was carried out at room temperature for 30 min. The reaction solution was concentrated, and then dry acetonitrile (10 mL) and dimethylamine tetrahydrofuran solution (4.0 mL, 8.0 mmol, 2M in THF) were added, and reacted at room temperature for 2 h. The reaction solution was concentrated, dried, and purified by column chromatography (PE/EtOAc=0-20%) to obtain compound **52c** (480 mg), yellow oily liquid

MS (ESI) *m/z* 349.2 $[M + H]^+$.

### Step 3 1,1,1-trifluoro-*N'*- (3-fluoro-4- (4,4,5,5-tetramethyl-1,3,2-dioxaborolan-2- yl ) phenyl) - *N, N*-dimethylsulfonylimide 52d

Compound **52c** (300 mg, 0.87 mmol), bis(pinacolato)diboron (261 mg, 1.02 mmol), $Pd(dppf)Cl_2$ (126 mg, 0.174 mmol), and potassium acetate (255 mg, 2.61 mmol) were placed into a bottle, 1,4-dioxane (mL) was added, after replaced with $N_2$ three times, the reaction was carried out at 100 °C for 18 h. The reaction solution was filtered and concentrated, and the crude product was purified by column chromatography (PE/EtOAc=0-20%) to obtain compound **52d** (280 mg), yellow oily liquid.

MS (ESI) *m/z* 397 $[M + H]^+$.

### Step 4 N'-(4-(4-amino-7-methyl-7*H*-pyrrolo[2,3-*d*]pyrimidin-5-yl)-3- fluorophenyl ) -1,1,1-trifluoro-*N, N*-dimethylmethanesulfonimide 52e

Compound **1g** (115 mg, 0.42 mmol), compound **52d** (200mg, 0.5 mmol), $Pd(PPh_3)_4$ (97 mg, 0.084 mmol), and $K_3PO_4$ (267mg, 1.26 mmol) were added into a sealed tube, after replaced with $N_2$ three times, 1,4-dioxane (4 mL) and $H_2O$ (0.5 mL) were added, sealed, and the reaction solution was heated up to 100 °C and stirred for 16 h. Dichloromethane (20 mL) and saturated saline solution (10 mL) were addedto extract, and the organic phase was dried over anhydrous sodium sulfate, and concentrated under reduced pressure to obtain a crude product. The crude product was purified by column chromatography (MeOH/DCM=1/30) to obtain compound 52e (85 mg), yellow solid.

MS (ESI) *m/z* 417 $[M + H]^+$.

### Step 5 N'-(4-(4-amino-6-bromo-7-methyl-7H-pyrrolo [2,3-*d*] pyrimidin-5-yl)-3-fluorophenyl) -1,1,1-trifluoro-N, *N*-dimethylmethanesulfonimide 52f

Compound **52e** (85 mg, 0.21 mmol) was dissolved in DMF (3 mL), and a solution of N-bromosuccinimide (41 mg, 0.23 mmol) in DMF (0.5 mL) was added dropwise at 0 °C, and the reaction solution was stirred at 0 °C for 1 h. Then DCM (20 mL) was added, and washed with saturated saline solution (10 mL × 3), and the organic phase was dried over anhydrous sodium sulfate, and concentrated under reduced pressure to obtain a crude product. The crude product was purified by column chromatography (MeOH/DCM=1/40) to obtain compound **52f** (51 mg), yellow solid.

MS (ESI) *m/z* 495 $[M + H]^+$.

### Step 6 *N-* (4- (4-amino-5- (4- (((dimethylamino) (oxo) (trifluoromethyl) - $\lambda^6$-sulfanylidene ) amino) -2-fluorophenyl) -7-methyl-7H-pyrrolo [2,3-*d*] pyrimidin-6-yl) phenyl) methacrylamide 52

Compound **52f** (51mg, 0.103 mmol), compound **4a** (61.2 mg, 0.124 mmol), $Pd(PPh_3)_4$ (23 mg, 0.021 mmol), and $K_3PO_4$ (66 mg, 0.31 mmol) was added into a sealed tube, after replaced with $N_2$ three times, 1,4-dioxane (5 mL) and $H_2O$ (0.5 mL) were added, sealed, and the reaction solution was heated up to 100 °C and stirred for 16 h. Dichloromethane (30 mL) and saturated saline solution (20 mL) were added to extract, and the organic phase was dried over anhydrous sodium sulfate, and concentrated under reduced pressure to obtain a crude product. The crude product was purified by column chromatography (MeOH/DCM=1/30) to obtain compound **52** (18 mg), white solid.

MS (ESI) *m/z* 576.44 $[M + H]^+$.

$^1H$ NMR (400 MHz, MeOD-d4) $\delta$ 8.18 (s, 1H), 7.64 (d, *J* = 8.2 Hz, 2H), *7.28* - 7.19 *(m,* 3H), 6.87 - 6.79 (m, 2H), 5.80 (s, 1H), 5.52 (s, 1H), 4.58 (s, 2H), *3.71* (s, 3H), 3.08 (s, 5H), 2.02 (s, 3H).

**Example 53 *N*-(4-(4-amino-5-(4-(((ethyl(methyl)amino)(oxo)(trifluoromethyl)-λ$^6$-sulfanylidene)amino)-3-fluorophenyl)-7-methyl-7*H*-pyrrolo[2,3-*d*]pyrimidin-6-yl)phenyl) methacrylamide**

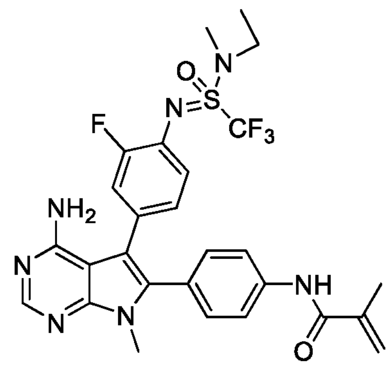

**53**

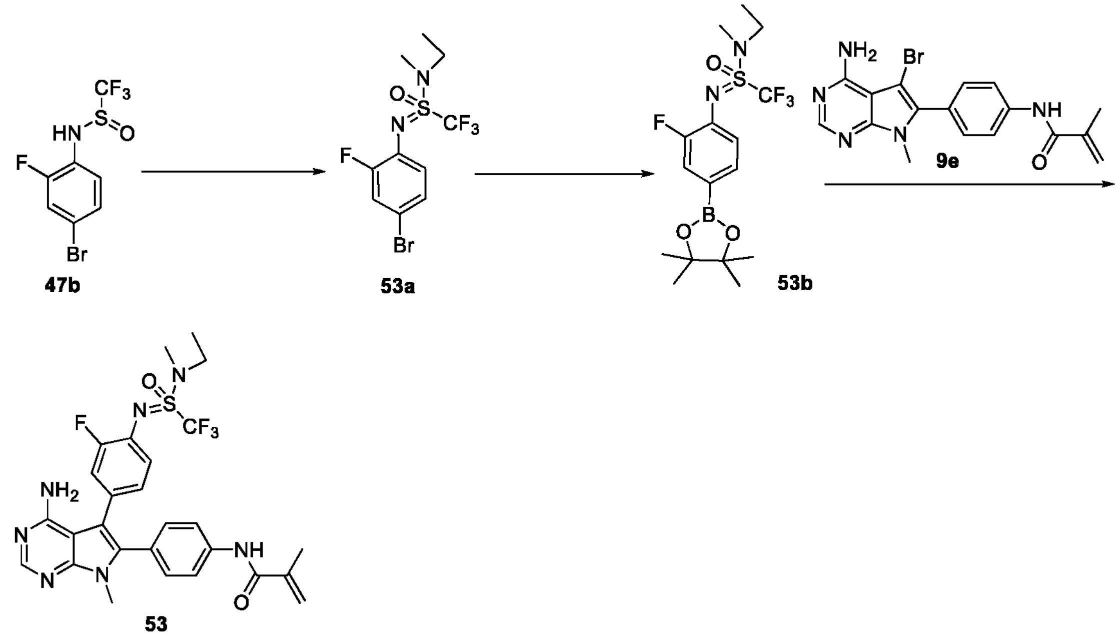

47b 53a 53b 9e

53

**Step 1 *N'*-(4-bromo-2-fluorophenyl)-*N*-ethyl-1,1,1-trifluoro-*N*- methylsulfonylimide 53a**

Compound **47b** (500 mg, 1.49 mmol) and N-chlorosuccinimide (292 mg, 1.64 mmol) were added in acetonitrile (8 mL), a solution of tetrabutylammonium fluoride in THF(1.64 mL, 1.64 mmol, 1M) was added at 0 °C, and the reaction was carried out at room temperature for 30 min. The reaction solution was concentrated, and then acetonitrile (8 mL) and N-methylethylamine (360 mg, 6.0 mmol) were added, and reacted at room temperature for 2 h. The reaction solution was concentrated and dried, and the obtained crude product was purified by column chromatography (PE/EtOAc=0-20%) to obtain compound **53a** (420 mg).

MS (ESI) *m/z* 363.2 [M + H]$^+$

**Step 2 *N*-ethyl-1,1,1-trifluoro-*N'*-(2-fluoro-4-(4,4,5,5-tetramethyl-1,3,2- dioxaborolan-2-yl) phenyl) - *N*-methylmethane sulfonamide 53b**

Compound **53a** (420 mg, 1.16 mmol), bis(pinacolato)diboron (352 mg, 1.39 mmol), Pd(dppf)$Cl_2$ (170 mg, 0.23 mmol), and potassium acetate (340 mg, 3.48 mmol) were placed in a bottle, and 1,4-dioxane (8 mL) was added, after replaced with $N_2$ three timesThe reaction was carried out at 100 °C for 18 h. The reaction solution was filtered and concentrated, and the crude product was purified by column chromatography (PE/EtOAc=0-20%) to obtain compound 53b (360 mg).

MS (ESI) *m/z* 411 $[M + H]^+$

**Step 3 *N*- (4- (4-amino-5- (4- (((ethyl (methyl) amino) (oxo) (trifluoromethyl)-$\lambda^6$-sulfanylidene) amino) -3-fluorophenyl) -7-methyl-7*H*-pyrrolo [2,3-*d*] pyrimidin-6-yl) phenyl) methacrylamide 53**

Compound **9e** (117 mg, 0.305 mmol), compound **53b** (150 mg, 0.366 mmol), $Pd(PPh_3)_4$ (70 mg, 0.061 mmol), and $K_3PO_4$ (200 mg, 0.915 mmol) were added into a sealed tube, after replaced with $N_2$ three times, 1,4-dioxane (5 mL) and $H_2O$ (0.5 mL) were added, sealed, and the reaction solution was heated up to 100 °C and stirred for 16 h. Dichloromethane (30 mL) and saturated saline solution (20 mL) were added to extract, and the organic phase was dried over anhydrous sodium sulfate, and concentrated under reduced pressure to obtain a crude product. The crude product was purified by column chromatography (MeOH/DCM=1/30) to obtain compound 53 (80 mg), white solid.

MS (ESI) *m/z* 589.90 $[M + H]^+$.

$^1H$ NMR (400 MHz, MeOD-d4) $\delta$ 8.32 (s, 1H), 7.78 - 7.52 (m, 2H), 7.26 (dd, *J* = 8.4, 3.5 Hz, 2H), 7.21 - 7.10 (m, 1H), 7.05 - 6.90 (m, 2H), 5.81 (s, 1H), 5.52 (s, 1H), 4.65 (s, 3H), 4.56 (s, 1H), 3.68 (d, *J* = 3.3 *Hz,* 3H), 3.47 (p, *J* = 7.3 Hz, 2H), 3.03 (s, 3H), 1.11 (td, *J* = 7.1, 1.9 Hz, 3H).

**Example 54 *N*-(4- (4-amino-5- (4- (((ethyl (methyl) amino) (oxo) (trifluoromethyl) -$\lambda^6$-sulfanylidene) amino) phenyl) -7-methyl-7*H*-pyrrolo [2,3-*d*] pyrimidin-6-yl) phenyl) methacrylamide**

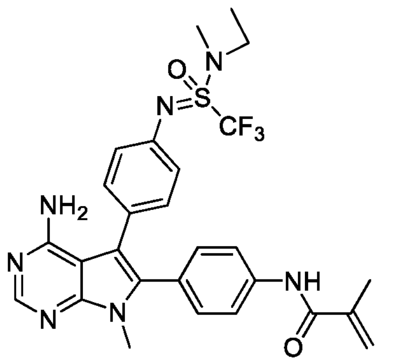

**54**

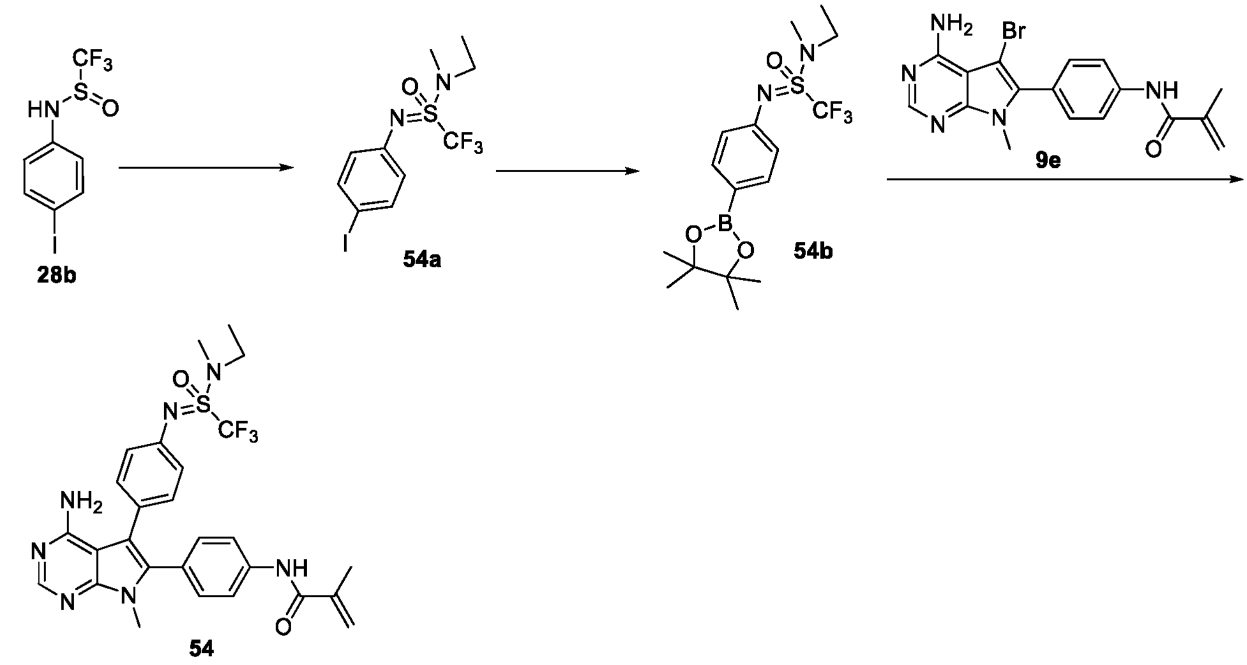

**28b**

**54a**

**54b**

**9e**

**54**

**Step 1 *N*-ethyl-1,1,1-trifluoro-*N*'-(4-iodophenyl)-*N*-methylmethylsulfonyl imide 54a**

Compound **28b** (500 mg, 1.49 mmol) and N-chlorosuccinimide (292 mg, 1.64 mmol) were added in acetonitrile (8 mL), a solution of tetrabutylammonium fluoride in THF(1.64 mL, 1.64 mmol, 1M) was added at 0 °C, and the reaction was carried out at room temperature for 30 min. The reaction solution was concentrated, then acetonitrile (8 mL) and N-methylethylamine (360 mg, 5.96 mmol) were added, and reacted at room temperature for 2 h. The reaction solution was concentrated and dried, and the crude product was purified by column chromatography (PE/EtOAc=0-20%) to obtain compound **54a** (420 mg).

MS (ESI) *m/z* 391.97 $[M + H]^+$

**Step 2 *N*-ethyl-1,1,1-trifluoro-*N*-methyl-*N*'-(4- (4,4,5,5-tetramethyl-1,3,2- dioxaborolan -2-yl) phenyl) methane sulfonamide 54b**

Compound **54a** (220 mg, 0.56 mmol), bis(pinacolato)diboron **(171 mg,** 0.67 mmol), Pd(dppf)$Cl_2$ (83 mg, 0.11 mmol), and potassium acetate (165 mg, 1.68 mmol) were placed in a bottle, and dioxane (6 mL) was added. After replaced with $N_2$ three times, the reaction was carried out at 100 °C for 18 h. The reaction solution was filtered and concentrated, and the crude product was purified by column chromatography (PE/EtOAc=0-20%) to obtain compound **54b** (180 mg).

MS (ESI) *m/z* 393 $[M + H]^+$.

**Step 3 *N- (4- (4-amino-5-* (4- (((ethyl (methyl) amino) (oxo) (trifluoromethyl)-$\lambda^6$-sulfanylidene) amino) phenyl) -7-methyl-7*H*-pyrrolo [2,3-*d*] pyrimidin-6-yl) phenyl) methacrylamide 54**

Compound **9e** (24.6 mg, 0.064 mmol), compound **54b** (30 mg, 0.077 mmol), $Pd(PPh_3)_4$ (14.7 mg, 0.013 mmol), and $K_3PO_4$ (41 mg, 0.192 mmol) were added into a sealed tube, after replaced with $N_2$ three times, 1,4-dioxane (3 mL) and $H_2O$ (0.4 mL) were added, sealed, and the reaction solution was heated up to 100 °C and stirred for 16 h. Then dichloromethane (20 mL) and saturated saline solution (10 mL) were added to extracted, and the organic phase was dried over anhydrous sodium sulfate, and concentrated under reduced pressure to obtain a crude product. The crude product was purified by column chromatography (MeOH/DCM=1/30) to obtain compound 54 (12 mg), white solid.

MS (ESI) *m/z* 572.68 $[M + H]^+$.

$^1H$ NMR (400 MHz, MeOD-d4) $\delta$ 8.18 (s, 1H), 7.68 - 7.60 (m, 2H), 7.30 - 7.22 (m, 2H), 7.22 - 7.14 (m, 2H), 7.08 - 7.00 (m, 2H), 5.80 (s, 1H), 5.52 (d, *J* = 1.9 Hz, 1H), 4.57 (s, 2H), 3.69 (s, 3H), 3.47- 3.41 (m, 2H), 3.01 (s, 3H), 2.02 (d, *J*= 1.2 Hz, 3H), 1.10 (t, *J* = 7.1 Hz, 3H).

**Example 55 *N*-(4-(4-amino-5-(4-((azetidine 1-yl (oxo) (trifluoromethyl) -$\lambda^6$-sulfanylidene)amino)-3-fluorophenyl)-7-methyl-7*H*-pyrrolo[2,3-*d*]pyrimidin-6-yl)phenyl) methacrylamide**

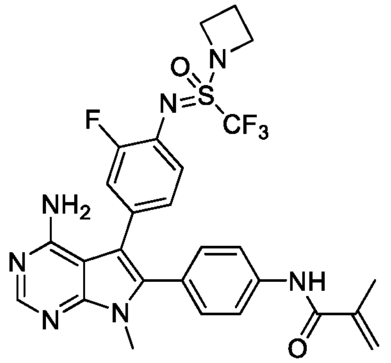

**55**

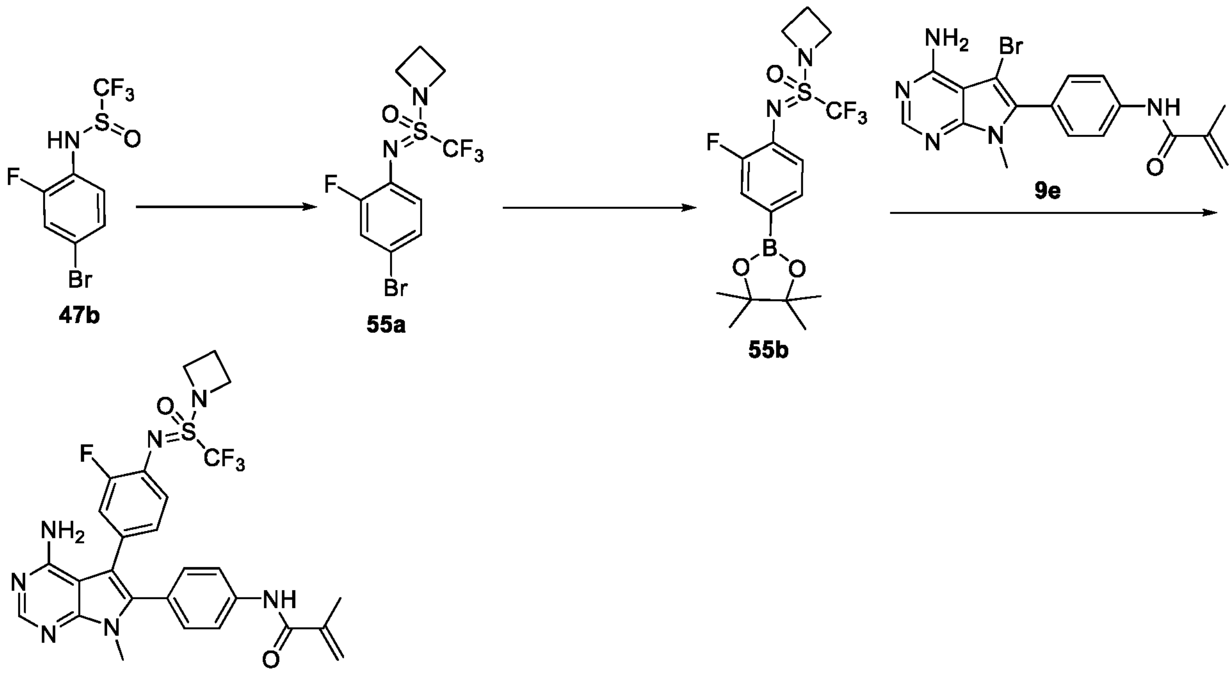

47b

55a

55b

9e

55

**Step 1 1- (N- (4-bromo-2-fluorophenyl)- S-(trifluoromethyl)sulfonylimino)azetidine 55a**

Compound **47b** (200 mg, 0.66 mmol) and N-chlorosuccinimide (106 mg, 0.73 mmol) were added in dry acetonitrile (6 mL), a solution of tetrabutylammonium fluoride in tetrahydrofuran (0.73 mL, 0.73 mmol, 1M) was added at 0 °C, and the reaction was carried out at room temperature for 30 min. The reaction solution was concentrated, then dry acetonitrile (8 mL) and azetidine (128 mg, 2.6 mmol) were added, and reacted at room temperature for 2 h. The reaction solution was concentrated and dried, and the crude product was purified by column chromatography (PE/EtOAc=0-20%) to obtain compound **55a** (130 mg), yellow oily liquid.

MS (ESI) *m/z* 361.2 $[M + H]^+$.

**Step 2 1- (*N*- (2-bromo-4- (4,4,5,5-tetramethyl-1,3,2-dioxaborolan-2-yl) phenyl) - S - (trifluoromethyl) sulfonylimide) azetidine 55b**

Compound **55a** (130 mg, 0.36 mmol), bis(pinacolato)diboron (110 mg, 0.43 mmol), $Pd(dppf)Cl_2$ (53 mg, 0.072 mmol), and potassium acetate (110 mg, 1.1 mmol) were placed in a bottle, 1,4-dioxane (6 mL) was added, after replaced with $N_2$ three times, the reaction was carried out at 100 °C for 18 h. The reaction solution was filtered and concentrated, and the crude product was purified by column chromatography (PE/EtOAc=0-20%) to obtain compound 55b (100 mg).

MS (ESI) *m/z* 408 $[M + H]^+$.

**Step 3 *N*-(4- (4-amino-5- (4- ((azetidine 1-yl (oxo) (trifluoromethyl) -λ⁶-sulfanylidene) amino) -3-fluorophenyl) -7-methyl-7*H*-pyrrolo [2,3-*d*] pyrimidin-6-yl) phenyl) methacrylamide 55**

Compound **9e** (75 mg, 0.19 mmol), compound **55b** (94 mg, 0.23 mmol), Pd(dppf)$Cl_2$ (28 mg, 0.0384 mmol), and $K_3PO_4$ (120 mg, 0.58 mmol) were added into a sealed tube, after replaced with $N_2$ three times, 1,4-dioxane (3 mL) and $H_2O$ (0.4 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/30) to obtain compound 55 (20 mg), white solid.

MS (ESI) *m/z* 588.64 [M + H]$^+$.

$^1$H NMR (400 MHz, MeOD-$d_4$) $\delta$ 8.19 (s, 1H), 7.67 (s, 2H), 7.28 (d, *J* = 7.9 Hz, 3H), 7.16 (s, 1H), 7.00 (s, 2H), 5.81 (s, 1H), 5.53 (s, 1H), 4.24 (s, 2H), 4.18 (s, 2H), 3.68 (s, 3H), 2.37 (s, 2H), 2.03 (s, 3H), 1.30 (s, 1H).

**Example 56 *N*-(4- (4-amino-5- (3-fluoro-4- (1-oxo-1 λ⁶-thiocyclobutan-1-ylidene)amino) phenyl)-7-methyl-7*H*-pyrrolo[2,3-*d*]pyrimidin-6-yl) - phenyl)methacrylamide**

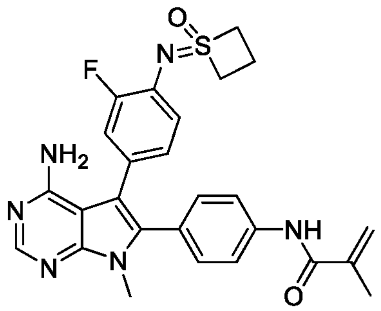

56

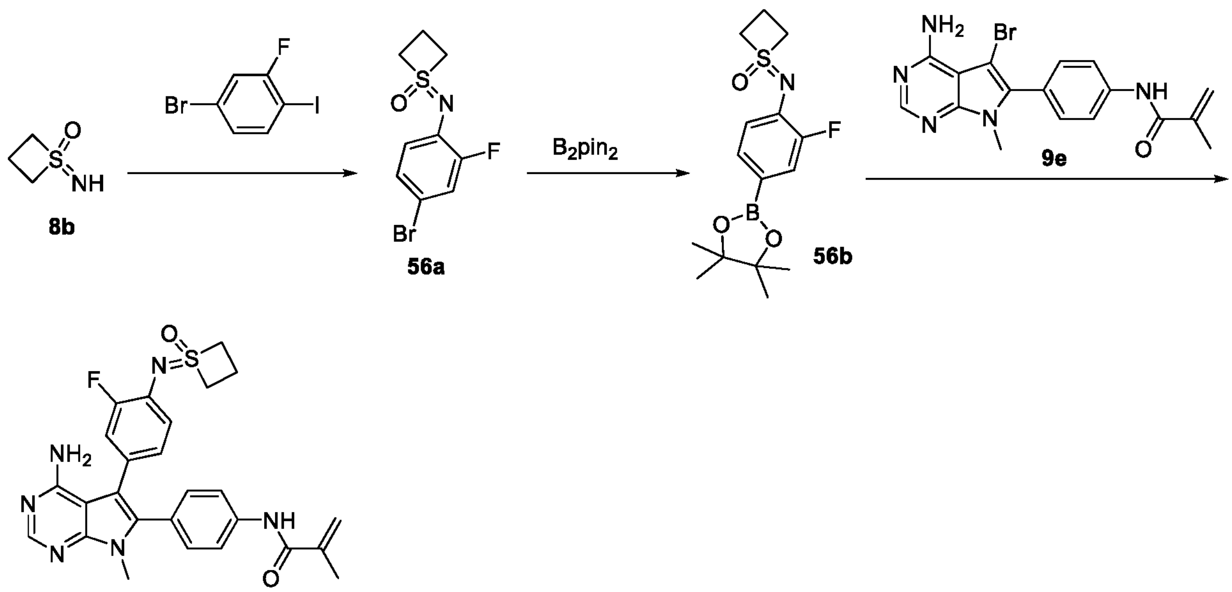


8b

56a

56b

9e

56

**Step 11-((4-bromo-2-fluorophenyl) imino) -1 λ⁶-thietane-1-oxide 56a**

Compound **8b** (720 mg, 6.8 mmol), cesium carbonate (3.3 g, 10.2 mmol), 1-bromo-3-fluoro-4-iodobenzene (2.47 g, 8.2 mmol), $Pd_2(dba)_3$ (187 mg, 0.2 mmol), and Xantphos (354 mg, 0.6 mmol) were added to 1,4-dioxane (15 mL), and the reaction solution was heated up to 100 °C for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product

was purified by column chromatography (PE/EtOAc=4/1 to 3/2) to obtain compound 56a (1.1 g), light yellow solid.

$^1H$ NMR (400 MHz, $CDCl_3$) $\delta$ 7.20 (dd, *J* = 10.0, 2.4 Hz, 1H), 7.13 (dd, *J* = 8.4, 2.4 Hz,, 1H), 6.96 (t, *J* = 8.4 Hz, 1H), 4.31-4.19 (m, 4H), 2.32 (p, *J* = 8.4 Hz, 2H).

**Step 2 1- ((2-fluoro-4- (4,4,5,5-tetramethyl-1,3,2-dioxaborolan-2-yl) phenyl) imino) -1 λ$^6$-thietane-1-oxide 56b**

Compound **56a** (556 mg, 2.0 mmol), bis(pinacolato)diboron (660 mg, 2.6 mmol), Pd(dppf)$Cl_2$ (293 mg, 0.4 mmol), potassium acetate (588 mg, 6.0 mmol), and 1,4-dioxane (10 mL) were added into a sealed tube, after replaced with argon gas three times, sealed, and the reaction solution was heated up to 90 °C and stirred for 16 h. Water (10 mL) was added and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **56b** (356 mg), white solid.

$^1H$ NMR (400 MHz, $CDCl_3$) $\delta$ 7.60-7.34 (m, 2H), 7.06 (t, *J* = 8.1 Hz, 1H), 4.38-4.18 (m, *4H),* 2.39-2.27 (m, 2H), 1.32 (s, 12H).

**Step 3 *N-* (4- (4-amino-5- (3-fluoro-4- (1-oxo-1 λ$^6$-thiocyclobutan-1-ylidene) amino) phenyl) -7-methyl-7*H*-pyrrolo[2,3-*d*]pyrimidin-6-yl) - phenyl)methacrylamide 56**

Compound **56b** (65 mg, 0.2 mmol), compound **9e** (64 mg, 0.17 mmol), $Pd(PPh_3)_4$ (23 mg, 0.02 mmol), and $K_3PO_4$ (106 mg, 0.5 mmol) were added into a sealed tube, after replaced with argon gas three times, 1,4-dioxane (5 mL) and $H_2O$ (0.5 mL) were added , sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/10) to obtain compound **56** (12 mg), white solid.

MS (ESI) *m/z* 505 [M + H]$^+$.

$^1H$ NMR (400 MHz, $CDCl_3$) $\delta$ 8.33 (s, 1H), 7.57 (d, *J* = 8.4 Hz, 3H), 7.21 *(d, J* = 8.4 Hz, 2H), 7.02 (dd, *J* = 9.2, 7.8 Hz, 1H), 6.97-6.86 *(m,* 2H), 5.80 (s, 1H), 5.49 (s, 1H), 5.18 (brs, 2H), 4.35-4.22 (m, 4H), 3.69 *(s,* 3H), 2.35 (q, *J* = 8.4 Hz, 2H), 2.07 (s, 3H).

**Example 57 *N-* (4- (4-amino-5- (4-((3,3-dimethyl-1-oxo-1λ$^6$-thiocyclobutyl-1-ylidene) amino) -3-fluorophenyl) -7-methyl-7H-pyrrolo [2,3-d] pyrimidin-6-yl) phenyl) methacrylamide**

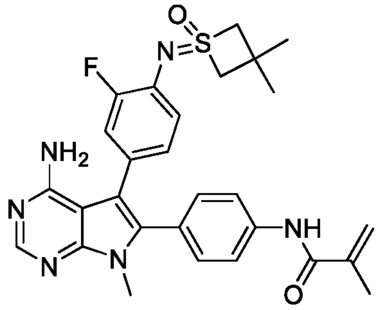

**57**

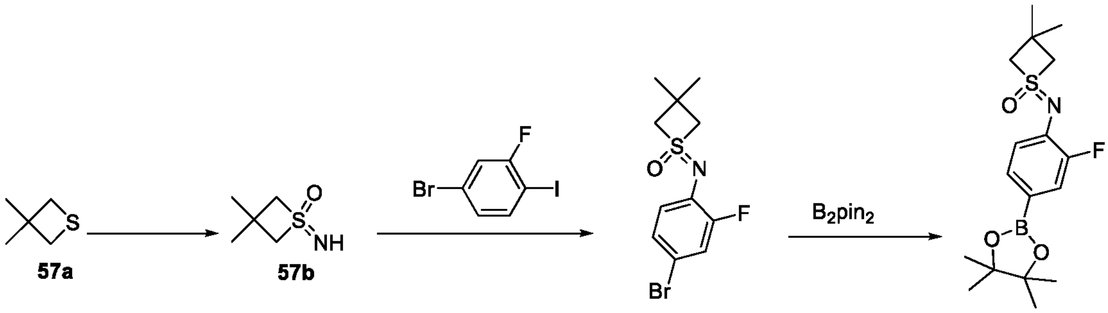


57a 57b 57c 57d

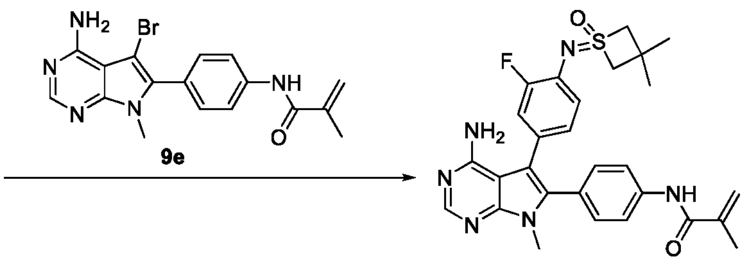

9e

57

**Step 11-amino-3,3-dimethyl-1 λ$^6$-thietane-1-oxide 57b**

3,3-dimethylthietane 57a (1 g, 10 mmol) and ammonium carbamate (1.17 g, 15 mmol) were added into a flask in sequence, then MeOH (100 mL) and iodobenzene diethyl ester (6.44 g, 20 mmol) were added, the reaction solution was stirred open at room temperature for 30 min, and then it was concentrated under reduced pressure to remove the solvent to obtain a crude product. The crude product was purified by column chromatography (MeOH/DCM=1/20) to obtain compound **57b** (1.1 g), colorless oil.

$^1$H NMR (400 MHz, $CDCl_3$) $\delta$ 3.86 (s, 4H), 2.07 (s, 2H), 1.49 (d, *J* = 7.3 Hz, *6H).*

**Step 21-((4-bromo-2-fluorophenyl)imino)-3,3-dimethyl-1λ$^6$-thietan-1-oxide 57c**

Compound **57b** (266 mg, 2 mmol), cesium carbonate (978 mg, 3 mmol), 1-bromo-3-fluoro-4-iodobenzene (722 mg, 2.4 mmol), $Pd_2(dba)_3$ (55 mg, 0.06 mmol), and Xantphos (104 mg, 0.18 mmol) were added to 1,4-dioxane (5 mL), and the reaction solution was heated up to 100 °C for 16 h. The reaction solution was filtered through diatomaceous earth, and the filter cake was washed with ethyl acetate (100 mL) and saturated saline solution (50 mL). The organic phase was dried over anhydrous sodium sulfate and concentrated under reduced pressure to obtain a crude product. The crude product was purified by column chromatography (PE/EtOAc=4/1 to 3/2) to obtain compound **57c** (0.2 g), light yellow solid.

$^1$H NMR (400 MHz, $CDCl_3$) $\delta$ 7.20 (dd, *J* = 10.4, 2.4 Hz, 1H), 7.13 (dd, *J = 8.4, 2.4* Hz, 1H), 6.97 (t, *J* = 8.4 Hz, 1H), 4.04 (d, *J* = 12.8 Hz, 2H), 3.99 (d, *J* = 12.8 Hz, 2H), 1.54 (s, *3H),* 1.52 (s, 3H).

**Step 3 1- ((2-fluoro-4- (4,4,5,5-tetramethyl-1,3,2-dioxaborolan-2-yl) phenyl) imino) -3,3-dimethyl-1 λ$^6$-thietane-1-oxide 57d**

Compound **57c** (200 mg, 0.65 mmol), bis(pinacolato)diboron (216 mg, 0.85 mmol), Pd(dppf)$Cl_2$ (95 mg, 0.13 mmol), potassium acetate (191 mg, 1.95 mmol), and 1,4-dioxane (4 mL) were added into a sealed tube, after replaced with argon gas three times, sealed, and the reaction solution was heated up to 90 °C and stirred for 16 h. Water (10 mL) was added and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **57d** (190 mg), white solid.

$^1$H NMR (400 MHz, $CDCl_3$) δ 7.61-7.34 (m, 2H), 7.06 (t, *J* = 8.1 Hz, 1H), 4.10-3.95 (m, *4H),* 1.53 (s, 3H), 1.50 (s, 3H), 1.32 (s, 12H).

**Step 4 *N*- (4- (4-amino-5- (4- (3,3-dimethyl-1-oxo-1λ$^6$-thiocyclobutyl-1-ylidene) amino) -3-fluorophenyl) -7-methyl-7*H*-pyrrolo[2,3-*d*]pyrimidin-6-yl) phenyl)methacrylamide 57**

Compound **57d** (190 mg, 0.53 mmol), compound **9e** (164 mg, 0.42 mmol), $Pd(PPh_3)_4$ (59 mg, 0.05 mmol), and $K_3PO_4$ (267 mg, 1.26 mmol) were added into a sealed tube, after replaced with argon gas three times, 1,4-dioxane (5 mL) and $H_2O$ (0.5 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/10) to obtain compound **57** (14 mg), white solid.

MS (ESI) *m/z* 533 [M + H]$^+$.

$^1$H NMR (400 MHz, $CDCl_3$) δ 8.34 (s, 1H), 7.58 (d, *J* = 8.8 Hz, 2H), 7.56 *(m,* 1H), 7.22 (d, *J* = 8.8 Hz, 2H), 7.02 (t, *J* = 8.4 Hz, 1H), 6.92 (dd, *J* = 6.8, 2.0 Hz, 1H), 6.90 (dd, *J* = 12.0, 2.0 Hz, 1H), 5.80 (s, 1H), 5.49 (s, 1H), 5.25 (brs, 2H), 4.09 (d, *J* = 12.0 Hz, 2H), 4.01 (d, *J* = 12.0 Hz, *2H),* 3.70 (s, 3H), 2.07 (s, 3H), 1.54 (s, 3H), 1.53 (s, 3H).

**Example 58 *N*-(4-(4-amino-5-(4-(diethyl(oxo)-λ$^6$-sulfanylidene)amino)phenyl) -7-methyl-7H-pyrrolo[2,3-*d*]pyrimidin-6-yl)-3-fluorophenyl)methacrylamide**

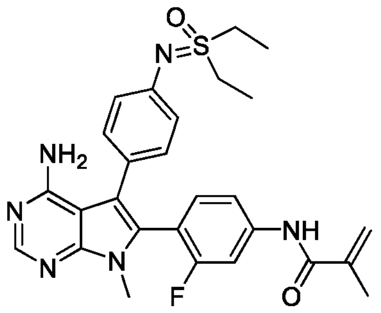

**58**

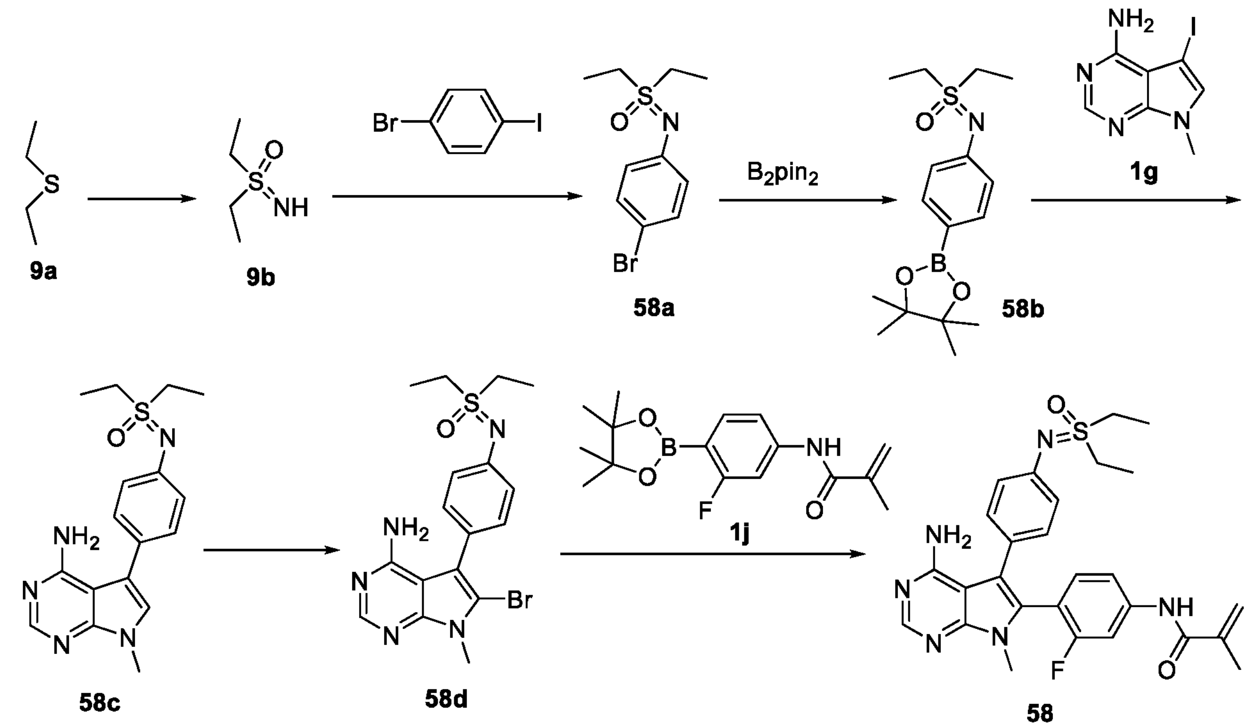


9a 9b 58a 58b 58c 58d 58

**Step 1 imine-1 λ⁶-diethylsulfoxide 9b**

Diethylthioether 9a (4.5 g, 50 mmol) and ammonium carbamate (5.85 g, 75 mmol) were added into a flask in sequence, then MeOH (100 mL) and iodobenzene diethyl ester (32.2 g, 100 mmol) were added. The reaction solution was stirred open at room temperature for 30 min, and then it was concentrated under reduced pressure to remove the solvent to obtain a crude product. The crude product was purified by column chromatography (MeOH/DCM=1/20) to obtain compound **9b** (3.6 g), colorless oil.

$^1$H NMR (400 MHz, $CDCl_3$) $\delta$ 3.12-2.93 (q, *J* = 7.5 Hz, 4H), 2.66 (s, 1H), 1.38 (t, *J* = 7.5 Hz, 6H).

**Step 2 ((4-bromo-phenyl) imino) -1 λ⁶ -diethylsulfoxide 58a**

Compound **9b** (182 mg, 1.5 mmol), cesium carbonate (0.74 g, 2.25 mmol), 1-bromo-4-iodobenzene (0.5 g, 1.8 mmol), $Pd_2(dba)_3$ (46 mg, 0.05 mmol), and Xantphos (83 mg, 0.15 mmol) were added to 1,4-dioxane (5 mL), and the reaction solution was heated up to 100 °C for 16 h. The reaction solution was filtered through diatomaceous earth, the filter cake was washed with ethyl acetate (100 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound **58a** (0.38 g), brownish yellow oil.

$^1$H NMR (400 MHz, $CDCl_3$) $\delta$ 7.30-7.28 (m, 2H), 7.00-6.96 (m, 2H), 3.22-3.13 (m, 4H), 1.39 (t, *J* = 7.5 Hz, 6H).

**Step 3 1- (4- (4,4,5,5-methyl-1,3,2-dioxaborolan-2-yl) - phenyl) imino) -1$\lambda^6$-diethylsulfoxide 58b**

Compound **58a** (380 mg, 1.37 mmol), bis(pinacolato)diboron (420 mg, 1.65 mmol), Pd(dppf)$Cl_2$ (200 mg, 0.274 mmol), potassium acetate (403 mg, 4.11 mmol), and 1,4-dioxane (10 mL) were added into a sealed tube, after replaced with argon gas three times, sealed, and the reaction solution was heated up to 90 °C and stirred for 16 h. Water (10 mL) was added and extracted with DCM (20 mL $\times$ 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **58b** (320 mg), white solid.

**Step 4 (4- (4-amino-7-methyl-7*H*-pyrrolo [2,3-*d*] pyrimidin-6-yl) - phenyl) imino) -1 $\lambda^6$-diethylsulfoxide 58c**

Compound **58b** (265 mg, 0.82 mmol), compound **1g** (206 mg, 0.75 mmol), $Pd(PPh_3)_4$ (87 mg, 0.075 mmol), and $K_3PO_4$ (477 mg, 2.25 mmol) were added into a sealed tube, after replaced with argon gas three times, 1,4-dioxane (8 mL) and $H_2O$ (0.8 mL) were added, sealed, and the reaction solution was heated up to 100 °C and stirred for 16 h. Dichloromethane (20 mL) and saturated saline solution (10 mL) were added to extract, the organic phase was dried over anhydrous sodium sulfate, and concentrated under reduced pressure to obtain a crude product. The crude product was purified by column chromatography (MeOH/DCM=1/10) to obtain compound **58c** (152 mg), light yellow solid.

$^1H$ NMR (400 MHz, $CDCl_3$) $\delta$ 8.33 (s, 1H), 7.31 (d, *J* = 8.3 Hz, 2H), 7.18 *(d, J* = 8.3 Hz, 2H), 6.88 (s, 1H), 5.11 (s, 2H), 3.83 (s, 3H), 3.24 (qd, *J* = 7.5, 4.2 Hz, 4H), 1.44 (t, *J* = 7.5 Hz, 6H).

**Step 5 (4- (4-amino-6-bromo-7-methyl-7*H*-pyrrolo [2,3-*d*] pyrimidin-5-yl) - phenyl) imino) -1 $\lambda^6$-diethylsulfoxide 58d**

Compound **58c** (70 mg, 0.2 mmol) was dissolved in anhydrous DMF (2 mL), a solution of N-bromosuccinimide (40 mg, 0.22 mmol) in DMF (2 mL) was added slowly dropwise at 0 °C, and the reaction was carried out for 1 h. After the reaction was completed, the reaction was quenched with added sodium thiosulfate aqueous solution, extracted with DCM/$H_2O$ twice, and the organic layers were combined, and washed once with saturated salt water, and the organic phase was dried over anhydrous sodium sulfate, concentrated under reduced pressure, and subjected to column chromatography (DCM/MEOH=20/1) to obtain compound 58d (39 mg), light yellow solid.

$^1H$ NMR (400 MHz, $CDCl_3$) $\delta$ 8.26 (s, 1H), 7.30 (d, *J* = 8.4 Hz, 2H), 7.20 (d, *J* = 8.4 Hz, 2H), 5.25 (s, 2H), 3.82 (s, 3H), 3.30-3.22 (m, 4H), 1.45 (t, *J* = 7.4 Hz, 6H).

**Step 6 *N*-(4-(4-amino-5-(4-(diethyl(oxo)-$\lambda^6$-sulfanylidene) amino) phenyl) -7-methyl-7H-pyrrolo [2,3-*d*] pyrimidin-6-yl)-3-fluorophenyl) methacrylamide 58**

Compound **58d** (40 mg, 0.1 mmol), compound 1j (39 mg, 0.12 mmol), $Pd(PPh_3)_4$ (12 mg, 0.01 mmol), and $K_3PO_4$ (64 mg, 0.3 mmol) were added into a sealed tube, after replaced with argon gas three times, 1,4-dioxane (2 mL) and $H_2O$ (0.2 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/10) to obtain compound 58 (22 mg), white solid.

MS (ESI) *m/z* 521 $[M + H]^+$.

$^1H$ NMR (400 MHz, $CDCl_3$) $\delta$ 8.35 (s, 1H), 7.69 (dd, *J* = 11.6, 1.9 Hz, 1H), *7.61* (s, 1H), 7.10-7.00 (m, 6H), 5.80 (s, 1H), 5.51 (d, *J*= 1.5 Hz, 1H), *5.04* (s, 2H), 3.65 (s, 3H), 3.28-3.12 (m, 4H), 2.07 (s, 3H), 1.41 (t, *J* = 7.4 Hz, 6H).

**Example 59 *N*-(4- (4-amino-5- (3-fluoro-4- ((1-oxotetrahydro-1 $\lambda^6$-thiophene-1-ylidene) amino) phenyl) -7-methyl-7*H*-pyrrolo [2,3-*d*] pyrimidin-6-yl) -3- (difluoromethyl) phenyl) methacrylamide**

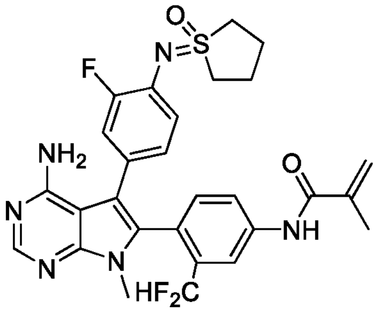

59

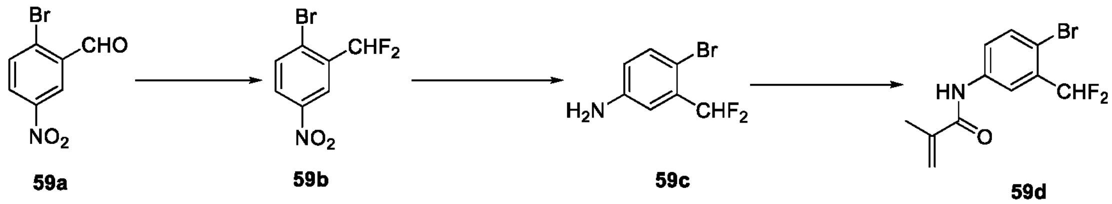

59a 59b 59c 59d

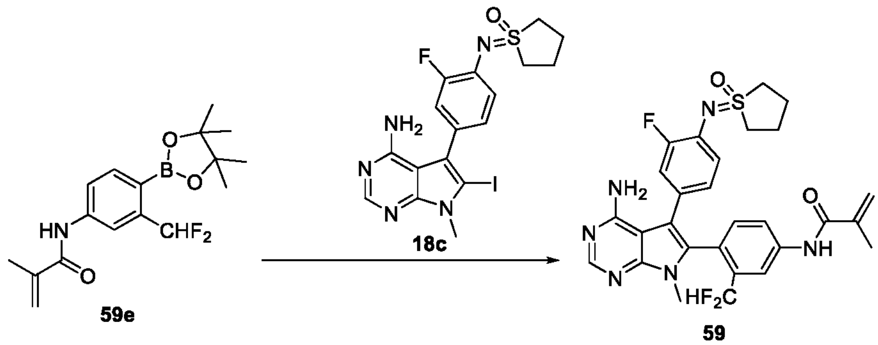

18c

59e 59

**Step 1 1-bromo-2-difluoromethyl-4-nitrobenzene 59b**

To a 100 mL round bottom flask, compound **59a** (2 g, 8.69 mmol), bis (2-methoxyethyl) aminotrifluoride (2.88 g, 13.02 mmol), anhydrous dichloromethane (20 mL) were added sequentially, and the reaction was carried out at room temperature for 4 h, after TLC monitored the completion of the reaction, the reaction was quenched with saturated ammonium chloride solution, extracted with dichloromethane (30 mL × 3), and the organic phases were combined, washed with saturated saline solution (30 mL), and concentrated under reduced pressure to obtain a crude product. The crude product was purified by column chromatography (PE/EA=100/0) to obtain compound **59b** (2.75 g), white solid.

**Step 2 4-bromo-3-difluoromethylaniline 59c**

To a 100 mL round bottom flask, compound **59b** (2.7 g, 10.71 mmol), reduced iron powder (3.0 g, 53.57 mmol), ammonium chloride (2.8 g, 52.35 mmol), ethanol (20 mL), and water (10 mL) were added in sequence, and the reaction was carried out at 80 °C for 4 h, after TLC monitored the completion of the reaction, it was cooled down to room temperature, filtered through diatomaceous earth, extracted with dichloromethane (30 mL × 3), and the organic phases were combined, washed with saturated saline (30 mL), and concentrated under reduced pressure to obtain a crude product. The crude product was purified by column chromatography (PE/EA=3/1) to obtain compound **59c** (1.02 g), white solid.

**Step 3 4-bromo-3-difluoromethylphenylmethacrylamide 59d**

To a 100 mL round bottom flask, compound **59c** (1.02 g, 4.59 mmol), methyl acryloyl chloride (0.458 g, 5.05 mmol), trifluoroacetic acid (0.929 g, 9.19 mmol), and dichloromethane (20 mL) were added in sequence, the reaction was carried out in an ice bath for 1 h, after TLC monitored the completion of the reaction, the reaction was quenched with saturated sodium bicarbonate solution, extracted with dichloromethane (30 mL × 3), and the organic phases were combined, washed with saturated saline solution (30 mL), and concentrated under reduced pressure to obtain a crude

product. The crude product was purified by column chromatography (PE/EA=1/1) to obtain compound **59d** (1.01 g), white solid.

**Step 4 *N*- (3- (difluoromethyl) -4- (4,4,5,5-tetramethyl-1,3,2-dioxaborolan-2-yl) phenyl) methacrylamide 59e**

To a 100 mL round bottom flask, compound **59d** (1.01 g, 3.49 mmol), diborate pinacol ester (1.07 g, 4.19 mmol), $Pd(dppf)Cl_2$ (507 mg, 0.70 mmol), potassium acetate (157 mg, 1.59 mmol) and 1,4-dioxane (20 mL) were added sequentially, the reaction was heated up to 90 °C and reacted for 3 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (PE/EA=1/1) to obtain compound **59e** (120 mg), light yellow solid.

**Step *5* *N*-(4-(4-amino-5-(3-fluoro-4- ((1-oxotetrahydro-1 λ$^6$-thiophene-1-ylidene) amino) phenyl) -7-methyl-7*H*-pyrrolo [2,3-*d*] pyrimidin-6-yl) -3- (difluoromethyl) phenyl) methacrylamide 59**

To a 100 mL round bottom flask, compound **59e** (0.91 g, 0.18 mmol), compound **18c** (0.100 g, 0.21 mmol), and tetrakis (triphenylphosphine) palladium (20.8 mg, 0.018 mmol), anhydrous potassium phosphate (130 mg, 0.54 mmol), 1,4-dioxane (10 mL), and water (1 mL) were added in sequence, the reaction was heated up to 90 °C and reacted for 2 h. After TLC monitored the completion of the reaction, it was cooled down to room temperature, filtered through diatomaceous earth, extracted with ethyl acetate (30 mL × 3), and the organic phases were combined, washed with saturated saline (30 mL), and concentrated under reduced pressure to obtain a crude product. The crude product was purified by column chromatography (DCM/MeOH=6/1) to obtain compound **59** (44 mg), white solid.

MS (ESI) *m*/*z* 568.10 $[M + H]^+$.

$^1H$ NMR (400 MHz, $CDCl_3$) $\delta$ 8.41 (s, 1H), 7.99 (d, *J* = 8.0 Hz, 1H), 7.77 (d, *J* = 5.0 Hz, 2H), 7.37 (d, *J* = 8.3 Hz, 1H), 7.29 (s, 1H), 7.12 *(t, J* = 8.5 Hz, 1H), 6.90 (d, *J* = 8.0 Hz, 2H), 5.87 (s, 1H), *5.56* (s, 1H), 5.13 (s, 2H), 3.56 (s, 3H), 3.51 - 3.40 (m, 2H), 3.25 (s, *2H),* 2.33 (d, *J* = 7.4 Hz, 4H), 2.12 (s, 3H).

**Example 60 *N*-(4- (4-amino-7-methyl-5- (4- ((1-oxotetrahydro-1 λ$^6$-thiophene-1-ylidene) amino) phenyl) -7*H*-pyrrolo [2,3-*d*]pyrimidin-6-yl) phenyl) but-2-ynamide**

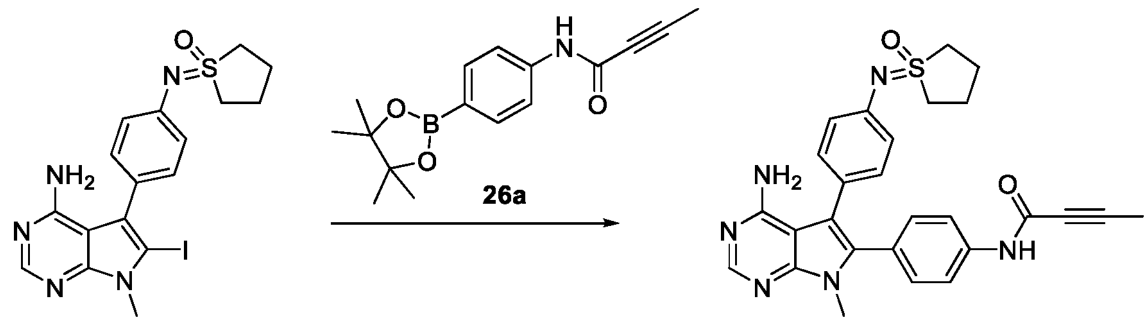


60a

60

Compound 60 was synthesized with reference to Example **12**

MS (ESI) *m*/*z* 499.70 $[M + H]^+$.

$^1H$ NMR (400 MHz, $CDCl_3$) $\delta$ 8.37 (s, 1H), 7.63 - 7.50 (m, 4H), 7.40 (d, *J* = 27.0 Hz, 3H), 6.95 (s, 1H), 5.26 (s, 2H), 3.86 (s, 3H), 3.25 (d, *J* = 5.5 Hz, 2H), 3.13 (s, 2H), 2.05 (s, 3H), 2.03 (d, *J* = 21.8 Hz, 4H).

**Example 61 *N*-(4-(4-amino-5-(2-fluoro-4-((1-oxotetrahydro-1λ$^6$-thiophene-1-ylidene) amino)phenyl)-7-methyl-7*H*-pyrrolo[2,3-*d*]pyrimidin-6-yl)-3-fluorophenyl) methacrylamide**

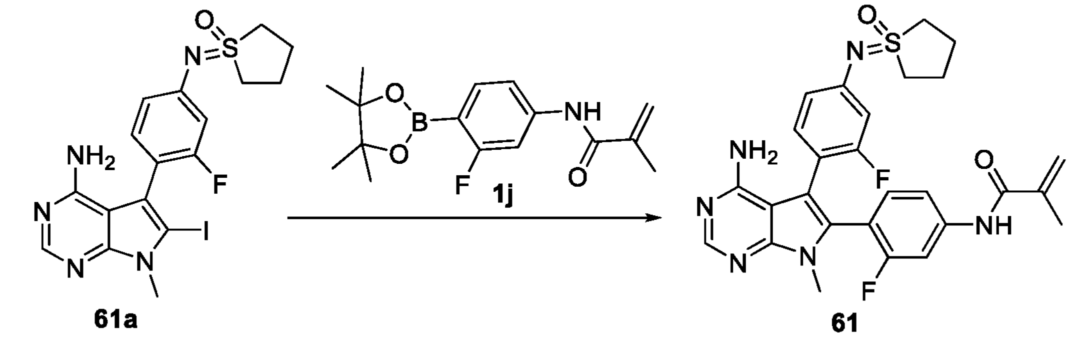


61a 61

Compound **61** was synthesized with reference to Example **12.**

MS (ESI) *m/z* 537.57 [M + H]+.

$^1H$ NMR (400 MHz, $CDCl_3$) δ 8.36 (s, 1H), 7.81 - 7.66 (m, 2H), 7.30 (dd, *J* = *8.9,* 4.2 Hz, 1H), 7.23 (d, *J* = 11.4 Hz, 1H), 7.15 (dd, *J* = 8.3, 1.7 Hz, 1H), 7.04 (s, 1H), 5.84 (s, 1H), 5.53 (s, 1H), 5.18 (s, 2H), *3.86* (s, 3H), 3.33 (dd, *J* = 12.7, 6.5 Hz, 2H), 3.18 (dd, *J* = 12.9, 6.7 Hz, 2H), *2.38* - 2.13 (m, 4H), 2.10 (s, 3H).

**Example 62 *N*-(4-(4-amino-5-(4-(ethyl(isopropyl)(oxo)-1λ$^6$-sulfanylidene)phenyl) -7-methyl-7*H*-pyrrolo[2,3-*d*] pyrimidin-6-yl)-phenyl)methacrylamide**

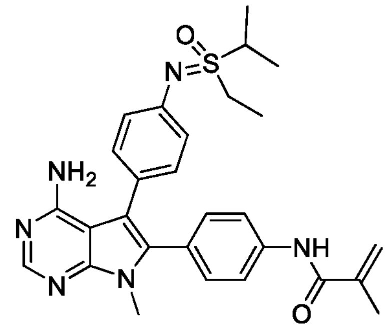

**62**

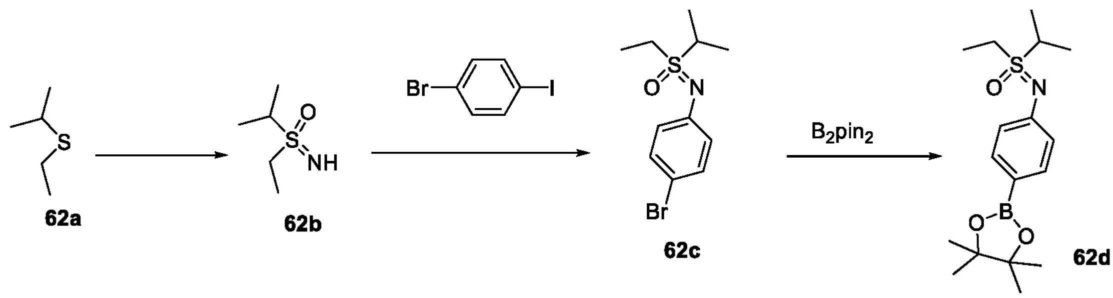


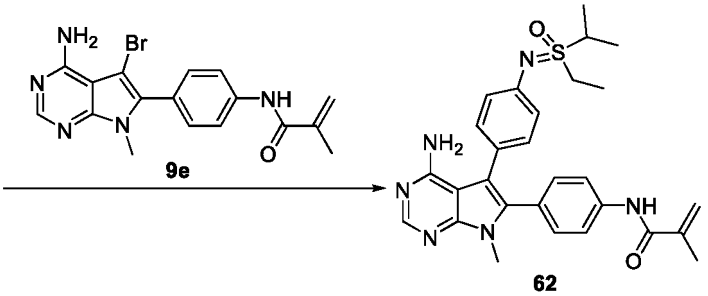

**Step 1 (imino) (isopropyl) -1 λ$^6$-ethylsulfoxide**

Ethyl isopropyl sulfide (3 g, 30 mmol) and ammonium carbamate (3.5 g, 45 mmol) were added to a flask sequentially, and then MeOH (100 mL) and iodobenzene diethyl ester (19.3 g, 60 mmol) were added. The reaction solution was stirred open at room temperature for 30 min, and then concentrated under reduced pressure to remove the solvent to obtain a crude product. The crude product was purified by column chromatography (MeOH/DCM=1/20) to obtain compound **62b** (4 g), light brown oil.

$^1$H NMR (400 MHz, $CDCl_3$) $\delta$ 3.18 (hept, *J* = 6.8 Hz, 1H), 3.03 (q, *J* = 7.5 *Hz,* 2H), 2.05 (s, 1H), 1.42-1.37 (m, 9H).

**Step 2 ((4-bromophenyl) imino) (isopropyl) -1 λ$^6$-ethylsulfoxide 62c**

Compound **62b** (675 mg, 5 mmol), cesium carbonate (2.45 g, 7.5 mmol), 1-bromo-4-iodobenzene (1.7 g, 6 mmol), $Pd_2(dba)_3$ (137 mg, 0.15 mmol), and Xantphos (260 mg, 0.45 mmol) were added to 1,4-dioxane (10 mL), and the reaction solution was heated up to 100 °C for 16 h. The reaction solution was filtered through diatomaceous earth, the filter cake was washed with ethyl acetate (100 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound **62c** (1.3 g), brownish yellow oil.

$^1$H NMR (400 MHz, $CDCl_3$) $\delta$ 7.27 (d, *J* = 8.7 Hz, 2H), 6.98 (d, *J* = 8.7 Hz, 2H), 3.39 (hept, *J= 6.9* Hz, 1H), 3.22-3.06 (m, 2H), 1.46 (d, *J* = 6.8 Hz, 3H), 1.41-1.35 *(m,* 6H).

**Step 3 (isopropyl) ((4- (4,4,5,5-tetramethyl-1,3,2-dioxaborolan-2-yl) phenyl) imino) -1 λ$^6$-diethylsulfoxide 62d**

Compound **62c** (1.3 g, 4.5 mmol), bis(pinacolato)diboron (1.4 g, 5.4 mmol), Pd(dppf)$Cl_2$ (656 mg, 0.9 mmol), potassium acetate (1.3 g, 13.5 mmol), and 1,4-dioxane (12 mL) were added into a sealed tube, after replaced with argon gas three times, sealed, and the reaction solution was heated up to 90 °C and stirred for 16 h. Water (10 mL) was added and extracted with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **62d** (1.4 g), light yellow solid.

**Step 4: *N-* (4- (4-amino-5- (4- (ethyl (isopropyl) (oxo) -1 λ$^6$-sulfanylidene) phenyl) -7-methyl-7*H*-pyrrolo [2,3-*d*] pyrimidin-6-yl) - phenyl) methacrylamide 62**

Compound **62d** (85 mg, 0.24 mmol), compound **9e** (77 mg, 0.2 mmol), $Pd(PPh_3)_4$ (23 mg, 0.02 mmol), and $K_3PO_4$ (127 mg, 0.6 mmol) were added into a sealed tube, after replaced with argon gas three times, 1,4-dioxane (2 mL) and $H_2O$ (0.2 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (MeOH/DCM=1/10) to obtain compound **62** (33 mg), white solid.

MS (ESI) *m/z* 517 $[M + H]^+$.

$^1$H NMR (400 MHz, $CDCl_3$) $\delta$ 8.34 (s, 1H), 7.53 (d, *J* = 8.4, 2H), 7.51 (s, 1H), 7.22 (d, *J* = 8.4, 2H), 7.09-7.03 (m, 4H), 5.79 (s, 1H), 5.48 (q, *J = 1.6* Hz, 1H), 4.99 (s, 2H), 3.70 (s, 3H), 3.41 (hept, *J* = 6.9 Hz, 1H), 3.26-3.08 (m, 2H), 2.07 (s, 3H), 1.48 (d, *J* = 6.8 Hz, 3H), 1.43 (d, *J* = 7.0 Hz, 3H), 1.40 (t, *J* = 7.4 Hz, 3H).

**Example 63 *N-* (4- (4-amino-5- (4- ((3,3-dimethyl-1-oxo-1*λ*$^6$- thiocyclobutyl-1- ylidene) amino) phenyl) -7-methyl-7*H*-pyrrolo [2,3-*d*] pyrimidin-6-yl) phenyl) methacrylamide**

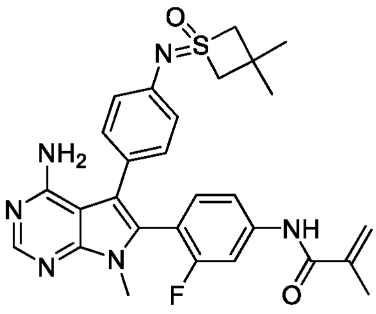

**63**

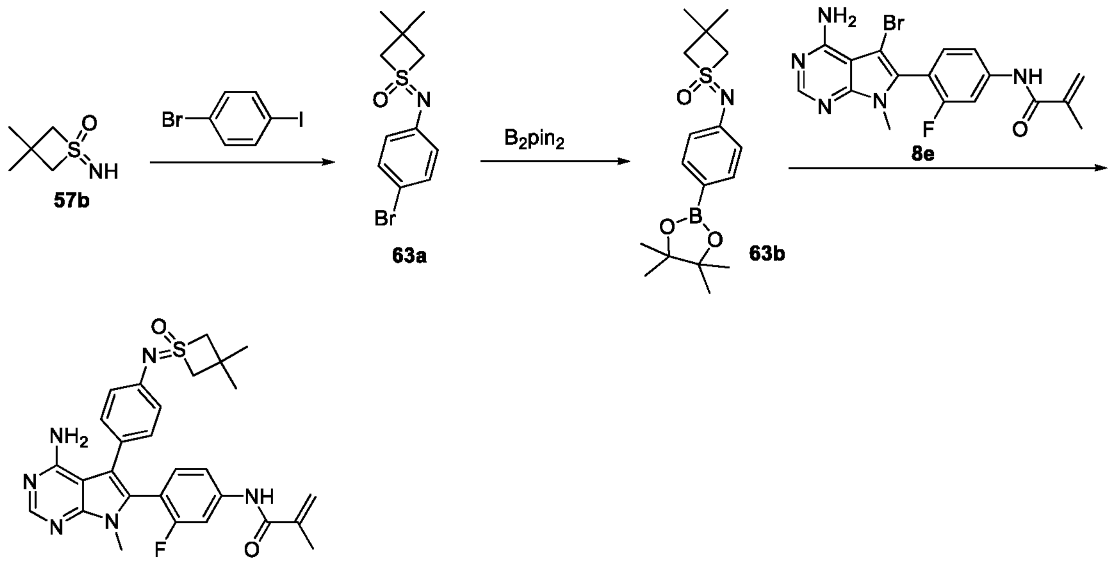


57b 63a 63b 63

**Step 11- ((4-bromo-phenyl) imino) -3,3-dimethyl-1 $\lambda^6$-thietane-1-oxide 63a**

Compound **57b** (760 mg, 5.7 mmol), cesium carbonate (2.78 g, 8.6 mmol), 1-bromo-4-iodobenzene (1.94 g, 6.9 mmol), $Pd_2(dba)_3$ (155 mg, 0.17 mmol), and Xantphos (297 mg, 0.51 mmol) were added to 1,4-dioxane (15 mL), and the reaction solution was heated up to 100 °C for 16 h. The reaction solution was filtered through diatomaceous earth, the filter cake was washed with ethyl acetate (100 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound 63a (1.2 g), light yellow solid.

**Step 3 1- (4- (4,4,5,5-tetramethyl-1,3,2-dioxaborolan-2-yl) phenyl) imino) -3,3-dimethyl-1 $\lambda^6$-thietane-1-oxide 63b**

Compound **63a** (611 mg, 2.1 mmol), bis(pinacolato)diboron ester (700 mg, 2.75 mmol), $Pd(dppf)Cl_2$ (307 mg, 0.42 mmol), potassium acetate (617 mg, 6.3 mmol), and 1,4-dioxane (10 mL) were added into a sealed tube, after replaced with argon gas three times, sealed, and the reaction solution was heated up to 90 °C and stirred for 16 h. Water (10 mL) was added to extract with DCM (20 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **63b** (590 mg), white solid.

$^1$H NMR (400 MHz, $CDCl_3$) $\delta$ 7.70 (d, $J$ = 8.3 Hz, 2H), 7.00 (d, $J$ = 8.3 Hz, 2H), 3.95 (d, $J$= 1.6 Hz, 4H), 1.53 (s, 3H), 1.48 (s, 3H), 1.33 (s, 12H).

**Step 4 *N*- (4- (4-amino-5- (4- (3,3-dimethyl-1-oxo-1$\lambda^6$-thiocyclobutyl-1-ylidene) amino) phenyl) -7-methyl-7*H*-pyrrolo [2,3-*d*] pyrimidin-6-yl) phenyl) methacrylamide 63**

Compound **63b** (144 mg, 0.43 mmol), compound **8e** (145 mg, 0.36 mmol), $Pd(PPh_3)_4$ (42 mg, 0.04 mmol), and $K_3PO_4$ (229 mg, 1.08 mmol) were added into a sealed tube, after replaced with argon three times, 1,4-dioxane (4 mL) and $H_2O$ (0.4 mL) were added, sealed, and the reaction solution was heated up to 100 °C and stirred for 16 h. Dichloromethane (20 mL) and saturated saline solution (10 mL) were added to extract, and the organic phase was dried over anhydrous sodium sulfate, and concentrated under reduced pressure to obtain a crude product. The crude product was purified by column chromatography (DCM/MeOH=10/1) to obtain compound 63 (90 mg), white solid.

MS (ESI) *m/z* 533 $[M + H]^+$.

$^1$H NMR (400 MHz, $CDCl_3$) $\delta$ 8.36 (s, 1H), 7.71 (dd, *J* = 11.5, 1.9 Hz, 1H), 7.58 *(s,* 1H), 7.15-7.03 (m, 4H), 6.94 (d, *J* = 8.1 Hz, 2H), 5.80 (s, 1H), 5.51 *(d, J* = 1.7 Hz, 1H), 5.02 (s, 2H), 4.00 (d, *J* = 12.8 Hz, 2H), 3.94 (d, *J* = 12.8 Hz, 2H), 3.65 (s, 3H), 2.07 (s, 3H), 1.54 (s, 3H), 1.52 (s, 3H).

**Example 64 *N*- (4- (4-amino-7-cyano-1-methyl-3- (4- ((1-oxotetrahydro-2*H*-1$\lambda^6$-thiopyran-1-ylidene) amino) phenyl) -1H pyrrolo [3,2-c] pyridine-2-yl) -3-fluorophenyl) methacrylamide**

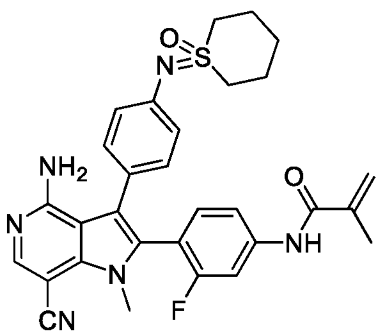

**64**

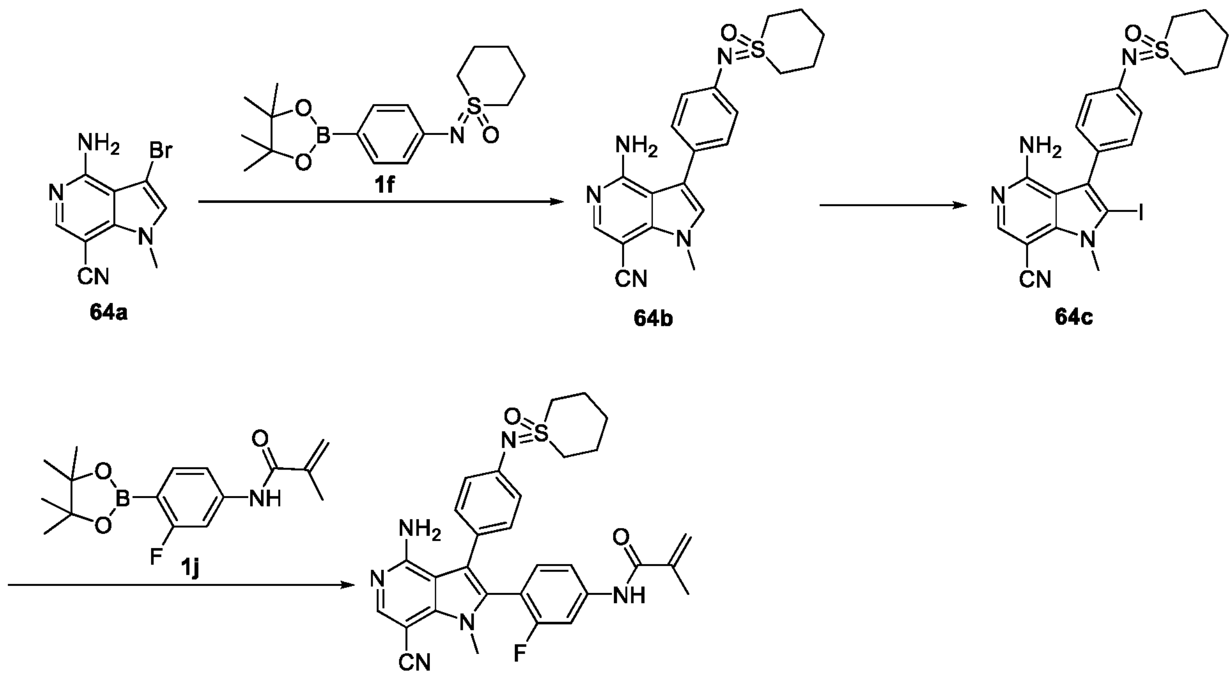

**64a** **1f** **64b** **64c** **1j** **64**

**Step 1 4-amino-1-methyl-3- (4- ((1-oxotetrahydro-2*H*-1$\lambda^6$-thiopyran-1-ylidene) amino) phenyl)-1*H*-pyrrolo[3,2-c] pyridine-7-nitrile 64b**

Compound 64a (45 mg, 0.179 mmol), compound **1f** (73 mg, 0.215 mmol), Pd(DtBPF)$Cl_2$ (12 mg, 0.018 mmol), and cesium fluoride (82 mg, 0.538 mmol) were placed in a 25 mL two necked flask, after replaced with argon gas three times, DMF/$H_2O$ (v/v=8/1, 2.7 mL) was added to react overnight at 90 °C. After the reaction was completed, the reaction solution was poured into water (20 mL) and extracted with ethyl acetate (50 mL × 3) three times. The organic phase was concentrated, and the crude product was purified by column chromatography (DCM/MeOH=30/1) to obtain compound **64b** (40 mg), white solid.

MS (ESI) *m/z* 380.1 $[M + H]^+$.

**Step 2 4-amino-2-iodo-1-methyl-3- (4- ((1-oxotetrahydro-2*H*-1$\lambda^6$-thiopyran-1-ylidene) amino) phenyl) -1*H*-pyrrolo [3,2-c]pyridine-7-nitrile 64c**

Compound **64b** (40 mg, 0.105 mmol) was placed in a 25 mL reaction flask, dichloromethane (4.5 mL) and trifluoroacetic acid (25 μL) were added in sequence. At 0 °C, N-iodosuccinimide (27 mg, 0.116 mmol) was added, and the reaction was carried out at maintained 0 °C for 1 h. After the reaction was completed, the reaction solution was concentrated, and the crude product was purified by column chromatography (DCM/MeOH=30/1) to obtain compound 64c (42 mg), white solid.

MS (ESI) *m/z* 505.9 $[M + H]^+$.

**Step 3 *N*-(4-(4-amino-7-cyano-1-methyl-3-(4-((1-oxotetrahydro-2*H*-1 $\lambda^6$-thiopyran -1-ylidene) amino) phenyl) -1*H* pyrrolo [3,2-c] pyridine-2-yl) -3-fluorophenyl) methacrylamide 64**

Compound **64c** (40 mg, 0.083 mmol), compound **1j** (31 mg, 0.100 mmol), Pd(dppf)$Cl_2$ (7 mg, 0.008 mmol), and potassium phosphate (53 mg, 0.250 mmol) were placed in a 25 mL two necked flask, after replaced with argon gas three times, DMF/$H_2O$ (v/v=8/1, 1.8 mL) was added to dissolve, and the reaction was carried out at 90 °C. After the reaction was completed, the reaction solution was poured into water (20 mL) and extracted with ethyl acetate (50 mL × 3) three times. The organic phase was concentrated, and the crude product was purified by column chromatography (DCM/MeOH=20/1) to obtain compound **64** (13 mg), white solid.

MS (ESI) *m/z* 557.0 $[M + H]^+$.

$^1H$ NMR (400 MHz, $CDCl_3$) δ 8.15 (s, 1H), 7.70 (d, *J* = 11.4 Hz, 1H), 7.63 (s, *1H),* 7.39 (dd, *J* = 20.0, 8.1 Hz, 2H), 7.33 - 7.27 (m, 2H), 7.18 (d, *J* = 8.0 Hz, 1H), 6.89 (s, 1H), 5.83 (s, 1H), 5.51 (s, 1H), 5.31 (s, 2H), 4.06 (s, 3H), 3.24 (s, 2H), 3.05 (t, *J* = 11.1 Hz, 2H), 2.08 (s, 3H), 1.99 - 1.87 (m, 2H), 1.86 - 1.75 (m, 2H), 1.56 - 1.42 (m, 2H).

**Example 65 1-(4-(4-amino-7-methyl-5-(4-((1-oxotetrahydro-2*H*-1 $\lambda^6$-thiopyran-1-ylidene)amino)phenyl)-7*H*-pyrrolo[2,3-*d*]pyrimidin-6-yl)-3,6-dihydropyridine-1(2H)-yl) -2-fluoropropan-2-enyl-1-one**

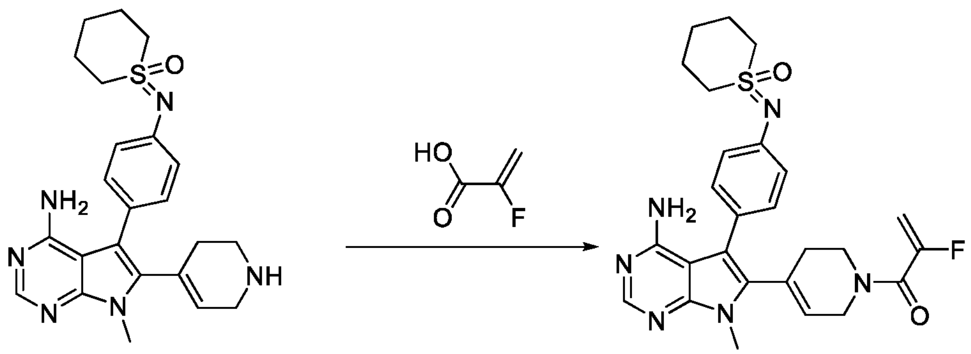

2c

65

Compound **2c** (176 mg, 0.40 mmol), 2-fluoroacrylic acid (36.3 mg, 0.40 mmol), and DIPEA (517 mg, 4.0 mmol) were added into a round bottomed reaction flask, dry DMF (5 mL) was added to dissolve, then HATU (228 mg, 0.60 mmol) was added, and stirred at room temperature for 4 h. After the reaction was completed, it was extracted with EA/$H_2O$ three times, and the organic layers were combined, washed with saturated saline solution, dried over anhydrous sodium sulfate,

concentrated under reduced pressure, and purified by column chromatography (DCM/MeOH=30/1) to obtain compound **65** (136 mg), yellow solid.

MS (ESI) *m/z* 509.2 [M+H]+.

$^1$H NMR (400 MHz, MeOD-d4) $\delta$ 8.35 (s, 1H), 7.46-7.33 (m, 4H), 6.05 (s, 1H), 5.36-5.11 (m, 2H), *4.25* (d, *J* = 33.9 Hz, 2H), 3.85 (s, 3H), 3.81-3.73 (m, 2H), 3.70 (t, *J* = 5.5 Hz, 2H), 3.67-3.57 (m, 2H), 2.30-2.19 (m, 2H), 2.18-2.07 (m, 4H), 1.90-1.79 (m, 1H), 1.78-1.68 (m, 1H).

**Example 66 1- (4- (4-amino-7-methyl-5- (4- (1-oxotetrahydro-2*H*-1λ⁶-thiopyran-1-ylidene) amino) phenyl) -7*H*-pyrrolo[2,3-*d*]pyrimidin-6-yl) -3,6-dihydropyridine-1 *(2H)* - yl) but-2-ynyl-1-one**

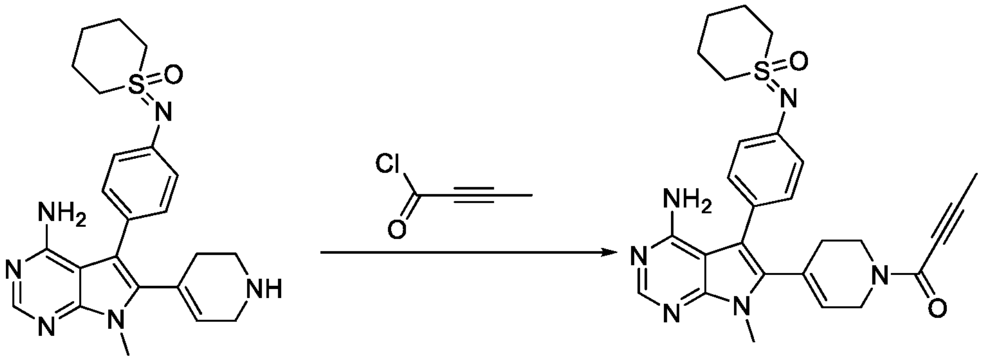

2c

66

Compound 2c (150 mg, 0.34 mmol) was dissolved in dry DCM (3 mL), and triethylamine (35 mg, 0.34 mmol) was added at 0 °C. But-2-ynyl chloride (34.9 mg, 0.34 mmol) was diluted with DCM (2 mL), and added slowly dropwise into the above reaction solution, and stirred at 0 °C for 1 h. After the reaction was completed, it was extracted with DCM/$H_2O$ three times, and the organic layers were combined, washed with saturated saline solution, dried over anhydrous sodium sulfate, concentrated under reduced pressure, and purified by column chromatography (DCM/MeOH=30/1) to obtain compound **66** (73 mg), yellow solid

MS (ESI) *m/z* 503.2 [M+H]+.

$^1$H NMR (400 MHz, MeOD-d4) $\delta$ 8.35 (s, 1H), 7.48-7.22 (m, 4H), 6.10-5.96 (m, 1H), 4.43 (q, *J* = *2.8* Hz, 1H), 4.18 (q, *J* = 2.8 Hz, 1H), 3.88 (t, *J* = 5.6 Hz, 1H), 3.84 (d, *J* = 1.2 Hz, 3H), 3.75-3.70 (m, 1H), 3.69-3.63 (m, 2H), 3.61-3.50 (m, 2H), 2.28-2.21 (m, 1H), 2.20-2.09 (m, 5H), 2.07 (s, 1H), 2.03 (s, *2H),* 1.88-1.77 (m, 1H), 1.76-1.67 (m, 1H).

**Example 67 (*E*)-1-(4-(4-amino-7-methyl-5- (4- ((1-oxotetrahydro-2*H*-1λ⁶- thiopyran -1-ylidene) amino) phenyl) -7*H*-pyrrolo [2,3-*d*] pyrimidin-6-yl) -3,6-dihydropyridine-1 (2*H*)-yl) -4-(dimethylamino) but-2-enyl-1-one**

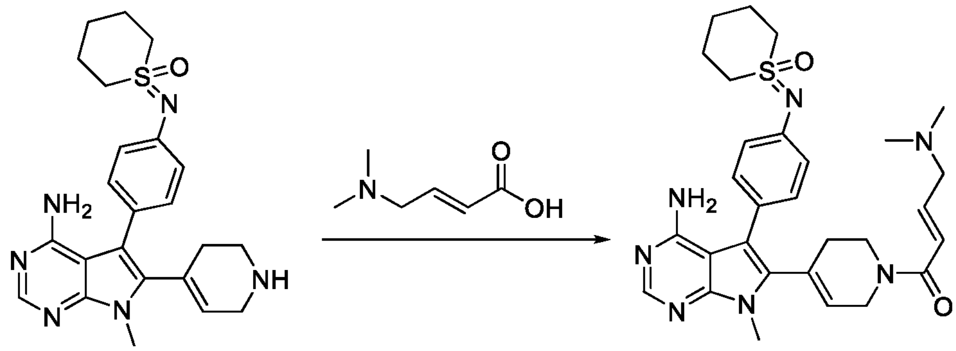

2c

67

Compound **2c** (176 mg, 0.4 mmol) and (*E*) -4- (dimethylamino) but-2-olefine acid (66 mg, 0.4 mmol) were dissolved in DMF (4 mL), triethylamine (404.8 mg, 4 mmol) and T3P (191 mg, 0.6 mmol) were added in sequence at 0 °C overnight. After the reaction was completed, it was extracted with ethyl acetate/$H_2O$ three times , the organic layers were

combined, washed with saturated saline solution, dried over anhydrous sodium sulfate, concentrated under reduced pressure, and perform column chromatography (DCM/MeOH=20/1) to obtain compound 67 (41 mg), yellow solid.

MS (ESI) *m/z* 548.3 $[M+H]^+$.

$^1H$ NMR (400 MHz, MeOD-d4) $\delta$ 8.37 (s, 1H), 7.61-7.35 (m, 4H), 7.10-6.91 (m, 1H), 6.82-6.60 (m, 1H), 6.06 (s, 1H), 4.34 (d, *J* = 3.3 Hz, 1H), 4.25 (d, *J* = 3.3 Hz, 1H), 4.04-3.92 (m, 4H), 3.85 (s, 3H), 3.84-3.67 (m, 4H), 2.92 (d, *J* = 6.4 Hz, 6H), 2.32-2.12 (m, 6H), 1.94-1.72 (m, 2H).

**Example 68 *N*-(4-(4-amino-7-methyl-5-(4-((1-oxotetrahydro-2*H*-1 $\lambda^6$-thiopyran -1-ylidene)amino)phenyl)-7*H*-pyrrolo[2,3-*d*]pyrimidin-6-yl)phenyl)-2-fluoroacrylamide**

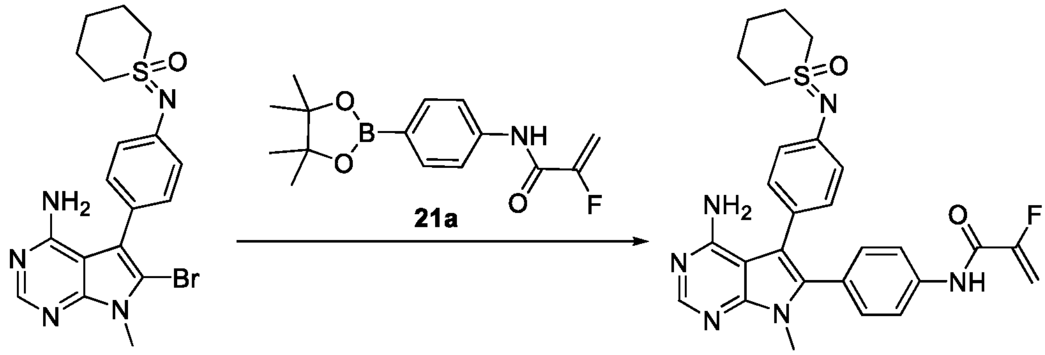


**1i** **68**

Compound **1i** (200 mg, 0.46 mmol) was dissolved in 1,4-dioxane (5 mL) and water (1 mL), compound **21a** (161.3 mg, 0.55 mmol), $Pd(PPh_3)_4$ (106.3 mg, 0.092 mmol), and $K_3PO_4$ (293 mg, 1.38 mmol) were added in sequence. After the air in the reaction bottle was replaced with nitrogen gas, the reaction was stirred overnight at 100 °C. After the reaction was completed, it was filtered with silica gel, and the filtrate was recovered, concentrated under reduced pressure, dissolved in ethyl acetate, washed with saturated saline solution, dried over anhydrous sodium sulfate, concentrated under reduced pressure and subjected to column chromatography (DCM/MeOH=30/1) to obtain compound **68** (118 mg), white solid.

MS (ESI) *m/z* 519.2 $[M+H]^+$.

$^1H$ NMR (400 MHz, MeOD-d4) $\delta$ 8.36 (s, 1H), 7.74 (d, *J* = 8.7 Hz, 2H), *7.32 (d, J* = 8.7 Hz, 2H), 7.15 (d, *J* = 8.5 Hz, 2H), 7.08 (d, *J* = 8.5 Hz, 2H), 5.73 (dd, *J* = 46.5, 3.4 Hz, 1H), 5.32 (dd, *J* = 15.1, 3.5 Hz, 1H), 3.78 (s, 3H), 3.43-3.34 (m, 2H), 3.29-3.19 (m, 2H), 2.09-1.98 (m, 4H), 1.79-1.61 (m, 2H).

**Example 69 *N*- (4- (4-amino-5- (3-fluoro-4- ((1-oxotetrahydro-2*H*-1$\lambda^6$-thiopyran -1-ylidene) amino) phenyl) -7-methyl-7*H*-pyrrolo [2,3-*d*] pyrimidin-6-yl) phenyl) methacrylamide**

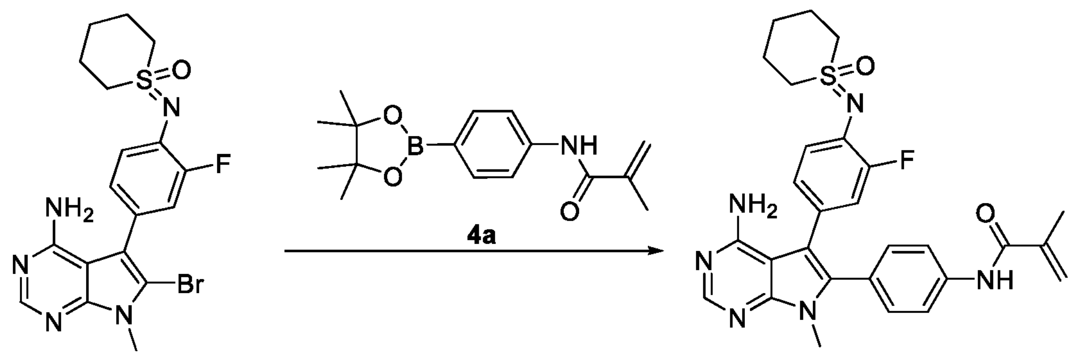


**7d** **69**

Compound **7d** (300 mg, 0.67 mmol) was dissolved in 1,4-dioxane (8 mL) and water (2 mL), and compound **4a** (229.2 mg, 0.8 mmol), $Pd(PPh_3)_4$ (154.8 mg, 0.13 mmol), and $K_3PO_4$ (426.7 mg, 2.01 mmol) were added in sequence. After the air in the reaction flask was replaced with nitrogen gas, the reaction was stirred overnight at 100 °C. After the

reaction was completed, it was filtered with silica gel, and the filtrate was recovered, concentrated under reduced pressure, dissolved in ethyl acetate, washed with saturated saline solution, dried over anhydrous sodium sulfate, concentrated under reduced pressure and perform column chromatography (DCM/MeOH=30/1) to obtain compound **69** (116 mg), white solid.

MS (ESI) *m/z* 533.2 $[M+H]^+$.

$^1H$ NMR (400 MHz, DMSO-$d_6$) δ 9.90 (s, 1H), 8.17 (s, 1H), 7.71 (d, *J* = 8.7 *Hz, 2H), 7.26 (d, J*= 8.7 Hz, 2H), 7.10 (dd, *J* = 9.3, 8.3 Hz, 1H), 6.94-6.79 (m, 2H), 5.99 (s, 1H), 5.80 (s, 1H), 5.53 (s, *1H),* 4.08-4.00 (m, 1H), 3.58 (s, 3H), 3.32 (s, 2H), 3.31-3.22 (m, 2H), 1.95 (s, 3H), *1.93-1.83* (m, 4H), 1.68-1.50 (m, 2H).

**Example 70 *N*-(4-(4-amino-5-(5-fluoro-4-(1-oxo-1λ$^6$-thiocyclobutan-1-ylidene)amino) cyclohexyl-1,5-dienyl-1-yl)-7-methyl-7*H*-pyrrolo[2,3-*d*]pyrimidin-6-yl)-3-methylphenyl) methacrylamide**

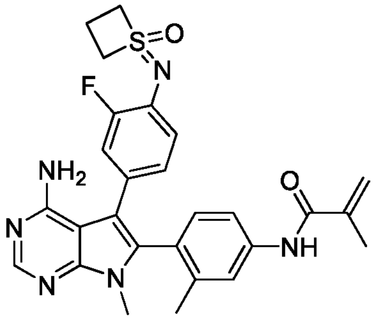

**70**

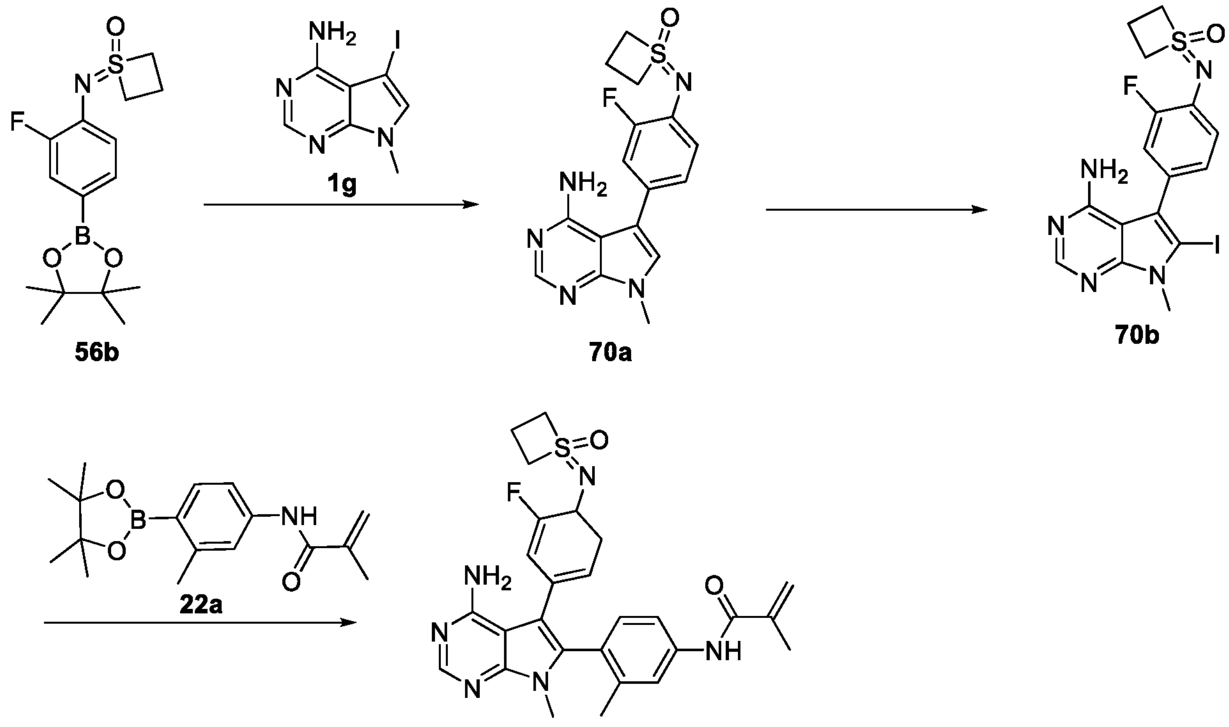

56b

1g

70a

70b

22a

70

Compound 70 was synthesized with reference to Example **29**.

MS (ESI) *m/z* 519.47 $[M + H]^+$.

$^1H$ NMR (400 MHz, $CDCl_3$) δ 8.30 (s, 1H), 7.43 (d, *J* = 2.1 Hz, 2H), 7.35 (dd, *J* = 8.2, 2.0 Hz, 1H), 7.10 (d, *J* = 8.2 Hz, 1H), 6.92 (t, *J* = 8.6 Hz, 1H), 6.81 (s, 2H), 5.73 (s, 1H), 5.42 (d, *J* = 1.1 Hz, 1H), 4.96 (s, 2H), 4.21 (d, *J* = 8.5 Hz, 4H), 3.45 (s, 1H), 2.26 (s, 2H), 2.01 (s, 3H), 1.94 (s, 1H).

**Example 71 *N*-(4-(4-amino-5-(4-(((ethylamino)(oxo)(trifluoromethyl)-1 λ⁶-sulfaneylidene)amino)-3-fluorophenyl)-7-methyl-7*H*-pyrrolo[2,3-*d*]pyrimidin-6-yl)phenyl) methacrylamide KTH021161**

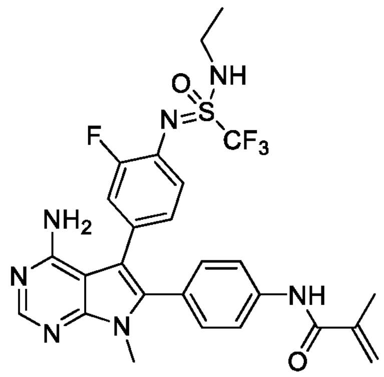

**71**

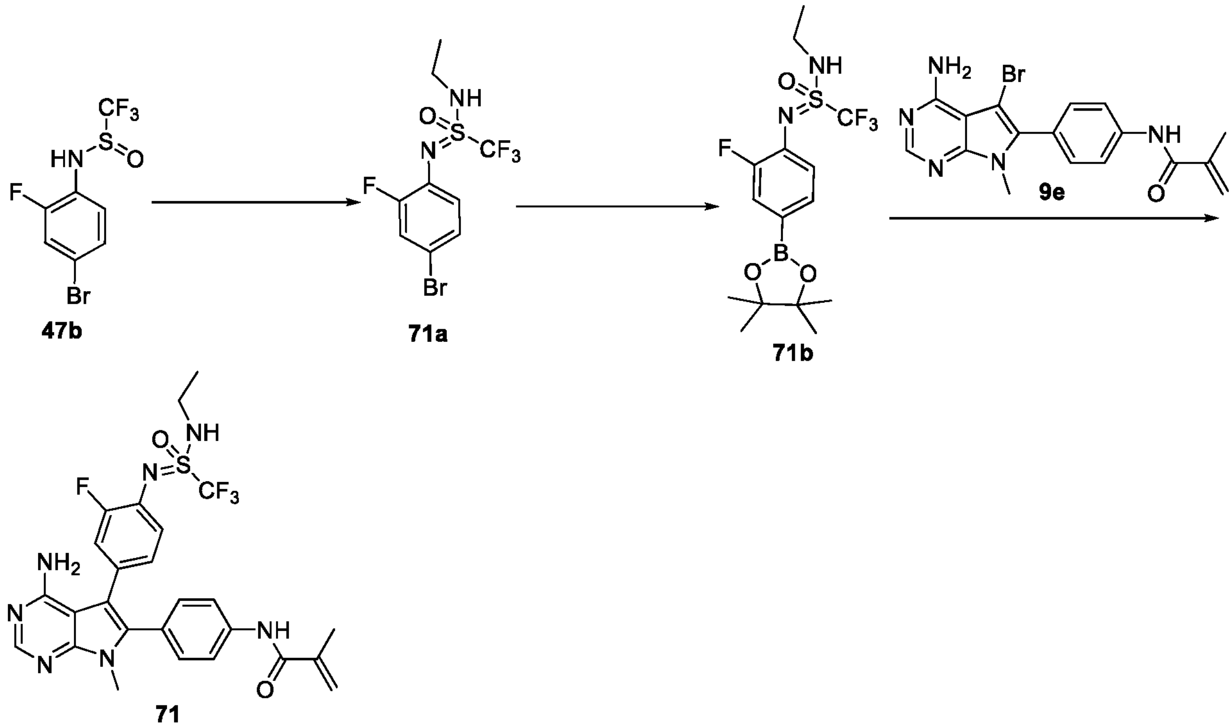

**47b** **71a** **71b** **9e** **71**

**Step 1 N-ethyl-1,1,1-trifluoro-*N'*-(4-iodophenyl) -*N*-methylmethanesulfonimide 71a**

Compound **47b** (600 mg, 1.96 mmol) and N-chlorosuccinimide (292 mg, 2.16 mmol) were dissolved in acetonitrile (8 mL), tetrabutylammonium fluoride (2.16 mL, 2.16 mmol, 1M) was added at 0 °C, and the reaction was carried out at room temperature for 30 min. The reaction solution was concentrated, and then acetonitrile (8 mL) and ethylamine (360 mg, 8.0 mmol) were added, and reacted at room temperature for 2 h. The reaction solution was concentrated, and dried, and the crude product was purified by column chromatography (PE/EtOAc=0-20%) to obtain compound **71a** (422 mg).

MS (ESI) *m/z* 349 $[M + H]^+$

**Step 2 N-ethyl-1,1,1-trifluoro-*N'*-(2-fluoro-4-(4,4,5,5-tetramethyl-1,3,2- dioxaborolan -2-yl)phenyl) methylsulfonyl imine 71b**

Compound **71a** (260 mg, 0.74 mmol), bis(pinacolato)diboron (227 mg, 0.89 mmol), $Pd(dppf)Cl_2$ (110 mg, 0.15 mmol), and potassium acetate (220 mg, 2.23 mmol) were placed in a bottle, 1,4-dioxane (6 mL) was added, after replaced

$N_2$ three times, the reaction was carried out at 100 °C for 12 h. The reaction solution was filtered and concentrated, and the crude product was purified by column chromatography (PE/EtOAc =0-20%) to obtain compound 71b (250 mg).

MS (ESI) *m/z* 396 $[M + H]^+$

**Step 3 *N*-(4-(4-amino-5-(4-(((ethylamino)(oxo)(trifluoromethyl)-1λ$^6$-sulfaneylidene) amino)-3-fluorophenyl) -7-methyl-7*H*-pyrrolo [2,3-*d*] pyrimidin-6-yl) phenyl) methacrylamide 71**

Compound **9e** (100 mg, 0.26 mmol), compound **71b** (123 mg, 0.31 mmol), $Pd(PPh_3)_4$ (65 mg, 0.056 mmol), and $K_3PO_4$ (165 mg, 0.78 mmol) were added into a sealed tube, 1,4-dioxane (5 mL) and $H_2O$ (0.5 mL) were added, after replaced with $N_2$ three times, the reaction solution was heated up to 100 °C and stirred for 16 h. Dichloromethane (20 mL) and saturated saline solution (20 mL) were added to extract, and the organic phase was dried over anhydrous sodium sulfate, and concentrated under reduced pressure to obtain a crude product. The crude product was purified by column chromatography (MeOH/DCM=1/20) to obtain compound 71 (20 mg), white solid.

MS (ESI) *m/z* 576.64 $[M + H]^+$.

$^1$H NMR (400 MHz, MeOD-$d_4$) δ 8.18 (s, 1H), 7.67 (d, *J* = 8.3 Hz, 2H), *7.28 (d, J* = 8.3 Hz, 2H), 7.17 (t, *J* = 8.4 Hz, 1H), 7.02 - 6.93 (m, 2H), *5.81* (s, 1H), 5.52 (s, 1H), 3.68 (s, 3H), 2.03 (s, 3H), 1.33-1.29 (m, 2H), 1.15 *(t, J* = 7.2 Hz, 3H).

**Example 72 *N*- (4- (4-amino-7-methyl-5-(4-(5-oxo-5λ$^6$-thiaspiro[2.3]hexane-5-ylidene) amino)phenyl)-7*H*-pyrrolo[2,3-*d*]pyrimidin-6-yl)-3-fluorophenyl)methacrylamide**

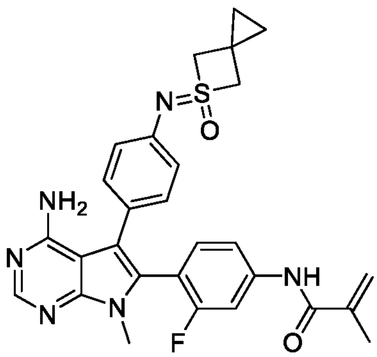

**72**

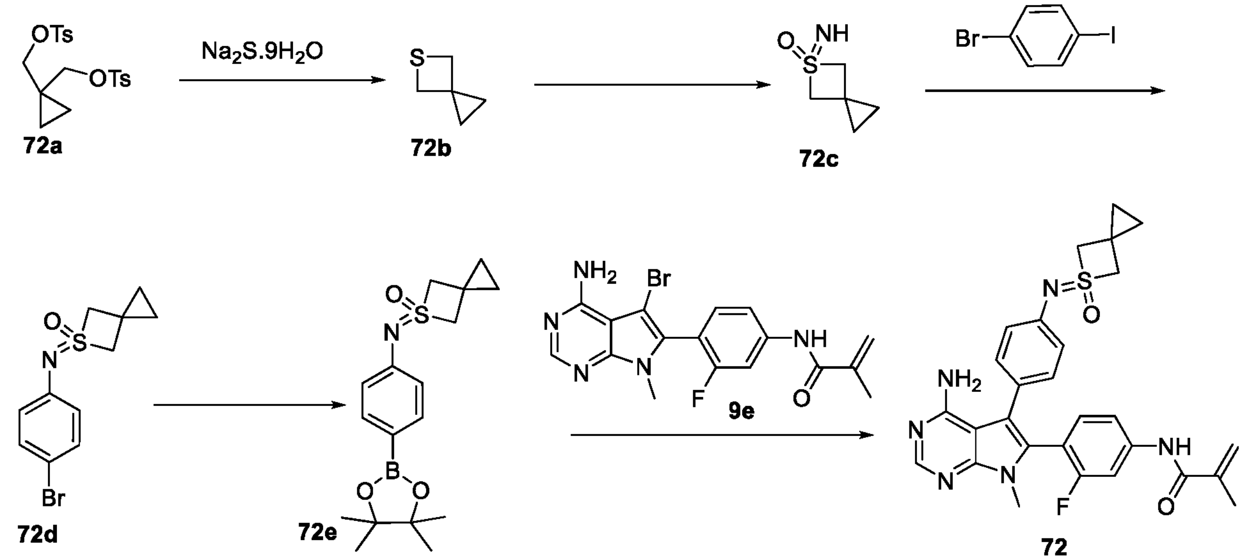


72a

72b

72c

72d

72e

9e

72

**Step 1 5-thiaclopril [2.3] hexane 72b**

Compound **72a** was synthesized reference to WO2022152705 A1. Compound **72a** (3 g, 7.32 mmol) and sodium sulfide nonahydrate (2.1 g, 8.78 mmol) were placed in a 250 mL thick walled pressure resistant bottle, DMF (15 mL) was added and reacted overnight at 120 °C. After the reaction was completed, $H_2O$ (50 mL) was added and extracted with ethyl acetate three times (10 mL x3), and the organic layers were combined, washed with saturated saline solution, and dried over anhydrous sodium sulfate to obtain a ethyl acetate solution of compound **72b,** which was directly used in the next step.

**Step 2 5-amino-5λ$^6$-thiaspiro [2.3] hexane 5-oxide 72c**

The ethyl acetate solution of **72b** and ammonium carbamate (856mg, 11.0 mmol) were added into a flask in sequence, and then MeOH (20 mL) and iodobenzene diethyl ester (5.3 g, 16.47mmol) were added. The reaction solution was stirred open at room temperature for 30 min, and then concentrated under reduced pressure to remove the solvent to obtain a crude product. The crude product was purified by column chromatography (MeOH/DCM =1/20) to obtain compound **72c** (500 mg), light brown oil.
MS (ESI) *m/z* 131 $[M + H]^+$

**Step 3 5-((4-bromophenyl) imino)-5λ$^6$-thiaspiro[2.3]hexane5-oxide 72d**

Compound **72c** (300 mg, 0.29 mmol), cesium carbonate (1.5 g, 4.58 mmol), 1-bromo-4-iodobenzene (0.65 g, 2.29 mmol), $Pd_2(dba)_3$ (53 mg, 0.057 mmol), and Xantphos (99 mg, 0.17 mmol) were dissolved in 1,4-dioxane (10 mL), and the reaction solution was heated up to 100 °C to react for 16 h. After the reaction was completed, the reaction solution was filtered through diatomaceous earth, the filter cake was washed with ethyl acetate (100 mL), and saturated saline solution (50 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound **72d** (310 mg).
MS (ESI) *m/z* 286 $[M + H]^+$

**Step 4 5-((4-(4,4,5,5-tetramethyl-1,3,2-dioxaborolan-2-yl)phenyl)imino)-5λ$^6$-thiaspiro[2.3]hexane 5-oxide 72e**

Compound **72d** (310 mg, 1.1 mmol), bis(pinacolato)diboron (330 mg, 1.3 mmol), $Pd(dppf)Cl_2$ (160 mg, 0.22 mmol), potassium acetate (330 mg, 3.3 mmol), and 1,4-dioxane (10 mL) were added into a round bottom flask, after replaced with argon gas three times, the reaction solution was heated up to 100 °C and stirred for 16 h. After the reaction was completed, it was cooled down to room temperature, water (20 mL) was addedand extracted with ethyl acetate (15 mL × 3). The organic phase was washed with saturated saline solution (20 mL), dried over anhydrous sodium sulfate, and concentrated under reduced pressure to obtain a crude product. The crude product was purified by column chromatography (PE/EtOAc=3/2) to obtain compound **72e** (280 mg), light yellow solid.
MS (ESI) *m/z* 333 $[M + H]^+$

**Step *5* *N*-(4-(4-amino-7-methyl-5-(4-(5-oxo-5λ$^6$-thiaspiro[2.3]hex-5-ylidene)amino) phenyl)-7*H*-pyrrolo[2,3-*d*] pyrimidin-6-yl)-3-fluorophenyl)methacrylamide 72**

Compound **72e** (50 mg, 0.15 mmol), compound **9e** (50 mg, 0.12 mmol), $Pd(PPh_3)_4$ (28 mg, 0.025 mmol), $K_3PO_4$ (80 mg, 0.37 mmol), 1,4-dioxane (2 mL), and $H_2O$ (0.2 mL) were added into a round bottom flask, after replaced with argon gas three times, and the reaction solution was heated up to 100 °C and stirred for 16 h. After the reaction was completed, dichloromethane (20 mL) and saturated saline solution (10 mL) were added and extracted with dichloromethane. The organic phase was dried over anhydrous sodium sulfate and concentrated under reduced pressure to obtain a crude product. The crude product was purified by column chromatography (MeOH/DCM=1/15) to obtain compound **72** (15 mg), white solid.
MS (ESI) *m/z* 531.6 $[M + H]^+$
$^1$H NMR (400 MHz, MeOD-$d_4$) δ 8.21 (s, 1H), 7.74 (dd, *J* = 12.1, 2.1 Hz, *1H), 7.38* (dd, *J* = 8.5, 2.1 Hz, 1H), 7.18 (dt, *J* = 8.3, 4.3 Hz, 3H), 7.10 - 6.89 (m, 2H), 5.83 (s, 1H), 5.57 (d, *J* = 1.6 Hz, 1H), 4.44 - 4.28 (m, 2H), 4.23 - *4.11* (m, 2H), 3.64 (s, 3H), 2.04 (t, *J* = 1.2 Hz, 3H), 1.09 - 0.81 (m, 4H).

**Example 73 *N*-(4-(4-amino-5-(4-(diethyl(oxo)-λ$^6$-sulfaneylidene)amino)phenyl) -7-methyl-7*H*-pyrrolo[2,3-*d*]pyrimidin-6-yl)-3-(difluoromethyl)phenyl)methacrylamide**

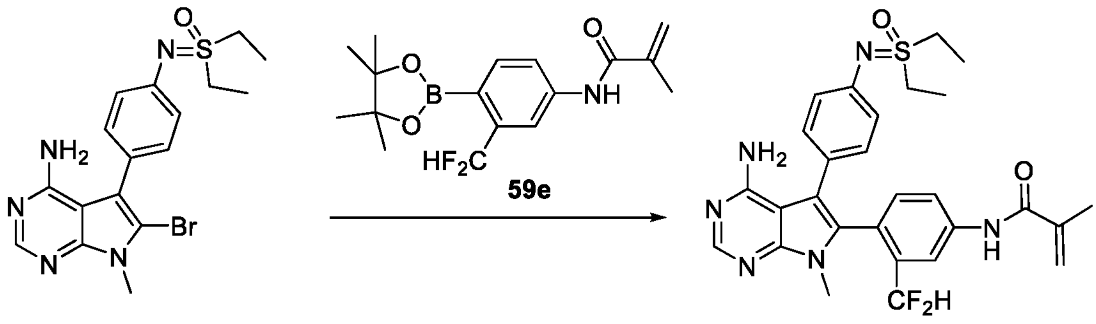

59e

58d

73

Compound **73** was synthesized with reference to the similar synthetic route and steps of compound **12,** wherein compound **12a** was replaced with compound 58d, compound 12b was replaced with compound **59e.**

MS (ESI) *m/z* 553 $[M + H]^+$.

$^1H$ NMR (400 MHz, DMSO-$d_6$) $\delta$ 10.1 (s, 1H), 8.19 (s, 1H), 8.05 (s, 1H), 7.95 (d, J=8.0 Hz, 1H), 7.48 (d, J=8.0 Hz, 1H), 6.99 (d, J=8.0 Hz, 2H), 6.86 (d, J=8.0 Hz, 2H), 6.46 (t, J=8.0 Hz, 1H), 6.05 (s, 2H), 5.85 (s, 1H), 5.57 (s, 1H), 3.41 (s, 3H), 3.26-3.23 (m, *4H),* 1.96 (s) 3H), 1.23 (s, 6H).

**Example 74 *N*-(4-(4-amino-5-(3-fluoro-4-(1-oxo-1$\lambda^6$-thietan-1-imino)phenyl) -7-methyl-7*H*-pyrrolo[2,3-*d*]pyrimidin-6-yl)-3-chlorophenyl)methacrylamide**

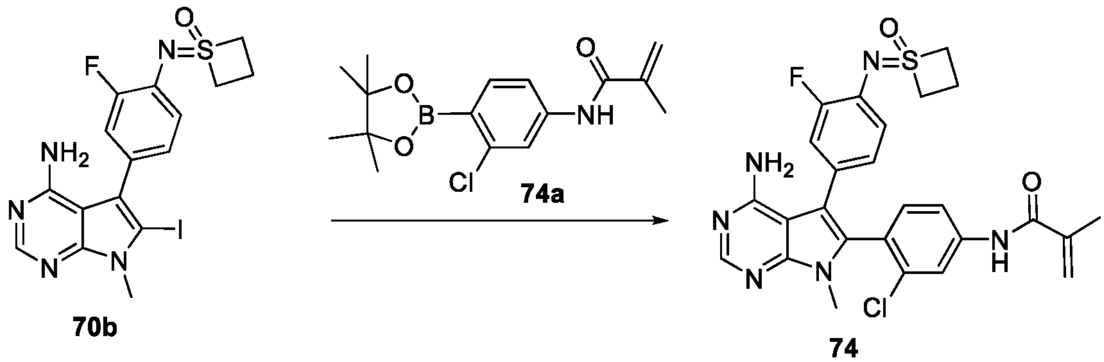

74a

70b

74

Compound **74** was synthesized with reference to the similar synthetic route and steps of compound **12,** wherein, compound **12a** was replaced with compound **70b,** compound **12b** was replaced with compound **74a.**

MS (ESI) m/z 539 $[M + H]^+$.

$^1H$ NMR (400 MHz, DMSO-$d_6$) $\delta$ 10.08 (s, 1H), 8.21 (s, 1H), 8.03 (s, 1H), 7.67 (d, J=8.0 Hz, 1H), 7.59-7.56 (m, 3H), 7.35 (t, J=12.0 Hz, 1H), 6.14 (s, 2H), 5.84 (s, 1H), 5.58 (s, 1H), 4.29-4.26 (m, 4H), 3.46 (s, 3H), 2.28-2.18 (m, 2H), 1.96 (s, 3H).

**Example 75 *N*-(4-(4-amino-5-(3-fluoro-4-(1-oxo-1$\lambda^6$-thiocyclobutan-1-ylidene)amino) phenyl)-7-methyl-7*H*-pyrrolo[2,3-*d*]pyrimidin-6-yl)fluorophenyl]methacrylamide**

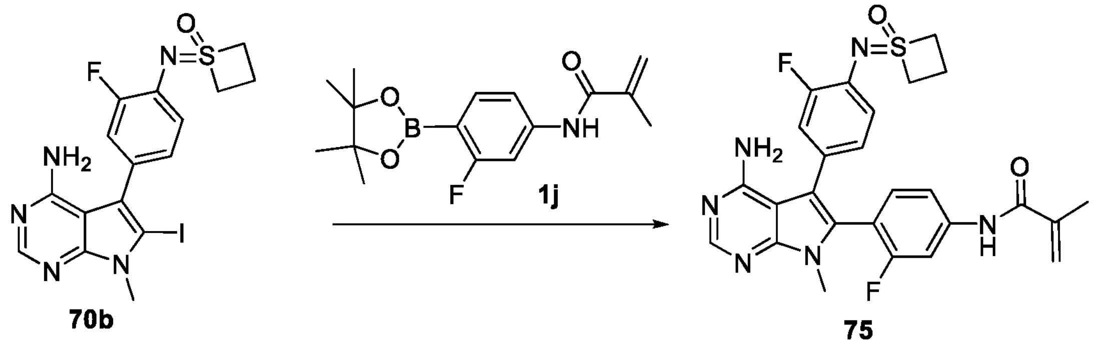

70b 1j 75

Compound 75 was synthesized with reference to the similar synthetic route and steps of compound **12,** wherein, compound **12a** was replaced with compound **70b,** compound **12b** was replaced with compound **1j.**

MS (ESI) *m/z* 523 [M + H]$^+$.

$^1$H NMR (400 MHz, DMSO-$d_6$) $\delta$ 10.12 (s, 1H), 8.21 (s, 1H), 7.79 (d, *J*=8.0 Hz, 1H), 7.52 (d, *J=8.0 Hz,* 1H), 7.32 (t, J=12.0 Hz, 2H), 7.00-6.89 (m, 2H), 6.12 (s, 2H), 5.84 (s, 1H), 5.59 (s, 1H), 4.31-4.27 (m, 4H), 3.53 (s, 3H), 2.22-2.19 (m, 2H), 1.96 (s, 3H).

**Example 76 *N*-(4-(4-amino-5-(4-(diethyl(oxo)-λ$^6$-sulfaneyliden)amino)-2,5-difluorophenyl) -7-methyl-7*H*-pyrrolo [2,3-*d*] pyrimidin-6-yl) -3-fluorophenyl) methacrylamide KTH021179**

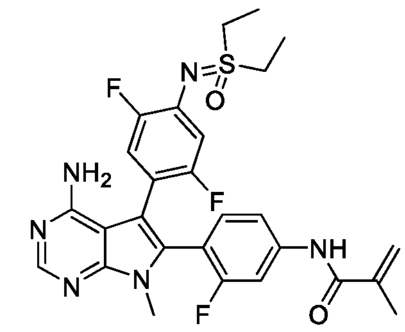

76

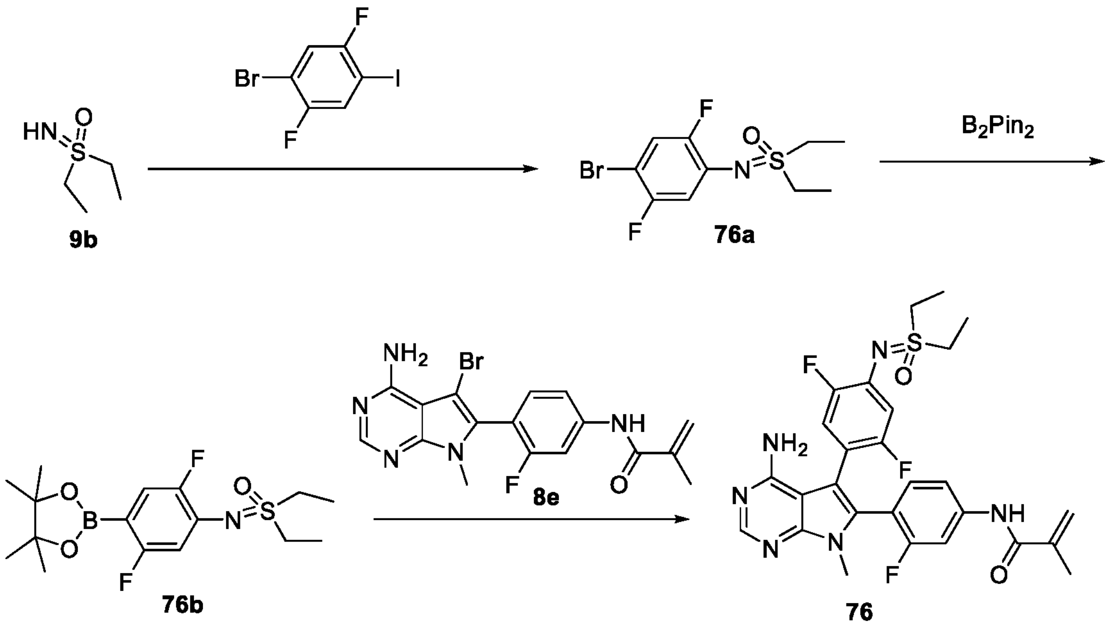


9b 76a 8e 76b 76

**Step 1 ((4-bromo-2,5-difluorophenyl)imino)diethyl-λ$^6$-sulfone 76a**

At room temperature, compound **9b** (1 g, 8.25 mmol), 5-bromo-1,4-difluoro-2- iodobenzene (3.16 g, 9.91 mmol), tris (dibenzylideneacetone) dipalladium (183.14 mg, 0.19 mmol), 4,5-diphenylphosphine-9,9-dimethyloxanthracene (324.17 mg, 0.56 mmol), and cesium carbonate (3.76 g, 11.55 mmol) were added sequentially to a round bottom flask, 1,4-dioxane (20 mL) was added, followed by vacuum pumping and nitrogen gas purging three times, and heated up to 100 °C and stirred for 3 h. After the reaction was completed, the mixture was cooled down to room temperature and concentrated under reduced pressure to obtain a crude product. The crude product was purified by column chromatography (PE/EtOAc=1/3) to obtain compound 76a (800 mg), yellow solid.

MS (ESI) *m/z* 311 [M + H]$^+$.

**Step 2 (2,5-difluoro-4-(4,4,5,5-tetramethyl-1,3,2-dioxaborolan-2-yl) phenyl) imino diethyl-λ$^6$-sulfone 76b**

At room temperature, compound **76a** (800 mg, 2.05 mmol), bis(pinacolato)diboron (782.54 mg, 3.08 mmol), potassium acetate (197.49 mg, 54.75 mmol), 1,1'- biphenylphosphine ferrocene palladium chloride (197.49 mg, 0.26 mmol), and 1,4-dioxane (10 mL) were added to a round bottom flask sequentially, followed by vacuum pumping and nitrogen gas purging three times, and heated up to 100 °C and stirred for 1 h. After the reaction was completed, the mixture was cooled down to room temperature and filtered under reduced pressure, the filter cake was washed three times with dichloromethane, and the filtrate was concentrated under reduced pressure to obtain a crude product. The crude product was purified by column chromatography (PE/EtOAc=1/5) to obtain compound **76b** (700 mg), light yellow solid.

MS (ESI) *m/z* 360 [M + H]$^+$.

**Step 3 *N*-(4-(4-amino-5-(4-(diethyl(oxo)-λ$^6$-sulfaneylidene)amino) -2,5-difluorophenyl) -7-methyl-7*H*-pyrrolo [2,3-*d*] pyrimidin-6-yl) -3-fluorophenyl) methacrylamide 76**

At room temperature, compound **76b** (106 mg, 0.30 mmol), compound **8e** (100 mg, 0.25 mmol), tetrakis (triphenylphosphine) palladium (28.65 mg, 0.025 mmol), potassium phosphate (157.93 mg, 0.74 mmol), 1,4-dioxane (2 mL), and water (0.4 mL) were added to a round bottom flask sequentially, followed by vacuum pumping and nitrogen gas purging three times, and heated up to 90 °C and stirred for 2 h. After the reaction was completed, the mixture was cooled down to room temperature and concentrated under reduced pressure to obtain a crude product. The crude product was purified by column chromatography (DCM/MeOH=15/1) to obtain compound **76** (9.1 mg), white solid.

MS (ESI) *m/z* 557 [M + H]$^+$.

$^1$H NMR (400 MHz, DMSO-$d_6$) $\delta$ 10.1 (s, 1H), 8.20 (s, 1H), 7.76 (d, J=8.0 Hz, 1H), *7.49* (d, *J=8.0 Hz,* 1H), 7.23-7.04 (m, 2H), 6.89 (s, 1H), 6.05 (s, 2H), 5.83 (s, 1H), 5.58 (s, 1H), 3.56 (s, 3H), 3.37-3.34 (m, 4H), 1.96 (s, *3H),* 1.27 (s, 6H).

**Example 77 *N*-(4- (4-amino-5- (2,5-difluoro-4-((1-oxo-1λ$^6$-thiocyclobutan-1- ylidene) amino) phenyl) -7-methyl-7*H*-pyrrolo [2,3-*d*] pyrimidin-6-yl) -3-fluorophenyl) methacrylamide**

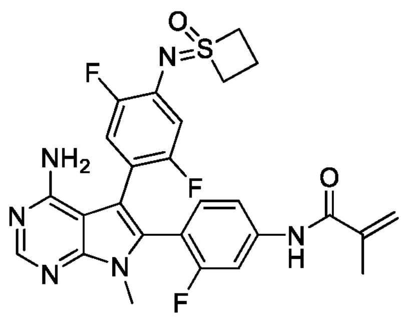

**77**

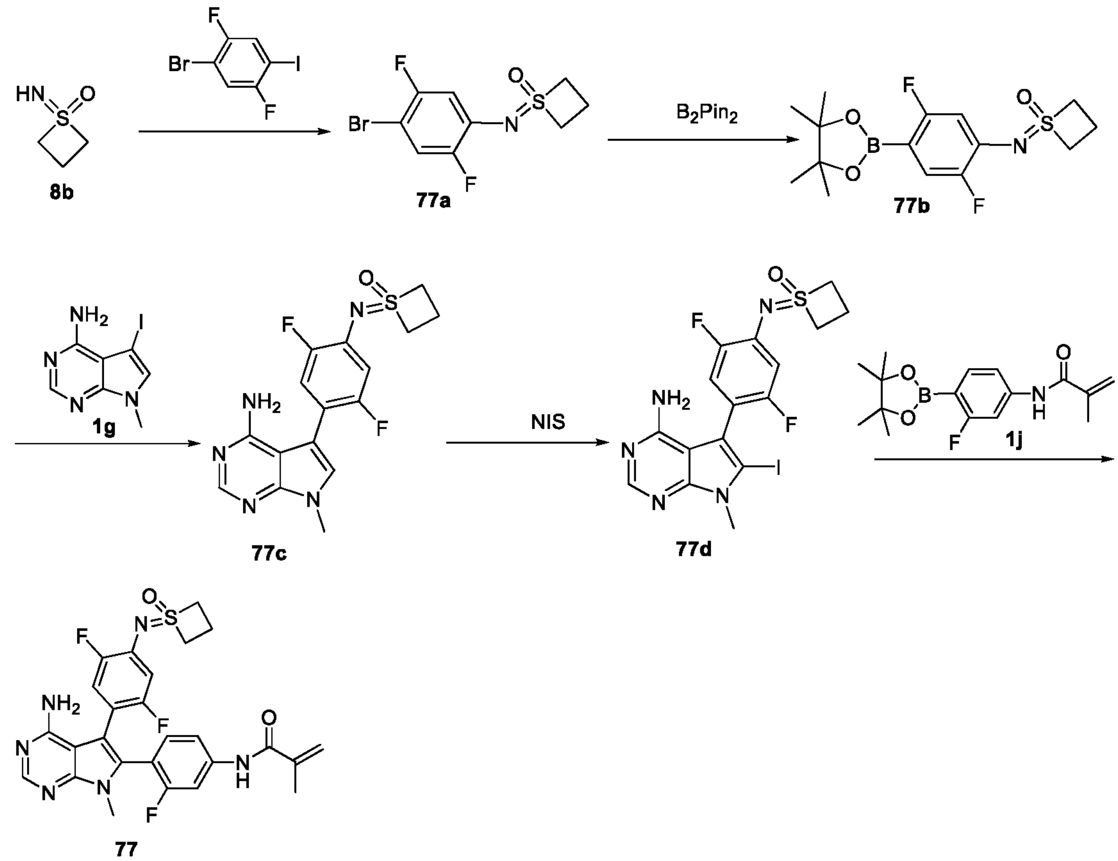


**Step 1 1- ((4-bromo-2,5-difluorophenyl) imino) -1λ$^6$-thietane-1-oxide 77a**

At room temperature, compound **8b** (1 g, 9.51 mmol), 1-bromo-2,5-difluoro-4- iodobenzene (3.63 g, 11.41 mmol), tris (dibenzylideneacetone) dipalladium (219.77 mg, 0.24 mmol), 4,5-diphenylphosphine-9,9-dimethyloxanthracene (410.82 mg, 0.71 mmol), cesium carbonate (4.34 g, 13.31 mmol), and 1,4-dioxane (20 mL) were added to a round bottom flask sequentially, followed by vacuum pumping and nitrogen gas purging three times, and heated up to 90 °C and stirred for 16 h. After TLC monitored the completion of the reaction, the mixture was cooled down to room temperature and concentrated under reduced pressure to obtain a crude product. The crude product was purified by column chromatography (PE/EtOAc=3/1) to obtain compound **77a** (630 mg), light yellow solid.

MS (ESI) *m/z* 295 [M + H]$^+$.

**Step 2 1- (2,5-difluoro-4- (4,4,5,5-tetramethyl-1,3,2-dioxaborolan-2-yl) phenyl) imino)-1 λ$^6$-thietane-1-oxide 77b**

At room temperature, compound **77a** (630 mg, 2.14 mmol), diboronic acid pinal ester (652.63 mg, 2.57 mmol), 1,1'- biphenylphosphine ferrocene palladium chloride (156.58 mg, 0.214 mmol), potassium acetate (525.05 mg, 5.35 mmol), and 1,4-dioxane (6 mL) were added to a round bottom flask sequentially, followed by vacuum pumping and nitrogen gas purging three times, and heated up to 100 °C and stirred for 1 h. After the reaction was completed, the mixture was concentrated under reduced pressure to obtain a crude product. The crude product was purified by column chromatography (PE/EtOAc=3/1) to obtain compound **77b** (500 mg), brown solid.

MS (ESI) *m/z* 344[M + H]$^+$.

**Step 3 1- (4- (4-amino-7-methyl-7*H*-pyrrolo[2,3-*d*]pyrimidin-5-yl) -2,5- difluorophenyl ) imino) -1λ$^6$-thietane-1-oxide 77c**

At room temperature, compound **77b** (500 mg, 1.45 mmol), compound **1g** (40.20 mg, 0.14 mmol), tetrakis (triphenylphosphine) palladium (167.55 mg, 0.145 mmol), potassium phosphate (923.37 mg, 4.35 mmol), 1,4-dioxane (10 mL), and water (2 mL) were added to a round bottom flask sequentially, followed by vacuum pumping and nitrogen gas purging three times, and heated up to 90 °C and stirred for 3 h. After TLC monitored the completion of the reaction, the mixture was cooled down to room temperature and concentrated under reduced pressure to obtain a crude product. The

crude product was purified by column chromatography (DCM/MeOH=15/1) to obtain compound 77c (300 mg), reddish brown solid.

MS (ESI) *m*/*z* 364 [M + H]$^+$.

**Step 4 1- (4- (4-amino-6-iodo-7-methyl-7*H*-pyrrolo [2,3-*d*] pyrimidin-5-yl) -2,5-difluorophenyl) imino) -1 λ$^6$-thietane-1-oxide 77d**

At room temperature, compound **77c** (300 mg, 0.83 mmol) was dissolved in dichloromethane (5 mL), and then trifluoroacetic acid (188.36 mg, 1.65 mmol) was added. After it was cooled down to 0 °C, NIS (278.75 mg, 1.24 mmol) was added in batches, and the reaction was carried out at room temperature for 2 h. After the reaction was completed, saturated sodium bicarbonate solution was added and extracted with dichloromethane. The organic phase was concentrated under reduced pressure to obtain a crude product. The crude product was purified by column chromatography (DCM/MeOH=15/1) to obtain compound **77d** (50 mg), light yellow solid.

MS (ESI) *m*/*z* 489 [M + H]$^+$.

**Step 5 *N*-(4-*(4-amino-5-* (2,5-difluoro-4- (1-oxo-1 λ$^6$-thiocyclobutan-1-ylidene)amino) phenyl) -7-methyl-7H-pyrrolo [2,3-*d*]pyrimidin-6-yl) -3-fluorophenyl)methacrylamide 77**

At room temperature, compound **77d** (50 mg, 0.10 mmol), compound **1j** (37.23 mg, 0.12 mmol), tetrakis (triphenylphosphine) palladium (11.56 mg, 0.01 mmol), potassium phosphate (64.95 mg, 0.31 mmol), 1,4-dioxane (2 mL), and water (0.4 mL) were added to a round bottom flask sequentially, followed by vacuum pumping and nitrogen gas purging three times, and heated up to 90 °C and stirred for 2 h. After the reaction was completed, the mixture was cooled down to room temperature and concentrated under reduced pressure to obtain a crude product. The crude product was purified by column chromatography (DCM/MeOH=15/1) to obtain compound 77 (20 mg), white solid.

MS (ESI) *m*/*z* 541 [M + H]$^+$.

$^1$H NMR (400 MHz, DMSO-$d_6$) δ 10.10 (s, 1H), 8.19 (s, 1H), 8.16 (s, 1H), 7.77 (d, J=8.0 Hz, 1H), 7.48 (d, *J*=8.0 Hz, 2H), 7.21 (t, *J*=12.0 Hz, 1H), 6.02 (s, 2H), 5.82 (s, 1H), 5.57 (s, 1H), 4.37-4.28 (m, 4H), 3.51 (s, 3H), 2.27-2.21 (m, 2H), 1.98 (s, 3H).

**Example 78 *N-* (4- (4-amino-7- (2- (dimethylamino) ethyl) -5- (4- ((1-hydroperoxide -1λ$^6$ -thiophene-1-ylidene) amino) phenyl) -7*H*-pyrrolo [2,3-*d*] pyrimidin-6-yl) -3-fluorophenyl) methacrylamide**

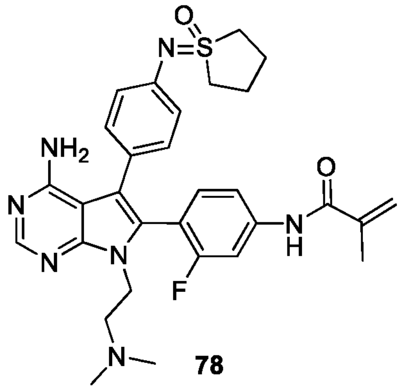

78

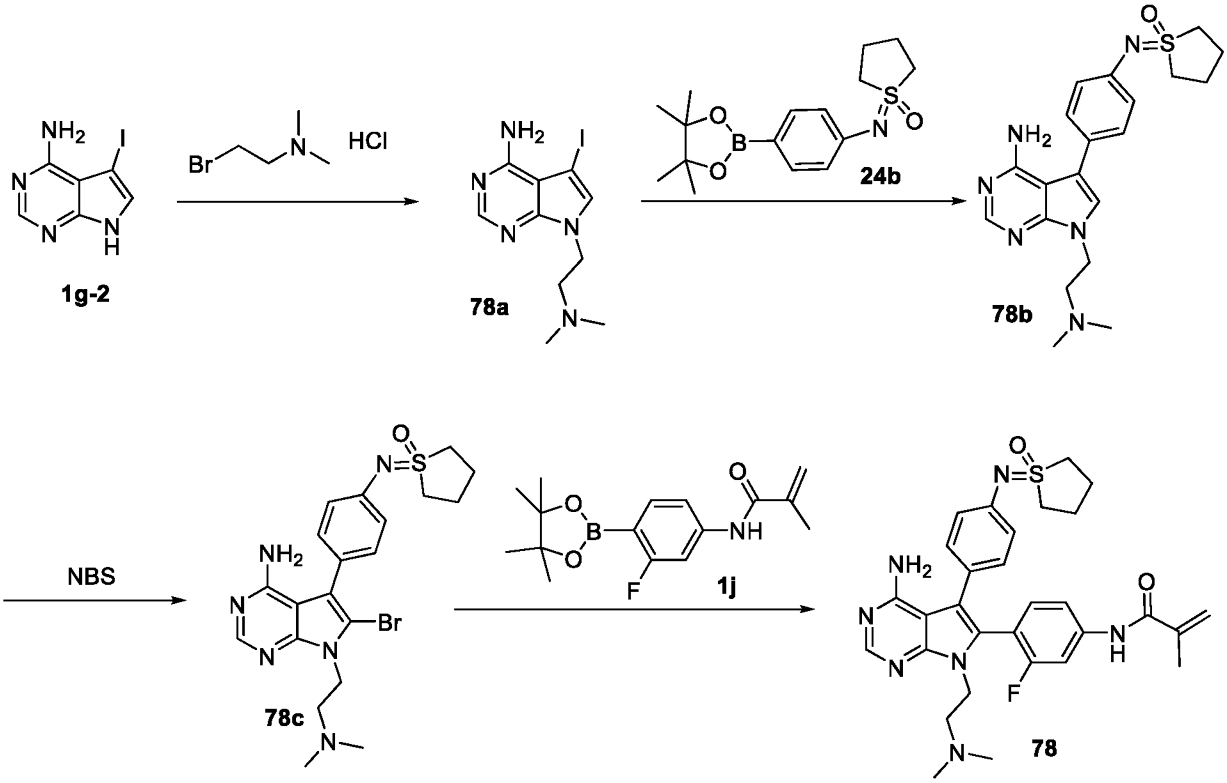


**Step 1 7- (2-(dimethylamino)ethyl) -5-iodo-7*H*-pyrrolo [2,3-*d*]pyrimidin-4-amine 78a**

At room temperature, compound **1g-2** (1 g, 3.85 mmol), (2-bromomethyl) dimethylamine hydrochloride (872.78 mg, 5.78 mmol), cesium carbonate (2.51 g, 7.7 mmol), and N, N-dimethylformamide (10 mL) were added to a round bottom flask sequentially, and the reaction was stirred at room temperature for 16 h. After TLC monitored the completion of the reaction, the mixture was diluted with water, extracted with ethyl acetate (30 mL × 3), and washed with saturated saline (30 mL), and the organic phases were combined, dried over anhydrous sodium sulfate and concentrated under reduced pressure to obtain a crude product. The crude product was purified by column chromatography (DCM/MeOH=15/1) to obtain compound **78a** (400 mg), gray solid.
MS (ESI) *m*/*z* 332 $[M + H]^+$.

**Step 21- (4-(4-amino-7-(2-(dimethylamino)ethyl)-7*H*-pyrrolo[2,3-*d*]pyrimidin-5-yl) phenyl)imino)tetrahydro-1*H*-1$\lambda^6$-thiophene-1-oxide 78b**

At room temperature, compound **78a** (400 mg, 1.2 mmol), compound **24b** (423.72 mg, 0.13 mmol), tetrakis (triphenylphosphine) palladium (138.67 mg, 0.12 mmol), potassium phosphate (764.17 mg, 3.6 mmol), 1,4-dioxane (10 mL), and water (2 mL) were added to a round bottom flask sequentially, followed by vacuum pumping and nitrogen gas purging three times, and heated up to 90 °C and stirred for 3 h. After TLC monitored the completion of the reaction, the mixture was cooled down to room temperature and concentrated under reduced pressure to obtain a crude product. The

crude product was purified by column chromatography (DCM/MeOH=15/1) to obtain compound **78b** (210 mg), reddish brown solid.

MS (ESI) *m/z* 399 $[M + H]^+$.

**Step 3 1-(4-(4-amino-6-bromo-7-(2-(dimethylamino)ethyl)-7*H*-pyrrolo [2,3-*d*] pyrimidin-5-yl)phenyl)imino)tetrahydro-1*H*-1$\lambda^6$-thiophene-1-oxide 78c**

At room temperature, compound 78b (210 mg, 0.53 mmol) was dissolved in DMF (5 mL), and it was cooled down to 0 °C, and a solution of NBS in DMF (98.55 mg NBS dissolved in 2 mL DMF) was added dropwise, and then temperature was restored to room temperature to react for additional 2 h. After the reaction was completed, the reaction was quenched by added saturated sodium bisulfite aqueous solution, extracted with dichloromethane, and the organic phase was concentrated to obtain a crude product. The crude product was purified by column chromatography (DCM/MeOH=15/1) to obtain compound **78c** (100 mg), light yellow solid.

MS (ESI) *m/z* 477 $[M + H]^+$.

**Step 4 *N-(4-(4-amino-7-*(2-(dimethylamino)ethyl)-5-(4-((1-hydroperoxide-1 $\lambda^6$-thiophene-1-ylidene)amino)phenyl)-7*H*-pyrrolo [2,3-*d*] pyrimidin-6-yl) -3- fluorophenyl) methacrylamide 78**

At room temperature, compound **78c** (100 mg, 0.21 mmol), compound **1j** (76.9 mg, 0.25 mmol), tetrakis (triphenylphosphine) palladium (24.26 mg, 0.02 mmol), potassium phosphate (133.62 mg, 0.63 mmol), 1,4-dioxane (2 mL), and water (0.4 mL) were added to a round bottom flask sequentially, followed by vacuum pumping and nitrogen gas purging three times, and heated up to 90 °C and stirred for 2 h. After TLC monitored the completion of the reaction, the mixture was cooled down to room temperature and concentrated under reduced pressure to obtain a crude product. The crude product was purified by column chromatography (DCM/MeOH=15/1) to obtain compound **78** (7.7 mg), white solid.

MS (ESI) *m/z* 576 $[M + H]^+$.

$^1$H NMR (400 MHz, DMSO-$d_6$) $\delta$ 10.10 (s, 1H), 8.17 (s, 1H), 7.74 (d, *J* = 8.0 *Hz,* 1H), 7.51 (d, *J* = 8.0 Hz, 1H), 7.31 (t, *J* = 12.0 Hz, 1H), 7.05 (d, *J* = 8.0 Hz, 2H), 6.65 (d, *J* = 8.0 *Hz,* 2H), 6.02 (s, 2H), 5.82 (s, 1H), 5.57 (s, 1H), 4.16-3.99 (m, 2H), 3.35-3.21 *(m,* 4H), 2.38-2.30 (m, 2H), 2.21-2.08 (m, 4H), 1.98 (s, 6H), 1.94 (s, 3H).

**Example 79 *N*-(4-(4-amino-7-(2-hydroxy-2-methylpropyl)-5-(4-((1-hydroperoxid -1$\lambda^6$-thiophene-1-ylidene) amino) phenyl) -7*H*-pyrrolo [2,3-*d*] pyrimidin -6-yl) -3-fluorophenyl) methacrylamide**

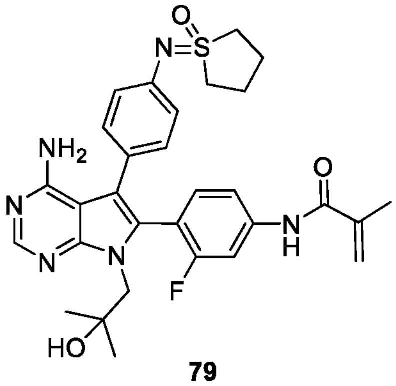

**79**

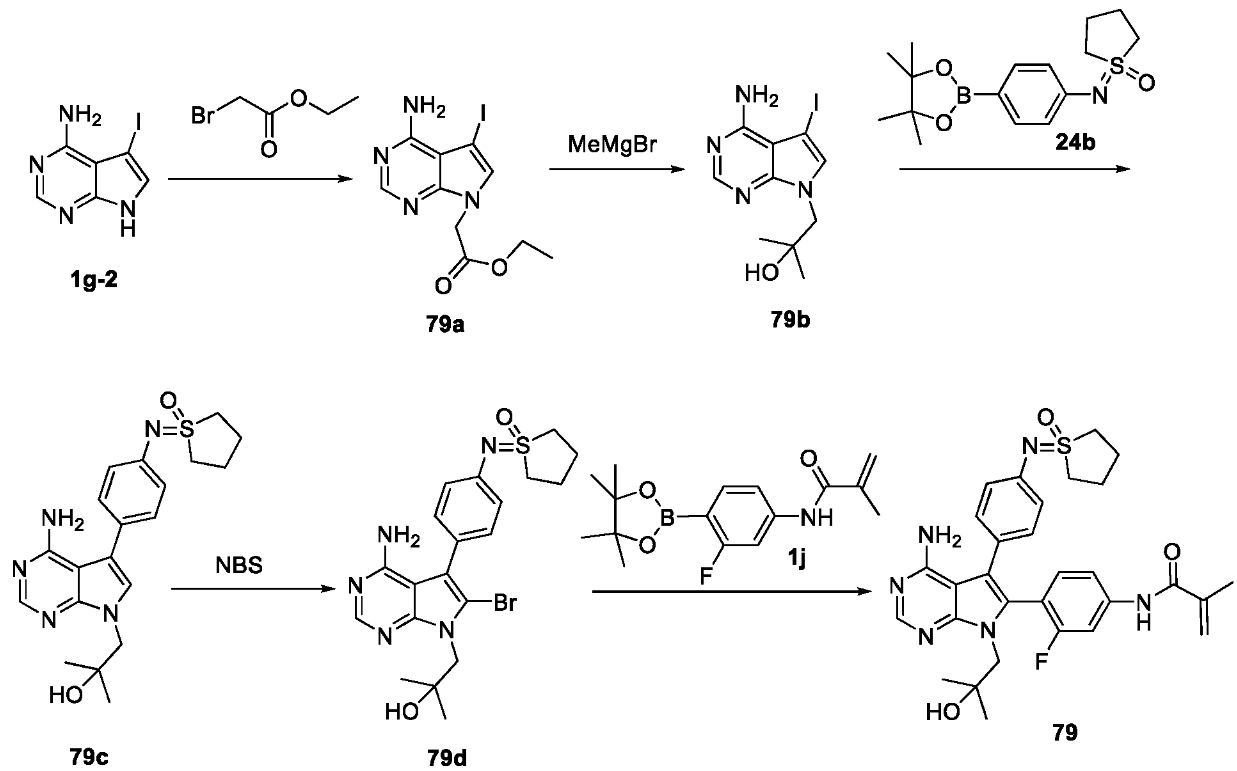


**1g-2** **79a** **79b** **79c** **79d** **79**

**Step 1 ethyl 2- (4-amino-5-iodo-7H-pyrrolo [2,3-*d*] pyrimidin-7-yl) acetate 79a**

Compound **1g-2** (1 g, 3.85 mmol), ethyl bromoacetate (963.59 mg, 5.77 mmol), and cesium carbonate (2.51 g, 7.7 mmol) were dissolved in N,N-dimethylformamide (10 mL) and the reaction was carried out at room temperature for 16 h. After the reaction was completed, water (100 mL) was added and extracted with ethyl acetate (30 mL × 3), and washed with saturated saline (30 mL). The organic phases were combined, dried over anhydrous sodium sulfate and concentrated under reduced pressure to obtain a crude product. The crude product was purified by column chromatography (DCM/MeOH=15/1) to obtain compound **79a** (600 mg), gray solid.

MS (ESI) *m/z* 347 $[M + H]^+$.

**Step 21- (4-amino-5-iodo-7*H*-pyrrolo [2,3-*d*] pyrimidin-7-yl)-2-methylpropyl-2-ol 79b**

Compound **79a** (600 mg, 1.67 mmol) was dissolved in tetrahydrofuran (10 mL), followed by vacuum pumping and nitrogen gas purging three times. After it was cooled down to -56 °C ,1.0 M methyl magnesium bromide-tetrahydrofuran solution (8.4 ml, 8.34 mmol) was added slowly dropwise in batches, and the reaction was stirred at -56 °C for additional 2 h. After the reaction was completed, the mixture was warmed up to room temperature naturally, diluted with water, extracted with ethyl acetate (30 mL × 3), and washed with saturated saline (30 mL), and the organic phases were combined, dried over anhydrous sodium sulfate and concentrated under reduced pressure to obtain a crude product. The crude product was purified by column chromatography (DCM/MeOH=15/1) to obtain compound **79b** (400 mg), light yellow solid.

MS (ESI) *m/z* 333 $[M + H]^+$.

**Step 31-(4-(4-amino-7-(2-hydroxy-2-methylpropyl)-7*H*-pyrrolo [2,3-*d*] pyrimidin-5-yl) phenyl)imino)tetrahydro-1*H*-1λ6-thiophene-1-oxide 79c**

To a round bottom flask, compound **79b** (400 mg, 1.2 mmol), compound **24b** (423.72 mg, 0.13 mmol), tetrakis (triphenylphosphine) palladium (138.67 mg, 0.12 mmol), potassium phosphate (764.17 mg, 3.6 mmol), 1,4-dioxane (10 mL), and water (2 mL) were added in sequence, followed by vacuum pumping and nitrogen gas purging three times, and heated up to 90 °C and stirred for 3 h. After the reaction was completed, the mixture was cooled down to room temperature and concentrated under reduced pressure to obtain a crude product. The crude product was purified by column chromatography (DCM/MeOH=15/1) to obtain compound 79c (150 mg), reddish brown solid.
MS (ESI) *m/z* 400 $[M + H]^+$.

**Step 4 1-(4-(4-amino-6-bromo-7-(2-hydroxy-2-methylpropyl)-7*H*-pyrrolo [2,3-*d*] pyrimidin-5-yl)phenyl)imino) tetrahydro-1H-1λ6-thiophene-1-oxide 79d**

Compound **79c** (150 mg, 0.37 mmol) was dissolved in N, N-dimethylformamide (5 mL). After it was cooled down to 0 °C, N-bromosuccinimide (69.93 mg, 0.39 mmol) was added in batches, and the reaction was stirred at room temperature for 2 h. After the reaction was completed, the mixture was concentrated under reduced pressure to obtain a crude product. The crude product was purified by column chromatography (DCM/MeOH=15/1) to obtain compound **79d** (100 mg), light yellow solid.
MS (ESI) *m/z* 478 $[M + H]^+$.

**Step 5 *N*-(4-(4-amino-7-(2-hydroxy-2-methylpropyl)-5-(4-((1-hydroperoxid-1 λ6-thiophene-1-ylidene)amino)phenyl)-7*H*-pyrrolo[2,3-*d*]pyrimidin-6-yl)-3-fluorophenyl) methacrylamide 79**

To a round bottom flask, compound **79d** (100 mg, 0.21 mmol), compound **1j** (70.19 mg, 0.23 mmol), tetrakis (triphenylphosphine) palladium (24.26 mg, 0.02 mmol), potassium phosphate (133.62 mg, 0.63 mmol), 1,4-dioxane (2 mL), and water (0.4 mL) were added in sequence, followed by vacuum pumping and nitrogen gas purging three times, and heated up to 90 °C and reacted for 2 h. After the reaction was completed, the mixture was cooled down to room temperature and concentrated under reduced pressure to obtain a crude product. The crude product was purified by column chromatography (DCM/MeOH=15/1) to obtain compound 79 (13.4 mg), white solid.
MS (ESI) *m/z* 577 $[M + H]^+$.
$^1$H NMR (400 MHz, DMSO-$d_6$) δ 10.06 (s, 1H), 8.17 (s, 1H), 7.71 (d, *J*=8.0 Hz, 1H), 7.47 (d, *J=8.0 Hz,* 1H), 7.27 (t, *J*=12.0 Hz, 1H), 7.28-7.03 (m, 2H), 6.88-6.84 (m, 2H), 6.02 (s, 2H), 5.80 (s, 1H), 5.56 (s, 1H), 4.91 (s, 1H), 4.16-3.99 (m, 2H), 3.28-3.21 (m, 4H), 2.22-2.02 4 (m, 4H), 1.94 (s, 3H), 0.88 (s, 6H).

**Example 80 *N*-(4-(4-amino-5-(3-methoxy-4-((1-hydroperoxid-1λ6-thiophene-1-ylidene)amino)phenyl)-7-methyl-7*H*-pyrrolo[2,3-*d*]pyrimidin-6-yl)-3-fluorophenyl) methacrylamide**

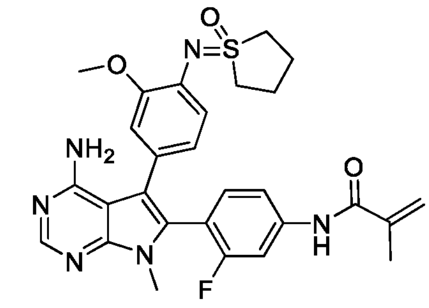

**80**

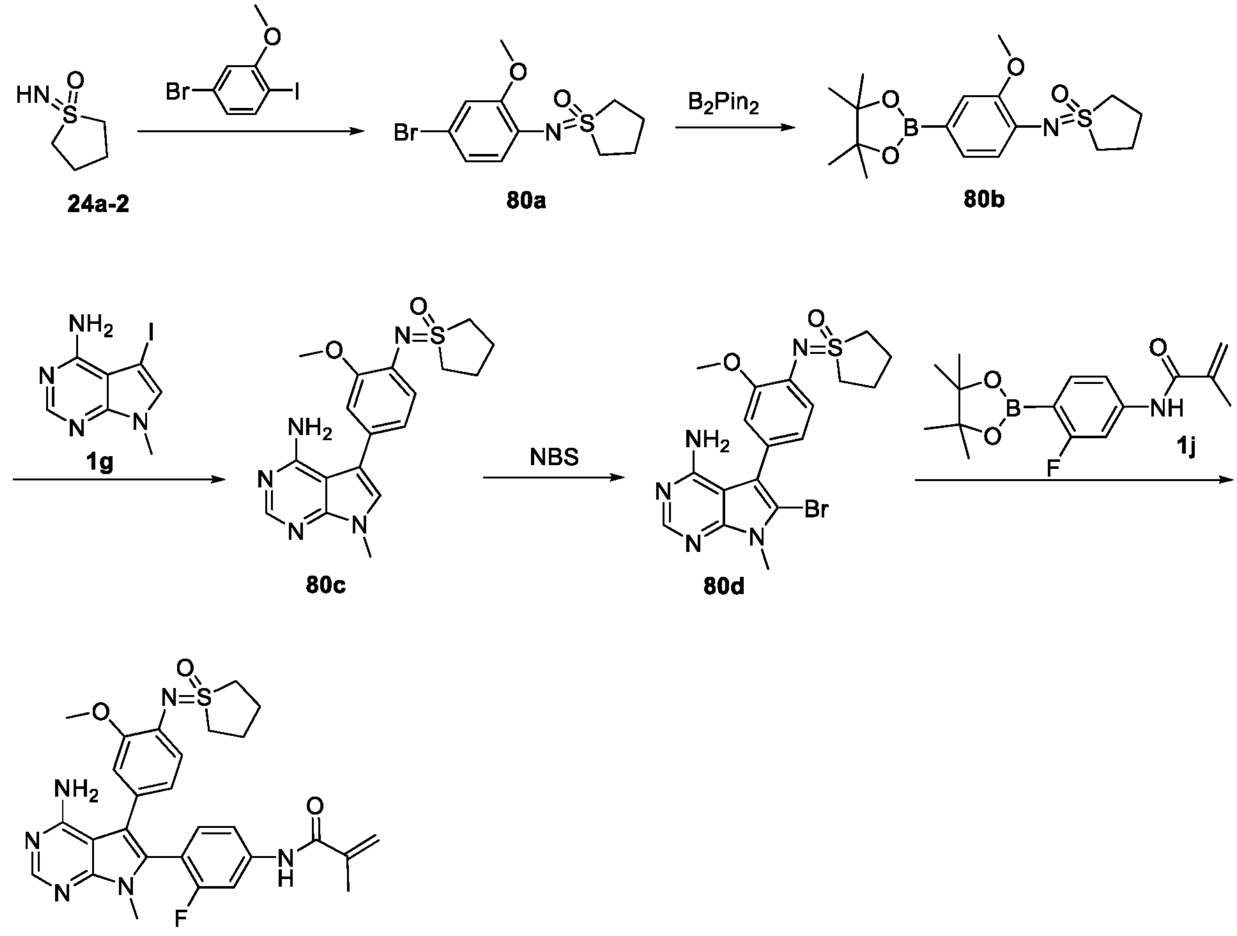


24a-2 80a 80b 1g 80c 80d 1j 80

**Step 1 1- ((4-bromo-2-methoxyphenyl) imino)tetrahydro-1H-1λ6-thiophene-1- oxide 80a**

At room temperature, compound 24a-2 (1 g, 8.4 mmol), 4-bromo-1-iodo-2- methoxybenzene (3.14 g, 10.08 mmol), tris (dibenzylideneacetone) dipalladium (192.3 mg, 0.21 mmol), 4,5-diphenylphosphine-9,9-dimethyloxaanthracene (364.53 mg, 0.63 mmol), cesium carbonate (3.83 g, 11.76 mmol), and 1,4-dioxane (20 mL) were added to a round bottom flask sequentially, followed by vacuum pumping and nitrogen gas purging three times, and heated up to 90 °C and stirred for 3 h. After TLC monitored the completion of the reaction, the mixture was cooled down to room temperature and concentrated under reduced pressure to obtain a crude product. The crude product was purified by column chromatography (PE/EtOAc=1/3) to obtain compound **80a** (1.2 g), yellow solid.

MS (ESI) *m/z* 303 $[M + H]^+$.

**Step 2 1- ((2-methoxy-4- (4,4,5,5-tetramethyl-1,3,2-dioxaborolan-2-yl) phenyl) imino) tetrahydro-1H-1 λ6-thiophene-1-oxide 80b**

To a round bottom flask, compound **80a** (1.2 g, 3.96 mmol), bis(pinacolato)diboron (1.21 g, 4.75 mmol),

potassium acetate (971.59 mg, 9.9 mmol), 1,1'- biphenylphosphine ferrocene palladium chloride (285.36 mg, 0.39 mmol), and 1,4-dioxane (20 mL) were added in sequence, followed by vacuum pumping and nitrogen gas purging three times, and heated up to 100 °C and stirred for 1 h. After the reaction was completed, the mixture was cooled down to room temperature and filtered under reduced pressure, the filter cake was washed with dichloromethane three times, and all filtrates were combined, concentrated under reduced pressure to obtain a crude product. The crude product was purified by column chromatography (PE/EtOAc=1/5) to obtain compound **80b** (1 g), light yellow solid.

MS (ESI) *m/z* 352 $[M + H]^+$.

**Step 3 1- (4- (4-amino-7-methyl-7*H*-pyrrolo [2,3-*d*] pyrimidin-5-yl) -2- methoxyphenyl) imino) tetrahydro-1H-1 $\lambda^6$-thiophene-1-oxide 80c**

To a round bottom flask, compound **80b** (1 g, 2.84 mmol), compound **1g** (654.10 mg, 2.37 mmol), tetrakis (triphenylphosphine) palladium (273.87 mg, 0.237 mmol), potassium phosphate (1.51 g, 7.11 mmol), 1,4-dioxane (20 mL), and water (4 mL) were added in sequence, followed by vacuum pumping and nitrogen gas purging three times, and heated up to 90 °C and reacted for 3 h. After TLC monitored the completion of the reaction, the mixture was cooled down to room temperature and concentrated under reduced pressure to obtain a crude product. The crude product was purified by column chromatography (DCM/MeOH=15/1) to obtain compound 80c (430 mg), reddish brown solid.

MS (ESI) *m/z* 372 $[M + H]^+$.

**Step 4 1- (4- (4-amino-6-bromo-7-methyl-7*H*-pyrrolo [2,3-*d*] pyrimidin-5-yl) -2-methoxyphenyl) imino) tetrahydro-1H-1$\lambda^6$-thiophene-1-oxide 80d**

Compound **80c** (430 mg, 1.16mmol) was dissolved in N, N-dimethylformamide (5 mL), and it was cooled down down to 0 °C, and then N-bromosuccinimide (217.13 mg, 1.22 mmol) was added in batches, and heated up to room temperature and reacted for 2 h. After the reaction was completed, the mixture was concentrated under reduced pressure to obtain a crude product. The crude product was purified by column chromatography (DCM/MeOH=15/1) to obtain compound 80d (300 mg), light yellow solid.

MS (ESI) *m/z* 450 $[M + H]^+$.

**Step 5 *N*-(4-(4-amino-5-(3-methoxy-4-((1-hydroperoxid-1$\lambda^6$-thiophene-1-ylidene) amino)phenyl)-7-methyl-7*H*-pyrrolo [2,3-*d*] pyrimidin-6-yl) -3-fluorophenyl) methacrylamide 80**

To a round bottom flask, compound **80d** (100 mg, 0.22 mmol), compound **1j** (77.08 mg, 0.24 mmol), tetrakis (triphenylphosphine) palladium (25.42 mg, 0.022 mmol), potassium phosphate (140.1 mg, 0.66 mmol), 1,4-dioxane (2 mL), and water (0.4 mL) were added in sequence, followed by vacuum pumping and nitrogen gas purging three times, and heated up to 90 °C and reacted for 2 h. After the reaction was completed, the mixture was cooled down to room temperature and concentrated under reduced pressure to obtain a crude product. The crude product was purified by column chromatography (DCM/MeOH=15/1) to obtain compound 80 (17.4mg), white solid.

MS (ESI) *m/z* 549 $[M + H]^+$.

$^1H$ NMR (400 MHz, DMSO-$d_6$) $\delta$ 10.11 (s, 1H), 8.19 (s, 1H), 7.78 (d, *J*=8.0 Hz, *1H),* 7.48 (d, *J=8.0* Hz, 1H), 7.29 (t, *J*=12.0 Hz, 1H), 6.85 (d, *J*=8.0 Hz, 1H), 6.73-6.65 (m, 2H), 6.12 (s, 2H), 5.82 (s, 1H), 5.57 (s, 1H), 3.60 *(s,* 3H), 3.53 (s, 3H), 3.31-3.16 (m, 4H), 2.16-2.09 (m, 4H), 1.95 (s, 3H).

**Example 81 *N*-(4-(4-amino-5-(3-methoxy-4-((1-hydroperoxid-1 $\lambda^6$-thiophene-1-ylidene) amino) phenyl) -7-methyl-7*H*-pyrrolo [2,3-*d*] pyrimidin-6-yl) -3-chlorophenyl) methacrylamide**

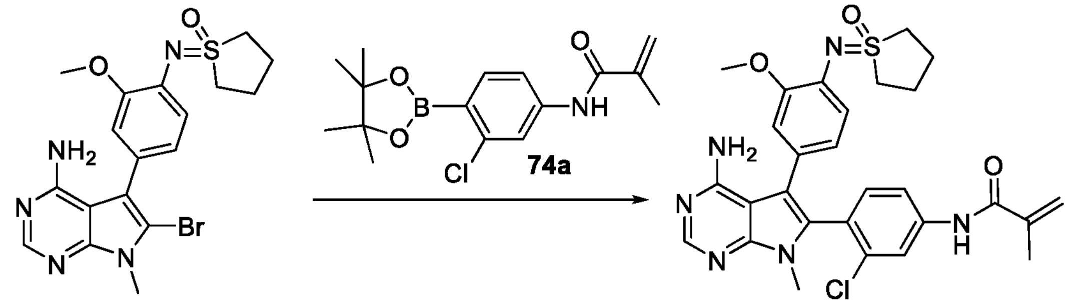

**80d**

**81**

Compound **81** was synthesized with reference to the similar synthetic route and steps of compound **12,** wherein, compound **12a** was replaced with compound **80b,** compound **12b** was replaced with compound **74a.**

MS (ESI) *m*/*z* 566 [M + H]$^+$.

$^1$H NMR (400 MHz, DMSO-$d_6$) $\delta$ 10.08 (s, 1H), 8.19 (s, 1H), 8.02 (d, *J*=8.0 Hz, 1H), *7.64 (d, J=8.0 Hz,* 1H), 7.35 (t, *J*=20.0 Hz, 2H), 6.84 (d, *J*=8.0 Hz, 2H), 6.72-6.66 (m, 4H), 6.12 (s, 2H), 5.83 (s, 1H), 5.57 (s, 1H), 4.31-4.27 (m, *4H),* 3.59 (s, 3H), 3.46 (s, 3H), 1.94 (s, 3H) 3H).

**Example 82 *N*-(4-(4-amino-5-(3-chloro-4-((1-oxide tetrahydro-1λ$^6$-thiophene-1-ylidene)amino)phenyl)-7-(methyl-*d*$_3$)-7*H*-pyrrolo[2,3-*d*]pyrimidin-6-yl)-3-fluorophenyl) methacrylamide**

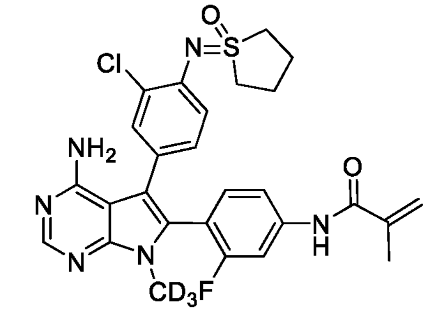

**82**

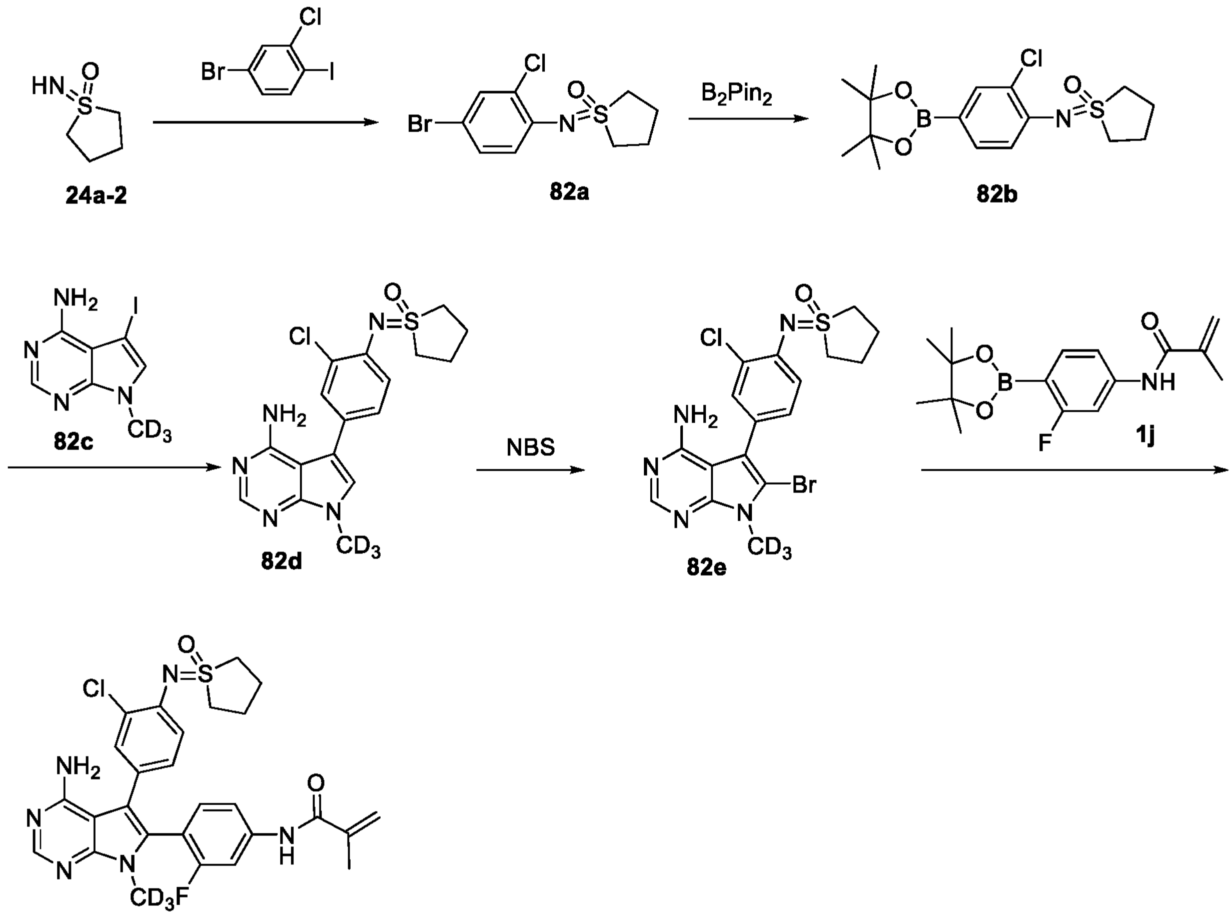


24a-2 82a 82b 82c 82d 82e 1j

**82**

**Step 1 1- ((4-bromo-2-chlorophenyl) imino) tetrahydro-1H-1 λ6-thiophene-1-oxide 82a**

To a round bottom flask, compound **24a-2** (1 g, 8.4 mmol), 4-bromo-2-chloro-1-iodobenzene (3.18 g, 10.08 mmol), tris (dibenzylideneacetone) dipalladium (153.84 mg, 0.17 mmol), 4,5- diphenylphosphine-9,9-dimethyloxanthracene (364.53 mg, 0.50 mmol), cesium carbonate (3.56 g, 10.92 mmol), and 1,4-dioxane (10 mL) were added in sequence, followed by vacuum pumping and nitrogen gas purging three times, and heated up to 90 °C and reacted for 3 h. After the reaction was completed, it was cooled down to room temperature, and the mixture was concentrated under reduced pressure to obtain a crude product. The crude product was purified by column chromatography (PE/EtOAc=1/3) to obtain compound **82a** (1.2 g), yellow solid.

MS (ESI) *m/z* 307 [M + H]+.

**Step 2 1- ((2-chloro-4- (4,4,5,5-tetramethyl-1,3,2-dioxaborolan-2-yl) phenyl) imino) tetrahydro-1H-1 λ$^6$-thiophene-1-oxide 82b**

To a round bottom flask, **82a** (1.2 g, 3.9 mmol), bis(pinacolato)diboron (1.19 g, 4.68 mmol), potassium acetate (956.86 mg, 9.75 mmol), 1,1'- biphenylphosphine ferrocene palladium chloride (285.36 mg, 0.39 mmol), and 1,4-dioxane (20 mL) were added sequentially, followed by vacuum pumping and nitrogen gas purging three times, and heated up to 100 °C and reacted for 1 h. After the reaction was completed, the mixture was cooled down to room temperature and filtered under reduced pressure, the filter cake was washed with dichloromethane three times, and all filtrates were combined and concentrated under reduced pressure to obtain a crude product. The crude product was purified by column chromatography (PE/EtOAc=1/5) to obtain compound **82b** (1 g), light yellow solid.
MS (ESI) *m/z* 356 $[M + H]^+$.

**Step 3 1- (4- (4-amino-7- (methyl-*d*$_3$) -7*H*-pyrrolo [2,3-*d*] pyrimidin-5-yl) -2-chlorophenyl) imino) tetrahydro-1*H*-1λ$^6$-thiophene-1-oxide 82d**

To a round bottom flask, compound **82b** (1 g, 2.82 mmol), compound **82c** (709.09 mg, 2.56 mmol), tetrakis (triphenylphosphine) palladium (325.87 mg, 0.282 mmol), potassium phosphate (1.80 g, 8.46 mmol), 1,4-dioxane (10 mL), and water (2 mL) were added in sequence, followed by vacuum pumping and nitrogen gas purging three times, and heated up to 90 °C and reacted for 3 h. After the reaction was completed, the mixture was cooled down to room temperature and concentrated under reduced pressure to obtain a crude product. The crude product was purified by column chromatography (DCM/MeOH=15/1) to obtain compound **82d** (530 mg), reddish brown solid.
MS (ESI) *m/z* 379 $[M + H]^+$.

**Step 4 1- (4- (4-amino-6-bromo-7-(methyl-*d*$_3$)-7*H*-pyrrolo [2,3-*d*] pyrimidin-5-yl) -2-chlorophenyl) imino) tetrahydro-1H-1λ$^6$-thiophene-1-oxide 82e**

Compound **82d** (530 mg, 1.40 mmol) was dissolved in N, N-dimethylformamide (6 mL). After it was cooled down to 0 °C, NBS (261.63 mg, 1.47 mmol) was added in batches, and it was heated up to room temperature and reacted for 2 h. After the reaction was completed, the mixture was concentrated under reduced pressure to obtain a crude product. The crude product was purified by column chromatography (DCM/MeOH=15/1) to obtain compound 82e (460 mg), yellow solid.
MS (ESI) *m/z* 457 $[M + H]^+$.

**Step 5 *N*-(4-*(4-amino-5*-(3-chloro-4-((1-hydroperoxid-1λ$^6$-thiophene-1-ylidene)amino) phenyl)-7-(methyl-*d*$_3$)-7*H*-pyrrolo[2,3-*d*]pyrimidin-6-yl)-3-fluorophenyl)methacrylamide 82**

To a round bottom flask, compound **82e** (100 mg, 0.22 mmol), compound **1j** (73.85 mg, 0.24 mmol), tetrakis (triphenylphosphine) palladium (25.42 mg, 0.022 mmol), potassium phosphate (140.1 mg, 0.66 mmol), 1,4-dioxane (2 mL), and water (0.4 mL) were added in sequence, followed by vacuum pumping and nitrogen gas purging three times, and heated up to 90 °C and reacted for 2 h. After the reaction was completed, the mixture was cooled down to room temperature and concentrated under reduced pressure to obtain a crude product. The crude product was purified by column chromatography (DCM/MeOH=15/1) to obtain compound 82 (15.7mg), white solid.
MS (ESI) *m/z* 556 $[M + H]^+$.
$^1$H NMR (400 MHz, DMSO-*d*$_6$) δ 10.11 (s, 1H), 8.19 (s, 1H), 7.78 (d, J=8.0 Hz, *1H), 7.51* (d, J=8.0 Hz, 1H), 7.29 (t, J=20.0 Hz, 1H), 7.17 (s, 1H), 7.07 (s, 1H), 7.01 (s, 1H), 6.11-5.95 (m, 2H), 5.83 (s, 1H), 5.58 (s, 1H), *3.40-3.37* (m, 4H), 2.22-2.12 (m, 4H), 1.95 (s, 3H).

**Example 83 *N*-(4-(4-amino-7-(methyl-*d*$_3$)-5-*(4*-(1-oxotetrahydro-1 λ$^6$-thiophene -1-imino)phenyl)-7*H*-pyrrolo[2,3-*d*]pyrimidin-6-yl)-3-fluorophenyl)methacrylamide**

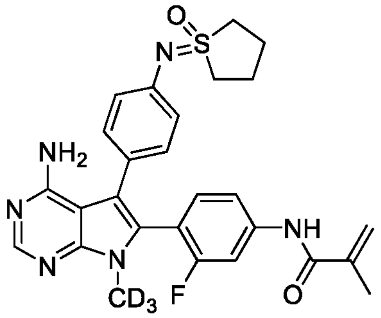

**83**

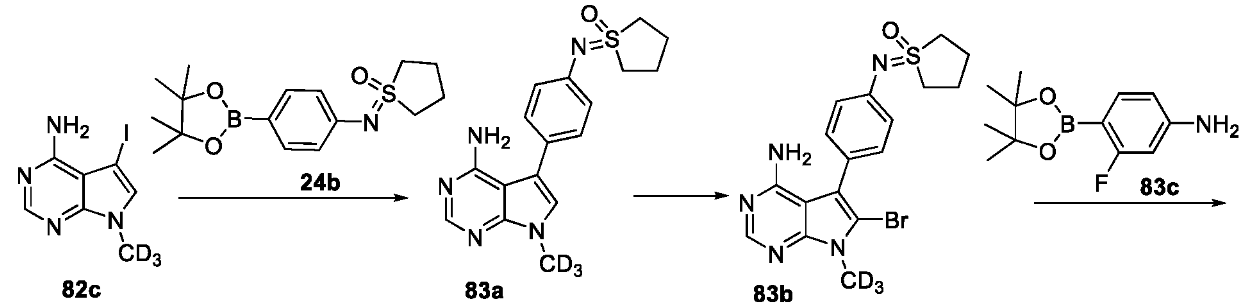


82c 83a 83b

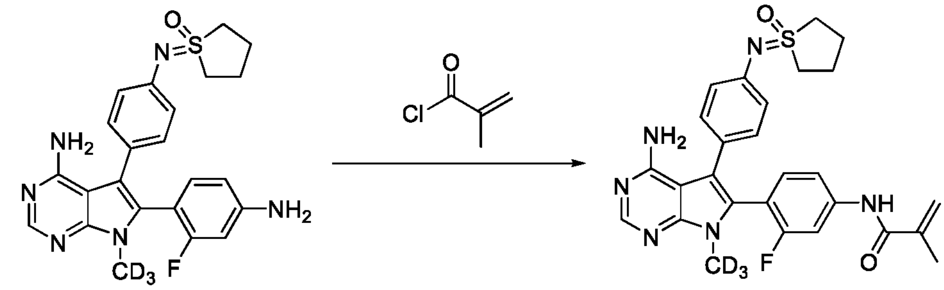

83d 83

**Step 1 1- (4- (4-amino-7- (methyl-$d_3$) -7H-pyrrolo [2,3-*d*] pyrimidin-5-yl) phenyl) imino) tetrahydro-1*H*-1λ$^6$-thiophene-1-oxide 83a**

To a reaction flask, compound **82c** (18.9 g, 62 mmol), compound **24b** (16.8 g, 52 mmol), $Pd(PPh_3)_4$ (6.0 g, 5.2 mmol), $K_3PO_4$ (33 g, 156 mmol), 1,4-dioxane (300 mL), and $H_2O$ (30 mL) were added, after replaced with argon gas three times, the reaction solution was heated up to 70 °C and stirred for 16 h. Dioxane was removed by distillated under reduced pressure, water (50 mL) and DCM (200 mL) were added, and it was stirred, filtered through diatomaceous earth, extracted, and concentrated under reduced pressure to obtain crude product. The crude product was purified by column chromatography (MeOH/DCM=1/20) to obtain compound **83a** (9.3 g), light yellow solid.

$^1$H NMR (400 MHz, $CDCl_3$) $\delta$ 8.33 (s, 1H), 7.34 (d, $J$ = 8.4 Hz, 2H), 7.14 *(d, J* = 8.4 Hz, 2H), 6.88 (s, 1H), 5.12 (s, 2H), 3.50-3.40 (m, 2H), *3.25-3.18* (m, 2H), 2.40 -2.20 (m, 4H).

**Step 2 1- (4-(4-amino-6-bromo-7-(methyl-$d_3$)-7*H*-pyrrolo[2,3-*d*]pyrimidin-5-yl) phenyl)imino)tetrahydro-1*H*-1λ$^6$-thiophene-1-oxide 83b**

Compound **83a** (7.0 g, 20 mmol) was dissolved in DMF (30 mL), and a solution of NBS (3.8 g, 21 mmol) in DMF (30 mL) was added dropwise at 0 °C. The reaction solution was stirred at 0 °C for 1 h, and then quenched with saturated $Na_2S_2O_3$ aqueous solution (30 mL). Water (20 mL) was added, and it was stirred for half an hour to precipitate a solid, filtered, washed with water, washed with ethyl acetate, and dried to obtain compound **83b** (5.8 g), yellow solid.

$^1$H NMR (400 MHz, $CDCl_3$) $\delta$ 8.29 (s, 1H), 7.33 (d, $J$ = 8.4 Hz, 2H), 7.16 *(d, J* = 8.4 Hz, 2H), 4.98 (s, 2H), 3.52-3.43 (m, 2H), 3.28-3.20 (m, 2H), 2.42-2.22 (m, 4H).

**Step 3 1- (4- (4-amino-6-(4-amino-2-fluorophenyl) -7-(methyl-*d*$_3$)-7*H*-pyrrolo[2,3-*d*] pyrimidin-5-yl)phenyl)imino) tetrahydro-1*H*-1λ$^6$-thiophene-1-oxide 83d**

To a reaction flask, compound **83b** (5.0 g, 12 mmol), **83c** (4.27 g, 18 mmol), $Pd(PPh_3)_4$ (1.39 g, 1.2 mmol), $K_3PO_4$ (7.6 g, 36 mmol), dioxane (60 mL), and water (6 mL) were added, after replaced with argon gas three times, the reaction solution was heated up to 90 °C and stirred for 16 h, and then Dichloromethane (40 mL) and $H_2O$ (40 mL) were added, stirred for half an hour, filtered, washed with water, and washed with dichloromethane to obtain compound 83d (4.0 g), gray solid.

$^1$H NMR (400 MHz, $CDCl_3$) δ 8.32 (s, 1H), 7.13 (d, *J* = 8.4 Hz, 2H), 6.98 (d, *J* = 8.4 Hz, 2H), 6.85 (t, *J* = 8.2 Hz, 1H), 6.42 (dd, *J* = *11.4,* 2.4 Hz, 1H), 6.35 (dd, *J* = 8.2, 2.4 Hz, 1H), 5.01 (s, 2H), 3.89 (s, 2H), 3.46-3.36 (m, 2H), 3.24-3.14 (m, 2H), 2.40-2.18 (m, 4H).

**Step 4 *N*-(4-(4-amino-7-(methyl-*d*$_3$)-5-(4-(1-oxotetrahydro-1λ$^6$-thiophene-1-imino) phenyl)-7*H*-pyrrolo[2,3-*d*]pyrimidin-6-yl)-3-fluorophenyl)methacrylamide 83**

Compound **83d** (4.2 g, 9.3 mmol), pyridine (5 mL), and DMF (20 mL) were added to a reaction flask, a solution of methacryloyl chloride (1.36 g, 13 mmol) in DMF (20 mL) was added dropwise at 0 °C, and it was heated up to room temperature to react for 1 h. The reaction was quenched with added water, extracted with ethyl acetate (200 mL × 3), washed with saturated saline solution, and concentrated under reduced pressure to obtain a crude product. The crude product was purified by column chromatography (MeOH/DCM=1/20) to obtain compound **83** (2.9 g), white solid.

MS (ESI) *m*/*z* 522 [M + H]$^+$.

$^1$H NMR (400 MHz, $CDCl_3$) δ 8.36 (s, 1H), 7.70 (dd, *J* = 11.8, 1.8 Hz, 1H), 7.57 (s, 1H), 7.17-6.93 (m, 6H), 5.80 (s, 1H), 5.51 (d, *J* = 1.6 Hz, 1H), 5.00 *(s,* 2H), 3.49-3.35 (m, 2H), 3.24-3.14 (m, 2H), 2.40-2.19 (m, 4H), 2.07 (s, 3H).

**Example 84 *N*-(4-(4-amino-5-(3-fluoro-4-(1-oxo-1λ$^6$-thietan-1-imino)phenyl)-7-(methyl-*d*$_3$)-7*H*-pyrrolo[2,3-*d*]pyrimidin-6-yl)-3-chlorophenyl)methacrylamide**

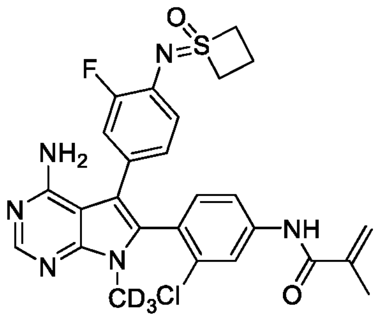

84

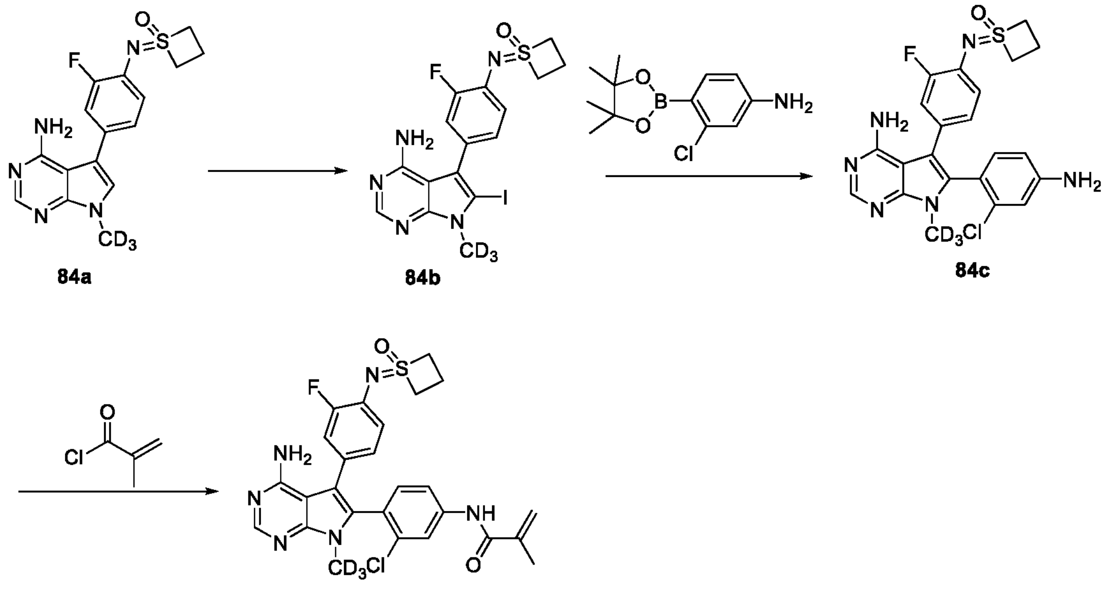

84a

84b

84c

84

**Step 1 1- (4- (4-amino-6-iodo-7- (methyl-$d_3$) -7*H*-pyrrolo [2,3-*d*] pyrimidin-5-yl) -2-fluorophenyl) imino)-1$\lambda^6$-thietane-1-oxide 84b**

Compound 84a (216 mg, 0.6 mmol) was dissolved in dichloromethane (6 mL), trifluoroacetic acid (0.14 mL, 1.8 mmol) was added at 0 °C, and NIS (168 mg, 0.72 mmol) was added in batches. The reaction solution was heated up to room temperature and stirred for 1.5 h, and then quenched with added $Na_2S_2O_3$ (sat. 3 mL), extracted with water (10 mL), dichloromethane, and concentrated under reduced pressure to obtain a crude product. The crude product was purified by column chromatography (MeOH/DCM=1/20) to obtain compound 84b (light yellow solid, 183 mg, 64%).

$^1$H NMR (400 MHz, $CDCl_3$) $\delta$ 8.24 (s, 1H), 7.21-7.06 (m, 3H), 5.11 (s, 2H), 4.41-4.26 (m, 4H), 2.43-2.32 (m, 2H).

**Step 2 1-(4-(4-amino-6-(4-amino-2-chlorophenyl)-7-(methyl-$d_3$)-7*H*-pyrrolo [2,3-*d*] pyrimidin-5-yl)-2-fluorophenyl)imino)-1$\lambda^6$-thietane-1-oxide 84c**

To a reaction flask, compound **84b** (183 mg, 0.4 mmol), 3-chloro-4-(4,4,5,5-tetramethyl-1,3,2-dioxaborolan-2-yl) aniline (150 mg, 0.6 mmol), $Pd(PPh_3)_4$ (45 mg, 0.04 mmol), $K_3PO_4$ (0.25 g, 1.2 mmol), dioxane (4 mL), and water (0.4 mL) were added, after replaced with argon gas three times, the reaction solution was heated up to 80 °C and stirred for 16 h, and then extracted with dichloromethane (40 mL) and $H_2O$ (10 mL). The crude product was concentrated under reduced pressure and purified by column chromatography (MeOH/DCM=1/20) to obtain compound **84c** (light yellow solid, 106 mg, 58%).).

$^1$H NMR (400 MHz, $CDCl_3$) $\delta$ 8.36 (s, 1H), 7.05-6.88 (m, 4H), 6.77 (d, $J$ = 2.4 *Hz*, 1H), 6.50 (dd, $J$ = 8.3, 2.4 Hz, 1H), 5.02 (s, 2H), 4.38-4.20 (m, 4H), 3.86 (s, 2H), 2.41-2.28 (m, 2H).

**Step 3 *N-(4-*(4-amino-5-(3-fluoro-4-(1-oxo-1 λ$^6$-thietan-1-imino) phenyl) -7-(methyl-*d*$_3$)-7*H*-pyrrolo[2,3-*d*]pyrimidin-6-yl)-3-chlorophenyl)methacrylamide 84**

Compound 84c (50 mg, 0.1 mmol), pyridine (0.5 mL), and DMF (2 mL) were added to a reaction flask, a solution of methacryloyl chloride (14 mg, 0.13 mmol) in DMF (2 mL) was added dropwise at 0 °C, and heated up to room temperature to react for 1 h. The reaction was quenched with added water, extracted with ethyl acetate (100 mL × 3), washed with saturated saline solution, and concentrated under reduced pressure to obtain a crude product. The crude product was purified by column chromatography (MeOH/DCM=1/20) to obtain compound **84** (38 mg), white solid.

MS (ESI) *m/z* 542 [M + H]$^+$.

$^1$H NMR (400 MHz, $CDCl_3$) *δ* 8.35 (s, 1H), 7.89 (d, *J* = 2.2 Hz, 1H), 7.73 *(s,* 1H), 7.37 (dd, *J* = 8.4, 2.2 Hz, 1H), 7.11 (d, *J* = 8.4 *Hz,* 1H), 6.99 (t, *J* = 8.4 Hz, 1H), 6.92 (d, *J* = 10.0 Hz, 2H), 5.80 (s, 1H), 5.50 (d, *J* = 1.6 Hz, *1H),* 5.13 (s, 2H), 4.34-4.20 (m, 4H), 2.37-2.27 (m, 2H).

**Example 85 *N*-(4-(4-amino-5-(4-(diethyl(oxo)-λ$^6$-sulfaneylidene) amino) -2-fluorophenyl) -7-methyl-7*H*-pyrrolo [2,3-*d*] pyrimidin-6-yl) -3-fluorophenyl) methacrylamide**

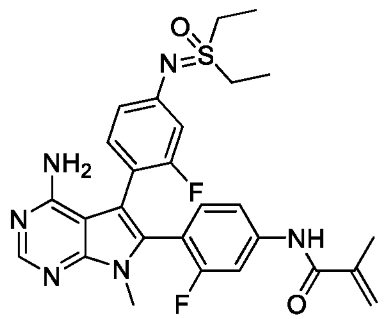

**85**

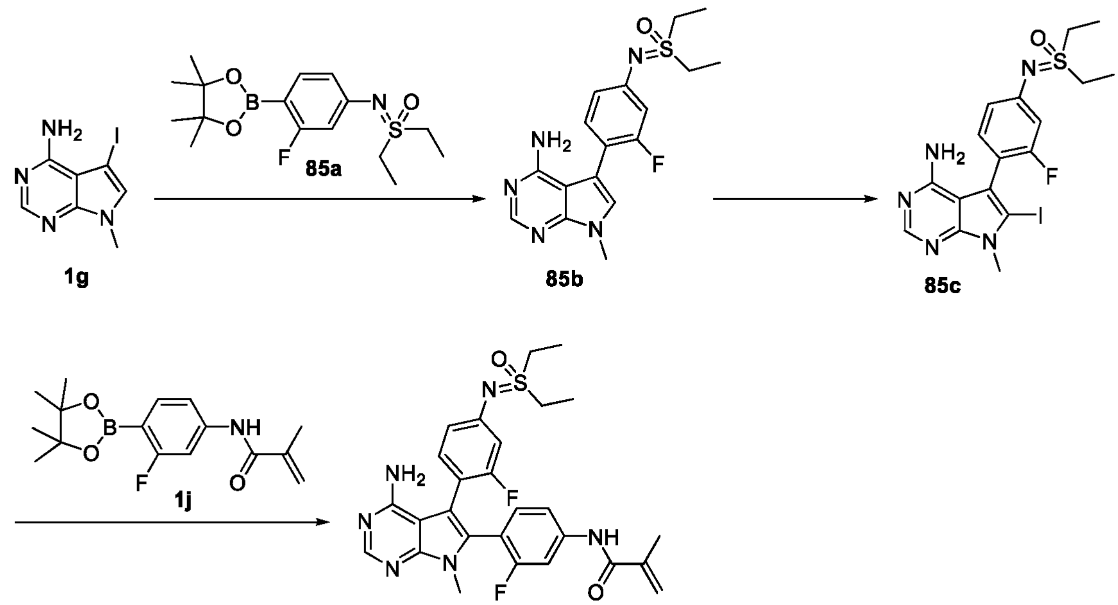
1g
85a
85b
85c
1j

**85**

**Step 1 1 (4- (4-amino-7-methyl-7*H*-pyrrolo [2,3-*d*] pyrimidin-5-yl) -3-fluorophenyl) imino*)* diethyl - λ$^6$-sulfone 85b**

To the reaction flask, compound **85a** (500 mg, 1.54 mmol), 1,4-dioxane (5 mL), $H_2O$ (1 mL), compound **1g** (383 mg, 1.40 mmol), potassium phosphate (890 mg, 4.2 mmol), and $Pd(PPh_3)_4$ (162 mg, 0.14 mmol) were added in sequence,

after replaced with nitrogen gas three times, heated up to 90 °C and reacted for 3 h. New spots were observed in TLC monitoring, and the reaction solution was diluted with ethyl acetate and $H_2O$. After filtrated through diatomaceous earth, it was extracted with ethyl acetate, the organic phase was washed with saturated saline solution, dried over anhydrous sodium sulfate, concentrated under reduced pressure, and purified by column chromatography (DCM/MeOH=10/1) to obtain compound **85b** (262 mg), yellow solid.

MS (ESI) *m/z* 362 $[M + H]^+$

**Step 2 (4- (4-amino-6-iodo-7-methyl-7H-pyrrolo [2,3-d] pyrimidin-5-yl) -3- fluorophenyl) imino) diethyl - $\lambda^6$-sulfone 85c**

Compound **85b** (262 mg, 0.72 mmol) was dissolved in anhydrous DCM (3 mL), TFA (246 mg, 2.16 mmol) and NIS (163 mg, 0.72 mmol) were added at 0 °C for 1 h, and then the reaction was quenched with added sodium thiosulfate solution, extracted with saturated sodium bicarbonate aqueous solution and dichloromethane, the organic layers were combined, and washed with saturated salt water once. The organic phase was dried over anhydrous sodium sulfate, concentrated under reduced pressure, and purified by column chromatography (PE/EA=1:1) to obtain compound **85c** (176 mg), yellow solid.

MS (ESI) *m/z* 488 $[M + H]^+$

**Step 3 *N-* (4- (4-amino-5- (4- (diethyl (oxo) - $\lambda^6$- sulfaneylidene) amino) -2-fluorophenyl) -7- methyl-7*H*-pyrrolo [2,3-*d*] pyrimidin-6-yl) -3-fluorophenyl) methacrylamide 85**

To a reaction flask, compound **85c** (176 mg, 0.36 mmol), 1,4-dioxane (4 mL), water (1 mL), compound **1j** (132 mg, 0.433 mmol), $Pd(PPh_3)_4$ (42 mg, 0.036 mmol), and potassium phosphate (82.8 mg, 0.39 mmol) were added, after replaced with nitrogen gas three times, the reaction was carried out at 90 °C for 3 h. The reaction solution was diluted with added Ethyl acetate (10 mL) and $H_2O$ (10 mL), filtered through diatomaceous earth, and extracted with ethyl acetate, and the organic phase was washed with saturated saline solution, dried over anhydrous sodium sulfate, concentrated under reduced pressure, and purified by column chromatography (DCM/MeOH=10/1) to obtain compound **85** (57.5 mg), white solid.

MS (ESI) *m/z* 539.2 $[M + H]^+$.

$^1H$ NMR (400 MHz, MeOD-d4) $\delta$ 8.17 (s, 1H), 7.64 (dd, *J* = 12.2, 1.9 Hz, 1H), 7.42 - 7.34 (m, 2H), 7.29 - 7.19 (m, 3H), 5.83 (s, 1H), 5.56 (s, 1H), 3.84 (s, *3H),* 3.23 (m, 4H), 2.05 (s, 3H), 1.29 (t, *J* = 7.4 Hz, 6H).

**Example 86 *N-* (4- (4-amino-5- (4- (diethyl (oxo) - $\lambda^6$- sulfaneylidene) amino) phenyl) -7-(2-methoxyethyl) -7H-pyrrolo [2,3-*d*] pyrimidin-6-yl) -3-fluorophenyl) methacrylamide**

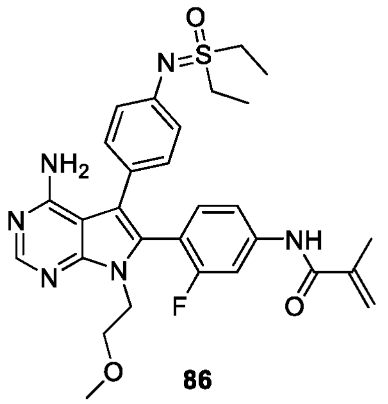

86

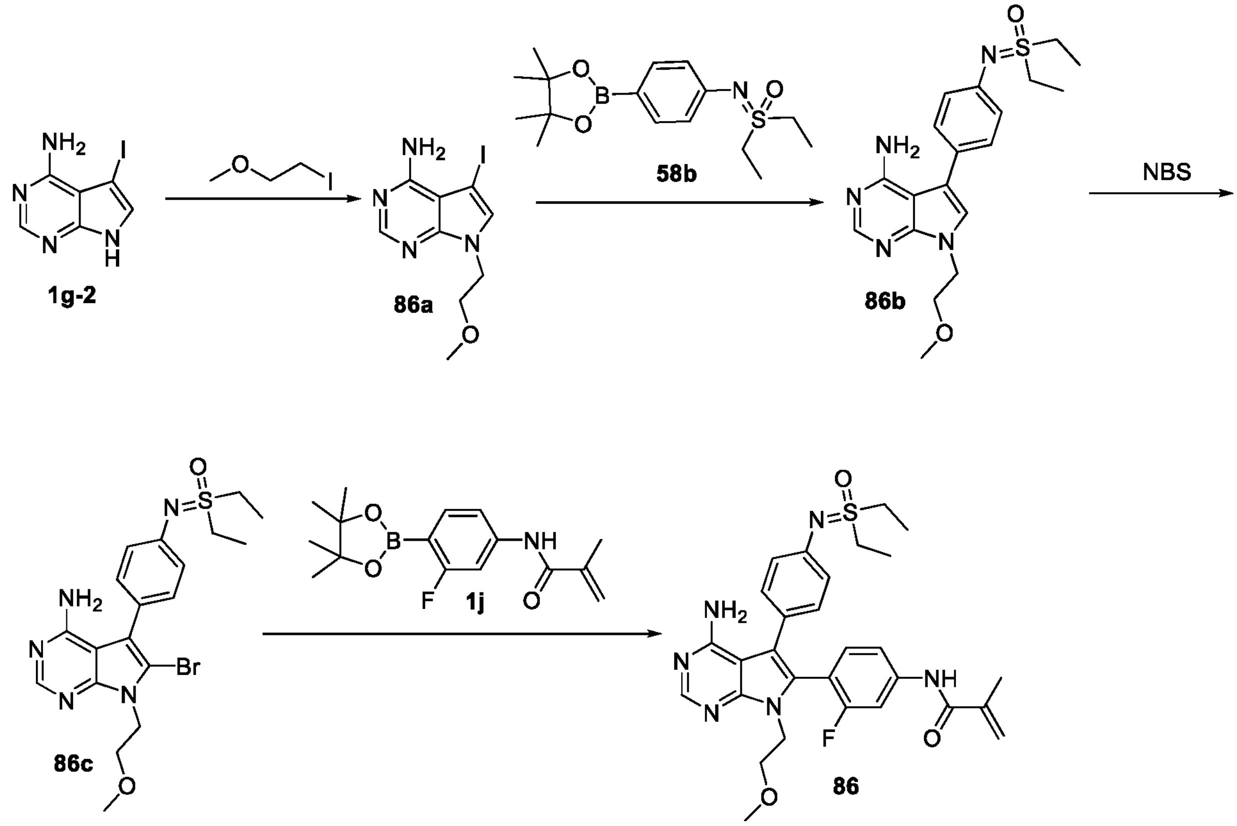


1g-2 86a 58b 86b 86c 1j 86

**Step 1 5-iodo-7- (2-methoxyethyl) -7H-pyrrolo [2,3-d] pyrimidin-4-amine 86a**

To a reaction flask, compound **1g-2** (500 mg, 1.92 mmol), DMF (5 mL), cesium carbonate (1.25 g, 3.84 mmol) and 1-iodo-2-methoxyethane (429 mg, 2.31 mmol) were added sequentially, the reaction was carried out at room temperature for 2 h. $H_2O$ (50 mL) was added and extracted with ethyl acetate, and the organic phase was washed with saturated saline solution, dried over anhydrous sodium sulfate, concentrated under reduced pressure, and purified by column chromatography (DCM/MeOH=10/1) to obtain compound **86a** (464 mg).

MS (ESI) *m/z* 319 $[M + H]^+$

**Step 2 (4- (4-amino-7- (2-methoxyethyl) -7*H*-pyrrolo [2,3-*d*] pyrimidin-5-yl) phenyl) imino) diethyl - $\lambda^6$-sulfone 86b**

To a reaction flask, compound **86a** (440 mg, 1.38 mmol), 1,4-dioxane (5 mL), water (1 mL), compound **58b** (537 mg, 1.66 mmol), $Pd(PPh_3)_4$ (160 mg, 0.138 mmol), and potassium phosphate (292 mg, 1.38 mmol) were added in sequence, after replaced with nitrogen gas three times, the reaction was carried out at 90 °C for 2 h. After the reaction was completed, it was cooled down to room temperature, and the reaction solution was diluted with added ethyl acetate and $H_2O$. After filtrated through diatomaceous earth to extract with EA, and the organic phase was washed with saturated saline solution, dried over anhydrous sodium sulfate, concentrated under reduced pressure, and purified by column chromatography (DCM/MeOH=10/1) to obtain compound **86b** (140 mg), white solid.

MS (ESI) *m/z* 388 [M + H]$^+$

**Step 3 (4- (4-amino-6-bromo-7- (2-methoxyethyl) -7*H*-pyrrolo [2,3-*d*] pyrimidin -5-yl) phenyl) imino) diethyl-6-sulfone 86c**

Compound **86b** (92 mg, 0.24 mmol) was dissolved in anhydrous DMF (4 mL), NBS (44 mg, 0.25 mmol) was added at 0 °C, and the reaction was carried out at 0 °C for 1 h, and then the reaction was quenched with added sodium thiosulfate solution, extracted with added $H_2O$ (40 mL) and ethyl acetate 3 times, and the organic phase was dried over anhydrous sodium sulfate, concentrated under reduced pressure, and purified by column chromatography (DCM/MEOH=10/1) to obtain compound **86c** (87 mg), yellow oil.

MS (ESI) *m/z* 467 [M + H]$^+$

**Step 4 *N*- (4- (4-amino-5- (4- (diethyl (oxo) - $\lambda^6$- sulfaneylidene) amino) phenyl) -7- (2-methoxyethyl) -7*H*-pyrrolo [2,3-*d*] pyrimidin-6-yl) -3-fluorophenyl) methacrylamide 86**

To a reaction flask, compound **86c** (87 mg, 0.19 mmol), 1,4-dioxane (2.4 mL), water (0.6 mL), compound **1j** (68 mg, 0.22 mmol), potassium phosphate (119 mg, 0.56 mmol), and $Pd(PPh_3)_4$ (22 mg, 0.019 mmol) were added in sequence, after replaced with nitrogen gas three times, the reaction was carried out at 90 °C for 3 h. After the reaction was completed, it was cooled down to room temperature, and the reaction solution was diluted with ethyl acetate and $H_2O$. After filtered through diatomaceous earth, it was extracted with ethyl acetate, and the organic phase was washed with saturated saline solution, dried over anhydrous sodium sulfate, concentrated under reduced pressure, and purified by column chromatography (DCM/MeOH=10/1) to obtain compound **86** (9.1 mg), white solid.

MS (ESI) *m/z* 565.38 [M + H]$^+$.

$^1$H NMR (400 MHz, MeOD-d4) $\delta$ 8.19 (s, 1H), 7.69 (dd, *J* = 12.0, 2.0 Hz, 1H), *7.39 (dd, J* = 8.4, 2.0 Hz, 1H), 7.25 (t, *J* = 8.3 Hz, 1H), 7.14 (d, *J* = 8.2 *Hz,* 2H), 7.05 (d, *J* = 8.6 Hz, 2H), 5.83 (s, 1H), 5.57 (s, 1H), 4.38 (s,1H), 4.21(s,1H), 3.59 (s, *1H),* 3.50 (s, 1H), 3.29 (d, *J* = 7.4 Hz, 4H), 3.16 (d, *J* = 8.9 Hz, 3H), *2.04* (s, 3H), 1.46 - 1.29 (m, 6H).

**Example 87 *N*-(4-(4-amino-7-methyl-5-(3-methyl-4-((1-tetrahydro-1$\lambda^6$-thiophene -1-ylidene) amino) phenyl) -7*H*-pyrrolo [2,3-*d*] pyrimidin-6-yl) -3-fluorophenyl) methacrylamide**

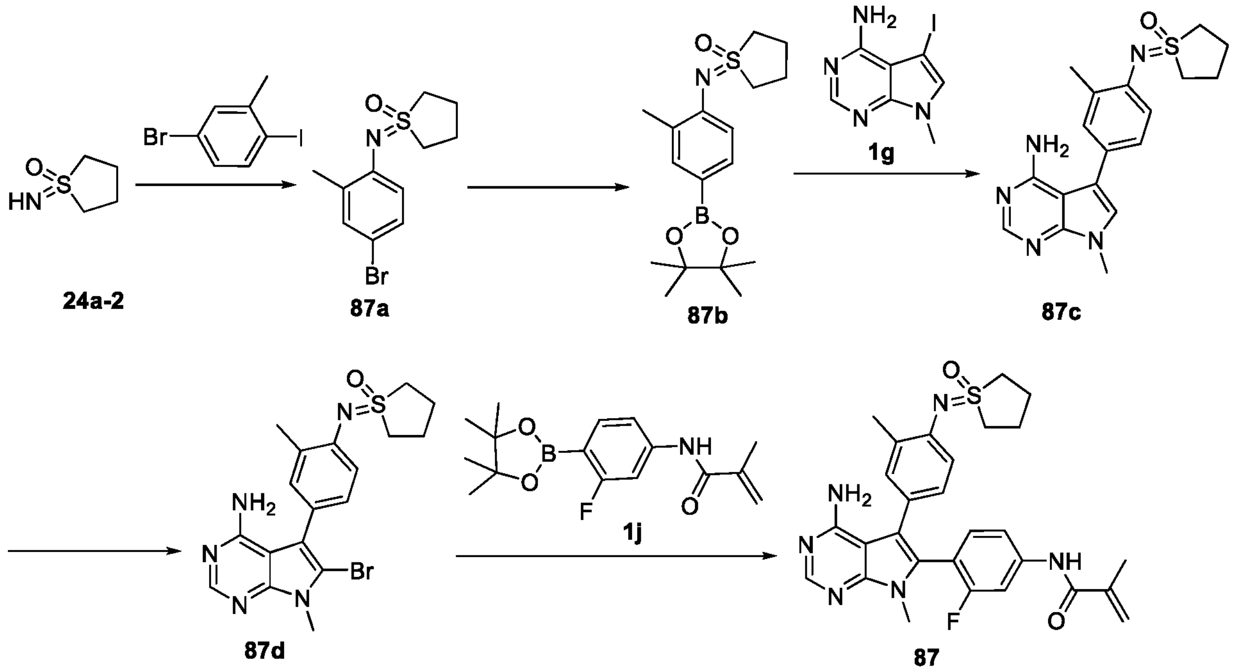

24a-2
87a
87b
1g
87c
87d
1j
87

Compound 87 was synthesized with reference to the similar synthetic route and steps of compound **82,** wherein, 4-bromo-2-chloroiodobenzene was replaced with 4-bromo-1-iodo-2-methylbenzene.

MS (ESI) *m/z* 533.35[M + H]$^+$.

$^{1}$H NMR (400 MHz, DMSO-$d_6$) $\delta$ 10.03 (s, 1H), 8.11 (s, 1H), 7.69 (dd, $J$ = 12.4, 1.9 Hz, 1H), *7.41* (dd, $J$ = 8.4, 2.0 Hz, 1H), 7.20 (t, $J$ = 8.4 Hz, 1H), 7.02 - 6.76 (m, 3H), 5.75 (s, 1H), 5.50 (s, 1H), 3.44 *(s,* 3H), 3.18 (m, 4H), 2.18 - 1.99 (m, 7*H*), 1.88 (s, 3H).

**Example 88 *N*- (4- (4-amino-5- (3-chloro-4- ((1-oxide tetrahydro-1 λ$^{6}$-thiophene-1-ylidene) amino) phenyl) -7-methyl-7*H*-pyrrolo [2,3-*d*] pyrimidin-6-yl) -3-fluorophenyl) methacrylamide**

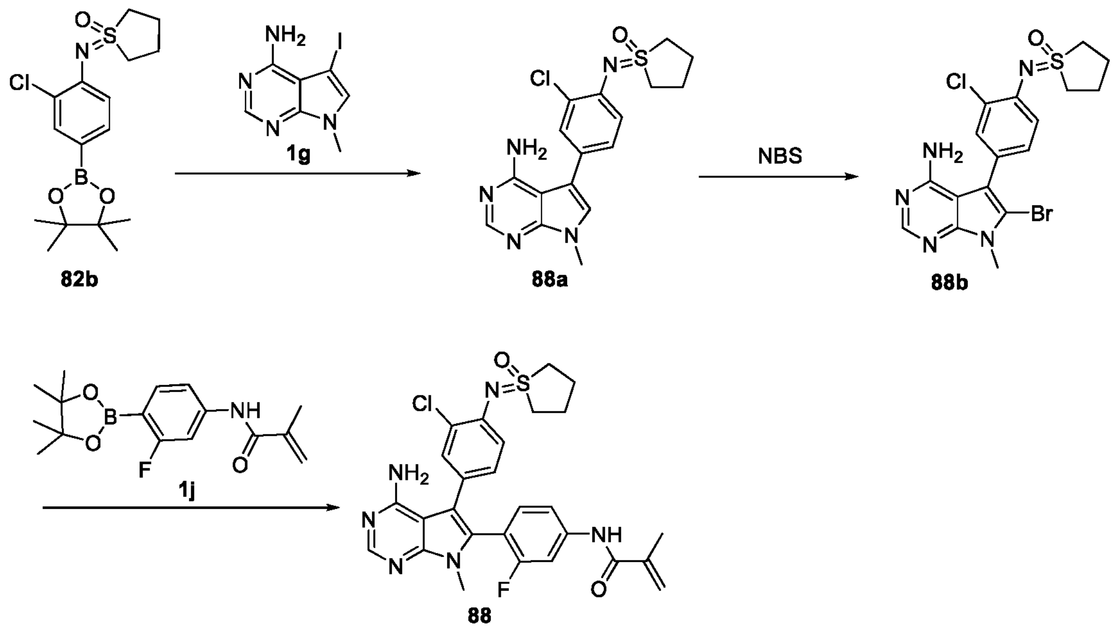

82b 88a 88b

88

Compound **88** was synthesized with reference to the similar synthetic route and steps of compound **87,** wherein, compound **87b** was replaced with compound **82b.**

MS (ESI) *m/z* 553.3 [M + H]$^{+}$.

$^{1}$H NMR (400 MHz, DMSO-$d_6$) $\delta$ 10.12 (s, 1H), 8.19 (s, 1H), 7.77 (dd, $J$ = 12.4, 1.8 Hz, 1H), *7.50* (dd, $J$ = 8.5, 1.9 Hz, 1H), 7.29 (t, $J$ = 8.4 Hz, 1H), 7.16 (d, $J$ = 1.9 Hz, 1H), 7.07 (d, $J$ = 8.2 *Hz,* 1H), 7.02 (dd, $J$ = 8.2, 1.9 Hz, 1H), 5.83 (s, 1H), 5.58 (s, 1H), 3.51 (s, 3H), 3.28 (m, *4H),* 2.17 (m, 4H), 1.95 (s, 3H).

**Example 89 *N*- (4- (4-amino-5- (3- (difluoromethyl) -4- ((1-tetrahydro-1λ$^{6}$- thiophene -1-ylidene) amino) phenyl) -7-methyl-7*H*-pyrrolo [2,3-*d*] pyrimidin-6-yl) -3-fluorophenyl) methacrylamide**

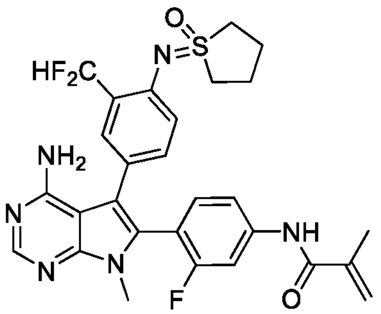

**89**

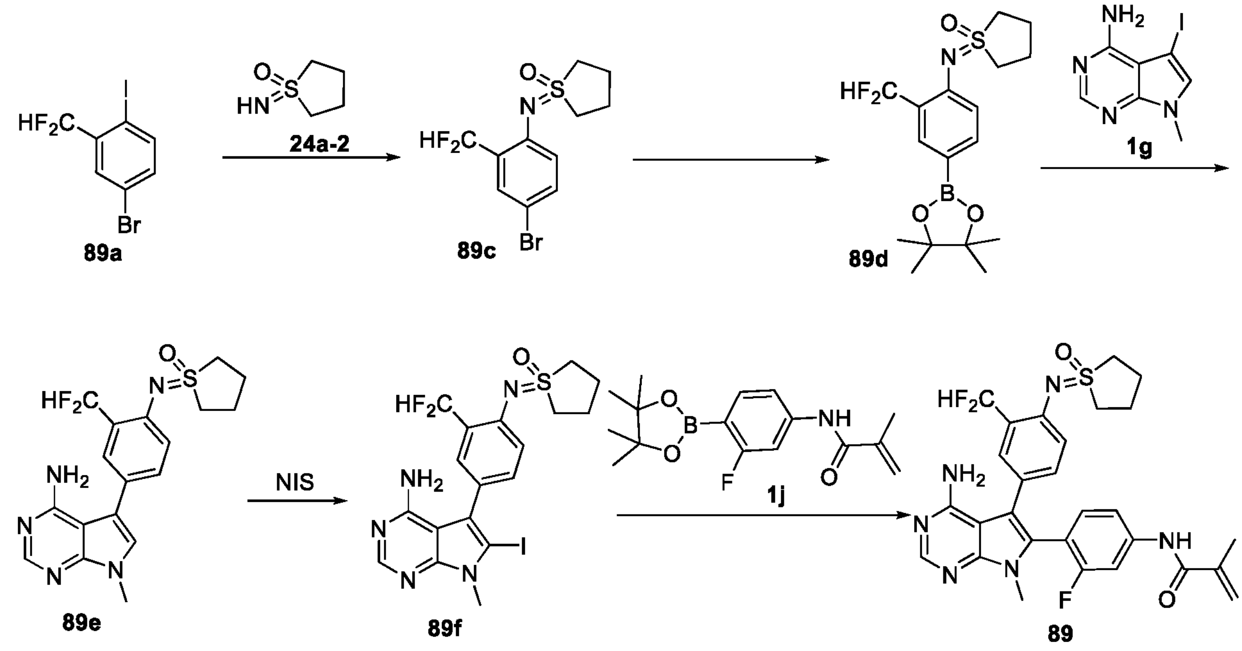


Compound **89** was synthesized with reference to the similar synthetic route and steps of compound **77,** wherein, 1-bromo-2,5-difluoro-4-iodobenzene was replaced with compound **89a.** Compound **89a** was synthesized with reference to WO 2023/001794A1.

MS (ESI) *m/z* 569.15 $[M + H]^+$.

$^1H$ NMR (400 MHz, MeOD-d4) $\delta$ 8.19 (s, 1H), 7.72 (dd, *J* = 12.1, 1.8 Hz, 1H), 7.44 - 7.34 *(m,* 2H), 7.24 - 6.83 (m, 4H), 5.81 (s, 1H), 5.55 (s, 1H), 3.62 (s, 3H), 3.46 - 3.39 (m, 2H), 3.27 - 3.19 (m, 2H), 2.27 (m, 4H), 2.02 (s, 3H).

**Example 90 *N*- (4- (4-amino-5- (3-fluoro-4- ((1-tetrahydro-1λ$^6$-thiophene-1-ylidene) amino) phenyl) -7- (methyl-*d*$_3$) -7H-pyrrolo [2,3-*d*] pyrimidin-6-yl) -5-chloro-2-methylphenyl) methacrylamide**

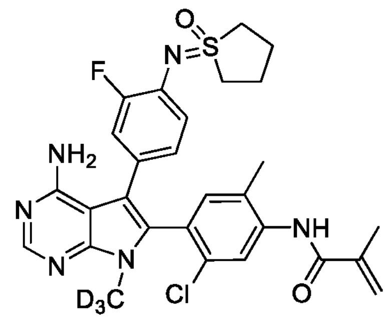

**90**

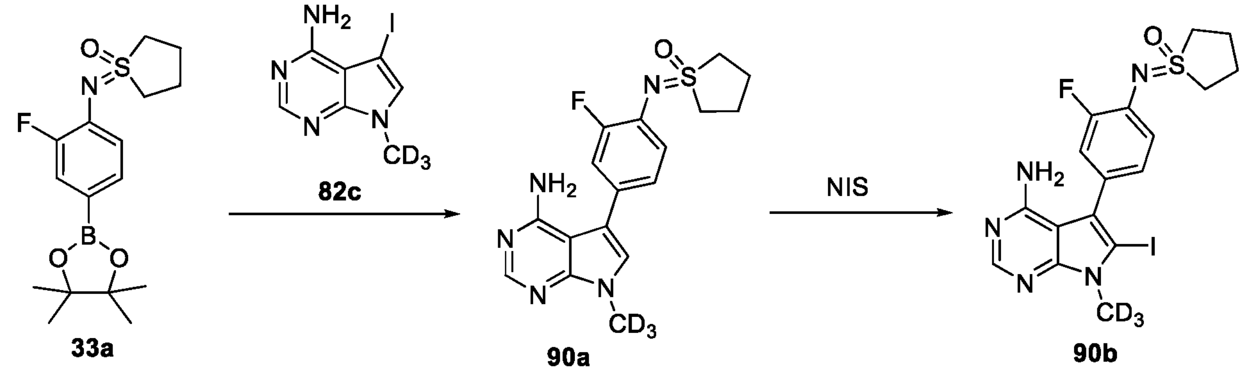


33a 82c 90a 90b

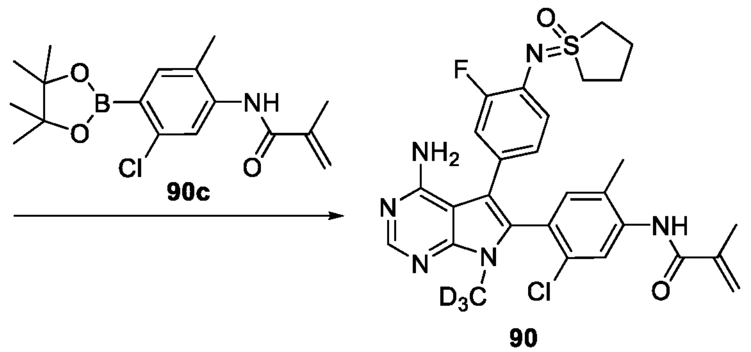

90c 90

Compound **90** was synthesized with reference to the similar synthetic route and steps of compound **85,** wherein, compound **85a** was replaced with compound **33a,** and compound **1g** was replaced with compound **82c** in step 1, and compound **1j** was replaced with compound **90c** in step 3.

MS (ESI) *m/z* 570.0 $[M + H]^+$.

$^1H$ NMR (400 MHz, DMSO-$d_6$) $\delta$ 9.39 (s, 1H), 8.20 (s, 1H), 7.65 (s, 1H), 7.32 (s, 1H), 7.11 - 6.81 (m, 3H), 5.88 (s, 1H), 5.55 (s, 1H), 3.45 - 3.36 (m, 2H), 3.27 (dd, *J* = 13.2, 6.6 Hz, 2H), 2.23 - 2.07 (m, 7*H*), 1.97 (s, 3H).

**Example 91 *N*- (4- (4-amino-5- (3-fluoro-4- ((1-tetrahydro-1λ$^6$-thiophene-1-ylidene) amino) phenyl) -7- (methyl-$d_3$) -7*H*-pyrrolo [2,3-*d*] pyrimidin-6-yl) -5-fluoro-2- methylphenyl) methacrylamide**

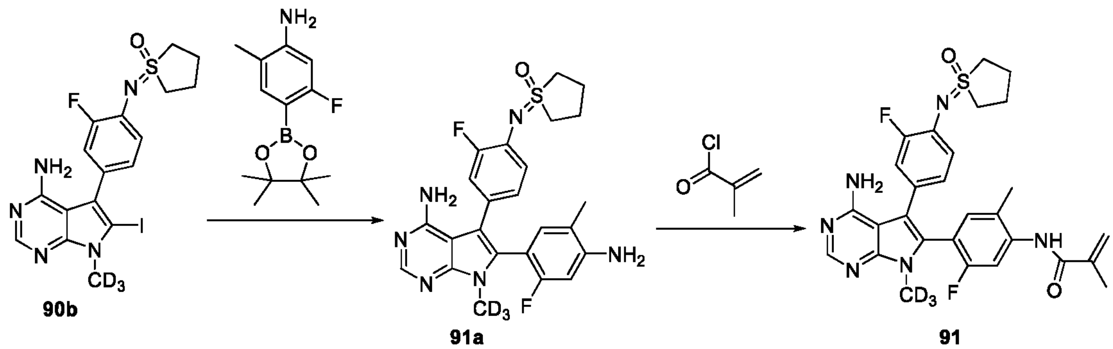

90b 91a 91

**Step 1 1- (4- (4-amino-6- (4-amino-2-fluoro-5-methylphenyl) -7- (methyl-$d_3$) -7*H*-pyrrolo [2,3-*d*] pyrimidin-5-yl) -2-fluorophenyl) imino) tetrahydro-1H-1 λ$^6$- thiophene-1-oxide 91a**

Compound **90b** (210 mg, 0.43 mmol), 5-fluoro-2-methyl-4-(4,4,5,5-tetramethyl-1,3,2-dioxaborolan-2-yl)aniline (160 mg, 0.63 mmol), $Pd(PPh_3)_4$ (49 mg, 0.04 mmol), and potassium phosphate (270 mg, 1.29 mmol) were dissolved in 1,4-dioxane (8 mL) and water (2 mL), after replaced with nitrogen gas three times, the reaction was refluxed at 90 °C for 12 h. The reaction solution was cooled down to room temperature, filtered through diatomaceous earth, concentrated under

reduced pressure to remove the solvent, extracted with DCM/$H_2O$ three times, and the organic layers were combined, washed with saturated saline solution, dried over anhydrous sodium sulfate, concentrated under reduced pressure, purified by column chromatography (DCM/MEOH=40/1) to obtain compound **91a** (100 mg), light yellow solid.

MS (ESI) *m/z* 485.90 $[M + H]^+$.

**Step 2 *N-* (4- (4-amino-5- (3-fluoro-4- ((1-tetrahydro-1λ$^6$-thiophene-1-ylidene) amino) phenyl) -7- (methyl-*d*$_3$) -7H-pyrrolo [2,3-*d*] pyrimidin-6-yl) -5-fluoro-2- methylphenyl) methacrylamide 91**

Compound **91a** (44 mg, 0.09 mmol) was dissolved with anhydrous DMF (10 mL), and pyridine (0.5 mL) was added dropwise. Methacryloyl chloride (11 mg, 0.11 mmol) was dissolved in anhydrous DMF (1 mL), and the solution of methacryloyl chloride in DMF was added dropwise to the DMF solution of compound **91a** at 0 °C, and the reaction was carried out for 1 h, quenched with water, extracted with DCM/$H_2O$ three times, and the organic layers were combined, washed with saturated saline solution, dried over anhydrous sodium sulfate, concentrated under reduced pressure, and purified by reverse column chromatography to obtain compound **91** (13 mg), white solid.

MS (ESI) *m/z* 553.88 $[M + H]^+$.

$^1H$ NMR (400 MHz, $CDCl_3$) $\delta$ 8.39 (s, 1H), 8.15 (d, *J* = 11.6 Hz, 1H), 7.48 (s, *1H),* 7.13 (t, *J* = 8.5 Hz, 1H), 7.01 - 6.86 (m, 3H), 5.88 (s, 1H), 5.55 (d, *J* = 1.8 Hz, 1H), 5.06 (s, 2H), 3.46 (dt, *J* = 13.3, 6.8 Hz, 2H), 3.25 (dt, *J* = 13.0, 6.6 Hz, 2H), 2.34 (q, *J* = 7.7 Hz, 4H), 2.20 (s, 3H), 2.12 (s, 3H).

**Example 92 *N-* (4- (4-amino-5- (3-fluoro-4- ((1-tetrahydro-1λ 6-thiophene-1- ylidene) amino) phenyl) -7- methyl-7*H*-pyrrolo [2,3-*d*] pyrimidin-6-yl) phenyl) -2- (dimethylamino) methyl) acrylamide**

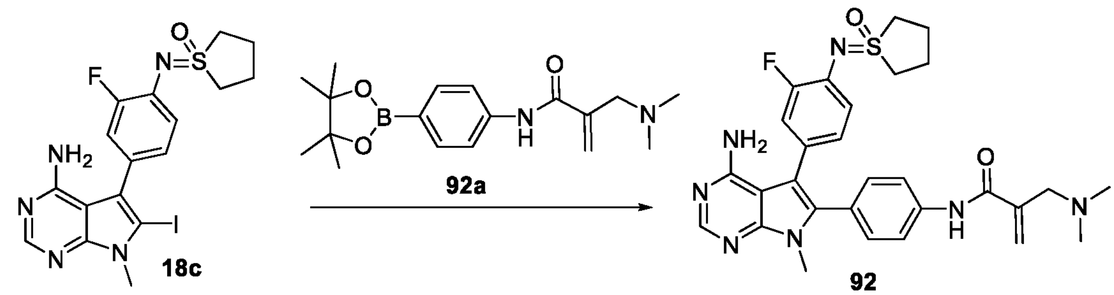

18c

92a

92

Compound **92** was synthesized with reference to the similar synthetic route and steps of compound **12,** wherein, compound **12a** was replaced with compound **18c,** compound **12b** was replaced with compound **92a.**

MS (ESI) *m/z* 562 $[M + H]^+$.

$^1H$ NMR (400 MHz, DMSO-*d*$_6$) $\delta$ 11.25 (s, 1H), 8.19 (s, 1H), 7.67 (d, *J*=8.0 Hz, 2H), 7.32 (d, *J*=8.0 Hz, 2H), 7.05 (t, *J*=12.0 Hz, 1H), 6.99-6.91 (m, 2H), 6.12 (s, 2H), 6.03 (s, 1H), 5.59 (s, 1H), 4.22 (s, 2H), 3.41-3.23 (m, 4H), 2.51-2.47 (m, 4H), 2.25 (s, 3H), 0.88 (s, 6H).

**Example 93 *N-* (4- (4-amino-5- (4- ((dicyclopropyl (oxo)-λ$^6$- sulfaneylidene) amino) phenyl)-7- methyl-7*H*-pyrrolo [2,3-*d*] pyrimidin-6-yl) phenyl) methacrylamide**

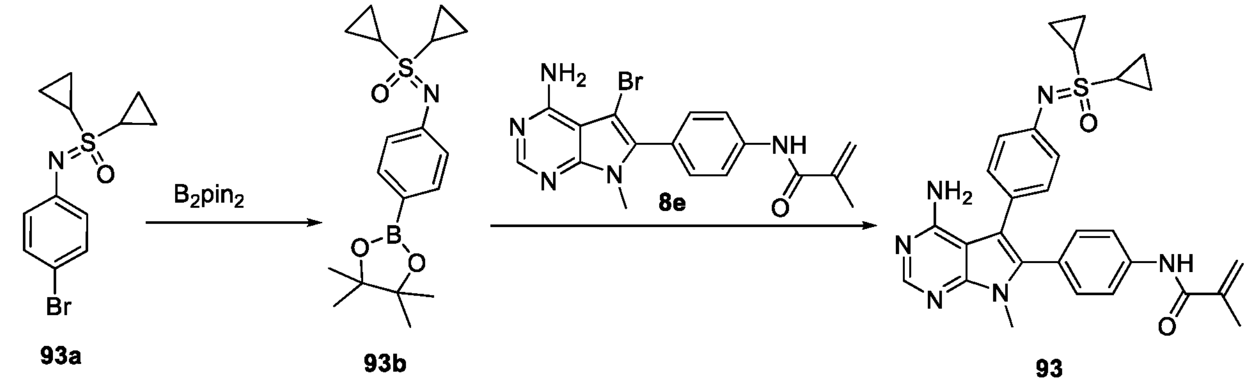


93a 93b 93

Compound **93** was synthesized with reference to the similar synthetic route and steps of compound **11,** wherein, compound **11c** was replaced with compound **93a.**

MS (ESI) *m/z* 527.2 [M + H]$^+$.

$^1$H NMR (400 MHz, $CDCl_3$) $\delta$ 8.34 (s, 1H), 7.61 - 7.50 (m, 3H), 7.23 (d, *J*=8.46, 2H), 7.10-6.94 (m, 2H), 5.81 (s, 1H), 5.50 (s, 1H), 5.17 (br. s., 2H), 3.77 - 3.69 (s, 3H), 2.57 - 2.45 (m, 2H), 2.08 (s, 3H), 1.39 - 1.16 (m, 4H), 1.11 - 0.97 (m, 4H)

**Example 94 *N*- (4-amino-5- (3-fluoro-4- (1-oxotetrahydro-1 λ$^6$-thiophene-1-ylidene) amino) phenyl) -7- (methyl-*d*$_3$) -7*H*-pyrrolo [2,3-*d*] pyrimidin-6-yl) -3-fluorophenyl) methacrylamide**

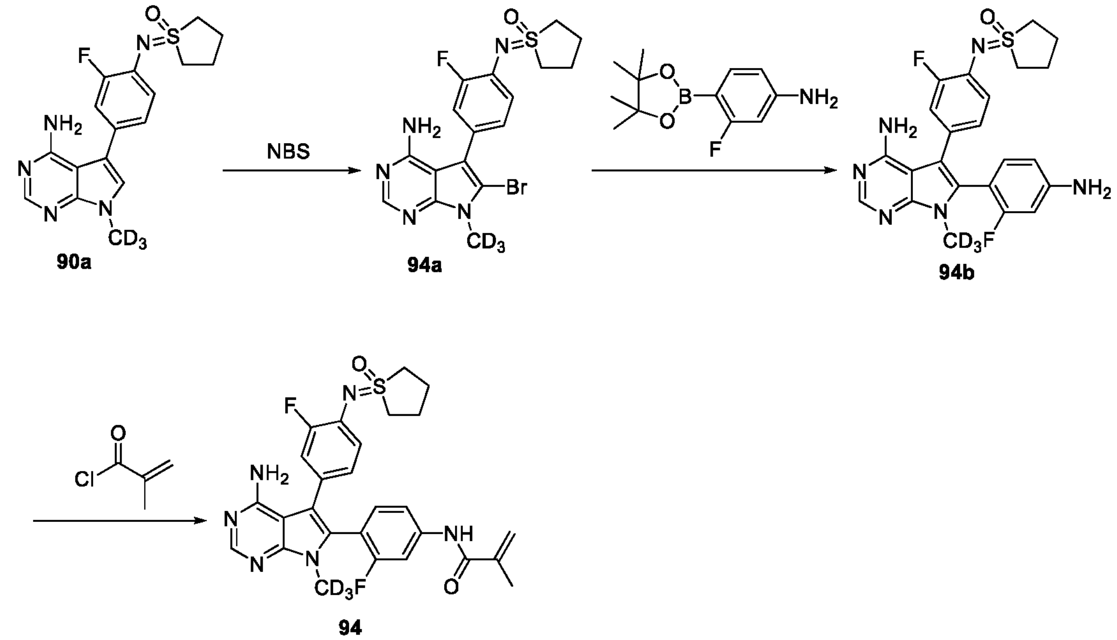


90a 94a 94b 94

**Step 1 1- (4- (4-amino-6-bromo-7- (methyl-*d*$_3$) -7*H*-pyrrolo [2,3-*d*] pyrimidin-5-yl) -2-fluorophenyl) imino) tetrahydro-1*H*-1 λ$^6$-thiophene-1-oxide 94a**

Compound **90a** (2.1 g, 5.79 mmol) was dissolved with anhydrous DMF (10 mL), NBS (1.08 g, 6.08 mmol) was added at 0 °C, and the reaction was carried out for 2 h, and then quenched with added sodium thiosulfate solution, and extracted with dichloromethane, and the organic layers were combined, and washed with saturated salt water once. The organic phase was dried over anhydrous sodium sulfate, concentrated under reduced pressure, and purified by column chromatography (DCM/MEOH=30/1) to obtain compound **94a** (1.4 g), yellow solid.

MS (ESI) *m/z* 441 [M + H]$^+$.

**Step 2 1- (4- (4-amino-6- (4-amino-2-fluorophenyl) -7- (methyl-*d*$_3$) -7*H*-pyrrolo [2,3-*d*] pyrimidin-5-yl) -2-fluorophenyl) imino) tetrahydro-1*H*-1 λ$^6$-thiophene-1-oxide 94b**

Compound **94a** (1.4 g, 3.17 mmol), 3-fluoro-4-(4,4,5,5-tetramethyl-1,3,2- dioxaborolan-2-yl) aniline (1.13 g, 4.76 mmol), $Pd(PPh_3)_4$ (366 mg, 0.32 mmol), and potassium phosphate (2.02 g, 9.52 mmol) were dissolved in 1,4-dioxane (20 mL) and water (2 mL), after replaced with nitrogen three times, the reaction was refluxed at 100 °C for 12 h. The reaction solution was cooled down to room temperature, filtered through diatomaceous earth, concentrated under reduced pressure to remove the solvent, extracted with DCM/$H_2O$ three times, and the organic layers were combined, washed with saturated saline solution and dried over anhydrous sodium sulfate, concentrated under reduced pressure, purified by column chromatography (DCM/MEOH=40/1) to obtain compound **94b** (1.33 mg), white solid.

MS (ESI) *m/z* 472 $[M + H]^+$.

**Step 3 *N*- (4- (4-amino-5- (3-fluoro-4- ((1-tetrahydro-1λ$^6$-thiophene-1-ylidene) amino) phenyl) -7- (methyl-*d*$_3$) -7*H*-pyrrolo [2,3-*d*] pyrimidin-6-yl) -3-fluorophenyl) methacrylamide 94**

Compound **94b** (610 mg, 1.29 mmol) was dissolved with anhydrous DMF (10 mL), methacryloyl chloride (162 mg, 4.76 mmol) was added at 0 °C, the reaction was carried out for 1 h, and then quenched with water, extracted with dichloromethane, and the organic layers were combined, and washed with saturated salt once. The organic phase was dried over anhydrous sodium sulfate, concentrated under reduced pressure, and purified by column chromatography (DCM/MEOH=20/1) to obtain compound **94** (140 mg), white solid.

MS (ESI) *m/z* 540.2$[M + H]^+$.

$^1H$ NMR (400 MHz, $CDCl_3$) $\delta$ 8.30 (s, 1H), 7.65 (dd, $J$ = 11.6, 2.1 Hz, 1H), 7.54 *(s,* 1H), 7.04 (ddd, $J$ = 8.6, 5.4, 3.3 Hz, 2H), 6.98 (t, $J$ = 8.0 Hz, *1H),* 6.86 (d, $J$ = 9.7 Hz, 2H), 5.74 (s, 1H), 5.45 (d, $J$ = 1.6 Hz, 1H), 4.96 (s, 2H), 3.36 (d, $J$ = 6.3 Hz, 2H), 3.18 (s, 2H), 2.24 (d, $J$ = 8.1 Hz, 4H), 2.00 (t, $J$ = 1.2 Hz, 3H).

**Example 95 *N*- (4- (4-amino-5- (3-chloro-4- ((1-oxo-1 λ$^6$-thietan-1-ylidene) amino) phenyl) -7- (methyl-*d*$_3$) -7*H*-pyrrolo [2,3-*d*] pyrimidin-6-yl) -3-chlorophenyl) methacrylamide**

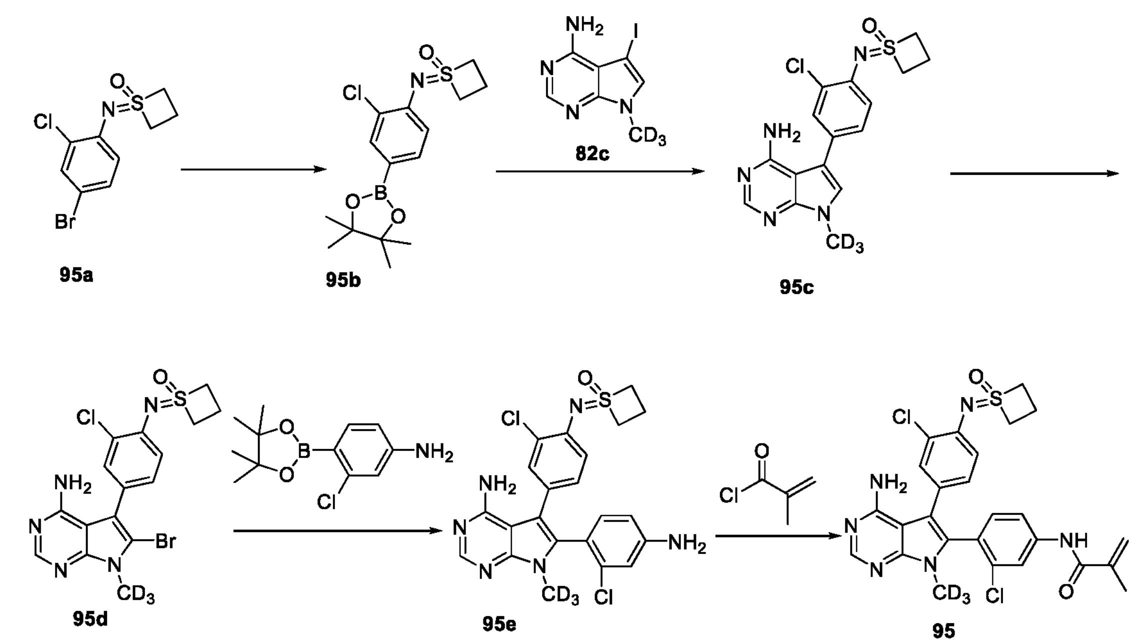

95a 95b 82c 95c

95d 95e 95

**Step 1 1- (2-chloro-4- (4,4,5,5-tetramethyl-1,3,2-dioxaborolan-2-yl) phenyl) imino) -1 λ 1$^6$-thietane-1-oxide 95b**

Compound **95a** (4 g, 13.58 mmol), bis(pinacolato)diboron (4.14 g, 16.29 mmol), $Pd(dppf)Cl_2$ (1.99 g, 2.72 mmol), and potassium acetate (3.99 g, 40.73 mmol) were added to 1,4-dioxane (50 mL), after replaced with argon gas three times, the reaction solution was heated up to 90 °C and stirred for 16 h. Water (100 mL) was added and extracted with DCM (100 mL × 3). The organic phase was washed with saturated saline solution (200 mL), dried over anhydrous sodium sulfate, and

concentrated under reduced pressure to obtain a crude product. The crude product was purified by column chromatography (PE/EtOAc=3/2) to obtain compound **95b** (4.17 g), light yellow solid.

MS (ESI) *m/z* 342 $[M + H]^+$

**Step 2 1- (4- (4-amino-7- (methyl-$d_3$) -7H-pyrrolo [2,3-d] pyrimidin-5-yl) -2-chlorophenyl) imino) -1 $\lambda^6$-thietane-1-oxide 95c**

Compound **95b** (3.57 g, 10.45 mmol), compound **82c** (3.76 g, 13.58 mmol), tetrakis (triphenylphosphine) palladium (1.21 g, 1.04 mmol), and potassium phosphate (6.65 g, 31.35 mmol) were dissolved in 1,4-dioxane (30 mL) and water (3 mL), after replaced with nitrogen three times, the reaction was carried out at 70 °C overnight. After the reaction was completed, it was cooled down to room temperature, filtered through diatomaceous earth, concentrated under reduced pressure to remove the solvent, extracted with DCM/$H_2O$ three times, and the organic layers were combined, and washed with saturated sodium chloride once, and the organic phase was dried over anhydrous sodium sulfate, concentrated under reduced pressure, and purified by column chromatography (DCM/MEOH=30/1) to obtain compound 95c (2.91 g), yellow solid.

MS (ESI) *m/z* 365 $[M + H]^+$.

**Step 3 1- (4- (4-amino-6-bromo-7- (methyl-$d_3$) -7*H*-pyrrolo [2,3-*d*] pyrimidin-5-yl) -2-chlorophenyl) imino) -1 $\lambda^6$-thietane-1-oxide 95d**

Compound **95c** (1 g, 2.74 mmol) was dissolved with anhydrous DMF (10 mL), NBS (512 mg, 2.88 mmol) was added at 0 °C, and the reaction was carried out for 2 h, and then quenched with sodium thiosulfate solution, extracted with EA/$H_2O$ 4 times, and the organic layers were combined, washed with saturated salt water once, dried over anhydrous sodium sulfate, concentrated under reduced pressure, and purified by column chromatography (DCM/MEOH=30/1) to obtain compound **95d** (800 mg), yellow solid.

MS (ESI) *m/z* 443 $[M + H]^+$.

**Step 4 1- ((4- (4-amino-6- (4-amino-2-chlorophenyl) -7- (methyl-$d_3$) -7*H*-pyrrolo [2,3-*d*] pyrimidin-5-yl) -2-chlorophenyl) imino) -1 $\lambda^6$-thietane-1-oxide 95e**

Compound 95d (800 mg, 1.80 mmol), 3-chloro-4- (4,4,5,5-tetramethyl-1,3,2 -dioxaborolan-2-yl) aniline (686 mg, 2.70 mmol), tetrakis (triphenylphosphine) palladium (208 mg, 0.18 mmol), and potassium phosphate (1.15 g, 5.41 mmol) were dissolved in 1,4-dioxane (20 mL) and water (2 mL), after replaced with nitrogen gas three times, the reaction was refluxed at 100 °C for 12 h. The reaction solution was cooled down to room temperature, filtered through diatomaceous earth, concentrated under the reduced pressure to remove the solvent, extracted with DCM/$H_2O$ three times, and the organic layers were combined, washed with saturated saline solution, dried over anhydrous sodium sulfate. concentrated under the reduced pressure, and purified by column chromatography (DCM/MEOH=40/1) to obtain compound **95e** (376 mg), white solid.

MS (ESI) *m/z* 490 $[M + H]^+$.

**Step *5 N-* (4- (4-amino-5- (3-chloro-4- ((1-oxo-1 $\lambda^6$-thietan-1-ylidene) amino) phenyl) -7-(methyl-$d_3$) -7*H*-pyrrolo [2,3-*d*] pyrimidin-6-yl) -3-chlorophenyl) methacrylamide 95**

Compound **95e** (376 mg, 0.77 mmol) was dissolved with anhydrous DMF (10 mL), methacryloyl chloride (96 mg, 0.92 mmol) was added at 0 °C, the reaction was carried out for 1 h, and then quenched with water, extracted with dichloromethane, and the organic layers were combined, washed with saturated salt once, and the organic phase was dried over anhydrous sodium sulfate, concentrated under reduced pressure, and purified by column chromatography (DCM/MEOH=20/1) to obtain compound **95** (60 mg), white solid.

MS (ESI) *m/z* 558 $[M + H]^+$.

$^1H$ NMR (400 MHz, $CDCl_3$) $\delta$ 8.37 (s, 1H), 7.89 (d, *J* = 2.1 Hz, 1H), 7.55 (s, *1H),* 7.38 (dd, *J* = 8.3, 2.2 Hz, 1H), 7.29 (s, 1H), 7.14 (d, *J* = *8.3* Hz, 1H), 7.06 (d, *J* = 8.1 Hz, 1H), 7.02 (s, 1H), 5.81 (s, 1H), 5.52 (d, *J*= 1.6 Hz, 1H), 5.02 (s, 2H), 4.33 - 4.19 (m, 4H), 2.44 - 2.31 (m, 2H), 2.07 (t, *J*= 1.3 Hz, 3H).

**Example 96 *N-* (4- (4-amino-5- (3-fluoro-4- ((methyl *(oxo)* (phenyl) - $\lambda^6$- sulfaneylidene) amino) phenyl) -7-(methyl-$d_3$) -7*H*-pyrrolo [2,3-*d*] pyrimidin-6-yl) -3-fluorophenyl) methacrylamide**

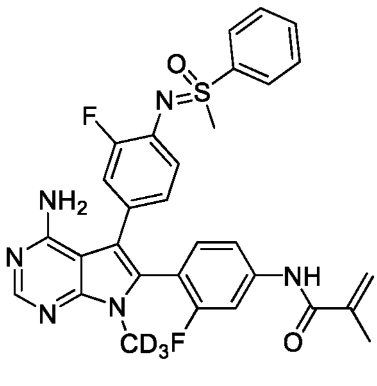

96

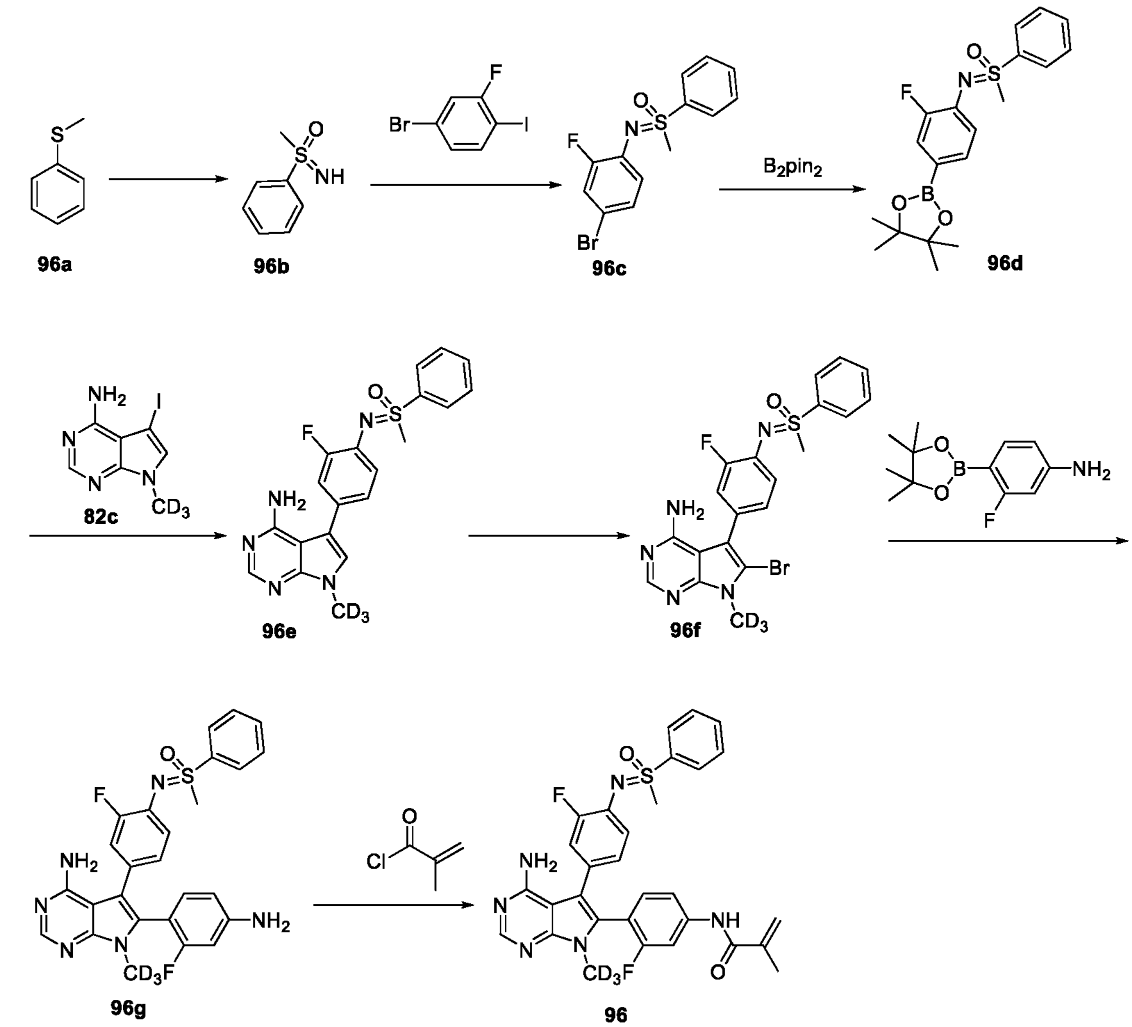


96a 96b 96c 96d

96e 96f

96g 96

**Step 1 imino (methyl) phenyl) - λ6-sulfone 96b**

Compound **96a** (10 g, 81 mmol) and ammonium carbamate (9.43 **g,** 120 mmol) were added to a flask sequentially, and then MeOH (200 mL) and iodobenzene diethyl ester (52 g, 161 mmol) were added. The reaction solution was stirred open at room temperature for 30 min, and then concentrated under reduced pressure to remove the solvent to obtain a crude product. The crude product was purified by column chromatography (MeOH/DCM=1/20) to obtain compound **96b** (5.76 g), colorless oil.

MS (ESI) *m/z* 156 $[M + H]^+$

**Step 2 ((4-bromo-2-fluorophenyl) imino) (methyl) phenyl - $\lambda^6$-sulfone 96c**

Compound **96b** (5.76 g, 37.11 mmol), cesium carbonate (18.15 g, 55.66 mmol), 1-bromo-3-fluoro-4-iodobenzene (11.17 g, 37.11 mmol), $Pd_2(dba)_3$ (1.02 g, 1.11 mmol), and Xantphos (1.29 g, 2.23 mmol) were added to 1,4-dioxane (100 mL), the reaction solution was heated up to 100 °C to react for 16 h. The reaction solution was filtered through diatomaceous earth, and the filter cake was washed with ethyl acetate (300 mL), and saturated saline solution (500 mL) was used for washing. The organic phase was dried over anhydrous sodium sulfate and concentrated under reduced pressure to obtain a crude product. The crude product was purified by column chromatography (PE/EtOAc=4/1 to 3/2) to obtain compound **96c** (11.14 g), light yellow solid.
MS (ESI) *m/z* 328 $[M + H]^+$

**Step 3 (2-fluoro-4- (4,4,5,5-tetramethyl-1,3,2-dioxaborolan-2-yl) phenyl) imino) (methyl) phenyl - $\lambda^6$-sulfone 96d**

Compound **96c** (2 g, 6.09 mmol), bis(pinacolato)diboron (1.86 mg, 7.31 mmol), $Pd(dppf)Cl_2$ (892 mg, 1.22 mmol), and potassium acetate (1.79 g, 18.28 mmol) were added to 1,4-dioxane (30 mL), after replaced with argon gas three times, and the reaction solution was heated up to 90 °C and stirred for 16 h. Water (100 mL) as added and extracted with DCM (50 mL $\times$ 3). The organic phase was washed with saturated saline solution (200 mL), dried over anhydrous sodium sulfate, and concentrated under reduced pressure to obtain a crude product. The crude product was purified by column chromatography (PE/EtOAc=3/2) to obtain compound **96d** (2.08 g), light yellow solid.
MS (ESI) *m/z* 376 $[M + H]^+$

**Step 4 (4- (4-amino-7-methyl-*d*$_3$) -7*H*-pyrrolo [2,3-*d*] pyrimidin-5-yl) -2- fluorophenyl) imino) (methyl) phenyl - $\lambda^6$-sulfone 96e**

Compound **96d** (2.08 g, 5.54 mmol), compound **82c** (2.00 g, 7.21 mmol), tetrakis (triphenylphosphine) palladium (640 mg, 0.55 mmol), and potassium phosphate (4.20 g, 19.83 mmol) were added in 1,4-dioxane (30 mL) and water (3 mL), after replaced with nitrogen gas three times, the reaction was carried out at 70 °C overnight. After the reaction was completed, it was cooled down to room temperature, filtered through diatomaceous earth, concentrated under reduced pressure to remove the solvent, extracted with DCM/$H_2O$ three times, and the organic layers were combined, washed with saturated sodium chloride once, dried over anhydrous sodium sulfate, concentrated under reduced pressure, and purified by column chromatography (DCM/MEOH=30/1) to obtain compound **96e** (1.26 g), yellow solid.
MS (ESI) *m/z* 399 $[M + H]^+$.

**Step 5 (4- (4-amino-6-bromo-7-methyl-*d*$_3$) -7*H*-pyrrolo [2,3-*d*] pyrimidin-5-yl) -2-fluorophenyl) imino) (methyl) phenyl - $\lambda^6$-sulfone 96f**

Compound **96e** (1.26 g, 3.16 mmol) was dissolved with anhydrous DMF (15 mL), NBS (591 mg, 3.32 mmol) was added at 0 °C, the reaction was carried out for 2 h, and then quenched with sodium thiosulfate solution, extracted with dichloromethane, and the organic layers were combined, and washed with saturated salt water once, and the organic phase was dried over anhydrous sodium sulfate, concentrated under reduced pressure, and purified by column chromatography (DCM/MEOH=30/1) to obtain compound **96f** (726 g), yellow solid.
MS (ESI) *m/z* 477 $[M + H]^+$.

**Step 6 (4- (4-amino-6- (4-amino-2-fluorophenyl) -7-methyl-*d*$_3$) -7*H*-pyrrolo [2,3-*d*] pyrimidin-5-yl) -2-fluorophenyl) imino) (methyl) phenyl - $\lambda^6$-sulfone 96g**

Compound **96f** (726 mg, 1.52 mmol), 3-fluoro-4- (4,4,5,5-tetramethyl-1,3,2-dioxaborolan-2-yl) aniline (578 mg, 2.28 mmol), tetrakis (triphenylphosphine) palladium (176 mg, 0.15 mmol), and potassium phosphate (967 mg, 4.56 mmol) were added in 1,4-dioxane (20 mL) and water (2 mL), after replaced with nitrogen gas three times, the reaction was refluxed at 100 °C for 12 h. The reaction solution was cooled down to room temperature, and filtered through diatomaceous earth, concentrated under reduced pressure to remove the solvent, extracted with DCM/$H_2O$ three times, and the organic layers were combined, washed with saturated saline solution, dried over anhydrous sodium sulfate, concentrated under reduced pressure, and purified by column chromatography (DCM/ MEOH=40/1) to obtain compound **96g** (550 mg), white solid.
MS (ESI) *m/z* 508 $[M + H]^+$.

**Step 7 *N- (4-* (4-amino-5- (3-fluoro-4- ((methyl (oxo) (phenyl) - λ$^6$- sulfaneylidene) amino) phenyl) -7- (methyl-*d*$_3$) -7*H*-pyrrolo [2,3-*d*] pyrimidin-6-yl) -3-fluorophenyl) methacrylamide 96**

Compound **96g** (550 mg, 1.08 mmol) was dissolved with anhydrous DMF (10 mL), methacryloyl chloride (136 mg, 1.30 mmol) was added at 0 °C, and the reaction was carried out for 1 h, and then quenched with water, extracted with dichloromethane, and the organic layers were combined, washed with saturated salt once, and the organic phase was dried over anhydrous sodium sulfate, concentrated under reduced pressure, and purified by column chromatography (DCM/MEQOH=20/1) to obtain compound **96** (25 mg), white solid.

MS (ESI) *m/z* 576 [M + H]$^+$.

$^1$H NMR (400 MHz, $CDCl_3$) $\delta$ 8.34 (s, 1H), 8.00 (dd, *J* = 7.2, 1.7 Hz, 2H), 7.64 (d, *J* = 7.3 Hz, 2H), 7.60 - 7.54 (m, 3H), 7.05 (d, *J* = 8.3 *Hz,* 1H), 6.98 (d, *J* = 8.0 Hz, 1H), 6.87 (d, *J* = 11.3 Hz, 1H), 6.77 (s, 1H), 5.82 (s, 1H), 5.53 (d, *J* = 1.6 Hz, 1H), 4.95 (s, 2H), 3.29 (s, 3H), 2.09 (d, *J* = 0.6 Hz, 3H).

**Example 97 *N-* (4- (4-amino-5- (3-fluoro-4- ((1-oxo-1 λ$^6$-thiophene-1-ylidene) amino) phenyl) -7- (methyl-*d*$_3$) -7*H*-pyrrole [2,3-*d*] pyrimidin-6-yl) -3-fluorophenyl) methacrylamide**

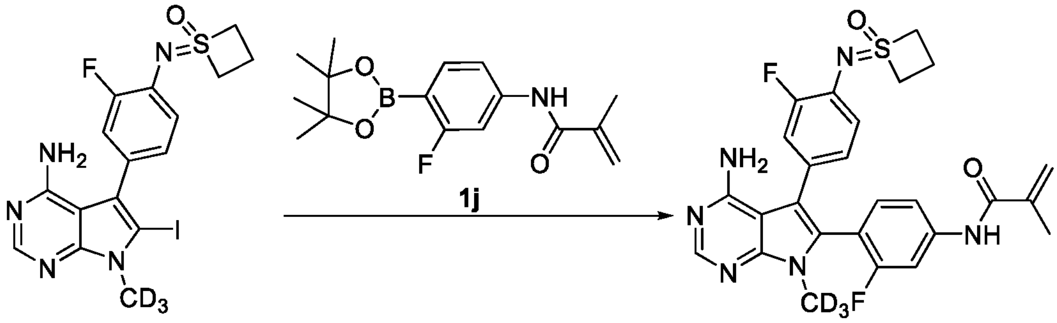


**84b** **97**

Compound 97 was synthesized with reference to the similar synthetic route and steps of compound **12,** wherein, compound **12a** was replaced with compound **84b,** compound 12b was replaced with compound **1j.**

MS (ESI) *m/z* 526.10 [M + H]$^+$.

$^1$H NMR (400 MHz, $CDCl_3$) $\delta$ 8.37 (s, 1H), 7.73 (dd, *J* = 11.5, 2.0 Hz, 1H), 7.58 (s, 2H), 7.07-6.92 (m, 3H), 6.94 (d, *J* = 9.6 Hz, 1H), 5.81 (s, 1H), 5.52 (s, *1H),* 5.00 (s, 2H), 4.42 - 4.21 (m, 4H), 2.41 - 2.28 (m, 2H), 2.07 (s, 3H).

**Example 98 *N-* (4- (4-amino-7- (methyl-*d*$_3$) -5- (4- ((1-oxo-1 λ$^6$-thiophene-1-ylidene) amino) phenyl) -7*H*-pyrrolo [2,3-*d*] pyrimidin-6-yl) -3-fluorophenyl) methacrylamide**

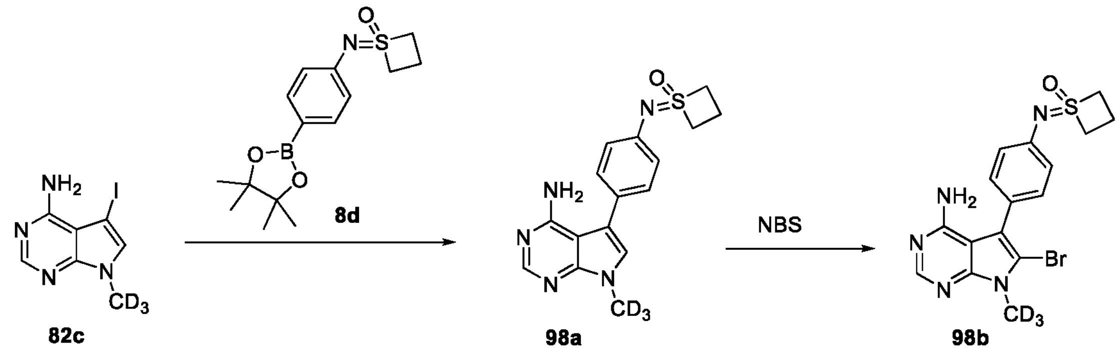


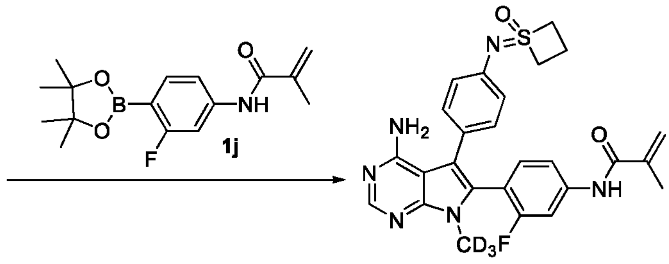

98

**Step 1 1- (4- (4-amino-7- (methyl-*d*$_3$) -7*H*-pyrrolo [2,3-*d*] pyrimidin-5-ylphenyl) imino) -1 λ$^6$-thiophene-1-oxide 98a**

Compound **82c** (2.0 g, 7.22 mmol) was dissolved in 1,4-dioxane (20 mL) and $H_2O$ (4 mL), compound **8d** (2.44 g, 7.94 mmol), potassium phosphate (4.70 g, 22.0 mmol), and tetratriphenylphosphine palladium (0.80 g, 0.70 mmol) were added, and the reaction was carried out at 90 °C for 14 h under $N_2$ protection. After TLC monitored the completion of the reaction, it was extracted with ethyl acetate (50 mL × 3), and the organic phases were combined and concentrated under reduced pressure to obtain a crude product. The crude product was purified by column chromatography (DCM/MeOH=10/1) to obtain compound **98a** (1300 mg)

MS (ESI) *m/z* 331.10 $[M + H]^+$.

**Step 2 1- (4- (4-amino-6-bromo-7- (methyl-*d*$_3$) -7*H*-pyrrolo [2,3-*d*] pyrimidin-5-yl) phenyl) imino) -1 λ$^6$-thiophene-1-oxide 98b**

Compound **98a** (0.30g, 0.91 mmol) was dissolved in DMF (5 mL), and it was cooled down to 0 °C, NBS (0.18g, 1.00 mmol) was added, and the reaction was carried out at room temperature for 1 h. After TLC monitored the completion of the reaction, it was extracted with dichloromethane (30 mL × 3), and the organic phases were combined and concentrated under reduced pressure to obtain crude product compound **98b** (0.20 g), which was directly used in the next step.

MS (ESI) *m/z* 409.10 $[M + H]^+$.

**Step 3 *N*- (4- (4-amino-7- (methyl-*d*$_3$) -5- (4- ((1-oxo-1 λ$^6$-thiophene-1-ylidene) amino) phenyl) -7*H*-pyrrolo [2,3-*d*] pyrimidin-6-yl) -3-fluorophenyl) methacrylamide 98**

Compound **98b** (0.20 g, 0.49 mmol) was dissolved in 1,4-dioxane (5 mL) and $H_2O$ (1 mL), compound **1j** (0.17 g, 0.54 mmol), potassium phosphate (320 mg, 1.50 mmol), and tetrakis (triphenylphosphine) palladium (0.036 g, 0.05 mmol) were added, and the reaction was carried out at 90 °C for 14 h under $N_2$ protection. After TLC monitored the completion of the reaction, it was extracted with ethyl acetate (50 mL × 3), and the organic phases were combined and concentrated under reduced pressure to obtain a crude product. The crude product was purified by column chromatography (DCM/MeOH=10/1) to obtain compound **98** (22.0 mg).

MS (ESI) *m/z* 508.10 $[M + H]^+$.

$^1H$ NMR (400 MHz, $CDCl_3$) δ 8.37 (d, *J* = 7.8 Hz, 1H), 7.73 (dd, *J* = 11.5, *2.0* Hz, 1H), 7.61 (s, 1H), 7.23 - 7.04 (m, 4H), 6.97 (d, *J* = 8.1 Hz, 2H), 5.83 (s, 1H), 5.54 (d, *J* = 1.6 Hz, 1H), 5.10 (s, 2H), 4.24 (dd, *J* = 9.3, 7.2 Hz, 4H), 2.55 - 2.30 (m, 2H), 2.09 (d, *J* = 1.3 Hz, 3H).

**Example 99 *N*- (4- (4-amino-5- (3-fluoro-4- ((1-oxo-1 λ$^6$-thiophene-1-ylidene) amino) phenyl) -7-methyl-7*H*-pyrrolo [2,3-*d*] pyrimidin-6-yl) -3-methylphenyl) methacrylamide**

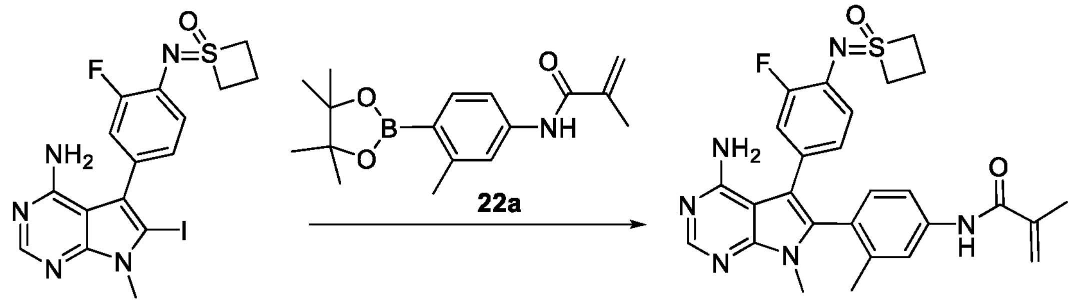

**22a**

**70b** **99**

Compound **99** was synthesized with reference to the similar synthetic route and steps of compound **12,** wherein, compound **12a** was replaced with compound **70b,** compound **12b** was replaced with compound **22a.**
MS (ESI) *m/z* 519.2 [M + H]$^+$.
$^1$H NMR (400 MHz, $CDCl_3$) $\delta$ 8.30 (s, 1H), 7.43 (d, *J* = 2.8 Hz, 2H), 7.35 *(dd, J* = 8.2, 2.3 Hz, 1H), 7.10 (d, *J* = 8.3 Hz, 1H), 6.91 *(d, J* = 8.3 Hz, 1H), 6.85 - 6.77 (m, 2H), 5.73 (s, 1H), 5.42 (d, *J* = 1.6 Hz, 1H), 4.95 (s, 2H), 4.20 (q, *J* = 8.4, 8.0 Hz, 4H), 3.45 (s, *3H),* 2.26 (t, *J* = 8.3 Hz, 2H), 2.02 - 1.90 (m, 6H).

**Example 100 *N*- (4- (4-amino-5- (4- (diethyl (oxo) -1 λ$^6$- sulfaneylidene) amino) -3-fluorophenyl) -7-methyl-7*H*-pyrrolo [2,3-*d*] pyrimidin-6-yl) -3-fluorophenyl) methacrylamide**

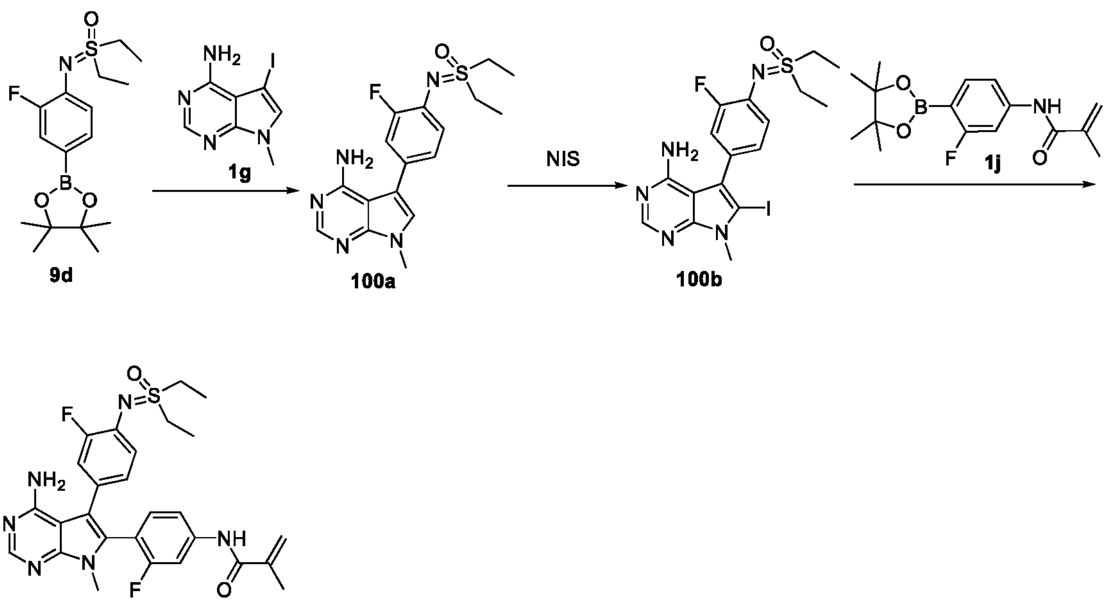


**9d** **1g** **100a** **100b** **1j** **100**

Compound **100** was synthesized with reference to the similar synthetic route and steps of compound **85,** wherein, compound **85a** was replaced with compound **9d.**
MS (ESI) *m/z* 538.2 [M + H]$^+$.
$^1$H NMR (400 MHz, $CDCl_3$) $\delta$ 8.30 (s, 1H), 7.64 (dd, *J* = 11.6, 2.1 Hz, 1H), 7.50 *(s,* 1H), 7.13 (t, *J* = 8.5 Hz, 1H), 7.06 - 6.96 (m, 2H), 6.85 (d, *J* = 10.4 Hz, 2H), 5.74 (s, 1H), 5.45 (q, *J* = 1.6 Hz, 1H), 4.95 (s, 2H), 3.58 (d, *J* = 1.1 Hz, 3H), 3.15 (tq, *J* = *14.3,* 7.2 Hz, 4H), 1.97 (s, 3H), 1.37 (t, *J* = 7.5 Hz, 6H).

**Example 101 *N*- (4- (4-amino-5- (4- (diethyl (oxo) -1 λ$^6$- sulfaneylidene) amino) -3-fluorophenyl) -7-methyl-7*H*-pyrrolo [2,3-*d*] pyrimidin-6-yl) -3-methylphenyl) methacrylamide**

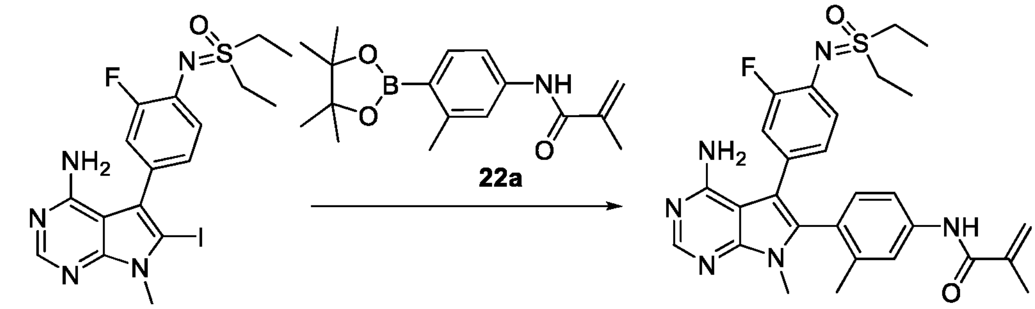


**100b** **101**

Compound **101** was synthesized with reference to the similar synthetic route and steps of compound **12,** wherein, compound **12a** was replaced with compound **100b,** compound **12b** was replaced with compound **22a.**

MS (ESI) *m*/*z* 535.3 [M + H]$^+$.

$^1$H NMR (400 MHz, $CDCl_3$) δ 8.36 (s, 1H), 7.50 (d, *J* = 2.5 Hz, 2H), 7.41 *(dd, J* = 8.1, 2.2 Hz, 1H), 7.17 (dt, *J* = 8.4, 4.3 Hz, 2H), *6.85* (d, *J* = 9.5 Hz, 2H), 5.80 (s, 1H), 5.49 (d, *J* = 1.8 Hz, *1H),* 5.09 (s, 2H), 3.52 (s, 3H), 3.20 (m, 4H), 2.08 (t, *J* = 1.2 Hz, 3H), 2.00 (s, *3H),* 1.43 (t, *J*= *7.5* Hz, 6H).

**Example 102 *N*- (4- (4-amino-5- (4- (diethyl (oxo) -1 λ$^6$- sulfaneylidene) amino) phenyl) -7-methyl-7*H*-pyrrolo [2,3-*d*] pyrimidin-6-yl) -3-chlorophenyl) methacrylamide**

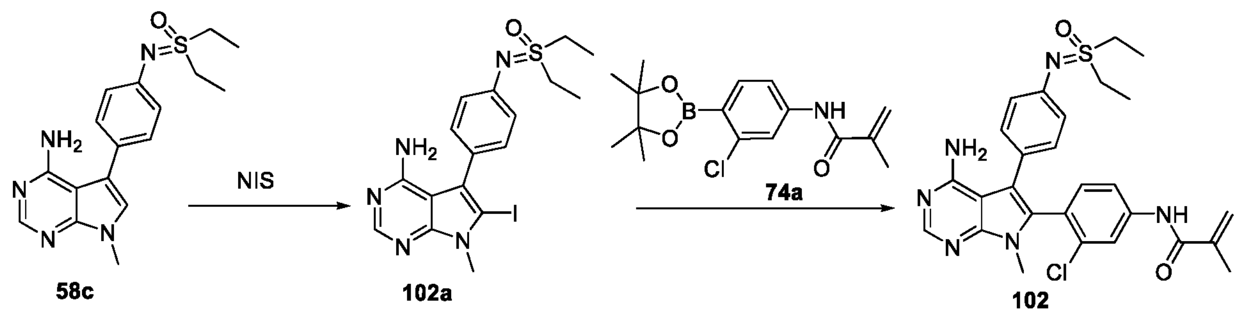


**58c** **102a** **102**

Compound **102** was synthesized with reference to the similar synthetic route and steps of compound 77, wherein, compound **12a** was replaced with compound **58c,** compound **1j** was replaced with compound **74a.**

MS (ESI) *m*/*z* 537.3 [M + H]$^+$.

$^1$H NMR (400 MHz, $CDCl_3$) δ 8.39 (s, 1H), 7.89 (d, *J* = 2.2 Hz, 1H), 7.61 (s, *1H),* 7.37 (dd, *J* = 8.3, 2.2 Hz, 1H), 7.14 (m, 3H), 7.03 (d, *J* = *8.0* Hz, 2H), 5.83 (s, 1H), 5.54 (s, 1H), 5.08 (s, 2H), 3.61 (s, 3H), 3.31 - 3.10 (m, 4H), 2.10 (s, 3H), 1.43 (t, *J* = 7.4 Hz, 6H).

**Example 103 *N*- (4- (4-amino-5- (4- (diethyl (oxo) -1 λ$^6$- sulfaneylidene) amino) phenyl) -7- (2,2,2-trifluoroethyl) -7*H*-pyrrolo [2,3-*d*] pyrimidin-6-yl) -3-fluorophenyl) methacrylamide**

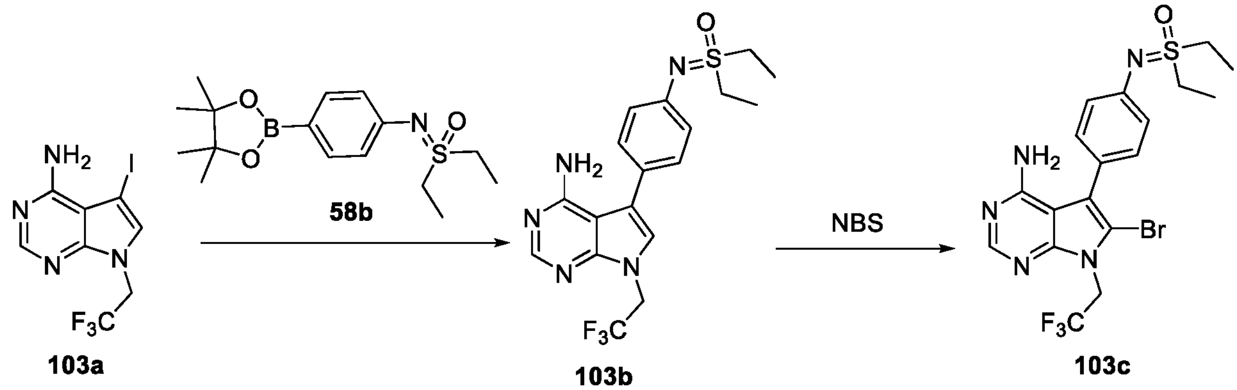


103a 103b 103c

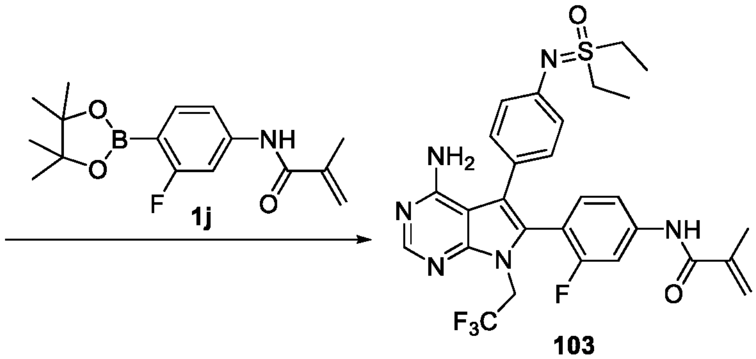


103

Compound **103** was synthesized with reference to the similar synthetic route and steps of compound **85,** wherein, in step 1, compound **1g** was replaced with compound **103a,** compound **58d** was replaced with compound **85a.**

MS (ESI) *m/z* 589.3 [M + H]$^+$.

$^1$H NMR (400 MHz, $CDCl_3$) $\delta$ 8.41 (s, 1H), 7.77 (d, *J* = 11.5 Hz, 1H), 7.62 *(s,* 1H), 7.23 - 6.94 (m, 6H), 5.83 (s, 1H), 5.55 (s, 1H), 5.11 (s, 2H), 3.24 (m, 4H), 2.10 (s, 3H), 1.65 (s, 2H), 1.44 (t, *J* = 7.5 Hz, 6H).

**Example 104 *N*- (4- (4-amino-5- (3-chloro-4- ((1-oxo-1 λ$^6$-thiophene-1-ylidene) amino) phenyl) -7-methyl-7*H*-pyrrolo [2,3-*d*] pyrimidin-6-yl) -3-fluorophenyl) methacrylamide**

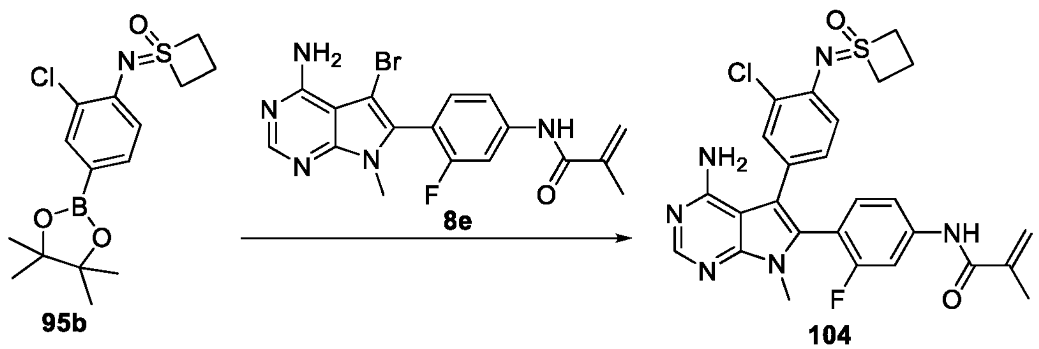


95b 104

Compound **104** was synthesized with reference to the similar synthetic route and steps of compound **31,** wherein, compound **24b** was replaced with compound **95b,** compound **9e** was replaced with compound **8e.**

MS (ESI) *m/z* 539.1 [M + H]$^+$.

$^1$H NMR (400 MHz, $CDCl_3$) $\delta$ 8.37 (s, 1H), 7.73 (dd, *J* = 11.6, 2.0 Hz, 1H), 7.58 *(s,* 1H), 7.29 (s, 3H), 7.14 - 6.97 (m, 3H), 5.81 (s, 1H), 5.52 (q, *J* = 1.6 Hz, 1H), 5.00 (s, 1H), 4.42 - 4.15 (m, 3H), 2.47 - 2.28 (m, 3H), 1.59 (s, 6H).

**Example 105 *N*- (4- (4-amino-7-methyl-5- (4- (4-oxo-1,4 λ⁶-oxathiohexane-4-ylidene) amino) phenyl) -7*H*-pyrrolo [2,3-*d*] pyrimidin-6-yl) -3-fluorophenyl) methacrylamide**

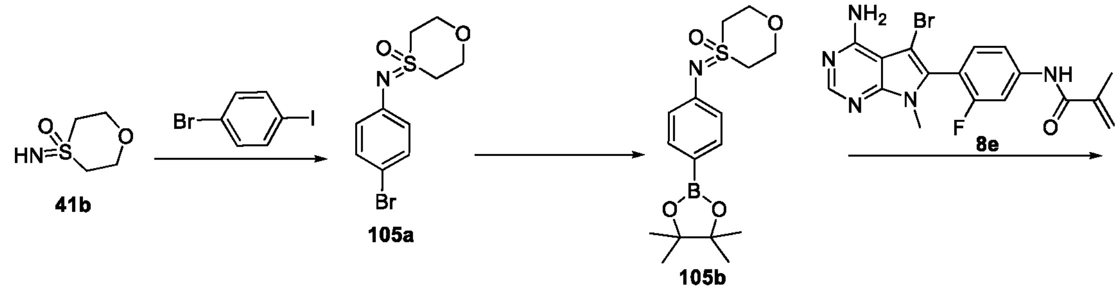

41b 105a 105b 8e

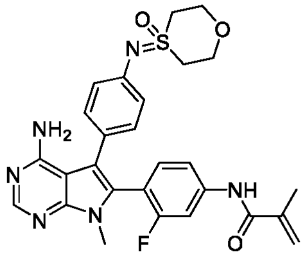

105

Compound **105** was synthesized with reference to the similar synthetic route and steps of compound **8,** wherein, compound **8b** was replaced with compound **41b.**

MS (ESI) *m/z* 535.2 $[M + H]^+$.

$^1H$ NMR (400 MHz, $CDCl_3$) δ 8.39 (s, 1H), 7.71 (dd, *J* = 11.5, 2.0 Hz, 1H), 7.59 *(s,* 1H), 7.19 - 7.05 (m, 6H), 5.83 (s, 1H), 5.55 (q, *J* = 1.5 Hz, 1H), *5.04* (s, 2H), 4.22 (dt, *J* = 12.8, 4.3 Hz, 2H), 4.12 (m, 2H), 3.68 (d, *J* = 1.1 Hz, 4H), 3.48 - 3.36 (m, 3H), 3.30 (m, 3H).

**Example 106 *N*- (4- (4-amino-5- (4- ((3,3-dimethyl-1-oxo-1 λ⁶- thiocyclobutan-1- ylidene) amino) -3-fluorophenyl) -7-methyl-7*H*-pyrrolo [2,3-*d*] pyrimidin-6-yl) -3- fluorophenyl) methacrylamide**

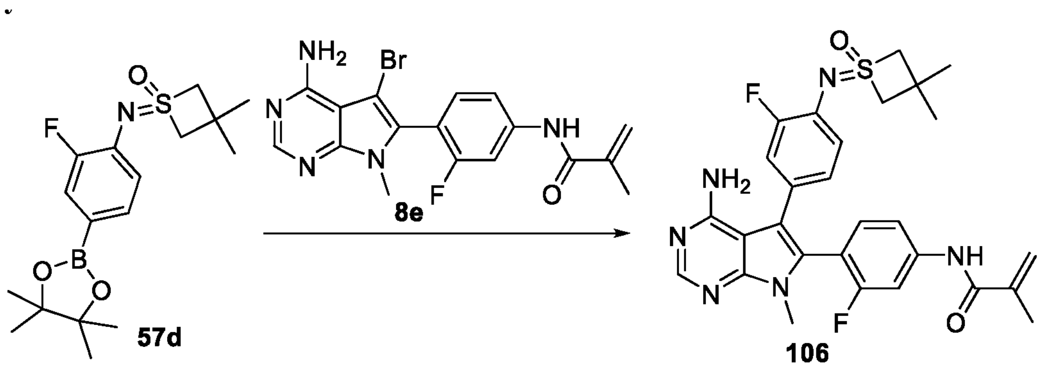

57d 8e 106

Compound **106** was synthesized with reference to the similar synthetic route and steps of compound **8,** wherein, compound **8d** was replaced with compound **57d.**

MS (ESI) *m/z* 551.1 $[M + H]^+$.

$^1H$ NMR (400 MHz, $CDCl_3$) δ 8.39 (s, 1H), 7.76 (dd, *J* = 11.6, 2.1 Hz, 1H), 7.60 *(s,* 1H), 7.17 - 7.02 (m, 3H), 6.96 (d, *J* = 9.9 Hz, 2H), 5.84 (s, 1H), *5.55 (d, J* = 1.8 Hz, 1H), 5.04 (s, 2H), 4.20 - 4.01 (m, 4H), 3.68 (d, *J* = 1.1 Hz, 2H), 2.10 (s, 2H), 1.56 (m, 4H), 1.30 (d, *J* = 9.4 Hz, 4H).

**Example 107 *N*- (4- (4-amino-5- (4- (dimethyl (oxo) -1 λ$^6$- sulfaneylidene) amino) phenyl) -7-methyl-7*H*-pyrrolo [2,3-*d*] pyrimidin-6-yl) -3-fluorophenyl) methacrylamide**

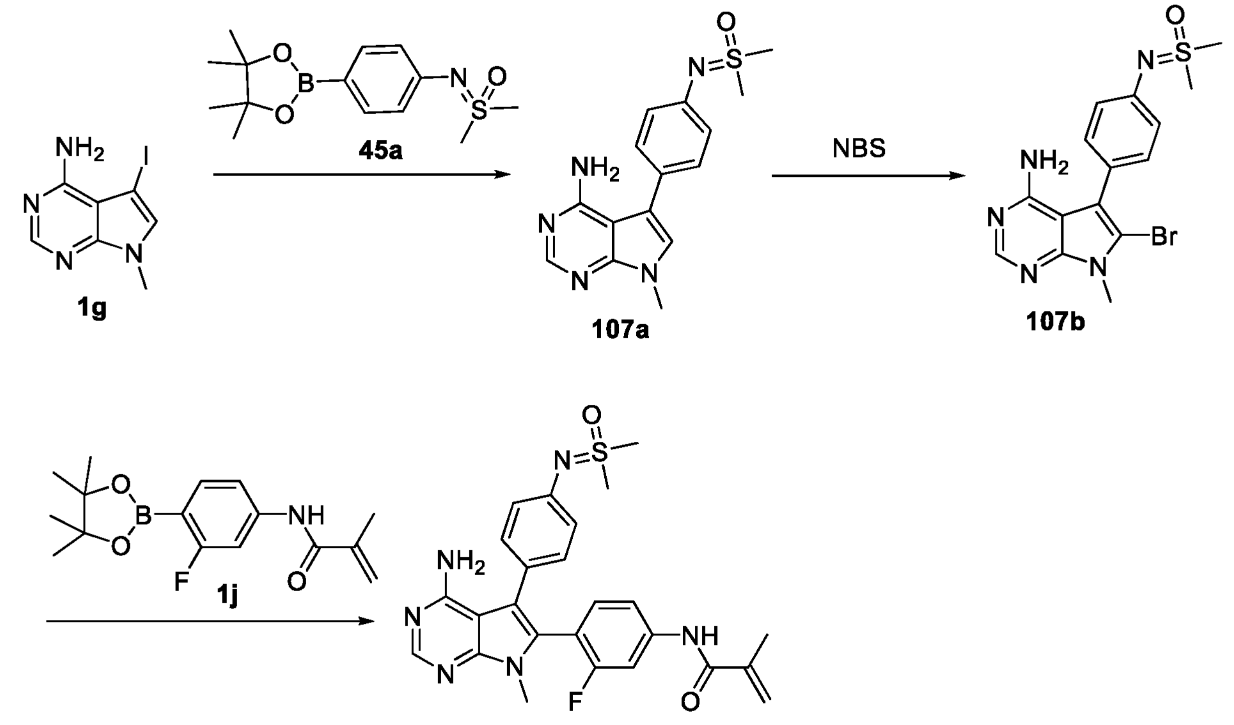


**45a**

**1g**

**107a**

**107b**

**1j**

**107**

It was synthesized with reference to the similar synthetic route and steps of compound **85.** MS (ESI) *m/z* 493.2 [M + H]$^+$.

$^1$H NMR (400 MHz, $CDCl_3$) δ 8.29 (s, 1H), 7.63 (d, *J* = 11.7 Hz, 1H), 7.49 (s, *1H),* 7.11 - 6.85 (m, 5H), 5.73 (s, 1H), 5.45 (s, 1H), 5.28 (s, 1H), 4.96 *(s,* 2H), 3.59 (s, 3H), 2.00 (s, 3H), 1.19 (s, 6H).

**Example 108 *N*- (4- (4-amino-5- (4- ((3,3-dimethyl-1-oxo-1 λ$^6$-thiophene-1-ylidene) amino) phenyl) -7- (methyl-*d*$_3$) -7*H*-pyrrolo [2,3-*d*] pyrimidin-6-yl) -3-fluorophenyl) methacrylamide**

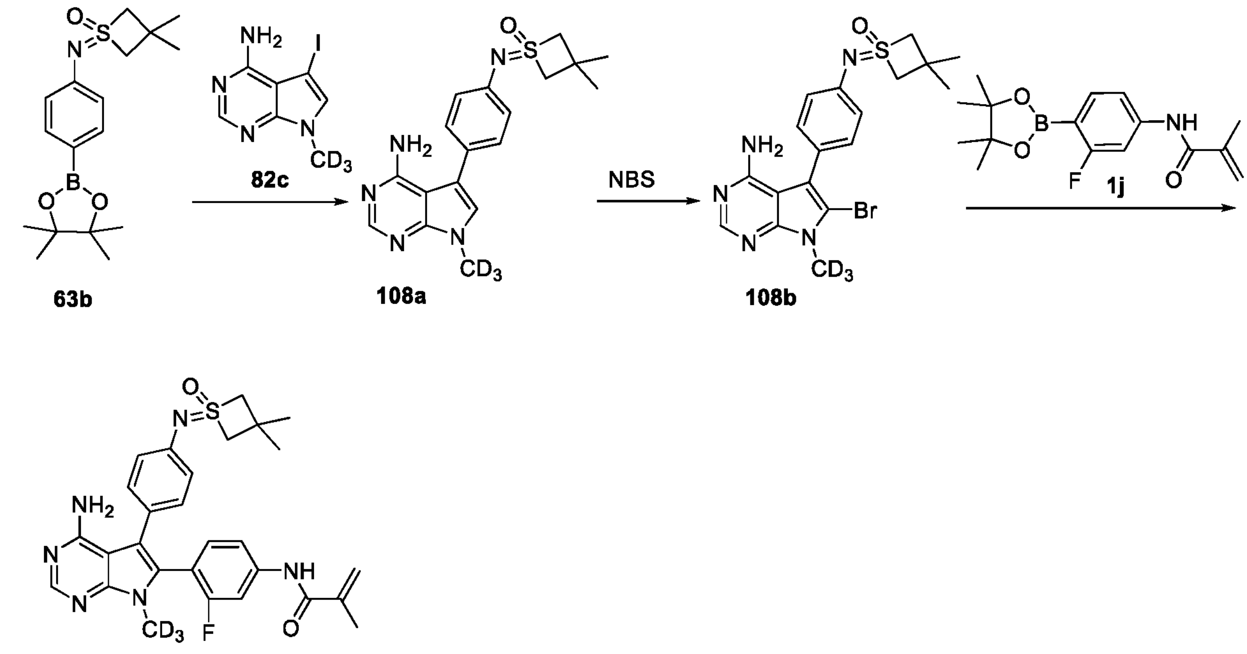


63b 108a 108b

108

Compound **108** was synthesized with reference to the similar synthetic route and steps of compound **85,** wherein, in step 1, compound **85a** was replaced with compound **63b,** compound **1g** was replaced with compound **82c.**

MS (ESI) *m/z* 536.9 [M + H]$^+$.

$^1$H NMR (400 MHz, $CDCl_3$) $\delta$ 8.36 (s, 1H), 7.71 (dd, *J* = 11.8, 1.8 Hz, 1H), *7.56* (s, 1H), 7.17 - 7.03 (m, 4H), 6.94 (d, *J* = 8.1 Hz, 2H), 5.80 *(s,* 1H), 5.52 (d, *J* = 1.6 Hz, 1H), 5.02 (s, 2H), 4.05 - 3.90 (m, 4H), 2.05 (d, *J* = 10.4 Hz, 3H), 1.54 (s, 3H), 1.52 (s, 3H).

**Example 109 *N*- (4- (4-amino-5- (4- (diethyl (oxo) - λ$^6$- sulfaneylidene) amino) phenyl) -7- (methyl-*d*$_3$) -7*H*-pyrrolo [2,3-*d*] pyrimidin-6-yl) -3-fluorophenyl) methacrylamide**

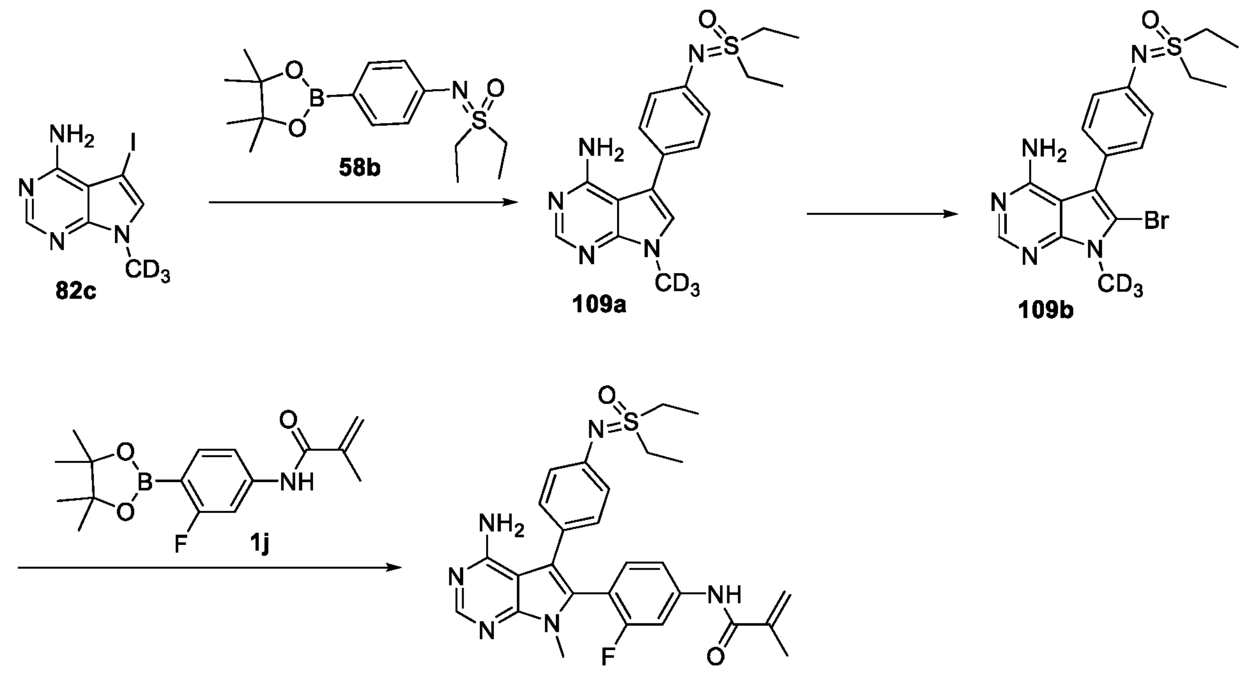

82c 58b 109a 109b 1j

109

Compound **109** was synthesized with reference to the similar synthetic route and steps of compound **58,** wherein, in step 1, compound **1g** was replaced with compound **82c.**

MS (ESI) *m/z* 542.10 [M + H]$^+$.

$^1$H NMR (400 MHz, $CDCl_3$) $\delta$ 8.27 (s, 1H), 7.94-7.82 (m, 1H), 7.57-7.42 (m, 2H), 7.38-7.24 (m, *3H),* 5.79 (s, 1H), 5.72 (s, 1H), 5.00 (s, 2H), 2.61 (q, 4H), 2.07 (s, 3H), 1.22 (t, 6H).

**Example 110 *N-* (4- (4-amino-7-methyl-5- (4- (1-tetrahydro-1λ$^6$-thiophene-1- ylidene) amino) phenyl) -7*H*-pyrrolo [2,3-*d*] pyrimidin-6-yl) -3-fluorophenyl) acrylamide**

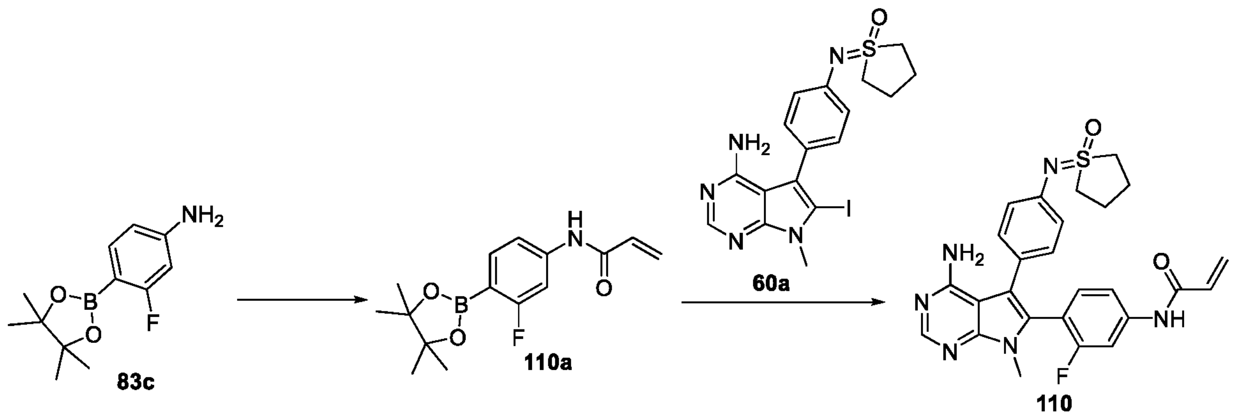


**Step 1 *N-* (3-fluoro-4- (4,4,5,5-tetramethyl-1,3,2-dioxaborolan-2-yl) phenyl) acrylamide 110a**

Compound **83c** (100 mg, 0.42 mmol), acrylic acid (45 mg, 0.63 mmol), and triethylamine (127 mg, 1.26 mmol) were dissolved in THF (5 mL), and then T3P (50% DMF solution) (0.63 mmol) was added, and the reaction was carried out at room temperature for 2 h, then concentrated under reduced pressure to remove the solvent, extracted with DCM/$H_2O$ three times, and the organic layers were combined, and washed once with saturated sodium chloride, and the organic phase was dried over anhydrous sodium sulfate, concentrated under reduced pressure, and purified by column chromatography (PE/EA=5/1) to obtain compound 110a (80 mg), yellow solid.

MS (ESI) *m/z* 292.02 [M + H]$^+$.

**Step 2 *N-* (4- (4-amino-7-methyl-5- (4- (1-tetrahydro-1λ$^6$-thiophene-1-ylidene) amino) phenyl) -7*H*-pyrrolo [2,3-*d*] pyrimidin-6-yl) -3-fluorophenyl) acrylamide 110**

Compound **110a** (80 mg, 0.17 mmol), compound **60a** (59 mg, 0.20 mmol), Pd(dppf)$Cl_2$ (25 mg, 0.034 mmol), and potassium phosphate (108 mg, 0.51 mmol) were dissolved in 1,4-dioxane (2 mL) and water (0.5 mL), after replaced with nitrogen gas three times, the reaction was refluxed at 90 °C for 6 h. The reaction solution was cooled down to room temperature, filtered through diatomaceous earth, concentrated under reduced pressure to remove the solvent, extracted with DCM/$H_2O$ three times, and the organic layers were combined and washed with saturated saline solution, and the organic phase was dried over anhydrous sodium sulfate, concentrated under reduced pressure, and purified by column chromatography (DCM/MEOH=40/1) to obtain compound **110** (40 mg), yellow solid.

MS (ESI) *m/z* 505.03 [M + H]$^+$.

$^1$H NMR (400 MHz, $CDCl_3$) $\delta$ 8.28 (s, 1H), 7.66 (d, *J* = 11.3 Hz, 1H), 7.48 (s, *1H),* 7.08 - 7.01 (m, 3H), 6.99 (d, *J* = 7.8 Hz, 1H), 6.91 (d, *J* = 8.1 Hz, 2H), 6.39 (d, *J* = 16.8 Hz, 1H), 6.18 (dd, *J* = 16.9, 10.2 Hz, 1H), 5.75 (d, *J* = 10.2 Hz, 1H), 4.97 (s, 2H), 3.58 (s, 3H), 3.36 (dd, *J* = 13.0, 6.6 Hz, 2H), 3.12 (dt, *J* = 12.7, 6.6 Hz, 2H), 2.33 - 2.13 (m, 4H).

**Example 111 *N-* (4- (4-amino-7-methyl-5- (4- (1-tetrahydro-1λ$^6$-thiophene-1 -ylidene) amino) phenyl) -7*H*-pyrrolo [2,3-*d*] pyrimidin-6-yl) -3-fluorophenyl) -2-fluoroacrylamide**

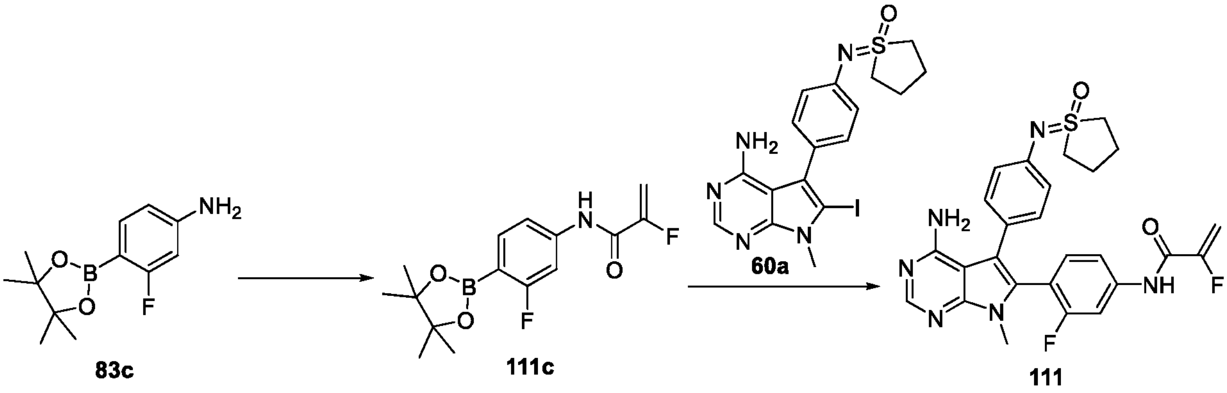


83c 111c 111

**Step 1 2-fluoro-N- (3-fluoro-4- (4,4,5,5-tetramethyl-1,3,2-dioxaborolan-2-yl) phenyl) acrylamide 111c**

Compound **83c** (100 mg, 0.42 mmol), 2-fluoroacrylic acid (57 mg, 0.63 mmol), and triethylamine (127 mg, 1.26 mmol) was dissolved in THF (5 mL), and then T3P (50% DMF solution) (0.63 mmol) was added, and the reaction was carried out at room temperature for 2 h, concentrated under reduced pressure to remove the solvent, extracted with DCM/$H_2O$ three times, and the organic layers were combined, and washed with saturated salt twice, and the organic phase was dried over anhydrous sodium sulfate, concentrated under reduced pressure, and purified by column chromatography (PE/EA=5/1) to obtain compound **111c** (85 mg), yellow solid.

MS (ESI) *m/z* 310.02 $[M + H]^+$.

**Step 2 *N*- (4- (4-amino-7-methyl-5- (4- (1-hydroperoxide-1 λ$^6$-thiophene-1-ylidene) amino) phenyl) -7*H*-pyrrolo [2,3-*d*] pyrimidin-6-yl) -3-fluorophenyl) -2- fluoroacrylamide 111**

Compound **111c** (30 mg, 0.097 mmol), compound **60a** (56 mg, 0.12 mmol), Pd(dppf)$Cl_2$ (14 mg, 0.019 mmol), and potassium phosphate (61 mg, 0.29 mmol) were dissolved in 1,4-dioxane (2 mL) and water (0.5 mL), after replaced with nitrogen gas three times, the reaction was refluxed at 90 °C for 6 h. The reaction solution was cooled down to room temperature, filtered through diatomaceous earth, concentrated under reduced pressure to remove the solvent, extracted with DCM/$H_2O$ three times, and the organic layers were combined, washed with saturated saline solution, dried over anhydrous sodium sulfate, concentrated under reduced pressure, purified by column chromatography (DCM/MEOH =40/1) to obtain compound **111** (4 mg), white solid.

MS (ESI) *m/z* 523.10 $[M + H]^+$.

$^1$H NMR (400 MHz, $CDCl_3$) $\delta$ 8.29 (s, 1H), 7.93 (d, *J* = 5.4 Hz, 1H), 7.70 - 7.59 (m, 1H), 7.12 - 6.97 (m, 4H), 6.91 (d, *J* = 8.1 Hz, *2H),* 5.79 (dd, *J* = 47.8, 3.6 Hz, 1H), 5.23 (dd, *J* = 15.1, 3.5 Hz, 1H), 4.99 (s, 2H), 3.59 (s, 3H), 3.35 (dt, *J* = 12.6, 6.4 Hz, 2H), 3.12 (dt, *J* = 12.9, 6.6 Hz, 2H), 2.38 - 2.11 (m, *4H).*

**Example 112 *N*- (4- (4-amino-7-methyl-5- (4- ((1-tetrahydro-1λ$^6$-thiophene-1-ylidene) amino) phenyl) -7*H*-pyrrolo [2,3-*d*] pyrimidin-6-yl) -3-fluorophenyl) but-2-ynamide**

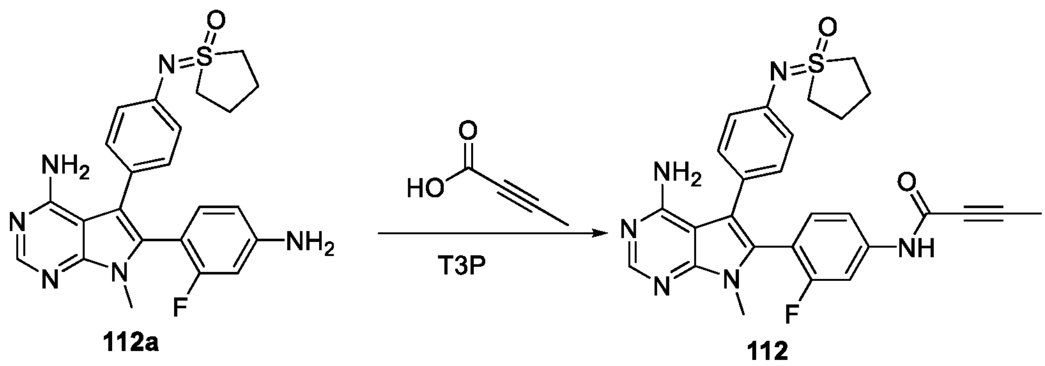


112a 112

Compound **112a** (100 mg, 0.22 mmol), 2-butyne acid (27 mg, 0.33 mmol), and triethylamine (67 mg, 0.66 mmol) were dissolved in THF (5 mL), and then T3P (50% DMF solution) (0.33 mmol) was added, and the reaction was carried out

at room temperature for 2 h, then concentrated under reduced pressure to remove the solvent, extracted with DCM/$H_2O$ three times, and the organic layers were combined, washed with saturated sodium chloride once, dried over anhydrous sodium sulfate, concentrated under reduced pressure, and purified by column chromatography (DCM/MeOH=40/1) to obtain compound **112** (10 mg), white solid.

MS (ESI) *m/z* 517.20 $[M + H]^+$.

$^1H$ NMR (400 MHz, $CDCl_3$) $\delta$ 8.28 (s, 1H), 7.53 (d, *J* = 10.3 Hz, 2H), 7.04 (d, *J* = 7.8 Hz, 2H), 7.00 - 6.95 (m, 2H), 6.91 (d, *J* = 8.0 Hz, *2H),* 4.97 (s, 2H), 3.59 - 3.56 (m, 3H), 3.35 (dt, *J* = 13.2, 6.5 Hz, 2H), 3.13 (dt, *J* = 12.9, 6.6 Hz, 2H), 2.32 - 2.09 (m, 5H).

**Example 113 *N-* (4- (4-amino-7-methyl-5- (4- (1-tetrahydro-1$\lambda^6$-thiophene-1-ylidene) amino) phenyl-3,5-d2)-7*H*-pyrrolo [2,3-*d*] pyrimidin-6-yl) -3-fluorophenyl) methacrylamide**

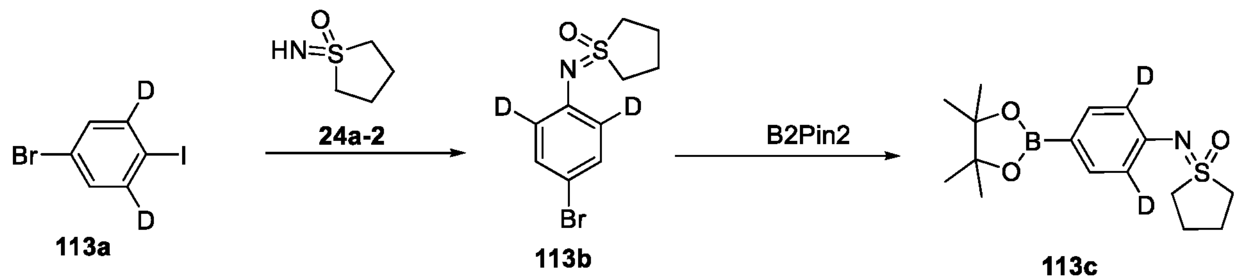


113a 113b 113c

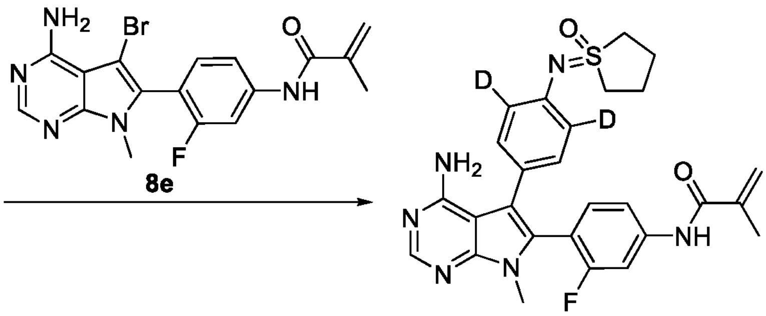


113

Compound **113** was synthesized with reference to the similar synthetic route and steps of compound **105,** wherein, in step 1, p-bromoiodobenzene was replaced with compound **113a.**

MS (ESI) *m/z* 521.2 $[M + H]^+$

$^1H$ NMR (400 MHz, MeOD-$d_4$) $\delta$ 8.18 (s, 1H), 7.71 (dd, *J* = 12.1, 2.0 Hz, 1H), 7.35 (dd, *J* = 8.4, 2.0 Hz, 1H), 7.15 (t, *J* = 4.1 Hz, 3H), 5.81 (s, 1H), 5.54 (d, *J* = 1.1 Hz, 1H), 3.61 (s, 3H), 3.42 (m, 2H), 3.25 - 3.20 (m, 2H), 2.37 - 2.30 (m, 2H), 2.22 (m, 2H), 2.02 (s, 3H).

**Example 114 *N-* (4- (4-amino-7-methyl-5- (4- ((1-oxide tetrahydro-1 $\lambda^6$- thiophene-1-ylidene) amino) phenyl-2,3,5,6-d4) -7*H*-pyrrolo [2,3-*d*] pyrimidin-6-yl) -3-fluorophenyl) methacrylamide**

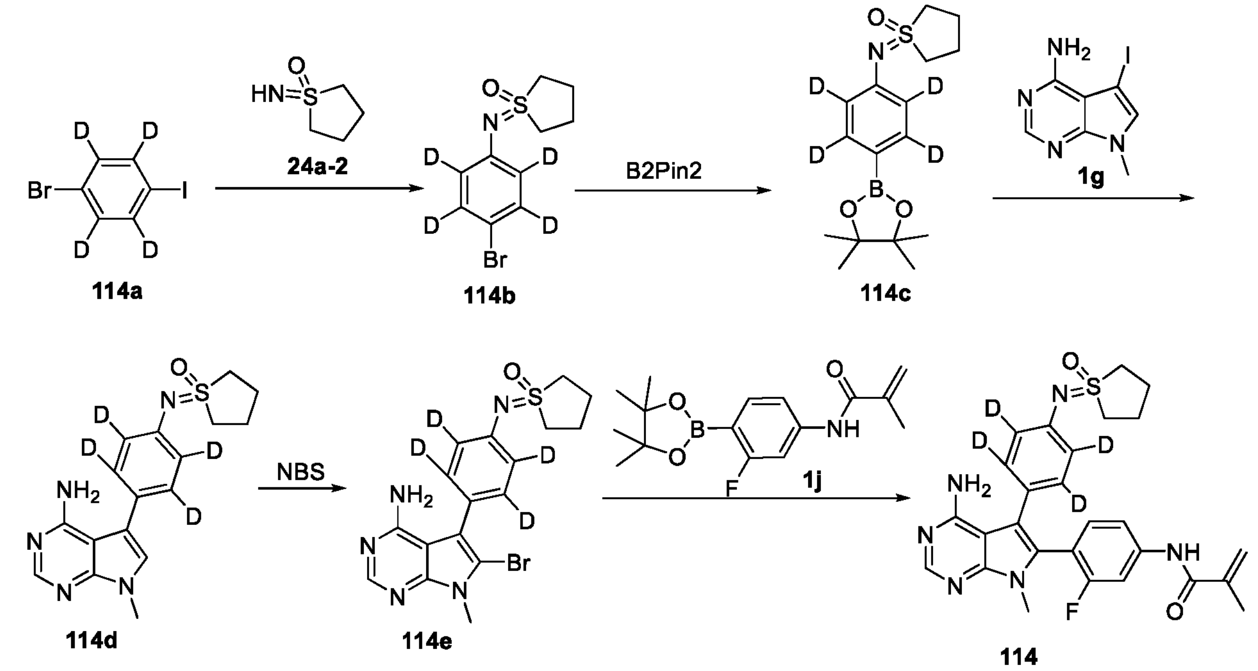


**Step 1 1- ((4-bromophenyl-2,3,5,6-d4) imino) tetrahydro-1*H*-1 λ⁶-thiophene-1-oxide 114b**

Compound **114a** was synthesized with reference to Organic Letters (2021), 23 (5), 1554-1560. To the reaction flask, compound **114a** (1.13 g, 3.95 mmol), anhydrous 1,4-dioxane (11 mL), compound **24a-2** (471 mg, 3.95 mmol), Xantphos (171 mg, 0.30 mmol), cesium carbonate (1.80 g, 5.53 mmol), and $Pd_2(dba)_3$ (90 mg, 0.099 mmol) were added in sequence, after replaced with nitrogen gas three times, the reaction was refluxed at 100 °C for 3 h. TLC showed the formation of new dots, the reaction was diluted with ethyl acetate and $H_2O$, filtered through diatomaceous earth, extracted with ethyl acetate, and the organic phase was washed with saturated saline solution, dried over anhydrous sodium sulfate, concentrated under reduced pressure, and purified by silica gel column chromatography (PE/EA=1/1) to obtain compound **114b** (775 mg), light yellow oil.

MS (ESI) *m/z* 278 $[M + H]^+$.

**Step 2 1- (4- (4,4,5,5-tetramethyl-1,3,2-dioxaborolan-2-yl) phenyl-2,3,5,6-d4) imino) tetrahydro-1*H*-1 λ⁶-thiophene-1-oxide 114c**

To the reaction flask, compound **114b** (725 mg, 2.61 mmol), anhydrous 1,4-dioxane (8 mL), bis(pinacolato) diboron (993 mg, 3.91 mmol), potassium acetate (768 mg, 7.83 mmol), and $Pd(dppf)Cl_2$ (189 mg, 0.261 mmol) were added in sequence, after replaced with nitrogen gas three times, the reaction was heated up to 90 °C for 4 h. TLC showed the formation of new dots, and after the reaction was completed, the reaction solution was cooled down to room temperature, diluted with ethyl acetate and $H_2O$, filtered through diatomaceous earth, extracted with ethyl acetate, and the organic phase was washed with saturated saline solution, dried over anhydrous sodium sulfate, concentrated under reduced pressure, and purified by silica gel column chromatography (PE/EA=1/1) to obtain compound **114c** (532 mg), yellow solid.

MS (ESI) *m/z* 326 $[M + H]^+$.

**Step 3 1- (4- (4-amino-7-methyl-7H-pyrrolo [2,3-d] pyrimidin-5-yl) phenyl- 2,3,5,6 -$d_4$) imino) tetrahydro-1*H*-1 λ⁶-thiophene-1-oxide 114d**

To a reaction flask, compound **114c** (467 mg, 1.44 mmol), 1,4-dioxane (5 mL), $H_2O$ (1 mL), compound **1g** (359 mg, 1.31 mmol), potassium phosphate (1.66 g, 7.8 mmol), and $Pd(PPh_3)_4$ (300 mg, 0.26 mmol) were added in sequence, after replaced with nitrogen gas three times, the reaction was carried at 90 °C for 5 h. TLC showed the formation of new dots, and after the reaction was completed, the reaction solution was cooled down to room temperature, diluted with dichloromethane and $H_2O$, filtered through diatomaceous earth, extracted with dichloromethane, and the organic phase was washed with saturated saline solution, dried over anhydrous sodium sulfate, concentrated under reduced pressure, and purified by silica gel column chromatography (DCM/MeOH=10/1) to obtain compound **114d** (144 mg), yellow solid.

MS (ESI) *m/z* 346 $[M + H]^+$.

**Step 4 1- (4- (4-amino-6-bromo-7-methyl-7*H*-pyrrolo [2,3-*d*] pyrimidin-5-yl) phenyl-2,3,5,6-$d_4$) imino) tetrahydro-1*H*-1 $\lambda^6$-thiophene-1-oxide 114e**

Compound **114d** (94 mg, 0.27 mmol) was dissolved in DMF (2 mL), and a solution of NBS in DMF (51 mg of NBS dissolved in 2 mL of DMF) was added dropwise in an ice water bath, and reacted under ice water bath for 1 h, and TLC showed the formation of new spots. The reaction was quenched with added saturated $NaHSO_3$ solution (10 mL) and $H_2O$ (20 mL), extracted with dichloromethane (20 mL × 3), and the organic phases were combined, washed with saturated saline solution, dried over anhydrous sodium sulfate, concentrated under reduced pressure, and purified by silica gel column chromatography to obtain **114e** (70 mg), yellow solid.

MS (ESI) *m/z* 425 $[M + H]^+$.

**Step *5 N-* (4- (4-amino-7-methyl-5- (4- ((1-oxide tetrahydro-1 $\lambda^6$-thiophene -1-ylidene) amino) phenyl-2,3,5,6-d4) -7H-pyrrolo [2,3-d] pyrimidin-6-yl) -3- fluorophenyl) methacrylamide 114**

To a reaction flask, compound **114c** (70 mg, 0.16 mmol), 1,4-dioxane (4 mL), water (1 mL), compound **1j** (60 mg, 0.20 mmol), potassium phosphate (104 mg, 0.49 mmol), and $Pd(PPh_3)_4$ (9 mg, 0.016 mmol) were added in sequence, after replaced with nitrogen gas three times, the reaction was refluxed at 90 °C for 5 h. TLC showed the formation of new spots, and after the reaction was completed, it was cooled down to room temperature, and the reaction solution was diluted with dichloromethane and $H_2O$. After filtered through diatomaceous earth, it was extracted with dichloromethane, and the organic phase was washed with saturated saline solution, dried over anhydrous sodium sulfate, concentrated under reduced pressure, and purified by silica gel column chromatography (DCM/MeOH=10/1) to obtain compound **114** (5.0 mg), white solid.

MS (ESI) *m/z* 523.2 $[M + H]^+$.

$^1H$ NMR (400 MHz, DMSO-$d_6$) $\delta$ 10.11 (s, 1H), 8.20 (s, 1H), 7.77 (dd, *J* = 12.4, 1.9 Hz, 1H), *7.50* (dd, *J* = 8.5, 1.9 Hz, 1H), 7.28 (t, *J* = 8.4 Hz, 1H), 5.84 (s, 1H), 5.59 (s, 1H), 3.54 (s, 3H), 3.38 (d, *J* = 6.7 Hz, 2H), 3.28 - 3.22 (m, 2H), 2.26 - 2.06 (m, 4H), 1.97 (s, 3H).

**Internal control compound BGJ398**

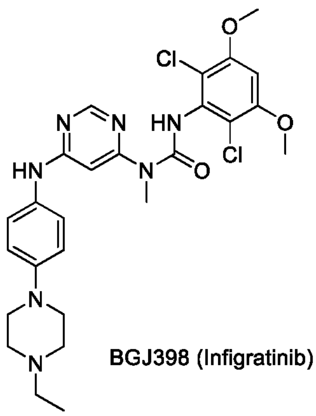

BGJ398 (Infigratinib)

This compound was purchased commercially, manufacturer: MCE, item number: HY-13311

**Control compound I**

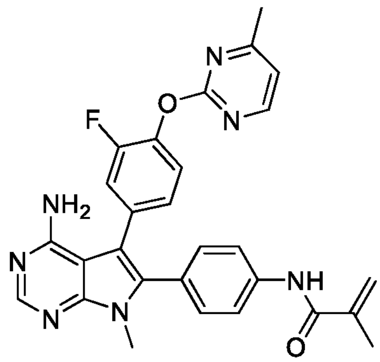

It was synthesized with reference to WO2020231990A1.

**Test Example 1: the test of compounds on cell viability of Ba/F3 Cells**

In this experiment, the inhibitory effect of compounds on cell proliferation was studied by detecting their effects on in vitro cell viability of Ba/F3 cell lines (Ba/F3-TL-FGFR1, Ba/F3-TL-FGFR2, Ba/F3-TL-FGFR3). BJG398 is an internal control compound in this project, a cell line stably expresses the fusion protein of the intracellular kinase domain of TEL-FGFR subtype obtained by transfection into Ba/F3 cells (Hefei PreceDo Biomedical Technology Co., Ltd.) for testing.

The cell line was cultured in a incubator under the culture condition of 37 °C and 5% $CO_2$, regularly passaged, and cells in logarithmic growth phase were taken for plating. The test compounds and the internal control compound BGJ398 were prepared into a 10 mM solution using DMSO.

Preparation of compound stock plate (tube): It was diluted with DMSO from the highest concentration to the lowest concentration in a 4-fold gradient, for a total of 9 concentrations, and the internal control compound was diluted with 4-fold diluent, for a total of 9 concentrations. Then, the preparation of the compound working solution: 98 μL cell culture medium was added to a flat bottomed 96 well transparent drug plate, and 2 μL compound was aspirated from the compound stock plate and added to the cell culture medium in 96 well transparent drug plate. 2 μL DMSO was added to the solvent control. After the compound or DMSO was added, and then mixed well by blowed with a pipette.

Cell plating and administration: Suspension cells were plated according to the following steps: 1) cells were stained with trypan blue, and live cells were counted to ensure a cell viability rate of over 90%; 2) the cell concentration was adjusted to an appropriate level; 3) 95 μL cell suspension (2000 cells/cell) was added to each well of the detection cell plate of compounds, and culture medium without cells (containing 0.1%) was added to the Min control well; 4) administration in cell plate for compounds detection: 5 μL of 20 × compound working solution was taken and added to the cell culture plate as shown in Table 1. 5 μL DMSO cell culture medium mixture was added to the Max control. The final concentration of DMSO was 0.1%; 5) the culture plate was cultured in a 37 °C, 5% $CO_2$ incubator for 72 h.

It was performed according to the instructions of the PromegaCellTiter Glo luminescent cell viability assay kit (Promega-G7573): 1) the CellTiter Glo buffer was thawed and placed to room temperature; 2) the CellTiter Glo substrate was placed to room temperature; 3) CellTiter Glo buffer was added to a bottle of CellTiter Glo substrate to dissolve the substrate and prepare CellTiter Glo working solution; 4) slow vortex oscillation for sufficient dissolution; 5) the cell culture plate was taken out and placed for 10 min for equilibrated to room temperature; 6) 50 μL (equivalent to half the volume of cell culture medium in each well) of CellTiter Glo working solution was added to each well; 7) the culture plate was shaken on the track shaker for 2 min to induce cell lysis; 8) the culture plate was placed at room temperature for 10 min to stabilize the luminescence signal; 9) the luminous signal was detected on the SpectraMaxParadigm reader.

The data on inhibition rate of cell proliferation was processed using the following formula:

$$\text{Inhibition Rate (Inh\%)} = 100 - (RLU_{Drug} - RLU_{Min})/(RLU_{Max} - RLU_{Min}) * 100\%.$$

The inhibition rates of compounds at different concentrations were calculate in EXCEL, then GraphPad Prism software was used to make inhibition rate curves and calculate relevant parameters, including maximum and minimum cell inhibition rates, $IC_{50}$ values.

Table 1 Results of proliferation inhibition of test compounds on Ba/F3 cell lines (Ba/F3-TEL-FGFR$_1$, Ba/F3-TEL-FGFR2, Ba/F3-TEL-FGFR3)

| Compound number | Ba/F3 cell | | |
|---|---|---|---|
| | **FGFR2** ($IC_{50}$ nM) | **FGFR1** ($IC_{50}$ nM) | **FGFR3** ($IC_{50}$ nM) |
| BGJ398 | 4.2 | 11.3 | 6.7 |
| **Control compound I** | 2.5 | 1323 | 1615 |
| **1** | 2.3 | 656.1 | 869.2 |
| **2** | 11.2 | 1509 | 846.5 |
| **3** | 699.7 | - | - |
| **4** | 9.9 | 2371 | 3361 |
| **5** | 29.3 | - | - |
| **6** | 9.3 | - | - |
| **7** | 2.4 | 626.8 | 544.9 |
| **8** | 0.7 | 600.3 | - |
| **9** | 14.9 | - | - |
| **10** | 106.6 | - | - |
| **11** | 171.8 | - | - |
| **12** | 275.7 | - | - |
| **13** | 0.6 | - | - |
| **14** | 9.9 | - | - |
| **15** | 15.4 | - | - |
| **16** | 50.1 | - | - |
| **17** | 4616 | - | - |
| **18** | 21.5 | - | - |
| **19** | 2982 | - | - |
| **20** | 86.7 | - | - |
| **21** | 0.8 | - | - |
| **22** | 2.7 | 716.7 | - |
| **23** | 0.7 | 568.2 | - |
| **24** | 2.1 | 1909 | - |
| **25** | 38.7 | - | - |
| **26** | 4.5 | - | 109.6 |
| **27** | 26.0 | - | - |
| **28** | 0.9 | - | - |
| **29** | 1209 | - | - |
| **30** | 1442 | - | - |
| **31** | 8.3 | 2094 | - |
| **32** | 0.4 | - | - |
| **33** | 11.2 | 2310 | 1292 |
| **34** | 17.9 | - | - |
| **35** | 4.4 | - | - |
| **36** | 53.5 | - | - |

(continued)

| Compound number | Ba/F3 cell | | |
|---|---|---|---|
| | **FGFR2** ($IC_{50}$ nM) | **FGFR1** ($IC_{50}$ nM) | **FGFR3** ($IC_{50}$ nM) |
| **37** | 32.0 | - | - |
| **38** | 246.9 | - | - |
| **39** | 10.2 | - | - |
| **40** | 7.7 | - | - |
| **41** | 123.6 | - | - |
| **42** | 32.9 | - | - |
| **43** | 14.2 | - | - |
| **44** | 216.4 | - | - |
| **45** | 83.1 | - | - |
| **46** | 161.7 | - | - |
| **47** | 2.7 | - | - |
| **48** | 0.9 | 625.1 | 577.3 |
| **49** | 12.8 | - | - |
| **50** | 7.6 | - | - |
| **51** | 19.1 | - | - |
| **52** | 2.3 | - | - |
| **53** | 10.2 | - | - |
| **54** | 2.8 | - | - |
| **55** | 3.0 | - | - |
| **56** | 12.6 | - | - |
| **57** | 17.4 | - | - |
| **58** | 3.0 | 2026 | 441.7 |
| **59** | 7.8 | 1316 | - |
| **60** | 13.1 | - | - |
| **61** | 150.2 | - | - |
| **62** | 9.2 | - | - |
| **63** | 1.1 | 395.9 | - |
| **64** | 658.3 | - | - |
| **65** | 120.9 | - | - |
| **66** | 27.8 | - | - |
| **67** | 105.0 | - | - |
| **68** | 0.5 | - | - |
| **69** | 11.4 | - | - |
| **70** | 2.7 | 774.6 | - |
| **71** | 2.5 | - | - |
| **72** | 2.2 | - | - |
| **73** | 11.1 | - | - |
| **74** | 0.5 | 299.6 | - |

(continued)

| Compound number | Ba/F3 cell | | |
|---|---|---|---|
| | **FGFR2** ($IC_{50}$ nM) | **FGFR1** ($IC_{50}$ nM) | **FGFR3** ($IC_{50}$ nM) |
| **75** | 0.8 | 700.9 | - |
| **76** | 6.1 | - | - |
| **77** | 4.7 | 883.7 | - |
| **78** | 585.1 | - | - |
| **79** | 61.1 | - | - |
| **80** | 46.0 | - | - |
| **81** | 18.1 | - | - |
| **82** | 0.8 | 484.1 | - |
| **83** | 1.1 | 660.7 | 319.2 |
| **84** | 0.8 | 250.4 | - |
| **85** | 907.3 | - | - |
| **86** | 67.4 | - | - |
| **87** | 2.1 | 665.7 | - |
| **88** | 1.5 | 561.1 | 215.9 |
| **89** | 6.5 | 685.9 | - |
| **90** | 3.6 | - | 173.8 |
| **91** | 23.9 | - | - |
| **92** | 0.8 | - | - |
| **93** | 31.7 | - | - |
| **94** | 1.1 | 625.4 | 204.7 |
| **95** | 0.7 | 483.6 | - |
| **96** | 29.8 | - | - |
| **97** | 1.9 | 479.8 | - |
| **98** | 0.6 | 416.1 | - |
| **99** | 2.7 | - | - |
| **100** | 1.9 | 823.2 | 414.4 |
| **101** | 4.7 | - | - |
| **102** | 0.6 | - | - |
| **103** | 13.9 | - | - |
| **104** | 0.9 | 640.1 | - |
| **105** | 20.9 | - | - |
| **106** | 2.2 | - | - |
| **107** | 15.6 | 3629 | - |
| **108** | 2.5 | - | - |
| **109** | 4.5 | - | - |
| **110** | 2.0 | - | - |
| **111** | <0.15 | - | - |
| **112** | 2.0 | - | - |

(continued)

| Compound number | Ba/F3 cell | | |
|---|---|---|---|
| | **FGFR2** ($IC_{50}$ nM) | **FGFR1** ($IC_{50}$ nM) | **FGFR3** ($IC_{50}$ nM) |
| **113** | 1.8 | - | - |
| **114** | 1.2 | - | - |
| -: Not tested | | | |

As can be seen from the above table, the compounds of the present invention have a good inhibitory effect on the proliferation in Ba/F3-TEL-FGFR2 cells, and exhibit high selectivity on Ba/F3-TEL-FGFR1 and Ba/F3-TEL-FGFR3.

**Test Example 2: Human Liver Microsomes Stability Test**

Human liver microsomes stability test was performed by co-incubating compounds with human liver microsomes in vitro. Firstly, a 10 mM stock solution of the test compound in DMSO solvent was prepared, and then the compound was diluted with methanol to a 0.15 mM working solution. A certain amount PBS, 12mM $MgCl_2$, human liver microsomes (Corning), and compound working solution were mixed to achieve a compound concentration of 1.0 μM, a human liver microsome concentration of 0.5 mg/mL, and the $MgCl_2$ concentration of 3 mM in the reaction system. A deep well plate was taken, 270 μL microsomal buffer mixture solution was added to each well, and then 30 μL preheated 10 mM NADPH solution was added to initiate the reaction, and it was incubated at 37 °C. At 0, 5, 15, 30, and 60 min of incubation, 200 μL methanol: acetonitrile = 1: 1 containing an internal standard was added to the corresponding well to terminate the reaction. After the reaction was terminated at the last 60 min, the deep well plate was subjected to vortex vibration for 5 min (600 rpm/min), and then centrifugated for 10 min. After centrifugation, the supernatant was taken, and purified water was added at a 1:1 ratio for LC-MS/MS detection, and the peak area ratio of the compound to the internal standard at each time point was obtained, and the peak area ratio of the compound at 5, 15, 30, and 60 min was compared with the peak area ratio at 0 minute to calculate the remaining percentage of the compound at each time point, and $T_{1/2}$ was calculated using Graphpad 7 software.

Table 2 Results of human liver microsomes stability test

| Compound | Remaining percentage of compound after 60 min of incubation (%) | $T_{1/2}$ (min) | $CL_{int}$ (mL/min/kg) |
|---|---|---|---|
| Control compound I | **36.4** | **42.0** | **29.7** |
| **2** | **54.0** | **69.3** | **18.0** |
| **4** | **61.6** | **85.6** | **14.6** |

The experimental results showed that compared with the control compound I, the compound of the present invention exhibited better liver metabolic stability and better druggability.

**Test Example 3: Pharmacokinetic Test in Rats**

Rat pharmacokinetic experiments were performed using male SD rats, 180-220g, provided by Beijing Vital River Laboratory Animal Technology Co., Ltd. 3 rats that were fasted overnight were taken, and administered orally by gavage (10 mg/kg). Blood samples were collected before administration, as well as 5, 15, 30 min and 1, 2, 4, 6, 8, 24 h after administration; another 3 rats were taken and administered intravenously (1 mg/kg), blood samples were collected before administration, as well as 5, 15, 30 min and 1, 2, 4, 6, 8, 24 h after administration. Blood sample was centrifuged at 6800g, 2-8 °C for 6 min, and plasma was collected, and stored at -80 °C. Plasma of each time point was taken, 10 times the amount of 50% methanol acetonitrile solution containing internal standard was added, and mixed by vortex for 5 min, centrifuged at 18000 rpm at 4 °C for 10 min, and the supernatant was taken directly for LC-MS/MS analysis. The main pharmacokinetic parameters were analyzed using WinNonlin8.2 software for non compartmental model analysis.

Table 3: pharmacokinetic test in rats results of test compounds

| Compound | Pharmacokinetic parameters in rats (Oral gavage administration) (10 mg/kg) | | | |
|---|---|---|---|---|
| | Cmax (ng/mL) | Tmax (hr) | $AUC_{0-t}$ (h*ng/mL) | $T_{1/2}$ (hr) |
| Control compound I | 141 | 0.67 | 606 | 0.85 |
| 8 | 352 | 0.25 | 630 | 1.81 |
| 24 | 270 | 0.25 | 871 | 1.43 |
| 33 | 306 | 0.42 | 778 | 1.32 |
| 48 | 212 | 0.83 | 883 | 3.44 |
| 58 | 604 | 0.50 | 1423 | 3.18 |
| 63 | 556 | 0.5 | 1696 | 2.59 |
| 83 | 414 | 0.33 | 1483 | 1.87 |

The results of pharmacokinetic test in rats showed that the compounds in the present invention have high oral exposure, good pharmacokinetic properties, and good druggability.

**Test Example 4: Manual Patch Clamp Technique for Detecting the Effect of Test Compounds on Stable Overexpressed hERG Channel Current**

4.1. Preparation of Test Compound

A small amount of compound 24 was weighed, and prepared into a 31.346 mM stock solution using DMSO. The test compound stock solution was diluted with DMSO to 10 mM, 3.33 mM, 1 mM, 0.33 mM, and 0.1 mM diluent in sequence. The test compound diluents were diluted with extracellular fluid containing 0.5% F68 in sequence to prepare working solutions of 30 μM, 10 μM, 3 μM, 1 μM and 0.3 μM, and 0.3% DMSO was ensured. All concentrations were dissolved without visible precipitation.

A small amount of control compound I was weighed, and prepared into a 31.311 mM stock solution using DMSO. The test compound stock solution was diluted with DMSO to 10 mM, 3.33 mM, 1 mM, 0.33 mM, and 0.1 mM diluent in sequence. The test compound diluents were diluted with extracellular fluid containing 0.5% F68 in sequence to prepare working solutions with concentrations of 30 μM, 10 μM, 3 μM, 1 μM and 0.3 μM, and 0.3% DMSO was ensured. All concentrations were dissolved without visible precipitation.

The positive control Cisapride was prepared into a 10 mM stock solution using DMSO. Cisapride stock solution was diluted with DMSO to 1 mM, 333.33 μM, 33.33 μM, 3.33 μM, 0.33 μM, and 0.033 μM diluent in sequence. Cisapride diluent was diluted with extracellular fluid in sequence to prepare working solutions of 1000 nM, 100 nM, 10 nM, 1 nM and 0.1 nM, and 0.3% DMSO was ensured. All concentrations were dissolved without visible precipitation.

4.2 Cell Culture

The HEK-293 cell line stably expressing hERG potassium channels was cultured in DMEM medium containing 10% fetal bovine serum and 0.8 mg/mL G418 at a temperature of 37 °C and a carbon dioxide concentration of 5%. Cells were inoculated into 6 cm cell culture dishes, with a total of $2.5 \times 10^5$ cells per dish (final volume: 5 mL). To maintain the electrophysiological activity of cells, the cell density must not exceed 80%. Before patch clamp detection, cells were isolated with TrypLE™ Express, $4 \times 10^3$ cells were spread onto cover glass, and cultured in a 24 well plate (final volume: 500 μL) for 18 h, and then experimental test was performed.

4.3. Patch Clamp Detection

The voltage stimulation protocol for recording hERG currents using whole cell patch clamp was as follows: when a whole cell seal was formed, the cell membrane voltage was clamped at -80 mV. The clamping voltage was reduced from -80 mV to -50 mV and maintained for 0.5 s (as a leakage current detection), and then stepped up to 30 mV and maintained for 2.5 s, and then quickly restored to -50 mV and maintained for 4 s to excite the tail current of the hERG channel. Data was collected repeatedly every 10 s to observe the effect of the drug on hERG tail current. The experimental data was collected by IPA amplifier (Sutter Instrument) and stored in SutterPatch (with Igor Pro) software.

When the hERG current recorded by the whole cell was stabilized, administration began, and each drug

concentration acted for about 5 min (or until the current was stabilized), and then the next concentration was detected, multiple concentrations were detected for each test compound. The cover glass covered with cells was placed in the recording bath under an inverted microscope, the blank control solution and the test compound working solution sequentially flowed through the recording bath from low concentration to high concentration using gravity perfusion to act on the cells, liquid exchange was performed using a peristaltic pump during recording. The current of each cell detected in the extracellular fluid without the compound serves as its own control group. Each concentration should be independently tested twice using at least two cells. All electrophysiological tests were performed at room temperature.

4.4 Test Results

The inhibitory effects of compound 24 and control compound I on hERG channels were detected in two independent replicate tests, and the $IC_{50}$ values of the test compounds on hERG current were calculated by fitting the concentration effect curve. The test results were as follows:

Table 4. Inhibition rates and $IC_{50}$ results of compound I and compound 24 on hERG current

| Compound | HERG current inhibition rate% (Mean ± SE) | | | | | n | $IC_{50}$ |
|---|---|---|---|---|---|---|---|
| | 0.3 μM | 1 μM | 3 μM | 10 μM | 30 μM | | |
| Control compound I | 6.28% ± 0.31% | 16.23% ± 0.51% | 28.98% ± 0.56% | 64.51% ± 1.31% | 89.42% ± 0.20% | 2 | 6.046 μM |
| 24 | -1.24% ± 1.48% | 7.73% ± 0.87% | 10.17% ± 0.62% | 32.17% ± 2.92% | 14.21% ± 2.95% | 2 | >10 μM |

The experimental results show that compared with the control compound I, the compound of the present invention have weak inhibition on hERG and have good safety.

**Test Example 5: In Vitro Evaluation of The Inhibitory Effect of The Test Compound on Cytochrome P450 Enzyme**

5.1 Experimental Materials

5.1.1 Human liver microsomes

| Vendor | CAS | Cat No. | Lot No. | Concentration |
|---|---|---|---|---|
| CORNING | NA | 452161 | 1151001 | 20 mg/mL |

5.1.2 Positive control

| Name | Vendor | Cat No. | Lot No. | Concentration of stock solution | Solvent |
|---|---|---|---|---|---|
| α- naphthoflavone | SIGMA | 604-59-1 | SLBN2386V | 0.3 mM | DMSO |
| Sulfamethoxazole | J&K | 178125 | LP20Q121 | 10 mM | DMSO |
| Omeprazole | TCI | 73590-58-6 | X5JEB | 100 mM | DMSO |
| Quinidine | Energy Chemical | 56-54-2 | 990XROHQ | 10 mM | DMSO |
| Ketoconazole | J&K | 973125 | LI30P36 | 2.5 mM | DMSO |

5.1.3 Probe substrate

| Name | Vendor | Cat No. | Lot No. | Concentration of stock solution | Solvent |
|---|---|---|---|---|---|
| Phenacetin | aladdin | NA | F2101248 | 30mM | DMSO |
| Diclofenac acid | TC1 | D3748 | U5XOJ | 10mM | DMSO |
| S-methomyl | MCE | HY-B1184 | 117020 | 35mM | DMSO |
| Dextromethorphan hydrobromide | National Institute for Food and Drug Control | 6700-34-1 | 100201-2012 04 | 5 mM | DMSO |
| Testosterone | aladdin | NA | F2126139 | 80mM | DMSO |
| Midazolam | National Institute for Food and Drug Control | 59467-70-8 | 171265-2014 02 | 5 mM | DMSO |

5.1.4 Internal standard

| Name | Vendor | Cat No. | Lot No. |
|---|---|---|---|
| Toluene sulfonamide | Supelco | T0891 | SLCK4822 |
| Verapamil | TCI | V0118 | RFMWJ |

5.1.5 Other reagents

| Name | Vendor | Cat No. | Lot No. |
|---|---|---|---|
| NADPH | MCE | HY-F0003 | 139299 |
| Phosphate buffered saline (PBS) | Yuanpei | B320KJ | K211021 |

Termination solution containing internal standard: toluene sulfonamide concentration of 20.0 ng/mL, verapamil concentration of 10.0 ng/mL

5.2 Experimental Preparation

Preparation of positive control inhibitor and compound working solution: 16 μL inhibitor stock solution or compound stock solution for each CYP subtype were taken and added to 24 μL DMSO. Then, they were diluted gradually three times with diluent to obtain different concentrations of test sample/positive control inhibitor working solution (7 concentrations each except for blank)

Preparation of substrates for each CYP subtype: Preparation of substrates for each CYP subtype: for CYP1A2, CYP2C9, CYP3A4 (midazolam), and CYP2D6, 8μ L substrate stock solution was added to 1992 μ L PBS; for CYP2C19, 40 μ L substrate stock solution and 160 μL human liver microsomes (20.0 mg/mL) were added to 1800 μ L of PBS; for CYP3A4 (testosterone), 40 μL substrate stock solution was added to 1960 μL PBS.

Preparation of liver microsomal working solution: 300 μL human liver microsomes (20.0 mg/mL) was taken and added to 29700 μL PBS and mixed well, and the concentration of the prepared human liver microsomal working solution was 0.200 mg/mL.

Preparation of 4 x NADPH working solution: A certain weight of NADPH tetrasodium salt was weighted and dissolved to a suitable volume of PBS to obtain NADPH working solution, with a concentration of 8.00 mM.

Preparation of test sample group working solution: The human liver microsomal working solution was divided into 96 well plates at 796 μL per well, and then 4 μL test sample working solution was added.

Preparation of positive control group working solution: The human liver microsomal working solution was divided into 96 well plates at 199 μL per well, and then 1 μL positive control inhibitor working solution was added.

Blank solvent working solution preparation: The human liver microsomal working solution was divided into 96 well plates at 199 μL per well, and then 1 μL DMSO was added.

5.3 Experimental steps

(a) 30 μL test sample/positive control inhibitor working solution or 30 μL blank solvent working solution per well were transferred into a 96 well plate, and 15 μL substrate working solution of different enzyme subtypes transferred into each well, and mixed evenly.
(b) The above incubation plates and 4 x NADPH working solution were preheated at 37 °C for 5 min.
(c) 15 μL preheated 4 × NADPH working solution per well was transferred into the incubation plate, and the reaction initiated, and continued to be incubated at 37 °C. The incubation time is as follows:

| CYP | Substrate | Final concentration (μM) | Inhibitor | Final highest concentration (μM) | Liver microsomal concentration (mg/mL) | Incubation time (min) |
|---|---|---|---|---|---|---|
| 1A2 | Phenacetin | 30.0 | α- naphthoflavone | 0.300 | 0.100 | 10 |
| 2C9 | Diclofenac acid | 10.0 | Sulfamethoxazole | 10.0 | 0.100 | 10 |
| 2C19 | S-meifentuin | 35.0 | Omeprazole | 100 | 0.500 | 45 |
| 2D6 | Dextromethorphan | 5.00 | Quinidine | 10.0 | 0.100 | 20 |
| 3A4 | Testosterone | 80.0 | Ketoconazole | 2.50 | 0.100 | 5 |
| 3A4 | Midazolam | 5.00 | Ketoconazole | 2.50 | 0.1 | 5 |

(d) After the reaction time was reached, 180 μL termination solution was added to terminate the reaction.
(e) The incubation plate was vibrated at 600 rpm for 10 min, and then centrifuged at 6000 rpm for 15 min.
(f) After centrifugation, 80 μL supernatant was taken and added to 120 μL ultrapure water, and after it was mixed evenly, LC-MS/MS was used to analyze the metabolites of the probe substrate.

Table 5 Inhibition tests results of control compound I and the compounds of the present invention on CYP subtypes

| Compound | $IC_{50}$ (μM) | | | | | |
|---|---|---|---|---|---|---|
| | 1A2 | 2C9 | 2C19 | 2D6 | 3A4 (Testosterone) | 3A4 (Testosterone) |
| Control compound I | >10 | 1.68 | 7.78 | 1.50 | 11.84 | 10.85 |
| 9 | - | - | - | 24.61 | - | 13.52 |
| 24 | >10 | 29 | 19.37 | 14.27 | 10.68 | - |
| 33 | - | - | - | 9.52 | - | 19.19 |
| 56 | - | - | - | 5.54 | - | 13.23 |
| 58 | >10 | 29.68 | 5.42 | 9.81 | >10 | - |
| 83 | >10 | 14.40 | 14.13 | 8.77 | 11.57 | - |
| -: Not tested | | | | | | |

The experimental results indicate that compared with the control compound I, the compounds of the present invention have weaker inhibitory effects on CYP subtypes and have lower potential drug interaction risks.

**Test Example 6: In Vitro Evaluation of Rat Liver Cells Metabolic Stability of Test Compound**

6.1 Rat liver cells and reagents

| Species | Strain | Cat No. | Lot No. | Gender | Supplier |
|---|---|---|---|---|---|
| Rat | SD | M00005 | DVB | Male | BioIVT |

| Reagent | Vendor | Cat No. |
|---|---|---|
| Isotonic percoll | Cytiva | 10303640 |
| HEPES | Sigma | RNBH8969 |
| DPBS | Gibco | 2224922 |
| FBS | Corning | 35-076-CV |
| Human-insulin | Gibco | 12585-014 |
| Dexamethasone | NICPBP | - |
| Williams E Medium | Gibco | 2336217 |
| GlutaMAX (100x) | Gibco | 22777239 |

6.2 Experimental Design

6.2.1 Preparation of compound working solution

Test compound and control drug verapamil powder were prepared into 10 mm stock solution using DMSO, and they were diluted with 50% acetonitrile/50% water to 100 μM working solution before use.

6.2.2 Preparation of liver cells

1) 49.5 mL Williams E Medium and 0.5 mL glutaMAX were mixed as incubation medium. The liver cell resuscitation solution and incubation solution were preheated in a 37 °C water bath for at least 15 min before use.
2) A tube of liver cells stored at ultra-low temperature was taken and ensured that they remain in a low-temperature frozen state before resuscitation. The liver cells were placed quickly in a 37 °C water bath and shaken gently until all ice crystals were dispersed.
3) The contents of the liver cells tubes were poured into a centrifuge tube containing 50 mL of resuscitation medium, and they were centrifuged at 100 g for 10 min. After centrifugation, the resuscitation medium was aspirated, and sufficient incubation medium was added to obtain a cell suspension with a cell density of approximately $1.0 \times 10^6$ cells/mL.
4) AO/PI staining was used to count liver cells and determine viable cell density, the survival rate of liver cells must be greater than 75%. The liver cell suspension was diluted with incubation medium to a viable cell density of $0.5 \times 10^6$ cells/mL.

6.3 Experimental Methods

1) 198 μL live cell suspension was transferred to a 96 well deep well plate, and the deep well plate was placed on a vortex and preheat in the incubator for 10 min.
2) 2 μL 100 μM test compound or positive control compound was added to each well for reaction initiation, and the deep well plate was placed back on the incubator vortex.
3) The incubation plate was placed into the incubator for incubation. 25 μL suspension was taken at 0.5, 15, 30, 60, 90, and 120 min, and 6-fold (150 μL) acetonitrile containing internal standard quencher (100 nM alprazolam, 200 nM caffeine, and 100 nM toluenesulfonylbutamide) was added to a new 96 well plate to terminate the reaction. The plate was vortexed for 5 min and centrifuged at 3220 rpm and 4 °C for 45 min to precipitate the protein. 100 μL of supernatant was transferred to the sample plate, and 100 μL pure water was added and mixed well for UPLC-MS/MS analysis. All incubation systems were conducted in a dual parallel manner.

6.4 Data Analysis and Experimental Results

All data was calculated using Microsoft Excel software. Peak area was detected by extracting ion chromatogram. In vitro half-life ($T_{1/2}$) and in vitro clearance rate (in μL/min/$10^6$ cells) of the parent drug were detected by linearly fitting of the natural logarithm of the elimination percentage of the parent drug versus time.

Table 6 Results of rat liver cell stability test

| Compound | Remaining percentage of compound after 60 min of incubation (%) | in vitro $t_{1/2}$ (min) | in vitro $CL_{int}$ (μL/min/$10^6$ cells) |
|---|---|---|---|
| Control compound I | 18.14 | 24.52 | 56.66 |
| 23 | 24.45 | 28.65 | 48.40 |
| 24 | 37.55 | 37.34 | 37.13 |
| 83 | 35.86 | 37.15 | 37.31 |

The experimental results showed that compared with the control compound I, the compounds in the present invention exhibited better liver cell metabolic stability and better druggability.

**Test Example 7: The Test of The Proliferation Inhibitory Activity of Compounds on SNU16 and A204 Cells**

CCK-8 (Bimake/B34304) colorimetric method was used to detect the in vitro proliferation inhibitory effect of compounds on suspended or adherent cells in this experiment. The specific experimental steps were as follows:

1) Cell plating: SNU16 and A204 tumor cells (cells from Shanghai Institute of Organic Chemistry, Chinese Academy of Sciences) in the logarithmic growth phase were digested and centrifuged for trypan blue counting, and the cells were seeded into a transparent 96 well plate at a density of 4000 cells/100 μ 1/well. Blank control group: only culture medium was added.
2) Preparation of working solution: The test compound and internal control compound BGJ398 were both prepared into 10 mM stock solutions using DMSO. The test compound and control compound stock solution were diluted with the corresponding culture medium required for cell culture (with or without solvent DMSO) to obtain a series of working solutions, with concentrations of twice the final concentration. The solvent DMSO contents in each concentration group were consistent with that of the solvent control group. The final concentration of A204 cells started at 10 μM and was diluted twice. The final concentration of SNU-16 cells started at 1 μM and was diluted 4-fold.
3) Co-incubation: After 24 h of plate plating, 100 μl/well mother solution of compound with series concentration was added to a 96 well plate and mixed well, then co-cultured for 72 h. All groups should have at least 4 replicates, with 10 concentrations of each compound. Blank control group: Only culture medium was added, and no cells or drugs were added, to eliminate interference from the contrast color of the culture medium.
4) Measurement of absorbance: 30 μl of CCK8/culture medium (1: 2) mixed system was added to each well, and co-cultured for 2 h, then shaken thoroughly to make it uniform, and the absorbance values at A450 and A650 were measured on a BioTek Synergy H1 enzyme-linked immunosorbent assay reader.
5) Process of data: The raw data of A450-A650 was obtained, the cell survival rates of each treatment well were obtained, and a nonlinear regression model was used to calculate the $IC_{50}$ values of the compound on different cells.

Calculation of cell survival rate: Cell survival rate (%)=[(As-Ac)/(Ab-Ac)] × 100%] (As: experimental well; Ab: Solvent control well; Ac: Blank well).

Table 7 Results of proliferation inhibition of test compounds on SNU16 and A204 cell lines

| Compound number | Cells | |
|---|---|---|
| | SNU16($IC_{50}$ nM) | A204($IC_{50}$ nM) |
| BGJ398 | 4.3 | 100 |
| Control compound I | 13.2 | 3220 |
| 1 | 5.57 | 1314 |
| 7 | 5.43 | 582.7 |

(continued)

| Compound number | Cells | |
|---|---|---|
| | SNU16($IC_{50}$ nM) | A204($IC_{50}$ nM) |
| 8 | 1.34 | 618 |
| 21 | 1.72 | 332.7 |
| 22 | 4.97 | 1606 |
| 23 | 2.4 | 617.6 |
| 24 | 1.59 | 678.9 |
| 26 | 3.36 | 285.4 |
| 32 | 9.56 | 2250 |
| 35 | 4.91 | 406.4 |
| 48 | 13.8 | 1350 |
| 58 | 9.52 | 1271 |
| 63 | 8.71 | 459.7 |
| 68 | 1.65 | 267.4 |
| 74 | 1.19 | 595 |
| 83 | 1.89 | 627.1 |
| 87 | 7.74 | 3595 |
| 88 | 5.01 | 858.4 |
| 97 | 2.66 | 763.7 |
| 98 | 1.81 | 845.4 |
| 100 | 4.78 | 642.2 |
| 102 | 2.63 | 502 |
| 104 | 5.51 | 1018 |
| 109 | 9.38 | 1045 |

As can be seen from the above table, the compounds of the present invention have a good proliferation inhibitory effect on SNU-16 cells and weak proliferation inhibitory activity on A204 cells, which showed high selectivity.

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to this application.

## Claims

1. A compound as shown in formula (I), a tautomer, a stereoisomer, a hydrate, a solvate, or a pharmaceutically acceptable salt thereof:

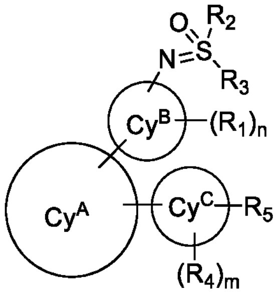

(I)

wherein, n and m are each independently 0, 1, 2, or 3;
$Cy^A$ is selected from the group consisting of:

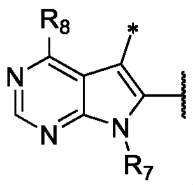
,
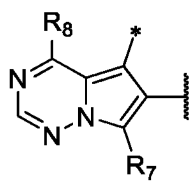
,
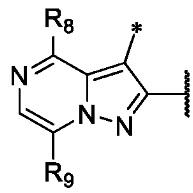
,
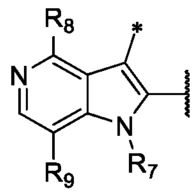
,

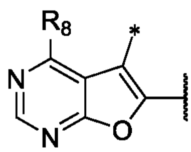
,
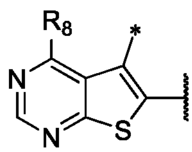
,
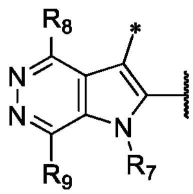
and
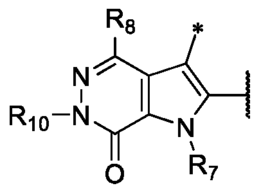
,

wherein 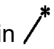 denotes a bond connected to $Cy^B$, and 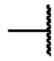 denotes a bond connected to $Cy^C$;
$Cy^B$ is selected from the group consisting of: $C_{6-10}$ aryl and 5-12 membered heteroaryl;
$Cy^C$ is selected from the group consisting of: $C_{6-10}$ aryl, 5-12-membered heteroaryl, saturated or partially unsaturated $C_{3-6}$ carbocyclyl and saturated or partially unsaturated 4-12-membered heterocyclyl;
$R_1$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ saturated or partially unsaturated carbocyclyl-O-, $SF_5$ and 4-12 membered heterocyclyl;
$R_2$ and $R_3$ are each independently selected from the group consisting of: $C_{1-6}$ alkyl, $C_{1-6}$ alkoxy, $C_{1-6}$ haloalkyl, $C_{1-6}$ haloalkoxy, $C_{1-6}$ alkylamino, $C_{3-6}$ saturated or partially unsaturated carbocyclyl and 4-12-membered heterocyclyl; wherein $R_2$ and $R_3$ can be the same or different;
or $R_2$ and $R_3$ together with the sulfur atom to which they are connected form a 4-12-membered heterocyclyl;
$R_4$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ carbocyclyl-O-, $SF_5$ and 4-12-membered heterocyclyl;
$R_5$ is -L-Rw, wherein L is selected from the group consisting of: covalent bond, -$C_{1-4}$ alkyl-, -$NR_6$-, -$C_{1-4}$ alkyl $NR_6$-;
$R_6$ is selected from the group consisting of: H, $C_{1-6}$ alkyl, $C_{1-6}$ alkoxy, $C_{1-6}$ haloalkyl and $C_{1-6}$ haloalkoxy;
Rw is selected from the group consisting of: halogen, cyano,

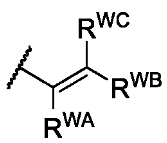
,
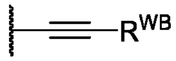
,

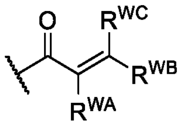, 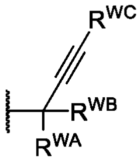, 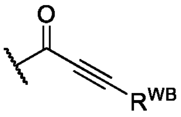, 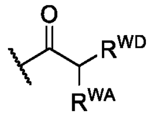, 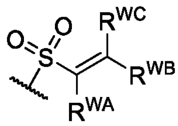, 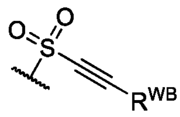,

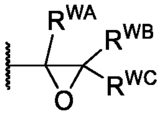, 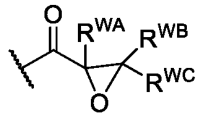, 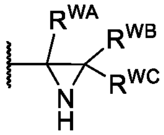, 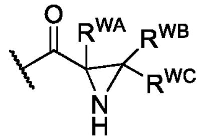, 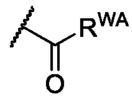, 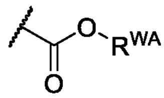

and 5-12-membered nitrogen-containing heterocyclyl;

$R^{WA}$, $R^{WB}$, and $R^{WC}$ are each independently selected from the group consisting of: H, D, halogen, CN, C(O)Ra, C(O)ORa, NRaRb, C(O)NRaRb, C(O)NRaORb, or selected from the group consisting of: $C_{1-6}$ alkyl, $C_{1-6}$ alkoxy, $C_{1-6}$ haloalkyl, $C_{1-6}$ haloalkoxy, phenyl, 3 to 7-membered saturated or partially unsaturated heterocyclyl with 1 to 2 heteroatoms independently selected from nitrogen, oxygen and sulfur, and 5 to 6-membered heteroaryl with 1 to 4 heteroatoms independently selected from nitrogen, oxygen and sulfur; and the $R^{WA}$, $R^{WB}$, and $R^{WC}$ can each be independently substituted with 1, 2, or 3 substituents selected from the group consisting of: $C_{1-6}$ alkylamino and $C_{1-6}$ alkoxy;

or $R_6$ and Rw together form a ring; and the ring is selected from the group consisting of: 5-12 membered heteroaryl and 4-12 membered heterocyclyl;

$R_7$ is selected from the group consisting of: H, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{6-10}$ aryl, 5-12 membered heteroaryl, 4-12-membered heterocyclyl; or $R_4$ and $R_7$, as well as the atoms between them, together form a ring, and the ring is selected from the group consisting of: $C_{6-8}$ saturated or partially unsaturated carbocyclyl, $C_{6-10}$ aryl, 6-12 membered heteroaryl and 6-12 membered saturated or partially unsaturated heterocyclyl;

$R_8$ is selected from the group consisting of: H, OH, $NH_2$, $C_{1-6}$ alkyl, $C_{1-6}$ alkoxy, $C_{1-6}$ haloalkyl, $C_{1-6}$ haloalkoxy and $C_{1-6}$ alkylamino;

$R_9$ is selected from the group consisting of: H, D, halogen, CN, $NH_2$, $C_{1-6}$ alkyl, $C_{1-6}$ alkoxy, $C_{1-6}$ haloalkyl, $C_{1-6}$ haloalkoxy and $C_{1-6}$ alkylamino;

unless otherwise specified, each of the above-mentioned alkyl, alkoxy, haloalkyl, haloalkoxy, alkylamino, alkenyl, haloalkenyl, alkynyl, haloalkynyl, saturated or partially unsaturated carbocyclyl, halogenated saturated or partially unsaturated carbocyclyl, saturated or partially unsaturated heterocyclyl, aryl and heteroaryl can be substituted with one or more Ra;

Ra and Rb are each independently selected from H, D, halogen, CN, oxo (=O), OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkyenyl, $C_{1-4}$ haloalkenyl, $C_{2-4}$ alkynyl, $C_{1-4}$ haloalkynyl, $C_{3-6}$ saturated or partially unsaturated carbocyclyl, $C_{3-6}$ halogenated saturated or partially unsaturated carbocyclyl, $C_{3-6}$ saturated or partially unsaturated carbocyclyl-O-, 4-12 membered heterocyclyl;

wherein, the heterocyclyl is a saturated or partially unsaturated non-aromatic group; the carbocyclyl or heterocyclyl can be optionally in the form of a monocycle, bridged ring, spirocycle, or fused ring.

2. The compound according to claim 1, or a tautomer, a stereoisomer, a hydrate, a solvate, or a pharmaceutically acceptable salt thereof, wherein, the compound has a structure as shown in formula (I-1):

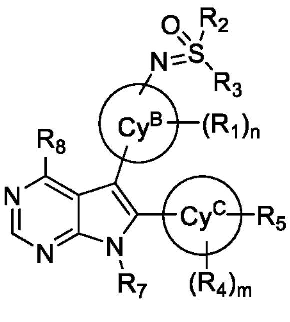

I-1

wherein, $R_1$, $R_2$, $R_3$, $R_4$, $R_5$, $R_7$, $R_8$, $Cy^B$ and $Cy^C$ are as defined in claim 1;
or $R_7$, $R_4$, and atoms to which they connected together form a 5-12 membered carbocyclyl or heterocyclyl.

**3.** The compound according to claim 1, or a tautomer, a stereoisomer, a hydrate, a solvate, or a pharmaceutically acceptable salt thereof, wherein, the compound has a structure as shown in formula (I-2):

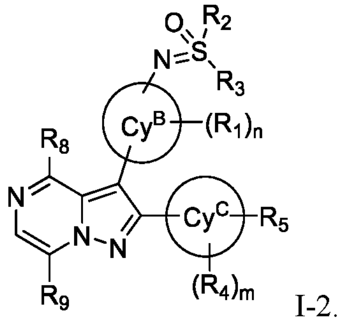
I-2.

In another preferred embodiment, $R_8$ is selected from the group consisting of H, $NH_2$, $C_{1-6}$ alkyl, and $C_{1-6}$ alkylamino.
In another preferred embodiment, $R_9$ is selected from the group consisting of: H, D, halogen, CN, $C_{1-6}$ alkyl and $C_{1-6}$ haloalkyl.

**4.** The compound according to claim 1, or a tautomer, a stereoisomer, a hydrate, a solvate, or a pharmaceutically acceptable salt thereof, wherein, $Cy^B$ is selected from the group consisting of: phenyl and 5-7 membered heteroaryl. In another preferred embodiment, $Cy^B$ is selected from the group consisting of: phenyl, pyridyl, pyrimidinyl, pyridazinyl, pyrrolyl, furanyl, thienyl, imidazolyl, pyrazolyl, thiazolyl, triazolyl, and oxazolyl.

**5.** The compound according to claim 1, or a tautomer, a stereoisomer, a hydrate, a solvate, or a pharmaceutically acceptable salt thereof, wherein, $Cy^C$ is selected from the group consisting of: phenyl, 5-7 membered heteroaryl, saturated or partially unsaturated $C_{3-6}$ saturated or partially unsaturated carbocyclyl, and saturated or partially unsaturated 4-7 membered heterocyclyl.
In another preferred embodiment, $Cy^C$ is selected from the group consisting of: phenyl, pyridyl, pyrimidinyl, pyridazinyl, pyrrolyl, furanyl, thiophenyl, imidazolyl, pyrazolyl, thiazolyl, triazolyl, oxazolyl, and 5-7 membered saturated or partially unsaturated heterocyclyl.

**6.** The compound according to claim 1, or a tautomer, a stereoisomer, a hydrate, a solvate, or a pharmaceutically acceptable salt thereof, wherein, $R_5$ is -L-Rw, wherein L is selected from the group consisting of: covalent bonds, -$C_{1-4}$ alkyl, and -$NR_6$-; $R_6$ is selected from the group consisting of: H, and $C_{1-6}$ alkyl;

Rw is selected from the group consisting of: cyano,

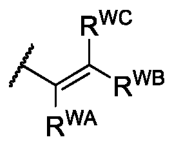
,
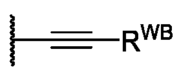
,

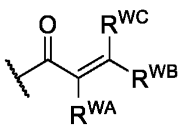
,
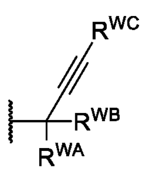
,
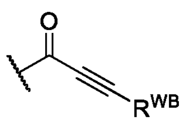
,
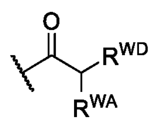
,
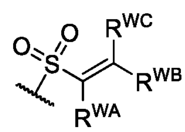
,
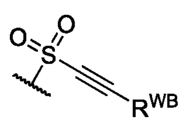
,

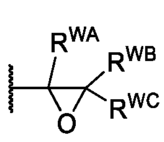 , 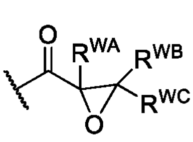 , 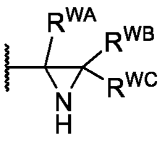 , 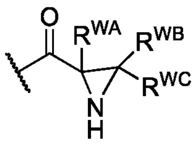 , 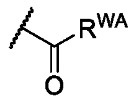 , 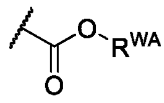

and 5-7 membered nitrogen-containing heterocyclyl;

$R^{WA}$, $R^{WB}$, and $R^{WC}$ are each independently selected from the group consisting of: H, D, halogen, $C_{1-6}$ alkyl, and $C_{1-6}$ haloalkyl; and $R^{WA}$, $R^{WB}$, and $R^{WC}$ can each be independently substituted with 1, 2, or 3 substituents selected from the group consisting of: $C_{1-6}$ alkylamino and $C_{1-6}$ alkoxy;

or $R_6$ and Rw together form a ring; and the ring is selected from the group consisting of: 5-7 membered heteroaryl and 4-7 membered heterocyclyl.

**7.** The compound according to claim 1, or a tautomer, a stereoisomer, a hydrate, a solvate, or a pharmaceutically acceptable salt thereof, wherein,

$R_1$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy, $C_{1-6}$ alkylamino, $C_{2-4}$ alkenyl, $C_{1-4}$ haloalkenyl, $C_{2-4}$ alkynyl, $C_{1-4}$ haloalkynyl and $SF_5$;

$R_2$ and $R_3$ are each independently selected from the group consisting of: $C_{1-6}$ alkyl, $C_{1-6}$ alkoxy, $C_{1-6}$ haloalkyl, $C_{1-6}$ haloalkoxy and $C_{1-6}$ alkylamino; wherein, $R_2$ and $R_3$ can be the same or different;

or $R_2$ and $R_3$ together with the sulfur atom to which they are connected form a 3-7 membered heterocyclyl (preferably a 4-6 membered heterocyclyl);

$R_4$ is selected from the group consisting of: H, D, halogen, CN, OH, $NH_2$, $C_{1-6}$ alkyl, $C_{1-6}$ alkoxy, $C_{1-6}$ haloalkyl, $C_{1-6}$ haloalkoxy and $C_{1-6}$ alkylamino.

In another preferred embodiment, $R_1$ is selected from the group consisting of: H, D, halogen, $C_{1-6}$ alkyl, $C_{1-6}$ alkoxy, $C_{1-6}$ haloalkyl and $C_{1-6}$ haloalkoxy;

$R_2$ and $R_3$ are each independently selected from the group consisting of: $C_{1-6}$ alkyl, $C_{1-6}$ haloalkyl, $C_{1-6}$ alkylamino and $C_{1-6}$ haloalkylamino; wherein, $R_2$ and $R_3$ can be the same or different; or $R_2$ and $R_3$ together with the sulfur atom to which they are connected form a 4-8 membered heterocyclyl;

$R_4$ is selected from the group consisting of: H, D, halogen, $C_{1-6}$ alkyl, $C_{1-6}$ alkoxy, $C_{1-6}$ haloalkyl, and $C_{1-6}$ haloalkoxy.

**8.** The compound according to claim 1, or a tautomer, a stereoisomer, a hydrate, a solvate, or a pharmaceutically acceptable salt thereof, wherein the compound is selected from the group consisting of:

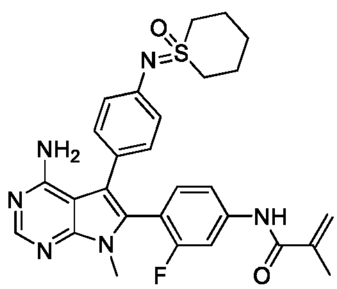 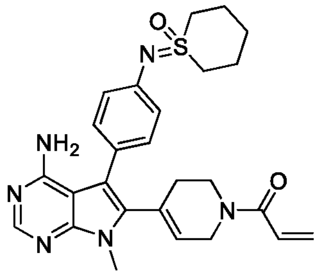 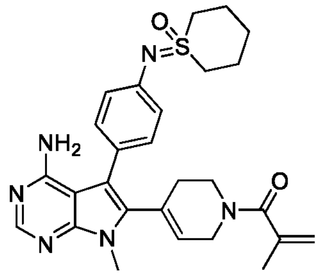

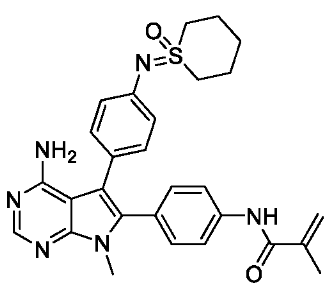 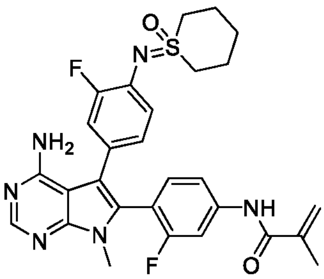 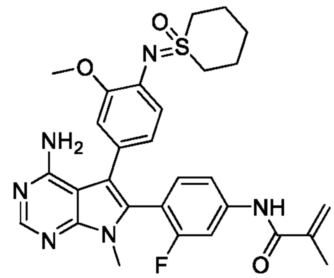

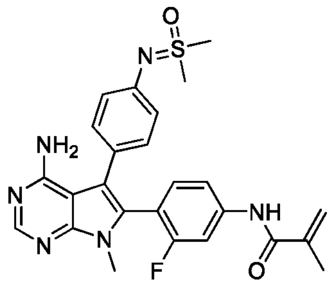

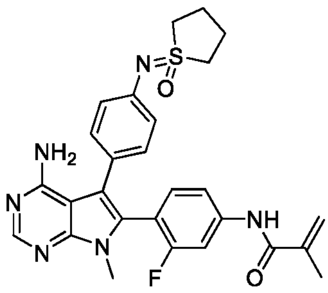

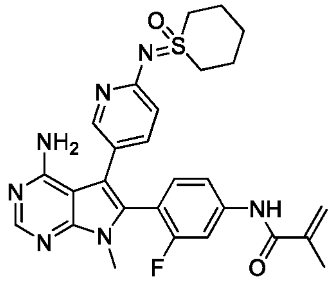

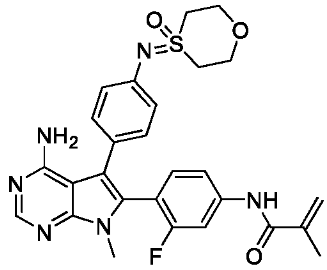

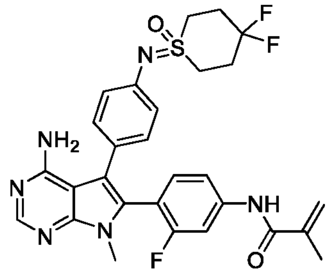

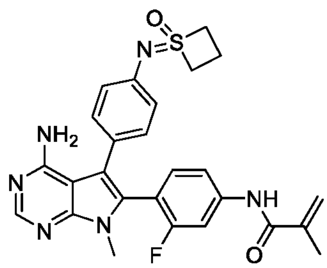

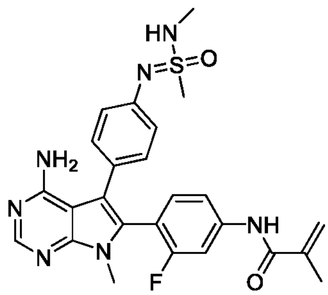

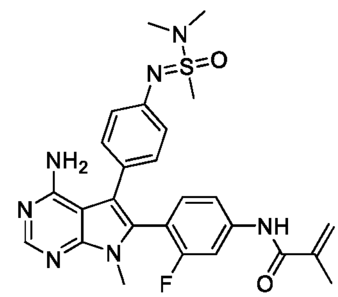

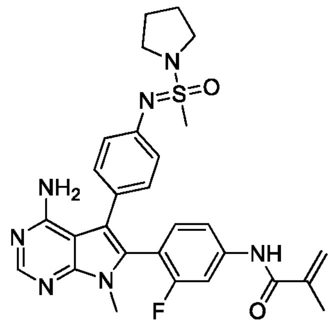

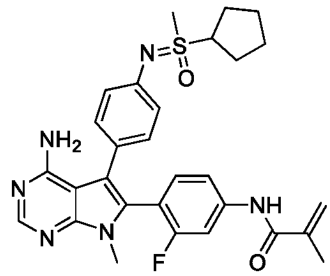

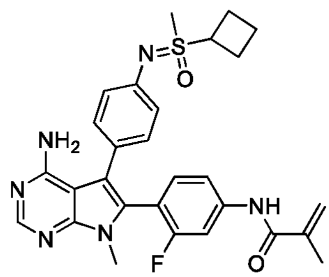

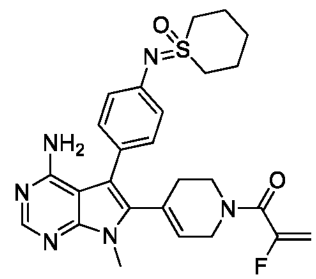

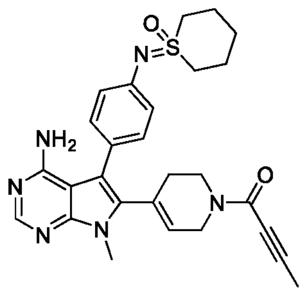

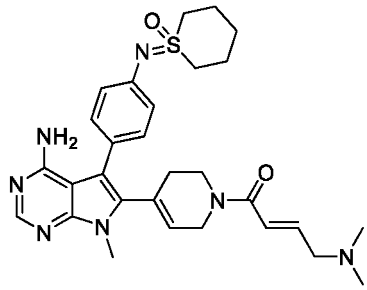

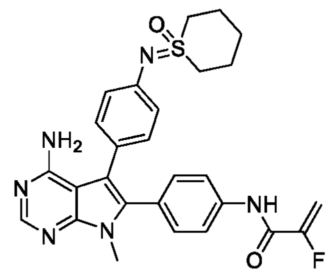

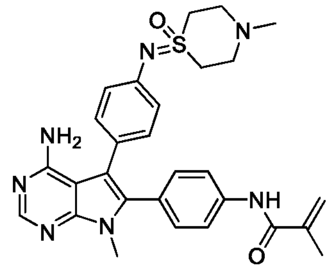

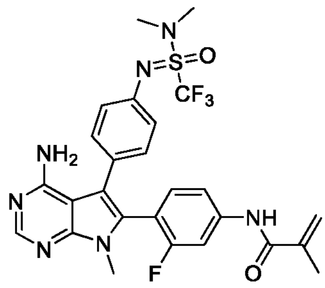

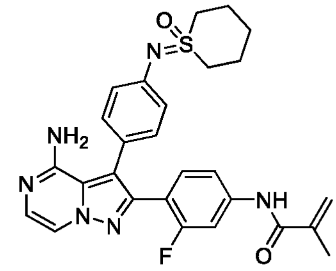

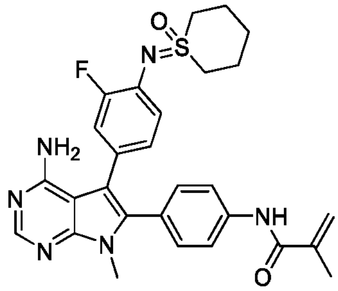

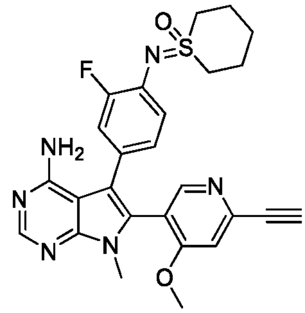

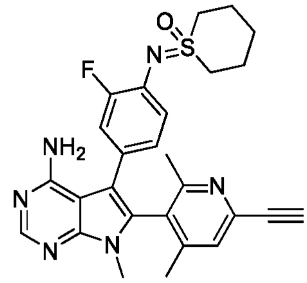

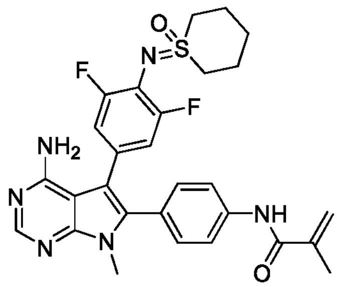

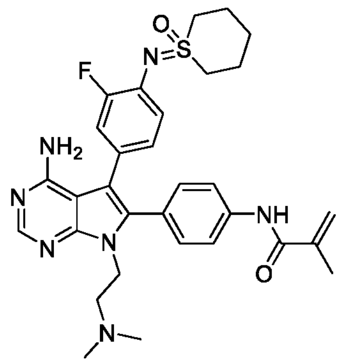

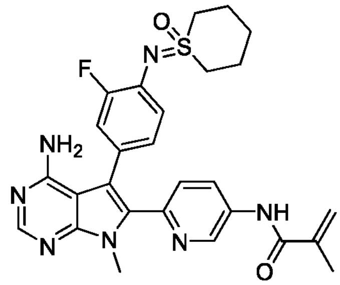

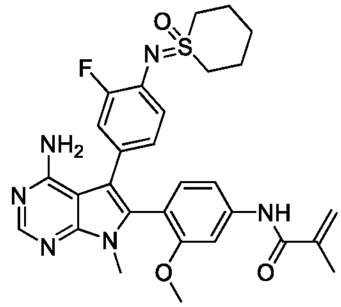

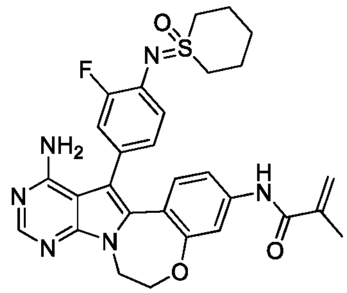

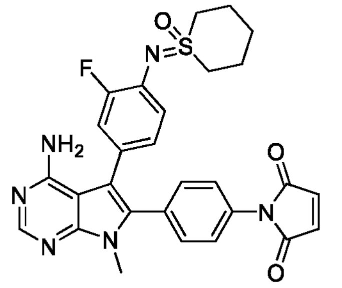

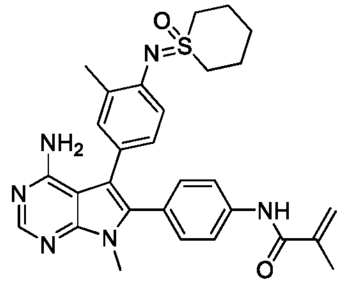

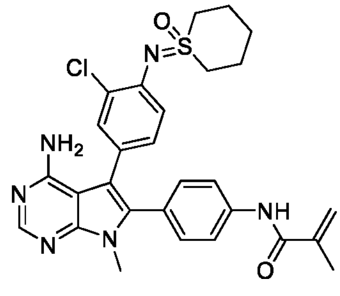

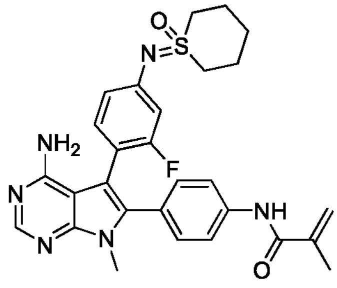

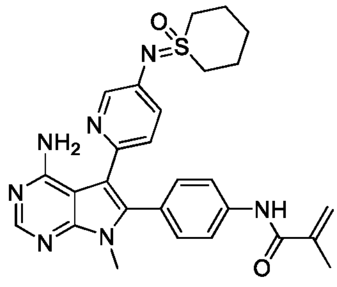
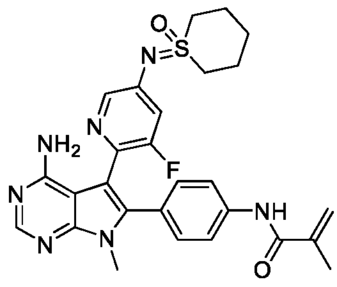
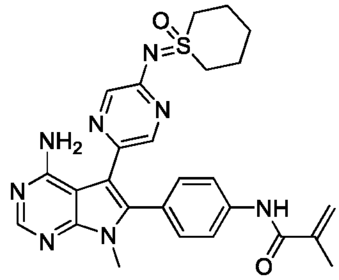

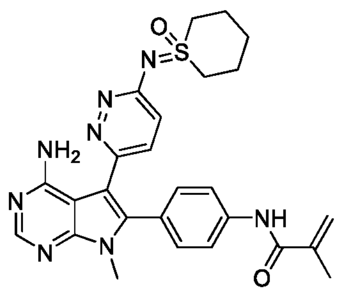
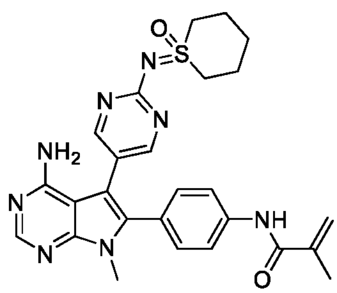
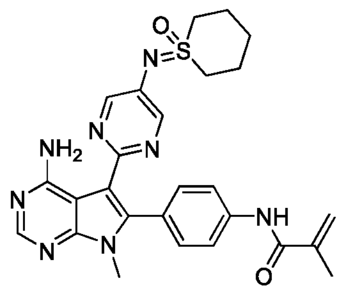

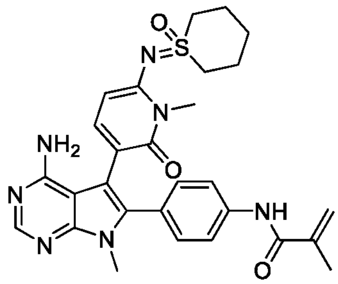
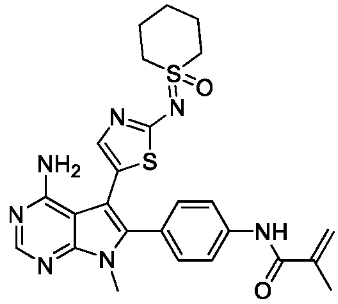
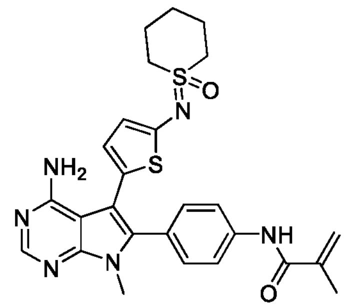

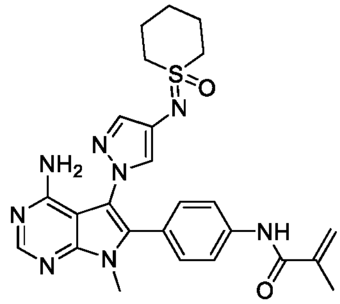
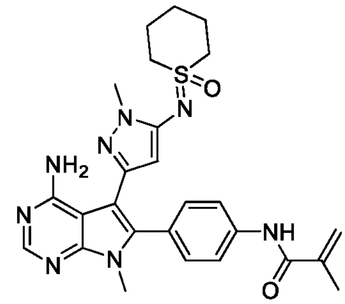
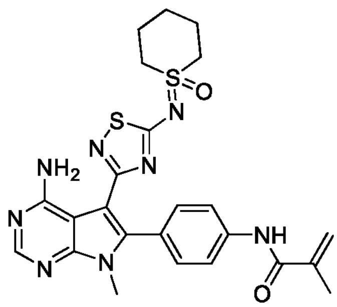

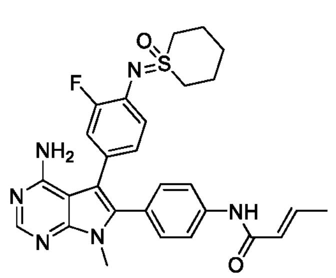
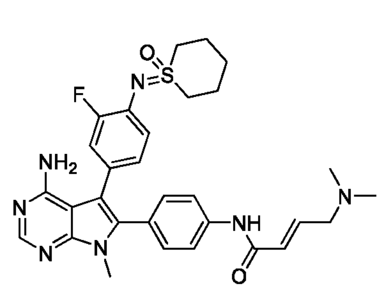
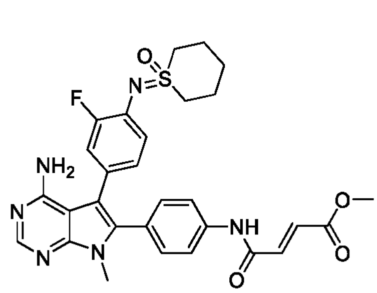

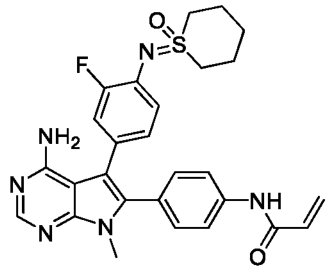

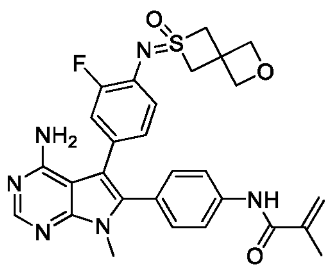

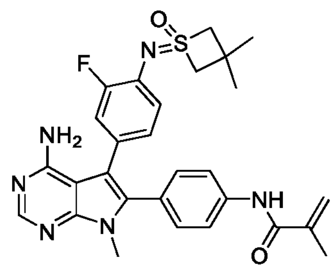

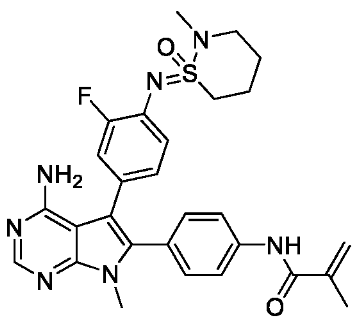

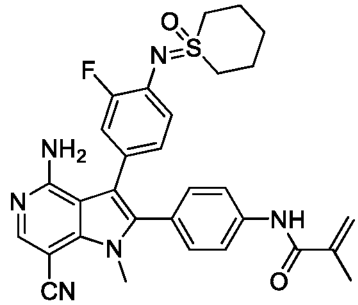

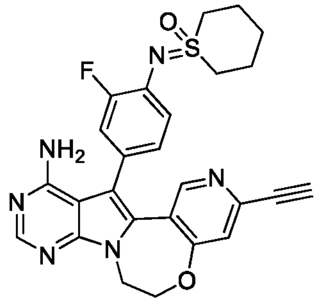

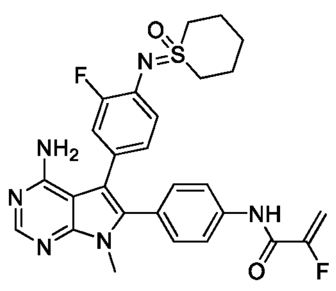

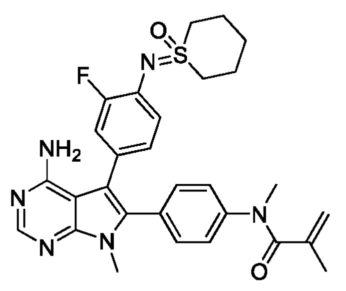

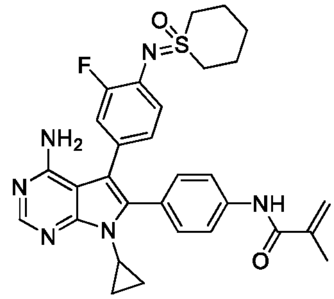

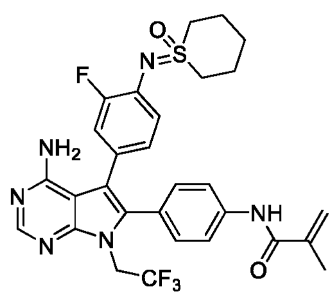

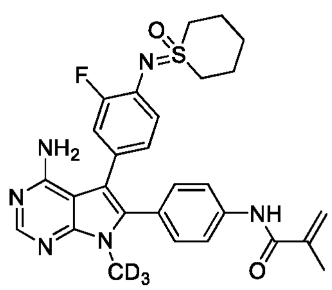

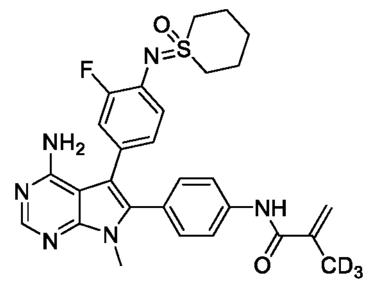

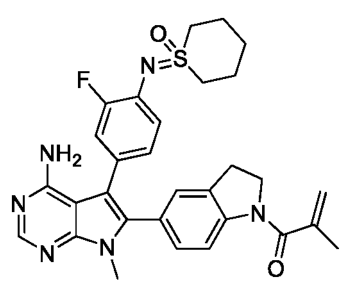

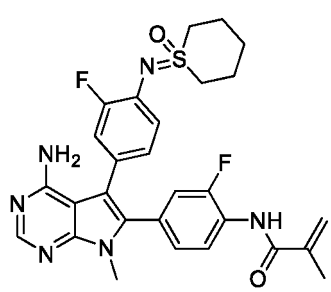

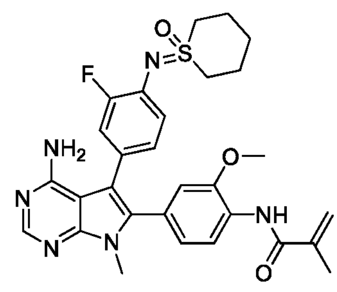

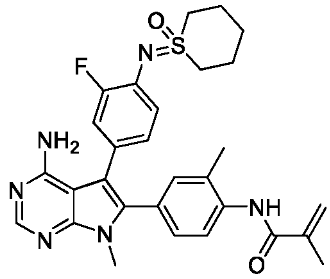
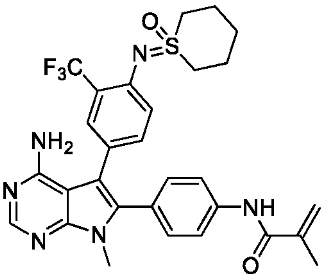
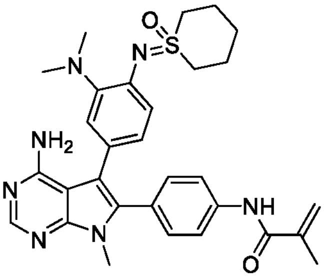

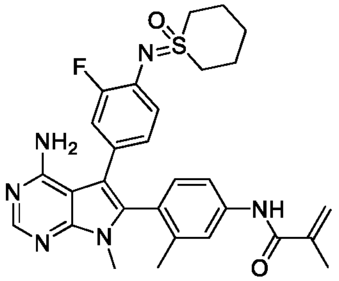
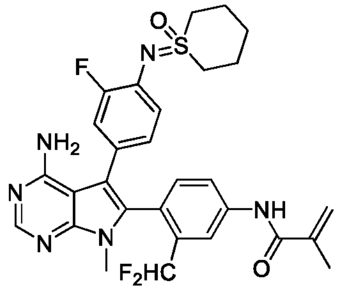
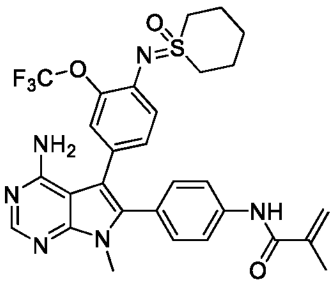

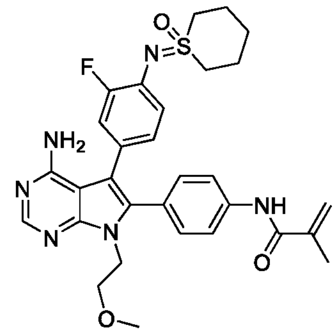
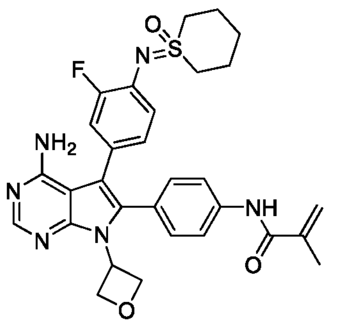
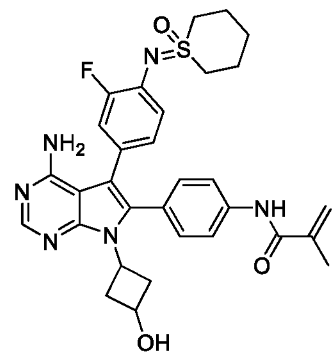

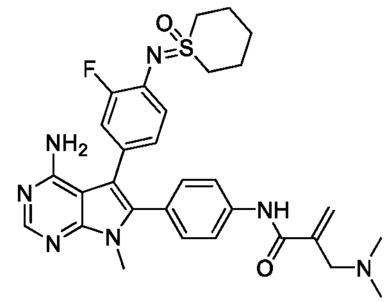
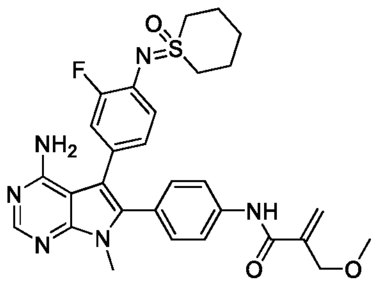
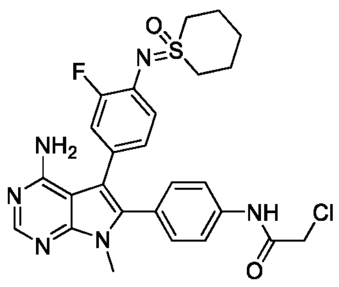

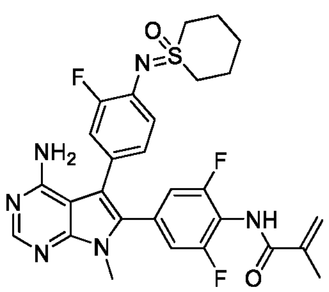
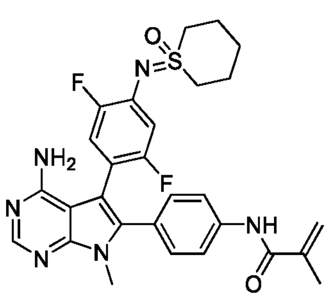
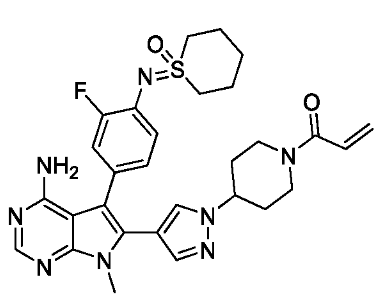

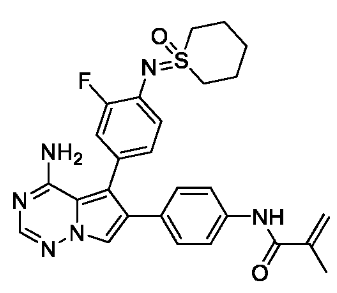
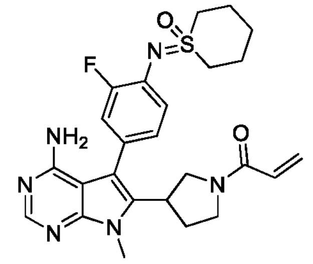
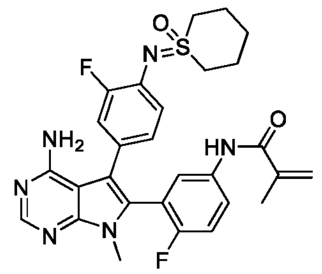

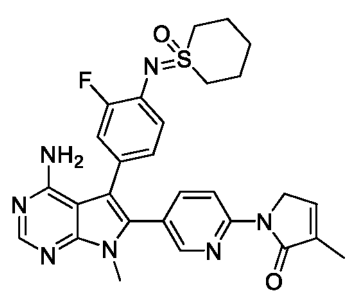
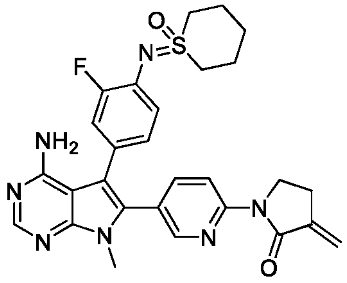
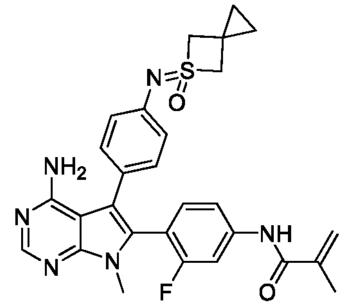

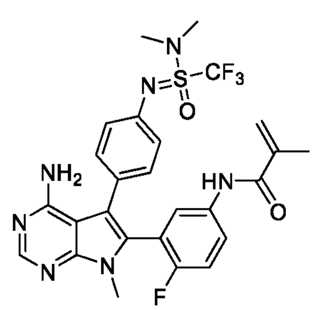
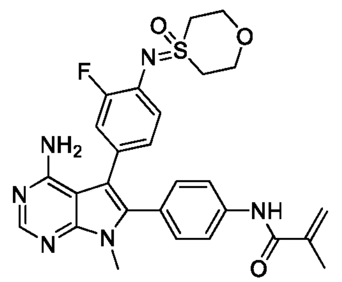
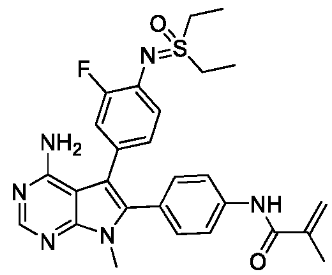

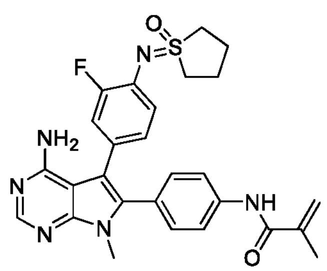
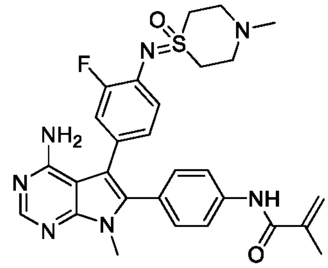
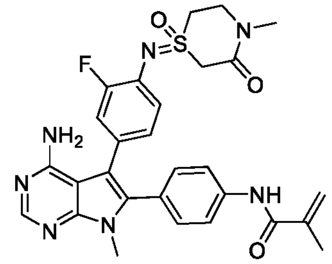

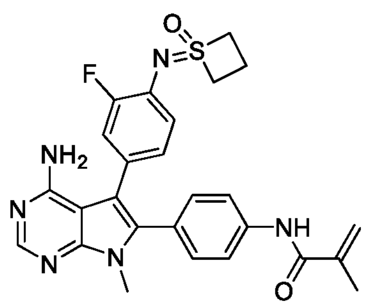
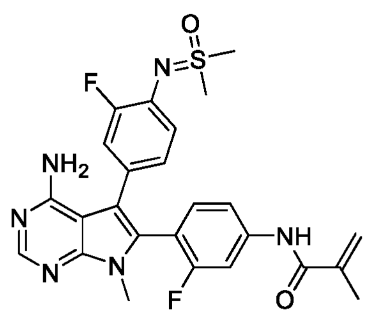
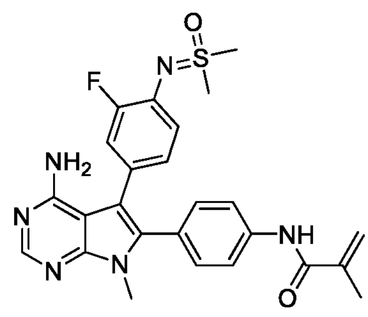

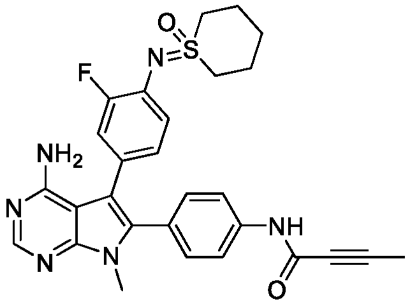

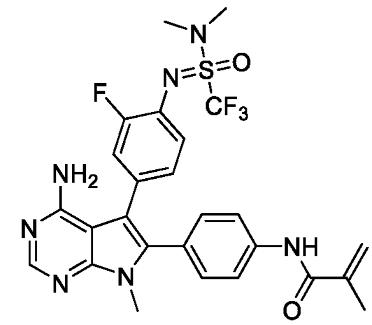

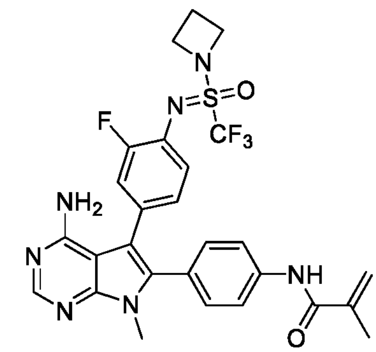

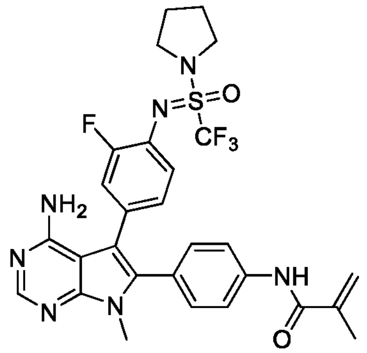

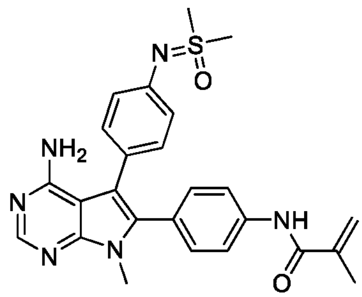

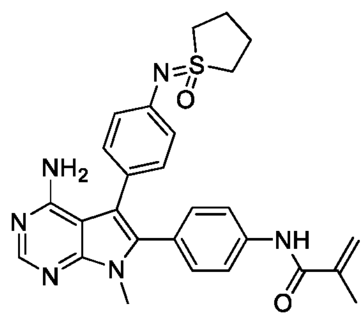

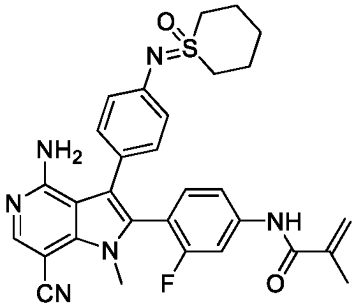

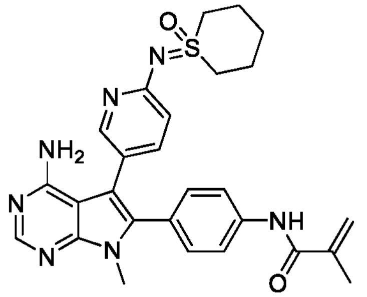

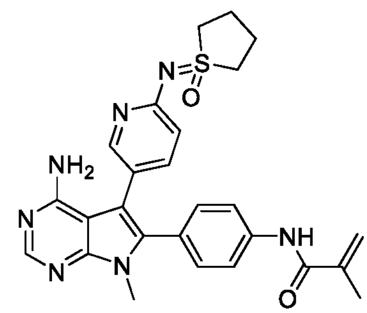

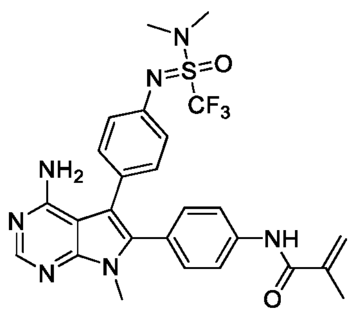

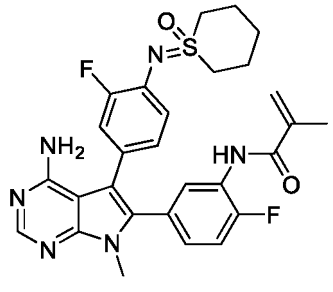

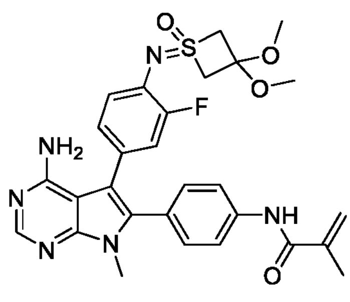

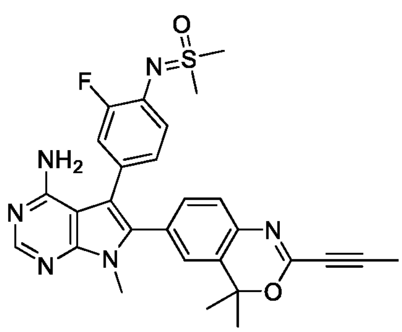
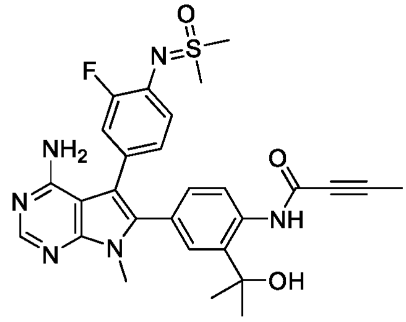
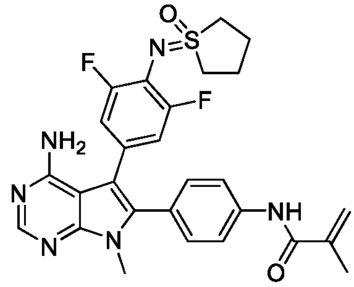

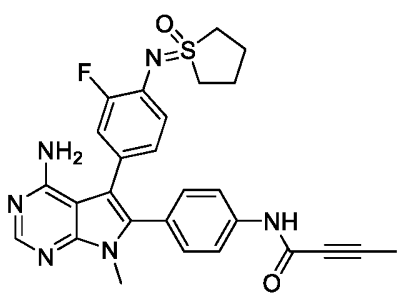
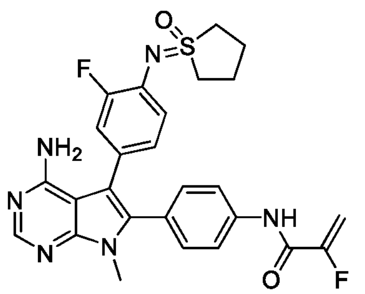
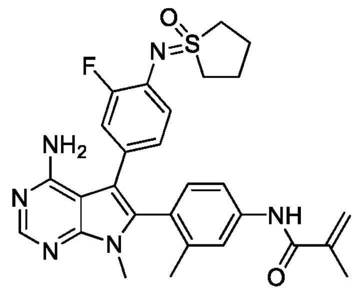

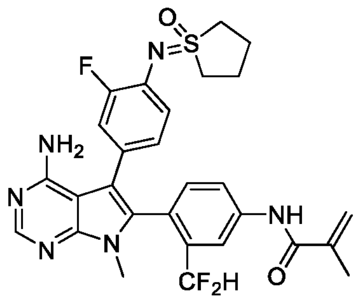
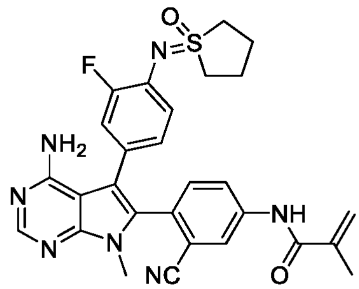
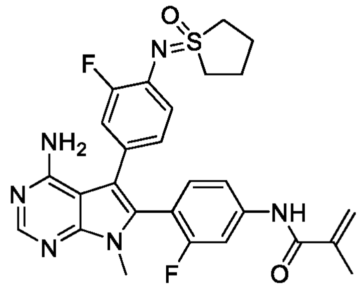

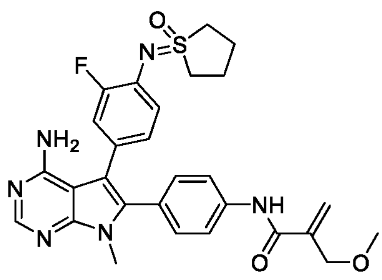
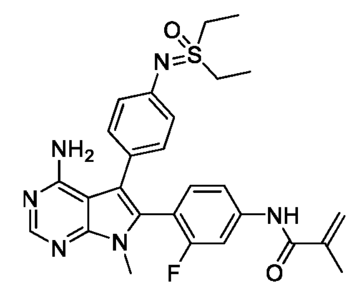
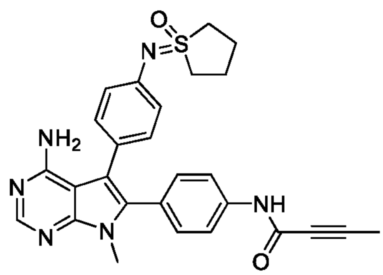

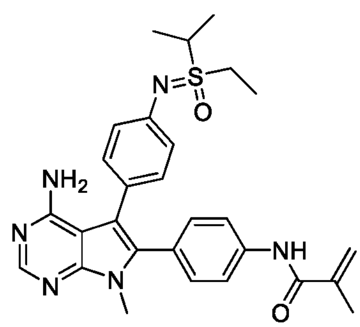
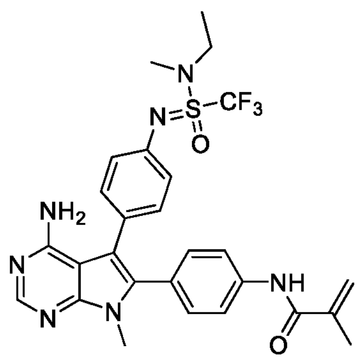
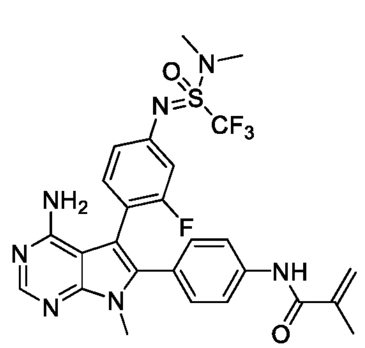

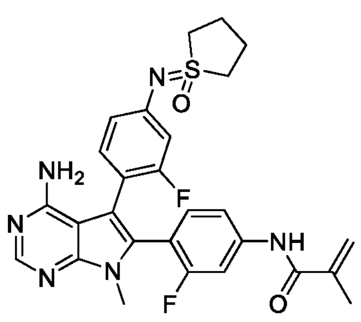

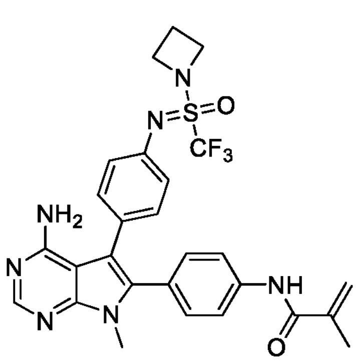

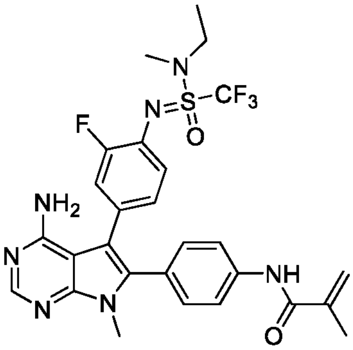

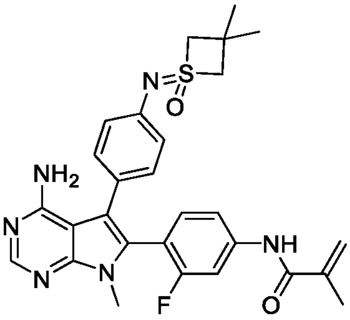

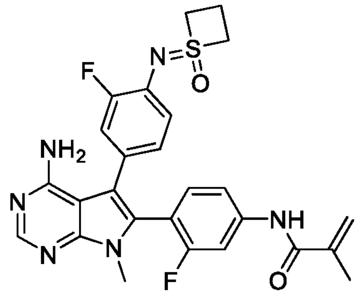

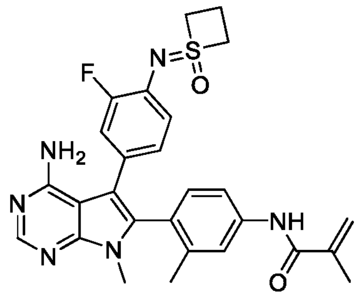

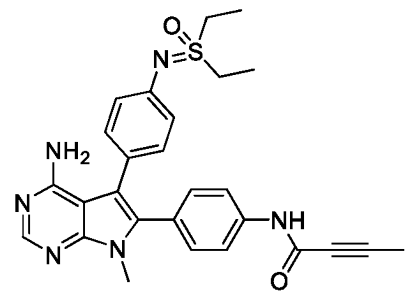

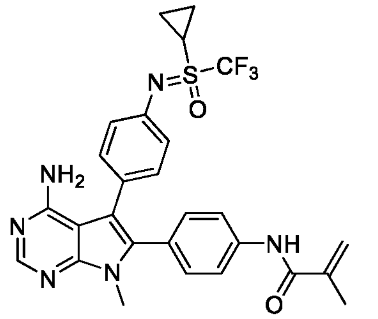

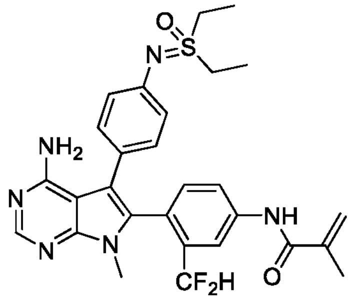

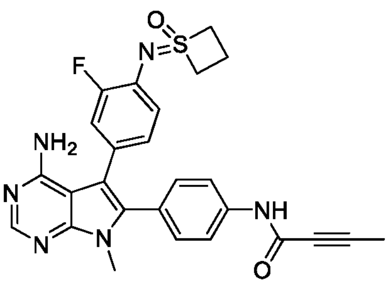

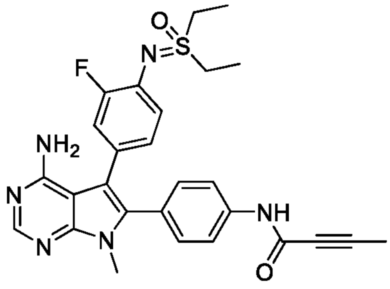

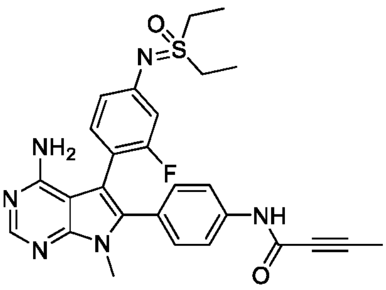

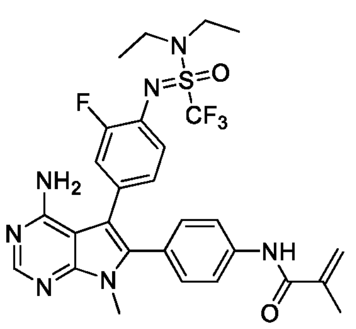
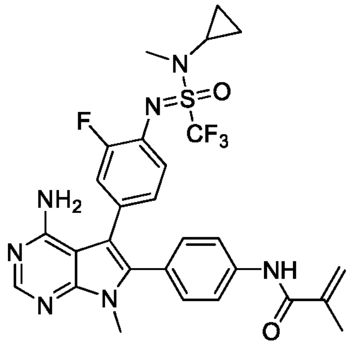
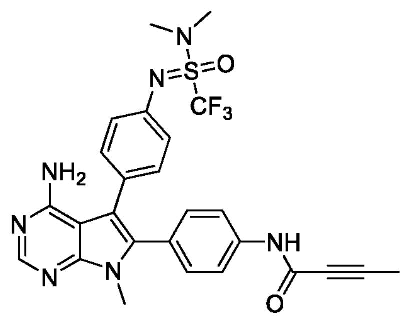

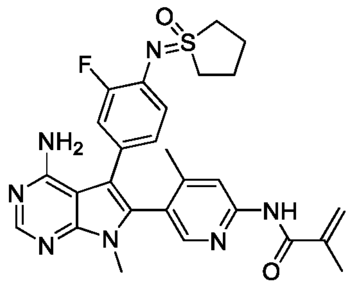
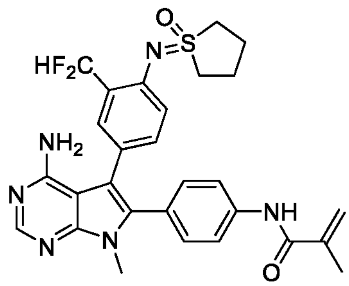
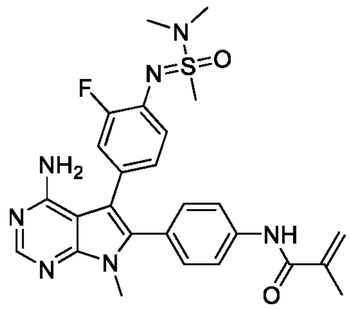

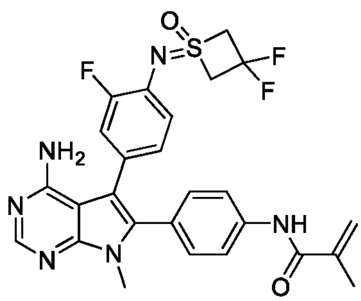
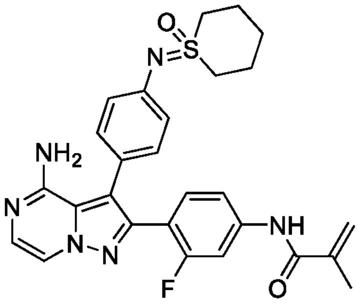
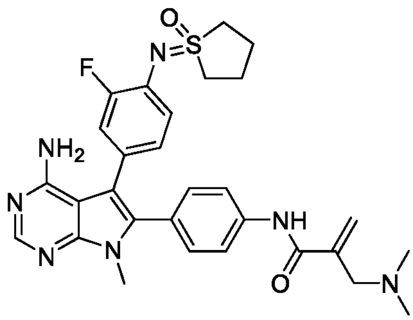

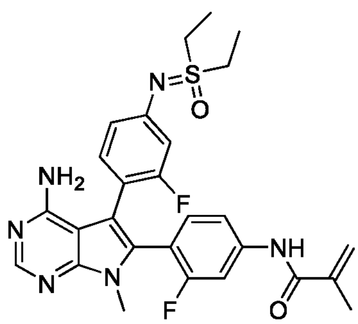
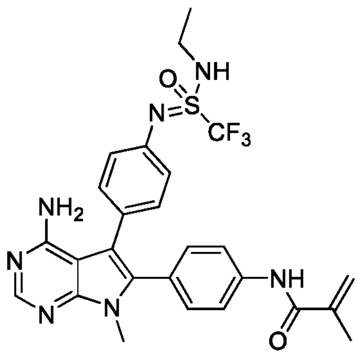
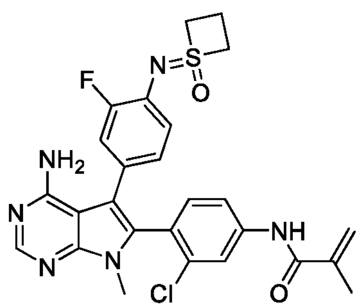

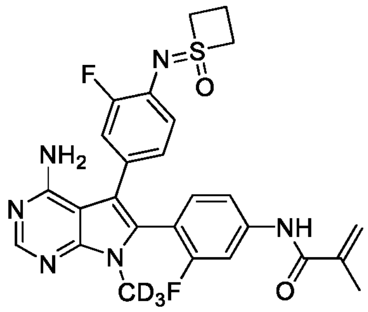

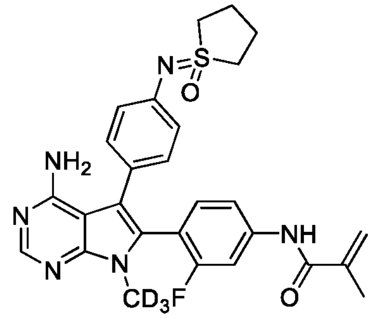

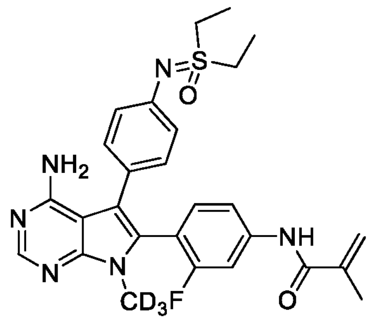

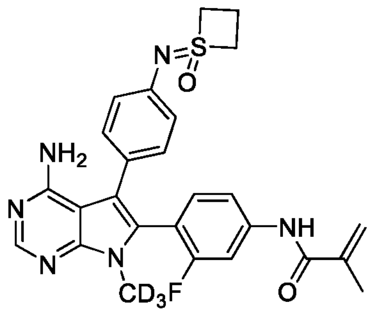

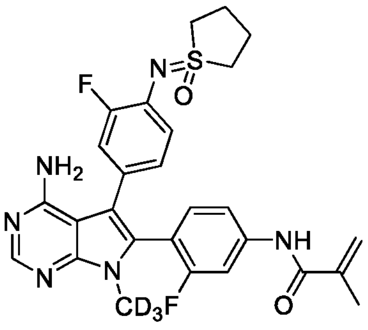

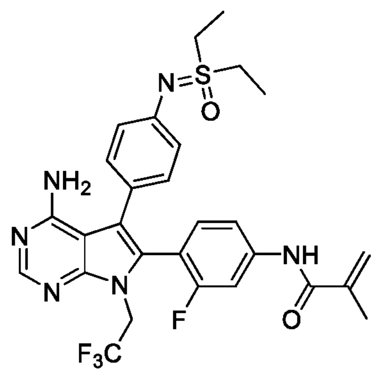

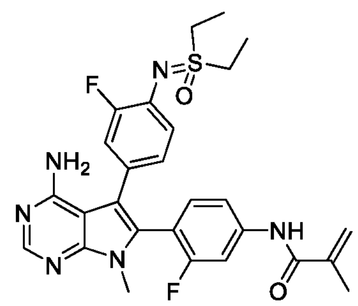

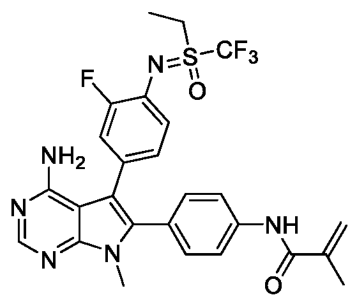

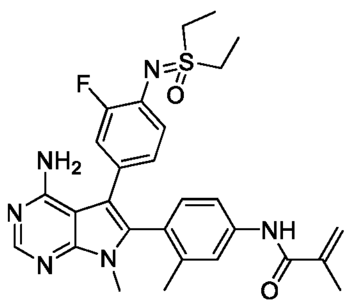

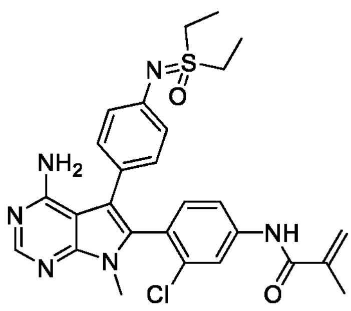

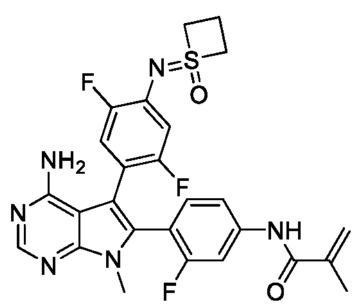

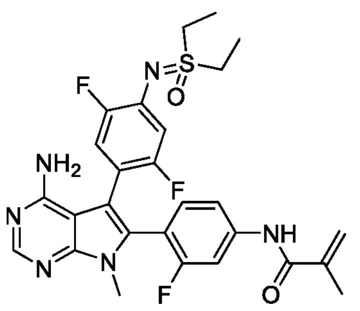

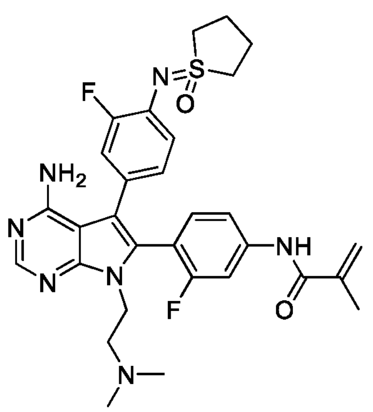
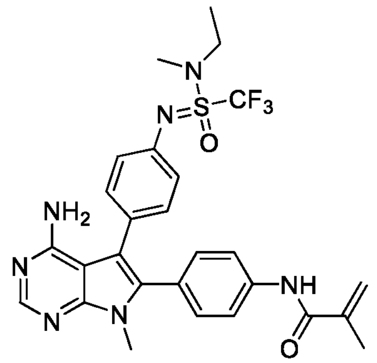
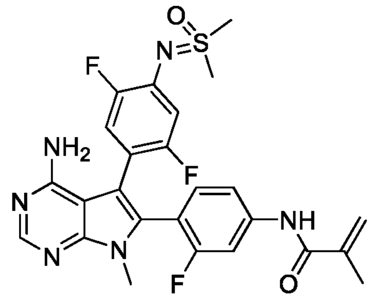

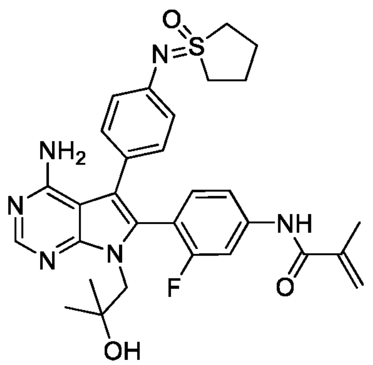
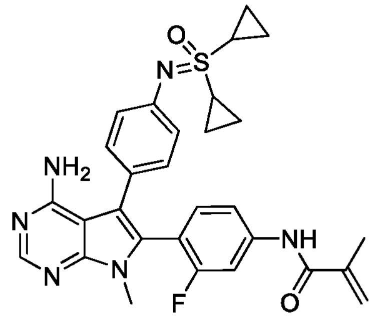
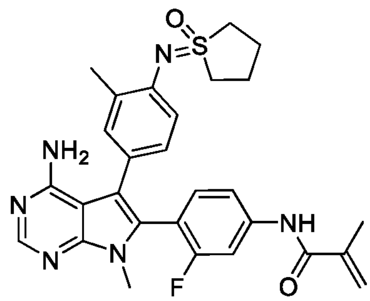

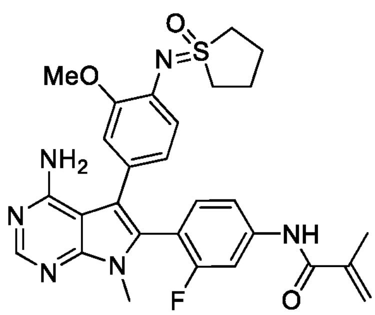
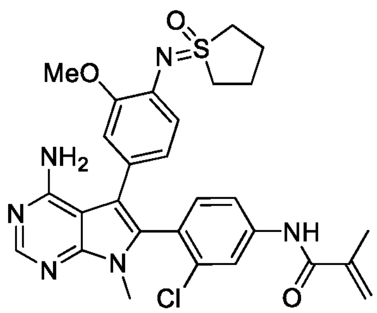
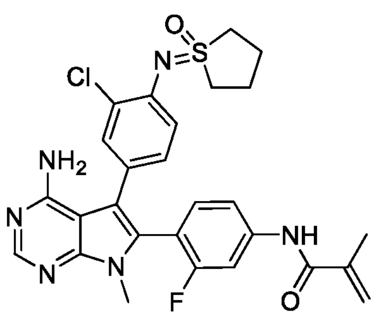

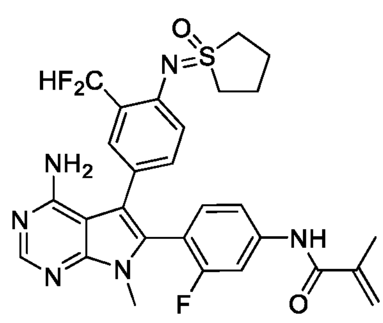
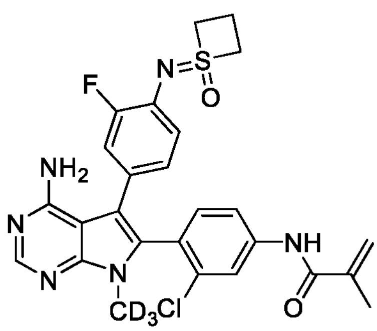
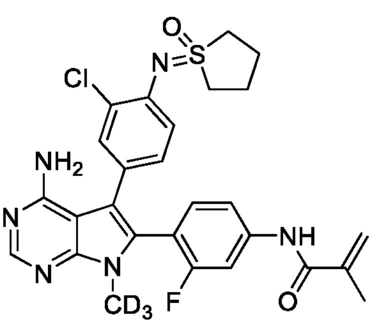

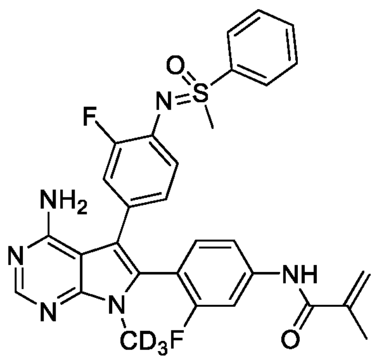
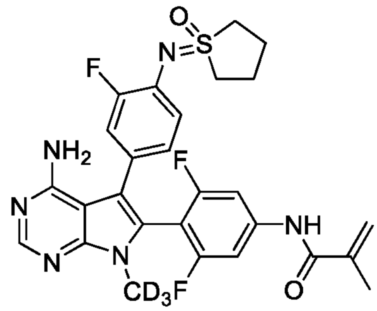
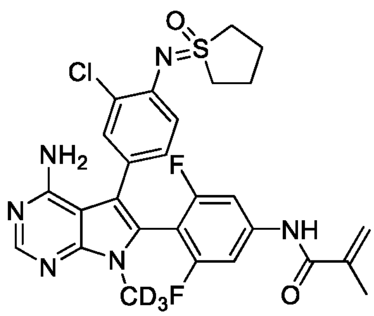

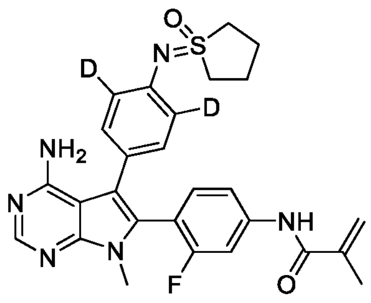
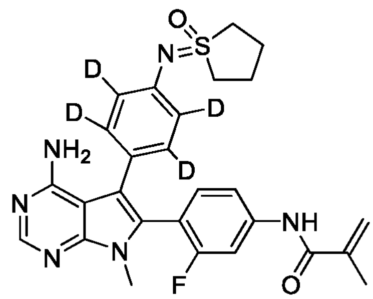
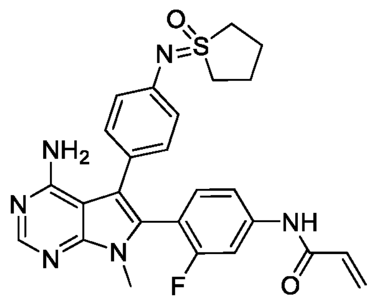

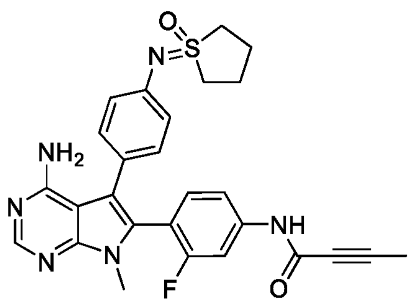
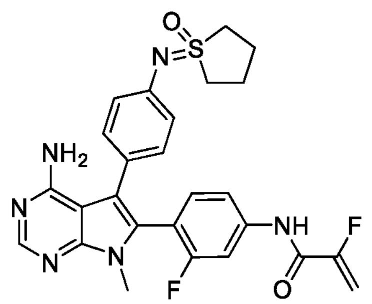
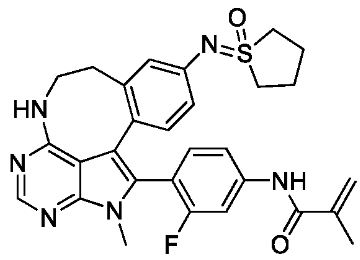.

**9.** A pharmaceutical composition, comprising:

(1) one or more of the compound according to claim 1, or a tautomer, a stereoisomer, a hydrate, a solvate, or a pharmaceutically acceptable salt thereof;
(2) one or more pharmaceutically acceptable carriers, excipients, adjuvants, excipients, and/or diluents.
In another preferred embodiment, the pharmaceutical composition is used for treating or preventing diseases or condition associated with abnormal activity or expression levels of FGFR; preferably, the disease or condition is selected from the group consisting of: cholangiocarcinoma, hepatic carcinoma, breast carcinoma, prostate carcinoma, lung carcinoma, thyroid carcinoma, gastric carcinoma, ovarian carcinoma, rectal carcinoma, endometrial carcinoma and uroepithelial carcinoma.
In another preferred embodiment, the cholangiocarcinoma is intrahepatic cholangiocarcinoma.
In another preferred embodiment, the hepatic carcinoma is hepatocellular carcinoma.
In another preferred embodiment, the lung carcinoma is squamous cell carcinoma or non-small cell lung carcinoma.

**10.** The use of the compound according to claim 1, or a tautomer, a stereoisomer, a hydrate, a solvate, or a pharmaceutically acceptable salt thereof, or mixtures thereof in the preparation a pharmaceutical composition for treating or preventing diseases or conditions associated with abnormal activity or expression levels of FGFR.
In another preferred embodiment, the disease or condition is specifically associated with a subtype of FGFR; preferably, the FGFR subtype is selected from the group consisting of: FGFR2.

## INTERNATIONAL SEARCH REPORT

International application No.
**PCT/CN2023/105960**

**A. CLASSIFICATION OF SUBJECT MATTER**

C07D487/04(2006.01)i; A61K31/519(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; VCN; VEN; CNABS; DWPI; WPABS; ENTXT; USTXT; EPTXT; WOTXT; STN; CNKI; ISI-Web of Science; 超星读秀, DUXIU; 万方, WANFANG: 亚砜, 亚胺, 纤维母细胞生长因子, 受体, 糖蛋白, 磷酸化, 癌, 肿瘤, 高磷酸盐血症, 组织矿化, 抑制剂, Sulfoxide, Imine, Fibroblast growth factor, Receptor, Glycoprotein, Phosphorylation, Cancer, Tumor, Hyperphosphatemia, Tissue Mineralization, Inhibitor, fgfr, 通式I-1, 通式I-2

**C. DOCUMENTS CONSIDERED TO BE RELEVANT**

| Category* | Citation of document, with indication, where appropriate, of the relevant passages | Relevant to claim No. |
|---|---|---|
| A | CN 114126620 A (COMMUNICATING THERAPY COMPANIES, LTD. et al.) 01 March 2022 (2022-03-01) description, paragraphs [0016]-[0107] and paragraphs [0417]-[0813] | 1-10 |
| A | CN 104245700 A (BAYER PHARMACEUTICAL AG et al.) 24 December 2014 (2014-12-24) entire document | 1-10 |
| A | CN 1326457 A (BASF AG) 12 December 2001 (2001-12-12) entire document | 1-10 |
| A | CN 1335849 A (BASF AG) 13 February 2002 (2002-02-13) entire document | 1-10 |
| A | WO 2022007841 A1 (ABBISKO THERAPEUTICS CO., LTD.) 13 January 2022 (2022-01-13) entire document | 1-10 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 October 2023** | **06 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/105960**

| Patent document cited in search report | Publication date (day/month/year) | Patent family member(s) | Publication date (day/month/year) |
|---|---|---|---|
| CN 114126620 A | 01 March 2022 | CA 3137458 A1 | 19 November 2020 |
| | | TW 202108141 A | 01 March 2021 |
| | | MX 2021013146 A | 24 January 2022 |
| | | IL 287940 A | 01 January 2022 |
| | | EP 3968999 A1 | 23 March 2022 |
| | | EP 3968999 A4 | 03 August 2022 |
| | | KR 20220007889 A | 19 January 2022 |
| | | JP 2022533570 A | 25 July 2022 |
| | | WO 2020231990 A1 | 19 November 2020 |
| | | BR 112021022457 A2 | 22 March 2022 |
| | | US 2023104574 A1 | 06 April 2023 |
| | | PE 20220573 A1 | 20 April 2022 |
| | | CL 2021002882 A1 | 07 October 2022 |
| | | US 2023192709 A1 | 22 June 2023 |
| | | AU 2020274083 A1 | 18 November 2021 |
| | | AR 118926 A1 | 10 November 2021 |
| | | SG 11202111327 A | 29 November 2021 |
| | | IN 202117048938 A | 25 February 2022 |
| | | HK 40070481 A0 | 28 October 2022 |
| CN 104245700 A | 24 December 2014 | ZA 201405140 B | 26 April 2017 |
| | | EP 2791140 A1 | 22 October 2014 |
| | | EP 2791140 B1 | 15 June 2016 |
| | | AU 2017206140 A1 | 03 August 2017 |
| | | BR 112014014531 A2 | 06 August 2019 |
| | | BR 112014014531 B1 | 08 February 2022 |
| | | US 2014336173 A1 | 13 November 2014 |
| | | MX 2014006905 A | 08 September 2014 |
| | | MX 367158 B | 07 August 2019 |
| | | EA 029556 B1 | 30 April 2018 |
| | | AU 2012350750 A1 | 12 June 2014 |
| | | AU 2012350750 B2 | 03 August 2017 |
| | | US 2019016724 A1 | 17 January 2019 |
| | | JP 2015500307 A | 05 January 2015 |
| | | JP 6050829 B2 | 21 December 2016 |
| | | US 2022153745 A1 | 19 May 2022 |
| | | MY 178660 A | 20 October 2020 |
| | | US 2013158000 A1 | 20 June 2013 |
| | | US 9206184 B2 | 08 December 2015 |
| | | CA 2859133 A1 | 20 June 2013 |
| | | CA 2859133 C | 24 March 2020 |
| | | PH 12014501355 A1 | 22 September 2014 |
| | | NZ 625073 A | 29 July 2016 |
| | | TW 201339162 A | 01 October 2013 |
| | | WO 2013087578 A1 | 20 June 2013 |
| | | KR 20140103328 A | 26 August 2014 |
| | | KR 102057444 B1 | 19 December 2019 |
| | | AU 2019204255 A1 | 04 July 2019 |
| | | ES 2591203 T3 | 25 November 2016 |
| | | IL 232611 A1 | 30 June 2014 |
| | | SG 11201402325 A1 | 26 September 2014 |

Form PCT/ISA/210 (patent family annex) (July 2022)

**INTERNATIONAL SEARCH REPORT**
**Information on patent family members**

International application No.
**PCT/CN2023/105960**

| Patent document cited in search report | Publication date (day/month/year) | Patent family member(s) | Publication date (day/month/year) |
|---|---|---|---|
| | | MX 2014006905 A1 | 30 September 2014 |
| | | VN 39660 A | 27 October 2014 |
| | | IN 201404310 P4 | 04 September 2015 |
| | | HK 1205123 A0 | 11 December 2015 |
| | | ID 201603319 A | 04 May 2016 |
| | | GC 5640 A | 30 June 2016 |
| | | CN 104245700 B | 16 November 2016 |
| | | SG 11201402325 B | 21 November 2016 |
| | | TW 567076 B1 | 21 January 2017 |
| | | ZA 201405140 A | 26 April 2017 |
| | | HK 1205123 A1 | 02 February 2018 |
| | | VN 10020601 B | 25 March 2019 |
| | | IN 327756 B | 20 December 2019 |
| | | PH 12014501355 B1 | 07 January 2020 |
| CN 1326457 A | 12 December 2001 | JP 2002527359 A | 27 August 2002 |
| | | CZ 2001959 A3 | 12 December 2001 |
| | | NZ 510587 A | 28 November 2003 |
| | | HU 0200355 A2 | 29 June 2002 |
| | | BR 9913888 A | 08 January 2002 |
| | | ES 2253930 T3 | 01 June 2006 |
| | | EP 1114052 A1 | 11 July 2001 |
| | | EP 1114052 B1 | 16 November 2005 |
| | | AU 6047599 A | 10 April 2000 |
| | | AU 752474 B2 | 19 September 2002 |
| | | DE 69928414 D1 | 22 December 2005 |
| | | DE 69928414 T2 | 03 August 2006 |
| | | KR 20010085822 A | 07 September 2001 |
| | | SK 3852001 A3 | 04 March 2003 |
| | | WO 0017202 A1 | 30 March 2000 |
| | | NO 20011357 A | 14 May 2001 |
| | | MX 2001002784 A1 | 01 November 2001 |
| | | ZA 200102201 A | 29 May 2002 |
| | | HU 0200355 A2 | 28 June 2002 |
| | | US 2003187001 A1 | 02 October 2003 |
| | | IN 200100364 P4 | 04 March 2005 |
| | | US 7863444 B2 | 04 January 2011 |
| CN 1335849 A | 13 February 2002 | EP 1114053 A1 | 11 July 2001 |
| | | AU 6048499 A | 10 April 2000 |
| | | AU 753555 B2 | 24 October 2002 |
| | | KR 20010085824 A | 07 September 2001 |
| | | JP 2002526500 A | 20 August 2002 |
| | | CZ 2001960 A3 | 17 October 2001 |
| | | WO 0017203 A1 | 30 March 2000 |
| | | SK 3842001 A3 | 04 April 2002 |
| | | NZ 510588 A | 29 August 2003 |
| | | BR 9913887 A | 23 October 2001 |
| | | NO 20011356 A | 16 May 2001 |
| | | NO 321521 B1 | 15 May 2006 |
| | | MX 2001002785 A1 | 01 November 2001 |
| | | ZA 200102204 A | 29 May 2002 |

Form PCT/ISA/210 (patent family annex) (July 2022)

**INTERNATIONAL SEARCH REPORT**
**Information on patent family members**

International application No.
**PCT/CN2023/105960**

| Patent document cited in search report | Publication date (day/month/year) | Patent family member(s) | Publication date (day/month/year) |
|---|---|---|---|
| | | HU 0200403 A2 | 28 June 2002 |
| | | US 2003153752 A1 | 14 August 2003 |
| | | US 6713474 B2 | 30 March 2004 |
| | | IN 200100376 P4 | 04 March 2005 |
| WO 2022007841 A1 | 13 January 2022 | CN 116096372 A | 09 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020231990 A1
- WO 2022109577 A1
- WO 2023046117 A1
- WO 2022152705 A1
- WO 2023001794 A1


### Non-patent literature cited in the description


- *ACS Catalysis*, 2016, vol. 6 (11), 7814-7823
- *Organic Letters*, 2021, vol. 23 (5), 1554-1560